# EUROPEAN PATENT SPECIFICATION

(11) **EP 1 882 045 B1**
(45) Date of publication and mention of the grant of the patent: **29.08.2012**
(21) Application number: 06751025.5
(22) Date of filing: 21.04.2006
(51) Int. Cl.: C12Q 1/68

(54) **COMPOSITION FOR IDENTIFICATION OF Staphylococcus aureus**
ZUSAMMENSETZUNG ZUR IDENTIFIZIERUNG VON Staphylococcus aureus
COMPOSITION A UTILISER DANS L'IDENTIFICATION DE Staphylococcus aureus

(30) Priority: 21.04.2005 US 674118 P; 03.08.2005 US 705631 P; 01.11.2005 US 732539 P; 13.02.2006 US 773124 P
(43) Date of publication of application: 30.01.2008
(73) Proprietor: Ibis Biosciences, Inc., Carlsbad, CA 92008 (US)
(72) Inventor: SAMPATH, Rangarajan, San Diego, California 92129 (US); HALL, Thomas, A., Oceanside, California 92056 (US); BLYN, Lawrence, Mission Viejo, CA 92692 (US)
(74) Representative: Hallybone, Huw George
(86) International application number: PCT/US2006/015160
(87) International publication number: WO 2006/116127

(56) References cited:
- US-A- 5 702 895
- DATABASE EMBL embl; arabidopsis thaliana T-DNA flanking sequence AJ 29 March 2003 (2003-03-29), BRUNAUD V. ET AL.: XP002395797 retrieved from EMBL Database accession no. AJ552897
- DATABASE EMBL embl; 18 August 2003 (2003-08-18), XP002395798 retrieved from EMBL Database accession no. AR321656 & US 6 563 025 B1 (BOARD OF TRUSTEES OF THE UNIVERSITY OF ILLINOIS) 13 May 2003 (2003-05-13)
- DEURENBERG R H ET AL: "Rapid detection of Panton-Valentine leukocidin from clinical isolates of Staphylococcus aureus strains by real-time PCR" FEMS MICROBIOLOGY LETTERS, AMSTERDAM, NL, vol. 240, no. 2, 15 November 2004 (2004-11-15), pages 225-228, XP004797128 ISSN: 0378-1097
- NAKAGAWA S ET AL: "Gene sequences and specific detection for Panton-Valentine leukocidin" BIOCHEMICAL AND BIOPHYSICAL RESEARCH COMMUNICATIONS, ACADEMIC PRESS INC. ORLANDO, FL, US, vol. 328, no. 4, 25 March 2005 (2005-03-25), pages 995-1002, XP004747569 ISSN: 0006-291X
- GRAVET A ET AL: "Characterization of a novel structural member, LukE-LukD, of the bi-component staphylococcal leucotoxins family" FEBS LETTERS, ELSEVIER, AMSTERDAM, NL, vol. 436, no. 2, 2 October 1998 (1998-10-02), pages 202-208, XP004258421 ISSN: 0014-5793
- NARITA S ET AL: "Phage conversion of Panton-Valentine leukocidin in Staphylococcus aureus: molecular analysis of a PVL-converting phage, phiSLT" GENE, ELSEVIER, AMSTERDAM, NL, vol. 268, no. 1-2, 2 May 2001 (2001-05-02), pages 195-206, XP004240577 ISSN: 0378-1119
- BABA T. ET AL.,: "genome and virulence determinants of high virulence community -acquired MRSA" THE LANCET, vol. 359, 25 May 2002 (2002-05-25), pages 1819-1827, XP002395794
- HAMELS S ET AL: "CONSENSUS PCR AND MICROARRAY FOR DIAGNOSIS OF THE GENUS STAPHYLOCOCCUS, SPECIES, AND METHICILLIN RESISTANCE" BIOTECHNIQUES, INFORMA LIFE SCIENCES PUBLISHING, WESTBOROUGH, MA, US, vol. 31, no. 6, December 2001 (2001-12), pages 1364-1366,1368, XP001153876 ISSN: 0736-6205

## Description

### STATEMENT OF GOVERNMENT SUPPORT

This invention was made with United States Government support under CDC contract RO1 CI000099-01. The United States Government has certain rights in the invention.

### FIELD OF THE INVENTION

The present invention provides compositions, kits and methods for rapid identification and quantification of bacteria by molecular mass and base composition analysis.

### BACKGROUND OF THE INVENTION

A problem in determining the cause of a natural infectious outbreak or a bioterrorist attack is the sheer variety of organisms that can cause human disease. There are over 1400 organisms infectious to humans; many of these have the potential to emerge suddenly in a natural epidemic or to be used in a malicious attack by bioterrorists (Taylor et al. Philos. Trans. R. Soc. London B. Biol. Sci., 2001, 356, 983-989). This number does not include numerous strain variants, bioengineered versions, or pathogens that infect plants or animals.

Much of the new technology being developed for detection of biological weapons incorporates a polymerase chain reaction (PCR) step based upon the use of highly specific primers and probes designed to selectively detect certain pathogenic organisms. Although this approach is appropriate for the most obvious bioterrorist organisms, like smallpox and anthrax, experience has shown that it is very difficult to predict which of hundreds of possible pathogenic organisms might be employed in a terrorist attack. Likewise, naturally emerging human disease that has caused devastating consequence in public health has come from unexpected families of bacteria, viruses, fungi, or protozoa. Plants and animals also have their natural burden of infectious disease agents and there are equally important biosafety and security concerns for agriculture.

A major conundrum in public health protection, biodefense, and agricultural safety and security is that these disciplines need to be able to rapidly identify and characterize infectious agents, while there is no existing technology with the breadth of function to meet this need. Currently used methods for identification of bacteria rely upon culturing the bacterium to effect isolation from other organisms and to obtain sufficient quantities of nucleic acid followed by sequencing of the nucleic acid, both processes which are time and labor intensive.
Deurenberg R H et al. (FEMS Microbiology Letters, 2004, vol. 240 pages 225-228) describes rapid detection of Panton-Valentine leukocidin from clinical isolates of *Staphylococcus aureus* strains by real-time PCR.
Nakagawa S et al. (Biochemical and Biophysical Research Communications, 2005, vol. 328, pages 995-1002) describes gene sequences and specific detection of Panton-Valentine leukocidin.
Gravet A et al. (FEBS Letters, 1998, vol. 436, pages 202-208) describes the characterization of a novel structural member, LukE-LukD, of the bi-component staphylococcal leucotoxins family.
US 5702895 describes methods and kits for detecting methicillin-resistant *Staphylococcus aureus.*
Baba T et al. (The Lancet, 2002, vol. 359, pages 1819-1827) describes virulence determinants of high virulence community-acquired MRSA.
Hamels S et al. (Biotechniques, 2001, vol. 31, no. 6, pages 1364-1372) describes using DNA microarrays for diagnosing *Staphylococcus*, e.g. methicillin resistance.
Narita S et al. (Gene, 2001, vol. 268, pages 195-206) describes phage conversion of Panton-Valentine leukocidin in *Staphylococcus aureus* and provides a molecular analysis of a PVL-converting phage, ΦSLT.

Mass spectrometry provides detailed information about the molecules being analyzed, including high mass accuracy. It is also a process that can be easily automated. DNA chips with specific probes can only determine the presence or absence of specifically anticipated organisms. Because there are hundreds of thousands of species of benign bacteria, some very similar in sequence to threat organisms, even arrays with 10,000 probes lack the breadth needed to identify a particular organism.

The present invention provides compositions and kits containing oligonucleotide primers, which define bacterial bioagent identifying amplicons and, upon amplification, produce corresponding amplification products whose molecular masses provide the means to identify bacteria, for example, at and below the species taxonomic level.

### SUMMARY OF THE INVENTION

The invention provides a composition comprising: an oligonucleotide primer having SEQ ID NO: 456; and an oligonucleotide primer having SEQ ID NO: 1261.

The invention also provides a kit comprising the composition of the invention, and further comprising one or more primer pairs from the group of primer pairs represented by SEQ ID NOs: 288:1269, 698:1420, 217:1167, 399:1041, 430:1321, 174:853, and 172:1360.

The invention also provides a method for identification of a strain of *Staphylococcus aureus* in a sample comprising: amplifying nucleic acid from said strain of *Staphylococcus aureus* using the composition of the invention to obtain an amplification product; determining the molecular mass of said amplification product; optionally, determining the base composition of said amplification product from said molecular mass; and comparing said molecular mass or said base composition with a plurality of molecular masses or base compositions of known amplification products of strains of *Staphylococcus aureus* defined by the composition of the invention, wherein a match between said molecular mass or base composition and a member of said plurality of molecular masses or base compositions identifies said strain of *Staphylococcus aureus.*

The invention further provides a method for determination of the quantity of a strain of *Staphylococcus aureus* in a sample comprising: contacting said sample with the composition of the invention and a known quality of a calibration polynucleotide comprising a calibration sequence; concurrently amplifying nucleic acid from said strain of *Staphylococcus aureus* and nucleic acid from said calibration polynucleotide in said sample with the composition of the invention to obtain a first amplification product comprising a bacterial bioagent identifying amplicon and a second amplification product comprising a calibration amplicon; determining the molecular mass and abundance for said bacterial bioagent identifying amplicon and said calibration amplicon; and distinguishing said bacterial bioagent identifying amplicon from said calibration amplicon based on molecular mass, wherein comparison of bacterial bioagent identifying amplicon abundance and calibration amplicon abundance indicates the quantity of said strain of *Staphylococcus aureus* in said sample.

### SUMMARY OF THE DISCLOSURE

The present disclosure includes compositions, kits and methods for rapid identification and quantification of bacteria by molecular mass and base composition analysis.

One embodiment is an oligonucleotide primer 14 to 35 nucleobases in length having at least 70% sequence identity with SEQ ID NO: 456.

Another embodiment is an oligonucleotide primer 14 to 35 nucleobases in length having at least 70% sequence identity with SEQ ID NO: 1261.

Another embodiment is an oligonucleotide primer pair including an oligonucleotide primer 14 to 35 nucleobases in length having at least 70% sequence identity with SEQ ID NO: 456 and an oligonucleotide primer 14 to 35 nucleobases in length having at least 70% sequence identity with SEQ ID NO: 1261.

One embodiment is an oligonucleotide primer 14 to 35 nucleobases in length having at least 70% sequence identity with SEQ ID NO: 288.

Another embodiment is an oligonucleotide primer 14 to 35 nucleobases in length having at least 70% sequence identity with SEQ ID NO: 1269.

Another embodiment is an oligonucleotide primer pair including an oligonucleotide primer 14 to 35 nucleobases in length having at least 70% sequence identity with SEQ ID NO: 288 and an oligonucleotide primer 14 to 35 nucleobases in length having at least 70% sequence identity with SEQ ID NO: 1269.

One embodiment is an oligonucleotide primer 14 to 35 nucleobases in length having at least 70% sequence identity with SEQ ID NO: 698.

Another embodiment is an oligonucleotide primer 14 to 35 nucleobases in length having at least 70% sequence identity with SEQ ID NO: 1420.

Another embodiment is an oligonucleotide primer pair including an oligonucleotide primer 14 to 35 nucleobases in length having at least 70% sequence identity with SEQ ID NO: 698 and an oligonucleotide primer 14 to 35 nucleobases in length having at least 70% sequence identity with SEQ ID NO: 1420.

One embodiment is an oligonucleotide primer 14 to 35 nucleobases in length having at least 70% sequence identity with SEQ ID NO: 217.

Another embodiment is an oligonucleotide primer 14 to 35 nucleobases in length having at least 70% sequence identity with SEQ ID NO: 1167

Another embodiment is an oligonucleotide primer pair including an oligonucleotide primer 14 to 35 nucleobases in length having at least 70% sequence identity with SEQ ID NO: 217 and an oligonucleotide primer 14 to 35 nucleobases in length having at least 70% sequence identity with SEQ ID NO: 1167.

One embodiment is an oligonucleotide primer 14 to 35 nucleobases in length having at least 70% sequence identity with SEQ ID NO: 399.

Another embodiment is an oligonucleotide primer 14 to 35 nucleobases in length having at least 70% sequence identity with SEQ ID NO: 1041.

Another embodiment is an oligonucleotide primer pair including an oligonucleotide primer 14 to 35 nucleobases in length having at least 70% sequence identity with SEQ ID NO: 399 and an oligonucleotide primer 14 to 35 nucleobases in length having at least 70% sequence identity with SEQ ID NO: 1041.

One embodiment is an oligonucleotide primer 14 to 35 nucleobases in length having at least 70% sequence identity with SEQ ID NO: 430.

Another embodiment is an oligonucleotide primer 14 to 35 nucleobases in length having at least 70% sequence identity with SEQ ID NO: 1321.

Another embodiment is an oligonucleotide primer pair including an oligonucleotide primer 14 to 35 nucleobases in length having at least 70% sequence identity with SEQ ID NO: 430 and an oligonucleotide primer 14 to 35 nucleobases in length having at least 70% sequence identity with SEQ ID NO: 1321.

One embodiment is an oligonucleotide primer 14 to 35 nucleobases in length having at least 70% sequence identity with SEQ ID NO: 174.

Another embodiment is an oligonucleotide primer 14 to 35 nucleobases in length having at least 70% sequence identity with SEQ ID NO: 853.

Another embodiment is an oligonucleotide primer pair including an oligonucleotide primer 14 to 35 nucleobases in length having at least 70% sequence identity with SEQ ID NO: 174 and an oligonucleotide primer 14 to 35 nucleobases in length having at least 70% sequence identity with SEQ ID NO: 853.

One embodiment is an oligonucleotide primer 14 to 35 nucleobases in length having at least 70% sequence identity with SEQ ID NO: 172.

Another embodiment is an oligonucleotide primer 14 to 35 nucleobases in length having at least 70% sequence identity with SEQ ID NO: 1360.

Another embodiment is an oligonucleotide primer pair including an oligonucleotide primer 14 to 35 nucleobases in length having at least 70% sequence identity with SEQ ID NO: 172 and an oligonucleotide primer 14 to 35 nucleobases in length having at least 70% sequence identity with SEQ ID NO: 1360.

Another embodiment is a kit comprising an oligonucleotide primer pair including an oligonucleotide primer 14 to 35 nucleobases in length having at least 70% sequence identity with SEQ ID NO: 456 and an oligonucleotide primer 14 to 35 nucleobases in length having at least 70% sequence identity with SEQ ID NO: 1261.

Another embodiment is a kit comprising an oligonucleotide primer pair including an oligonucleotide primer 14 to 35 nucleobases in length having at least 70% sequence identity with SEQ ID NO: 456 and an oligonucleotide primer 14 to 35 nucleobases in length having at least 70% sequence identity with SEQ ID NO: 1261 and further comprising one or more primer pairs wherein each member of said one or more primer pairs is of a length of 14 to 35 nucleobases and has 70% to 100% sequence identity with the corresponding member from the group of primer pairs represented by SEQ ID NOs: 288:1269, 698:1420, 217:1167, 399:1041, 430:1321, 174:853, and 172:1360.

One embodiment is an oligonucleotide primer 14 to 35 nucleobases in length having at least 70% sequence identity with SEQ ID NO: 681.

Another embodiment is an oligonucleotide primer 14 to 35 nucleobases in length having at least 70% sequence identity with SEQ ID NO: 1022.

Another embodiment is an oligonucleotide primer pair including an oligonucleotide primer 14 to 35 nucleobases in length having at least 70% sequence identity with SEQ ID NO: 681 and an oligonucleotide primer 14 to 35 nucleobases in length having at least 70% sequence identity with SEQ ID NO: 1022.

One embodiment is an oligonucleotide primer 14 to 35 nucleobases in length having at least 70% sequence identity with SEQ ID NO: 315.

Another embodiment is an oligonucleotide primer 14 to 35 nucleobases in length having at least 70% sequence identity with SEQ ID NO: 1379.

Another embodiment is an oligonucleotide primer pair including an oligonucleotide primer 14 to 35 nucleobases in length having at least 70% sequence identity with SEQ ID NO: 315 and an oligonucleotide primer 14 to 35 nucleobases in length having at least 70% sequence identity with SEQ ID NO: 1379.

One embodiment is an oligonucleotide primer 14 to 35 nucleobases in length having at least 70% sequence identity with SEQ ID NO: 346.

Another embodiment is an oligonucleotide primer 14 to 35 nucleobases in length having at least 70% sequence identity with SEQ ID NO: 955.

Another embodiment is an oligonucleotide primer pair including an oligonucleotide primer 14 to 35 nucleobases in length having at least 70% sequence identity with SEQ ID NO: 346 and an oligonucleotide primer 14 to 35 nucleobases in length having at least 70% sequence identity with SEQ ID NO: 955.

One embodiment is an oligonucleotide primer 14 to 35 nucleobases in length having at least 70% sequence identity with SEQ ID NO: 504.

Another embodiment is an oligonucleotide primer 14 to 35 nucleobases in length having at least 70% sequence identity with SEQ ID NO: 1409.

Another embodiment is an oligonucleotide primer pair including an oligonucleotide primer 14 to 35 nucleobases in length having at least 70% sequence identity with SEQ ID NO: 504 and an oligonucleotide primer 14 to 35 nucleobases in length having at least 70% sequence identity with SEQ ID NO: 1409.

One embodiment is an oligonucleotide primer 14 to 35 nucleobases in length having at least 70% sequence identity with SEQ ID NO: 323.

Another embodiment is an oligonucleotide primer 14 to 35 nucleobases in length having at least 70% sequence identity with SEQ ID NO: 1068.

Another embodiment is an oligonucleotide primer pair including an oligonucleotide primer 14 to 35 nucleobases in length having at least 70% sequence identity with SEQ ID NO: 323 and an oligonucleotide primer 14 to 35 nucleobases in length having at least 70% sequence identity with SEQ ID NO: 1068.

One embodiment is an oligonucleotide primer 14 to 35 nucleobases in length having at least 70% sequence identity with SEQ ID NO: 479.

Another embodiment is an oligonucleotide primer 14 to 35 nucleobases in length having at least 70% sequence identity with SEQ ID NO: 938.

Another embodiment is an oligonucleotide primer pair including an oligonucleotide primer 14 to 35 nucleobases in length having at least 70% sequence identity with SEQ ID NO: 479 and an oligonucleotide primer 14 to 35 nucleobases in length having at least 70% sequence identity with SEQ ID NO: 938.

Another embodiment is a kit comprising an oligonucleotide primer pair including an oligonucleotide primer 14 to 35 nucleobases in length having at least 70% sequence identity with SEQ ID NO: 681 and an oligonucleotide primer 14 to 35 nucleobases in length having at least 70% sequence identity with SEQ ID NO: 1022.

Another embodiment is a kit comprising an oligonucleotide primer pair including an oligonucleotide primer 14 to 35 nucleobases in length having at least 70% sequence identity with SEQ ID NO: 681 and an oligonucleotide primer 14 to 35 nucleobases in length having at least 70% sequence identity with SEQ ID NO: 1022 and further comprising one or more primer pairs wherein each member of said one or more primer pairs is of a length of 14 to 35 nucleobases and has 70% to 100% sequence identity with the corresponding member from the group of primer pairs represented by SEQ ID NOs: 315:1379, 346:955, 504:1409, 323:1068, 479:938.

One embodiment is an oligonucleotide primer 14 to 35 nucleobases in length having at least 70% sequence identity with SEQ ID NO: 583.

Another embodiment is an oligonucleotide primer 14 to 35 nucleobases in length having at least 70% sequence identity with SEQ ID NO: 923.

Another embodiment is a kit comprising an oligonucleotide primer pair including an oligonucleotide primer 14 to 35 nucleobases in length having at least 70% sequence identity with SEQ ID NO: 583 and an oligonucleotide primer 14 to 35 nucleobases in length having at least 70% sequence identity with SEQ ID NO: 923.

One embodiment is an oligonucleotide primer 14 to 35 nucleobases in length having at least 70% sequence identity with SEQ ID NO: 454.

Another embodiment is an oligonucleotide primer 14 to 35 nucleobases in length having at least 70% sequence identity with SEQ ID NO: 1418.

Another embodiment is a kit comprising an oligonucleotide primer pair including an oligonucleotide primer 14 to 35 nucleobases in length having at least 70% sequence identity with SEQ ID NO: 454 and an oligonucleotide primer 14 to 35 nucleobases in length having at least 70% sequence identity with SEQ ID NO: 1418.

One embodiment is an oligonucleotide primer 14 to 35 nucleobases in length having at least 70% sequence identity with SEQ ID NO: 250.

Another embodiment is an oligonucleotide primer 14 to 35 nucleobases in length having at least 70% sequence identity with SEQ ID NO: 902.

Another embodiment is a kit comprising an oligonucleotide primer pair including an oligonucleotide primer 14 to 35 nucleobases in length having at least 70% sequence identity with SEQ ID NO: 250 and an oligonucleotide primer 14 to 35 nucleobases in length having at least 70% sequence identity with SEQ ID NO: 902.

One embodiment is an oligonucleotide primer 14 to 35 nucleobases in length having at least 70% sequence identity with SEQ ID NO: 384.

Another embodiment is an oligonucleotide primer 14 to 35 nucleobases in length having at least 70% sequence identity with SEQ ID NO: 878.

Another embodiment is a kit comprising an oligonucleotide primer pair including an oligonucleotide primer 14 to 35 nucleobases in length having at least 70% sequence identity with SEQ ID NO: 384 and an oligonucleotide primer 14 to 35 nucleobases in length having at least 70% sequence identity with SEQ ID NO: 878.

One embodiment is an oligonucleotide primer 14 to 35 nucleobases in length having at least 70% sequence identity with SEQ ID NO: 694.

Another embodiment is an oligonucleotide primer 14 to 35 nucleobases in length having at least 70% sequence identity with SEQ ID NO: 1215.

Another embodiment is a kit comprising an oligonucleotide primer pair including an oligonucleotide primer 14 to 35 nucleobases in length having at least 70% sequence identity with SEQ ID NO: 694 and an oligonucleotide primer 14 to 35 nucleobases in length having at least 70% sequence identity with SEQ ID NO: 1215.

One embodiment is an oligonucleotide primer 14 to 35 nucleobases in length having at least 70% sequence identity with SEQ ID NO: 194.

Another embodiment is an oligonucleotide primer 14 to 35 nucleobases in length having at least 70% sequence identity with SEQ ID NO: 1173.

Another embodiment is a kit comprising an oligonucleotide primer pair including an oligonucleotide primer 14 to 35 nucleobases in length having at least 70% sequence identity with SEQ ID NO: 194 and an oligonucleotide primer 14 to 35 nucleobases in length having at least 70% sequence identity with SEQ ID NO: 1173.

One embodiment is an oligonucleotide primer 14 to 35 nucleobases in length having at least 70% sequence identity with SEQ ID NO: 375.

Another embodiment is an oligonucleotide primer 14 to 35 nucleobases in length having at least 70% sequence identity with SEQ ID NO: 890.

Another embodiment is a kit comprising an oligonucleotide primer pair including an oligonucleotide primer 14 to 35 nucleobases in length having at least 70% sequence identity with SEQ ID NO: 375 and an oligonucleotide primer 14 to 35 nucleobases in length having at least 70% sequence identity with SEQ ID NO: 890.

One embodiment is an oligonucleotide primer 14 to 35 nucleobases in length having at least 70% sequence identity with SEQ ID NO: 656.

Another embodiment is an oligonucleotide primer 14 to 35 nucleobases in length having at least 70% sequence identity with SEQ ID NO: 1224.

Another embodiment is a kit comprising an oligonucleotide primer pair including an oligonucleotide primer 14 to 35 nucleobases in length having at least 70% sequence identity with SEQ ID NO: 656 and an oligonucleotide primer 14 to 35 nucleobases in length having at least 70% sequence identity with SEQ ID NO: 1224.

One embodiment is an oligonucleotide primer 14 to 35 nucleobases in length having at least 70% sequence identity with SEQ ID NO: 618.

Another embodiment is an oligonucleotide primer 14 to 35 nucleobases in length having at least 70% sequence identity with SEQ ID NO: 1157.

Another embodiment is a kit comprising an oligonucleotide primer pair including an oligonucleotide primer 14 to 35 nucleobases in length having at least 70% sequence identity with SEQ ID NO: 618 and an oligonucleotide primer 14 to 35 nucleobases in length having at least 70% sequence identity with SEQ ID NO: 1157.

One embodiment is an oligonucleotide primer 14 to 35 nucleobases in length having at least 70% sequence identity with SEQ ID NO: 302.

Another embodiment is an oligonucleotide primer 14 to 35 nucleobases in length having at least 70% sequence identity with SEQ ID NO: 852.

Another embodiment is a kit comprising an oligonucleotide primer pair including an oligonucleotide primer 14 to 35 nucleobases in length having at least 70% sequence identity with SEQ ID NO: 302 and an oligonucleotide primer 14 to 35 nucleobases in length having at least 70% sequence identity with SEQ ID NO: 852.

One embodiment is an oligonucleotide primer 14 to 35 nucleobases in length having at least 70% sequence identity with SEQ ID NO: 199.

Another embodiment is an oligonucleotide primer 14 to 35 nucleobases in length having at least 70% sequence identity with SEQ ID NO: 889.

Another embodiment is a kit comprising an oligonucleotide primer pair including an oligonucleotide primer 14 to 35 nucleobases in length having at least 70% sequence identity with SEQ ID NO: 199 and an oligonucleotide primer 14 to 35 nucleobases in length having at least 70% sequence identity with SEQ ID NO: 889.

One embodiment is an oligonucleotide primer 14 to 35 nucleobases in length having at least 70% sequence identity with SEQ ID NO: 596.

Another embodiment is an oligonucleotide primer 14 to 35 nucleobases in length having at least 70% sequence identity with SEQ ID NO: 1169.

Another embodiment is a kit comprising an oligonucleotide primer pair including an oligonucleotide primer 14 to 35 nucleobases in length having at least 70% sequence identity with SEQ ID NO: 596 and an oligonucleotide primer 14 to 35 nucleobases in length having at least 70% sequence identity with SEQ ID NO: 1169.

One embodiment is an oligonucleotide primer 14 to 35 nucleobases in length having at least 70% sequence identity with SEQ ID NO: 150.

Another embodiment is an oligonucleotide primer 14 to 35 nucleobases in length having at least 70% sequence identity with SEQ ID NO: 1242.

Another embodiment is a kit comprising an oligonucleotide primer pair including an oligonucleotide primer 14 to 35 nucleobases in length having at least 70% sequence identity with SEQ ID NO: 150 and an oligonucleotide primer 14 to 35 nucleobases in length having at least 70% sequence identity with SEQ ID NO: 1242.

One embodiment is an oligonucleotide primer 14 to 35 nucleobases in length having at least 70% sequence identity with SEQ ID NO: 166.

Another embodiment is an oligonucleotide primer 14 to 35 nucleobases in length having at least 70% sequence identity with SEQ ID NO: 1069.

Another embodiment is a kit comprising an oligonucleotide primer pair including an oligonucleotide primer 14 to 35 nucleobases in length having at least 70% sequence identity with SEQ ID NO: 166 and an oligonucleotide primer 14 to 35 nucleobases in length having at least 70% sequence identity with SEQ ID NO: 1069.

One embodiment is an oligonucleotide primer 14 to 35 nucleobases in length having at least 70% sequence identity with SEQ ID NO: 166.

Another embodiment is an oligonucleotide primer 14 to 35 nucleobases in length having at least 70% sequence identity with SEQ ID NO: 1168.

Another embodiment is a kit comprising an oligonucleotide primer pair including an oligonucleotide primer 14 to 35 nucleobases in length having at least 70% sequence identity with SEQ ID NO: 166 and an oligonucleotide primer 14 to 35 nucleobases in length having at least 70% sequence identity with SEQ ID NO: 1168.

Another embodiment is a kit comprising an oligonucleotide primer pair including an oligonucleotide primer 14 to 35 nucleobases in length having at least 70% sequence identity with SEQ ID NO: 583 and an oligonucleotide primer 14 to 35 nucleobases in length having at least 70% sequence identity with SEQ ID NO: 923 and further comprising one or more primer pairs wherein each member of said one or more primer pairs is of a length of 14 to 35 nucleobases and has 70% to 100% sequence identity with the corresponding member from the group of primer pairs represented by SEQ ID NOs: 454:1418, 250:902, 384:878, 694:1215, 194:1173, 375:890, 656:1224, 618:1157, 302:852, 199:889, 596:1169, 150:1242, 166:1069 and 166:1168.

One embodiment is an oligonucleotide primer 14 to 35 nucleobases in length having at least 70% sequence identity with SEQ ID NO: 437.

Another embodiment is an oligonucleotide primer 14 to 35 nucleobases in length having at least 70% sequence identity with SEQ ID NO: 1137.

Another embodiment is a kit comprising an oligonucleotide primer pair including an oligonucleotide primer 14 to 35 nucleobases in length having at least 70% sequence identity with SEQ ID NO: 437 and an oligonucleotide primer 14 to 35 nucleobases in length having at least 70% sequence identity with SEQ ID NO: 1137.

One embodiment is an oligonucleotide primer 14 to 35 nucleobases in length having at least 70% sequence identity with SEQ ID NO: 530.

Another embodiment is an oligonucleotide primer 14 to 35 nucleobases in length having at least 70% sequence identity with SEQ ID NO: 891.

Another embodiment is a kit comprising an oligonucleotide primer pair including an oligonucleotide primer 14 to 35 nucleobases in length having at least 70% sequence identity with SEQ ID NO: 530 and an oligonucleotide primer 14 to 35 nucleobases in length having at least 70% sequence identity with SEQ ID NO: 891.

One embodiment is an oligonucleotide primer 14 to 35 nucleobases in length having at least 70% sequence identity with SEQ ID NO: 474.

Another embodiment is an oligonucleotide primer 14 to 35 nucleobases in length having at least 70% sequence identity with SEQ ID NO: 869.

Another embodiment is a kit comprising an oligonucleotide primer pair including an oligonucleotide primer 14 to 35 nucleobases in length having at least 70% sequence identity with SEQ ID NO: 474 and an oligonucleotide primer 14 to 35 nucleobases in length having at least 70% sequence identity with SEQ ID NO: 869.

One embodiment is an oligonucleotide primer 14 to 35 nucleobases in length having at least 70% sequence identity with SEQ ID NO: 268.

Another embodiment is an oligonucleotide primer 14 to 35 nucleobases in length having at least 70% sequence identity with SEQ ID NO: 1284.

Another embodiment is a kit comprising an oligonucleotide primer pair including an oligonucleotide primer 14 to 35 nucleobases in length having at least 70% sequence identity with SEQ ID NO: 268 and an oligonucleotide primer 14 to 35 nucleobases in length having at least 70% sequence identity with SEQ ID NO: 1284.

One embodiment is an oligonucleotide primer 14 to 35 nucleobases in length having at least 70% sequence identity with SEQ ID NO: 418.

Another embodiment is an oligonucleotide primer 14 to 35 nucleobases in length having at least 70% sequence identity with SEQ ID NO: 1301.

Another embodiment is a kit comprising an oligonucleotide primer pair including an oligonucleotide primer 14 to 35 nucleobases in length having at least 70% sequence identity with SEQ ID NO: 418 and an oligonucleotide primer 14 to 35 nucleobases in length having at least 70% sequence identity with SEQ ID NO: 1301.

One embodiment is an oligonucleotide primer 14 to 35 nucleobases in length having at least 70% sequence identity with SEQ ID NO: 318.

Another embodiment is an oligonucleotide primer 14 to 35 nucleobases in length having at least 70% sequence identity with SEQ ID NO: 1300.

Another embodiment is a kit comprising an oligonucleotide primer pair including an oligonucleotide primer 14 to 35 nucleobases in length having at least 70% sequence identity with SEQ ID NO: 318 and an oligonucleotide primer 14 to 35 nucleobases in length having at least 70% sequence identity with SEQ ID NO: 1300.

One embodiment is an oligonucleotide primer 14 to 35 nucleobases in length having at least 70% sequence identity with SEQ ID NO: 440.

Another embodiment is an oligonucleotide primer 14 to 35 nucleobases in length having at least 70% sequence identity with SEQ ID NO: 1076.

Another embodiment is a kit comprising an oligonucleotide primer pair including an oligonucleotide primer 14 to 35 nucleobases in length having at least 70% sequence identity with SEQ ID NO: 440 and an oligonucleotide primer 14 to 35 nucleobases in length having at least 70% sequence identity with SEQ ID NO: 1076.

One embodiment is an oligonucleotide primer 14 to 35 nucleobases in length having at least 70% sequence identity with SEQ ID NO: 219.

Another embodiment is an oligonucleotide primer 14 to 35 nucleobases in length having at least 70% sequence identity with SEQ ID NO: 1013.

Another embodiment is a kit comprising an oligonucleotide primer pair including an oligonucleotide primer 14 to 35 nucleobases in length having at least 70% sequence identity with SEQ ID NO: 219 and an oligonucleotide primer 14 to 35 nucleobases in length having at least 70% sequence identity with SEQ ID NO: 1013.

Another embodiment is a kit comprising an oligonucleotide primer pair including an oligonucleotide primer 14 to 35 nucleobases in length having at least 70% sequence identity with SEQ ID NO: 437 and an oligonucleotide primer 14 to 35 nucleobases in length having at least 70% sequence identity with SEQ ID NO: 1137 and further comprising one or more primer pairs wherein each member of said one or more primer pairs is of a length of 14 to 35 nucleobases and has 70% to 100% sequence identity with the corresponding member from the group of primer pairs represented by SEQ ID NOs: 530:891, 474:869, 268:1284, 418:1301, 318:1300, 440:1076 and 219:1013.

One embodiment is an oligonucleotide primer 14 to 35 nucleobases in length having at least 70% sequence identity with SEQ ID NO: 325.

Another embodiment is an oligonucleotide primer 14 to 35 nucleobases in length having at least 70% sequence identity with SEQ ID NO: 1163.

Another embodiment is a kit comprising an oligonucleotide primer pair including an oligonucleotide primer 14 to 35 nucleobases in length having at least 70% sequence identity with SEQ ID NO: 325 and an oligonucleotide primer 14 to 35 nucleobases in length having at least 70% sequence identity with SEQ ID NO: 1163.

One embodiment is an oligonucleotide primer 14 to 35 nucleobases in length having at least 70% sequence identity with SEQ ID NO: 278.

Another embodiment is an oligonucleotide primer 14 to 35 nucleobases in length having at least 70% sequence identity with SEQ ID NO: 1039.

Another embodiment is a kit comprising an oligonucleotide primer pair including an oligonucleotide primer 14 to 35 nucleobases in length having at least 70% sequence identity with SEQ ID NO: 278 and an oligonucleotide primer 14 to 35 nucleobases in length having at least 70% sequence identity with SEQ ID NO: 1039.

One embodiment is an oligonucleotide primer 14 to 35 nucleobases in length having at least 70% sequence identity with SEQ ID NO: 465.

Another embodiment is an oligonucleotide primer 14 to 35 nucleobases in length having at least 70% sequence identity with SEQ ID NO: 1037.

Another embodiment is a kit comprising an oligonucleotide primer pair including an oligonucleotide primer 14 to 35 nucleobases in length having at least 70% sequence identity with SEQ ID NO: 465 and an oligonucleotide primer 14 to 35 nucleobases in length having at least 70% sequence identity with SEQ ID NO: 1037.

One embodiment is an oligonucleotide primer 14 to 35 nucleobases in length having at least 70% sequence identity with SEQ ID NO: 148.

Another embodiment is an oligonucleotide primer 14 to 35 nucleobases in length having at least 70% sequence identity with SEQ ID NO: 1172.

Another embodiment is a kit comprising an oligonucleotide primer pair including an oligonucleotide primer 14 to 35 nucleobases in length having at least 70% sequence identity with SEQ ID NO: 148 and an oligonucleotide primer 14 to 35 nucleobases in length having at least 70% sequence identity with SEQ ID NO: 1172.

One embodiment is an oligonucleotide primer 14 to 35 nucleobases in length having at least 70% sequence identity with SEQ ID NO: 190.

Another embodiment is an oligonucleotide primer 14 to 35 nucleobases in length having at least 70% sequence identity with SEQ ID NO: 1254.

Another embodiment is a kit comprising an oligonucleotide primer pair including an oligonucleotide primer 14 to 35 nucleobases in length having at least 70% sequence identity with SEQ ID NO: 190 and an oligonucleotide primer 14 to 35 nucleobases in length having at least 70% sequence identity with SEQ ID NO: 1254.

One embodiment is an oligonucleotide primer 14 to 35 nucleobases in length having at least 70% sequence identity with SEQ ID NO: 266.

Another embodiment is an oligonucleotide primer 14 to 35 nucleobases in length having at least 70% sequence identity with SEQ ID NO: 1094.

Another embodiment is a kit comprising an oligonucleotide primer pair including an oligonucleotide primer 14 to 35 nucleobases in length having at least 70% sequence identity with SEQ ID NO: 266 and an oligonucleotide primer 14 to 35 nucleobases in length having at least 70% sequence identity with SEQ ID NO: 1094.

One embodiment is an oligonucleotide primer 14 to 35 nucleobases in length having at least 70% sequence identity with SEQ ID NO: 508.

Another embodiment is an oligonucleotide primer 14 to 35 nucleobases in length having at least 70% sequence identity with SEQ ID NO: 1297.

Another embodiment is a kit comprising an oligonucleotide primer pair including an oligonucleotide primer 14 to 35 nucleobases in length having at least 70% sequence identity with SEQ ID NO: 508 and an oligonucleotide primer 14 to 35 nucleobases in length having at least 70% sequence identity with SEQ ID NO: 1297.

One embodiment is an oligonucleotide primer 14 to 35 nucleobases in length having at least 70% sequence identity with SEQ ID NO: 259.

Another embodiment is an oligonucleotide primer 14 to 35 nucleobases in length having at least 70% sequence identity with SEQ ID NO: 1060.

Another embodiment is a kit comprising an oligonucleotide primer pair including an oligonucleotide primer 14 to 35 nucleobases in length having at least 70% sequence identity with SEQ ID NO: 259 and an oligonucleotide primer 14 to 35 nucleobases in length having at least 70% sequence identity with SEQ ID NO: 1060.

Another embodiment is a kit comprising an oligonucleotide primer pair including an oligonucleotide primer 14 to 35 nucleobases in length having at least 70% sequence identity with SEQ ID NO: 325 and an oligonucleotide primer 14 to 35 nucleobases in length having at least 70% sequence identity with SEQ ID NO: 1163 and further comprising one or more primer pairs wherein each member of said one or more primer pairs is of a length of 14 to 35 nucleobases and has 70% to 100% sequence identity with the corresponding member from the group of primer pairs represented by SEQ ID NOs: 278:1039: 465:1037, 148:1172, 190:1254, 266:1094, 508:1297 and 259:1060.

One embodiment is an oligonucleotide primer 14 to 35 nucleobases in length having at least 70% sequence identity with SEQ ID NO: 376.

Another embodiment is an oligonucleotide primer 14 to 35 nucleobases in length having at least 70% sequence identity with SEQ ID NO: 1265.

Another embodiment is a kit comprising an oligonucleotide primer pair including an oligonucleotide primer 14 to 35 nucleobases in length having at least 70% sequence identity with SEQ ID NO: 376 and an oligonucleotide primer 14 to 35 nucleobases in length having at least 70% sequence identity with SEQ ID NO: 1265.

One embodiment is an oligonucleotide primer 14 to 35 nucleobases in length having at least 70% sequence identity with SEQ ID NO: 267.

Another embodiment is an oligonucleotide primer 14 to 35 nucleobases in length having at least 70% sequence identity with SEQ ID NO: 1341.

Another embodiment is a kit comprising an oligonucleotide primer pair including an oligonucleotide primer 14 to 35 nucleobases in length having at least 70% sequence identity with SEQ ID NO: 267 and an oligonucleotide primer 14 to 35 nucleobases in length having at least 70% sequence identity with SEQ ID NO: 1341.

One embodiment is an oligonucleotide primer 14 to 35 nucleobases in length having at least 70% sequence identity with SEQ ID NO: 705.

Another embodiment is an oligonucleotide primer 14 to 35 nucleobases in length having at least 70% sequence identity with SEQ ID NO: 1056.

Another embodiment is a kit comprising an oligonucleotide primer pair including an oligonucleotide primer 14 to 35 nucleobases in length having at least 70% sequence identity with SEQ ID NO: 705 and an oligonucleotide primer 14 to 35 nucleobases in length having at least 70% sequence identity with SEQ ID NO: 1056.

One embodiment is an oligonucleotide primer 14 to 35 nucleobases in length having at least 70% sequence identity with SEQ ID NO: 710.

Another embodiment is an oligonucleotide primer 14 to 35 nucleobases in length having at least 70% sequence identity with SEQ ID NO: 1259.

Another embodiment is a kit comprising an oligonucleotide primer pair including an oligonucleotide primer 14 to 35 nucleobases in length having at least 70% sequence identity with SEQ ID NO: 710 and an oligonucleotide primer 14 to 35 nucleobases in length having at least 70% sequence identity with SEQ ID NO: 1259.

One embodiment is an oligonucleotide primer 14 to 35 nucleobases in length having at least 70% sequence identity with SEQ ID NO: 374.

Another embodiment is an oligonucleotide primer 14 to 35 nucleobases in length having at least 70% sequence identity with SEQ ID NO: 1111.

Another embodiment is a kit comprising an oligonucleotide primer pair including an oligonucleotide primer 14 to 35 nucleobases in length having at least 70% sequence identity with SEQ ID NO: 374 and an oligonucleotide primer 14 to 35 nucleobases in length having at least 70% sequence identity with SEQ ID NO: 1111.

One embodiment is an oligonucleotide primer 14 to 35 nucleobases in length having at least 70% sequence identity with SEQ ID NO: 545.

Another embodiment is an oligonucleotide primer 14 to 35 nucleobases in length having at least 70% sequence identity with SEQ ID NO: 978.

Another embodiment is a kit comprising an oligonucleotide primer pair including an oligonucleotide primer 14 to 35 nucleobases in length having at least 70% sequence identity with SEQ ID NO: 545 and an oligonucleotide primer 14 to 35 nucleobases in length having at least 70% sequence identity with SEQ ID NO: 978.

One embodiment is an oligonucleotide primer 14 to 35 nucleobases in length having at least 70% sequence identity with SEQ ID NO: 249.

Another embodiment is an oligonucleotide primer 14 to 35 nucleobases in length having at least 70% sequence identity with SEQ ID NO: 1095.

Another embodiment is a kit comprising an oligonucleotide primer pair including an oligonucleotide primer 14 to 35 nucleobases in length having at least 70% sequence identity with SEQ ID NO: 249 and an oligonucleotide primer 14 to 35 nucleobases in length having at least 70% sequence identity with SEQ ID NO: 1095.

One embodiment is an oligonucleotide primer 14 to 35 nucleobases in length having at least 70% sequence identity with SEQ ID NO: 195.

Another embodiment is an oligonucleotide primer 14 to 35 nucleobases in length having at least 70% sequence identity with SEQ ID NO: 1376.

Another embodiment is a kit comprising an oligonucleotide primer pair including an oligonucleotide primer 14 to 35 nucleobases in length having at least 70% sequence identity with SEQ ID NO: 195 and an oligonucleotide primer 14 to 35 nucleobases in length having at least 70% sequence identity with SEQ ID NO: 1376.

One embodiment is an oligonucleotide primer 14 to 35 nucleobases in length having at least 70% sequence identity with SEQ ID NO: 311.

Another embodiment is an oligonucleotide primer 14 to 35 nucleobases in length having at least 70% sequence identity with SEQ ID NO: 1014.

Another embodiment is a kit comprising an oligonucleotide primer pair including an oligonucleotide primer 14 to 35 nucleobases in length having at least 70% sequence identity with SEQ ID NO: 311 and an oligonucleotide primer 14 to 35 nucleobases in length having at least 70% sequence identity with SEQ ID NO: 1014.

One embodiment is an oligonucleotide primer 14 to 35 nucleobases in length having at least 70% sequence identity with SEQ ID NO: 365.

Another embodiment is an oligonucleotide primer 14 to 35 nucleobases in length having at least 70% sequence identity with SEQ ID NO: 1052.

Another embodiment is a kit comprising an oligonucleotide primer pair including an oligonucleotide primer 14 to 35 nucleobases in length having at least 70% sequence identity with SEQ ID NO: 365 and an oligonucleotide primer 14 to 35 nucleobases in length having at least 70% sequence identity with SEQ ID NO: 1052.

One embodiment is an oligonucleotide primer 14 to 35 nucleobases in length having at least 70% sequence identity with SEQ ID NO: 527.

Another embodiment is an oligonucleotide primer 14 to 35 nucleobases in length having at least 70% sequence identity with SEQ ID NO: 1071.

Another embodiment is a kit comprising an oligonucleotide primer pair including an oligonucleotide primer 14 to 35 nucleobases in length having at least 70% sequence identity with SEQ ID NO: 527 and an oligonucleotide primer 14 to 35 nucleobases in length having at least 70% sequence identity with SEQ ID NO: 1071.

One embodiment is an oligonucleotide primer 14 to 35 nucleobases in length having at least 70% sequence identity with SEQ ID NO: 490.

Another embodiment is an oligonucleotide primer 14 to 35 nucleobases in length having at least 70% sequence identity with SEQ ID NO: 1182.

Another embodiment is a kit comprising an oligonucleotide primer pair including an oligonucleotide primer 14 to 35 nucleobases in length having at least 70% sequence identity with SEQ ID NO: 490 and an oligonucleotide primer 14 to 35 nucleobases in length having at least 70% sequence identity with SEQ ID NO: 1182.

Another embodiment is a kit comprising an oligonucleotide primer pair including an oligonucleotide primer 14 to 35 nucleobases in length having at least 70% sequence identity with SEQ ID NO: 376 and an oligonucleotide primer 14 to 35 nucleobases in length having at least 70% sequence identity with SEQ ID NO: 1265 and further comprising one or more primer pairs wherein each member of said one or more primer pairs is of a length of 14 to 35 nucleobases and has 70% to 100% sequence identity with the corresponding member from the group of primer pairs represented by SEQ ID NOs: 267:1341, 705:1056, 710:1259, 374:1111, 545:978, 249:1095, 195:1376, 311:1014, 365:1052, 527:1071 and 490:1182.

In some embodiments, either or both of the primers of a primer pair composition contain at least one modified nucleobase such as 5-propynyluracil or 5-propynylcytosine for example.

In some embodiments, either or both of the primers of the primer pair comprises at least one universal nucleobase such as inosine for example.

In some embodiments, either or both of the primers of the primer pair comprises at least one non-templated T residue on the 5'-end.

In some embodiments, either or both of the primers of the primer pair comprises at least one non-template tag.

In some embodiments, either or both of the primers of the primer pair comprises at least one molecular mass modifying tag.

In some embodiments, the present disclosure includes primers and compositions comprising pairs of primers, and kits containing the same, and methods for use in identification of bacteria. The primers are designed to produce amplification products of DNA encoding genes that have conserved and variable regions across different subgroups and genotypes of bacteria.

Some embodiments are kits that contain one or more of the primer pair compositions. In some embodiments, each member of the one or more primer pairs of the kit is of a length of 14 to 35 nucleobases and has 70% to 100% sequence identity with the corresponding member from any of the primer pairs listed in Table 2.

Some embodiments of the kits contain at least one calibration polynucleotide for use in quantitiation of bacteria in a given sample, and also for use as a positive control for amplification.

Some embodiments of the kits contain at least one anion exchange functional group linked to a magnetic bead.

In some embodiments, the present disclosure includes methods for identification of bacteria. Nucleic acid from the bacterium is amplified using the primers described above to obtain an amplification product. The molecular mass of the amplification product is measured. Optionally, the base composition of the amplification product is determined from the molecular mass. The molecular mass or base composition is compared with a plurality of molecular masses or base compositions of known analogous bacterial identifying amplicons, wherein a match between the molecular mass or base composition and a member of the plurality of molecular masses or base compositions identifies the bacterium. In some embodiments, the molecular mass is measured by mass spectrometry in a modality such as electrospray ionization (ESI) time of flight (TOF) mass spectrometry or ESI Fourier transform ion cyclotron resonance (FTICR) mass spectrometry, for example. Other mass spectrometry techniques can also be used to measure the molecular mass of bacterial bioagent identifying amplicons.

In some embodiments, the disclosure also includes to a method for determining the presence or absence of a bacterium in a sample. Nucleic acid from the sample is amplified using the composition described above to obtain an amplification product. The molecular mass of the amplification product is determined. Optionally, the base composition of the amplification product is determined from the molecular mass. The molecular mass or base composition of the amplification product is compared with the known molecular masses or base compositions of one or more known analogous bacterial bioagent identifying amplicons, wherein a match between the molecular mass or base composition of the amplification product and the molecular mass or base composition of one or more known bacterial bioagent identifying amplicons indicates the presence of the bacterium in the sample. In some embodiments, the molecular mass is measured by mass spectrometry.

In some embodiments, the present disclosure includes methods for determination of the quantity of an unknown bacterium in a sample. The sample is contacted with the composition described above and a known quantity of a calibration polynucleotide comprising a calibration sequence. Nucleic acid from the unknown bacterium in the sample is concurrently amplified with the composition described above and nucleic acid from the calibration polynucleotide in the sample is concurrently amplified with the composition described above to obtain a first amplification product comprising a bacterial bioagent identifying amplicon and a second amplification product comprising a calibration amplicon. The molecular masses and abundances for the bacterial bioagent identifying amplicon and the calibration amplicon are determined. The bacterial bioagent identifying amplicon is distinguished from the calibration amplicon based on molecular mass and comparison of bacterial bioagent identifying amplicon abundance and calibration amplicon abundance indicates the quantity of bacterium in the sample. In some embodiments, the base composition of the bacterial bioagent identifying amplicon is determined.

In some embodiments, the present disclosure includes methods for detecting or quantifying bacteria by combining a nucleic acid amplification process with a mass determination process. In some embodiments, such methods identify or otherwise analyze the bacterium by comparing mass information from an amplification product with a calibration or control product. Such methods can be carried out in a highly multiplexed and/or parallel manner allowing for the analysis of as many as 300 samples per 24 hours on a single mass measurement platform. The accuracy of the mass determination methods in some embodiments of the present invention permits allows for the ability to discriminate between different bacteria such as, for example, various genotypes and drug resistant strains of *Staphylococcus aureus.*

### BRIEF DESCRIPTION OF THE DRAWINGS

The foregoing summary of the invention, as well as the following detailed description of the invention, is better understood when read in conjunction with the accompanying drawings which are included by way of example and not by way of limitation.

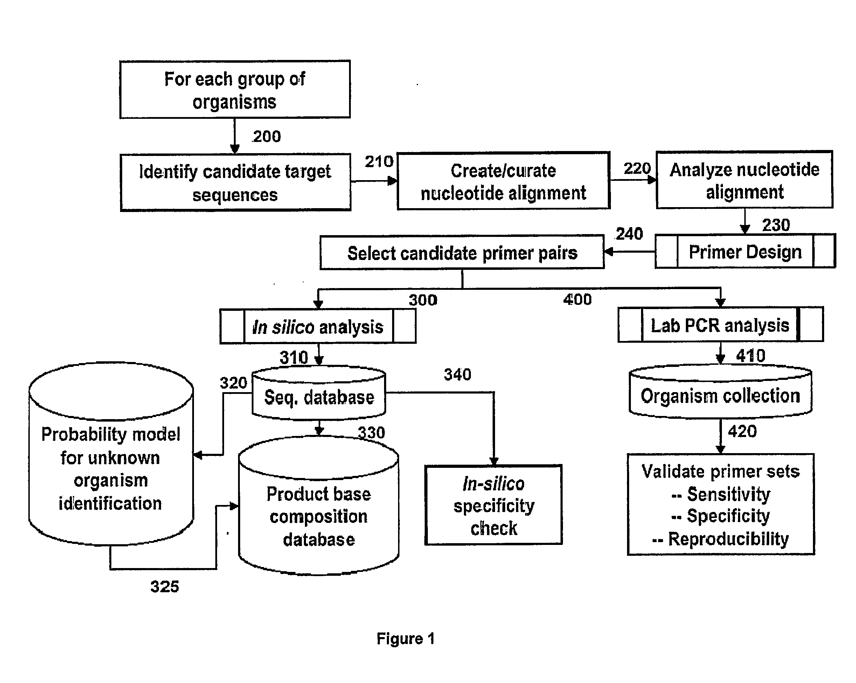

**Figure 1****:** process diagram illustrating a representative primer pair selection process.

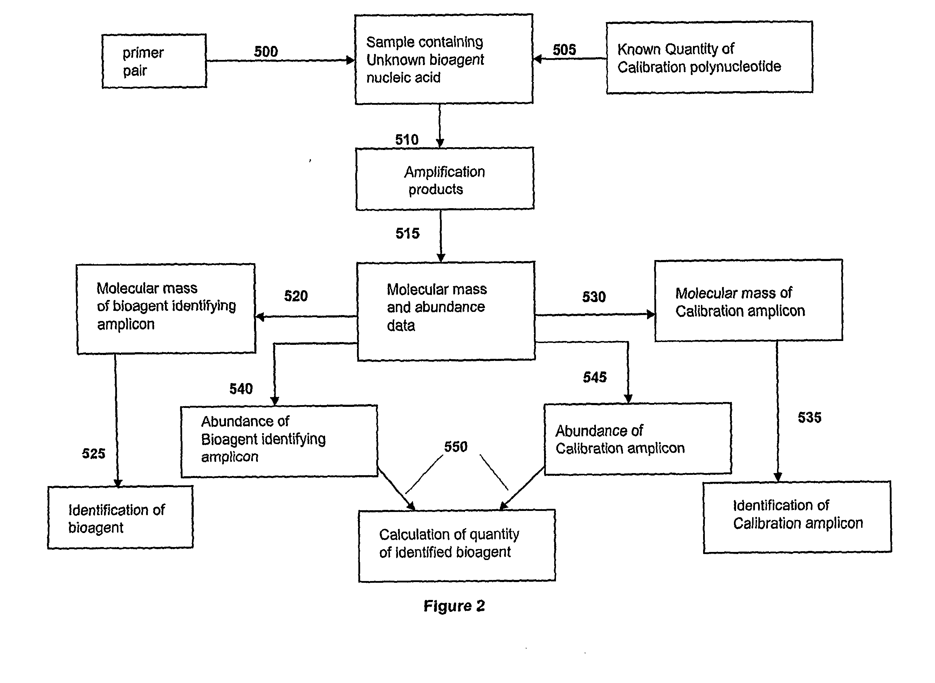

**Figure 2****:** process diagram illustrating an embodiment of the calibration method.

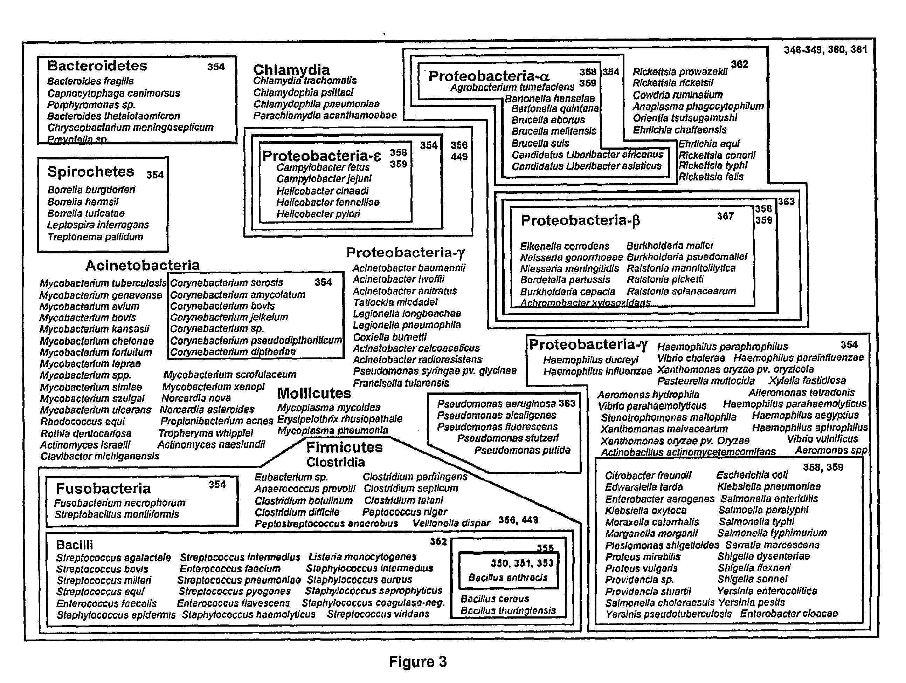

**Figure 3****:** common pathogenic bacteria and primer pair coverage. The primer pair number in the upper right hand corner of each polygon indicates that the primer pair can produce a bioagent identifying amplicon for all species within that polygon.

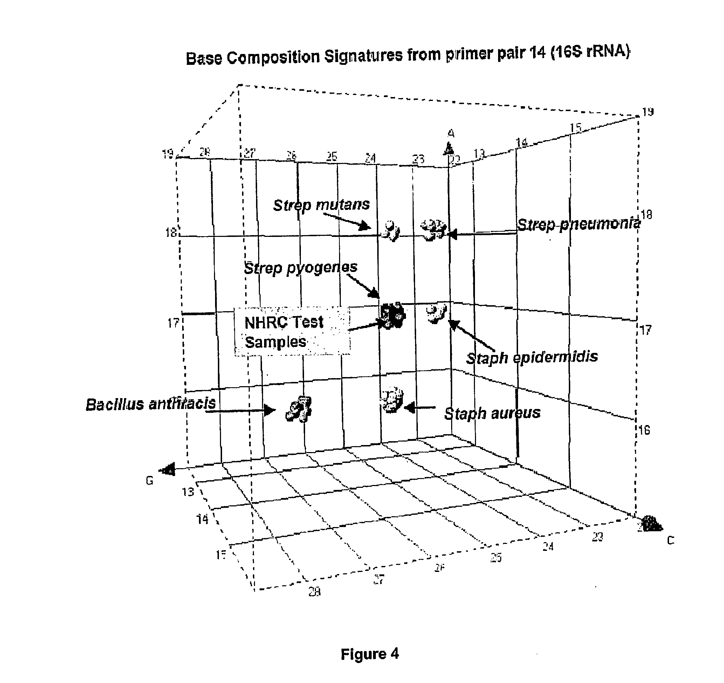

**Figure 4****:** a representative 3D diagram of base composition (axes A, G and C) of bioagent identifying amplicons obtained with primer pair number 14 (a precursor of primer pair number 348 which targets 16S rRNA). The diagram indicates that the experimentally determined base compositions of the clinical samples (labeled NHRC samples) closely match the base compositions expected for *Streptococcus pyogenes* and are distinct from the expected base compositions of other organisms.

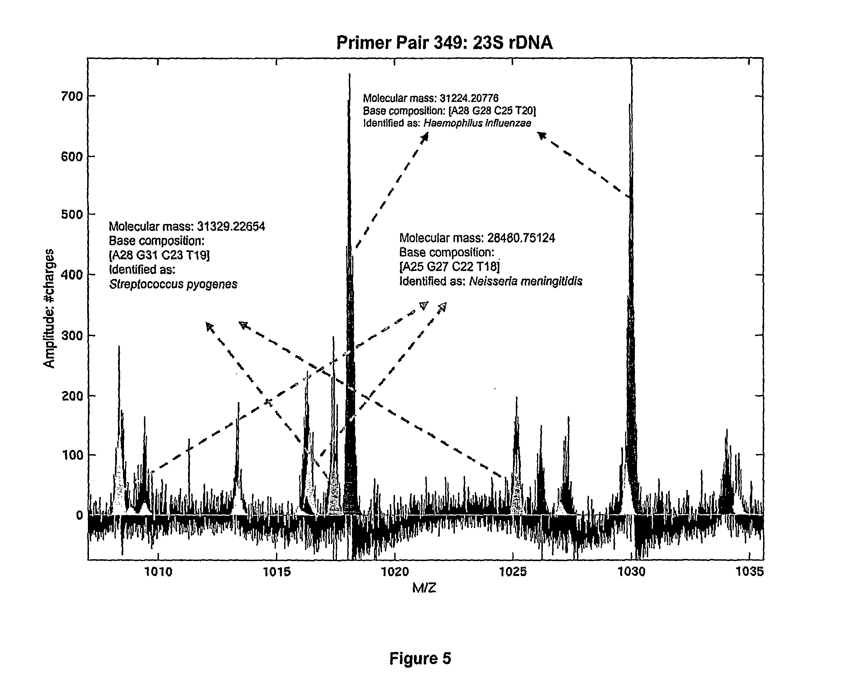

**Figure 5****:** a representative mass spectrum of amplification products indicating the presence of bioagent identifying amplicons of *Streptococcus pyogenes*, *Neisseria* meningitidis, and *Haemophilus influenzae* obtained from amplification of nucleic acid from a clinical sample with primer pair number 349 which targets 23S rRNA. Experimentally determined molecular masses and base compositions for the sense strand of each amplification product are shown.

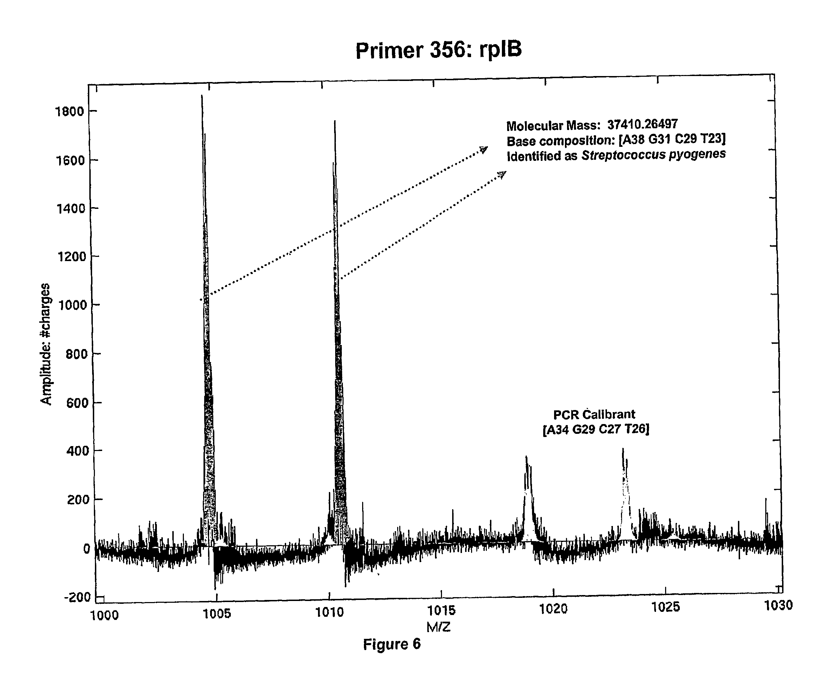

**Figure 6****:** a representative mass spectrum of amplification products representing a bioagent identifying amplicon of *Streptococcus pyogenes*, and a calibration amplicon obtained from amplification of nucleic acid from a clinical sample with primer pair number 356 which targets rplB. The experimentally determined molecular mass and base composition for the sense strand of the *Streptococcus pyogenes* amplification product is shown.

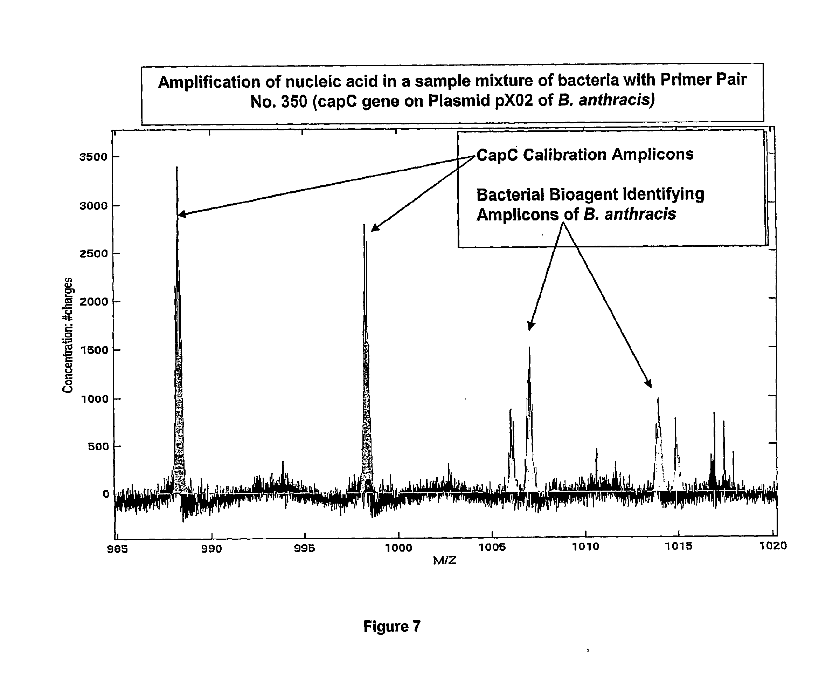

**Figure 7****:** a representative mass spectrum of an amplified nucleic acid mixture which contained the Ames strain of *Bacillus anthracis*, a known quantity of combination calibration polynucleotide (SEQ ID NO: 1464), and primer pair number 350 which targets the capC gene on the virulence plasmid pX02 of *Bacillus anthracis*. Calibration amplicons produced in the amplification reaction are visible in the mass spectrum as indicated and abundance data (peak height) are used to calculate the quantity of the Ames strain of *Bacillus anthracis.*

### DEFINITIONS

As used herein, the term "abundance" refers to an amount. The amount may be described in terms of concentration which are common in molecular biology such as "copy number," "pfu or plateforming unit" which are well known to those with ordinary skill. Concentration may be relative to a known standard or may be absolute.

As used herein, the term "amplifiable nucleic acid" is used in reference to nucleic acids that may be amplified by any amplification method. It is contemplated that "amplifiable nucleic acid" also comprises "sample template."

As used herein the term "amplification" refers to a special case of nucleic acid replication involving template specificity. It is to be contrasted with non-specific template replication (i.e., replication that is template-dependent but not dependent on a specific template). Template specificity is here distinguished from fidelity of replication (i.e., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out. Template specificity is achieved in most amplification techniques by the choice of enzyme. Amplification enzymes are enzymes that, under conditions they are used, will process only specific sequences of nucleic acid in a heterogeneous mixture of nucleic acid. For example, in the case of Qß replicase, MDV-1 RNA is the specific template for the replicase (D.L. Kacian et al., Proc. Natl. Acad. Sci. USA 69:3038 [1972]). Other nucleic acid will not be replicated by this amplification enzyme. Similarly, in the case of T7 RNA polymerase, this amplification enzyme has a stringent specificity for its own promoters (Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (D.Y. Wu and R. B. Wallace, Genomics 4:560 [1989]). Finally, Taq and Pfu polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences (H.A. Erlich (ed.), PCR Technology, Stockton Press [1989]).

As used herein, the term "amplification reagents" refers to those reagents (deoxyribonucleotide triphosphates, buffer, etc.), needed for amplification, excluding primers, nucleic acid template, and the amplification enzyme. Typically, amplification reagents along with other reaction components are placed and contained in a reaction vessel (test tube, microwell, etc.).

As used herein, the term "analogous" when used in context of comparison of bioagent identifying amplicons indicates that the bioagent identifying amplicons being compared are produced with the same pair of primers. For example, bioagent identifying amplicon "A" and bioagent identifying amplicon "B", produced with the same pair of primers are analogous with respect to each other. Bioagent identifying amplicon "C", produced with a different pair of primers is not analogous to either bioagent identifying amplicon "A" or bioagent identifying amplicon "B".

As used herein, the term "anion exchange functional group" refers to a positively charged functional group capable of binding an anion through an electrostatic interaction. The most well known anion exchange functional groups are the amines, including primary, secondary, tertiary and quaternary amines.

The term "bacteria" or "bacterium" refers to any member of the groups of eubacteria and archaebacteria.

As used herein, a "base composition" is the exact number of each nucleobase (for example, A, T, C and G) in a segment of nucleic acid. For example, amplification of nucleic acid of *Staphylococcus aureus* strain carrying the lukS-PV gene with primer pair number 2095 (SEQ ID NOs: 456:1261) produces an amplification product 117 nucleobases in length from nucleic acid of the lukS-PV gene that has a base composition of A35 G17 C19 T46 (by convention - with reference to the sense strand of the amplification product). Because the molecular masses of each of the four natural nucleotides and chemical modifications thereof are known (if applicable), a measured molecular mass can be deconvoluted to a list of possible base compositions. Identification of a base composition of a sense strand which is complementary to the corresponding antisense strand in terms of base composition provides a confirmation of the true base composition of an unknown amplification product. For example, the base composition of the antisense strand of the 139 nucleobase amplification product described above is A46 G19 C17 T35.

As used herein, a "base composition probability cloud" is a representation of the diversity in base composition resulting from a variation in sequence that occurs among different isolates of a given species. The "base composition probability cloud" represents the base composition constraints for each species and is typically visualized using a pseudo four-dimensional plot.

In the context of this invention, a "bioagent" is any organism, cell, or virus, living or dead, or a nucleic acid derived from such an organism, cell or virus. Examples of bioagents include, but are not limited, to cells, (including but not limited to human clinical samples, bacterial cells and other pathogens), viruses, fungi, protists, parasites, and pathogenicity markers (including but not limited to: pathogenicity islands, antibiotic resistance genes, virulence factors, toxin genes and other bioregulating compounds). Samples may be alive or dead or in a vegetative state (for example, vegetative bacteria or spores) and may be encapsulated or bioengineered. In the context of this invention, a "pathogen" is a bioagent which causes a disease or disorder.

As used herein, a "bioagent division" is defined as group of bioagents above the species level and includes but is not limited to, orders, families, classes, clades, genera or other such groupings of bioagents above the species level.

As used herein, the term "bioagent identifying amplicon" refers to a polynucleotide that is amplified from a bioagent in an amplification reaction and which 1) provides sufficient variability to distinguish among bioagents from whose nucleic acid the bioagent identifying amplicon is produced and 2) whose molecular mass is amenable to a rapid and convenient molecular mass determination modality such as mass spectrometry, for example.

As used herein, the term "biological product" refers to any product originating from an organism. Biological products are often products of processes of biotechnology. Examples of biological products include, but are not limited to: cultured cell lines, cellular components, antibodies, proteins and other cell-derived biomolecules, growth media, growth harvest fluids, natural products and biopharmaceutical products.

The terms "biowarfare agent" and "bioweapon" are synonymous and refer to a bacterium, virus, fungus or protozoan that could be deployed as a weapon to cause bodily harm to individuals. Military or terrorist groups may be implicated in deployment of biowarfare agents.

In context of this invention, the term "broad range survey primer pair" refers to a primer pair designed to produce bioagent identifying amplicons across different broad groupings of bioagents. For example, the ribosomal RNA-targeted primer pairs are broad range survey primer pairs which have the capability of producing bacterial bioagent identifying amplicons for essentially all known bacteria. With respect to broad range primer pairs employed for identification of bacteria, a broad range survey primer pair for bacteria such as 16S rRNA primer pair number 346 (SEQ ID NOs: 202:1110) for example, will produce an bacterial bioagent identifying amplicon for essentially all known bacteria.

The term "calibration amplicon" refers to a nucleic acid segment representing an amplification product obtained by amplification of a calibration sequence with a pair of primers designed to produce a bioagent identifying amplicon.

The term "calibration sequence" refers to a polynucleotide sequence to which a given pair of primers hybridizes for the purpose of producing an internal (i.e: included in the reaction) calibration standard amplification product for use in determining the quantity of a bioagent in a sample. The calibration sequence may be expressly added to an amplification reaction, or may already be present in the sample prior to analysis.

The term "clade primer pair" refers to a primer pair designed to produce bioagent identifying amplicons for species belonging to a clade group. A clade primer pair may also be considered as a "speciating" primer pair which is useful for distinguishing among closely related species.

The term "codon" refers to a set of three adjoined nucleotides (triplet) that codes for an amino acid or a termination signal.

In context of this invention, the term "codon base composition analysis," refers to determination of the base composition of an individual codon by obtaining a bioagent identifying amplicon that includes the codon. The bioagent identifying amplicon will at least include regions of the target nucleic acid sequence to which the primers hybridize for generation of the bioagent identifying amplicon as well as the codon being analyzed, located between the two primer hybridization regions.

As used herein, the terms "complementary" or "complementarity" are used in reference to polynucleotides (i.e., a sequence of nucleotides such as an oligonucleotide or a target nucleic acid) related by the base-pairing rules. For example, for the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids. Either term may also be used in reference to individual nucleotides, especially within the context of polynucleotides. For example, a particular nucleotide within an oligonucleotide may be noted for its complementarity, or lack thereof, to a nucleotide within another nucleic acid strand, in contrast or comparison to the complementarity between the rest of the oligonucleotide and the nucleic acid strand.

The term "complement of a nucleic acid sequence" as used herein refers to an oligonucleotide which, when aligned with the nucleic acid sequence such that the 5' end of one sequence is paired with the 3' end of the other, is in "antiparallel association." Certain bases not commonly found in natural nucleic acids may be included in the nucleic acids of the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs. Where a first oligonucleotide is complementary to a region of a target nucleic acid and a second oligonucleotide has complementary to the same region (or a portion of this region) a "region of overlap" exists along the target nucleic acid. The degree of overlap will vary depending upon the extent of the complementarity.

In context of this invention, the term "division-wide primer pair" refers to a primer pair designed to produce bioagent identifying amplicons within sections of a broader spectrum of bioagents For example, primer pair number 352 (SEQ ID NOs: 687:1411), a division-wide primer pair, is designed to produce bacterial bioagent identifying amplicons for members of the Bacillus group of bacteria which comprises, for example, members of the genera *Streptococci, Enterococci,* and *Staphylococci.* Other division-wide primer pairs may be used to produce bacterial bioagent identifying amplicons for other groups of bacterial bioagents.

As used herein, the term "concurrently amplifying" used with respect to more than one amplification reaction refers to the act of simultaneously amplifying more than one nucleic acid in a single reaction mixture.

As used herein, the term "drill-down primer pair" refers to a primer pair designed to produce bioagent identifying amplicons for identification of sub-species characteristics or confirmation of a species assignment. For example, primer pair number 2146 (SEQ ID NOs: 437:1137), a drill-down *Staphylococcus aureus* genotyping primer pair, is designed to produce *Staphylococcus aureus* genotyping amplicons. Other drill-down primer pairs may be used to produce bioagent identifying amplicons for *Staphylococcus aureus* and other bacterial species.

The term "duplex" refers to the state of nucleic acids in which the base portions of the nucleotides on one strand are bound through hydrogen bonding the their complementary bases arrayed on a second strand. The condition of being in a duplex form reflects on the state of the bases of a nucleic acid. By virtue of base pairing, the strands of nucleic acid also generally assume the tertiary structure of a double helix, having a major and a minor groove. The assumption of the helical form is implicit in the act of becoming duplexed.

As used herein, the term "etiology" refers to the causes or origins, of diseases or abnormal physiological conditions.

The term "gene" refers to a DNA sequence that comprises control and coding sequences necessary for the production of an RNA having a non-coding function (e.g., a ribosomal or transfer RNA), a polypeptide or a precursor. The RNA or polypeptide can be encoded by a full length coding sequence or by any portion of the coding sequence so long as the desired activity or function is retained.

The terms "homology," "homologous" and "sequence identity" refer to a degree of identity. There may be partial homology or complete homology. A partially homologous sequence is one that is less than 100% identical to another sequence. Determination of sequence identity is described in the following example: a primer 20 nucleobases in length which is otherwise identical to another 20 nucleobase primer but having two non-identical residues has 18 of 20 identical residues (18/20 = 0.9 or 90% sequence identity). In another example, a primer 15 nucleobases in length having all residues identical to a 15 nucleobase segment of a primer 20 nucleobases in length would have 15/20 = 0.75 or 75% sequence identity with the 20 nucleobase primer. In context of the present invention, sequence identity is meant to be properly determined when the query sequence and the subject sequence are both described and aligned in the 5' to 3' direction. Sequence alignment algorithms such as BLAST, will return results in two different alignment orientations. In the Plus/Plus orientation, both the query sequence and the subject sequence are aligned in the 5' to 3' direction. On the other hand, in the Plus/Minus orientation, the query sequence is in the 5' to 3' direction while the subject sequence is in the 3' to 5' direction. It should be understood that with respect to the primers of the present invention, sequence identity is properly determined when the alignment is designated as Plus/Plus. Sequence identity may also encompass alternate or modified nucleobases that perform in a functionally similar manner to the regular nucleobases adenine, thymine, guanine and cytosine with respect to hybridization and primer extension in amplification reactions. In a non-limiting example, if the 5-propynyl pyrimidines propyne C and/or propyne T replace one or more C or T residues in one primer which is otherwise identical to another primer in sequence and length, the two primers will have 100% sequence identity with each other. In another non-limiting example, Inosine (I) may be used as a replacement for G or T and effectively hybridize to C, A or U (uracil). Thus, if inosine replaces one or more C, A or U residues in one primer which is otherwise identical to another primer in sequence and length, the two primers will have 100% sequence identity with each other. Other such modified or universal bases may exist which would perform in a functionally similar manner for hybridization and amplification reactions and will be understood to fall within this definition of sequence identity.

As used herein, "housekeeping gene" refers to a gene encoding a protein or RNA involved in basic functions required for survival and reproduction of a bioagent. Housekeeping genes include, but are not limited to genes encoding RNA or proteins involved in translation, replication, recombination and repair, transcription, nucleotide metabolism, amino acid metabolism, lipid metabolism, energy generation, uptake, secretion and the like.

As used herein, the term "hybridization" is used in reference to the pairing of complementary nucleic acids. Hybridization and the strength of hybridization (i.e., the strength of the association between the nucleic acids) is influenced by such factors as the degree of complementary between the nucleic acids, stringency of the conditions involved, and the Tₘ of the formed hybrid. "Hybridization" methods involve the annealing of one nucleic acid to another, complementary nucleic acid, i.e., a nucleic acid having a complementary nucleotide sequence. The ability of two polymers of nucleic acid containing complementary sequences to find each other and anneal through base pairing interaction is a well-recognized phenomenon. The initial observations of the "hybridization" process by Marmur and Lane, Proc. Natl. Acad. Sci. USA 46:453 (1960) and Doty et al., Proc. Natl. Acad. Sci. USA 46:461 (1960) have been followed by the refinement of this process into an essential tool of modem biology.

The term "*in silico*" refers to processes taking place via computer calculations. For example, electronic PCR (ePCR) is a process analogous to ordinary PCR except that it is carried out using nucleic acid sequences and primer pair sequences stored on a computer formatted medium.

As used herein, "intelligent primers" are primers that are designed to bind to highly conserved sequence regions of a bioagent identifying amplicon that flank an intervening variable region and, upon amplification, yield amplification products which ideally provide enough variability to distinguish individual bioagents, and which are amenable to molecular mass analysis. By the term "highly conserved," it is meant that the sequence regions exhibit between about 80-100%, or between about 90-100%, or between about 95-100% identity among all, or at least 70%, at least 80%, at least 90%, at least 95%, or at least 99% of species or strains.

The "ligase chain reaction" (LCR; sometimes referred to as "Ligase Amplification Reaction" (LAR) described by Barany, Proc. Natl. Acad. Sci., 88:189 (1991); Barany, PCR Methods and Applic., 1:5 (1991); and Wu and Wallace, Genomics 4:560 (1989) has developed into a well-recognized alternative method for amplifying nucleic acids. In LCR, four oligonucleotides, two adjacent oligonucleotides which uniquely hybridize to one strand of target DNA, and a complementary set of adjacent oligonucleotides, that hybridize to the opposite strand are mixed and DNA ligase is added to the mixture. Provided that there is complete complementarity at the junction, ligase will covalently link each set of hybridized molecules. Importantly, in LCR, two probes are ligated together only when they basepair with sequences in the target sample, without gaps or mismatches. Repeated cycles of denaturation, hybridization and ligation amplify a short segment of DNA. LCR has also been used in combination with PCR to achieve enhanced detection of single-base changes. However, because the four oligonucleotides used in this assay can pair to form two short ligatable fragments, there is the potential for the generation of target-independent background signal. The use of LCR for mutant screening is limited to the examination of specific nucleic acid positions.

The term "locked nucleic acid" or "LNA" refers to a nucleic acid analogue containing one or more 2'-O, 4-C-methylene-β-D-ribofuranosyl nucleotide monomers in an RNA mimicking sugar conformation. LNA oligonucleotides display unprecedented hybridization affinity toward complementary single-stranded RNA and complementary single- or double-stranded DNA. LNA oligonucleotides induce A-type (RNA-like) duplex conformations. The primers of the present invention may contain LNA modifications.

As used herein, the term "mass-modifying tag" refers to any modification to a given nucleotide which results in an increase in mass relative to the analogous non-mass modified nucleotide. Mass-modifying tags can include heavy isotopes of one or more elements included in the nucleotide such as carbon-13 for example. Other possible modifications include addition of substituents such as iodine or bromine at the 5 position of the nucleobase for example.

The term "mass spectrometry" refers to measurement of the mass of atoms or molecules. The molecules are first converted to ions, which are separated using electric or magnetic fields according to the ratio of their mass to electric charge. The measured masses are used to identity the molecules.

The term "microorganism" as used herein means an organism too small to be observed with the unaided eye and includes, but is not limited to bacteria, virus, protozoans, fungi; and ciliates.

The term "multi-drug resistant" or multiple-drug resistant" refers to a microorganism which is resistant to more than one of the antibiotics or antimicrobial agents used in the treatment of said microorganism.

The term "multiplex PCR" refers to a PCR reaction where more than one primer set is included in the reaction pool allowing 2 or more different DNA targets to be amplified by PCR in a single reaction tube.

The term "non-template tag" refers to a stretch of at least three guanine or cytosine nucleobases of a primer used to produce a bioagent identifying amplicon which are not complementary to the template. A non-template tag is incorporated into a primer for the purpose of increasing the primer-duplex stability of later cycles of amplification by incorporation of extra G-C pairs which each have one additional hydrogen bond relative to an A-T pair.

The term "nucleic acid sequence" as used herein refers to the linear composition of the nucleic acid residues A, T, C or G or any modifications thereof, within an oligonucleotide, nucleotide or polynucleotide, and fragments or portions thereof, and to DNA or RNA of genomic or synthetic origin which may be single or double stranded, and represent the sense or antisense strand

As used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

The term "nucleotide analog" as used herein refers to modified or non-naturally occurring nucleotides such as 5-propynyl pyrimidines (i.e., 5-propynyl-dTTP and 5-propynyl-dTCP), 7-deaza purines (i.e., 7-deaza-dATP and 7-deaza-dGTP). Nucleotide analogs include base analogs and comprise modified forms of deoxyribonucleotides as well as ribonucleotides.

The term "oligonucleotide" as used herein is defined as a molecule comprising two or more deoxyribonucleotides or ribonucleotides, preferably at least 5 nucleotides, more preferably at least about 13 to 35 nucleotides. The exact size will depend on many factors, which in turn depend on the ultimate function or use of the oligonucleotide. The oligonucleotide may be generated in any manner, including chemical synthesis, DNA replication, reverse transcription, PCR, or a combination thereof. Because mononucleotides are reacted to make oligonucleotides in a manner such that the 5' phosphate of one mononucleotide pentose ring is attached to the 3' oxygen of its neighbor in one direction via a phosphodiester linkage, an end of an oligonucleotide is referred to as the "5'-end" if its 5' phosphate is not linked to the 3' oxygen of a mononucleotide pentose ring and as the "3'-end" if its 3'oxygen is not linked to a 5' phosphate of a subsequent mononucleotide pentose ring. As used herein, a nucleic acid sequence, even if internal to a larger oligonucleotide, also may be said to have 5' and 3' ends. A first region along a nucleic acid strand is said to be upstream of another region if the 3' end of the first region is before the 5' end of the second region when moving along a strand of nucleic acid in a 5' to 3' direction. All oligonucleotide primers disclosed herein are understood to be presented in the 5' to 3' direction when reading left to right. When two different, non-overlapping oligonucleotides anneal to different regions of the same linear complementary nucleic acid sequence, and the 3' end of one oligonucleotide points towards the 5' end of the other, the former may be called the "upstream" oligonucleotide and the latter the "downstream" oligonucleotide. Similarly, when two overlapping oligonucleotides are hybridized to the same linear complementary nucleic acid sequence, with the first oligonucleotide positioned such that its 5' end is upstream of the 5' end of the second oligonucleotide, and the 3' end of the first oligonucleotide is upstream of the 3' end of the second oligonucleotide, the first oligonucleotide may be called the "upstream" oligonucleotide and the second oligonucleotide may be called the "downstream" oligonucleotide.

In the context of this invention, a "pathogen" is a bioagent which causes a disease or disorder.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

The term "peptide nucleic acid" ("PNA") as used herein refers to a molecule comprising bases or base analogs such as would be found in natural nucleic acid, but attached to a peptide backbone rather than the sugar-phosphate backbone typical of nucleic acids. The attachment of the bases to the peptide is such as to allow the bases to base pair with complementary bases of nucleic acid in a manner similar to that of an oligonucleotide. These small molecules, also designated anti gene agents, stop transcript elongation by binding to their complementary strand of nucleic acid (Nielsen, et al. Anticancer Drug Des. 8:53 63). The primers of the present invention may comprise PNAs.

The term "polymerase" refers to an enzyme having the ability to synthesize a complementary strand of nucleic acid from a starting template nucleic acid strand and free dNTPs.

As used herein, the term "polymerase chain reaction" ("PCR") refers to the method of K.B. Mullis U.S. Patent Nos. 4,683,195,4,683,202, and 4,965,188, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified." With PCR, it is possible to amplify a single copy of a specific target sequence in genomic DNA to a level detectable by several different methodologies (e.g., hybridization with a labelled probe; incorporation of biotinylated primers followed by avidin-enzyme conjugate detection; incorporation of 32P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

The term "polymerization means" or "polymerization agent" refers to any agent capable of facilitating the addition of nucleoside triphosphates to an oligonucleotide. Preferred polymerization means comprise DNA and RNA polymerases.

As used herein, the terms "pair of primers," or "primer pair" are synonymous. A primer pair is used for amplification of a nucleic acid sequence. A pair of primers comprises a forward primer and a reverse primer. The forward primer hybridizes to a sense strand of a target gene sequence to be amplified and primes synthesis of an antisense strand (complementary to the sense strand) using the target sequence as a template. A reverse primer hybridizes to the antisense strand of a target gene sequence to be amplified and primes synthesis of a sense strand (complementary to the antisense strand) using the target sequence as a template.

The primers are designed to bind to highly conserved sequence regions of a bioagent identifying amplicon that flank an intervening variable region and yield amplification products which ideally provide enough variability to distinguish each individual bioagent, and which are amenable to molecular mass analysis. In some embodiments, the highly conserved sequence regions exhibit between about 80-100%, or between about 90-100%, or between about 95-100% identity, or between about 99-100% identity. The molecular mass of a given amplification product provides a means of identifying the bioagent from which it was obtained, due to the variability of the variable region. Thus design of the primers requires selection of a variable region with appropriate variability to resolve the identity of a given bioagent. Bioagent identifying amplicons are ideally specific to the identity of the bioagent.

Properties of the primers may include any number of properties related to structure including, but not limited to: nucleobase length which may be contiguous (linked together) or non-contiguous (for example, two or more contiguous segments which are joined by a linker or loop moiety), modified or universal nucleobases (used for specific purposes such as for example, increasing hybridization affinity, preventing non-templated adenylation and modifying molecular mass) percent complementarity to a given target sequences.

Properties of the primers also include functional features including, but not limited to, orientation of hybridization (forward or reverse) relative to a nucleic acid template. The coding or sense strand is the strand to which the forward priming primer hybridizes (forward priming orientation) while the reverse priming primer hybridizes to the non-coding or antisense strand (reverse priming orientation). The functional properties of a given primer pair also include the generic template nucleic acid to which the primer pair hybridizes. For example, identification of bioagents can be accomplished at different levels using primers suited to resolution of each individual level of identification. Broad range survey primers are designed with the objective of identifying a bioagent as a member of a particular division (e.g., an order, family, genus or other such grouping ofbioagents above the species level of bioagents). In some embodiments, broad range survey intelligent primers are capable of identification of bioagents at the species or sub-species level. Other primers may have the functionality of producing bioagent identifying amplicons for members of a given taxonomic genus, clade, species, sub-species or genotype (including genetic variants which may include presence of virulence genes or antibiotic resistance genes or mutations). Additional functional properties of primer pairs include the functionality of performing amplification either singly (single primer pair per amplification reaction vessel) or in a multiplex fashion (multiple primer pairs and multiple amplification reactions within a single reaction vessel).

As used herein, the terms "purified" or "substantially purified" refer to molecules, either nucleic or amino acid sequences, that are removed from their natural environment, isolated or separated, and are at least 60% free, preferably 75% free, and most preferably 90% free from other components with which they are naturally associated. An "isolated polynucleotide" or "isolated oligonucleotide" is therefore a substantially purified polynucleotide.

The term "reverse transcriptase" refers to an enzyme having the ability to transcribe DNA from an RNA template. This enzymatic activity is known as reverse transcriptase activity. Reverse transcriptase activity is desirable in order to obtain DNA from RNA viruses which can then be amplified and analyzed by the methods of the present invention.

The term "ribosomal RNA" or "rRNA" refers to the primary ribonucleic acid constituent of ribosomes. Ribosomes are the protein-manufacturing organelles of cells and exist in the cytoplasm. Ribosomal RNAs are transcribed from the DNA genes encoding them.

The term "sample" in the present specification and claims is used in its broadest sense. On the one hand it is meant to include a specimen or culture (e.g., microbiological cultures). On the other hand, it is meant to include both biological and environmental samples. A sample may include a specimen of synthetic origin. Biological samples may be animal, including human, fluid, solid (e.g., stool) or tissue, as well as liquid and solid food and feed products and ingredients such as dairy items, vegetables, meat and meat by-products, and waste. Biological samples may be obtained from all of the various families of domestic animals, as well as feral or wild animals, including, but not limited to, such animals as ungulates, bear, fish, lagamorphs, rodents, etc. Environmental samples include environmental material such as surface matter, soil, water, air and industrial samples, as well as samples obtained from food and dairy processing instruments, apparatus, equipment, utensils, disposable and non-disposable items. These examples are not to be construed as limiting the sample types applicable to the present invention. The term "source of target nucleic acid" refers to any sample that contains nucleic acids (RNA or DNA). Particularly preferred sources of target nucleic acids are biological samples including, but not limited to blood, saliva, cerebral spinal fluid, pleural fluid, milk, lymph, sputum and semen.

As used herein, the term "sample template" refers to nucleic acid originating from a sample that is analyzed for the presence of "target" (defined below). In contrast, "background template" is used in reference to nucleic acid other than sample template that may or may not be present in a sample. Background template is often a contaminant. It may be the result of carryover, or it may be due to the presence of nucleic acid contaminants sought to be purified away from the sample. For example, nucleic acids from organisms other than those to be detected may be present as background in a test sample.

A "segment" is defined herein as a region of nucleic acid within a target sequence.

The "self-sustained sequence replication reaction" (3SR) (Guatelli et al., Proc. Natl. Acad. Sci., 87:1874-1878 [1990], with an erratum at Proc. Natl. Acad. Sci., 87:7797 [1990]) is a transcription-based *in vitro* amplification system (Kwok et al., Proc. Natl. Acad. Sci., 86:1173-1177 [1989]) that can exponentially amplify RNA sequences at a uniform temperature. The amplified RNA can then be utilized for mutation detection (Fahy et al., PCR Meth. Appl., 1:25-33 [1991]). In this method, an oligonucleotide primer is used to add a phage RNA polymerase promoter to the 5' end of the sequence of interest In a cocktail of enzymes and substrates that includes a second primer, reverse transcriptase, RNase H, RNA polymerase and ribo- and deoxyribonucleoside triphosphates, the target sequence undergoes repeated rounds of transcription, cDNA synthesis and second-strand synthesis to amplify the area of interest. The use of 3SR to detect mutations is kinetically limited to screening small segments of DNA (e.g., 200-300 base pairs).

As used herein, the term ""sequence alignment"" refers to a listing of multiple DNA or amino acid sequences and aligns them to highlight their similarities. The listings can be made using bioinformatics computer programs.

In context of this invention, the term "speciating primer pair" refers to a primer pair designed to produce a bioagent identifying amplicon with the diagnostic capability of identifying species members of a group of genera or a particular genus of bioagents. Primer pair number 2249 (SEQ ID NOs: 430:1321), for example, is a speciating primer pair used to distinguish *Staphylococcus atreus* from other species of the genus *Staphylococcus.*

As used herein, a "sub-species characteristic" is a genetic characteristic that provides the means to distinguish two members of the same bioagent species. For example, one viral strain could be distinguished from another viral strain of the same species by possessing a genetic change (e.g., for example, a nucleotide deletion, addition or substitution) in one of the viral genes, such as the RNA-dependent RNA polymerase. Sub-species characteristics such as virulence genes and drug-are responsible for the phenotypic differences among the different strains of bacteria.

As used herein, the term "target" is used in a broad sense to indicate the gene or genomic region being amplified by the primers. Because the present invention provides a plurality of amplification products from any given primer pair (depending on the bioagent being analyzed), multiple amplification products from different specific nucleic acid sequences may be obtained. Thus, the term "target" is not used to refer to a single specific nucleic acid sequence. The "target" is sought to be sorted out from other nucleic acid sequences and contains a sequence that has at least partial complementarity with an oligonucleotide primer. The target nucleic acid may comprise single- or double-stranded DNA or RNA. A "segment" is defined as a region of nucleic acid within the target sequence.

The term "template" refers to a strand of nucleic acid on which a complementary copy is built from nucleoside triphosphates through the activity of a template-dependent nucleic acid polymerase. Within a duplex the template strand is, by convention, depicted and described as the "bottom" strand. Similarly, the non-template strand is often depicted and described as the "top" strand.

As used herein, the term "Tm" is used in reference to the "melting temperature." The melting temperature is the temperature at which a population of double-stranded nucleic acid molecules becomes half dissociated into single strands. Several equations for calculating the Tₘ of nucleic acids are well known in the art. As indicated by standard references, a simple estimate of the Tₘ value may be calculated by the equation: Tₘ=81.5+0.41(% G+C), when a nucleic acid is in aqueous solution at 1 M NaCl (see e.g., Anderson and Young, Quantitative Filter Hybridization, in Nucleic Acid Hybridization (1985). Other references (e.g., Allawi, H. T. & SantaLucia, J., Jr. Thermodynamics and NMR of internal G.T mismatches in DNA. Biochemistry 36, 10581-94 (1997) include more sophisticated computations which take structural and environmental, as well as sequence characteristics into account for the calculation of Tₘ.

The term "triangulation genotyping analysis" refers to a method of genotyping a bioagent by measurement of molecular masses or base compositions of amplification products, corresponding to bioagent identifying amplicons, obtained by amplification of regions of more than one gene. In this sense, the term "triangulation" refers to a method of establishing the accuracy of information by comparing three or more types of independent points of view bearing on the same findings. Triangulation genotyping analysis carried out with a plurality of triangulation genotyping analysis primers yields a plurality of base compositions that then provide a pattern or "barcode" from which a species type can be assigned. The species type may represent a previously known sub-species or strain, or may be a previously unknown strain having a specific and previously unobserved base composition barcode indicating the existence of a previously unknown genotype.

As used herein, the term "triangulation genotyping analysis primer pair" is a primer pair designed to produce bioagent identifying amplicons for determining species types in a triangulation genotyping analysis.

The employment of more than one bioagent identifying amplicon for identification of a bioagent is herein referred to as "triangulation identification." Triangulation identification is pursued by WO 2006/116127 PCT/US2006/015160 analyzing a plurality of bioagent identifying amplicons produced with different primer pairs. This process is used to reduce false negative and false positive signals, and enable reconstruction of the origin of hybrid or otherwise engineered bioagents. For example, identification of the three part toxin genes typical of *B. anthracis* (Bowen et al J. Appl. Microbiol., 1999, 87, 270-278) in the absence of the expected signatures from the *B. anthracis* genome would suggest a genetic engineering event.

In the context of this invention, the term "unknown bioagent" may mean either: (i) a bioagent whose existence is known (such as the well known bacterial species *Staphylococcus aureus* for example) but which is not know to be in a sample to be analyzed, or (ii) a bioagent whose existence is not know (for example, the SARS coronavirus was unknown prior to April 2003). For example, if the method for identification of coronaviruses disclosed in commonly owned U.S. Patent Serial No. 10/829,826 was to be employed prior to April 2003 to identify the SARS coronavirus in a clinical sample, both meanings of "unknown" bioagent are applicable since the SARS coronavirus was unknown to science prior to April, 2003 and since it was not known what bioagent (in this case a coronavirus) was present in the sample. On the other hand, if the method of U.S. Patent Serial No. 10/829,826 was to be employed subsequent to April 2003 to identify the SARS coronavirus in a clinical sample, only the first meaning (i) of "unknown" bioagent would apply since the SARS coronavirus became known to science subsequent to April 2003 and since it was not known what bioagent was present in the sample.

The term "variable sequence" as used herein refers to differences in nucleic acid sequence between two nucleic acids. For example, the genes of two different bacterial species may vary in sequence by the presence of single base substitutions and/or deletions or insertions of one or more nucleotides. These two forms of the structural gene are said to vary in sequence from one another. In the context of the present invention, viral nucleic acid" includes, but is not limited to, DNA, RNA, or DNA that has been obtained from viral RNA, such as, for example, by performing a reverse transcription reaction. Viral RNA can either be single-stranded (of positive or negative polarity) or double-stranded.

The term "virus" refers to obligate, ultramicroscopic, parasites that are incapable of autonomous replication (i.e., replication requires the use of the host cell's machinery). Viruses can survive outside of a host cell but cannot replicate.

The term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. In contrast, the term "modified", "mutant" or "polymorphic" refers to a gene or gene product 40 that displays modifications in sequence and or functional properties (i.e., altered characteristics) when compared to the wild-type gene or gene product It is noted that naturally-occurring mutants can be isolated; these are identified by the fact that they have altered characteristics when compared to the wild-type gene or gene product.

As used herein, a "wobble base" is a variation in a codon found at the third nucleotide position of a DNA triplet. Variations in conserved regions of sequence are often found at the third nucleotide position due to redundancy in the amino acid code.

### DETAILED DESCRIPTION OF EMBODIMENTS

### A. Bioagent Identifying Amplicons

The present invention provides methods for detection and identification of unknown bioagents using bioagent identifying amplicons. Primers are selected to hybridize to conserved sequence regions of nucleic acids derived from a bioagent, and which bracket variable sequence regions to yield a bioagent identifying amplicon, which can be amplified and which is amenable to molecular mass determination. The molecular mass then provides a means to uniquely identify the bioagent without a requirement for prior knowledge of the possible identity of the bioagent. The molecular mass or corresponding base composition signature of the amplification product is then matched against a database of molecular masses or base composition signatures. A match is obtained when an experimentally-determined molecular mass or base composition of an analyzed amplification product is compared with known molecular masses or base compositions of known bioagent identifying amplicons and the experimentally determined molecular mass or base composition is the same as the molecular mass or base composition of one of the known bioagent identifying amplicons. Alternatively, the experimentally-determined molecular mass or base composition may be within experimental error of the molecular mass or base composition of a known bioagent identifying amplicon and still be classified as a match. In some cases, the match may also be classified using a probability of match model such as the models described in U.S. Serial No. 11/073,362. Furthermore, the method can be applied to rapid parallel multiplex analyses, the results of which can be employed in a triangulation identification strategy. The present method provides rapid throughput and does not require nucleic acid sequencing of the amplified target sequence for bioagent detection and identification.

Despite enormous biological diversity, all forms of life on earth share sets of essential, common features in their genomes. Since genetic data provide the underlying basis for identification of bioagents by the methods of the present invention, it is necessary to select segments of nucleic acids which ideally provide enough variability to distinguish each individual bioagent and whose molecular mass is amenable to molecular mass determination.

Unlike bacterial genomes, which exhibit conservation of numerous genes (i.e. housekeeping genes) across all organisms, viruses do not share a gene that is essential and conserved among all virus families. Therefore, viral identification is achieved within smaller groups of related viruses, such as members of a particular virus family or genus. For example, RNA-dependent RNA polymerase is present in all single-stranded RNA viruses and can be used for broad priming as well as resolution within the virus family.

In some embodiments of the present invention, at least one bacterial nucleic acid segment is amplified in the process of identifying the bacterial bioagent. Thus, the nucleic acid segments that can be amplified by the primers disclosed herein and that provide enough variability to distinguish each individual bioagent and whose molecular masses are amenable to molecular mass determination are herein described as bioagent identifying amplicons.

In some embodiments of the present invention, bioagent identifying amplicons comprise from about 45 to about 150 nucleobases (i.e. from about 45 to about 200 linked nucleosides), although both longer and short regions may be used. One of ordinary skill in the art will appreciate that the invention embodies compounds of 45, 46, 47, 48, 49, 50, 51, 52, 53, 54, 55, 56, 57, 5 8, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139,140,141, 142, 143, 144, 145, 146, 147, 148, 149, and 150 nucleobases in length, or any range therewithin.

It is the combination of the portions of the bioagent nucleic acid segment to which the primers hybridize (hybridization sites) and the variable region between the primer hybridization sites that comprises the bioagent identifying amplicon. Thus, it can be said that a given bioagent identifying amplicon is "defined by" a given pair of primers.

In some embodiments, bioagent identifying amplicons amenable to molecular mass determination which are produced by the primers described herein are either of a length, size or mass compatible with the particular mode of molecular mass determination or compatible with a means of providing a predictable fragmentation pattern in order to obtain predictable fragments of a length compatible with the particular mode of molecular mass determination. Such means of providing a predictable fragmentation pattern of an amplification product include, but are not limited to, cleavage with chemical reagents, restriction enzymes or cleavage primers, for example. Thus, in some embodiments, bioagent identifying amplicons are larger than 150 nucleobases and are amenable to molecular mass determination following restriction digestion. Methods of using restriction enzymes and cleavage primers are well known to those with ordinary skill in the art.

In some embodiments, amplification products corresponding to bioagent identifying amplicons are obtained using the polymerase chain reaction (PCR) that is a routine method to those with ordinary skill in the molecular biology arts. Other amplification methods may be used such as ligase chain reaction (LCR), low-stringency single primer PCR, and multiple strand displacement amplification (MDA). These methods are also known to those with ordinary skill.

### B. Primers and Primer Pairs

In some embodiments, the primers are designed to bind to conserved sequence regions of a bioagent identifying amplicon that flank an intervening variable region and yield amplification products which provide variability sufficient to distinguish each individual bioagent, and which are amenable to molecular mass analysis. In some embodiments, the highly conserved sequence regions exhibit between about 80-100%, or between about 90-100%, or between about 95-100% identity, or between about 99-100% identity. The molecular mass of a given amplification product provides a means of identifying the bioagent from which it was obtained, due to the variability of the variable region. Thus, design of the primers involves selection of a variable region with sufficient variability to resolve the identity of a given bioagent. In some embodiments, bioagent identifying amplicons are specific to the identity of the bioagent.

In some embodiments, identification of bioagents is accomplished at different levels using primers suited to resolution of each individual level of identification. Broad range survey primers are designed with the objective of identifying a bioagent as a member of a particular division (e.g., an order, family, genus or other such grouping of bioagents above the species level ofbioagents). In some embodiments, broad range survey intelligent primers are capable of identification of bioagents at the species or sub-species level. Examples of broad range survey primers include, but are not limited to: primer pair numbers: 346 (SEQ ID NOs: 202:1110), 347 (SEQ ID NOs: 560:1278), 348 SEQ ID NOs: 706:895), and 361 (SEQ ID NOs: 697:1398) which target DNA encoding 16S rRNA, and primer pair numbers 349 (SEQ ID NOs: 401:1156) and 360 (SEQ ID NOs: 409:1434) which target DNA encoding 23S rRNA.

In some embodiments, drill-down primers are designed with the objective of identifying a bioagent at the sub-species level (including strains, subtypes, variants and isolates) based on sub-species characteristics which may, for example, include single nucleotide polymorphisms (SNPs), variable number tandem repeats (VNTRs), deletions, drug resistance mutations or any other modification of a nucleic acid sequence of a bioagent relative to other members of a species having different sub-species characteristics. Drill-down intelligent primers are not always required for identification at the sub-species level because broad range survey intelligent primers may, in some cases provide sufficient identification resolution to accomplishing this identification objective. Examples of drill-down primers include, but are not limited to: confirmation primer pairs such as primer pair numbers 351 (SEQ ID NOs: 355:1423) and 353 (SEQ ID NOs: 220:1394), which target the pX01 virulence plasmid of *Bacillus anthracis.* Other examples of drill-down primer pairs are found in sets of triangulation genotyping primer pairs such as, for example, the primer pair number 2146 (SEQ ID NOs: 437:1137) which targets the arcC gene (encoding carmabate kinase) and is included in an 8 primer pair panel or kit for use in genotyping *Staphylococcus aureus,* or in other panels or kits of primer pairs used for determining drug-resistant bacterial strains, such as, for example, primer pair number 2095 (SEQ ID NOs: 456:1261) which targets the pv-luk gene (encoding Panton-Valentine leukocidin) and is included in an 8 primer pair panel or kit for use in identification of drug resistant strains of *Staphylococcus aureus.*

A representative process flow diagram used for primer selection and validation process is outlined in Figure 1. For each group of organisms, candidate target sequences are identified (**200**) from which nucleotide alignments are created (**210**) and analyzed (**220**). Primers are then designed by selecting appropriate priming regions (**230**) to facilitate the selection of candidate primer pairs (**240**). The primer pairs are then subjected to *in silico* analysis by electronic PCR (ePCR) (**300**) wherein bioagent identifying amplicons are obtained from sequence databases such as GenBank or other sequence collections (**310**) and checked for specificity *in silica* (**320**). Bioagent identifying amplicons obtained from GenBank sequences (**310**) can also be analyzed by a probability model which predicts the capability of a given amplicon to identify unknown bioagents such that the base compositions of amplicons with favorable probability scores are then stored in a base composition database (**325**). Alternatively, base compositions of the bioagent identifying amplicons obtained from the primers and GenBank sequences can be directly entered into the base composition database (**330**). Candidate primer pairs (**240**) are validated by testing their ability to hybridize to target nucleic acid by an *in* vitro amplification by a method such as PCR analysis (**400**) of nucleic acid from a collection of organisms (**410**). Amplification products thus obtained are analyzed by gel electrophoresis or by mass spectrometry to confirm the sensitivity, specificity and reproducibility of the primers used to obtain the amplification products (**420**).

Many of the important pathogens, including the organisms of greatest concern as biowarfare agents, have been completely sequenced. This effort has greatly facilitated the design of primers for the detection of unknown bioagents. The combination of broad-range priming with division-wide and drill-down priming has been used very successfully in several applications of the technology, including environmental surveillance for biowarfare threat agents and clinical sample analysis for medically important pathogens.

Synthesis of primers is well known and routine in the art. The primers may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed.

In some embodiments primers are employed as compositions for use in methods for identification of bacterial bioagents as follows: a primer pair composition is contacted with nucleic acid (such as, for example, bacterial DNA or DNA reverse transcribed from the rRNA) of an unknown bacterial bioagent. The nucleic acid is then amplified by a nucleic acid amplification technique, such as PCR for example, to obtain an amplification product that represents a bioagent identifying amplicon. The molecular mass of each strand of the double-stranded amplification product is determined by a molecular mass measurement technique such as mass spectrometry for example, wherein the two strands of the double-stranded amplification product are separated during the ionization process. In some embodiments, the mass spectrometry is electrospray Fourier transform ion cyclotron resonance mass spectrometry (ESI-FTICR-MS) or electrospray time of flight mass spectrometry (ESI-TOF-MS). A list of possible base compositions can be generated for the molecular mass value obtained for each strand and the choice of the correct base composition from the list is facilitated by matching the base composition of one strand with a complementary base composition of the other strand. The molecular mass or base composition thus determined is then compared with a database of molecular masses or base compositions of analogous bioagent identifying amplicons for known viral bioagents. A match between the molecular mass or base composition of the amplification product and the molecular mass or base composition of an analogous bioagent identifying amplicon for a known viral bioagent indicates the identity of the unknown bioagent. In some embodiments, the primer pair used is one of the primer pairs of Table 2. In some embodiments, the method is repeated using one or more different primer pairs to resolve possible ambiguities in the identification process or to improve the confidence level for the identification assignment.

In some embodiments, a bioagent identifying amplicon may be produced using only a single primer (either the forward or reverse primer of any given primer pair), provided an appropriate amplification method is chosen, such as, for example, low stringency single primer PCR (LSSP-PCR). Adaptation of this amplification method in order to produce bioagent identifying amplicons can be accomplished by one with ordinary skill in the art without undue experimentation.

In some embodiments, the oligonucleotide primers are broad range survey primers which hybridize to conserved regions of nucleic acid encoding the hexon gene of all (or between 80% and 100%, between 85% and 100%, between 90% and 100% or between 95% and 100%) known bacteria and produce bacterial bioagent identifying amplicons.

In some cases, the molecular mass or base composition of a bacterial bioagent identifying amplicon defined by a broad range survey primer pair does not provide enough resolution to unambiguously identify a bacterial bioagent at or below the species level. These cases benefit from further analysis of one or more bacterial bioagent identifying amplicons generated from at least one additional broad range survey primer pair or from at least one additional division-wide primer pair. The employment of more than one bioagent identifying amplicon for identification of a bioagent is herein referred to as triangulation identification.

In other embodiments, the oligonucleotide primers are division-wide primers which hybridize to nucleic acid encoding genes of species within a genus of bacteria. In other embodiments, the oligonucleotide primers are drill-down primers which enable the identification of sub-species characteristics. Drill down primers provide the functionality of producing bioagent identifying amplicons for drill-down analyses such as strain typing when contacted with nucleic acid under amplification conditions. Identification of such sub-species characteristics is often critical for determining proper clinical treatment of viral infections. In some embodiments, sub-species characteristics are identified using only broad range survey primers and division-wide and drill-down primers are not used.

In some embodiments, the primers used for amplification hybridize to and amplify genomic DNA, and DNA of bacterial plasmids.

In some embodiments, various computer software programs may be used to aid in design of primers for amplification reactions such as *Primer Premier 5* (Premier Biosoft, Palo Alto, CA) or *OLIGO* Primer Analysis Software (Molecular Biology Insights, Cascade, CO). These programs allow the user to input desired hybridization conditions such as melting temperature of a primer-template duplex for example. In some embodiments, an *in silico* PCR search algorithm, such as (ePCR) is used to analyze primer specificity across a plurality of template sequences which can be readily obtained from public sequence databases such as GenBank for example. An existing RNA structure search algorithm (Macke et al., Nucl. Acids Res., 2001, 29, 4724-4735, which is incorporated herein by reference in its entirety) has been modified to include PCR parameters such as hybridization conditions, mismatches, and thermodynamic calculations (SantaLucia, Proc. Natl. Acad. Sci. U.S.A., 1998, 95, 1460-1465). This also provides information on primer specificity of the selected primer pairs. In some embodiments, the hybridization conditions applied to the algorithm can limit the results of primer specificity obtained from the algorithm. In some embodiments, the melting temperature threshold for the primer template duplex is specified to be 35°C or a higher temperature. In some embodiments the number of acceptable mismatches is specified to be seven mismatches or less. In some embodiments, the buffer components and concentrations and primer concentrations may be specified and incorporated into the algorithm, for example, an appropriate primer concentration is about 250 nM and appropriate buffer components are 50 mM sodium or potassium and 1.5 mM Mg²⁺.

One with ordinary skill in the art of design of amplification primers will recognize that a given primer need not hybridize with 100% complementarity in order to effectively prime the synthesis of a complementary nucleic acid strand in an amplification reaction. Moreover, a primer may hybridize over one or more segments such that intervening or adjacent segments are not involved in the hybridization event. (e.g., for example, a loop structure or a hairpin structure). The primers of the present invention may comprise at least 70%, at least 75%, at least 80%, at least 85%, at least 90%, at least 95% or at least 99% sequence identity with any of the primers listed in Table 2. Thus, in some embodiments of the present invention, an extent of variation of 70% to 100%, or any range therewithin, of the sequence identity is possible relative to the specific primer sequences disclosed herein. Determination of sequence identity is described in the following example: a primer 20 nucleobases in length which is identical to another 20 nucleobase primer having two non-identical residues has 18 of 20 identical residues (18/20 = 0.9 or 90% sequence identity). In another example, a primer 15 nucleobases in length having all residues identical to a 15 nucleobase segment of primer 20 nucleobases in length would have 15/20 = 0.75 or 75% sequence identity with the 20 nucleobase primer.

Percent homology, sequence identity or complementarity, can be determined by, for example, the Gap program (Wisconsin Sequence Analysis Package, Version 8 for UNIX, Genetics Computer Group, University Research Park, Madison WI), using default settings, which uses the algorithm of Smith and Waterman (Adv. Appl. Math., 1981, 2, 482-489). In some embodiments, complementarity of primers with respect to the conserved priming regions of viral nucleic acid is between about 70% and about 75% 80%. In other embodiments, homology, sequence identity or complementarity, is between about 75% and about 80%. In yet other embodiments, homology, sequence identity or complementarity, is at least 85%, at least 90%, at least 92%, at least 94%, at least 95%, at least 96%, at least 97%, at least 98%, at least 99% or is 100%.

In some embodiments, the primers described herein comprise at least 70%, at least 75%, at least 80%, at least 85%, at least 90%, at least 92%, at least 94%, at least 95%, at least 96%, at least 98%, or at least 99%, or 100% (or any range therewithin) sequence identity with the primer sequences specifically disclosed herein.

One with ordinary skill is able to calculate percent sequence identity or percent sequence homology and able to determine, without undue experimentation, the effects of variation of primer sequence identity on the function of the primer in its role in priming synthesis of a complementary strand of nucleic acid for production of an amplification product of a corresponding bioagent identifying amplicon.

In one embodiment, the primers are at least 13 nucleobases in length. In another embodiment, the primers are less than 36 nucleobases in length.

In some embodiments of the present invention, the oligonucleotide primers are 13 to 35 nucleobases in length (13 to 35 linked nucleotide residues). These embodiments comprise oligonucleotide primers 13, 14, 15, 16, 17, 18, 19, 20, 21, 22, 23, 24, 25, 26, 27, 28, 29, 30, 31, 32, 33, 34 or 35 nucleobases in length, or any range therewithin. The present invention contemplates using both longer and shorter primers. Furthermore, the primers may also be linked to one or more other desired moieties, including, but not limited to, affinity groups, ligands, regions of nucleic acid that are not complementary to the nucleic acid to be amplified, labels, etc. Primers may also form hairpin structures. For example, hairpin primers may be used to amplify short target nucleic acid molecules. The presence of the hairpin may stabilize the amplification complex (see e.g., TAQMAN MicroRNA Assays, Applied Biosystems, Foster City, California).

In some embodiments, any oligonucleotide primer pair may have one or both primers with less then 70% sequence homology with a corresponding member of any of the primer pairs of Table 2 if the primer pair has the capability of producing an amplification product corresponding to a bioagent identifying amplicon. In other embodiments, any oligonucleotide primer pair may have one or both primers with a length greater than 35 nucleobases if the primer pair has the capability of producing an amplification product corresponding to a bioagent identifying amplicon.

In some embodiments, the function of a given primer may be substituted by a combination of two or more primers segments that hybridize adjacent to each other or that are linked by a nucleic acid loop structure or linker which allows a polymerase to extend the two or more primers in an amplification reaction.

In some embodiments, the primer pairs used for obtaining bioagent identifying amplicons are the primer pairs of Table 2. In other embodiments, other combinations of primer pairs are possible by combining certain members of the forward primers with certain members of the reverse primers. An example can be seen in Table 2 for two primer pair combinations of forward primer 16S_EC_789_810_F (SEQ ID NO: 206), with the reverse primers 16S_EC_880_894_R (SEQ ID NO: 796), or 16S_EC_882_899_R or (SEQ ID NO: 818). Arriving at a favorable alternate combination of primers in a primer pair depends upon the properties of the primer pair, most notably the size of the bioagent identifying amplicon that would be produced by the primer pair, which preferably is between about 45 to about 150 nucleobases in length. Alternatively, a bioagent identifying amplicon longer than 150 nucleobases in length could be cleaved into smaller segments by cleavage reagents such as chemical reagents, or restriction enzymes, for example.

In some embodiments, the primers are configured to amplify nucleic acid of a bioagent to produce amplification products that can be measured by mass spectrometry and from whose molecular masses candidate base compositions can be readily calculated.

In some embodiments, any given primer comprises a modification comprising the addition of a non-templated T residue to the 5' end of the primer (i.e., the added T residue does not necessarily hybridize to the nucleic acid being amplified). The addition of a non-templated T residue has an effect of minimizing the addition of non-templated adenosine residues as a result of the non-specific enzyme activity of *Taq* polymerase (Magnuson et al., Biotechniques, 1996, 21, 700-709), an occurrence which may lead to ambiguous results arising from molecular mass analysis.

In some embodiments of the present invention, primers may contain one or more universal bases. Because any variation (due to codon wobble in the 3^{rd} position) in the conserved regions among species is likely to occur in the third position of a DNA (or RNA) triplet, oligonucleotide primers can be designed such that the nucleotide corresponding to this position is a base which can bind to more than one nucleotide, referred to herein as a "universal nucleobase." For example, under this "wobble" pairing, inosine (I) binds to U, C or A; guanine (G) binds to U or C, and uridine (U) binds to U or C. Other examples of universal nucleobases include nitroindoles such as 5-nitroindole or 3-nitropyrrole (Loakes et al., Nucleosides and Nucleotides, 1995, 14, 1001-1003), the degenerate nucleotides dP or dK (Hill *et al*.), an acyclic nucleoside analog containing 5-nitroindazole (Van Aerschot et al., Nucleosides and Nucleotides, 1995, 14, 1053-1056) or the purine analog 1-(2-deoxy-β-D-ribofuranosyl)-imidazole-4-carboxamide (Sala et al., Nucl. Acids Res., 1996, 24, 3302-3306).

In some embodiments, to compensate for the somewhat weaker binding by the wobble base, the oligonucleotide primers are designed such that the first and second positions of each triplet are occupied by nucleotide analogs that bind with greater affinity than the unmodified nucleotide. Examples of these analogs include, but are not limited to, 2,6-diaminopurine which binds to thymine, 5-propynyluracil (also known as propynylated thymine) which binds to adenine and 5-propynylcytosine and phenoxazines, including G-clamp, which binds to G. Propynylated pyrimidines are described in U.S. Patent Nos. 5,645,985, 5,830,653 and 5,484,908 Propynylated primers are described in U.S Pre-Grant Publication No. 2003-0170682. Phenoxazines are described in U.S. Patent Nos. 5,502,177, 5,763,588, and 6,005,096. G-clamps are described in U.S. Patent Nos. 6,007,992 and 6,028,183.

In some embodiments, primer hybridization is enhanced using primers containing 5-propynyl deoxy-cytidine and deoxy-thymidine nucleotides. These modified primers offer increased affinity and base pairing selectivity.

In some embodiments, non-template primer tags are used to increase the melting temperature (Tₘ) of a primer-template duplex in order to improve amplification efficiency. A non-template tag is at least three consecutive A or T nucleotide residues on a primer which are not complementary to the template. In any given non-template tag, A can be replaced by C or G and T can also be replaced by C or G. Although Watson-Crick hybridization is not expected to occur for a non-template tag relative to the template, the extra hydrogen bond in a G-C pair relative to an A-T pair confers increased stability of the primer-template duplex and improves amplification efficiency for subsequent cycles of amplification when the primers hybridize to strands synthesized in previous cycles.

In other embodiments, propynylated tags may be used in a manner similar to that of the non-template tag, wherein two or more 5-propynylcytidine or 5-propynyluridine residues replace template matching residues on a primer. In other embodiments, a primer contains a modified internucleoside linkage such as a phosphorothioate linkage, for example.

In some embodiments, the primers contain mass-modifying tags. Reducing the total number of possible base compositions of a nucleic acid of specific molecular weight provides a means of avoiding a persistent source of ambiguity in determination of base composition of amplification products. Addition of mass-modifying tags to certain nucleobases of a given primer will result in simplification of *de novo* determination of base composition of a given bioagent identifying amplicon from its molecular mass.

In some embodiments of the present invention, the mass modified nucleobase comprises one or more of the following: for example, 7-deaza-2'-deoxyadenosine-5-triphosphate, 5-iodo-2'-deoxyuridine-5'-triphosphate, 5-bromo-2'-deoxyuridine-5'-triphosphate, 5-bromo-2'-deoxycytidine-5'-triphosphate, 5-iodo-2'-deoxycytidine-5'-triphosphate, 5-hydroxy-2'-deoxyuridine-5'-triphosphate, 4-thiothymidine-5'-triphosphate, 5-aza-2'-deoxyuridine-5'-triphosphate, 5-fluoro-2'-deoxyuridine-5'-triphosphate, 06-methyl-2'-deoxyguanosine-5'-triphosphate, N2-methyl-2'-deoxyguanosine-5'-triphosphate, 8-oxo-2'-deoxyguanosine-5'-triphosphate or thiothymidine-5'-triphosphate. In some embodiments, the mass-modified nucleobase comprises ¹⁵N or ¹³C or both ¹⁵N and ¹³C.

In some embodiments, multiplex amplification is performed where multiple bioagent identifying amplicons are amplified with a plurality of primer pairs. The advantages of multiplexing are that fewer reaction containers (for example, wells of a 96- or 384-well plate) are needed for each molecular mass measurement, providing time, resource and cost savings because additional bioagent identification data can be obtained within a single analysis. Multiplex amplification methods are well known to those with ordinary skill and can be developed without undue experimentation. However, in some embodiments, one useful and non-obvious step in selecting a plurality candidate bioagent identifying amplicons for multiplex amplification is to ensure that each strand of each amplification product will be sufficiently different in molecular mass that mass spectral signals will not overlap and lead to ambiguous analysis results. In some embodiments, a 10 Da difference in mass of two strands of one or more amplification products is sufficient to avoid overlap of mass spectral peaks.

In some embodiments, as an alternative to multiplex amplification, single amplification reactions can be pooled before analysis by mass spectrometry. In these embodiments, as for multiplex amplification embodiments, it is useful to select a plurality of candidate bioagent identifying amplicons to ensure that each strand of each amplification product will be sufficiently different in molecular mass that mass spectral signals will not overlap and lead to ambiguous analysis results.

### C Determination of Molecular Mass of Bioagent Identifying Amplicons

In some embodiments, the molecular mass of a given bioagent identifying amplicon is determined by mass spectrometry. Mass spectrometry has several advantages, not the least of which is high bandwidth characterized by the ability to separate (and isolate) many molecular peaks across a broad range of mass to charge ratio (m/z). Thus mass spectrometry is intrinsically a parallel detection scheme without the need for radioactive or fluorescent labels, since every amplification product is identified by its molecular mass. The current state of the art in mass spectrometry is such that less than femtomole quantities of material can be readily analyzed to afford information about the molecular contents of the sample. An accurate assessment of the molecular mass of the material can be quickly obtained, irrespective of whether the molecular weight of the sample is several hundred, or in excess of one hundred thousand atomic mass units (amu) or Daltons.

In some embodiments, intact molecular ions are generated from amplification products using one of a variety of ionization techniques to convert the sample to gas phase. These ionization methods include, but are not limited to, electrospray ionization (ES), matrix-assisted laser desorption ionization (MALDI) and fast atom bombardment (FAB). Upon ionization, several peaks are observed from one sample due to the formation of ions with different charges. Averaging the multiple readings of molecular mass obtained from a single mass spectrum affords an estimate of molecular mass of the bioagent identifying amplicon. Electrospray ionization mass spectrometry (ESI-MS) is particularly useful for very high molecular weight polymers such as proteins and nucleic acids having molecular weights greater than 10 kDa, since it yields a distribution of multiply-charged molecules of the sample without causing a significant amount of fragmentation.

The mass detectors used in the methods of the present invention include, but are not limited to, Fourier transform ion cyclotron resonance mass spectrometry (FT-ICR-MS), time of flight (TOF), ion trap, quadrupole, magnetic sector, Q-TOF, and triple quadrupole.

### D. Base Compositions of Bioagent Identifying Amplicons

Although the molecular mass of amplification products obtained using intelligent primers provides a means for identification of bioagents, conversion of molecular mass data to a base composition signature is useful for certain analyses. As used herein, "base composition" is the exact number of each nucleobase (A, T, C and G) determined from the molecular mass of a bioagent identifying amplicon. In some embodiments, a base composition provides an index of a specific organism. Base compositions can be calculated from known sequences of known bioagent identifying amplicons and can be experimentally determined by measuring the molecular mass of a given bioagent identifying amplicon, followed by determination of all possible base compositions which are consistent with the measured molecular mass within acceptable experimental error. The following example illustrates determination of base composition from an experimentally obtained molecular mass of a 46-mer amplification product originating at position 1337 of the 16S rRNA of *Bacillus anthracis.* The forward and reverse strands of the amplification product have measured molecular masses of 14208 and 14079 Da, respectively. The possible base compositions derived from the molecular masses of the forward and reverse strands for the *B. anthracis* products are listed in Table 1.

**Table 1**

| **Possible Base Compositions for *B. anthracis* 46mer Amplification Product** | | | | | |
|---|---|---|---|---|---|
| **Calc. Mass Forward Strand** | **Mass Error Forward Strand** | **Base composition of Forward Strand** | **Calc. Mass Reverse Strand** | **Mass Error Reverse Strand** | **Base Composition of Reverse Strand** |
| 14208.2935 | 0.079520 | A1 G17 C10 T18 | 14079.2624 | 0.080600 | A0 G14 C13 T19 |
| 14208.3160 | 0.056980 | A1 G20 C15- T10 | 14079.2849 | 0.058060 | A0 G17 C18 T11 |
| 14208.3386 | 0.034440 | A1 G23 C20 T2 | 14079.3075 | 0.035520 | A0 G20 C23 T3 |
| 14208.3074 | 0.065560 | A6 G11 C3 T26 | 14079.2538 | 0.089180 | A5 G5 C1 T35 |
| 14208.3300 | 0.043020 | A6 G14 C8 T18 | 14079.2764 | 0.066640 | A5 G8 C6 T27 |
| 14208.3525 | 0.020480 | A6 G17 C13 T10 | 14079.2989 | 0.044100 | AS G11 C11 T19 |
| 14208.3751 | 0.002060 | A6 G20 C18 T2 | 14079.3214 | 0.021560 | A5 G14 C16 T11 |
| 14208.3439 | 0.029060 | A11 G8 C1 T26 | 14079.3440 | 0.000980 | A5 G17 C21 T3 |
| 14208.3665 | 0.006520 | A11 G11 C6 T18 | 14079.3129 | 0.030140 | A10 G5 C4 T27 |
| 14208.3890 | 0.016020 | All G14 C11 T10 | 14079.3354 | 0.007600 | A10 G8 C9 T19 |
| 14208.4116 | 0.038560 | A11 G17 C16 T2 | 14079.3579 | 0.014940 | A10 G11 C14 T11 |
| 14208.4030 | 0.029980 | A16 G8 C4 T18 | 14079.3805 | 0.037480 | A10 G14 C19 T3 |
| 14208.4255 | 0.052520 | A16 G11 C9 T10 | 14079.3494 | 0.006360 | A15 G2 C2 T27 |
| 14208.4481 | 0.075060 | A16 G14 C14 T2 | 14079.3719 | 0.028900 | A15 G5 C7 T19 |
| 14208.4395 | 0.066480 | A21 G5 C2 T18 | 14079.3944 | 0.051440 | A15 GB C12 T11 |
| 14208.4620 | 0.089020 | A21 G8 C7 T10 | 14079.4170 | 0.073980 | A15 G11 C17 T3 |
| - | - | - | 14079.4084 | 0.065400 | A20 G2 C5 T19 |
| - | - | - | 14079.4309 | 0.087940 | A20 G5 C10 T13 |

Among the 16 possible base compositions for the forward strand and the 18 possible base compositions for the reverse strand that were calculated, only one pair (shown in bold) are complementary base compositions, which indicates the true base composition of the amplification product. It should be recognized that this logic is applicable for determination of base compositions of any bioagent identifying amplicon, regardless of the class of bioagent from which the corresponding' amplification product was obtained.

In some embodiments, assignment of previously unobserved base compositions (also known as "true unknown base compositions") to a given phylogeny can be accomplished via the use of pattern classifier model algorithms. Base compositions, like sequences, vary slightly from strain to strain within species, for example. In some embodiments, the pattern classifier model is the mutational probability model. On other embodiments, the pattern classifier is the polytope model. The mutational probability model and polytope model are both commonly owned and described in U.S. Patent application Serial No. 11/073,362.

In one embodiment, it is possible to manage this diversity by building "base composition probability clouds" around the composition constraints for each species. This permits identification of organisms in a fashion similar to sequence analysis. A "pseudo four-dimensional plot" can be used to visualize the concept of base composition probability clouds. Optimal primer design requires optimal choice of bioagent identifying amplicons and maximizes the separation between the base composition signatures of individual bioagents. Areas where clouds overlap indicate regions that may result in a misclassification, a problem which is overcome by a triangulation identification process using bioagent identifying amplicons not affected by overlap of base composition probability clouds.

In some embodiments, base composition probability clouds provide the means for screening potential primer pairs in order to avoid potential misclassifications of base compositions. In other embodiments, base composition probability clouds provide the means for predicting the identity of a bioagent whose assigned base composition was not previously observed and/or indexed in a bioagent identifying amplicon base composition database due to evolutionary transitions in its nucleic acid sequence. Thus, in contrast to probe-based techniques, mass spectrometry determination of base composition does not require prior knowledge of the composition or sequence in order to make the measurement.

The present invention provides bioagent classifying information similar to DNA sequencing and phylogenetic analysis at a level sufficient to identify a given bioagent. Furthermore, the process of determination of a previously unknown base composition for a given bioagent (for example, in a case where sequence information is unavailable) has downstream utility by providing additional bioagent indexing information with which to populate base composition databases. The process of future bioagent identification is thus greatly improved as more BCS indexes become available in base composition databases.

### E. Triangulation Identification

In some cases, a molecular mass of a single bioagent identifying amplicon alone does not provide enough resolution to unambiguously identify a given bioagent. The employment of more than one bioagent identifying amplicon for identification of a bioagent is herein referred to as "triangulation identification." Triangulation identification is pursued by determining the molecular masses of a plurality of bioagent identifying amplicons selected within a plurality of housekeeping genes. This process is used to reduce false negative and false positive signals, and enable reconstruction of the origin of hybrid or otherwise engineered bioagents. For example, identification of the three part toxin genes typical of *B. anthracis* (Bowen et al., J. Appl. Microbiol., 1999, 87, 270-278) in the absence of the expected signatures from the *B. anthracis* genome would suggest a genetic engineering event.

In some embodiments, the triangulation identification process can be pursued by characterization ofbioagent identifying amplicons in a massively parallel fashion using the polymerase chain reaction (PCR), such as multiplex PCR where multiple primers are employed in the same amplification reaction mixture, or PCR in multi-well plate format wherein a different and unique pair of primers is used in multiple wells containing otherwise identical reaction mixtures. Such multiplex and multi-well PCR methods are well known to those with ordinary skill in the arts of rapid throughput amplification of nucleic acids. In other related embodiments, one PCR reaction per well or container may be carried out, followed by an amplicon pooling step wherein the amplification products of different wells are combined in a single well or container which is then subjected to molecular mass analysis. The combination of pooled amplicons can be chosen such that the expected ranges of molecular masses of individual amplicons are not overlapping and thus will not complicate identification of signals.

### F. Codon Base Composition Analysis

In some embodiments of the present disclosure, one or more nucleotides substitutions within a codon of a gene of an infectious organism confer drug resistance upon an organism which can be determined by codon base composition analysis. The organism can be a bacterium, virus, fungus or protozoan.

In some embodiments, the amplification product containing the codon being analyzed is of a length of about 35 to about 200 nucleobases. The primers employed in obtaining the amplification product can hybridize to upstream and downstream sequences directly adjacent to the codon, or can hybridize to upstream and downstream sequences one or more sequence positions away from the codon. The primers may have between about 70% to 100% sequence complementarity with the sequence of the gene containing the codon being analyzed.

In some embodiments, the codon base composition analysis is undertaken

In some embodiments, the codon analysis is undertaken for the purpose of investigating genetic disease in an individual. In other embodiments, the codon analysis is undertaken for the purpose of investigating a drug resistance mutation or any other deleterious mutation in an infectious organism such as a bacterium, virus, fungus or protozoan. In some embodiments, the bioagent is a bacterium identified in a biological product.

In some embodiments, the molecular mass of an amplification product containing the codon being analyzed is measured by mass spectrometry. The mass spectrometry can be either electrospray (ESI) mass spectrometry or matrix-assisted laser desorption ionization (MALDI) mass spectrometry. Time-of-flight (TOF) is an example of one mode of mass spectrometry compatible with the analyses of the present invention.

The methods of the present invention can also be employed to determine the relative abundance of drug resistant strains of the organism being analyzed. Relative abundances can be calculated from amplitudes of mass spectral signals with relation to internal calibrants. In some embodiments, known quantities of internal amplification calibrants can be included in the amplification reactions and abundances of analyte amplification product estimated in relation to the known quantities of the calibrants.

In some embodiments, upon identification of one or more drug-resistant strains of an infectious organism infecting an individual, one or more alternative treatments can be devised to treat the individual.

### G. Determination of the Quantity of a Bioagent

In some embodiments, the identity and quantity of an unknown bioagent can be determined using the process illustrated in Figure 2. Primers (500) and a known quantity of a calibration polynucleotide (505) are added to a sample containing nucleic acid of an unknown bioagent. The total nucleic acid in the sample is then subjected to an amplification reaction (510) to obtain amplification products. The molecular masses of amplification products are determined (515) from which are obtained molecular mass and abundance data. The molecular mass of the bioagent identifying amplicon (520) provides the means for its identification (525) and the molecular mass of the calibration amplicon obtained from the calibration polynucleotide (530) provides the means for its identification (535). The abundance data of the bioagent identifying amplicon is recorded (540) and the abundance data for the calibration data is recorded (545), both of which are used in a calculation (550) which determines the quantity of unknown bioagent in the sample.

A sample comprising an unknown bioagent is contacted with a pair of primers that provide the means for amplification of nucleic acid from the bioagent, and a known quantity of a polynucleotide that comprises a calibration sequence. The nucleic acids of the bioagent and of the calibration sequence are amplified and the rate of amplification is reasonably assumed to be similar for the nucleic acid of the bioagent and of the calibration sequence. The amplification reaction then produces two amplification products: a bioagent identifying amplicon and a calibration amplicon. The bioagent identifying amplicon and the calibration amplicon should be distinguishable by molecular mass while being amplified at essentially the same rate. Effecting differential molecular masses can be accomplished by choosing as a calibration sequence, a representative bioagent identifying amplicon (from a specific species of bioagent) and performing, for example, a 2-8 nucleobase deletion or insertion within the variable region between the two priming sites. The amplified sample containing the bioagent identifying amplicon and the calibration amplicon is then subjected to molecular mass analysis by mass spectrometry, for example. The resulting molecular mass analysis of the nucleic acid of the bioagent and of the calibration sequence provides molecular mass data and abundance data for the nucleic acid of the bioagent and of the calibration sequence. The molecular mass data obtained for the nucleic acid of the bioagent enables identification of the Unknown bioagent and the abundance data enables calculation of the quantity of the bioagent, based on the knowledge of the quantity of calibration polynucleotide contacted with the sample.

In some embodiments, construction of a standard curve where the amount of calibration polynucleotide spiked into the sample is varied provides additional resolution and improved confidence for the determination of the quantity of bioagent in the sample. The use of standard curves for analytical determination of molecular quantities is well known to one with ordinary skill and can be performed without undue experimentation.

In some embodiments, multiplex amplification is performed where multiple bioagent identifying amplicons are amplified with multiple primer pairs which also amplify the corresponding standard calibration sequences. In this or other embodiments, the standard calibration sequences are optionally included within a single vector which functions as the calibration polynucleotide. Multiplex amplification methods are well known to those with ordinary skill and can be performed without undue experimentation.

In some embodiments, the calibrant polynucleotide is used as an internal positive control to confirm that amplification conditions and subsequent analysis steps are successful in producing a measurable amplicon. Even in the absence of copies of the genome of a bioagent, the calibration polynucleotide should give rise to a calibration amplicon. Failure to produce a measurable calibration amplicon indicates a failure of amplification or subsequent analysis step such as amplicon purification or molecular mass determination. Reaching a conclusion that such failures have occurred is in itself, a useful event.

In some embodiments, the calibration sequence is comprised of DNA. In some embodiments, the calibration sequence is comprised of RNA.

In some embodiments, the calibration sequence is inserted into a vector that itself functions as the calibration polynucleotide. In some embodiments, more than one calibration sequence is inserted into the vector that functions as the calibration polynucleotide. Such a calibration polynucleotide is herein termed a "combination calibration polynucleotide." The process of inserting polynucleotides into vectors is routine to those skilled in the art and can be accomplished without undue experimentation. Thus, it should be recognized that the calibration method should not be limited to the embodiments described herein. The calibration method can be applied for determination of the quantity of any bioagent identifying amplicon when an appropriate standard calibrant polynucleotide sequence is designed and used. The process of choosing an appropriate vector for insertion of a calibrant is also a routine operation that can be accomplished by one with ordinary skill without undue experimentation.

### H. Identification of Bacteria

In other embodiments, the primer pairs produce bioagent identifying amplicons within stable and highly conserved regions of bacteria. The advantage to characterization of an amplicon defined by priming regions that fall within a highly conserved region is that there is a low probability that the region will evolve past the point of primer recognition, in which case, the primer hybridization of the amplification step would fail. Such a primer set is thus useful as a broad range survey-type primer. In another embodiment, the intelligent primers produce bioagent identifying amplicons including a region which evolves more quickly than the stable region described above. The advantage of characterization bioagent identifying amplicon corresponding to an evolving genomic region is that it is useful for distinguishing emerging strain variants or the presence of virulence genes, drug resistance genes, or codon mutations that induce drug resistance.

The present invention also has significant advantages as a platform for identification of diseases caused by emerging bacterial strains such as, for example, drug-resistant strains of *Staphylococcus aureus.* The present invention eliminates the need for prior knowledge of bioagent sequence to generate hybridization probes. This is possible because the methods are not confounded by naturally occurring evolutionary variations occurring in the sequence acting as the template for production of the bioagent identifying amplicon. Measurement of molecular mass and determination of base composition is accomplished in an unbiased manner without sequence prejudice.

Another embodiment of the present invention also provides a means of tracking the spread of a bacterium, such as a particular drug-resistant strain when a plurality of samples obtained from different locations are analyzed by the methods described above in an epidemiological setting. In one embodiment, a plurality of samples from a plurality of different locations is analyzed with primer pairs which produce bioagent identifying amplicons, a subset of which contains a specific drug-resistant bacterial strain. The corresponding locations of the members of the drug-resistant strain subset indicate the spread of the specific drug-resistant strain to the corresponding locations.

### I. Kits

The present disclosure also includes kits for carrying out the methods described herein. In some embodiments, the kit may comprise a sufficient quantity of one or more primer pairs to perform an amplification reaction on a target polynucleotide from a bioagent to form a bioagent identifying amplicon. In some embodiments, the kit may comprise from one to fifty primer pairs, from one to twenty primer pairs, from one to ten primer pairs, or from two to five primer pairs. In some embodiments, the kit may comprise one or more primer pairs recited in Table 2.

In some embodiments, the kit comprises one or more broad range survey primer(s), division wide primer(s), or drill-down primer(s), or any combination thereof. If a given problem involves identification of a specific bioagent, the solution to the problem may require the selection of a particular combination of primers to provide the solution to the problem. A kit may be designed so as to comprise particular primer pairs for identification of a particular bioagent. A drill-down kit may be used, for example, to distinguish different genotypes or strains, drug-resistant, or otherwise. In some embodiments, the primer pair components of any of these kits may be additionally combined to comprise additional combinations of broad range survey primers and division-wide primers so as to be able to identify a bacterium.

In some embodiments, the kit contains standardized calibration polynucleotides for use as internal amplification calibrants. Internal calibrants are described in commonly owned U.S. Patent Application Serial No: 60/545,425.

In some embodiments, the kit comprises a sufficient quantity of reverse transcriptase (if RNA is to be analyzed for example), a DNA polymerase, suitable nucleoside triphosphates (including alternative dNTPs such as inosine or modified dNTPs such as the 5-propynyl pyrimidines or any dNTP containing molecular mass-modifying tags such as those described above), a DNA ligase, and/or reaction buffer, or any combination thereof, for the amplification processes described above. A kit may further include instructions pertinent for the particular embodiment of the kit, such instructions describing the primer pairs and amplification conditions for operation of the method. A kit may also comprise amplification reaction containers such as microcentrifuge tubes and the like. A kit may also comprise reagents or other materials for isolating bioagent nucleic acid or bioagent identifying amplicons from amplification, including, for example, detergents, solvents, or ion exchange resins which may be linked to magnetic beads. A kit may also comprise a table of measured or calculated molecular masses and/or base compositions of bioagents using the primer pairs of the kit

Some embodiments are kits that contain one or more survey bacterial primer pairs represented by primer pair compositions wherein each member of each pair of primers has 70% to 100% sequence identity with the corresponding member from the group of primer pairs represented by any of the primer pairs of Table S. The survey primer pairs may include broad range primer pairs which hybridize to ribosomal RNA, and may also include division-wide primer pairs which hybridize to housekeeping genes such as rplB, tufB, rpoB, rpoC, vals, and infB, for example.

In some embodiments, a kit may contain one or more survey bacterial primer pairs and one or more triangulation genotyping analysis primer pairs such as the primer pairs of Tables 8, 12, 14, 19, 21, 23, or 24. In some embodiments, the kit may represent a less expansive genotyping analysis but include triangulation genotyping analysis primer pairs for more than one genus or species of bacteria. For example, a kit for surveying nosocomial infections at a health care facility may include, for example, one or more broad range survey primer pairs, one or more division wide primer pairs, one or more *Acinetobacter baumannii* triangulation genotyping analysis primer pairs and one or more *Staphylococcus aureus* triangulation genotyping analysis primer pairs. One with ordinary skill will be capable of analyzing *in silico* amplification data to determine which primer pairs will be able to provide optimal identification resolution for the bacterial bioagents of interest.

In some embodiments, a kit may be assembled for identification of strains of bacteria involved in contamination of food. An example of such a kit embodiment is a kit comprising one or more bacterial survey primer pairs of Table 5 with one or more triangulation genotyping analysis primer pairs of Table 12 which provide strain resolving capabilities for identification of specific strains of *Campylobacter jejuni.*

Some embodiments of the kits are 96-well or 384-well plates with a plurality of wells containing any or all of the following components: dNTPs, buffer salts, Mg²⁺, betaine, and primer pairs. In some embodiments, a polymerase is also included in the plurality of wells of the 96-well or 384-well plates.

Some embodiments of the kit contain instructions for PCR and mass spectrometry analysis of amplification products obtained using the primer pairs of the kits.

Some embodiments of the kit include a barcode which uniquely identifies the kit and the components contained therein according to production lots and may also include any other information relative to the components such as concentrations, storage temperatures, etc. The barcode may also include analysis information to be read by optical barcode readers and sent to a computer controlling amplification, purification and mass spectrometric measurements. In some embodiments, the barcode provides access to a subset of base compositions in a base composition database which is in digital communication with base composition analysis software such that a base composition measured with primer pairs from a given kit can be compared with known base compositions ofbioagent identifying amplicons defined by the primer pairs of that kit.

In some embodiments, the kit contains a database of base compositions of bioagent identifying amplicons defined by the primer pairs of the kit. The database is stored on a convenient computer readable medium such as a compact disk or USB drive, for example.

In some embodiments, the kit includes a computer program stored on a computer formatted medium (such as a compact disk or portable USB disk drive, for example) comprising instructions which direct a processor to analyze data obtained from the use of the primer pairs of the present invention. The instructions of the software transform data related to amplification products into a molecular mass or base composition which is a useful concrete and tangible result used in identification and/or classification of bioagents. In some embodiments, the kits of the present invention contain all of the reagents sufficient to carry out one or more of the methods described herein.

While the present invention has been described with specificity in accordance with certain of its embodiments, the following examples serve only to illustrate the invention and are not intended to limit the same. In order that the invention disclosed herein may be more efficiently understood, examples are provided below. It should be understood that these examples are for illustrative purposes only and are not to be construed as limiting the invention in any manner.

### EXAMPLES

### Example 1: Design and Validation of Primers that Define Bioagent Identifying Amplicons for Identification of Bacteria

For design of primers that define bacterial bioagent identifying amplicons, a series of bacterial genome segment sequences were obtained, aligned and scanned for regions where pairs of PCR primers would amplify products of about 45 to about 150 nucleotides in length and distinguish subgroups and/or individual strains from each other by their molecular masses or base compositions. A typical process shown in Figure 1 is employed for this type of analysis.

A database of expected base compositions for each primer region was generated using an in *silico* PCR search algorithm, such as (ePCR). An existing RNA structure search algorithm (Macke et al., Nucl. Acids Res., 2001,29,4724-4735) has been modified to include PCR parameters such as hybridization conditions, mismatches, and thermodynamic calculations (SantaLucia, Proc. Natl. Acad. Sci. U.S.A., 1998, 95, 1460-1465 This also provides information on primer specificity of the selected primer pairs.

Table 2 represents a collection of primers (sorted by primer pair number) designed to identify bacteria using the methods described herein. The primer pair number is an in-house database index number. Primer sites were identified on segments of genes, such as, for example, the 16S rRNA gene. The forward or reverse primer name shown in Table 2 indicates the gene region of the bacterial genome to which the primer hybridizes relative to a reference sequence. In Table 2, for example, the forward primer name 16S_EC_1077_1106_F indicates that the forward primer (_F) hybridizes to residues 1077-1106 of the reference sequence represented by a sequence extraction of coordinates 4033120..4034661 from GenBank gi number 16127994 (as indicated in Table 3). As an additional example: the forward primer name BONTA_X52066_450_473 indicates that the primer hybridizes to residues 450-437 of the gene encoding *Clostridium botulinum* neurotoxin type A (BoNT/A) represented by GenBank Accession No. X52066 (primer pair name codes appearing in Table 2 are defined in Table 3. One with ordinary skill knows how to obtain individual gene sequences or portions thereof from genomic sequences present in GenBank. In Table 2, Tp = 5-propynyluracil; Cp = 5-propynylcytosine; * = phosphorothioate linkage; I = inosine. T. GenBank Accession Numbers for reference sequences of bacteria are shown in Table 3 (below). In some cases, the reference sequences are extractions from bacterial genomic sequences or complements thereof.

**Table 2: Primer Pairs for Identification of Bacteria**

| **Primer Pair Number** | **Forward Primer Name** | **Forward Sequence** | **Forward SEQ ID NO:** | **Reverse Primer Name** | **Reverse Sequence** | **Reverse (SEQ ID NO:** |
|---|---|---|---|---|---|---|
| 1 | 16S EC 1077 1106 F | | 134 | 16S EC 1175 1195 R | GACGTCATCCCCACCTTCCTC | 809 |
| 2 | 16S EC 1082 1106 F | ATGTTGGGTTAAGTCCCGCAACGAG | 38 | 16S EC 1175 1197 R | TTGACGTCATCCCCACCTTCCTC | 1398 |
| 3 | 16S EC 1090 1111 F | TTAAGTCCCGCAACGATCGCAA | 651 | 16S EC 1175 1196 R | TGACGTCATCCCCACCTTCCTC | 1159 |
| 4 | 16S EC 1222 1241 F | GCTACACACGTGCTACAATG | 114 | 16S EC 1303 1323 R | CGAGTTGCAGACTGCGATCCG | 787 |
| 5 | 16S EC 1332 1353 F | AAGTCGGAATCGCTAGTAATCG | 10 | 16S EC 1389 1407 R | GACGGGCGGTGTGTACAAG | 806 |
| 6 | 16S EC 30 54 F | TGAACGCTGGTGGCATGCTTAACAC | 429 | 16S EC 105 126 R | TACGCATTACTCACCCGTCCGC | 897 |
| 7 | 16S EC 38 64 F | GTGGCATGCCTAATACATGCAAGTCG | 136 | 16S EC 101 120 R | TTACTCACCCGTCCGCCGCT | 1365 |
| 8 | 16S EC 49 68 F | TAACACATGCAAGTCGAACG | 152 | 16S EC 104 120 R | TTACTCACCCGTCCGCC | 1364 |
| 9 | 16S EC 683 700 F | GTGTAGCGGTGAAATGCG | 137 | 16S EC 774 795 R | GTATCTAATCCTGTTTGCTCCC | 839 |
| 10 | 16S EC 713 732 F | AGAACACCGATGGCGAAGGC | 21 | 16S EC 789 809_R | CGTGGACTACCAGGGTATCTA | 798 |
| 11 | 16S EC_785 806 F | GGATTAGAGACCCTGGTAGTCC | 118 | 16S_EC 880 897 R | GGCCGTACTCCCCAGGCG | 830 |
| 12 | 16S EC 785_810F | GGATTAGATACCCTGGTAGTCCACGC | 119 | 16S EC 880 897 2 R | GGCCGTACTCCCCAGGCG | 830 |
| 13 | 16S EC 789 810 F | TAGATACCCTGGTAGTCCACGC | 206 | 16S_EC 880_ 894 R | CGTACTCCCCAGGCG | 796 |
| 14 | 16S EC 960 981 F | TTCGATGCAACGCGAAGAACCT | 672 | 16S EC 1054 1073 R | ACGAGCTGACGACAGCCATG | 735 |
| 15 | 16S EC 969 985 F | ACGCGAAGAACCTTACC | 19 | 16S EC 1061 1078 R | ACGACACGAGCTGACGAC | 734 |
| 16 | 23S EC 1826 1843 F | CTGACACCTGCCCGGTGC | 80 | 23S EC 1906 1924 R | GACCGTTATAGTTACGGCC | 805 |
| 17 | 23S EC 2645 2669 F | TCTGTCCCTAGTACGAGAGGACCGG | 408 | 23S EC 2744 2761 R | TGCTTAGATGCTTTCAGC | 1252 |
| 18 | 23S_EC_2645_2669_2_F | CTGTCCCTAGTACGAGAGGACCGG | 83 | 23S EC 2751 2767 R | GTTTCATGCTTAGATGCTTTCAGC | 846 |
| 19 | 23S EC 493_518 F | GGGGAGTGAAAGAGATCCTGAAACCG | 125 | 23S EC_551 571 R | ACAAAAGGTACGCCGTCACCC | 717 |
| 20 | 23S EC_493 518 2 F | GGGGAGTGAAAGAGATCCTGAAACCG | 125 | 23S EC 551 571 2 R | ACAAAAGGCACGCCATCACCC | 716 |
| 21 | 23S EC 971 992 F | CGAGAGGGAAACAACCCAGACC | 66 | 23SEC 1059 1077 R | TGGCTGCTTCTAAGCCAAC | 1282 |
| 22 | CAPC BA 104 131 F | | 139 | CAPC BA 180 205 R | TGAATCTTGAAACACCATACGTAACG | 1150 |
| 23 | CAPC BA 114 133 F | ACTCGTTTTTAATCAGCCCG | 20 | CAPC BA 185 205 R | TGAATCTTGAAACACCATACG | 1149 |
| 24 | CAPC BA 274 303 F | | 109 | CAPC BA 349 376 R | GTAACCCTTGTCTTTGAATTGTATTTGC | 837 |
| 25 | CAPC BA 276 296 F | TTATTGTTATCCTGTTATGCC | 663 | CAPC AHA 358 377 R | GGTAACCCTTGTCTTTGAAT | 834 |
| 26 | CAPC BA 281 301 F | GTTATCCTGTTATGCCATTTG | 138 | CAPC BA 361 378 R | TGGTAACCCTTGTCTTTG | 1298 |
| 27 | CAPC BA 315 334 F | CCGTGGTATTGGAGTTATTG | 59 | CAPC BA 361 378 R | TGGTAACCCTTGTCTTTG | 1298 |
| 28 | CYA BA 1055 1072 F | GAAAGAGTTCGGATTGGG | 92 | CYA BA 1112 1130 R | TGTTGACCATGCTTCTTAG | 1352 |
| 29 | CYA_BA 1349_1370_F | ACAACGAAGTACAATACAAGAC | 12 | CYA_BA_1447_1426_R | CTTCTACATTTTTAGCCATCAC | 800 |
| 30 | CYA_BA 1353_1379_F | | 64 | CYA_BA_1448_1467_R | TGTTAACGGCTTCAAGACCC | 1342 |
| 31 | CYA_BA 1359_1379_F | ACAATACAAGACAAAAGAAGG | 13 | CYA_BA_1447_1461_R | CGGCTTCAAGACCCC | 794 |
| 32 | CYA_BA_914_937_F | CAGGTTTAGTACCAGAACATGCAG | 53 | CYA_BA_999_1026_R | ACCACTTTTAATAAGGTTTGTAGCTAAC | 728 |
| 33 | CYA_BA_916_935_F | GGTTTAGTACCAGAACATGC | 131 | CYA_BA_1003_1025_R | CCACTTTTAATAAGGTTTGTAGC | 768 |
| 34 | INFB_EC_1165_1393_F | | 524 | INFB_EC_1439_1467_R | | 1248 |
| 35 | LEF_BA 1033_1052_F | TCAAGAAGAAAAAGAGC | 254 | LEF_BA_1119_1135_R | GAATATCAATTTGTAGC | 803 |
| 36 | LEF_BA 1036_1066_F | | 44 | LEF_BA_1119_1149_R | | 745 |
| 37 | LEF_BA_756_781_F | AGCTTTTGCATATTATATCGAGCCAC | 26 | LEF_BA_843_872_R | | 1135 |
| 38 | LEF_BA_758_778_F | CTTTTGCATATTATATCGAGC | 90 | LEF_BA_843_865_R | AGGATAGATTTATTTCTTGTTCG | 748 |
| 39 | LEF_BA_795813_F | TTTACAGCTTTATGCACCG | 700 | LEF_BA_883_900_R | TCTTGACAGCATCCGTTG | 1140 |
| 40 | LEF_BA_883 899_F | CAACGGATGCTGGCAAG | 43 | LEF_BA_939_958_R | CAGATAAAGAATCGCTCCAG | 762 |
| 41 | PAG_BA_122_142_F | CAGAATCAAGTTCCCAGGGG | 49 | PAG_BA_190 209_R | CCTGTAGTAGAAGAGGTAAC | 781 |
| 42 | PAG_BA_123_145_F | AGAATCAAGTTCCCAGGGGTTAC | 22 | PAG_BA_187_210_R | CCCTGTAGTAGAAGAGGTAACCAC | 774 |
| 43 | PAG_BA_269_287_F | AATCTGCTATTTGGTCAGG | 11 | PAG_BA_326_344_R | TGATTATCAGCGGAAGTAG | 1186 |
| 44 | PAG_BA_655_675_F | GAAGGATATACGGTTGATGTC | 93 | PAG_BA_755_772_R | CCGTGCTCCATTTTTCAG | 778 |
| 45 | PAG_BA_753_772_F | TCCTGAAAAATGGAGCACGG | 341 | PAG_BA_849_868_R | TCGGATAAGCTGCCACAAGG | 1089 |
| 46 | PAG_BA_763_781_F | TGGAGCACGGCTTCTGATC | 552 | PAG_BA_849_868_R | TCGGATAAGCTGCCACAAGG | 1089 |
| 47 | RPOC_EC_1018_1045_F | | 39 | RPOC_EC_1095_1124_R | | 959 |
| 48 | RPOC_EC 1018_1045_2_ F | | 39 | RPOC_EC 1095 1124_2_R | | 958 |
| 49 | RPOC_EC_114_140_F | | 158 | RPOC_EC_213_232_R | GGCGCTTGTACTTACCGCAC | 831 |
| 50 | RPOC EC 2178_2196_F | TGATTCTGGTGCCCGTGGT | 478 | RPOC_EC_2225_2246_R | TTGGCCATCAGGCCACGCATAC | 1414 |
| 51 | RPOC_EC_2176_2196_2_ F | TGATTCCGGTGCCCGTGGT | 477 | RPOC_EC_2225_2246_2_R | TTGGCCATCAGACCACGCATAC | 1413 |
| 52 | RPOC_EC_2218_2241_F | CTGGCAGGTATGCGTGGTCTGATG | 81 | RPOC_EC_2313_2337_R | CGCACCGTGGGTTGAGATGAAGTAC | 790 |
| 53 | RPOC_EC_2 218_2241_2_ F | CTTGCTGGTATGCGTGGTCTGATG | 86 | RPOC_EC_2313_2337_2_R | CGCACCATGCGTAGAGATGAAGTAC | 789 |
| 54 | RPOC_EC_808_833_F | CGTCGGGTGATTAACCGTAACAACCG | 75 | RPOC_EC_865_889_R | GTTTTTCGTTGCGTACGATGATGTC | 847 |
| 55 | RPOC_EC 808_833_2_F | CGTCGTGTAATTAACCGTAACAACCG | 76 | RPOC_EC 865_ 891_R | ACGTTTTTCGTTTTGAACGATAATGCT | 741 |
| 56 | RPOC_EC_993_1019_F | | 41 | RPOC EC 1036 1059 R | CGAACGGCCTGAGTAGTCAACACG | 785 |
| 57 | RPOC_EC 993_1019_2_ F | | 40 | RPOC EC_1036 1059_2_R | CGAACGGCCAGAGTAGTCAACACG | 784 |
| 58 | SSPE_BA_115_137_F | CAAGCAAACGCACAATCAGAAGC | 45 | SSPE_BA_197_222_R | TGCACGTCTGTTTCAGTTGCAAATTC | 1201 |
| 59 | TUFB_EC_239_259_F | TAGACTGCCCAGGACACGCTG | 204 | TUFB_EC_283_303_R | GCCGTCCATCTGAGCAGCACC | 815 |
| 60 | TUFB_EC_239_259_2_F | TTGACTGCCCAGGTCACGCTG | 678 | TUFB_EC_283_303_2_R | GCCGTCCATTTGAGCAGCACC | 816 |
| 61 | TUFB_EC_976_1000_F | AACTACCGTCCGCAGTTCTACTTCC | 4 | TUFB_EC_1045_1068_R | GTTGTCGCCAGGCATAACCATTTC | 845 |
| 62 | TUFB_EC_976_1000_2_ F | AACTACCGTCCTCAGTTCTACTTCC | 5 | TUFB_EC_1045_1068_2_R | GTTGTCACCAGGCATTACCATTTC | 844 |
| 63 | TUFB_EC 985_1012_F | | 56 | TUFB_EC_1033_1062_R | | 1006 |
| 66 | RPLB_EC_650_679_F | | 98 | RPLB_EC_739 762 R | TCCAAGTGCTGGTTTACCCCATGG | 999 |
| 67 | RPLB_EC_688_710_F | CATCCACACGGTGGTGGTGAAGG | 54 | RPLB_EC_736_757_R | GTGCTGGTTTACCCCATGGAGT | 842 |
| 68 | RPOC_EC_1036_1060_F | CGTGTTGACTATTCGGGGCGTTCAG | 78 | RPOC_EC_1097_1126_R | | 754 |
| 69 | RPOB_EC_3762_3790_F | | 248 | RPOB_EC_3836_3865_R | | 1435 |
| 70 | RPLB_EC_688_710_F | CATCCACACGGTGGTGGTGAAGG | 54 | RPLB_EC_743_771_R | | 1356 |
| 71 | VALS_EC_1105_1124_F | CGTGGCGGCGTGGTTATCGA | 77 | VALS_EC_1195_1218_R | CGGTACGAACTGGATGTCGCCGTT | 795 |
| 72 | RPOB_EC_1845_1866_F | TATCGCTCAGGCGAACTCCAAC | 233 | RPOB_EC_1909_1929_R | GCTGGATTCGCCTTTGCTACG | 825 |
| 73 | RPLB_EC_669_698_F | | 623 | RPLB_EC_735_761_R | CCAAGTGCTGGTTTACCCCATGGAGTA | 767 |
| 74 | RPLB_EC_671_700_F | | 169 | RPLB_EC_737_762_R | TCCAAGTGCTGGTTTACCCCATGGAG | 1000 |
| 75 | SP101_SPET11_1_29_F | | 2 | SP101_SPET11_92_116_R | CCTACCCAACGTTCACCAAGGGCAG | 779 |
| 76 | SP101_SPET11_118_14_ 7_F | | 115 | SP101_SPET11_213_238_R | TGTGGCCGATTTCACCACCTGCTCCT | 1340 |
| 77 | SP101_SPET11_216_24 3_F | | 24 | SP101_SPET11_308_333_R | TGCCACTTTGACAACTCCTGTTGCTG | 1209 |
| 78 | SP101_SPET11_266_29 5_F | | 89 | SP101_SPET11_355_380_R | GCTGCTTTGATGGCTGAATCCCCTTC | 824 |
| 79 | SP101_SPET11_322_34 4_F | GTCAAAGTGGCACGTTTACTGGC | 132 | SP101_SPET11_423_441_R | ATCCCCTGCTTCTGCTGCC | 753 |
| 80 | SP101_SPET11_358_38 7_F | | 126 | SP101_SPET11_448_473_R | CCAACCTTTTCCACAACAGAATCAGC | 766 |
| 81 | SP101_SPET11_600_62 9_F | | 62 | SP101_SPET11_686_714_R | | 772 |
| 82 | SP101_SPET11_658_68 4_F | | 127 | SP101_SPET11_756_784_R | | 813 |
| 83 | SP101_SPET11_776_80 1_F | TCGCCAATCAAAACTAAGGGAATGGC | 364 | SP101_SPET11_871_896_R | GCCCACCAGAAAGACTAGCAGGATAA | 814 |
| 84 | SP101_SPET11_893_92 1_F | | 123 | SP101_SPET11_988_1012_R | CATGACAGCCAAGACCTCACCCACC | 763 |
| 85 | SP101_SPET11_1154_1 179_F | CAATACCGCAACAGCGGTGGCTTGGG | 47 | SP101_SPET11_1251_1277_R | GACCCCAACCTGGCCTTTTGTCGTTGA | 804 |
| 86 | SP101_SPET11_1314_1 336_F | CGCAAAAAAATCCAGCTATTAGC | 68 | SP101_SPET11_1403_1431_R | | 711 |
| 87 | SP101_SPET11_1408_1 437_F | | 67 | SP101_SPET11_1486_1515_R | | 828 |
| 88 | SP101_SPET11_1688_1 716_F | | 60 | SP101_SPET11_1783_1808_R | ATATGATTATCATTGAACTGCGGCCG | 752 |
| 89 | SP101_SPET11_1711_1 733_F | CTGGCTAAAACTTTGGCAACGGT | 82 | SP101_SPET11_1808_1835_R | GCGTGACGACCTTCTTGAATTGTAATCA | 821 |
| 90 | SP101_SPET11_1807_1 835_F | | 33 | SP101_SPET11_1901_1927_R | TTGGACCTGTAATCAGCTGAATACTGG | 1412 |
| 91 | SP101_SPET11_1967_1 991_F | TAACGGTTATCATGGCCCAGATGGG | 155 | SP101_SPET11_2062_2083_R | ATTGCCCAGAAATCAAATCATC | 755 |
| 92 | SP101_SPET11_2260_2 283_F | CAGAGACCGTTTTATCCTATCAGC | 50 | SP101_SPET11_2375_2397_R | TCTGGGTGACCTGGTGTTTTAGA | 1131 |
| 93 | SP101_SPET11_2375_2 399_F | TCTAAAACACCAGGTCACCCAGAAG | 390 | SP101_SPET11_2470_2497_R | AGCTGCTAGATGAGCTTCTGCCATGGCC | 747 |
| 94 | SP101_SPET11_2469_2 487_F | ATGGCCATGGCAGAAGCTCA | 35 | SP101_SPET11_2543_2570_R | CCATAAGGTCACCGTCACCATTCAAAGC | 770 |
| 95 | SP101_SPET11_2961_2 984_F | ACCATGACAGAAGGCATTTTGACA | 15 | SP101_SPET11_3023_3045_R | GGAATTTACCAGCGATAGACACC | 827 |
| 96 | SP101_SPET11_3075_3 103_F | | 108 | SP101_SPET11_3168_3196_R | | 715 |
| 97 | SP101_SPET11_3386_3 403_F | AGCGTAAAGGTGAACCTT | 25 | SP101_SPET11_3480_3506_R | CCAGCAGTTACTGTCCCCTCATCTTTG | 769 |
| 98 | SP101_SPET11_3511_3 535_F | GCTTCAGGAATCAATGATGGAGCAG | 116 | SP101_SPET11_3605_3629_R | GGGTCTACACCTGCACTTGCATAAC | 832 |
| 111 | RPOB_EC_3775_3803_F | | 87 | RPOB_EC_3829_3858_R | | 797 |
| 112 | VALS_EC 1833_1850_F | CGACGCGCTGCGCTTCAC | 65 | VALS_EC_1920_1943_R | GCGTTCCACAGCTTGTTGCAGAAG | 822 |
| 113 | RPOB_EC_1336_1353_F | GACCACCTCGGCAACCGT | 97 | RPOB_EC_1438_1455_R | TTCGCTCTCGGCCTGGCC | 1386 |
| 114 | TUFB_EC_225_251_F | | 111 | TUFB_EC_284_309_R | TATAGCACCATCCATCTGAGCGGCAC | 930 |
| 115 | DNAK_EC_428_449_F | CGGCGTACTTCAACGACAGCCA | 72 | DNAK_EC_503_522_R | CGCGGTCGGCTCGTTGATGA | 792 |
| 116 | VALS_EC_1920_1943_F | CTTCTGCAACAAGCTGTGGAACGC | 85 | VALS_EC_1948_1970_R | TCGCAGTTCATCAGCACGAAGCG | 1075 |
| 117 | TUFB_EC_757_774_F | AAGACGACCTGCACGGGC | 6 | TUFB_EC_849_867_R | GCGCTCCACGTCTTCACGC | 819 |
| 118 | 23S_EC_2646_2667_F | CTGTTCTTAGTACGAGAGGACC | 84 | 23S_EC_2745_2765_R | TTCGTGCTTAGATGCTTTCAG | 1389 |
| 119 | 16S_EC_969 985_1P_F | ACGCGAAGAACCTTACpC | 19 | 16S_EC_1061_1078_2P_R | ACGACACGAGCpTpGACGAC | 733 |
| 120 | 16S_EC_972 985_2P_F | CGAAGAACpCpTTACC | 63 | 16S_EC_1064 1075_2P_R | ACACGAGCpTpGAC | 727 |
| 121 | 16S_EC_972_985_F | CGAAGAACCTTACC | 63 | 16S_EC_10641075_R | ACACGAGCTGAC | 727 |
| 122 | TRNA_ILE_ RRNH_EC 32 50.2_F | CCTGATAAGGGTGAGGTCG | 61 | 23S_EC_40_59_R | ACGTCCTTCATCGCCTCTGA | 740 |
| 123 | 23S_EC_-7_15_F | GTTGTGAGGTTAAGCGACTAAG | 140 | 23S_EC_430_450_R | CTATCGGTCAGTCAGGAGTAT | 799 |
| 124 | 23S_EC_-7_15_F | GTTGTGAGGTTAAGCGACTAAG | 141 | 21S_EC_891_910_R | TTGCATCGGGTTGGTAAGTC | 1403 |
| 125 | 23S_EC_430_450_F | ATACTCCTGACTGACCGATAG | 30 | 23S_EC_1424 1442_R | AACATAGCCTTCTCCGTCC | 712 |
| 126 | 23S_EC_891_910_F | GACTTACCAACCCGATGCAA | 100 | 23S_EC_1908_1931_R | TACCTTAGGACCGTTATAGTTACG | 893 |
| 127 | 23S_EC_1424_1442_F | GGACGGAGAAGGCTATGTT | 117 | 23S_EC_2475_2494_R | CCAAACACCGCCGTCGATAT | 765 |
| 128 | 23S_EC_1908_1931_F | CGTAACTATAACGGTCCTAAGGTA | 73 | 23S_EC_2833_2852_R | GCTTACACACCCGGCCTATC | 826 |
| 129 | 23S_EC_2475_2494_F | ATATCGACGGCGGTGTTTGG | 31 | TRNA_ASP-RRNH_EC_23_41.2_R | GCGTGACAGGCAGGTATTC | 820 |
| 131 | 16S_EC_-60_-39_F | AGTCTCAAGAGTGAACACGTAA | 28 | 16S_EC_508_525_R | GCTGCTGGCACGGAGTTA | 823 |
| 132 | 16S_EC_326_345_F | GACACGGTCCAGACTCCTAC | 95 | 16S_EC_1041_1058_R | CCATGCAGCACCTGTCTC | 771 |
| 133 | 16S_EC_705_724_F | GATCTGGAGGAATACCGGTG | 107 | 16S_EC_1493_1512_R | ACGGTTACCTTGTTACGACT | 739 |
| 134 | 16S_EC_1268_1287_F | GAGAGCAAGCGGACCTCATA | 101 | TRNA_ALA-RRNH_EC_30_46.2_R | CCTCCTGCGTGCAAAGC | 780 |
| 135 | 16S_EC_969_985_F | ACGCGAAGAACTTACC | 19 | 16S_EC_1061_1078.2_R | ACAACACGAGCTGACGAC | 719 |
| 137 | 16S_EC_969_985_F | ACGCGAAGAACCTTACC | 19 | 16S_EC_1061_1078.2_I14_R | ACAACACGAGCTGICGAC | 721 |
| 138 | 16S_EC_969_985_F | ACGCGAZQAARCTTACC | 19 | 16S_EC_1061_1078.2_I12_R | ACAACACGAGCIGACGAC | 718 |
| 139 | 16S_EC_969_985_F | ACGCGAACCTTCC | 19 | 16S_EC_1061_1078.2_I11_R | ACAACACGAGITGACGAC | 722 |
| 140 | 16S_EC_969_985_F | ACGCAAGAATTACC | 19 | 16S_EC_1061_1078.2_I16_R | ACAACACGAGCTGACIAC | 720 |
| 141 | 16S_EC_969_985_F | ACGCGAAGGCCTTACC | 19 | 16S_EC_1061_1078.2_2I_R | ACAACACGAICTIACGAC | 723 |
| 142 | 16S_EC_969_985_F | ACGCGAAGAACCTTACC | 19 | 16S_EC_1061_1078.2_3I_R | ACAACACIAATATCTIACGAC | 724 |
| 143 | 16S_EC_969_985_F | ACGCGAAGAACCTTACC | 19 | 16S_EC_1061_1078.2_4I_R | ACAACACIAICTIACIAC | 725 |
| 147 | 23S_EC_2652_2669_F | CTAGTACGAGAGGACCGG | 79 | 23S_EC_2741_2760_R | ACTTAGATGCTTTCAGCGGT | 743 |
| 158 | 16S_EC_683_700_F | GTGTAGCGGTGAAATGCG | 137 | 16S_EC_880_894_R | CGTACTCCCCAGGCG | 796 |
| 159 | 16S_EC_1100_1116_F | CAACGAGCGCAACCCTT | 42 | 16S_EC_1174_1188_R | TCCCCACCTTCCTCC | 1019 |
| 215 | SSPE_BA_121_137 F | AACGCACAATCAGAAGC | 3 | SSPE_BA_197_216_R | TCTGTTTCAGTTGCAAATTC | 1132 |
| 220 | GROL_EC_941_959_F | TGGAAGATCTGGGTCAGGC | 544 | GROL_EC_1039_1060_R | CAATCTGCTGACGGATCTGAGC | 759 |
| 221 | INFB_EC_1103_1124_F | GTCGTGAAAACGAGCTGGAAGA | 133 | INFB_EC_1174_1191_R | CATGATGGTCACAACCGG | 764 |
| 222 | HFLB_EC_1082_1102_F | TGGCGAACCTGGTGAACGAAGC | 569 | HFLB_EC_1144_1168_R | CTTTCGCTTTCTCGAACTCAACCAT | 802 |
| 223 | INFB_EC_1969_1994_ F | CGTCAGGGTAAATTCCGTGAAGTTAA | 74 | INFB_EC_2038_2058_R | AACTTCGCCTTCGGTCATGTT | 713 |
| 224 | GROL_EC_219_242_F | GGTGAAAGAAGTTGCCTCTAAAGC | 128 | GROL_EC_328_350_R | TTCAGGTCCATCGGGTTCATGCC | 1377 |
| 225 | VALS_EC_1105_1124_F | CGTGGCGGCGTGGTTATCGA | 77 | VALS_EC_1195_1214_R | ACGAACTGGATGTCGCCGTT | 732 |
| 226 | 16S_EC_556_575_F | CGGAATTACTGGGCGTAAAG | 70 | 16S_EC_683_700_R | CGCATTTCACCGCTACAC | 791 |
| 227 | RPOC_EC_1256_1277_F | ACCCAGTGCTGCTGAACCGTGC | 16 | RPOC_EC_1295_1315_R | GTTCAAATGCCTGGATACCCA | 843 |
| 228 | 16S_EC_774_795_F | GGGAGCAAACAGGATTAGATAC | 122 | 16S_EC_880_894_R | CGTACTCCCCAGGCG | 796 |
| 229 | RPOC_EC_1584_1604_F | TGGCCCGAAAGAAGCTGAGCG | 567 | RPOC_EC_1623_1643_R | ACGCGGGCATGCAGAGATGCC | 737 |
| 230 | 16S_EC_1082_1100_F | ATGTTGGGTTAAGTCCCGC | 37 | 16S_EC_1177 1196_R | TGACGTCATCCCCACCTTCC | 1158 |
| 231 | 16S_EC_1389_1407_F | CTTCTACACCGCCCGTC | 88 | 16S_EC_1525 1541_R | AAGGAGGTGATCCAGCC | 714 |
| 232 | 16S_EC_1303_1323_F | CGGATTGGAGTCTGCAACTCG | 71 | 16S_EC_1389_1407_R | GACGGGCGGTGTGTACAAG | 808 |
| 233 | 23S_EC_23_37_F | GGTGGATGCCTTGGC | 129 | 23S_EC_115_130_R | GGGTTTCCCCATTCGG | 833 |
| 234 | 23S_EC_187_207_F | GGGAACTGAAACATCTAAGTA | 121 | 23S_EC_242_256_R | TTCGCTCGCCGCTAC | 1385 |
| 235 | 23S_EC_1602_1620_F | TACCCCAAACCGACACAGG | 184 | 23S_EC_1686_1703_R | CCTTCTCCCGAAGTTACG | 782 |
| 236 | 23S_EC_1685_1703_F | CCGTAACTTCGGGAGAAGG | 58 | 23S_EC1828_1842_R | CACCGGGCAGGCGTC | 760 |
| 237 | 23S_EC_1827_1843_F | GACGCCTGCCCGGTGC | 99 | 23S_EC_1929_1949_R | CCGACAAGGAATTTCGCTACC | 775 |
| 238 | 23S_EC_2434_2456_F | AAGGTACTCCGGGGATAACAGGC | 9 | 23S_EC_2490_2511_R | AGCCGACATCGAGGTGCCAAAC | 746 |
| 239 | 23S_EC_2599_2616_F | GACAGTTCGGTCCCTATC | 96 | 23S_EC_2653 2669_R | CCGGTCCTCTCGTACTA | 777 |
| 240 | 23S_EC_2653_2669_F | TAGTACGAGAGGACCGG | 227 | 23S_EC_2737_2758_R | TTAGATGCTTTCAGCACTTATC | 1369 |
| 241 | 23S_BS_-68_-44_F | AAACTAGATAACAGTAGACATCAC | 1 | 23S_BS_5_21_R | GTGCGCCCTTTCTAACTT | 841 |
| 242 | 16S_EC_8_27_F | AGAGTTTGATCATGGCTCAG | 23 | 16S_EC_342_358_R | ACTGCTGCCTCCCGTAG | 742 |
| 243 | 16S_EC_314_332_F | CACTGGAACTGAGACACGG | 48 | 16S_EC_556_575_R | CTTTACGCCCAGTAATTCCG | 801 |
| 244 | 16S_EC_518_536_F | CCAGCAGCCGCGGTAATAC | 57 | 16S_EC_774_795_R | GTATCTAATCCTGTTTGCTCCC | 839 |
| 245 | 16S_EC_683_700_F | GTGTAGCGGTGAAATGCG | 137 | 16S_EC_967_985_R | GGTAAGGTTCTTCGCGTTG | 835 |
| 246 | 16S_EC_937_954_F | AAGCGGTGGAGCATGTGG | 7 | 16S_EC_1220_1240_R | ATTGTAGCACGTGTGTAGCCC | 757 |
| 247 | 16S_EC_1195_1213_F | CAAGTCATCATGGCCCTTA | 46 | 16S_EC_1525_1541_R | AAGGAGGTGATCCAGCC | 714 |
| 248 | 16S_ETC_8_27_F | AGAGTTTGATCATGGCTCAG | 23 | 16S_EC_1525_1541_R | AAGGAGGTGATCCAGCC | 714 |
| 249 | 23S_EC_1831_1849_F | ACCTGCCCAGTGCTGGAAG | 18 | 23S_EC_1919_1936_R | TCGCTACCTTAGGACCGT | 1080 |
| 250 | 16S_EC_1387_1407_F | GCCTTGTACACACCTCCCGTC | 112 | 16S_EC_1494_1513_R | CACGGCTACCTTGTTACGAC | 761 |
| 251 | 16S_EC_1390_1411_F | TTGTACACACCGCCCGTCATAC | 693 | 16S_EC_1486_1505_R | CCTTGTTACGACTTCACCCC | 783 |
| 252 | 16S_EC_1367_1387_F | TACGGTGGAATACGTTCCCGGG | 191 | 16S_EC_1485_1506_R | ACCTTGTTACGACTTCACCCCA | 731 |
| 253 | 16S_EC_804_822_F | ACCACGCCGTAAACGATGA | 14 | 16S_EC_909_929_R | CCCCCGTCAATTCCTTTGAGT | 773 |
| 254 | 16S_EC_791_812_F | GATACCCTGGTAGTCCACACCG | 106 | 16S_EC_886_904_R | GCCTTGCGACCGTACTCCC | 817 |
| 255 | 16S_EC_789_810_F | TAGATACCCTGGTAGTCCACGC | 206 | 16S_EC_882_899_R | GCGACCGTACTCCCCAGG | 818 |
| 256 | 16S_EC_1092_1109_F | TAGTCCCGCAACGAGCGC | 228 | 16S_EC_1174_1195_R | GACGTCATCCCCACCTTCCTCC | 810 |
| 257 | 23S_EC_2586_2607_F | TAGAACGTCGCGAGACAGTTCG | 203 | 23S_EC_2658_2677_R | AGTCCATCCCGGTCCTCTCG | 749 |
| 258 | RNASEP_SA_31_49_F | GAGGAAAGTCCATGCTCAC | 103 | RNASEP_SA_358_379_R | ATAAGCCATGTTCTGTTCCATC | 750 |
| 258 | RNASEP_SA_31_49_F | GAGGAAAGTCCATGCTCAC | 103 | RNASEP_EC_345_362_R | ATAAGCCGGGTTCTGTCG | 751 |
| 258 | RNASEP_SA_31_49_F | GAGGAAAGTCCATGCTCAC | 103 | RNASEP_BS_363_384_R | GTAAGCCATGTTTTGTTCCATC | 838 |
| 258 | RNASEP_BS_43_61_F | GAGGAAAGTCCATGCTCGC | 104 | RNASEP_SA_358_379_R | ATAAGCCATGTTCTGTTCCATC | 750 |
| 258 | RNASEP_BS_43_61_F | GAGGAAAGTCCATGCTCGC | 104 | RNASEP_EC_345_362_R | ATAAGCCGGGTTCTGTCG | 751 |
| 258 | RNASEP_BS_43_61_F | GAGGAAAGTCCATGCTCGC | 104 | RNASEP_BS 363_384_R | GTAAGCCATGTTTTGTTCCATC | 838 |
| 258 | RNASEP_EC_61_77_F | GAGGAAAGTCCGGGCTC | 105 | RNASEP_SA 358_379_R | ATAAGCCATGTTCTGTTCCATC | 750 |
| 258 | RNASEP_EC_61_77_F | GAGGAAAGTCCGGGCTC | 105 | RNASEP_EC_345 362 R | ATAAGCCGGGTTCTGTCG | 751 |
| 258 | RNASEP_EC_61_77_F | GAGGAAAGTCCGGGCTC | 105 | RNASEP_BS_363_384_R | GTAAGCCATGTTTTGTTCCATC | 838 |
| 259 | RNASEP_BS_43_61_F | GAGGAAAGTCCATGCTCGC | 104 | RNASEP_BS_363_384_R | GTAAGCCATGTTTTGTTCCATC | 838 |
| 260 | RNASEP_EC_61_77_F | GAGGAAAGTCCGGGCTC | 105 | RNASEP EC 345 362 R | ATAAGCCGGGTTCTGTCG | 751 |
| 262 | RNASEPSA_31_49_F | GAGGAAAGTCCATGCTCAC | 103 | RNASEP SA 358 179 R | ATAAGCCATGTTCTGTTCCATC | 750 |
| 263 | 16S_EC_1082_1100_F | ATGTTGGGTTAAGTCCCGC | 37 | 16S EC 1525 1541 R | AAGGAGGTGATCCAGCC | 714 |
| 264 | 16S_EC_556_575_F | CGGAATTACTGGGCGTAAAG | 70 | 16S EC 774 795 R | GTATCTAATCCTGTTTGCTCCC | 839 |
| 265 | 16S_EC_1082_1100_F | ATGTTGGGTTAAGTCCCGC | 37 | 16S EC 1177 1196 10G R | TGACGTCATGCCCACCTTCC | 1160 |
| 266 | 16S_EC_1082_1100_F | ATGTTGGGTTAAGTCCCGC | 37 | 168_EC_1177_1196_10G_11G_ R | TGACGTCATGGCCACCTTCC | 1161 |
| 268 | YAED_EC_513_532_F_M OD | GGTGTTAAATAGCCTGGCAG | 130 | TRNA_ALA-RRNH_EC_30_49_F_MOD | AGACCTCCTGCGTGCAAAGC | 744 |
| 269 | 16S_EC_1082_1100_F_ MOD | ATGTTGGGTTAAGTCCCGC | 37 | 16S_EC_1177_1196_R_MOD | TGACGTCATCCCCACCTTCC | 1158 |
| 270 | 23S_EC_2586_2607_F_ MOD | TAGAACGTCGCGAGACAGTTCG | 203 | 23S_EC_2658_2677_R_MOD | AGTCCATCCCGGTCCTCTCG | 749 |
| 272 | 16S_EC_969_985_F | ACGCGAAGAACCTTACC | 19 | 16S_EC_1389_1407_R | GACGGGCGGTGTGTACAAG | 807 |
| 273 | 16S_EC_683_700_F | GTGTAGCGGTGAAATGCG | 137 | 16S_EC_1303_1323_R | CGAGTTGCAGACTGCGATCCG | 788 |
| 274 | 16S_EC_49_68_F | TAACACATGCAAGTCGAACG | 152 | 16S_EC_880_894_R | CGTACTCCCCAGGCG | 796 |
| 275 | 16S_EC_49_68_F | TAACACATGCAAGTCGAACG | 152 | 16S_EC_1061_1078_R | ACGACACGAGCTGACGAC | 734 |
| 277 | CYA_BA_1349_1370_F | ACAACGAAGTACAATACAAGAC | 12 | CYA_BA_1426_1447_R | CTTCTACATTTTTAGCCATCAC | 800 |
| 278 | 16S_EC_1090_1111_2_ F | TTAAGTCCCGCAACGAGCGCAA | 650 | 16S_EC_1175_1196_R | TGACGTCATCCCCACCTTCCTC | 1159 |
| 279 | 16S_EC_405_432_F | | 464 | 16S_EC_507_527_R | CGGCTGCTGGCACGAAGTTAG | 793 |
| 280 | GROL_EC_496_518_F | ATGGACAAGGTTGGCAAGGAAGG | 34 | GROL_EC_577_596_R | TAGCCGCGGTCGAATTGCAT | 914 |
| 281 | GROL_EC_511_536_F | AAGGAAGGCGTGATCACCGTTGAAGA | 8 | GROL_EC_571_593_R | CCGCGGTCGAATTGCATGCCTTC | 776 |
| 288 | RPOB_EC_3802_3821_F | CAGCGTTTCGGCGAAATGGA | 51 | RPOB_EC_3862_3885_R | CGACTTGACGGTTAACATTTCCTG | 786 |
| 289 | RPOB_EC_3799_3821_F | GGGCAGCGTTTCGGCGAAATGGA | 124 | RPOB_EC_3862_3888_R | GTCCGACTTGACGGTCAACATTTCCTG | 840 |
| 290 | RPOC_EC_2146_2174_F | | 52 | RPOC_EC_2227_2245_R | ACGCCATCAGGCCACGCAT | 736 |
| 291 | ASPS_EC_405_422_F | GCACAACCTGCGGCTGCG | 110 | ASPS_EC_521_538_R | ACGGCACGAGGTAGTCGC | 738 |
| 292 | RPOC_EC_1374_1393_F | CGCCGACTTCGACGGTGACC | 69 | RPOC_EC_1437_1455_R | GAGCATCAGCGTGCGTGCT | 811 |
| 293 | TUFB_EC_957_979_F | CCACACGCCGTTCTTCAAr_{AA}r_{T} | 55 | TUFB_EC_1034_1058_R | GGCATCACCATTTCCTTGTCCTTCG | 829 |
| 294 | 16S_EC_7_33_F | | 102 | 16S_EC_101_122_R | TGTTACTCACCCGTCTGCCACT | 1345 |
| 295 | VALS_EC_610_649_F | ACCGAGCAAGGAGACCAGC | 17 | VALS_EC_705_727_R | TATAACGCACATCGTCAGGGTGA | 929 |
| 344 | 16S_EC_971_990_F | GCGAAGAACCTTACCAGGTC | 113 | 16S_EC_1043_1062_R | ACAACCATGCACCACCTGTC | 726 |
| 346 | 16S_EC_13_732_TMOD_ F | TAGAACACCGATGGCGAAGGC | 202 | 16S_EC_789_809_TMOD_R | TCGTGGACTACCAGGGTATCTA | 1110 |
| 347 | 16S_EC_786 806_TMOD F | TGGATTAGAGACCCTGGTAGTCC | 560 | 16S_EC_880_897_TMOD_R | TGGCCGTACTCCCCAGGCG | 1278 |
| 348 | 16S_EC_960_981_TMOD | TTTCGATGCAACGCGAAGAACCT | 706 | 16S_EC_1054_1073_TMOD_R | TACGAGCTGACGACAGCCATG | 895 |
| | _F | | | | | |
| 349 | 23S_EC_1826_1843_TM OD_F | TCTGACACCTGCCCGGTGC | 401 | 23S_EC_1906_1924_TMOD_R | TGACCGTTATAGTTACGGCC | 1156 |
| 350 | CAPC_BA_274_303_TMO D_F | | 476 | CAPC_BA_349_376_TMOD_R | | 1314 |
| 351 | CYA_BA_1353_1379_TM OD_F | | 355 | CYA_BA_1448_1467_TMOD_R | TTGTTAACGGCTTCAAGACCC | 1423 |
| 352 | INFB_EC_1365_1393_T MOD_F | | 687 | INFB_EC_1439_1467_TMOD_R | | 1411 |
| 353 | LEF_BA_756 781_TMOD_ F | | 220 | LEF_BA_843 872_TMOD_R | | 1394 |
| 354 | RPOC_EC_2218_2241_T MOD_F | TCTGGCAGGTATGCGTGGTCTGATG | 405 | RPOC_EC_2313_2337_TMOD_R | TCGCACCGTGGGTTGAGATGAAGTAC | 1072 |
| 355 | SSPE_BA_115_137_TMO D_F | TCAAGCAAACGCACAATCAGAAGC | 255 | SSPE_BA_197_222_TMOD_R | TTGCACGTCTGTTTCAGTTGCAAATTC | 1402 |
| 356 | RPLB_EC_650_679_TMO D_F | | 449 | RPLB_EC_739_762_TMOD_R | TTCCAAGTGCTGGTTTACCCCATGG | 1380 |
| 357 | RPLB_EC_688_710_TMO D_F | TCATCCACACGGTGGTGGTGAAGG | 296 | RPLB_EC_736_757_TMOD_R | TGTGCTGGTTTACCCCATGGAGT | 1337 |
| 358 | VALS_EC_1105_1124_T MOD_F | TCGTGGCGGCGTGGTTATCGA | 385 | VALS_EC_1195_1218_TMOD_R | TCGGTACGAACTGGATGTCGCCGTT | 1093 |
| 359 | RPOB_EC_1845_1866_T MOD_F | TTATCGCTCAGGCGAACTCCAAC | 659 | RPOB_EC_1909_1929_TMOD_R | TGCTGGATTCGCCTTTGCTACG | 1250 |
| 360 | 235_EC_2646_2667_TM OD_F | TCTCGTTCTTAGTACGAGAGGACC AGTACGAGAGGACC | 409 | 23S_EC_2745 2765_TMOD_R | TTTCGTGCTTAGATGCTTTCAG | 1434 |
| 361 | 16S_EC_1090_1111_2_ TMOD_F | TTTAAGTCCCGCAACGAGCGCAA | 697 | 16S_EC_1175_1196_TMOD_R | TTGACGTCATCCCCACCTTCCTC | 1398 |
| 362 | RPOB_EC_3799_3821_T MOD_F | TGGGCAGCGTTTCGGCGAAATGGA | 581 | RPOB_EC_3862_3888_TMOD_R | TGTCCGACTTGACGGTCAACATTTCCTG | 1325 |
| 363 | RPOC_EC_2146_2174_T MOD_F | | 284 | RPOC_EC_2227_2245 TMOD_R | TACGCCATCAGGCCACGCAT | 898 |
| 364 | RPOC_EC_1374_1393_T MOD_F | TCGCCGACTTCGACGGTGACC | 367 | RPOC_EC_1437_1455_TMOD_R | TGAGCATCAGCGTGCGTGCT | 1166 |
| 367 | TUFB_EC_957_979_TMO D_F | TCCACACGCCGTTCAACAACT | 308 | TUFB_EC_1034_1058_TMOD_R | TGGCATCACCATTTCCTTGTCCTTCG | 1276 |
| 423 | SP101_SPET11_893_92 1_TMOD_F | | 580 | SP101_SPET11_988_1012_TMO D_R | TCATGACAGCCAAGACCTCACCCACC | 990 |
| 424 | SP101_SPET11_1154_1 179_TMOD_F | | 258 | SP101_SPETII_1251_1277_TM OD_R | TGACCCCAACCTGGCCTTTTGTCGTTGA | 1155 |
| 425 | SP101_SPET11_118_14_ 7_TMOD_F | | 528 | SP101_SPETII_213_238_TMOD_ R | TTGTGGCCGATTTCACCACCTGCTCCT | 1422 |
| 426 | SP101_SPET11_1314_1 336_TMOD_F | TCGCAAAAAAATCCAGCTATTAGC | 363 | SP101_SPET11_1403_1431_TM OD_R | | 849 |
| 427 | SP101_SPET11_1408_1 437_TMOD_F | | 359 | SP101_SPET11_1486_1515_TM OD_R | | 1268 |
| 428 | SP101_SPET11_1688_1 716_TMOD_F | | 334 | SP101_SPET11_1783_1808_TM OD_R | TATATGATTATCATTGAACTGCGGCCG | 932 |
| 429 | SP101_SPET11_1711_1 733_TMOD_F | TCTGGCTAAAACTTTGGCAACGGT | 406 | SP101_SPET11_1808_1835_TM OD_R | | 1239 |
| 430 | SP101_SPET11_1807_1 835_TMOD_F | | 235 | SP101_SPET11_1901_1927_TM OD_R | TTTGGACCTGTAATCAGCTGAATACTGG | 1439 |
| 431 | SP101_SPET11_1967_1 991_TMOD_F | TTAACGGTTATCATGGCCCAGATGGG | 649 | SP101_SPET11_2062_2083_TM OD_R | TATTGCCCAGAAATCAAATCATC | 940 |
| 432 | SP101_SPET11_216_24 3_TMOD_F | | 210 | SP101_SPET11_ 308_333_TMOD_ R | TTGCCACTTTGACAACTCCTGTTGCTG | 1404 |
| 433 | SP101_SPET11_2260_2 283_TMOD F | TCAGAGACCGTTTTATCCTATCAGC | 272 | SPI01_SPET11_2375_2397_TM OD_R | TTCTGGGTGACCTGGTGTTTTAGA | 1393 |
| 434 | SP101_SPET11_2375_2 399_TMOD_F | TTCTAAAACACCAGGTCACCCAGAAG | 675 | SPI01_SPET11_2470_2497_TM OD_R | | 918 |
| 435 | SP101_SPET11_2468_2 487_TMOD_F | TATGGCCATGGCAGAAGCTCA | 238 | SP101_SPET11_2543_2570_TM OD_R | | 1007 |
| 436 | SP101_SPET11_266_29 5_TMOD_F | | 417 | SP101_SPET11_355_380_TMOD_ R | TGCTGCTTTGATGGCTGAATCCCCTTC | 1249 |
| 437 | SP101_SPET11_2961_2 984_TMOD_F | TACCATGACAGAAGGCATTTTGACA | 183 | SP101_SPET11_3023_3045_TM OD_R | TGGAATTTACCAGCGATAGACACC | 1264 |
| 438 | SP101_SPET11_3075_3 103_TMOD_F | | 473 | SP101_SPET11_3168_3196_TM OD_R | | 875 |
| 439 | SP101_SPET11_322_34 4_TMOD_F | TGTCAAAGTGGCACGTTTACTGGC | 631 | SPI01_SPET11_423_441_TMOD_ R | TATCCCCTGCTTCTGCTGCC | 934 |
| 440 | SP101_SPET11_3386_3 403_TMOD_F | TAGCGTAAAGGTGAACCTT | 215 | SP101_SPET11_3480_3506_TM OD R | TCCAGCAGTTACTGTCCCCTCATCTTTG | 1005 |
| 441 | SP101_SPET11_3511_3 535_TMOD_F | TGCTTCAGGAATCAATGATGGAGCAG | 531 | SP101_SPETII_3605_3629_TM OD R | TGGGTCTACACCTGCACTTGCATAAC | 1294 |
| 442 | SP101_SPET11_358_38 7_TMOD_F | | 588 | SP101_SPET11_448_473_TMOD R | TCCAACCTTTTCCACAACAGAATCGC | 998 |
| 443 | SP101_SPET11_600_62 9_TMOD_F | | 348 | SP101_SPET11_686_714_TMOD R | | 1018 |
| 444 | SP101_SPET11_658_68 4_TMOD_F | | 589 | SP101_SPET11_756_784_TMOD R | | 1189 |
| 445 | SP101_SPET11_776_80 1_TMOD_F | | 673 | SP101_SPET11_871_896_TMOD R | TGCCCACCAGAAAGACTAGCAGGATAA | 1217 |
| 446 | SP101_SPET11_1_29_T MOD_F | | 154 | SP101_SPET11_92_11G_TMOD_ R | TCCTACCCAACGTTCACCAAGGGCAG | 1044 |
| 447 | SP101_SPET11_364_38 5_F | TCAGCCATCAAAGCAGCTATTG | 276 | SP101_SPET11 448_471_R | TACCTTTTCCAATCAGC | 894 |
| 448 | SP101_SPET11_3085_3 104_F | TAGCTAATGGTCAGGCAGCC | 216 | SP101_SPET11 3170_3194_R | TCGACGACCATCTTGGAAAGATTTC | 1066 |
| 449 | RPLB_EC_690_710_F | TCCACACGGTGGTGGTGAAGG | 309 | RPLB_EC_737 758_R | TGTGCTGGTTTACCCCATGGAG | 1336 |
| 481 | BONTA_X52066_538_55 2_F | TATGGCTCTACTCAA | 239 | BONTA_X52066_647_660_R | TGTTACTGCTGGAT | 1346 |
| 482 | BONTA_X52066_538_55 2P_F | | 143 | BONTA_X52066_647_660P_R | TG*Tp*TpA*Cp*TpG*TG*TpGGAT | 1146 |
| 483 | BONTA_X52066_701_72 0_F | GAATAGCAATTAATCCAAAT | 94 | BONTA_X52066_759_775_R | TTACTTCTAACCCACTC | 1367 |
| 484 | BONTA_X52066_701_72 0P_F | | 91 | BONTA_X52066_759 775P_R | | 1359 |
| 485 | BONTA_X52066_450_47 3_F | TCTAGTAATAATAGGACCCTCAGC | 393 | BONTA_X52066_517 539 R | TAACCATTTCGCGTAAGATTCAA | 859 |
| 486 | BONTA_X52066_450_47 3P_F | | 142 | BONTA_X52066_517_539P_R | | 857 |
| 487 | BONTA_X52066_591_62 0_F | | 463 | BONTA_X52066_644_671_R | TCATGTGCTAATGTTACTGCTGGATCTG | 992 |
| 608 | SSPE_BA_156_168P_F | TGGTpGCpTpAGCpATT | 616 | SSPE_BA_243_255P_R | TGCpAGCpTGATpTpGT | 1241 |
| 609 | SSPE_BA_75_89P_F | TACpAGAGTpTpTpGCpGAC | 192 | SSPE_BA_163_177P_R | TGTGCTpTpTpGAATpGCpT | 1338 |
| 610 | SSPE_BA_150_168P_F | TGCTTCTGGTpGCpTpAGCpATT | 533 | SSPE_BA_243_264P_R | TGATTGTTTTGCpAGCpTGATpTpGT | 1191 |
| 611 | SSPE_BA_72_89P_F | TGGTACpAGAGTpTpTpGCpGAC | 602 | SSPE_BA_163_182P_R | TCATTTGTGCTpTpTpGAATpGCpT | 995 |
| 612 | SSPE_BA_114_137P_F | TCAAGCAAACGCACAATpCpAGAAGC | 255 | SSPE_BA_196_222P_R | TTGCACGTCpTpGTTTCAGTTGCAAATT C | 1401 |
| 699 | SSPE_BA_123_153_F | | 488 | SSPE_BA_202_231_R | | 1431 |
| 700 | SSPE_BA_156_168_F | TGGTGCTAGCATT | 612 | SSPE_BA_243 255_R | TGCAGCTGATTGT | 1202 |
| 701 | SSPE_BA_75_89_F | TACAGAGTTTGCGAC | 179 | SSPE_BA_163 177_R | TGTGCTTTGAATGCT | 1338 |
| 702 | SSPE_BA_150_168_F | TGCTTCTGGTGCTAGCATT | 533 | SSPE_BA_243 264_R | TGATTGTTTTGCAGCTGATTGT | 1190 |
| 703 | SSPE_BA_72_899_F | TGGTACAGAGTTTGCGAC | 600 | SSPE_BA_163_182_R | TCATTTGTGCTTTGAATGCT | 995 |
| 704 | SSPE_BA_146_168_F | TGCAAGCTTCTGGTGCTAGCATT | 484 | SSPE_BA_242 267_R | TTGTGATTGTTTTGCAGCTGATTGTG | 1421 |
| 705 | SSPE_BA_63_89_F | | 518 | SSPE_BA_163 191_R | | 986 |
| 706 | SSPE_BA_114_137_F | TCAAGCAAACGCACAATCAGAAGC | 255 | SSPE_BA_196 222_R | TTGCACGTCTGTTTCAGTTGCAAATTC | 1402 |
| 770 | PLA_AF053945_7377_7 402_F | TGACATCCGGCTCACGTTATTATGGT | 442 | PLA_AF053945 7434_7462_R | | 1313 |
| 771 | PLA_AF053945_7382_7 404_F | TCCGGCTCACGTTATTATGGTAC | 327 | PLA_AF053945 7482_7502_R | TGGTCTGAGTACCTCCTTTGC | 1304 |
| 772 | PLA_AF053945_7481_7 503_F | TGCAAAGGAGGTACTCAGACCAT | 481 | PLA_AF053945 7539_7562_R | TATTGGAAATACCGGCAGCATCTC | 943 |
| 773 | PLA_AF053945_7186_7 211_F | TTATACCGGAAACTTCCCGAAAGGAG | 657 | PLA_AF053945_7257_7280_R | TAATGCGATACTGGCCTGCAAGTC | 879 |
| 774 | CAF1_AF0S3947_33407_ 33430_F | TCAGTTCCGTTATCGCCATTGCAT | 292 | CAF1_AF053947_33494_33514_ R | TGCGGGCTGGTTCAACAAGAG | 1235 |
| 775 | CAF1_AF053947_33515_ 33541_F | | 270 | CAF1_AF053947_33595_33621_ R | TCCTGTTTTATAGCCGCCAAGAGTAAG | 1053 |
| 776 | CAF1_AF053947_33435_ 33457_F | TGGAACTATTGCAACTGCTAATG | 542 | CAF1_AF053947_33499_33517_ R | TGATGCGGGCTGGTTCAAC | 1183 |
| 777 | CAF1_AF053947_33687_ 33716_F | | 286 | CAF1_AF053947_33755_33782_ R | TCAAGGTTCTCACCGTTTACCTTAGGAG | 962 |
| 778 | INV_U22457_515_539_ F | TGGCTCCTTGGTATGACTCTGCTTC | 573 | INV_U22457_571_598_R | TGTTAAGTGTGTTGCGGCTGTCTTTATT | 1343 |
| 779 | INV_U224S7_699_724_ F | TGCTGAGGCCTGGACCGATTATTTAC | 525 | INV_U22457_753_776_R | TCACGCGACGAGTGCCATCCATTG | 976 |
| 780 | INV_U22457_834_858_ F | TTATTTACCTGCACTCCCACAACTG | 664 | INV_U22457_942_966_R | TGACCCAAAGCTGAAAGCTTTACTG | 1154 |
| 781 | INV_U22457_1558_158 1_F | TGGTAACAGAGCCTTATAGGCGCA | 597 | INV_U22457_1619_1643_R | TTGCGTTGCAGATTATCTTTACCAA | 1408 |
| 782 | LL_NC003143_2366996 2367019_F | TGTAGCCGCTAAGCACTACCATCC | 627 | LL_NC003143_2367073_23670 97_R | TCTCATCCCGATATTACCGCCATGA | 1123 |
| 783 | LL_NC003143_2367172_ 2367194_F | TGGACGGCATCACGATTCTCTAC | 550 | LL_NCO03143_2367249_23672 71_R | TGGCAACAGCTCAACACCTTTGG | 1272 |
| 874 | RPLB_EC_649_679_F | | 620 | RPLB_EC_739_762_TMOD_R | TTCCAAGTGCTGGTTTACCCCATGG | 1380 |
| 875 | RPLB_EC_642_679P_F | | 646 | RPLB_EC_739_762_TMOD_R | TTCCAAGTGCTGGTTTACCCCATGG | 1380 |
| 876 | MECIA_Y14051_3315_3 341_F | | 653 | MECIA_Y14051_3367_3393_R | TGTGATATGGAGGTGTAGAAGGTGTTA | 1333 |
| 877 | MECA_Y14051_3774_38 02_F | | 144 | MECA_Y14051_3828_3854_R | TCCCAATCTAACTTCCACATACCATCT | 1015 |
| 878 | MECA_Y14051_3645_36 70_F | TGAAGTAGAAATGACGAACGTCCGA CTGAACGTCCGA | 434 | MECA_Y14051_3690_3719_R | | 1181 |
| 879 | MECA_Y14051_4507_45 30_F | TCAGGTACTGCTATCCACCCTCAA | 288 | MECA_Y14051_4555_4581_R | TGGATAGACGTCATATGAATGTGCT | 1269 |
| 880 | MECA_Y14051_4510_45 30_F | TGTACTGCTATCCACCCTCAA | 626 | MECA_Y14051_4586_4610_R | TATTCTTCGTTACTCATGCCATACA | 939 |
| 881 | MECA_Y14051_4669_46 98_F | | 262 | MECA_Y14051_4765_4793_R | | 858 |
| 882 | MECA_Y14051_4520_45 30P_F | TCpCpACpCpCpTpCpAA | 389 | MECA_Y14051_4590_4600P_R | TpACpTpCpATpGCpCpA | 1357 |
| 883 | MECA_Y14051_4520_45 30P_F | TCpCpACpCpCpTpCpAA | 389 | MECA_Y14051_4600_4610P_R | TpRTpTpCpTpTpCpGTpT | 1358 |
| 902 | TRPE_AY094355_1467_ 1491_F | ATGTCGATTGCAATCCGTACTTGTG | 36 | TRPE_AY094355_1569_1592_R | TGCGCGAGCTTTTATTTGGGTTTC | 1231 |
| 903 | TRPE_AY094355_1445_ 1471_F | | 557 | TRPE_AY094355_1551_1580_R | | 944 |
| 904 | TRPE_AY094355_1278_ 1303_F | TCAAATGTACAAGGTGAAGTGCGTGA | 247 | TRPE_AY094355_1392_1418_R | TCCTCTTTTCACAGGCTCTACTTCATC | 1048 |
| 905 | TRPE_AY094355_1064_ 1086_F | TCGACCTTTGGCAGGAACTAGAC | 357 | TRPE_AY094355_1171_1196_R | TACATCGTTTCGCCCAAGATCAATCA | 885 |
| 906 | TRPE_AY094355_666_6 88_F | GTGCATGCGGATACAGAGCAGAG | 135 | TRPE_AY094355_769_791_R | TTCAAAATGCGGAGGCGTATGTG | 1372 |
| 907 | TRPE AY094355 757_7 | TGCAAGCGCGACCACATACG | 483 | TRPE_AY094355_864_883_R | TGCCCAGGTACAACCTGCAT | 1218 |
| | 76_F | | | | | |
| 908 | RECA_AF251469_43_68_ F | TGGTACATGTGCCTTCATTGATGCTG | 601 | RECA_AF251469_140_163_R | TTCAAGTGCTTGCTCACCATTGTC | 1375 |
| 909 | RECA_AF2514691_69_1 90_F | TGACATGCTTGTCCGTTCAGGC | 446 | RECA_AF251469_277_300_R | TGGCTCATAAGCGCTTGTAGA | 1280 |
| 910 | PARC_X95819_87_110_ F | TGGTGACTCGGCATGTTATGAAGC | 609 | PARC_X95819_201_222_R | TTCGGTATAACGCATCGCAGCA | 1387 |
| 911 | PARC_X95819 87_110_ F | TGGTGACTCGGCATGTTATGAAGC | 609 | PARC_X95819_192_219_R | GGTATAACGCATCGCAGCAAAAGATTA | 836 |
| 912 | PARC_X95819_123_147_ F | GGCTCAGCCATTTAGTTACCGCTAT | 120 | PARC_X95819_232_260_R | | 1081 |
| 913 | PARC_X95819_43_63_F | TCAGCGCGTACAGTGGGTGAT | 277 | PARC_X95819_143_170_R | TTCCCCTGACCTTCGATTAAAGGATAGC | 1383 |
| 914 | OMPA_AY485227_272_3 01_F | | 655 | OMPA_AY485227_364_388_R | GAGCTGCGCCAACGAATAAATCGTC | 812 |
| 915 | OMPA_AY485227_379_4 01_F | TGCGCAGCTCTTGGTATCGAGTT | 509 | OMPA_AY485227_492_519_R | TGCCGTAACATAGAAGTTACCGTTGATT | 1223 |
| 916 | OMPA_AY485227_311_3 35_F | TACACAACAATGGCGGTAAAGATGG | 178 | OMPA_AY485227_424_453_R | | 901 |
| 917 | OMPA_AY485227_415_4 41_F | | 506 | OMPA_AY485227_514_546_R | | 1092 |
| 918 | OMPA_AY485227_494_5 20_F | TCAACGGTAACTTCTATGTTACTTCT G | 252 | OMPA_AY485227_569_596_R | TCGTCGTATTTATAGTGACCAGCACCTA | 1108 |
| 919 | OMPA_AY4 85227_551_5 77_F | TCAAGCCGTACGTATTATTAGGTGCT G | 257 | OMPA_AY485227_658_680_R | TTTAAGCGCCAGAAAGCACCAAC | 1425 |
| 920 | OMPA_AY485227_555_5 81_F | TCCGTACGTATTATTAGGTGCTGGTC A | 328 | OMPA_AY485227_635_662_R | TCAACACCAGCGTTACCTAAAGTACCTT | 954 |
| 921 | OMPA_AY485227_556_5 83_F | | 379 | OMPA_AY485227_659_683_R | TCGTTTAAGCGCCAGAAAGCACCAA | 1114 |
| 922 | OMPA_AY4 85227_657_6 79_F | TGTTGGTGCTTTCTGGCGCTTAA | 645 | OMPA_AY485227_739_765_R | TAAGCCAGCAAGAGCTGTATAGTTCCA | 871 |
| 923 | OMPA_AY485227_660_6 83_F | TGGTGCTTTCTGGCGCTTAAACGA | 613 | OMPA_AY485227_786_807_R | TACAGGAGCAGCAGGCTTCAAG | 884 |
| 924 | GYRA_AF100557_4_23_ F | TCTGCCCGTGTCGTTGGTGA | 402 | GYRA_AF100557_119_142_R | TCGAACCGAAGTTACCCTGACCAT | 1063 |
| 925 | GYRA_AF100557_70_94_ F | TCCATTGTTCGTATGGCTCAAGACT | 316 | GYRA_AF100557_178_201_R | TGCCAGCTTAGTCATACGGACTTC | 1211 |
| 926 | GYRB_AB008700_19_40_ F | TCAGGTGGCTTACACGGCGTAG | 289 | GYRB_AB008700_111_140_R | | 941 |
| 927 | GYRB_AB008700_265_2 92_F | | 420 | GYRE_AB008700_369_395_R | TCGTTGAGATGGTTTTTACCTTCGTTG | 1113 |
| 928 | GYRB_AB008700_368_3 94_F | | 251 | GYRB_AB008700_466_494_R | | 1440 |
| 929 | GYRB_AB008700_477_5 04_F | | 641 | GYRB_AB008700_611_632_R | TCACGCGCATCATCACCAGTCA | 977 |
| 930 | GYRB_AB008700_760_7 | TACTTACTTGAGAATCCACAAGCTGC | 198 | GYRB_AB008700_862_888_R | ACCTGCAATATCTAATGCACTCTTACG | 729 |
| | 87_F | AA | | | | |
| 931 | WAAA_Z96925_2_29_F | | 416 | WAAA_Z96925_115_138_R | CAAGCGGTTTGTCAAATAGTCA | 758 |
| 932 | WAAA_Z96925_286_311_ F | TCGATCTGGTTTCATGCTGTTTCAGT | 360 | WAAA_Z96925_394_412_R | TGGCACGAGCCTGACCTGT | 1274 |
| 939 | RPOB_EC_3798_3821_F | TGGGCAGCGTTTCGGCGAAATGGA | 581 | RPOB_EC_3862_3889_R | TGTCCGACTTGACGGTCAGCATTTCCTG | 1326 |
| 940 | RPOB_EC_3798_3821_F | TGGGCAGCGTTTCGGCGAAATGGA | 581 | RPOB_EC_3862_3889_2_R | TGTCCGACTTGACGGTTAGCATTTCCTG | 1327 |
| 941 | TUFB_EC_275_299_F | TGATCACTGGTGCTGCTCAGATGGA | 468 | TUFB_EC_337_362_R | TGGATGTGCTCACGAGTCTGTGGCAT | 1271 |
| 942 | TUFB_EC_251_278_F | | 493 | TUFB_EC_337_360_R | TATGTGCTCACGAGTTTGCGGCAT | 937 |
| 949 | GYRB_AB008700_760_7 87_F | | 198 | GYRB_AB008700_862_888_2_R | TCCTGCAATATCTAATGCACTCTTACG | 1050 |
| 958 | RPOC_EC_2223_2243_F | TGGTATGCGTGGTCTGATGGC | 605 | RPOC_EC_2329_2352_R | TGCTAGACCTTTACGTGCACCGTG | 1243 |
| 959 | RPOC_EC_918_938_F | TCTGGATAACGGTCGTCGCGG | 404 | RPOC_EC_1009_1031_R | TCCAGCAGGTTCTGACGGAAACG | 1004 |
| 960 | RPOC_EC_2334_2357_F | TGCTCGTAAGGGTCTGGCGGATAC | 523 | RPOC_EC_2380_2403_R | TACTAGACGACGGGTCAGGTAACC | 905 |
| 961 | RPOC_EC_917_938_F | TATTGGACAACGGTCGTCGCGG | 242 | RPOC_EC_1009_1034_R | TTACCGAGCAGGTTCTGACGGAAACG | 1362 |
| 962 | RPOB_EC_2005_2027_F | TCGTTCCTGGAACACGATGACGC | 387 | RPOB_EC_2041_2064_R | TTGACGTTGCATGTTCGAGCCCAT | 1399 |
| 963 | RPOB_EC_1527_1549_F | TCAGCTGTCGCAGTTCATGGACC | 282 | RPOB_EC_1630_1649_R | TCGTCGCGGACTTCGAAGCC | 1104 |
| 964 | INFB_EC_1347_1367_F | TGCGTTTACCGCAATGCGTGC | 515 | INFB_EC_1414_1432_R | TCGGCATCACGCCGTCGTC | 1090 |
| 965 | VALS_EC_1128_1151_F | TATGCTGACCGACCAGTGGTACGT | 237 | VALS_EC_1231_1257_R | TTCGCGCATCCAGGAGAAGTACATGTT | 1384 |
| 978 | RPOC_EC_2145_2175_F | | 285 | RPOC_EC_2228_2247_R | TTACGCCATCAGGCCACGCA | 1363 |
| 1045 | CJST_CJ_1668_1700_F | | 522 | CJST_CJ_1774_1799_R | TGAGCGTGTGGAAAAGGACTTGGATG | 1170 |
| 1046 | CJST_CJ_2171_2197_F | | 388 | CJST_CJ_2283_2313_R | | 1126 |
| 1047 | CJST_CJ_584_616_F | | 315 | CJST_CJ_663_692_R | | 1379 |
| 1048 | CJST_CJ_360_394_F | | 346 | CJST_CJ_442_476_R | | 955 |
| 1049 | CJST_CJ_2636_2668_F | | 504 | CJST_CJ_2753_2777_R | TTGCTGCCATAGCAAAGCCTACAGC | 1409 |
| 1050 | CJST_CJ_1290_1320_F | | 575 | CJST_CJ_1406_1433_R | TTTGCTCATGATCTGCATGAAGCATAAA | 1437 |
| 1051 | CJST_CJ_3267_3293_F | | 707 | CJST_CJ_3356_3385_R | | 951 |
| 1052 | CJST_CJ_5_39_F | | 222 | CJST_CJ_104_137_R | | 1029 |
| 1053 | CJST_CJ_1080_1110_F | | 681 | CJST_CJ_1166_1198_R | | 1022 |
| 1054 | CJST_CJ_2060_2090_F | | 323 | CJST_CJ_2148_2174_R | TCGATCCGCATCACCATCAAAAGCAAA | 1068 |
| 1055 | CJST_CJ_2869_2895_F | | 432 | CJST_CJ_2979_3007_R | | 1045 |
| 1056 | CJST_CJ_1880_1910_F | | 317 | CJST_CJ_1981_2011_R | | 1309 |
| 1057 | CJST_CJ_2185_2212_F | | 208 | CJST_CJ_2283_2316_R | | 1152 |
| 1058 | CJST_CJ_1643_1670_F | | 660 | CJST_CJ_1724_1752_R | | 1198 |
| 1059 | CJST_CJ_2165_2194_F | | 511 | CJST_CJ_2247_2278_R | | 1002 |
| 1060 | CJST_CJ_599_632_F | | 424 | CJST_CJ_711 743_R | | 1024 |
| 1061 | CJST_CJ_360_393_F | | 345 | CJST_CJ_443_477_R | | 882 |
| 1062 | CJST_CJ_2678_2703_F | | 321 | CJST_CJ_2760_2787_R | | 1339 |
| 1063 | CJST_CJ_1268_1299_F | | 29 | CJST_CJ_1349_1379_R | | 1096 |
| 1064 | CJST_CJ_1680_1713_F | | 479 | CJST_CJ_1795_1822_R | TATGTGTAGTTGAGCTTACTACATGAGC | 938 |
| 1065 | CJST_CJ_2857_2887_F | | 565 | CJST_CJ_2965_2998_R | | 1253 |
| 1070 | RNASEP_BKM_580_599_ F | TGCGGGTAGGGAGCTTGAGC | 512 | RNASEP_BKM_665_686_R | TCCGATAAGCCGGATTCTGTGC | 1034 |
| 1071 | RNASEP_BKM_616_637_ F | TCCTAGAGGAATGGCTGCCACG | 333 | RNASEP_BKM_665_687_R | TGCCGATAAGCCGGATTCTGTGC | 1222 |
| 1072 | RNASEP_BDP_574_592_ F | TGGCACGGCCATCTCCGTG | 561 | RNASEP_BDP_616_635_R | TCGTTTCACCCTGTCATGCCG | 1115 |
| 1073 | 23S BRM_1110_1129_F | TGCGCGGAAGATGTAACGGG | 510 | 23S_BRM_1176_1201_R | TCGCAGGCTTACAGAACGCTCTCCTA | 1074 |
| 1074 | 23S_BRM_515_536_F | TGCATACAAACAGTCGGAGCCT | 496 | 23S_BRM_616_635_R | TCGGACTCGCTTTCGCTACG | 1088 |
| 1075 | RNASEP_CLB_459_487_ F | TAAGGATAGTGCAACAGAGATATACC GCC | 162 | RNASEP_CLB_498_526_R | | 1247 |
| 1076 | RNASEP_CLB_459_487_ F | | 162 | RNASEP_CLB_498_522_R | TTTACCTCGCCTTTCCACCCTTACC | 1426 |
| 1077 | ICD_CXB_93_120_F | | 343 | ICD_CXB_172_194_R | TAGGATTTTTCCACGGCGGCATC | 921 |
| 1078 | ICD_CXB_92_120_F | | 671 | ICD_CXB_172_194_R | TAGGATTTTTCCACGGCGGCATC | 921 |
| 1079 | ICD_CXB_176_198_F | TCGCCGTGGAAAAAATCCTACGCT | 369 | ICD_CXB_224_247_R | TAGCCTTTTCTCCGGCGTAGATCT | 916 |
| 1080 | IS1111A_NC002971_68 66_6891_F | TCAGTATGTATCCACCGTAGCCAGTC | 290 | IS1111A_NC002971_6928_695 4_R | TAAACGTCCGATACCAATGGTTCGCTC | 848 |
| 1081 | IS1111A_NC002971_74 56_7483_F | | 594 | IS1111A_NC002971_7529_755 4_R | TCAACAACACCTCCTTATTCCCACTC | 952 |
| 1082 | RNASEP_RKP_419_448_ F | | 599 | RNASEP_RKP_542_565_R | TCAAGCGATCTACCCGCATTACAA | 957 |
| 1083 | RNASEP_RKP_422_443_ F | TAAGAGCGCACCGGTAAGTTGG | 159 | RNASEP_RKP_542_565_R | TCAAGCGATCTACCCGCATTACAA | 957 |
| 1084 | RNASEP_RKP_466_491_ F | TCCACCAAGAGCAAGATCAAATAGGC | 310 | RNASEP_RKP_542_565_R | TCAAGCGATCTACCCGCATTACAA | 957 |
| 1085 | RNASEP_RKP_264_287_ F | TCTAAATGGTCGTGCAGTTGCGTG | 391 | RNASEP_RKP_295_321_R | TCTATAGAGTCCGGACTTTCCTCGTGA | 1119 |
| 1086 | RNASEP_RKP_426_448_ F | TGCATACCGGTAAGTTGGCAACA | 497 | RNASEP_RKP_542_565_R | TCAAGCGATCTACCCGCATTACAA | 957 |
| 1087 | OMPB_RKP_860_890_F | TTACAGGAAGTTTAGGTGGTAATCTA AAAGG | 654 | OMPB_RKP_972_996_R | TCCTGCAGCTCTACCTGCTCCATTA | 1051 |
| 1088 | OMPB_RKP_1192_1221_ F | | 392 | OMPB_RKP_1288_1315_R | TAGCAgCAAAAGTTATCACACCTGCAGT | 910 |
| 1089 | OMPB_RKP_3417_3440_ F | TGCAAGTGGTACTTCAACATGGGG | 485 | OMPB_RKP_3520_3550_R | | 1310 |
| 1090 | GLTA_RKP_1043_1072_ F | | 576 | GLTA_RKP_1138_1162_R | TGAACATTTGCGACGGTATACCCAT | 1147 |
| 1091 | GLTA_RKP_400_428_F | | 413 | GLTA_RKP_499_529_R | | 1305 |
| 1092 | GLTA_RKP_1023_1055_ F | | 330 | GLTA_RKP_1129_1156_R | TTGGCGACGGTATACCCATAGCTTTATA | 1415 |
| 1093 | GLTA_RKP_1043_1072_ 2_F | | 553 | GLTA_RKP_1138_1162_R | TGAACATTTGCGACGGTATACCCAT | 1147 |
| 1094 | GLTA_RKP_1043_1072_ 3_F | | 543 | GLTA_RKP_1138_1164_R | TGTGAACATTTGCGACGGTATACCCAT | 1330 |
| 1095 | GLTA_RKP_400_428_F | | 413 | GLTA_RKP_505_534_R | | 1230 |
| 1096 | CTXA_VBC_117_142_F | TCTTATGCCAAGAGGACAGAGTGAGT | 410 | CTXA_VBC_194_218_R | TGCCTAACAAATCCCGTCTGAGTTC | 1226 |
| 1097 | CTXA_VBC_351_377_F | | 630 | CTXA_VBC_441_466_R | TGTCATCAAGCACCCCAAAATGAACT | 1324 |
| 1098 | RNASEP_VBC_331_349_ F | TCCGCGGAGTTGACTGGGT | 325 | RNASEP_VBC_388_414_R | TGACTTTCCTCCCCCTTATCAGTCTCC | 1163 |
| 1099 | TOXR_VBC_135_158_F | TCGATTAGGCAGCAACGAAAGCCG | 362 | TOXR_VBC_221_246_R | TTCAAAACCTTGCTCTCGCCAAACAA | 1370 |
| 1100 | ASD_FRT_1_29_F | | 690 | ASD_FRT_86_116_R | | 1164 |
| 1101 | ASD_FRT_43_76_F | | 295 | ASD_FRT_129_156_R | TCCATATTGTTGCATAAAACCTGTTGGC | 1009 |
| 1102 | GALE_FRT 168_199_F | | 658 | GALE_FRT_241_269_R | | 973 |
| 1103 | GALE_FRT_834_865_F | | 245 | GALE_FRT_901_925_R | TAGCCTTGGCAACATCAGCAAAACT | 915 |
| 1104 | GALE_FRT_308_339_F | | 306 | GALE_FRT_390_422_R | | 1136 |
| 1105 | IPAH_SGF_258_277_F | TGAGGACCGTGTCGCGCTCA | 458 | IPAH_SGF_301_327_R | TCCTTCTGATGCCTGATGGACCAGGAG | 1055 |
| 1106 | IPAH_SGF_113_134_F | TCCTTGACCGCCTTTCCGATAC | 350 | IPAH_SGF_172_191_R | TTTTCCAGCCATGCAGCGAC | 1441 |
| 1107 | IPAH_SGF_462_486_F | TCAGACCATGCTCGCAGAGAAACTT | 271 | IPAH_SGF_522_540_R | TGTCACTCCCGACACGCCA | 1322 |
| 1111 | RNASEP_BRM_461_488_ F | | 147 | RNASEP_BRM_542_561_R | TGCCTCGCGCAACCTACCCG | 1227 |
| 1112 | RNASEP_BRM_325_347_ F | TACCCCAGGGAAAGTGCCACAGA | 185 | RNASEP_BRM_402_428_R | TCTCTTACCCCACCCTTTCACCCTTAC | 1125 |
| 1128 | HUPB_CJ_113_134_F | TAGTTGCTCAAACAGCTGGGCT | 230 | HUPB_CJ_157_188_R | | 1028 |
| 1129 | HUPB_CJ_76_102_F | | 324 | HUPB_CJ_157_188_R | | 1028 |
| 1130 | HUPB_CJ_76_102_F | | 324 | HUPB_CJ_114_135_R | TAGCCCAGCTGTTTGAGCAACT | 913 |
| 1151 | AB_MLST-11-OIF007_62_91_F | | 454 | AB_MLST-11-OIF007_169_203_R | | 1418 |
| 1152 | AB_MLST-11-OIF007_185_214_F | | 243 | AB_MLST-11-OIF007_291_324_R | | 969 |
| 1153 | AB_MLST-11-OIF007_260_289_F | | 541 | AH_MLST-11-OIF007_364_393_R | | 1400 |
| 1154 | AB_MLST-11-OIF007_206_239_F | | 436 | AB_MLST-11-OIF007_318_344_R | TCCGCCAAAAACTCCCCTTTTCACAGG | 1036 |
| 1155 | AB_MLST-11-OIF007_522_552_F | | 378 | AB_MLST-11-OIF007_587_610_R | TTCTGCTTGAGGAATAGTGCGTGG | 1392 |
| 1156 | AB_MLST-11-OIF007_547_571_F | TCAACCTGACTGCGTGAATGGTTGT | 250 | AB_MLST-11-OIF007_656686_R | | 902 |
| 1157 | AB_MLST-11-OIF007_601_627_F | | 256 | AB_MLST-11-OIF007_710_736R | TACAACGTGATAAACACGACCAGAAGC | 881 |
| 1158 | AB_MLST-11-OIF007_1202_1225_F | TCGTGCCCGCAATTTGCATAAAGC | 384 | AB_MLST-11-OIF007_1266_1296_R | | 878 |
| 1159 | AB_MLST-11-OIF007_1202_1225_F | TCGTGCCCGCAATTTGCAATTTGCATAAAGC | 384 | AB_MLST-11-OIF007_1299_1316_R | TGCACCTGCGGTCGAGCG | 1199 |
| 1160 | AB_MLST-11-OIF007_1234_1264_F | | 694 | AB_MLST-11-OIF007_1335_1362_R | TGCCATCCATAATCACGCCATACTGACG | 1215 |
| 1161 | AB_MLST-11-OIF007_1327_1356_F | | 225 | AB_MLST-11-OIF007_1422_1448_R | TGCCAGTTTCCACATTTCACGTTCGTG | 1212 |
| 1162 | AB_MLST-11-OIF007_1345_1369_F | TCGTGATTATGGATGGCAACGTGAA | 383 | AB_MLST-11-OIF007_1470_1494_R | TCGCTTGAGTGTAGTCATGATTGCG | 1083 |
| 1163 | AB_MLST-11-OIF007_1351_1375_F | TTATGGATGGCAACGTGAAACGCGT | 662 | AB_MLST-11-OIF007_1470_1494_R | TCGCTTGAGTGTAGTCATGATTGCG | 1083 |
| 1164 | AB_MLST-11-OIF007_1387_1412_F | TCTTTGCCATTGAAGATGACTTAAGC | 422 | AB_MLST-11-OIF007_1470_1494_R | TCGCTTGAGTGTAGTCATGATTGCG | 1083 |
| 1165 | AB_MLST-11-OIF007_1542_1569_F | | 194 | AB_MLST-11-OIF007_1656_1680_R | TGAGTCGGGTTCACTTTACCTGGCA | 1173 |
| 1166 | AB_MLST-11-OIF007_1566_1593_F | | 684 | AH_MLST-11-OIF007_1656_1680_R | TGAGTCGGGTTCACTTTACCTGGCA | 1173 |
| 1167 | AB_MLST-11-OIF007_1611_1638_F | | 375 | AB_MLST-11-OIF007_1731_1757_R | TACCGGAAGCACCAGCGACATTAATAG | 890 |
| 1168 | AB_MLST-11-OIF007_1726_1752_F | | 182 | AB_MLST-11-OIF007_1790_1821_R | | 1195 |
| 1169 | AB_MLST-11-OIF007_1792_1826_F | | 656 | AB_MLST-11-OIF007_1876_1909_R | | 1151 |
| 1170 | AB_MLST-11-OIF007_1792_1826_F | | 656 | AS_MLST-11-OIF007_1895_1927_R | | 1224 |
| 1171 | AB_MLST-11-OIF007_1970_2002_F_F | | 618 | AB_MLST-11-OIF007_2097_2118_R | TGACGGCATCGATACCACCGTC | 1157 |
| 1172 | RNASEP_BRM_461_488_ F | | 147 | RNASEP_BRM_542_561_2_R | TGCCTCGTGCAACCCACCCG | 1228 |
| 2000 | CTXB_NC002505_46_70_ F | TCAGCGTATGCACATGGAACTCCTC | 278 | CTXB_NC002505_132_162_R | | 1039 |
| 2001 | FUR_NC0025_05_87_113_ F | | 465 | FUR_NC002505_205_228_R | TCCGCCTTCAAAATGGTGGCGAGT | 1037 |
| 2002 | FUR_NC002505_87_113 _F | | 465 | FUR_NC002505_178_205_R | TCACGATACCTGCATCATCAAATTGGTT | 974 |
| 2003 | GAPA_NC002505_533_5 60_F | | 356 | GAPA_NC002505_646_671_R | TCAGAATCGATGCCAAATGCGTCATC | 980 |
| 2004 | GAPA_NC002505_694_7 21_F | | 259 | GAPA_NC002505_769_798_R | | 1046 |
| 2005 | GAPA_NC002505_753_7 82_F | | 517 | GAPA_NC002505_856_881_R | TCCATCGCAGTCACGTTTACTGTTGG | 1011 |
| 2006 | GYRB_NC0025 05_2_32_ F | | 501 | GYRB_NC002505_109_134_R | TCCACCACCTCAAAGACCATGTGGTG | 1003 |
| 2007 | GYRB_NC002505_123_1_ 52_F | | 460 | GYRB_NC002505_199_225_R | TCCGTCATCGCTGACAGAAACTGAGTT | 1042 |
| 2008 | GYRH_NC002505_768_7 94_F | | 236 | GYRB_NC002505_832_860_R | | 1262 |
| 2009 | GYRB_NC002505_837_8_ 60_F | TGGTACTCACTTAGCGGGTTTCCG | 603 | GYRB_NC002505_937_957_R | TCCTTCACGCGCATCATCACC | 1054 |
| 2010 | GYRB_NC002505_934_9_ 56_F | TCGGGTGATGATGCGCGTGAAGG | 377 | GYRB_NC002505_982_1007_R | TGGCTTGAGAATTTAGGATCCGGCAC | 1283 |
| 2011 | GYRB_NC002505_1161_ 1190_F | | 148 | GYRB_NC002505_1255_1284_R | | 1172 |
| 2012 | OMPU_NCO02505_85_11_ 0_F | TACGCTGACGGAATCAACCAAAGCGG | 190 | OMPU_NC002505_154_180_R | TGCTTCAGCACGGCCACCAACTTCTAG | 1254 |
| 2013 | OMPU NC002505_258_2 83_F | TGACGGCCTATACGGTGTTGGTTTCT | 451 | OMPU_NC002505_346_369_R | TCCGAGACCAGCGTAGGTGTAACG | 1033 |
| 2014 | OMPU_NC002505_431_4 _ 55_F | TCACCGATATCATGGCTTACCACGG | 266 | OMPU_NC002505_544_567_R | TCGGTCAGCAAAACGGTAGCTTGC | 1094 |
| 2015 | OMPU_NC002505_533_5 _ 57_F | TAGGCGTGAAAGCAAGCTACCGTTT | 223 | OMPU_NC002505_625_651_R | TAGAGAGTAGCCATCTTCACCGTTGTC | 908 |
| 2016 | OMPU_NC002505_689_7 _ 13_F | TAGGTGCTGGTTACGCAGATCAAGA | 224 | OMPU_NC002505_725_751_R | TGGGGTAAGACGCGGCTAGCATGTATT | 1291 |
| 2017 | OMPU_NC002505_727_7 | TACATGCTAGCCGCGTCTTAC | 181 | OMPU NC002505_811_835_R | TAGCAGCTAGCTCGTAACCAGTGTA | 911 |
| | 47_F | | | | | |
| 2018 | OMPU_NC002505_931_9 53_F | TACTACTTCAAGCCGAACTTCCG | 193 | OMPU_NC002505_1033_1053_R | TTAGAAGTCGTAACGTGGACC | 1368 |
| 2019 | OMPU_NC002505_927_9 53_F | | 197 | OMPU_NC002505_1033_1054_R | TGGTTAGAAGTCGTAACGTGGACC | 1307 |
| 2020 | TCPA_NC002505_48_73_ F | TCACGATAAGAAAACCGGTCAAGAGG | 269 | TCPA_NC002505_148_170_R | TTCTGCGAATCAATCGCACGCTG | 1391 |
| 2021 | TDH_NC00460S_265_28_ 9_F | TGGCTGACATCCTACATGACTGTGA | 574 | TDH_NC004605_357_386_R | | 1351 |
| 2022 | WHA_NC004460_772_ 02_F | | 412 | VVHA_NC004460_862_886_R | TACCAAAGCGTGCACGATAGTTGAG | 887 |
| 2023 | 23S_EC_2643_2667_F | TGCCTGTTCTTAGTACGAGAGGACC | 508 | 23S_EC_2746_2770_R | TGGGTTTCGCGCTTAGATGCTTTCA | 1297 |
| 2024 | 16S_EC_713_732_TMOD_ F | TAGAACACCGATGGCGAAGGC | 202 | 16S_EC_789_811_R | TGCGTGGACTACCAGGGTATCTA | 1240 |
| 2025 | 16S_EC_784_806_F | TGGATTAGAGACCCTGGTAGTCC | 560 | 16S_EC_880_897_TMOD_R | TGGCCGTACTCCCCAGGCG | 1278 |
| 2026 | 16S_EC_959_981_F | TGTCGATGCAACGCGAAGAACCT | 634 | 16S_EC_1052_1074_R | TACGAGCTGACGACAGCCATGCA | 896 |
| 2027 | TUFB_EC_956_979_F | TGCACACGCCGTTCTTCAACAACT | 489 | TUFB_EC_1034_1058_2_R | TGCATCACCATTTCCTTGTCCTTCG | 1204 |
| 2028 | RPOC_EC_2146_2174_T MOD_F | | 284 | RPOC_EC_2227_2249_R | TGCTAGGCCATCAGGCCACGCAT | 1244 |
| 2029 | RPOB_EC_1841_1866_F | TGGTTATCGCTCAGGCGAACTCCAAC | 617 | RPOB_EC_1909_1929_TMOD_R | TGCTGGATTCGCCTTTGCTACG | 1250 |
| 2030 | RPLB_EC_650_679_TMO D_F | TGACCTACAGTAAGAGGTTCTGTAAT GAACC | 449 | RPLB_EC_739_763_R | TGCCAAGTGCTGGTTTACCCCATGG | 1208 |
| 2031 | RPLB_EC_690_710_F | TCCACACGGTGGTGGTGAAGG | 309 | RPLB_EC_737_760_R | TGGGTGCTGGTTTACCCCATGGAG | 1295 |
| 2032 | INFB_EC_1366_1393_F | | 397 | INFB_EC_1439_1469_R | | 1335 |
| 2033 | VALS_EC_1105_1124_T MOD_F | TCGTGGCGGCGTGGTTATCGA | 385 | VALS_EC_1195_1219_R | TGGGTACGAACTGGATGTCGCCGTT | 1292 |
| 2034 | SSPE_BA_113_137_F | TGCAAGCAAACGCACAATCAGAAGC | 482 | SSPE_BA_197_222_TMOD_R | TTGCACGTCTGTTTCAGTTGCAAATTC | 1402 |
| 2035 | RPOC_EC_2218_2241_T MOD_F | TCTGGCAGGTATGCGTGGTCTGATG | 405 | RPOC_EC_2313_2338_R | TGGCACCGTGGGTTGAGATGAAGTAC | 1273 |
| 2056 | MECI-R_NC003923-41798-41609 33_60_F | | 698 | MECI-R_NC003923-41798-41609_86_113_R | TTGTGATATGGAGGTGTAGAAGGTGTTA | 1420 |
| 2057 | AGR-III_NC003923-2108074-2109507_1_23_F | TCACCAGTTTGCCACGTATCTTCAA | 263 | AGR-III_NC003923-2108074-2109507_56_79_R | ACCTGCATCCCTAAACGTACTTGC | 730 |
| 2058 | AGR-III_NC003923-2108074-2109507_569_596_F | | 457 | AGR-III_NC003923-2108074-2109507_622_653_R | | 906 |
| 2059 | AGR-III_NC003923-2108074-2109507 1024_1052_F | | 701 | AGR-III_NC003923-2108074-2109507_1070_1098_R | | 1319 |
| 2060 | AGR-I_AJ617706_622_651F | | 610 | AGR-1 AJ617706_694_726_R | | 1021 |
| 2061 | AGR-I_AJ617706_580_611_ F | | 579 | AGR-I AJ617706 626_655_R | | 1302 |
| 2062 | AGR-II_NC002745-2079448-2080879_620_651_F | | 415 | AGR-II_NC002745-2079448-2080879_700 731_R | | 1424 |
| 2063 | AGR-II_NC002745-2079448-2080879 649 679 F | | 624 | AGR-II_NC002745-2079448-2080879_715_745_R | | 1077 |
| 2064 | AGR-IV_AJ617711_931_961_ F | | 606 | AGR-IV AJ617711 1004_1035_R | | 1233 |
| 2065 | AGR-IV_AJ617711_250_283_ F | | 562 | AGR-IV AJ617711 309_335_R | TCCCATACCTATGGCGATAACTGTCAT | 1017 |
| 2066 | BLAZ_NC002952 (19138 27..1914672)_68_68_ F | | 312 | BLAZ_NC002952(1913827..19 14672)_68_68_R | | 1277 |
| 2067 | BLAZ_NC002952(19138 27..1914672)_68_68_ _2_F | | 494 | BLAZ_NC002952 (1913827..19 14672)_68_68_2_R | | 926 |
| 2068 | BLAZ_NC002952(19138 27..1914672)_68 68_ _3_F | TGATACTTCAACGCCTGCTGCTTTC | 467 | BLAZ_NC002952(1913827..19 14672)_68_68_3_R | | 1263 |
| 2069 | BLAZ_NC002952(19138 27..1914672)_68_68_ _4_F | TATACTTCAACGCCTGCTGCTTTC | 232 | BLAZ_NC002952(1913827..19 14672)_68_68_4_R | | 1145 |
| 2070 | BLAZ_NC002952(19138 27..1914672)_1_33_F | | 487 | BLAZ_NC002952(1913827..19 14672)_34_67_R | | 1366 |
| 2071 | BLAZ_NC002952 (19138 27..1914672) 3_34_F | | 351 | BLAZ_NC002952(1913827..19 14672)_40_68_R | | 1289 |
| 2072 | BSA-A_NC003923-1304065-_1303589 99_125_F | | 214 | BSA-A_NC003923-1304065-1303589_165_193_R | | 1197 |
| 2073 | BSA-A_NC003923-1304065-_1303589 194 218 F | ATCAATTTGGTGGCCAAGAACCTGG | 32 | BSA-A_NCO03923-1304065-1303589_253_278_R | TGCATAGGGAAGGTAACACCATAGTT | 1203 |
| 2074 | BSA-A_NC003923-1304065-1303589_328 349_F | TTGACTGCGGCACAACACGGAT | 679 | BSA-A_NC003923-1304065-1303589_388_415_R | TAACAACGTTACCTTCGCGATCCACTAA | 856 |
| 2075 | BSA-A_NC003923-1304065-_1303589_253 278 F | TGCTATGGTGTTACCTTCCCTATGCA | 519 | BSA-A_NC003923-1304065-1303589_317_344_R | TGTTGTGCCGCAGTCAAATATAAATA | 1353 |
| 2076 | BSA-B NC003923-1917149-1914156_953_982_F | TAGCAACAAATATATCTGAAGCAGCGTACT | 209 | BSA-B NC003923-1917149-1914156_1011_1039_R | TGTGAAGAACTTTCAAATCTGTGAATCCA | 1331 |
| 2077 | BSA-B_NC003923-1917149-1914156_1050_1081_F | | 426 | BSA-B_NC003923-1917149-1914156_1109_1136_R | TCTTCTTGAAAAATTGTTGTCCCGAAAC | 1138 |
| 2078 | BSA-B_NC003923-1917149-1914156 1260_1286_F | | 300 | BSA-B_NC003923-1917149-1914156_1323_1353_R | | 1267 |
| 2079 | BSA-B_NC003923-1917149-1914156_2126_2153_F | | 703 | BSA-B_NC003923-1917149-1914156_2186_2216_R | | 1148 |
| 2080 | ERMA_NC002952-55890-56621_366_392_F | | 372 | ERMA₋NC002952-55890-56621_487_513_R | TGAGTCTACACTTGGCTTAGGATGAAA | 1174 |
| 2081 | ERMA_NC002952-55890-56621_366_395_F | | 217 | ERMA_NC002952-55890-56621_438_465_R | TGAGCATTTTTATATCCATCTCCACCAT | 1167 |
| 2082 | ERMA_NC002952-55890-56621_374_402_F | | 470 | ERMA_NC002952-55890-56621_473_504_R | | 1143 |
| 2083 | ERMA_NC002952-55890-56621_404_427_F | TGCAAAATCTGCAACGAGCTTTGG | 480 | ERMA_NC002952-55890-56621_491_520_R | | 964 |
| 2084 | ERMA_NC002952-55890-56621_489_516_F | | 297 | ERMA_NC002952-55890-56621_586_615_R | | 1266 |
| 2085 | ERMA_NC002952-55890-56621_586_614_F | | 231 | ERMA_NC002952-55890-56621_640_665_R | TTGACATTTGCATGCTTCAAAGCCTG | 1397 |
| 2086 | ERMC_NC005908-2004-2738_85_116_F | | 399 | ERMC_NC005908-2004-2738_173_206_R | | 1041 |
| 2087 | ERMC_NC005908-2004-2738_90_120_F | | 298 | ERMC_NC005908-2004-2738_160_189_R | | 1429 |
| 2088 | ERMC_NC005908-2004-2738_115_139_F | TCAGGAAAAGGGCATTTTACCCTTG | 283 | ERMC_NC005908-2004-2738_161_187_R | TATGGTCTATTTCAATGGCAGTTACGA | 936 |
| 2089 | ERMC_NC005908-2004-2738_374_397_F | TAATCGTGGAATACGGGTTTGCTA | 168 | ERMC_NC005908-2004-2738_425_452_R | TCAACTTCTGCCATTAAAAGTAATGCCA | 956 |
| 2090 | ERMC_NC005908-2004-2738_101_125_F | TCTTTGAAATCGGCTCAGGAAAAGG | 421 | ERMC_NC005908-2004-2738_159_188_R | | 1185 |
| 2091 | ERMB_Y13600-625-1362_291_321_F | | 644 | ERMB_Y13600-625-1362_352_380_R | | 953 |
| 2092 | ERMB Y13600-625-1362_344_367_F | TGGAAAGCCATGCGTCTGACATCT | 536 | ERMB_Y13600-625-1362_415_437_R | TGCAAGAGCAACCCTAGTGTTCG | 1196 |
| 2093 | ERMB_Y13600-625-1362_404_429_F | TGGATATTCACCGAACACTAGGGTTG | 556 | ERMB_Y13600-625-1362_471_493_R | TAGGATGAAAGCATTCCGCTGGC | 919 |
| 2094 | ERMB_Y13600-625-1362_465_487_F | TAAGCTGCCAGCGGAATGCTTTC | 161 | ERMB_Y13600-625-1362_521_545_R | TCATCTGTGGTATGGCGGGTAAGTT | 989 |
| 2095 | PVLUK_NC003923-1529595-1531285_688_713_F | TGAGCTGCATCAACTGTATTGGATAG | 456 | PVLUK_NC003923-1529595-1531285_775_804_R | | 1261 |
| 2096 | PVLUK_NC003923-1529595-1531285_1039_1068_F | | 539 | PVLUK_NC003923-1529595-1531285_1095_1125_R | | 993 |
| 2097 | PVLUK_NC003923-1529595-1531285_908_936_F | | 461 | PVLUK_NC003923-1529595-1531285_950_978_R | | 1124 |
| 2098 | PVLUK_NC003923-1529595-1531285_610_633_F | TCGGAATCTGATGTTGCAGTTGTT | 373 | PVLUK_NC003923-1529595-1531285_654_682_R | | 968 |
| 2099 | SA442_NC003923-2538576-2538831_11_35_F | TGTCGGTACACGATATTCTTCACGA | 635 | SA442_NC003923-2538576-2538831_98_124_R | TTTCCGATGCAACGTAATGAGATTTCA | 1433 |
| 2100 | SA442_NC003923-2538576-2538831_98_124_F | | 427 | SA442_NC003923-2538576-2538831_163_188_R | TCGTATGACCAGCTTCGGTACTACTA | 1098 |
| 2101 | SA442_NC003923-2538576-2538831_103_126_F | TCTCATTACGTTGCATCGGAAACA | 395 | SA442_NC003923-2538576-2538831_161_187_R | TTTATGACCAGCTTCGGTACTACTAAA | 1428 |
| 2102 | SA442_NC003923-2538576-2538831_166_188_F | TAGTACCGAAGCTGGTCATACGA | 226 | SA442₋NCO03923-2538576-2538831_231_257_R | TGATAATGAAGGGAAACCTTTTTCACG | 1179 |
| 2103 | SEA_NC003923-2052219-2051456_115_135_F | TGCAGGGAACAGCTTTAGGCA | 495 | SEA_NC003923-2052219-2051456_173_200_R | TCGATCGTGACTCTCTTTATTTTCAGTT | 1070 |
| 2104 | SEA_NCO03923-2052219-2051456_572_598_F | | 156 | SEA_NC003923-2052219-2051456_621_651_R | | 1315 |
| 2105 | SEA_NC003923-2052219-2051456_382_414_F | | 629 | SEA_NC003923-2052219-2051456_464_492_R | | 861 |
| 2106 | SEA_NC003923-2052219-2051456_377_406_F | | 695 | SEA_NC003923-2052219-2051456_459_492_R | | 862 |
| 2107 | SEB_NC002758-2135540-2135140_208_237_F | | 702 | SEB_NC002758-2135540-2135140_273_298_R | TCATCTGGTTTAGGATCTGGTTGACT | 988 |
| 2108 | SEB_NC002758-2135540-2135140_206_235_F | | 244 | SEB_NC002758-2135540-2135140_281_304_R | TGCAACTCATCTGGTTTAGGATCT | 1194 |
| 2109 | SEB_NC002758-2135540-2135140_402_402_F | | 151 | SEB_NC002758-2135540-2135140_402_402_R | TGTGCAGGCATCATGTCATACCAA | 1334 |
| 2110 | SEB_NC002758-2135540-2135140_402_402_2_F | | 696 | SEB₋NC002758-2135540-2135140_402_402_2_R | TTACCATCTTCAAATACCCGAACAGTAA | 1361 |
| 2111 | SEC_NC003923-851678-852768_546_575_F | | 648 | SEC_NC003923-851678-852768_620_647_R | TGAGTTTGCACTTCAAAAGAAATTGTGT | 1177 |
| 2112 | SEC_NC003923-851678-852768_537_566_F | | 546 | SEC_NC003923-851678-852768_619_647_R | | 985 |
| 2113 | SEC_NC003923-851678-852768 720 749 F | | 466 | SEC_NC003923-851678-852768 794 815 R | TCGCCTGGTGCAGGCATCATAT | 1078 |
| 2114 | SEC_NC003923-851678-852768_ 787_810 F | TGGTATGATATGATGCCTGCACCA | 604 | SEC_NC003923-851678-852768 853 886 R | | 1133 |
| 2115 | SED_M28521_657_682_ F | TGGTGGTGAAATAGATAGGACTGCTT | 615 | SED M28521_741_770_R | | 1318 |
| 2116 | SED_M28521_690_711_ F | TGGAGGTGTCACTCCACACGAA | 554 | SED_M28521_739_770_R | | 1288 |
| 2117 | SED_M28521_833_854_ F | TTGCACAAGCAAGGCGCTATTT | 683 | SED_M28521_888_911_R | TCGCGCTGTATTTTTCCTCCGAGA | 1079 |
| 2118 | SED_M28521_962_987_ F | TGGATGTTAACGGTGATTTTCCCGAA | 559 | SED_M28521_1022_1048_R | TGTCAATATGAAGGTGCTCTGTGGATA | 1320 |
| 2119 | SEA-SEE_NC002952-2131289-2130703_16_45_F | | 699 | SEA-SEE_NC002952-2131289-2130703_71_98_R | TCATTTATTTCTTCGCTTTTCTCGCTAC | 994 |
| 2120 | SEA-SEE_NC002952-2131289-2130703_249_278_F | | 469 | SEA-SEE_NC002952-2131289-2130703 314 344 R | | 870 |
| 2121 | SEE_NC002952-2131289-2130703_409_437_F | | 445 | SEE_NC002952-2131289-2130703_465_494_R | TCTATAGGTACTGTAGTTTGTTTTCCGT CT | 1120 |
| 2122 | SEE_NC002952-2131289-2130703_525_550_F | TGTTCAAGAGCTAGATCTTCAGGCAA | 640 | SEE_NC002952-2131289-2130703_586_586_R | TTTGCACCTTACCGCCAAAGCT | 1436 |
| 2123 | SEE_NC002952-2131289-2130703_525_549_F | TGTTCAAGAGCTAGATCTTCAGGCA | 639 | SEE_NC002952-2131289-2130703_586_586_2_R | TACCTTACCGCCAAAGCTGTCT | 892 |
| 2124 | SEE_NC002952-2131289-2130703 361 384 F | TCTGGAGGCACACCAAATAAAACA | 403 | SEE_NC002952-2131289-2130703 444 471 R | TCCGTCTATCCACAAGTTAATTGGTACT | 1043 |
| 2125 | SEG_NC002758-1955100-1954171_225_251_F | | 520 | SEG_NC002758-1955100-1954171321_346_R | TAACTCCTCTTCCTTCAACAGGTGGA | 863 |
| 2126 | SEG_NC002758-1955100-1954171_623_651_F | | 548 | SEG_NC002758-1955100-1954171_671_702_R | | 1260 |
| 2127 | SEG_NC002758-1955100-1954171_540_564_F | TGGAGGTTGTTGTATGTATGGTGGT | 555 | SEG_NC002758-1955100-1954171_607_635_R | | 1329 |
| 2128 | SEG_NC002758-1955100-1954171_694_718_F | TACAAAGCAAGACACTGGCTCACTA | 173 | SEG_NC002758-1955100-1954171_735_762_R | TGATTCAAATGCAGAACCATCAAACTCG | 1187 |
| 2129 | SEH_NC002953^{.}-60024-60977_449_472_F | TTGCAACTGCTGATTTAGCTCAGA | 682 | SEH_NC002953-60024-60977_547_576_R | | 927 |
| 2130 | SEH_NC002953-60024-60977_408_434_F | | 201 | SEH_NC002953-60024-60977_450_473_R | TTCTGAGCTAAATCAGCAGTTGCA | 1390 |
| 2131 | SEH_NC002953-60024-60977_547_576_F | | 400 | SEH_NC002953-60024-60977_608_634_R | TACCATCTACCAAACATTAGCACCAA | 888 |
| 2132 | SEH_NC002953-60024-60977_546_575_F | | 677 | SEH_NC002953-60024-60977_594_616_R | TAGCACCAATCACCCTTTCCTGT | 909 |
| 2133 | SEI_NC002758-1957830-1956949_324_349_F | TCAACTCGAATTTTCAACAGGTACCA | 253 | SEI_NC002758-1957830-1956949_419_446_R | TCACAAGGACCATTATAATCAATGCCAA | 966 |
| 2134 | SEI_NC002758-1957830-1956949_336_363_F | | 666 | SEI_NC002758-1957830-1956949_420_447_R | TGTACAAGGACCATTATAATCAATGCCA | 1316 |
| 2135 | SEI_NC002758-1957830-1956949_356_384_F | | 471 | SEI_NC002758-1957830-1956949_449_474_R | TCTGGCCCCTCCATACATGTATTTAG | 1129 |
| 2136 | SEI_NC002758-1957830-1956949_223_253_F | | 394 | SEI_NC002758-1957830-1956949_290_316_R | TGGGTAGGTTTTTATCTGTGACGCCTT | 1293 |
| 2137 | SEJ_AF053140_1307_1 332_F | TGTGGAGTAACACTGCATGAAAACAA | 637 | SEJ_AF053140_1381_1404_R | TCTAGCGGAACAACAGTTCTGATG | 1118 |
| 2138 | SEJ_AF053140_1378_1 403_F | TAGCATCAGAACTGTTGTTCCGCTAG | 211 | SEJ_AF053140 1429_1458_R | | 1049 |
| 2139 | SEJ_AF053140_1431_1 459_F | | 153 | SEJ_AF053140_1500_1531_R | | 925 |
| 2140 | SEJ_AF053140_1434_1 461_F | | 301 | SEJ_AF053140 1521_1549_R | | 984 |
| 2141 | TSST_NC002758-2137564-2138293_206_236_F | | 619 | TSST_NC002758-2137564-2138293_278_305_R | TGTAAAAGCAGGGCTATAATAAGGACTC | 1312 |
| 2142 | TSST_NC002758-2137564-2138293_232_258_F | | 514 | TSST_NC002758-2137564-2138293_289_313_R | TGCCCTTTTGTAAAAGCAGGGCTAT | 1221 |
| 2143 | TSST_NC002758-2137564-2138293_382_410_F | | 304 | TSST_NC002758-2137564-2138293_448_478_R | | 907 |
| 2144 | TSST_NC002758-2137564-2138293_297_325_F | | 423 | TSST_NC002758-2137564-2138293_347_373_R | TAAGTTCCTTCGCTAGTATGTTGGCTT | 874 |
| 2145 | ARCC_NC003923-2725050-2724595_37_58_F | TCGCCGGCAATGCCATTGGATA | 368 | ARCC_NC003923-2725050-2724595_97_128_R | | 1175 |
| 2146 | ARCC_NC003923-2725050-2724595_131_161_F | | 437 | ARCC_NC003923-2725050-2724595_214_245_R | | 1137 |
| 2147 | ARCC_NC003923-2725050-2724595_218_249_F | | 691 | ARCC_NC003923-2725050-2724595_322_353_R | | 1306 |
| 2148 | AROE_NC003923-1674726-1674277_371_393_F | TTGCGAATAGAACGATGGCTCGT | 686 | AROE_NC003923-1674726-1674277_435_464_R | | 1064 |
| 2149 | AROE_NC003923-1674726-1674277_30_62_F | | 590 | AROE_NC003923-1674726-1674277_155_181_R | TACCTGCATTAATCGCTTGTTCATCAA | 891 |
| 2150 | AROE_NC003923-1674726-1674277_204_232_F | | 474 | AROE_NC003923-1674726-1674277_308_335_R | TAAGCAATACCTTTACTTGCACCACCTG | 869 |
| 2151 | GLPF_NC003923-1296927-1297391_270_301_F | | 491 | GLPF_NC003923-1296927-1297391_382_414_R | | 1193 |
| 2152 | GLPF_NC003923-1296927-1297391_27_51_F | TGGATGGGGATTAGCGGTTACAATG | 558 | GLPF_NC003923-1296927-1297391_81_108_R | TAAAGACACCGCTGGGTTTAAATGTGCA | 850 |
| 2153 | GLPF_NC003923-1296927-1297391_239_260_F | TAGCTGGCGCGAAATTAGGTGT | 218 | GLPF_NC003923-1296927-1297391_323_359_R | | 972 |
| 2154 | GMK_NC003923-1190906-1191334_91_122_F | | 200 | GMK_NC003923-1190906-1191334_166_197_R | | 1180 |
| 2155 | GMK_NC003923-1190906-1191334_240_267_F | | 435 | GMK_NC003923-1190906-1191334_305_333_R | | 1082 |
| 2156 | GMK_NC003923-1190906-1191334_301_329_F | | 268 | GMK_NC003923-1190906-1191334_403_432_R | | 1284 |
| 2157 | PTA_NC003923-628885-629355_237_263_F | | 418 | PTA_NC003923-628885-629355_314_345_R | | 1301 |
| 2158 | PTA_NC003923-628885-629355_141_171_F | | 439 | PTA_NC003923-628885-629355_211_239_R | | 1207 |
| 2159 | PTA_NC003923-628885-629355_328_356_F | | 303 | PTA_NC003923-628885-629355_393_422_R | | 1349 |
| 2160 | TPI_NC003923-830671-831072_131_160_F | | 486 | TPI_NC003923-830671-831072_209_239_R | | 1165 |
| 2161 | TPI_NC003923-830671-831072_1_34_F | | 318 | TPI_NC003923-830671-831072_97_129_R | | 1300 |
| 2162 | TPI_NC003923-830671-831072_199_227_F | | 246 | TPI_NC003923-830671-831072_253_286_R | | 1275 |
| 2163 | YQI_NC003923-378916-379431_142_167_F | TGAATTGCTGCTATGAAAGGTGGCTT | 440 | YQI_NC003923-378916-379431_259_284_R | TCGCCAGCTAGCACGATGTCATTTTC | 1076 |
| 2164 | YQI_NC003923-378916-379431_44_77_F | | 175 | YQI_NC003923-378916-379431_120_145_R | TTCGTGCTGGATTTTGTCCTTGTCCT | 1388 |
| 2165 | YQI_NC003923-378916-379431_135_160_F | TCCAGCACGAATTGCTGCTATGAAAG | 314 | YQI_NC003923-378916-379431_193_221_R | | 997 |
| 2166 | YQI_NC003923-378916-379431_275_300_F | TAGCTGGCGGTATGGAGAATATGTCT | 219 | YQI_NC003923-378916-379431_364_396_R | | 1013 |
| 2167 | BLAZ_(1913827..1914 672)_546 575_F | | 312 | BLAZ_(1913827..1914672)_6 55_683_R | | 1277 |
| 2168 | BLAZ_(1913827..1914 672)_546 575 _ F | | 494 | BLAZ_(1913827..1914672)_6 28_659_R | | 926 |
| 2169 | BLAZ_(1913827..1914 672)_507 531_F | TGATACTTCAACGCCTGCTGCTTTC | 467 | BLAZ_(1913827..1914672)_6 22_651_R | | 1263 |
| 2170 | BLAZ_(1913827..1914 672)_508 531_F | TATACTTCAACGCCTGCTGCTTTC | 232 | BLAZ_(1913827..1914672)_5 53_583_R | | 1145 |
| 2171 | BLAZ_(1913827..1914 672)_24_56_F | | 487 | BLAZ_(1913827..1914672)_1 21_154_R | | 1366 |
| 2172 | BLAZ_(1913827..1914 672)_26_58_F | | 351 | BLAZ_(1913827..1914672)_1 27_157_R | | 1289 |
| 2173 | BLAZ_NC002952-1913827-1914672_546_575_F | | 312 | BLAZ_NC002952-1913827-1914672_655_683_R | | 1277 |
| 2174 | BLAZ_NC002952-1913827-1914672_546_575_2_F | | 494 | BLAZ_NC002952-1913827-1914672_628 659_R | | 926 |
| 2175 | BLAZ_NC002952-1913827-1914672_507_531_F | TGATACTTCAACGCCTGCTGCTTTC | 467 | BLAZ_NC002952-1913827-1914672_622_651_R | | 1263 |
| 2176 | BLAZ_NC002952-1913827-1914672_508_531_F | TATACTTCAACGCCTGCTGCTTTC | 232 | BLAZ_NC002952-1913827-1914672_553_583_R | | 1145 |
| 2177 | BLAZ_NC002952-1913827-1914672_24_56_F | | 487 | BLAZ_NC002952-1913827-1914672_121_154_R | | 1366 |
| 2178 | BLAZ_NC002952-1913827-1914672_26_58_F | | 351 | BLAZ_NC002952-1913827-1914672_127_157_R | | 1289 |
| 2247 | TUFB_NC002758-615038-616222_693_721_F | | 643 | TUFB_NC002758-615038-616222_793_820_R | TGTCACCAGCTTCAGCGTAGTCTAATAA | 1321 |
| 2248 | TUFB_NC002758-615038-616222_690_716_F | | 386 | TUFB_NC002758-615038-616222_793_820_R | TGTCACCAGCTTCAGCGTAGTCTAATAA | 1321 |
| 2249 | TUFB_NC002758-615038-616222_696_725_F | | 430 | TUFB_NC002758-615038-616222 793 820 R | TGTCACCAGCTTCAGCGTAGTCTAATAA | 1321 |
| 2250 | TUFB_NC002758-615038-616222_488_513_F | TCCCAGGTGACGATGTACCTGTAATC | 320 | TUFB_NC002758-615038-616222_601_630_R | | 1311 |
| 2251 | TUFB_NC002758-615038-616222_945 972_F | | 433 | TUFB_NC002758-615038-616222_1030_1060_R | | 922 |
| 2252 | TUFB_NC002758-615038-616222_333_356_F | TCCAATGCCACAAACTCGTGAACA | 307 | TUFB_NC002758-615038-616222 424 459 R | | 1382 |
| 2253 | NUC_NC002758-894288-894974_402_424_F | TCCTGAAGCAAGTGCATTTACGA | 342 | NUC_NC002758-894288-894974_483_509_R | TACGCTAAGCCACGTCCATATTTATCA | 899 |
| 2254 | NUC_NC002758-894288-894974_53_81_F | | 349 | NUC_NC002758-894288-894974_165_189_R | TGTTTGTGATGCATTTGCTGAGCTA | 1354 |
| 2255 | NUC_NC002758-894288-894974_169_194_F | TCAGCAAATGCATCACAAACAGATAA | 273 | NUC_NC002758-894288-894974_222_250_R | | 928 |
| 2256 | NUC_NC002758-894288-894974_316_345_F | | 174 | NUC_NC002758-894288-894974_396_421_R | TAAATGCACTTGCTTCAGGGCCATAT | 853 |
| 2270 | RPOB_EC_3798_3821_1 F | TGGCCAGCGCTTCGGTGAAATGGA | 566 | RPOB_EC_3868_3895_R | TCACGTCGTCCGACTTCACGGTCAGCAT | 979 |
| 2271 | RPOB_EC_3789_3812_F | TCAGTTCGGCGGTCAGCGCTTCGG | 294 | RPOB_EC_3860_3890_R | | 1107 |
| 2272 | RPOB_EC_3789_3812_F | TCAGTTCGGCGGTCAGCGCTTCGG | 294 | RPOB_EC_3860_3890_2_R | | 1102 |
| 2273 | RPOB_EC_3789_3812_F | TCAGTTCGGCGGTCAGCGCTTCGG | 294 | RPOB_EC_3862_3890_R | | 1106 |
| 2274 | RPOB_EC_3789_3812_F | TCAGTTCGGCGGTCAGCGCTTCGG | 294 | RPOB_EC_3862_3890_2_R | | 1101 |
| 2275 | RPOB_EC_3793_3812_F | TTCGGCGGTCAGCGCTTCGG | 674 | RPOB_EC_3865 3890_R | TCGTCGGACTTAACGGTCAGCATTTC | 1105 |
| 2276 | RPOB_EC_3793_3812_F | TTCGGCGGTCAGCGCTTCGG | 674 | RPOB_EC_3865_3890_2_R | TCGTCCGACTTAACGGTCAGCATTTC | 1100 |
| 2309 | MUPR_X75439_1658_16 89_F | | 352 | MUPR_X75439_1744_1773_R | | 1030 |
| 2310 | MUPR_X75439_1330_13 53_F | TTCCTCCTTTTGAAAGCGACGGTT | 669 | MUPR_X75439_1413_1441_R | | 1171 |
| 2312 | MUPR_X75439_1314_13 38_F | TTTCCTCCTTTTGAAAGCGACGGTT | 704 | MUPR_X75439_1381_1409_R | | 931 |
| 2313 | MUPR_X75439_2486_25 16_F | | 172 | MUPR_X75439_2548_2574_R | TTAATCTGGCTGCGGAAGTGAAATCGT | 1360 |
| 2314 | MUPR_X75439_2547_25 72_F | TACGATTTCACTTCCGCAGCCAGATT | 188 | MUPR_X75439_2605_2630_R | TCGTCCTCTCGAATCTC"GhThThr^{c} | 1103 |
| 2315 | MUPR_X75439_2666_26 96_F | | 513 | MUPR_X75439_2711_2740_R | | 981 |
| 2316 | MUPR_X75439_2813_28 43_F | | 165 | MUPR_X75439_2867_2890_R | TCTGCATTTTTGCGAGCCTGTCTA | 1127 |
| 2317 | MUPR_X75439_884_914_ F | | 447 | MUPR_X75439_977_1007_R | | 1317 |
| 2318 | CTXA_NC002505-1568114-1567341_114_142_F | | 608 | CTXA_NC002505-1568114-1567341_194_221_R | TCGTGCCTAACAAATCCCGTCTGAGTTC | 1109 |
| 2319 | CTXR_NC002505-1568114-1567341_117_145_F | | 411 | CTXA_NC002505-1568114-1567341_194_221_R | TCGTGCCTAACAAATCCCGTCTGAGTTC | 1109 |
| 2320 | CTXA_NC002505-1568114-1567341_114_142_F | | 608 | CTXA_NC002505-1568114-1567341_186_214_R | | 855 |
| 2321 | CTXA_NC002505-1568114-1567341_117_145_F | | 411 | CTXA_NC002505-1568114-1567341_186_214_R | | 855 |
| 2322 | CTXA_NC002505-1568114-1567341_129_156_F | | 27 | CTXA_NC002505-1568114-1567341 180 207 R | TCCCGTCTGAGTTCCTCTTGCATGATCA | 1027 |
| 2323 | CTXA_NC002505-1568114-1567341_122_149_F | | 500 | CTXA_NC002505-1568114-1567341_186_214_R | | 855 |
| 2324 | INV_U22457-74-3772_831_858_F | | 530 | INV_U22457-74-3772_942_966_R | TGACCCAAAGCTGAAAGCTTTACTG | 1154 |
| 2325 | INV_U22457-74-3772_827_857_F | | 438 | INV_U22457-74-3772 942 970 R | | 864 |
| 2326 | INV_U22457-74-3772_1555_1581_F | TGCTGGTAACAGAGCCTTATAGGCGCA | 526 | INV_U22457-74-3772_1619_1647_R | TGGGTTGCGTTGCAGATTATCTTTACCAA | 1296 |
| 2327 | INV_U22457-74-3772_1558 1585_F | | 598 | INV_U22457-74-3772_1622_1652_R | | 987 |
| 2328 | ASD_NC006570_ 439714_ 438608_3_37_F | | 459 | ASD_NC006570-439714-438608_54_84_R | | 1188 |
| 2329 | ASD_NC006570_ 439714_ 438608_18 45_F | | 149 | ASD_NC006570-439714-438608_66_95_R | | 948 |
| 2330 | ASD_NC006570_ 439714_ 438608_17 45_F | | 647 | ASD_NC006570-439714-438608_67_95_R | | 1016 |
| 2331 | ASD_NC006570_ 439714_ 438608_9_40_F | | 709 | ASD_NC006570-439714-438608_107_134_R | TCTGCCTGAGATGTCGAAAAAAACGTTG | 1128 |
| 2332 | GALE_AF513299_171_2 00_F | | 280 | GALE_AF513299 241_271_R | | 1122 |
| 2333 | GALE_AF513299_168_1 99_F | | 658 | GALE_AF513299_245_271__R | TCTCACCTACAGCTTTAAAGCCAGCAA | 1121 |
| 2334 | GALE_AF513299_168_1 99_F | | 658 | GALE_AF513299_233_264_R | | 883 |
| 2335 | GALE_AF513299_169_1 98_F | | 319 | GALE_AF513299_252_279_R | TTCAACACTCTCACCTACAGCTTTAAAG | 1374 |
| 2336 | PLA_AF053945_7371_7 403_F | | 680 | PLA_AF053945_7434_7468_R | | 900 |
| 2337 | PLA_AF053945_7377_7 403_F | | 443 | PLA_AF053945_7428_7455_R | TCCGCAAAGACTTTGGCATTAGGTGTGA | 1035 |
| 2338 | PLA_AF053945_7377_7 404_F | | 444 | PLA_AF053945_7430_7460_R | | 854 |
| 2339 | CAF_AF053947_33412_ 33441_F | | 329 | CAF_AF053947_33498_33523_ R | TAAGAGTGATGCGGGCTGGTTCAACA | 866 |
| 2340 | CAF_AF053947_33426_ 33458_F | | 499 | CAF_AF053947_33483_33507_ R | TGGTTCAACAAGAGTTGCCGTTGCA | 1308 |
| 2341 | CAF_AF053947_33407_ 33429_F | TCAGTTCCGTTATCGCCATTGCA | 291 | CAF_AF053947_33483_33504_ R | TTCAACAAGAGTTGCCGTTGCA | 1373 |
| 2342 | CAF_AF053947_33407_ 33431_F | TCAGTTCCGTTATCGCCATTGCATT | 293 | CAF_AF053947 33494_33517_ R | TGATGCGGGCTGGTTCAACAAGAG | 1184 |
| 2344 | GAPA_NC_002505_1_28 F_1 | | 260 | GAPA_NC_002505_29_5_R_1 | | 1060 |
| 2472 | OMPA_NC000117_68_89_ F | TGCCTGTAGGGAATCCTGCTGA | 507 | OMPA_NC00011_7_145_167_R | TCACACCAAGTAGTGCAAGGATC | 967 |
| 2473 | OMPA_NC000117_798_8 21_F | TGATTACCATGAGTGGCAAGCAAG | 475 | OMPA_NC000117_865_893_R | | 947 |
| 2474 | OMPA_NC000117_645_6 71_F | | 521 | OMPA_NC000117_757_777_R | TGTCGCAGCATCTGTTCCTGC | 1328 |
| 2475 | OMPA_NCO00117_947_9 73 F | | 157 | OMPA_NC000117_1011_1040_R | | 1153 |
| 2476 | OMPA_NC000117_774_7 95_F | TACTGGAACAAAGTCTGCGACC | 196 | OMPA_NC000117_871_894_R | TTCAAAAGTTGCTCGAGACCATTG | 1371 |
| 2477 | OMPA_NC000117_457_4 83_F | | 676 | OMPA_NC000117_511_534_R | TAAAGAGACGTTTGGTAGTTCATTTGC | 851 |
| 2478 | OMPA_NC000117_687_7 10_F | TAGCCCAGCACAATTTGTGATTCA | 212 | OMPA_NC000117_787_816_R | | 1406 |
| 2479 | OMPA_NC000117_540_5_ 66_F | | 571 | OMPA_NC000117_649_672_R | TTCTTGAACGCGAGGTTTCGATTG | 1395 |
| 2480 | OMPA_NC000117_338_3_ 60_F | TGCACGATGCGGAATGGTTCACA | 492 | OMPA_NC000117_417_444_R | TCCTTTAAAATAACCGCTAGTAGCTCCT | 1058 |
| 2481 | OMP2_NC000117_18_40 F | TATGACCAAACTCATCAGACGAG | 234 | OMP2_NC000117_71_91_R | TCCCGCTGGCAAATAAACTCG | 1025 |
| 2482 | OMP2_NC000117_359_3 82_F | | 516 | OMP2_NC000117_445_471_R | TGGATCACTGCTTACGAACTCAGCTTC | 1270 |
| 2483 | OMP2_NC000117_1297_ 1319_F | TGGAAAGGTGTTGCAGCTACTCA | 537 | OMP2_NC000117_1396_1419_R | TACGTTTGTATCTTCTGCAGAACC | 903 |
| 2484 | OMP2_NC000117_1465_ 1493_F | | 407 | OMP2_NC000117_1541_1569_R | | 1062 |
| 2485 | OMP2_NC000117_44_66_ F | TGACGATCTTCGCGGTGACTAGT | 450 | OMP2_NC000117_120_148_R | | 1323 |
| 2486 | OMP2_NC000117_166_1 90_F | | 441 | OMP2 NC000117 240 261 R | TTGACATCGTCCCTCTTCACAG | 1396 |
| 2487 | GYRA_NC000117_514_5 36_F | TCAGGCATTGCGGTTGGGATGGC | 287 | GYRA_NC000117_640_660_R | TGCTGTAGGGAAATCAGGGCC | 1251 |
| 2488 | GYRA_NC000117_801_8 27_F | | 636 | GYRA_NC000117_871_893_R | TTGTCAGACTCATCGCGAACATC | 1419 |
| 2489 | GYRA_NC002952_219_2 42_F | TGTCATGGGTAAATATCACCCTCACCCTCA | 632 | GYRA_NC002952_319_345_R | TCCATCCATAGAACCAAAGTTACCTTG | 1010 |
| 2490 | GYRA_NC002952_964_9 83_F | TACAAGCACTCCCAGCTGCA | 176 | GYRA_NC002952_1024_1041_R | TCGCAGCGTGCGTGGCAC | 1073 |
| 2491 | GYRA_NC002952_1505_ 1520_F | TCGCCCGCGAGGACGT | 366 | GYRA_NC002952_1546 1562_R | TTGGTGCGCTTGGCGTA | 1416 |
| 2492 | GYRA_NC002952_59_81_ F | TCAGCTACATCGACTATGCGATG | 279 | GYRA_NC002952_124_143_R | TGGCGATGCACTGGCTTGAG | 1279 |
| 2493 | GYRA_NC002952_216_2 39_F | TGACGTCATCGGTAAGTACCACCC | 452 | GYRA_NC002952_313_333_R | TCCGAAGTTGCCCTGGCCGTC | 1032 |
| 2494 | GYRA_NC002952_219_2_ 42_2_F | TGTACTCGGTAAGTATCACCCGCA | 625 | GYRA_NC002952_308_330_R | TAAGTTACCTTGCCCGTCAACCA | 873 |
| 2495 | GYRA_NC002952_115_1 41_F | | 453 | GYRA_NC00295_2_220 242_R | TGCGGGTGATACTTACCGAGTAC | 1236 |
| 2496 | GYRA_NC002952_517_5 39_F | TCAGGCATTGCGGTTGGGATGGC | 287 | GYRA_NC002952_643_663_R | TGCTGTAGGGAAATCAGGGCC | 1251 |
| 2497 | GYRA_NC002952_273_2 | TCGTATGGCTCAATGGTGGAG | 380 | GYRA_NC002952_338_360_R | TGCGGCAGCACTATCACCATCCA | 1234 |
| | 93_F | | | | | |
| 2498 | GYRA_NC000912_257_2 78_F | TGAGTAAGTTCCACCCGCACGG | 462 | GYRA NC000912 _46_370_R | TCGAGCCGAAGTTACCCTGTCCGTC | 1067 |
| 2504 | ARCC_NC003923_ 2725050_ 2724595_135_161P_F | | 229 | ARCC_NC0039232725050_ 2724595_214 239P_R | | 1116 |
| 2505 | PTA_NC003923_ 628885_ 629355_237 263P_F | | 417 | PTA_NC003923-628885-629355_314 342P_R | | 904 |
| 2517 | CJMLST_ST1_1852_188 3_F | | 708 | CJMLST_ST1 1945_1977_R | | 1355 |
| 2518 | CJMLST_ST1_2963_299 2_F | | 428 | CJMLST_ST1 3073_3097_R | TCCCCATCTCCGCAAAGCAATAAA | 1020 |
| 2519 | CJMLST_ST1_2350_237 8_F | | 535 | CJMLST_ST1 2447_2481_R | | 1117 |
| 2520 | CJMLST_ST1_654_684_ F | | 240 | CJMLST_ST1_725_756_R | | 1084 |
| 2521 | CJMLST_ST1_360_395_ F | | 347 | CJMLST_ST1_454_487_R | | 1245 |
| 2522 | CJMLST_ST1_1231_125 8_F | | 564 | CJMLST_ST1_1312_1340_R | | 1427 |
| 2523 | CJMLST_ST1_3543_357 4_F | | 529 | CJMLST_ST1 3656_3685 R | | 950 |
| 2524 | CJMLST ST1_1_17_F | | 145 | CJMLST_ST1_55_84_R | | 1348 |
| 2525 | CJMLST_ST1_1312_134 2_F | | 538 | CJMLST_ST1_1383_1417_R | | 1432 |
| 2526 | CJMLST_ST1_2254_228 6_F | | 582 | CJMLST_ST1_2352_2379_R | TCCAAACGATCTGCATCACCATCAAAAG | 996 |
| 2527 | CJMLST_ST1_1380_141 1_F | | 534 | CJMLST_ST1 1486 1520_R | | 1205 |
| 2528 | CJMLST_ST1_3413_343 7_F | TTGTAAATGCCGGTGCTTCAGATCC | 692 | CJMLST_ST1 3511_3542_R | | 1257 |
| 2529 | CJMLST_ST1_1130_115 6_F | | 189 | CJMLST_ST1_1203_1230_R | TAGGATGAGCATTATCAGGGAAAGAATC | 920 |
| 2530 | CJMLST_ST1_2 84 0_2 6_7 2_F | | 591 | CJMLST_ST1_2940_2973_R | | 917 |
| 2531 | CJMLST_ST1_2058_208 4_F | | 241 | CJMLST_ST1 2131_2162_R | | 1417 |
| 2532 | CJMLST_ST1_553_585_ F | | 344 | CJMLST_ST1_655_685_R | | 942 |
| 2564 | GLTA_NC002163_ 1604930_ 1604529_306 338_F | | 299 | GLTA_NC002163_1604930_ 1604529_352 380_R | | 1443 |
| 2565 | UNCA NC002163_112166-112647 80_113 F | TCCCCCACGCTTTAATTGTTTATGATGATTTGAG | 322 | UNCA NC002163-112166-112647_146_171_R | TCGACCTGGAGGACGACGTAAAATCA | 1065 |
| 2566 | UNCA_NC002163-112166-112647 233 259 F | | 170 | UNCA_NC002163-112166-112647 294 329 R | | 1285 |
| 2567 | PGM_NC002163-327773-328270 273 305 F | | 414 | PGM_NC002163-327773-328270 365 396 R | | 1012 |
| 2568 | TKT_NC002163-1569415-1569873 255 284 F | | 661 | TKT_NC002163-1569415-1569873 350 383 R | | 946 |
| 2570 | GLTA_NC002163-1604930-1604529 39 68 F | | 381 | GLTA_NC002163-1604930-1604529 109 142 R | | 1347 |
| 2571 | TKT_NC002163-1569415-1569903 33 62 F | | 472 | TKT_NC002163-1569415-1569903 139 162_R | TGCCATAGCAAAGCCTACAGCATT | 1214 |
| 2572 | TKT_NC002163-1569415-1569903 207 239 F | | 164 | TKT_NC002163-1569415-1569903 313 345 R | | 886 |
| 2573 | TKT_NC002163-1569415-1569903 350 383 F | | 213 | TKT_NC002163-1569415-1569903 449 481_R | | 865 |
| 2574 | TKT_NC002163-1569415-1569903 60 92 F | | 665 | TKT_NC002163-1569415-1569903 139 163 R | TTGCCATAGCAAAGCCTACAGCATT | 1405 |
| 2575 | GLTA_NC002163-1604930-1604529 39 70_F | | 382 | GLTA_NC002163-1604930-1604529 139 168 R | | 1216 |
| 2576 | GLYA_NC002163-367572-368079 386 414 F | | 281 | GLYA_NC002163-367572-368079 476 508 R | | 756 |
| 2577 | GLYA_NC002163-367572-368079 148 174 F | | 611 | GLYA_NC002163-367572-368079 242 270 R | | 1246 |
| 2578 | GLYA_NC002163-367572-368079 298 327 F | | 622 | GLYA_NC002163-367572-36B079 384 416 R | | 1381 |
| 2579 | GLYA_NC002163-367572-368079 1 27 F | | 614 | GLYA_NC002163-367572-368079 52 B1_R | | 961 |
| 2580 | PGM_NC002163-327746-328270 254 285 F | | 455 | PGM_NC002163-327746-328270 356 379 R | TTTGCTCTCCGCCAAAGTTTCCAC | 1438 |
| 2581 | PGM_NC002163-327746-328270_153_182_F | | 425 | PGM_NC002163-327746-328270 241 267 R | TGCCCCATTGCTCATGATAGTAGCTAC | 1219 |
| 2582 | PGM_NC002163-327746-328270_19_50_F | | 568 | PGM_NC002163-327746-328270_79_102_R | TGCACGCAAACGCTTTACTTCAGC | 1200 |
| 2583 | UNCA_NC002163-112166-112647_114_141_F | | 160 | UNCA_NC002163-112166-112647_196_225_R | | 1220 |
| 2584 | UNCA_NC002163-112166-112647_3_29_F | | 532 | UNCA_NC002163-112166-112647_88_123_R | | 1206 |
| 2585 | ASPA_NC002163-96692-97166_308 335_F | | 652 | ASPA_NC002163-96692-97166_403_432_R | | 1192 |
| 2586 | ASPA_NC002163-96692-97166_228 258_F | | 370 | ASPA_NC002163-96692-97166_316_346_R | | 991 |
| 2587 | GLNA_NC002163-6580is-657609_244_275_F | | 547 | GLNA_NC002163-658085-657609_340_371_R | | 1176 |
| 2588 | TKT_NC002163-1569415-1569903_107_130_F | TCGCTACAGGCCCTTTAGGACAAG | 371 | TKT_NC002163-1569415-1569903_212_236_R | TCCCCATCTCCGCAAAGACAATAAA | 1020 |
| 2589 | TKT_NC002163-1569415-1569903_265_296_F | | 642 | TKT_NC002163-1569415-1569903_361_393_R | | 1057 |
| 2590 | GLYA_NC002163-367572-368095_214_246_F | | 505 | GLYA_NC002163-367572-368095_317_340_R | TCCTCTTGGGCCACGCAAAGTTTT | 1047 |
| 2591 | GLYA_NC002163-367572-368095_415_444_F | | 353 | GLYA_NC002163-367572-368095_485_516_R | | 1141 |
| 2592 | PGM_NC002163_21_54_ F | | 332 | PGM NC002163 116 142 R | TCAAACGATCCGCATCACCATCAAAAG | 949 |
| 2593 | PGM_NC002163_149_17_ 6_F | | 207 | PGM_NC002163 247_277_R | | 1023 |
| 2594 | GLNA_NC002163-658085-657609_79_106_F | | 633 | GLNA_NC002163-658085-657609_148_179_R | | 945 |
| 2595 | ASPA_NC002163-96685-97196_367 402_F | | 347 | ASPA_NC002163-96685-97196_467_497_R | | 960 |
| 2596 | ASPA_NC002163-96685-97196 1_33_F | | 502 | ASPA_NC002163-96685-97196_95_127 R | | 880 |
| 2597 | ASPA_NC002163-96685-97196_85_117_F | | 540 | ASPA_NC002163-96685-97196_185_210_R | TAAGCTCCCGTATCTTGAGTCGCCTC | 872 |
| 2598 | PGM_NC002163_ 327746-328270_165_195_F | | 563 | PGM_MC002163-327746-328270_230_261_R | | 975 |
| 2599 | PGM_NC002163-327746-328270_252_286_F | | 593 | PGM_NC002163-327746-328270_353_381_R | | 1443 |
| 2600 | PGM_NC002163-327746-328270_1_30_F | | 577 | PGM_NC002163-327746-328270_95_123_R | | 1178 |
| 2601 | PGM_NC002163-327746-328270_220 250_F | | 146 | PGM_NC002163-327746-328270_314_345_R | | 963 |
| 2602 | UNCA_NC002163-112166-112647_123_152_F | | 628 | UNCA_NC002163-112166-112647_199_229_R | | 1258 |
| 2603 | UNCA_NC002163-112166-112647_333 365_F | | 313 | UNCA_NCO02163-112166-112647_430_461_R | | 1031 |
| 2734 | GYRA_AY291534_237_2 64_F | | 265 | GYRA_AY291534 268_288_R | TTGCGCCATACGTACCATCGT | 1407 |
| 2735 | GYRA_AY291534_224_2 52_F | | 167 | GYRA_AY291534 256_285_R | | 1213 |
| 2736 | GYRA_AY291534_170_1 98_F | | 221 | GYRA_AY291534 268_288_R | TTGCGCCATACGTACCATCGT | 1407 |
| 2737 | GYRA_AY291534_224_2 52_F | | 167 | GYRA_AY291534 319_346_R | TATCGACAGATCCAAAGTTACCATGCCC | 935 |
| 2738 | GYRA_NC002953_7005_ 9668_166_195_F | | 163 | GYRA_NC002953-7005-9668_265_287_R | TCTTGAGCCATACGTACCATTGC | 1142 |
| 2739 | GYPA_NC002953_7005_ 9668_221_249_F | | 171 | GYRA_NC002953-7005-9668_316_343_R | TATCCATTGAACCAAAGTTACCTTGGCC | 933 |
| 2740 | GYRA_NC002953_7005_ 9668_221_249_F | | 171 | GYRA_NC002953-7005-9668_253_283_R | | 912 |
| 2741 | GYRA_NC002953_7005_ 9668_234_261_F | | 264 | GYRA_NC002953-7005-9668_265_287_R | TCTTGAGCCATACGTACCATTGC | 1142 |
| 2842 | CAPC_AF188935-56074-55628_271_304_F | | 578 | CAPC_AF188935-56074-55628_348_378_R | | 1299 |
| 2843 | CAPC_AF188935-56074-55628_273_303P_F | | 476 | CAPC_{_}AF188935-56074-55628_349_377P_R | | 1314 |
| 2844 | CAPC_AF188935-56074-55628_268_303_F | | 331 | CAPC_AF188935-56074-55628_349_384_R | | 1344 |
| 2845 | CAPC AF188935- | | 331 | CAPC AF188935-56074- | TAACCCTTGTCTTTGAATTGTATTTGCA | 860 |
| | 56074-55628_268_303_F | GCCATTTGAG | | 55628_337_375_R | ATTAATCCTGG | |
| 2846 | PARC_X95819 33_58_F | TCCAAAAAAATCAGCGCGTACAGTGG | 302 | PARC_X95819_121_153_R | | 852 |
| 2847 | PARC_X95819 65_92_F | | 199 | PARC_X95819_157_178_P | TACCCCAGTTCCCCTGACCTTC | 889 |
| 2848 | PARC X95819_69_93_F | TGGTAAATACCACATGGTGAC | 596 | PARC_X95819_97 128_R | | 1169 |
| 2849 | PARC_NC003997-3362578-3365001_181_205_F | TTCCGTAAGTCGGCTAAAACAGTCG | 668 | PARC_NC003997-3362578-3365001_256_283_R | TCCAAGTTTGACTTAAACGTACCATCGC | 1001 |
| 2850 | PARC_NC003997-3362578-3365001_217_240_F | TGTAACTATCACCCGCACGGTGAT | 621 | PARC_NC003997-3362578-3365001_304_335_R | | 1099 |
| 2851 | PARC_NC003997-3362578-3365001_217 240_F | TGTAACTATCACCCGCACGGTGAT | 621 | PARC_NC003997-3362578-3365001_244_275_R | | 1162 |
| 2852 | GYRA_AY642140_ 1_24_F | TAAATCTGCCCGTGTCGTTGGTGAC | 150 | GYRA_AY642140 71_100_R | | 1242 |
| 2853 | GYRA_AY642140_26_54 F | | 166 | GYRA_AY642140_121_146_R | TCGATCGAACCGAAGTTACCCTGACC | 1069 |
| 2854 | GYRA_AY642140_26_54 F | | 166 | GYRA_AY642140 58_89_R | | 1168 |
| *2860* | CYA_AF065404_1348_1 379_F | | 305 | CYA_AF065404 1448_1472_R | TCAGCTGTTAACGGCTTCAAGACCC | 983 |
| 2861 | LEF_BA_AF065404_751 _781_F | | 354 | LEF_BA_AF065404_843_881_R | | 1144 |
| 2862 | LEF_BA_AF065404_762_ 788 F | | 498 | LEF_BA_AF065404_843_881_R | | 1144 |
| 2917 | MUTS_AY698802_106_1 25_F | TCCGCTGAATCTGTCGCCGC | 326 | MUTS AY698802 172_193_R | TGCGGTCTGGCGCGCATATAGGTA | 1237 |
| 2918 | MUTS_AY698802_172_1_ 92_F | TACCTATATGCGCCAGACCGC | 187 | MUTS_AY698802 228_252_R | TCAATCTCGACTTTTTGTGCCGGTA | 965 |
| 2919 | MUTS_AY698802_228_2 52_F | TACCGGCGCAAAAAGTCGAGATTGG | 186 | MUTS_AY698802_314_342_R | | 1097 |
| 2920 | MUTS_AY698802_315_3 42 F | | 419 | MUTS_AY698802 413_433_R | TGCCAGCGACAGACCATCGTA | 1210 |
| 2921 | MUTS_AY698802_394_4_ 11_F | TGGGCTGGAACGTCCAC | 585 | MUTS_AY698802_497_519_R | TCCGGTAACTGGGTCAGCTCGAA | 1040 |
| 2922 | AB_MLST-11-OIF007_991_1018_F | | 583 | AB_MLST-11-OIF007_1110_1137_R | TAGTATCACCACGTACACCCGGATCAGT | 923 |
| 2927 | GAPA_NC002505_694_7 21_F | | 259 | GAPA_NC_002505_29_58_R_1 | | 1060 |
| 2928 | GAPA_NC002505_694_7 21_2_F | | 361 | GAPA_NC002505_769_798_2_R | | 1061 |
| 2929 | GAPA_NC002505_694_7 21_2 F | | 361 | GAPA_NC002505_769_798_3_R | | 1059 |
| 2932 | INFB_EC_1364_1394_F | | 688 | INFB_EC_1439_1468_R | | 1410 |
| 2933 | INFB_EC_1364_1394_2 F | | 689 | INFB_EC_1439_1468_R | | 1410 |
| 2934 | INFB_EC_80_110_F | | 685 | INFB_EC_1439_1468_R | | 1410 |
| 2949 | ACS_NC002516_ 970624-971013_299_316_F | TCGGCGCCTGCCTGATGA | 376 | ACS_NC002516-970624-971013_364 383 R | TGGACCACGCCGAAGAACGG | 1265 |
| 2950 | ARO_NC002516-26883-27380_4_26_F | TCACCGTGCCGTTCAAGGAAGAG | 267 | ARO_NC002516-26883-27380_111_128 R | TGTGTTGTCGCCGCGCAG | 1341 |
| 2951 | ARO_NC002516_26883_ 27380_356 377_F | TTTCGAAGGGCCTTTCGACCTG | 705 | ARO_NC002516-26883-27380_459_484_R | TCCTTGGCATACATCATGTCGTAGCA | 1056 |
| 2952 | GUA_NC002516-4226546-4226174_23_41_F | TGGACTCCTCGGTGGTCGC | 551 | GUA_NC002516-4226546-4226174_127_146_R | TCGGCGAACATGGCCATCAC | 1091 |
| 2953 | GUA_NC002516-4226546-4226174_120_142_F | TGACCAGGTGATGGCCATGTTCG | 448 | GUA_NC002516-4226546-4226174_214_233_R | TGCTTCTCTTCCGGGTCGGC | 1256 |
| 2954 | GUA_NC002516-4226546-4226174_155_178_F | TTTTGAAGGTGATCCGTGCCAACG | 710 | GUA_NC002516-4226546-4226174_265_287_R | TGCTTGGTGGCTTCTTCGTCGAA | 1259 |
| 2955 | GUA_NC002516-4226546-4226174_190_206_F | TTCCTCGGCCGCCTGGC | 670 | GUA_NC002516-4226546-4226174_288_309 R | TGCGAGGAACTTCACGTCCTGC | 1229 |
| 2956 | GUA_NC002516-4226546-4226174_242_263_F | TCGGCCGCACCTTCATCGAAGT | 374 | GUA_NC002516-4226546-4226174_355 371 R | TCGTGGGCCTTGCCGGT | 1111 |
| 2957 | MUT_NC002516-5551158-5550717_5_26_F | TGGAAGTCATCAAGCGCCTGGC | 545 | MUT_NC002516-5551158-5550717_99_116_R | TCACGGGCCAGCTCGTCT | 978 |
| 2958 | MUT_NC002516-5551158-5550717_152_168_F | TCGAGCAGGCGCTGCCG | 358 | MUT_{_}NC002516-5551158-5550717_256_277_R | TCACCATGCGCCCGTTCACATA | 971 |
| 2959 | NUO_NC002516-2984589-2984954_8_26_F | TCAACCTCGGCCCGAACCA | 249 | NUO_NC002516-2984589-2984954_97_117_R | TCGGTGGTGGTAGCCGATCTC | 1095 |
| 2960 | NUO_NC002516-2984589-2984954_218_239_F | TACTCTCGGTGGAGAAGCTCGC | 195 | NUO_NC002516-2984589-2984954_301_326_R | TTCAGGTACAGCAGGTGGTTCAGGAT | 1376 |
| 2961 | PPS_NC002516-1915014-1915383_44_63_F | TCCACGGTCATGGAGCGCTA | 311 | PPS_NC002516-1915014-1915383_140_165_R | TCCATTTCCGACACGTCGTTGATCAC | 1014 |
| 2962 | PPS_NC002516-1915014-1915383 240 258 F | TCGCCATCGTCACCAACCG | 365 | PPS_NC002516-1915014-1915363_341 360 R | TCCTGGCCATCCTGCAGGAT | 1052 |
| 2963 | TRP_NC002516-671831-672273_24_42 F | TGCTGGTACGGGTCGAGGA | 527 | TRP_NC002516-671831-672273 131 150 R | TCGATCTCCTTGGCGTCCGA | 1071 |
| 2964 | TRP_NC002516-671831-672273_261_282_F | TGCACATCGTGTCCAACGTCAC | 490 | TRP_NC002516-671831-672273 362 383 R | TGATCTCCATGGCGCGGATCTT | 1182 |
| 2972 | AB_MLST-11-OIF007_1007_1034_F | | 592 | AB_MLST-11-OIF007_1126_1153_R | TAGTATCACCACGTACICCIGGATCAGT | 924 |
| 2993 | OMPU_NC002505-674828-675880_428_455_F | | 667 | OMPU C002505_544_567_R | TCGGTCAGCAAAACGGTAGCTTGC | 1094 |
| 2994 | GAPA_NC002505-506780-507937_691_721_F | | 335 | GAPA_{_}NC002505-506780-507937_769_802_R | | 1442 |
| 2995 | GAPA_NC002505-506780-507937_691_721_2_F | | 339 | GAPA_NC002505-506780-507937_769_803_R | | 1008 |
| 2996 | GAPA_NC002505-506780-507937_692 721_F | | 396 | GAPA_NC002505-506780-507937_785_817_R | | 1085 |
| 2997 | GAPA_NC002505-506780-507937_691_721_3_F | | 337 | GAPA_NC002505-506780-507937_785_817_R | | 1085 |
| 2998 | GAPA_NC002505-506780-507937_691_721_4_F | | 336 | GAPA_NC002505-506780-507937_784_817_R | | 1087 |
| 2999 | GAPA_NC002505-506780-507937_691 721_5_F | | 340 | GAPA_NC002505-506780-507937_784_817_2_R | | 1086 |
| 3000 | GAPA_NC002505-506780-507937_691_721_6_F | | 338 | GAPA_NC002505-506780-507937 769 805 R | | 1430 |
| 3001 | CTXB_NC002505-1566967-1567341_46_71_F | TCAGCATATGCACATGGAACACCTCA | 275 | CTXB_NC002505-1566967-1567341_139_63_R | TCCCGGCTAGAGATTCTGTATACGA | 1026 |
| 3002 | CTXB_NC002505-1566967-1567341_46 70_F | TCAGCATATGCACATGGAACACCTC | 274 | CTXB_NC002505-1566967-1567341_132 162 R | TCCGGCTAGAGATTCTGTATACGAAAAT ATC | 1038 |
| 3003 | CTXB_NC002505-1566967-1567341_46_70_F | TCAGCATATGCACATGGAACACCTC | 274 | CTXB_NC002505-1566967-1567341_118_150_R | | 1225 |
| 3004 | TUFB_NC002758-615038-616222_684_704_F | TACAGGCCGTGTTGAACGTGG | 180 | TUFB_NC002758-615038-616222_778_809_R | | 982 |
| 3005 | TUFB_NC002756-615038-616222_688 710_F | TGCCGTGTTGAACGTGGTCAAAT | 503 | TUFB_NC002758-615038-616222_783_813_R | | 1255 |
| 3006 | TUFB_NC002758-615038-616222_700_726_F | | 638 | TUFB_NC002758-615038-616222_778_807_R | | 1238 |
| 3007 | TUFB_NC002758-615038-616222_702 726_F | TGGTCAAATCAAAGTTGGTGRAGAA | 607 | TUFB_NC002758-615038-616222_778_807_R | | 1238 |
| 3008 | TUFB_NC002758-615038-616222_696_726_F | | 431 | TUFB_NC002758-615038-616222_785_818_R | | 970 |
| 3009 | TUFB_NC002758-615038-616222_690 716_F | | 386 | TUFB_NC002758-615038-616222_778_812_R | | 1134 |
| 3010 | MECI-R_NC003923-41798-41609_36_59_F | TCACATATCGTGAGCMTGAACTG | 261 | MECI-R_NC003923-41798-41609_89_112_R | TGTGATATGGAGGTGTAGAAGGTG | 1332 |
| 3011 | MECI-R_NC003923-41798-41609_40_66_F | | 584 | MECI-R_NC003923-41798-41609_81_110_R | | 1287 |
| 3012 | MECI-R_NC003923-41798-41609_33_60 2_F | | 549 | MECI-R_NC003923-41798-41609_81_110_R | | 1286 |
| 3013 | MECI-R_NC003923-41798-41609_29_60_F | | 595 | MECI-R_NC003923-41798-41609_81_113_R | | 1290 |
| 3014 | MUPR_X75439_2490_25_ 14_F | TGGGCTCTTTCTCGCTTAAACACT | 587 | MUPR X75439_2548_2570_R | TCTGGCTGCGGAAGTGAAATCGT | 1130 |
| 3015 | MUPR_X75439_2490_25 13_F | TGGGCTCTTTCTCGCTTAAACACC | 586 | MUPR X75439 2547_2568_R | TGGCTGCGGAAGTGAAATCGTA | 1281 |
| 3016 | MUPR_X75439_2482_25_ 10_F | | 205 | MUPR X75439 2551_2573_R | TAATCTGGCTGCGGAAGTGAAAT | 876 |
| 3017 | MUPR_X75439_2490_25 14_F | TGGGCTCTTTCTCGCTTAAACACCT | 587 | MUPR X75439 2549_2573_R | TAATCTGGCTGCGGAAGTGAAATCG | 877 |
| 3018 | MUPR_X75439_2482_25_ 10_F | | 205 | MUPR_75439_2559_2589_R | | 1303 |
| 3019 | MUPR_X75439_2490_25 14_F | TGGGCTCTTTCTCGCTTAAACACCT | 587 | MUPR X75439_2554_2581_R | TCGTTAATTAATCTGGCTGCGGAAGTGA | 1112 |
| 3020 | AROE_NC003923-1674726-1674277_204_232_F | | 474 | AROE_NC003923-1674726-1674277_309_335_R | TAAGCAATACCTTTACTTGCACCACCT | 868 |
| 3021 | AROE_NC003923-1674726-1674277_207_232_F | TGGCGAGTGGATAGGGTATAATACAG | 570 | AROE_NC003923-1674726-1674277_311_339_R | | 1378 |
| 3022 | AROE_NC003923-1674726-1674277_207_232P_F | | 572 | AROE_NC003923-1674726-1674277_311_335P_R | | 867 |
| 3023 | ARCC_NC003923-2725050-2724595 124 155_F | | 398 | ARCC_NC003923-2725050-2724595_214 245 R | | 1137 |
| 3024 | ARCC_NC003923-2725050-2724595_131_161_F | | 437 | ARCC_NC003923-2725050-2724595_212_242_R | | 1139 |
| 3025 | ARCC_NC003923-2725050-2724595_131_161_F | | 437 | ARCC_NC003923-2725050-2724595_232_260_R | | 1232 |
| 3026 | PTA_NC003923-628885-629355_231 259_F | | 177 | PTA_NC003923-628885-629355_322_351_R | | 1350 |
| 3027 | PTA_NC003923-628885-629355_231 259_F | | 177 | PTA_NC003923-628885-629355_314_345_R | | 1301 |
| 3028 | PTA_NC003923-628885-629355_237 263_F | | 418 | PTA_NC003923-628885-629355_322_351_R | | 1350 |

Primer pair name codes and reference sequences are shown in Table 3. The primer name code typically represents the gene to which the given primer pair is targeted. The primer pair name may include specific coordinates with respect to a reference sequence defined by an extraction of a section of sequence or defined by a GenBank gi number, or the corresponding complementary sequence of the extraction, or the entire GenBank gi number as indicated by the label "no extraction." Where "no extraction" is indicated for a reference sequence, the coordinates of a primer pair named to the reference sequence are with respect to the GenBank gi listing. Gene abbreviations are shown in bold type in the "Gene Name" column.

To determine the exact primer hybridization coordinates of a given pair of primers on a given bioagent nucleic acid sequence and to determine the sequences, molecular masses and base compositions of an amplification product to be obtained upon amplification of nucleic acid of a known bioagent with known sequence information in the region of interest with a given pair of primers, one with ordinary skill in bioinformatics is capable of obtaining alignments of the primers of the present invention with the GenBank gi number of the relevant nucleic acid sequence of the known bioagent. For example, the reference sequence GenBank gi numbers (Table 3) provide the identities of the sequences which can be obtained from GenBank. Alignments can be done using a bioinformatics tool such as BLASTn provided to the public by NCBI (Bethesda, MD). Alternatively, a relevant GenBank sequence may be downloaded and imported into custom programmed or commercially available bioinformatics programs wherein the alignment can be carried out to determine the primer hybridization coordinates and the sequences, molecular masses and base compositions of the amplification product. For example, to obtain the hybridization coordinates of primer pair number 2095 (SEQ ID NOs: 456:1261), First the forward primer (SEQ ID NO: 456) is subjected to a BLASTn search on the publicly available NCBI BLAST website. "RefSeq_Genomic" is chosen as the BLAST database since the gi numbers refer to genomic sequences. The BLAST query is then performed. Among the top results returned is a match to GenBank gi number 21281729 (Accession Number NC_003923). The result shown below, indicates that the forward primer hybridizes to positions 1530282..1530307 of the genomic sequence of *Staphylococcus aureus* subsp. aureus MW2 (represented by gi number 21281729).

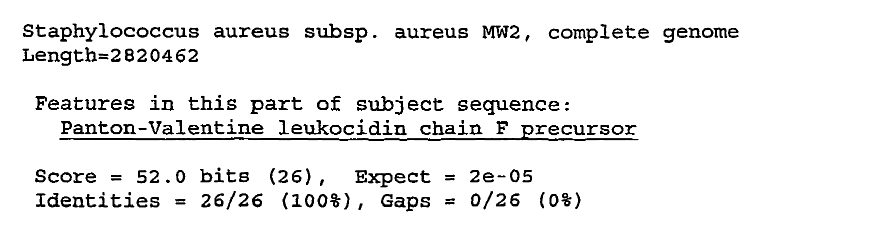

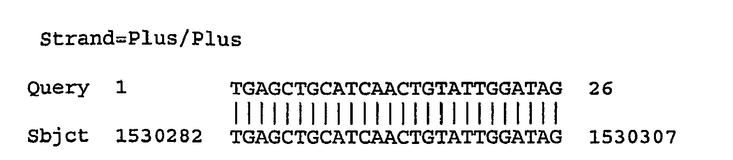

The hybridization coordinates of the reverse primer (SEQ ID NO: 1261) can be determined in a similar manner and thus, the bioagent identifying amplicon can be defined in terms of genomic coordinates. The query/subject arrangement of the result would be presented in Strand = Plus/Minus format because the reverse strand hybridizes to the reverse complement of the genomic sequence. HThe preceding sequence analyses are well known to one with ordinary skill in bioinformatics and thus, Table 3 contains sufficient information to determine the primer hybridization coordinates of any of the primers of Table 2 to the applicable reference sequences described therein.

**Table 3: Primer Name Codes and Reference Sequence**

| **Primer name code** | **Gene Name** | **Organism** | **Reference GenBank gi number** |
|---|---|---|---|
| 16S EC | 16S rRNA (16S ribosomal RNA gene) | *Escherichia coli* | 16127994 |
| 23S EC | 23S rRNA (23S ribosomal RNA gene) | Escherichia *coli* | 16127994 |
| CAPC BA | capC (capsule biosynthesis gene) | *Bacillus anthracis* | 6470151 |
| CYA BA | cya (cyclic AMP gene) | *Bacillus anthracis* | 4894216 |
| DNAK EC | dnaK (chaperone dnaK gene) dnaK (chaperone | *Escherichia coli* | 16127994 |
| GROL EC | groL (chaperonin groL) | *Escherichia coli* | 16127994 |
| HFLB EC | hflb (cell division protein peptidase ftsH) | *Escherichia coli* | 16127994 |
| INFB EC | infB (protein chain initiation factor infB gene) | *Escherichia coli* | 16127994 |
| LEF BA | lef (lethal factor) | *Bacillus anthracis* | 21392688 |
| PAG BA | pag (protective antigen) | *Bacillus anthracis* | 21392688 |
| RPLB EC | rplB (50S ribosomal protein L2) | *Escherichia coli* | 16127994 |
| RPOB EC | rpoB (DNA-directed RNA polymerase beta chain) | *Escherichia coli* | 6127994 |
| RPOC EC | rpoC (DNA-directed RNA polymerase beta' chain) | *Escherichia coli* | 16127994 |
| SP101ET_SPET_11 | Artificial Sequence Concatenation comprising: | Artificial Sequence* -partial gene sequences of *Streptococcus pyogenes* | 15674250 |
| | gki (glucose kinase) | | |
| | gtr (glutamine transporter protein) | | |
| | murI (glutamate racemase) | | |
| | mutS (DNA mismatch repair protein) | | |
| | xpt (xanthine phosphoribosyl transferase) | | |
| | yqiL (acetyl-CoA-acetyl transferase) | | |
| | tkt (transketolase) | | |
| SSPE BA | sspE (small acid-soluble spore protein) | *Bacillus anthracis* | 30253828 |
| TUFB_EC | tufB (Elongation factor Tu) | *Escherichia coli* | 16127994 |
| VALS_EC | valS (Valyl-tRNA synthetase) | *Escherichia* coli | 16127994 |
| ASPS_EC | aspS (Aspartyl-tRNA synthetase) | *Escherichia coli* | 16127994 |
| CAF1_AF053947 | caf1 (capsular protein caf1) | *Yersinia pestis* | 2996286 |
| INV_U22457 | inv (invasin) | *Yersinia pestis* | 1256565 |
| LL_NC003143 | *Y. pestis* specific chromosomal genes -difference region | *Yersinia pestis* | 16120353 |
| BONTA_X52066 | BoNT/A (neurotoxin type A) | *Clostridium botulinum* | 40381 |
| MECA_Y14051 | mecA methicillin resistance gene | *Staphylococcus aureus* | 2791983 |
| TRPE_AY094355 | trpE (anthranilate synthase (large component)) | *Acinetobacter baumanii* | 20853695 |
| RECA_AF251469 | recA (recombinase A) | *Acinetobacter baumani*i | 9965210 |
| GYRA_AF100557 | gyrA (DNA gyrase subunit A) | *Acinetobacter baumanii* | 4240540 |
| GYRB_AB008700 | gyrB (DNA gyrase subunit B) | *Acinetobacter baumanii* | 4514436 |
| WAAA_Z96925 | waaA (3-deoxy-D-manno-octulosonic-acid transferase) | *Acinetobacter baumanii* | 2765828 |
| CJST_CJ | Artificial Sequence Concatenation comprising: | Artificial Sequence* -partial gene sequences of *Campylobacter jejuni* | 15791399 |
| | tkt (transketolase) | | |
| | glyA (serine hydroxymethyltransferase) | | |
| | gltA (citrate synthase) | | |
| | aspA (aspartate ammonia lyase) | | |
| | glnA (glutamine synthase) | | |
| | pgm (phosphoglycerate mutase) | | |
| | uncA (ATP synthetase alpha chain) | | |
| RNASEP_BDP | RNase P (ribonuclease P) | *Bordetella pertussis* | 33591275 |
| RNASEP_BKM | RNase P (ribonuclease P) | *Burkholderia mallei* | 53723370 |
| RNASEP_BS | RNase P (ribonuclease P) | *Bacillus subtilis* | 16077068 |
| RNASEP_CLB | RNase P (ribonuclease P) | *Clostridium perfringens* | 18308982 |
| RNASEP_EC | RNase P (ribonuclease P) | *Escherichia coli* | 16127994 |
| RNASEP_RKP | RNase P (ribonuclease P) | *Rickettsia prowazekii* | 15603881 |
| RNASEP_SA | RNase P (ribonuclease P) | *Staphylococcus aureus* | 15922990 |
| RNASEP_VBC | RNase P (ribonuclease P) | *vibrio cholerae* | 15640032 |
| ICD_CXB | icd (isocitrate dehydrogenase) | *Coxiella burnetii* | 29732244 |
| IS1111A | multi-locus IS1111A insertion element | *Acinetobacter baumannii* | 29732244 |
| OMPA AY485227 | ompA (outer membrane protein A) | *Rickettsia prowazekii* | 40287451 |
| OMPB RKP | ompB (outer membrane protein B) | *Rickettsia prowazekii* | 15603881 |
| GLTA RKP | gltA (citrate synthase) | *Vibrio cholerae* | 15603881 |
| TOXR_VBC | toxR (transcription regulator toxR) | *Francisella tularensis* | 15640032 |
| ASD FRT | asd (Aspartate semialdehyde dehydrogenase) | *Francisella tularensis* | 56707187 |
| GALE FRT | galE (UDP-glucose 4-epimerase) | *Shigella flexne*ri | 56707187 |
| IPAH SGF | ipaH (invasion plasmid antigen) | *Campylobacter* jejuni | 30061571 |
| HUPB CJ | hupB (DNA-binding protein Hu-beta) | *Coxiella barnetii* | 15791399 |
| AB_MLST | Artificial Sequence Concatenation comprising: trpE (anthranilate synthase component | Artificial Sequence* -partial gene | Sequenced in-house (SEQ ID NO: 1444) |
| | I)) | sequences of Acinetobacter *baumannii* | |
| | adk (adenylate kinase) | | |
| | mutY (adenine glycosylase) | | |
| | fumC (fumarate hydratase) | | |
| | efp (elongation factor p) | | |
| | ppa (pyrophosphate phospho-hydratase | | |
| MUPR X75439 | mupR (mupriocin resistance gene) | *Staphylococcus* aureus | 438226 |
| PARC X95819 | parc (topoisomerase IV) | *Acinetobacter baumannii* | 1212748 |
| SED M28521 | sed (enterotoxin D) | *Staphylococcus aureus* | 1492109 |
| PLA_AF053945 | pla (plasminogen activator) | *Yersinia pestis* | 2996216 |
| SEJ_AF053140 | sej (enterotoxin J) | *Staphylococcus aureus* | 3372540 |
| GYRA_NC000912 | gyrA (DNA gyrase subunit A) | *Mycoplasma pneumoniae* | 13507739 |
| ACS_NC002516 | acsA (Acetyl CoA Synthase) | *Pseudomonas aeruginosa* | 15595198 |
| ARO_NC002516 | aroE (shikimate 5-dehydrogenase | *Pseudomonas aeruginosa* | 15595198 |
| GUA_NC002516 | guaA (GMP synthase) | *Pseudomonas aeruginosa* | 15595198 |
| MUT_NC002516 | mutL (DNA mismatch repair protein) | *Pseudomonas aeruginosa* | 15595198 |
| NUO_NC002516 | nuoD (NADH dehydrogenase I chain C, D) | *Pseudomonas aeruginosa* | 15595198 |
| PPS_NC002516 | ppsA (Phosphoenolpyruvate synthase) | *Pseudomonas aeruginosa* | 15595198 |
| TRP_NC002516 | trpE (Anthranilate synthetase component I) | *Pseudomonas aeruginosa* | 15595198 |
| OMP2_NC000117 | ompB (outer membrane protein B) | *Chlamydia trachomatis* | 15604717 |
| OMPA_NC000117 | ompA (outer membrane protein B) | *Chlamydia trachomatis* | 15604717 |
| GYRA_NC000117 | gyrA (DNA gyrase subunit A) | *Chlamydia trachomatis* | 15604717 |
| CTXA_NC002505 | ctxA (Cholera toxin A subunit) | *vibrio cholerae* | 15640032 |
| CTXB_NC002505 | ctxB (Cholera toxin B subunit) | *vibrio cholerae* | 15640032 |
| FUR_NC002505 | fur (ferric uptake regulator protein) | *Vibrio cholerae* | 15640032 |
| GAPA_NC_002505 | gapA (glyceraldehyde-3-phosphate dehydrogenase) | *Vibrio cholerae* | 15640032 |
| GYRB_NC002505 | gyrB (DNA gyrase subunit B) | *Vibrio cholerae* | 15640032 |
| OMPU_NC002505 | ompU (outer membrane protein) | *Vibrio cholerae* | 15640032 |
| TCPA_NC002505 | tcpA (toxin-coregulated pilus) | *Vibrio cholerae* | 15640032 |
| ASPA_NC002163 | aspA (aspartate ammonia lyase) | *Campylobacter jejuni* | 15791399 |
| GLNA_NC002163 | glnA (glutamine synthetase) | *Campylobacter jejuni* | 15791399 |
| GLTA_NC002163 | gltA (glutamate synthase) | *Campylobacter jejuni* | 15791399 |
| GLYA_NC002163 | glyA (seine hydroxymethyltransferase) | *Campylobacter jejuni* | 15791399 |
| PGM_NC002163 | pgm (phosphoglyceromutase) | *Campylobacter jejuni* | 15791399 |
| TKT_NC002163 | tkt (transketolase) | *Campylobacter jejuni* | 15791399 |
| UNCA_NC002163 | uncA (ATP synthetase alpha chain) | *Campylobacter jejuni* | 15791399 |
| AGR-III_NC003923 | agr-III (accessory gene regulator-III) | *Staphylococcus aureus* | 21281729 |
| ARCC_NC003923 | arcC (carbamate kinase) | *Staphylococcus aureus* | 21281729 |
| AROE_NC003923 | aroE (shikimate 5-dehydrogenase | *Staphylococcus* | 21281729 |
| | | *aureus* | |
| BSA-A NC003923 | bsa-a (glutathione peroxidase) | *Staphylococcus aureus* | 21281729 |
| BSA-B NC003923 | bsa-b (epidermin biosynthesis protein EpiB) | *Staphylococcus aureus* | 21281729 |
| GLPF NC003923 | glpF (glycerol transporter) | Staphylococcus *aureus* | 21281729 |
| GMK NC003923 | gmk (guanylate kinase) | *Staphylococcus aureus* | 21281729 |
| MECI-R NC003923 | mecR1 (truncated methicillin resistance protein) | *Staphylococcus aureus* | 21281729 |
| PTA NC003923 | pta (phosphate acetyltransferase) | *Staphylococcus aureus* | 21281729 |
| PVLUK NC003923 | pvluk (Panton-Valentine leukocidin chain F precursor) | *Staphylococcus aureus* | 21281729 |
| SA442 NC003923 | sa442 gene | *Staphylococcus aureus* | 21281729 |
| SEA NC003923 | sea (staphylococcal enterotoxin A precursor) | *Staphylococcus aureus* | 21281729 |
| SEC NC003923 | sec4 (enterotoxin type C precursor) | *Staphylococcus aureus* | 21281729 |
| TPI NC003923 | tpi (triosephosphate isomerase) | *Staphylococcus aureus* | 21281729 |
| YQI NC003923 | yqi (acetyl-CoA C-acetyltransferase homologue) | *Staphylococcus aureus* | 21281729 |
| GALE AF513299 | galE (galactose epimerase) | *Francisella tularensis* | 23506418 |
| VVHA NC004460 | vVhA (cytotoxin, cytolysin precursor) | *vibrio vulnificus* | 27366463 |
| TDH NC004605 | tdh (thermostable direct hemolysin A) | *vibrio parahaemolyticus* | 28899855 |
| AGR-II NC002745 | agr-II (accessory gene regulator-II) | *Staphylococcus aureus* | 29165615 |
| PARC NC003997 | parC (topoisomerase IV) | *Bacillus anthracis* | 30260195 |
| GYRA AY291534 | gyrA (DNA gyrase subunit A) | *Bacillus* anthracis | 31323274 |
| AGR-1 AJ617706 | agr-I (accessory gene regulator-I) | *Staphylococcus aureus* | 46019543 |
| AGR-IV AJ617711 | agr-IV (accessory gene regulator-III) | *Staphylococcus aureus* | 46019563 |
| BLAZ NC002952 | blaZ (beta lactamase III) | *Staphylococcus aureus* | 49482253 |
| ERMA NC002952 | ermA (rRNA methyltransferase A) | *Staphylococcus aureus* | 49482253 |
| ERMB Y13600 | ermB (rRNA methyltransferase B) | *Staphylococcus aureus* | 49482253 |
| SEA-SEE NC002952 | sea (staphylococcal enterotoxin A precursor) | *Staphylococcus aureus* | 49482253 |
| SEA-SEE NC002952 | sea (staphylococcal enterotoxin A precursor) | *Staphylococcus aureus* | 49482253 |
| SEE NC002952 | sea (staphylococcal enterotoxin A precursor) | *Staphylococcus aureus* | 49482253 |
| SEH NC002953 | seh (staphylococcal enterotoxin H) | *Staphylococcus aureus* | 49484912 |
| ERMC NC005908 | ermC (rRNA methyltransferase C) | *Staphylococcus aureus* | 49489772 |
| MUTS AY698802 | mutS (DNA mismatch repair protein) | *Shigella boydii* | 52698233 |
| NUC NC002758 | nuc (staphylococcal nuclease) | *Staphylococcus aureus* | 57634611 |
| SEB NC002758 | seb (enterotoxin type B precursor) | *Staphylococcus aureus* | 57634611 |
| SEG NC002758 | seg (staphylococcal enterotoxin G) | *Staphylococcus aureus* | 57634611 |
| SEI NC002758 | sei (staphylococcal enterotoxin I) | *Staphylococcus aureus* | 57634611 |
| TSST NC002758 | tsst (toxic shock syndrome toxin-1) | *Staphylococcus aureus* | 57634611 |
| TUFB NC002758 | tufB (Elongation factor Tu) | *Staphylococcus aureus* | 57634611 |

Note: artificial reference sequences represent concatenations of partial gene extractions from the indicated reference gi number. Partial sequences were used to create the concatenated sequence because complete gene sequences were not necessary for primer design.

### Example 2: Sample Preparation and PCR

Genomic DNA was prepared from samples using the DNeasy Tissue Kit (Qiagen, Valencia, CA) according to the manufacturer's protocols.

All PCR reactions were assembled in 50 µL reaction volumes in a 96-well microtiter plate format using a Packard MPII liquid handling robotic platform and M.J. Dyad thermocyclers (MJ research, Waltham, MA) or Eppendorf Mastercycler thermocyclers (Eppendorf, Westbury, NY). The PCR reaction mixture consisted of 4 units of Amplitaq Gold, 1x buffer II (Applied Biosystems, Foster City, CA), 1.5 mM MgCl₂, 0.4 M betaine, 800 µM dNTP mixture and 250 nM of each primer. The following typical PCR conditions were used: 95°C for 10 min followed by 8 cycles of 95°C for 30 seconds, 48°C for 30 seconds, and 72°C 30 seconds with the 48°C annealing temperature increasing 0.9°C with each of the eight cycles. The PCR was then continued for 37 additional cycles of 95°C for 15 seconds, 56°C for 20 seconds, and 72°C 20 seconds.

### Example 3: Purification of PCR Products for Mass Spectrometry with Ion Exchange Resin-Magnetic Beads

For solution capture of nucleic acids with ion exchange resin linked to magnetic beads, 25 µl of a 2.5 mg/mL suspension of BioClone amine terminated superparamagnetic beads were added to 25 to 50 µl of a PCR (or RT-PCR) reaction containing approximately 10 pM of a typical PCR amplification product. The above suspension was mixed for approximately 5 minutes by vortexing or pipetting, after which the liquid was removed after using a magnetic separator. The beads containing bound PCR amplification product were then washed three times with 50mM ammonium bicarbonate/50% MeOH or 100mM ammonium bicarbonate/50% MeOH, followed by three more washes with 50% MeOH. The bound PCR amplicon was eluted with a solution of 25mM piperidine, 25mM imidazole, 35% MeOH which included peptide calibration standards.

### Example 4: Mass Spectrometry and Base Composition Analysis

The ESI-FTICR mass spectrometer is based on a Bruker Daltonics (Billerica, MA) Apex II 70e electrospray ionization Fourier transform ion cyclotron resonance mass spectrometer that employs an actively shielded 7 Tesla superconducting magnet. The active shielding constrains the majority of the fringing magnetic field from the superconducting magnet to a relatively small volume. Thus, components that might be adversely affected by stray magnetic fields, such as CRT monitors, robotic components, and other electronics, can operate in close proximity to the FTICR spectrometer. All aspects of pulse sequence control and data acquisition were performed on a 600 MHz. Pentium II data station running Bruker's Xmass software under Windows NT 4.0 operating system. Sample aliquots, typically 15 µl, were extracted directly from 96-well microtiter plates using a CTC HTS PAL autosampler (LEAP Technologies, Carrboro, NC) triggered by the FRIAR data station. Samples were injected directly into a 10 µl sample loop integrated with a fluidics handling system that supplies the 100 µl /hr flow rate to the ESI source. Ions were formed via electrospray ionization in a modified Analytica (Branford, CT) source employing an off axis, grounded electrospray probe positioned approximately 1.5 cm from the metalized terminus of a glass desolvation capillary. The atmospheric pressure end of the glass capillary was biased at 6000 V relative to the ESI needle during data acquisition. A countercurrent flow of dry N₂ was employed to assist in the desolvation process. Ions were accumulated in an external ion reservoir comprised of an rf-only hexapole, a skimmer cone, and an auxiliary gate electrode, prior to injection into the trapped ion cell where they were mass analyzed. Ionization duty cycles greater than 99% were achieved by simultaneously accumulating ions in the external ion reservoir during ion detection. Each detection event consisted of 1M data points digitized over 2.3 s. To improve the signal-to-noise ratio (S/N), 32 scans were co-added for a total data acquisition time of 74 s.

The ESI-TOF mass spectrometer is based on a Bruker Daltonics MicroTOF™. Ions from the ESI source undergo orthogonal ion extraction and are focused in a reflectron prior to detection. The TOF and FTICR are equipped with the same automated sample handling and fluidics described above. Ions are formed in the standard MicroTOF™ ESI source that is equipped with the same off-axis sprayer and glass capillary as the FTICR ESI source. Consequently, source conditions were the same as those described above. External ion accumulation was also employed to improve ionization duty cycle during data acquisition. Each detection event on the TOF was comprised of 75,000 data points digitized over 75 µs.

The sample delivery scheme allows sample aliquots to be rapidly injected into the electrospray source at high flow rate and subsequently be electrosprayed at a much lower flow rate for improved ESI sensitivity. Prior to injecting a sample, a bolus of buffer was injected at a high flow rate to rinse the transfer line and spray needle to avoid sample contamination/carryover. Following the rinse step, the autosampler injected the next sample and the flow rate was switched to low flow. Following a brief equilibration delay, data acquisition commenced. As spectra were co-added, the autosampler continued rinsing the syringe and picking up buffer to rinse the injector and sample transfer line. In general, two syringe rinses and one injector rinse were required to minimize sample carryover. During a routine screening protocol a new sample mixture was injected every 106 seconds. More recently a fast wash station for the syringe needle has been implemented which, when combined with shorter acquisition times, facilitates the acquisition of mass spectra at a rate of just under one spectrum/minute.

Raw mass spectra were post-calibrated with an internal mass standard and deconvoluted to monoisotopic molecular masses. Unambiguous base compositions were derived from the exact mass measurements of the complementary single-stranded oligonucleotides. Quantitative results are obtained by comparing the peak heights with an internal PCR calibration standard present in every PCR well at 500 molecules per well. Calibration methods are commonly owned and disclosed in U.S. Provisional Patent Application Serial No. 60/545,425.

### Example 5: De Novo Determination of Base Composition of Amplification Products using Molecular Mass Modified Deoxynucleotide Triphosphates

Because the molecular masses of the four natural nucleobases have a relatively narrow molecular mass range (A = 313.058, G = 329.052, C = 289.046, T = 304.046 - See Table 4), a persistent source of ambiguity in assignment of base composition can occur as follows: two nucleic acid strands having different base composition may have a difference of about 1 Da when the base composition difference between the two strands is G ↔ A (-15.994) combined with C ↔ T (+15.000). For example, one 99-mer nucleic acid strand having a base composition of A₂₇G₃₀C₁T₂₁ has a theoretical molecular mass of 30779.058 while another 99-mer nucleic acid strand having a base composition of A₂₆G₃₁C₂₂T₂₀ has a theoretical molecular mass of 30780.052. A 1 Da difference in molecular mass may be within the experimental error of a molecular mass measurement and thus, the relatively narrow molecular mass range of the four natural nucleobases imposes an uncertainty factor.

The present invention provides for a means for removing this theoretical 1 Da uncertainty factor through amplification of a nucleic acid with one mass-tagged nucleobase and three natural nucleobases. The term "nucleobase" as used herein is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

Addition of significant mass to one of the 4 nucleobases (dNTPs) in an amplification reaction, or in the primers themselves, will result in a significant difference in mass of the resulting amplification product (significantly greater than 1 Da) arising from ambiguities arising from the G ↔ A combined with C ↔ T event (Table 4). Thus, the same the G ↔ A (-15.994) event combined with 5-Iodo-C ↔ T (-110.900) event would result in a molecular mass difference of 126.894. If the molecular mass of the base composition A₂₇G₃₀5-**Iodo**-C₂₁T₂₁ (33422.958) is compared with A₂₆G₃₁₋**Iodo**-C₂₂T₂₀, (33549.852) the theoretical molecular mass difference is +126.894. The experimental error of a molecular mass measurement is not significant with regard to this molecular mass difference. Furthermore, the only base composition consistent with a measured molecular mass of the 99-mer nucleic acid is A₂₇G₃₀5-**Iodo**-C₂₁T₂₁. In contrast, the analogous amplification without the mass tag has 18 possible base compositions.

**Table 4: Molecular Masses of Natural Nucleobases and the Mass-Modified Nucleobase 5-Iodo-C and Molecular Mass Differences Resulting from Transitions**

| **Nucleobase** | **Molecular Mass** | **Transition** | **Δ Molecular Mass** |
|---|---|---|---|
| A | 313.058 | A-->T | -9.012 |
| A | 313.058 | A-->C | -24.012 |
| A | 313.058 | A-->5-Iodo-C | 101.888 |
| A | 313.058 | A-->G | 15.994 |
| T | 304.046 | T-->A | 9.012 |
| T | 304.046 | T-->C | -15.000 |
| T | 304.046 | T-->5-Iodo-C | 110.900 |
| T | 304.046 | T-->G | 25.006 |
| C | 289.046 | C-->A | 24.012 |
| C | 89.046 | C-->T | 15.000 |
| C | 89.046 | C-->G | 40.006 |
| 5-Iodo-C | 414.946 | 5-Iodo-C-->A | -101.888 |
| 5-Iodo-C | 414.946 | 5-Iodo-C-->T | -110.900 |
| 5-Iodo-C | 414.946 | 5-Iodo-C-->G | -85.894 |
| G | 329.052 | G-->A | -15.994 |
| G | 329.052 | G-->T | -25.006 |
| G | 329.052 | G-->C | -40.006 |
| G | 329.052 | G-->5-Iodo-C | 85.894 |

Mass spectra of bioagent-identifying amplicons were analyzed independently using a maximum-likelihood processor, such as is widely used in radar signal processing. This processor, referred to as GenX, first makes maximum likelihood estimates of the input to the mass spectrometer for each primer by running matched filters for each base composition aggregate on the input data. This includes the GenX response to a calibrant for each primer.

The algorithm emphasizes performance predictions culminating in probability-of-detection versus probability-of-false-alarm plots for conditions involving complex backgrounds of naturally occurring organisms and environmental contaminants. Matched filters consist of *a priori* expectations of signal values given the set of primers used for each of the bioagents. A genomic sequence database is used to define the mass base count matched filters. The database contains the sequences of known bacterial bioagents and includes threat organisms as well as benign background organisms. The latter is used to estimate and subtract the spectral signature produced by the background organisms. A maximum likelihood detection of known background organisms is implemented using matched filters and a running-sum estimate of the noise covariance. Background signal strengths are estimated and used along with the matched filters to form signatures which are then subtracted. The maximum likelihood process is applied to this "cleaned up" data in a similar manner employing matched filters for the organisms and a running-sum estimate of the noise-covariance for the cleaned up data.

The amplitudes of all base compositions ofbioagent-identifying amplicons for each primer are calibrated and a final maximum likelihood amplitude estimate per organism is made based upon the multiple single primer estimates. Models of all system noise are factored into this two-stage maximum likelihood calculation. The processor reports the number of molecules of each base composition contained in the spectra. The quantity of amplification product corresponding to the appropriate primer set is reported as well as the quantities of primers remaining upon completion of the amplification reaction.

Base count blurring can be carried out as follows. "Electronic PCR" can be conducted on nucleotide sequences of the desired bioagents to obtain the different expected base counts that could be obtained for each primer pair. See for example, ncbi.nlm.nih.gov/sutils/e-pcr/; Schuler, Genome Res. 7:541-50, 1997. In one illustrative embodiment, one or more spreadsheets, such as Microsoft Excel workbooks contain a plurality of worksheets. First in this example, there is a worksheet with a name similar to the workbook name; this worksheet contains the raw electronic PCR data. Second, there is a worksheet named "filtered bioagents base count" that contains bioagent name and base count; there is a separate record for each strain after removing sequences that are not identified with a genus and species and removing all sequences for bioagents with less than 10 strains. Third, there is a worksheet, "Sheet1" that contains the frequency of substitutions, insertions, or deletions for this primer pair. This data is generated by first creating a pivot table from the data in the "filtered bioagents base count" worksheet and then executing an Excel VBA macro. The macro creates a table of differences in base counts for bioagents of the same species, but different strains. One of ordinary skill in the art may understand additional pathways for obtaining similar table differences without undo experimentation.

Application of an exemplary script, involves the user defining a threshold that specifies the fraction of the strains that are represented by the reference set of base counts for each bioagent. The reference set of base counts for each bioagent may contain as many different base counts as are needed to meet or exceed the threshold. The set of reference base counts is defined by taking the most abundant strain's base type composition and adding it to the reference set and then the next most abundant strain's base type composition is added until the threshold is met or exceeded. The current set of data was obtained using a threshold of 55%, which was obtained empirically.

For each base count not included in the reference base count set for that bioagent, the script then proceeds to determine the manner in which the current base count differs from each of the base counts in the reference set. This difference may be represented as a combination of substitutions, Si=Xi, and insertions, Ii=Yi, or deletions, Di=Zi. If there is more than one reference base count, then the reported difference is chosen using rules that aim to minimize the number of changes and, in instances with the same number of changes, minimize the number of insertions or deletions. Therefore, the primary rule is to identify the difference with the minimum sum (Xi+Yi) or (Xi+Zi), e.g., one insertion rather than two substitutions. If there are two or more differences with the minimum sum, then the one that will be reported is the one that contains the most substitutions.

Differences between a base count and a reference composition are categorized as one, two, or more substitutions, one, two, or more insertions, one, two, or more deletions, and combinations of substitutions and insertions or deletions. The different classes ofnucleobase changes and their probabilities of occurrence have been delineated in U.S. Patent Application Publication No. 2004209260 (U.S. Application Serial No. 10/418,514).

### Example 6: Use of Broad Range Survey and Division Wide Primer Pairs for Identification of Bacteria in an Epidemic Surveillance Investigation

This investigation employed a set of 16 primer pairs which is herein designated the "surveillance primer set" and comprises broad range survey primer pairs, division wide primer pairs and a single *Bacillus* clade primer pair. The surveillance primer set is shown in Table 5 and consists of primer pairs originally listed in Table 2. This surveillance set comprises primers with T modifications (note TMOD designation in primer names) which constitutes a functional improvement with regard to prevention of non-templated adenylation (*vide supra*) relative to originally selected primers which are displayed below in the same row. Primer pair 449 (non-T modified) has been modified twice. Its predecessors are primer pairs 70 and 357, displayed below in the same row. Primer pair 360 has also been modified twice and its predecessors are primer pairs 17 and 118.

**Table 5: Bacterial Primer Pairs of the Surveillance Primer Set**

| **Primer PairNo.** | **Forward Primer Name** | **Forward Primer(SEQ ID NO:)** | **Reverse Primer Name** | **Reverse Primer(SEQ ID NO:)** | **Target Gene** |
|---|---|---|---|---|---|
| 346 | 16S_EC_713_732_TMOD_F | 202 | 16S_EC_789_809_TMOD_R | 111 | 16S rRNA |
| 10 | 16S_EC_713_732_F | 21 | 16S_EC_789_809 | 798 | 16S rRNA |
| 347 | 16S_EC_785_806_TMOD_F | 560 | 16S_EC_880_897_TMOD_R | 1278 | 16S rRNA |
| 11 | 16S EC 785 806 F | 118 | 16S_EC_880_897_R | 830 | 16S rRNA |
| 348 | 16S_EC_960_981_TMOD_F | 706 | 16S_EC_1054_1073_TMOD_R | 895 | 16S rRNA |
| 14 | 16S_EC 960 981 F | 672 | 16S EC 1054 1073 R | 735 | 16S rRNA |
| 349 | 23S_EC_1826_1843_TMOD_F | 401 | 23S_EC_1906_1924_TMOD_R | 1156 | 23S rRNA |
| 16 | 23S EC 1826 1843 F | 80 | 23S_EC 1906 1924 R | 805 | 23S rRNA |
| 352 | INFB_EC_1365_1393_TMOD_F | 687 | INFB_EC_1439_1467_TMOD_R | 1411 | infB |
| 34 | INFB_EC_1365_1393_F | 524 | INFB_EC_1439_1467_R | 1248 | infB |
| 354 | RPOC_EC_2218_2241_TMOD_F | 405 | RPOC_EC_2313_2337_TMOD_R | 1072 | rpoc |
| 52 | RPOC_EC_2218_2241_F | 81 | RPOC_EC_2313_2337_R | 790 | rpoc |
| 355 | SSPE_BA_115_137_TMOD_F | 255 | SSPE_BA_197_222_TMOD_R | 1402 | sspE |
| 58 | SSPE_BA_115_137_F | 45 | SSPE_BA_197_222_R | 1201 | sspE |
| 356 | RPLB_EC_650_679_TMOD_F | 232 | RPLB_EC_739_762_TMOD_R | 592 | rplB |
| 66 | RPLB_EC_650_679_F | 98 | RPLB_EC_739_762_R | 999 | rplB |
| 358 | VALS_EC_1105_1124_TMOD_F | 385 | VMS_EC_1195_1218_TMOD_R | 1093 | valS |
| 71 | VALS_EC_1105_1124_F | 77 | VALS_EC_1195_1218_R | 795 | valS |
| 359 | RPOB_EC_1845_1866_TMOD_F | 659 | RPOB_EC_1909_1929_TMOD_R | 1250 | rpoB |
| 72 | RPOB_EC_1845_1866_F | 233 | RPOB_EC_1909_1929_R | 825 | rpob |
| 360 | 23S_EC_2646_2667_TMOD_F | 409 | 23S_EC_2745_2765_TMOD_R | 1434 | 23S rRNA |
| 118 | 23S_EC_2646_2667_F | 84 | 23S_EC_2745_2765_R | 1389 | 23S rRNA |
| 17 | 23S_EC_2645_2669_F | 408 | 23S_EC_2744_2761_R | 1252 | 23S rRNA |
| 361 | 16S_EC_1090_1111_2_TMOD_F | 697 | 16S_EC_1175_1196_TMOD_R | 1398 | 16S rRNA |
| 3 | 16S_EC_1090_1111_2_F | 651 | 16S_EC_1175_1196_R | 1159 | 16S rRNA |
| 362 | RPOB_EC_3799_3821_TMOD_F | 581 | RPOB_EC_3862_3888_TMOD_R | 1325 | rpoB |
| 289 | RPOB_EC_3799_3821_F | 124 | RPOB_EC 3862 3888 R | 840 | rpoB |
| 363 | RPOC_EC_2146_2174_TMOD_F | 284 | RPOC_EC_2227_2245_TMOD_R | 898 | rpoc |
| 290 | RPOC_EC_2146_2174_F | 52 | RPOC EC 2227 2245 R | 736 | rpoc |
| 367 | TUFB_EC_957_979_TMOD_F | 308 | TUFB_EC_1034_1058_TMOD_R | 1276 | tufB |
| 293 | TUFB_EC_957_979_F | 55 | TUFB_EC_1034_1058_R | 829 | tufB |
| 449 | RPLB_EC_690_710_F | 309 | RPLB_EC_737_758_R | 1336 | rplB |
| 357 | RPLB_EC_688_710_TMOD_F | 296 | RPLB_EC_736_757_TMOD_R | 1337 | rplB |
| 67 | RPLB_EC_688_710_F | 54 | RPLB_EC_736_757_R | 842 | rplB |

The 16 primer pairs of the surveillance set are used to produce bioagent identifying amplicons whose base compositions are sufficiently different amongst all known bacteria at the species level to identify, at a reasonable confidence level, any given bacterium at the species level. As shown in Tables 6A-E, common respiratory bacterial pathogens can be distinguished by the base compositions of bioagent identifying amplicons obtained using the 16 primer pairs of the surveillance set. In some cases, triangulation identification improves the confidence level for species assignment. For example, nucleic acid from *Streptococcus pyogenes* can be amplified by nine of the sixteen surveillance primer pairs and *Streptococcus pneumoniae* can be amplified by ten of the sixteen surveillance primer pairs. The base compositions of the bioagent identifying amplicons are identical for only one of the analogous bioagent identifying amplicons and differ in all of the remaining analogous bioagent identifying amplicons by up to four bases per bioagent identifying amplicon. The resolving power of the surveillance set was confirmed by determination of base compositions for 120 isolates of respiratory pathogens representing 70 different bacterial species and the results indicated that natural variations (usually only one or two base substitutions per bioagent identifying amplicon) amongst multiple isolates of the same species did not prevent correct identification of major pathogenic organisms at the species level.

*Bacillus anthracis* is a well known biological warfare agent which has emerged in domestic terrorism in recent years. Since it was envisioned to produce bioagent identifying amplicons for identification of *Bacillus anthracis,* additional drill-down analysis primers were designed to target genes present on virulence plasmids of *Bacillus anthracis* so that additional confidence could be reached in positive identification of this pathogenic organism. Three drill-down analysis primers were designed and are listed in Tables 2 and 6. In Table 6, the drill-down set comprises primers with T modifications (note TMOD designation in primer names) which constitutes a functional improvement with regard to prevention of non-templated adenylation (*vide supra*) relative to originally selected primers which are displayed below in the same row.

**Table 6: Drill-Down Primer Pairs for Confirmation of Identification of Bacillus anthracis**

| **Primer Pair No.** | **Forward Primer Name** | **Forward Primer (SEQ ID NO:)** | **Reverse Primer Name** | **Reverse Primer (SEQ ID NO:)** | **Target Gene** |
|---|---|---|---|---|---|
| 350 | CAPC_BA_274_303_TMOD_F | 476 | CAPC_8A_349_376_TMOD_R | 1314 | capC |
| 24 | CAPC_BA_274_303_F | 109 | CAPC BA 349 376 R | 837 | capc |
| 351 | CYA_BA_1353_1379_TMOD_F | 355 | CYA_BA_1448_1467_TMOD_R | 1423 | cyA |
| 30 | CYA BA 1353 1379 F | 64 | CYA_BA_1448 1467 R | 1342 | cyA |
| 353 | LEF_BA_756_781_TMOD_F | 220 | LEF_BA_843_872_TMOD_R | 1394 | lef |
| 37 | LEF BA 756 781 F | 26 | LEF BA 843 872 R | 1135 | lef |

Phylogenetic coverage of bacterial space of the sixteen surveillance primers of Table 5 and the three *Bacillus* anthracis drill-down primers of Table 6 is shown in Figure 3 which lists common pathogenic bacteria. Figure 3 is not meant to be comprehensive in illustrating all species identified by the primers. Only pathogenic bacteria are listed as representative examples of the bacterial species that can be identified by the primers and methods of the present invention. Nucleic acid of groups of bacteria enclosed within the polygons of Figure 3 can be amplified to obtain bioagent identifying amplicons using the primer pair numbers listed in the upper right hand corner of each polygon. Primer coverage for polygons within polygons is additive. As an illustrative example, bioagent identifying amplicons can be obtained for *Chlamydia trachomatis* by amplification with, for example, primer pairs 346-349, 360 and 361, but not with any of the remaining primers of the surveillance primer set. On the other hand, bioagent identifying amplicons can be obtained from nucleic acid originating from *Bacillus anthracis* (located within 5 successive polygons) using, for example, any of the following primer pairs: 346-349, 360, 361 (base polygon), 356, 449 (second polygon), 352 (third polygon), 355 (fourth polygon), 350, 351 and 353 (fifth polygon). Multiple coverage of a given organism with multiple primers provides for increased confidence level in identification of the organism as a result of enabling broad triangulation identification.

In Tables 7A-E, base compositions of respiratory pathogens for primer target regions are shown. Two entries in a cell, represent variation in ribosomal DNA operons. The most predominant base composition is shown first and the minor (frequently a single operon) is indicated by an asterisk (*). Entries with NO DATA mean that the primer would not be expected to prime this species due to mismatches between the primer and target region, as determined by theoretical PCR.

**Table 7A - Base Compositions of Common Respiratory Pathogens for Bioagent Identifying Amplicons Corresponding to Primer Pair Nos: 346,347 and 348**

| **Organism** | **Strain** | **Primer 346 [A G C T]** | **Primer 347 [A G C T]** | **Primer 348 [A G C T]** |
|---|---|---|---|---|
| *Klebsiella pneumoniae* | MGH78578 | [29 32 25 13] [29 31 25 13]* | [23 38 28 26] [23 37 28 26] * | [26 32 28 30] [26 31 28 30]* |
| *Yersinia pestis* | CO-92 Biovar Orientalis | [29 32 25 131 | [22 39 28 26] | [29 30 28 29] [30 30 27 29]* |
| *Yersinia pestis* | KIMS P12 (Biovar Mediaevalis) | [29 32 25 13] | [22 39 28 26] | [29 30 28 29] |
| *Yersinia pestis* | 91001 | [29 32 25 13] | [22 39 28 26] | [29 30 28 29] [30 30 27 29]* |
| *Haemophilus influenzae* | KW20 | [28 31 23 17] | [24 37 25 27] | [29 30 28 29] |
| *Pseudomonas aeruginosa* | PAO1 | [30 31 23 15] | [26 36 29 24] [27 36 29 23]* | [26 32 29 29] |
| *Pseudomonas fluorescens* | PfO-1 | [30 31 23 15] | [26 35 29 25] | [28 31 28 29] |
| *Pseudomonas putida* | KT2440 | [30 31 23 15] | [28 33 27 27] | [27 32 29 28] |
| *Legionella pneumophila* | Philadelphia-1 | [30 30 24 15] | [33 33 23 27] | [29 28 28 31] |
| *Francisella tularensis* | schu 4 | [32 29 22 16] | [28 38 26 26] | [25 32 28 31] |
| *Bordetella pertussis* | Tohama I | [30 29 24 16] | [23 37 30 24] | [30 32 30 26] |
| *Burkholderia cepacia* | J2315 | [29 29 27 14] | [27 32 26 29] | [27 36 31 24] [20 42 35 19]* |
| *Burkholderia pseudomallei* | K96243 | [29 29 27 14] | [27 32 26 29] | [27 36 31 24] |
| *Neisseria gonorrhoeae* | FA 1090, ATCC 700825 | [29 28 24 18] | [27 34 26 28] | [24 36 29 27] |
| *Neisseria meningitidis* | MC58 (serogroup B) | [29 28 26 16] | [27 34 27 27] | [25 35 30 26] |
| *Neisseria meningitidis* | serogroup C, FAM18 | [29 28 26 16] | [27 34 27 27] | [25 35 30 26] |
| *Neisseria meningitidis* | Z2491 (serogroup A) | [29 28 26 16] | [27 34 27 27] | [25 35 30 26] |
| *Chlamydophila pneumoniae* | TW-183 | [31 27 22 19] | NO DATA | [32 27 27 29] |
| *Chlamydophila pneumoniae* | AR39 | [31 27 22 19] | NO DATA | [32 27 27 29] |
| *Chlamydophila pneumoniae* | CWL029 | [31 27 22 19] | NO DATA | [32 27 27 29] |
| *Chlamydophila pneumoniae* | J138 | [31 27 22 19] | NO DATA | [32 27 27 29] |
| *Corynebacterium diphtheriae* | NCTC13129 | [29 34 21 15] | [22 38 31 25] | [22 33 25 34] |
| *Mycobacterium avium* | k10 | [27 36 21 15] | [22 37 30 28] | [21 36 27 30] |
| *Mycobacterium avium* | 104 | [27 36 21 15] | [22 37 30 28] | [21 36 27 30] |
| *Mycobacterium tuberculosis* | CSU#93 | [27 36 21 15] | [22 37 30 28] | [21 36 27 30] |
| *Mycobacterium tuberculosis* | CDC 1551 | [27 36 21 15] | [22 37 30 28] | [21 36 27 30] |
| *Mycobacterium tuberculosis* | H37Rv (lab strain) | [27 36 21 15] | [22 37 30 28] | [21 36 27 30] |
| *Mycoplasma pneumoniae* | M129 | [31 29 19 20] | NO DATA | NO DATA |
| *Staphylococcus aureus* | MRSA252 | [27 30 21 21] | [25 35 30 26] | [30 29 30 29] [29 31 30 29]* |
| *Staphylococcus aureus* | MSSA476 | [27 30 21 21] | [25 35 30 26] | [30 29 30 29] [30 29 29 30]* |
| *Staphylococcus aureus* | COL | [27 30 21 21] | [25 35 30 26] | [30 29 30 29] [30 29 29 30]* |
| *Staphylococcus aureus* | Mu50 | [27 30 21 21] | [25 35 30 26] | [30 29 30 29] [30 29 29 30]* |
| *Staphylococcus aureus* | MW2 | [27 30 21 21] | [25 35 30 26] | [30 29 30 29] [30 29 29 30]* |
| *Staphylococcus aureus* | N315 | [27 30 21 21] | [25 35 30 26] | [30 29 30 29] [30 29 29 30] * |
| *Staphylococcus aureus* | NCTC 8325 | [27 30 21 21] | [25 35 30 26] [25 35 31 26]* | [30 29 30 29] [30 29 29 30] |
| *Streptococcus agalactiae* | NEM316 | [26 32 23 18] | [24 36 31 25] [24 36 30 26]* | [25 32 29 30] |
| *Streptococcus equi* | NC 002955 | [26 32 23 18] | [23 37 31 25] | [29 30 25 32] |
| *Streptococcus pyogenes* | MGAS8232 | [26 32 23 18] | [24 37 30 25] | [25 31 29 31] |
| *Streptococcus pyogenes* | MGAS315 | [26 32 23 18] | [24 37 30 25] | [25 31 29 31] |
| *Streptococcus pyogenes* | SSI-1 | [26 32 23 18] | [24 37 30 25] | [25 31 29 31] |
| *Streptococcus pyogenes* | MGAS10394 | [26 32 23 18] | [24 37 30 25] | [25 31 29 31] |
| *Streptococcus pyogenes* | Manfredo (M5) | [26 32 23 18] | [24 37 30 25] | [25 31 29 31] |
| *Streptococcus pyogenes* | SF370 (M1) | [26 32 23 18] | [24 37 30 25] | [25 31 29 31] |
| *Streptococcus pneumoniae* | 670 | [26 32 23 18] | [25 35 28 28] | [25 32 29 30] |
| *Streptococcus pneumoniae* | R6 | [26 32 23 18] | [25 35 28 28] | [25 32 29 30] |
| *Streptococcus pneumoniae* | TIGR4 | [26 32 23 18] | [25 35 28 28] | [25 32 30 29] |
| *Streptococcus gordonii* | NCTC7868 | [25 33 23 18] | [24 36 31 25] | [25 31 29 31] |
| *Streptococcus mitis* | NCTC 12261 | [26 32 23 18] | [25 35 30 26] | [25 32 29 30] [24 31 35 29]* |
| *Streptococcus mutans* | UA159 | [24 32 24 19] | [25 37 30 24] | [28 31 26 31] |

**TABLE 7B - Base Compositions of Common Respiratory Pathogens for Bioagent Identifying Amplicons Corresponding to Primer Pair Nos: 349, 360, and 356**

| Organism | Strain | Primer 349 | Primer 360 | Primer 356 |
|---|---|---|---|---|
| | | [A G C T] | [A G C T] | [A G C T] |
| *Klebsiella pneumoniae* | MGH78578 | [25 31 25 22] | [33 37 25 27] | NO DATA |
| *Yersinia pestis* | CO-92 Biovar Orientalis | [25 31 27 20] [25 32 26 20]* | [34 35 25 28] | NO DATA |
| *Yersinia pestis* | KIM5 P12 (Biovar Mediaevalis) | [25 31 27 20] [25 32 26 20]* | [34 35 25 28] | NO DATA |
| *Yersinia pestis* | 91001 | [25 31 27 20] | [34 35 25 28] | NO DATA |
| *Haemophilus influenzae* | KW20 | [28 28 25 20] | [32 38 25 27] | NO DATA |
| *Pseudomonas aeruginosa* | PAO1 | [24 31 26 20] | [31 36 27 27] [31 36 27 28]* | NO DATA |
| *Pseudomonas fluorescens* | Pf0-1 | NO DATA | [30 37 27 28] [30 37 27 28] | NO DATA |
| *Pseudomonas putida* | KT2440 | [24 31 26 20] | [30 37 27 28] | NO DATA |
| *Legionella pneumophila* | Philadelphia-1 | [23 30 25 23] | [30 39 29 24] | NO DATA |
| *Francisella tularensis* | schu 4 | [26 31 25 19] | [32 36 27 27] | NO DATA |
| *Bordetella pertussis* | Tohama I | [21 29 24 18] | [33 36 26 27] | NO DATA |
| *Burkholderia cepacia* | J2315 | [23 27 22 20] | [31 37 28 26] | NO DATA |
| *Burkholderia pseudomallei* | K96243 | [23 27 22 20] | [31 37 28 26] | NO DATA |
| *Neisseria gonorrhoeae* | FA 1090, ATCC 700825 | [24 27 24 17] | [34 37 25 26] | NO DATA |
| *Neisseria meningitidis* | MC58 (serogroup B) | [25 27 22 18] | [34 37 25 26] | NO DATA |
| *Neisseria meningitidis* | serogroup C, FAM18 | [25 26 23 18] | [34 37 25 26] | NO DATA |
| *Neisseria meningitidis* | Z2491 (serogroup A) | [25 26 23 18] | [34 37 25 26] | NO DATA |
| *Chlamydophila pneumoniae* | TW-183 | [30 28 27 18] | NO DATA | NO DATA |
| *Chlamydophila pneumoniae* | AR39 | [30 28 27 18] | NO DATA | NO DATA |
| *Chlamydophila pneumoniae* | CWL029 | [30 28 27 18] | NO DATA | NO DATA |
| *Chlamydophila pneumoniae* | J138 | [30 28 27 18] | NO DATA | NO DATA |
| *Corynebacterium diphtheriae* | NCTC13129 | NO DATA | [29 40 28 25] | NO DATA |
| *Mycobacterium avium* | k10 | NO DATA | [33 35 32 22] | NO DATA |
| *Mycobacterium avium* | 104 | NO DATA | [33 35 32 22] | NO DATA |
| *Mycobacterium tuberculosis* | CSU#93 | NO DATA | [30 36 34 22] | NO DATA |
| *Mycobacterium tuberculosis* | CDC 1551 | NO DATA | [30 36 34 22] | NO DATA |
| *Mycobacterium tuberculosis* | H37Rv (lab strain) | NO DATA | [30 36 34 22] | NO DATA |
| *Mycoplasma pneumoniae* | M129 | [28 30 24 19] | [34 31 29 28] | NO DATA |
| *Staphylococcus aureus* | MRSA252 | [26 30 25 20] | [31 38 24 29] | [33 30 31 27] |
| *Staphylococcus aureus* | MSSA476 | [26 30 25 20] | [31 38 24 29] | [33 30 31 27] |
| *Staphylococcus aureus* | COL | [26 30 25 20] | [31 38 24 29] | [33 30 31 27] |
| *Staphylococcus aureus* | Mu50 | [26 30 25 20] | [31 38 24 29] | [33 30 31 27] |
| *Staphylococcus aureus* | MW2 | [26 30 25 20] | [31 38 24 29] | [33 30 31 27] |
| *Staphylococcus aureus* | N315 | [26 30 25 20] | [31 38 24 29] | [33 30 31 27] |
| *Staphylococcusaureus* | NCTC 8325 | [26 30 25 20] | [31 38 24 29] | [33 30 31 27] |
| *Streptococcus agalactiae* | NEM316 | [28 31 22 20] | [33 37 24 28] | [37 30 28 26] |
| *Streptococcus equi* | NC 002955 | [28 31 23 19] | [33 38 24 27] | [37 31 28 25] |
| *Streptococcus pyogenes* | MGAS8232 | [28 31 23 19] | [33 37 24 28] | [38 31 29 23] |
| *Streptococcus pyogenes* | MGAS315 | [28 31 23 19] | [33 37 24 28] | [38 31 29 23] |
| *Streptococcus pyogenes* | SSI-1 | [28 31 23 19] | [33 37 24 28] | [38 31 29 23] |
| *Streptococcus pyogenes* | MGAS10394 | [28 31 23 19] | [33 37 24 28] | [38 31 29 23] |
| *Streptococcus pyogenes* | Manfredo (M5) | [28 31 23 19] | [33 37 24 28] | [38 31 29 23] |
| *Streptococcus pyogenes* | SF370 (M1) | [28 31 23 19] [28 31 22 20]* | [33 37 24 28] | [38 31 29 23] |
| *Streptococcus pneumoniae* | 670 | [28 31 22 20] | [34 36 24 28] | [37 30 29 25] |
| *Streptococcus pneumoniae* | R6 | [28 31 22 20] | [34 36 24 28] | [37 30 29 25] |
| *Streptococcus pneumoniae* | TIGR4 | [28 31 22 20] | [34 36 24 28] | [37 30 29 25] |
| *Streptococcus gordonii* | NCTC7868 | [28 32 23 20] | [34 36 24 28] | [36 31 29 25] |
| *Streptococcus mitis* | NCTC 12261 | [28 31 22 20] [29 30 22 20]* | [34 36 24 28] | [37 30 29 25] |
| *Streptococcus mutans* | UA159 | [26 32 23 22] | [34 37 24 27] | NO DATA |

**Table 7C - Base Compositions of Common Respiratory Pathogens for Bioagent Identifying Amplicons Corresponding to Primer Pair Nos: 449, 354, and 352**

| **Organism** | **Strain** | **Primer 449 [A G C T]** | **Primer 354 [A G C T]** | **Primer 352 [A G C T]** |
|---|---|---|---|---|
| *Klebsiella pneumoniae* | MGH78578 | NO DATA | [27 33 36 26] | NO DATA |
| *Yersinia pestis* | CO-92 Biovar Orientalis | NO DATA | [29 31 33 29] | [32 28 20 25] |
| *Yersinia pestis* | KIM5 P12 (Biovar Mediaevalis) | NO DATA | [29 31 33 29] | [32 28 20 25] |
| *Yersinia pestis* | 91001 | NO DATA | [29 31 33 29] | NO DATA |
| *Haemophilus influenzae* | KW20 | NO DATA | [30 29 31 32] | NO DATA |
| *Pseudomonas aeruginosa* | PAO1 | NO DATA | [26 33 39 24] | NO DATA |
| *Pseudomonas fluorescens* | Pf0-1 | NO DATA | [26 33 34 29] | NO DATA |
| *Pseudomonas putida* | KT2440 | NO DATA | [25 34 36 27] | NO DATA |
| *Legionella pneumophila* | Philadelphia-1 | NO DATA | NO DATA | NO DATA |
| *Francisella tularensis* | schu 4 | NO DATA | [33 32 25 32] | NO DATA |
| *Bordetella pertussis* | Tohama I | NO DATA | [26 33 39 24] | NO DATA |
| *Burkholderia cepacia* | J2315 | NO DATA | [25 37 33 27] | NO DATA |
| *Burkholderia pseudomallei* | K96243 | NO DATA | [25 37 34 26] | NO DATA |
| *Neisseria gonorrhoeae* | FA 1090, ATCC 700825 | [17 23 22 10] | [29 31 32 30] | NO DATA |
| *Neisseria meningitidis* | MC58 (serogroup B) | NO DATA | [29 30 32 31] | NO DATA |
| *Neisseria meningitidis* | serogroup C, FAM18 | NO DATA | [29 30 32 31] | NO DATA |
| *Neisseria meningitidis* | Z2491 (serogroup A) | NO DATA | [29 30 32 31] | NO DATA |
| *Chlamydophila pneumoniae* | TW-183 | NO DATA | NO DATA | NO DATA |
| *Chlamydophila pneumoniae* | AR39 | NO DATA | NO DATA | NO DATA |
| *Chlamydophila pneumoniae* | CWL029 | NO DATA | NO DATA | NO DATA |
| *Chlamydophila pneumoniae* | J138 | NO DATA | NO DATA | NO DATA |
| *Corynebacterium diphtheriae* | NCTC13129 | NO DATA | NO DATA | NO DATA |
| *Mycobacterium avium* | k10 | NO DATA | NO DATA | NO DATA |
| *Mycobacterium avium* | 104 | NO DATA | NO DATA | NO DATA |
| *Mycobacterium tuberculosis* | CSU#93 | NO DATA | NO DATA | NO DATA |
| *Mycobacterium tuberculosis* | CDC 1551 | NO DATA | NO DATA | NO DATA |
| *Mycobacterium tuberculosis* | H37Rv (lab strain) | NO DATA | NO DATA | NO DATA |
| *Mycoplasma pneumoniae* | M129 | NO DATA | NO DATA | NO DATA |
| *Staphylococcus aureus* | MRSA252 | [1720 21 17] | [30 27 30 35] | [36 24 19 26] |
| *Staphylococcus aureus* | MSSA476 | [17 20 21 17] | [30 27 30 35] | [36 24 19 26] |
| *Staphylococcus aureus* | COL | [17 20 21 17] | [30 27 30 35] | [35 24 19 27] |
| *Staphylococcus aureus* | Mu50 | [17 20 21 17] | [30 27 30 35] | [36 24 19 26] |
| *Staphylococcus aureus* | MW2 | [17 20 21 17] | [30 27 30 35] | [36 24 19 26] |
| *staphylococcus aureus* | N315 | [17 20 21 17] | [30 27 30 35] | [36 24 19 26] |
| *Staphylococcus aureus* | NCTC 8325 | [17 20 21 17] | [30 27 30 35] | [35 24 19 27] |
| *Streptococcus agalactiae* | NEM316 | [22 20 19 14] | [26 31 27 38] | [29 26 22 28] |
| *Streptococcus equi* | NC 002955 | [22 21 19 13] | NO DATA | NO DATA |
| *Streptococcus pyogenes* | MGAS8232 | [23 21 19 12] | [24 32 30 36] | NO DATA |
| *Streptococcus pyogenes* | MGAS315 | [23 21 19 12] | [24 32 30 36] | NO DATA |
| *Streptococcus pyogenes* | SSI-1 | [23 21 19 12] | [24 32 30 36] | NO DATA |
| *Streptococcus pyogenes* | MGAS10394 | [23 21 19 12] | [24 32 30 36] | NO DATA |
| *Streptococcus pyogenes* | Manfredo (M5) | [23 21 19 12] | [24 32 30 36] | NO DATA |
| *Streptococcus pyogenes* | SF370 (M1) | [23 21 19 12] | [24 32 30 36] | NO DATA |
| *Streptococcus pneumoniae* | 670 | [22 20 19 14] | [25 33 29 35] | [30 29 21 25] |
| *Streptococcus pneumoniae* | R6 | [22 20 19 14] | [25 33 29 35] | [30 29 21 25] |
| *Streptococcus pneumoniae* | TIGR4 | [22 20 19 14] | [25 33 29 35] | [30 29 21 25] |
| *Streptococcus gordonii* | NCTC7868 | [21 21 19 14] | NO DATA | [29 26 22 28] |
| *Streptococcus mitis* | NCTC 12261 | [22 20 19 14] | [26 30 32 34] | NO DATA |
| *Streptococcus mutans* | UA159 | NO DATA | NO DATA | NO DATA |

**Table 7D - Base Compositions of Common Respiratory Pathogens for Bioagent Identifying Amplicons Corresponding to Primer Pair Nos: 355, 358, and 359**

| **Organism** | **Strain** | **Primer 355 [A G C T]** | **Primer 358 [A G C T]** | **Primer 359 [A G C T]** |
|---|---|---|---|---|
| *Klebsiella pneumoniae* | MGH78578 | NO DATA | [24 39 33 20] | [25 21 24 17] |
| *Yersinia pestis* | CO-92 Biovar Orientalis | NO DATA | [26 34 35 21] | [23 23 19 22] |
| *Yersinia pestis* | KIM5 P12 (Biovar Mediaevalis) | NO DATA | [26 34 35 21] | [23 23 19 22] |
| *Yersinia pestis* | 91001 | NO DATA | [26 34 35 21] | [23 23 19 22] |
| *Haemophilus influenzae* | KW20 | NO DATA | NO DATA | NO DATA |
| *Pseudomonas aeruginosa* | PAO1 | NO DATA | NO DATA | NO DATA |
| *Pseudomonas fluorescens* | pf0-1 | NO DATA | NO DATA | NO DATA |
| *Pseudomonas putida* | KT2440 | NO DATA | [21 37 37 21] | NO DATA |
| *Legionella pneumophila* | Philadelphia-1 | NO DATA | NO DATA | NO DATA |
| *Francisella tularensis* | schu 4 | NO DATA | NO DATA | NO DATA |
| *Bordetella pertussis* | Tohama I | NO DATA | NO DATA | NO DATA |
| *Burkholderia cepacia* | J2315 | NO DATA | NO DATA | NO DATA |
| *Burkholderia pseudomallei* | K96243 | NO DATA | NO DATA | NO DATA |
| *Neisseria gonorrhoeae* | FA 1090, ATCC 700825 | NO DATA | NO DATA | NO DATA |
| *Neisseria meningitidis* | MC58 (serogroup B) | NO DATA | NO DATA | NO DATA |
| *Neisseria meningitidis* | serogroup C, FAM18 | NO DATA | NO DATA | NO DATA |
| *Neisseria meningitidis* | Z2491 (serogroup A) | NO DATA | NO DATA | NO DATA |
| *Chlamydophila pneumoniae* | TW-183 | NO DATA | NO DATA | NO DATA |
| *Chlamydophila pneumoniae* | AR39 | NO DATA | NO DATA | NO DATA |
| *Chlamydophila pneumoniae* | CWL029 | NO DATA | NO DATA | NO DATA |
| *Chlamydophila pneumoniae* | J138 | NO DATA | NO DATA | NO DATA |
| *Corynebacterium diphtheriae* | NCTC13129 | NO DATA | NO DATA | NO DATA |
| *Mycobacterium avium* | k10 | NO DATA | NO DATA | NO DATA |
| *Mycobacterium avium* | 104 | NO DATA | NO DATA | NO DATA |
| *Mycobacterium tuberculosis* | CSU#93 | NO DATA | NO DATA | NO DATA |
| *Mycobacterium tuberculosis* | CDC 1551 | NO DATA | NO DATA | NO DATA |
| *Mycobacterium tuberculosis* | H37Rv (lab strain) | NO DATA | NO DATA | NO DATA |
| *Mycoplasma pneumoniae* | M129 | NO DATA | NO DATA | NO DATA |
| *Staphylococcus aureus* | MRSA252 | NO DATA | NO DATA | NO DATA |
| *Staphylococcus aureus* | MSSA476 | NO DATA | NO DATA | NO DATA |
| *Staphylococcus aureus* | COL | NO DATA | NO DATA | NO DATA |
| *Staphylococcus aureus* | Mu50 | NO DATA | NO DATA | NO DATA |
| *Staphylococcus aureus* | MW2 | NO DATA | NO DATA | NO DATA |
| *Staphylococcus aureus* | N315 | NO DATA | NO DATA | NO DATA |
| *Staphylococcus aureus* | NCTC 8325 | NO DATA | NO DATA | NO DATA |
| *Streptococcus agalactiae* | NEM316 | NO DATA | NO DATA | NO DATA |
| *Streptococcus equi* | NC 002955 | NO DATA | NO DATA | NO DATA |
| *Streptococcus pyogenes* | MGAS8232 | NO DATA | NO DATA | NO DATA |
| *Streptococcus pyogenes* | MGAS315 | NO DATA | NO DATA | NO DATA |
| *Streptococcus pyogenes* | SSI-1 | NO DATA | NO DATA | NO DATA |
| *Streptococcus pyogenes* | MGAS10394 | NO DATA | NO DATA | NO DATA |
| *Streptococcus pyogenes* | Manfredo (M5) | NO DATA | NO DATA | NO DATA |
| *Streptococcus pyogenes* | SF370 (M1) | NO DATA | NO DATA | NO DATA |
| *Streptococcus pneumoniae* | 670 | NO DATA | NO DATA | NO DATA |
| *Streptococcus pneumoniae* | R6 | NO DATA | NO DATA | NO DATA |
| *Streptococcus pneumoniae* | TIGR4 | NO DATA | NO DATA | NO DATA |
| *Streptococcus gordonii* | NCTC7868 | NO DATA | NO DATA | NO DATA |
| *Streptococcus mitis* | NCTC 12261 | NO DATA | NO DATA | NO DATA |
| *Streptococcus mutans* | UA159 | NO DATA | NO DATA | NO DATA |

**Table 7E - Base Compositions of Common Respiratory Pathogens for Bioagent Identifying Amplicons Corresponding to Primer Pair Nos: 362, 363, and 367**

| **Organism** | **Strain** | **Primer 362 [A G C T]** | **Primer 363 [A G C T]** | **Primer 367 [A G C T]** |
|---|---|---|---|---|
| *Klebsiella pneumoniae* | MGH78578 | [21 33 22 16] | [16 34 26 26] | NO DATA |
| *Yersinia pestis* | CO-92 Biovar Orientalis | [20 34 18 20] | NO DATA | NO DATA |
| *Yersinia pestis* | KIM5 P12 (Biovar Mediaevalis) | [20 34 18 20] | NO DATA | NO DATA |
| *Yersinia pestis* | 91001 | [20 34 18 20] | NO DATA | NO DATA |
| *Haemophilus influenza* | *KW20* | NO DATA | NO DATA | NO DATA |
| *Pseudomonas aeruginosa* | PAO1 | [19 35 21 17] | [16 36 28 22] | NO DATA |
| *Pseudomonas fluorescens* | Pf0-1 | NO DATA | [18 35 26 23] | NO DATA |
| *Pseudomonas putida* | KT2440 | NO DATA | [16 35 28 23] | NO DATA |
| *Legionella pneumophila* | Philadelphia-1 | NO DATA | NO DATA | NO DATA |
| *Francisella tularensis* | schu 4 | NO DATA | NO DATA | NO DATA |
| *Bordetella pertussis* | Tohama I | [20 31 24 17] | [15 34 32 21] | [26 25 34 19] |
| *Burkholderia cepacia* | J2315 | [20 33 21 18] | [15 36 26 25] | [25 27 32 20] |
| *Burkholderia pseudomallei* | K96243 | [19 34 19 20] | [15 37 28 22] | [25 27 32 20] |
| *Neisseria gonorrhoeae* | FA 1090, ATCC 700825 | NO DATA | NO DATA | NO DATA |
| *Neisseria meningitidis* | MC58 (serogroup B) | NO DATA | NO DATA | NO DATA |
| *Neisseria meningitidis* | serogroup C, FAM18 | NO DATA | NO DATA | NO DATA |
| *Neisseria meningitidis* | Z2491 (serogroup A) | NO DATA | NO DATA | NO DATA |
| *Chlamydophila pneumoniae* | TW-183 | NO DATA | NO DATA | NO DATA |
| *Chlamydophila pneumoniae* | AR39 | NO DATA | NO DATA | NO DATA |
| *Chlamydophila pneumoniae* | CNL029 | NO DATA | NO DATA | NO DATA |
| *Chlamydophila pneumoniae* | J138 | NO DATA | NO DATA | NO DATA |
| *Corynebacterium diphtheriae* | NCTC13129 | NO DATA | NO DATA | NO DATA |
| *Mycobacterium avium* | k10 | [19 34 23 16] | NO DATA | [24 26 35 19] |
| *Mycobacterium avium* | 104 | [19 34 23 16] | NO DATA | [24 26 35 19] |
| *Mycobacterium tuberculosis* | CSU#93 | [19 31 25 17] | NO DATA | [25 25 34 20] |
| *Mycobacterium tuberculosis* | CDC 1551 | [19 31 24 18] | NO DATA | [25 25 34 20] |
| *Mycobacterium tuberculosis* | H37Rv (lab strain) | [19 31 24 18] | NO DATA | [25 25 34 20] |
| *Mycoplasma pneumoniae* | M129 | NO DATA | NO DATA | NO DATA |
| *Staphylococcus aureus* | MRSA252 | NO DATA | NO DATA | NO DATA |
| *Staphylococcus aureus* | MSSA476 | NO DATA | NO DATA | NO DATA |
| *Staphylococcus aureus* | COL | NO DATA | NO DATA | NO DATA |
| *Staphylococcus aureus* | Mu50 | NO DATA | NO DATA | NO DATA |
| *Staphylococcus aureus* | MW2 | NO DATA | NO DATA | NO DATA |
| *Staphylococcus aureus* | N315 | NO DATA | NO DATA | NO DATA |
| *Staphylococcus aureus* | NCTC 8325 | NO DATA | NO DATA | NO DATA |
| *Streptococcus agalactiae* | NEM316 | NO DATA | NO DATA | NO DATA |
| *Streptococcus equi* | NC 002955 | NO DATA | NO DATA | NO DATA |
| *Streptococcus pyogenes* | MGAS8232 | NO DATA | NO DATA | NO DATA |
| *Streptococcus pyogenes* | MGAS315 | NO DATA | NO DATA | NO DATA |
| *Streptococcus pyogenes* | SSI-1 | NO DATA | NO DATA | NO DATA |
| *Streptococcus pyogenes* | MGAS10394 | NO DATA | NO DATA | NO DATA |
| *Streptococcus pyogenes* | Manfredo (M5) | NO DATA | NO DATA | NO DATA |
| *Streptococcus pyogenes* | SF370 (M1) | NO DATA | NO DATA | NO DATA |
| *Streptococcus pneumoniae* | 670 | NO DATA | NO DATA | NO DATA |
| *Streptococcus pneumoniae* | R6 | [20 30 19 23] | NO DATA | NO DATA |
| *Streptococcus pneumoniae* | TIGR4 | [20 30 19 23] | NO DATA | NO DATA |
| *Streptococcus gordonii* | NCTC7868 | NO DATA | NO DATA | NO DATA |
| *Streptococcus mitis* | NCTC 12261 | NO DATA | NO DATA | NO DATA |
| *Streptococcus mutans* | UA159 | NO DATA | NO DATA | NO DATA |

Four sets of throat samples from military recruits at different military facilities taken at different time points were analyzed using the primers of the present invention. The first set was collected at a military training center from November 1 to December 20, 2002 during one of the most severe outbreaks of pneumonia associated with group A *Streptococcus* in the United States since 1968. During this outbreak, fifty-one throat swabs were taken from both healthy and hospitalized recruits and plated on blood agar for selection of putative group A *Streptococcus* colonies. A second set of 15 original patient specimens was taken during the height of this group A *Streptococcus* -associated respiratory disease outbreak. The third set were historical samples, including twenty-seven isolates of group A *Streptococcus,* from disease outbreaks at this and other military training facilities during previous years. The fourth set of samples was collected from five geographically separated military facilities in the continental U.S. in the winter immediately following the severe November/December 2002 outbreak.

Pure colonies isolated from group A *Streptococcus*-selective media from all four collection periods were analyzed with the surveillance primer set. All samples showed base compositions that precisely matched the four completely sequenced strains of *Streptococcus pyogenes.* Shown in Figure 4 is a 3D diagram of base composition (axes A, G and C) of bioagent identifying amplicons obtained with primer pair number 14 (a precursor of primer pair number 348 which targets 16S rRNA). The diagram indicates that the experimentally determined base compositions of the clinical samples closely match the base compositions expected for *Streptococcus pyogenes* and are distinct from the expected base compositions of other organisms.

In addition to the identification of *Streptococcus pyogenes,* other potentially pathogenic organisms were identified concurrently. Mass spectral analysis of a sample whose nucleic acid was amplified by primer pair number 349 (SEQ ID NOs: 401:1156) exhibited signals of bioagent identifying amplicons with molecular masses that were found to correspond to analogous base compositions of bioagent identifying amplicons of *Streptococcus pyogenes* (A27 G32 C24 T18), *Neisseria meningitidis* (A25 G27 C22 T18), and *Haemophilus influenzae* (A28 G28 C25 T20) (see Figure 5 and Table 7B). These organisms were present in a ratio of 4:5:20 as determined by comparison of peak heights with peak height of an internal PCR calibration standard as described in commonly owned U.S. Patent Application Serial No: 60/545,425.

Since certain division-wide primers that target housekeeping genes are designed to provide coverage of specific divisions of bacteria to increase the confidence level for identification of bacterial species, they are not expected to yield bioagent identifying amplicons for organisms outside of the specific divisions. For example, primer pair number 356 (SEQ ID NOs: 449:1380) primarily amplifies the nucleic acid of members of the classes *Bacilli* and *Clostridia* and is not expected to amplify proteobacteria such as *Neisseria meningitidis* and *Haemophilus influenzae.* As expected, analysis of the mass spectrum of amplification products obtained with primer pair number 356 does not indicate the presence of *Neisseria meningitidis* and *Haemophilus influenzae* but does indicate the presence of *Streptococcus pyogenes* (Figures 3 and 6, Table 7B). Thus, these primers or types of primers can confirm the absence of particular bioagents from a sample.

The 15 throat swabs from military recruits were found to contain a relatively small set of microbes in high abundance. The most common were *Haemophilus influenza, Neisseria meningitides,* and *Streptococcus pyogenes. Staphylococcus epidennidis, Moraxella cattarhalis, Corynebacterium pseudodiphtheriticum*, and *Staphylococcus aureus* were present in fewer samples. An equal number of samples from healthy volunteers from three different geographic locations, were identically analyzed. Results indicated that the healthy volunteers have bacterial flora dominated by multiple, commensal non-beta-hemolytic *Streptococcal* species, including the viridans group *streptococci* (*S. parasangunis, S. vestibularis, S. mitis, S. oralis* and *S*. *pneumoniae;* data not shown), and none of the organisms found in the military recruits were found in the healthy controls at concentrations detectable by mass spectrometry. Thus, the military recruits in the midst of a respiratory disease outbreak had a dramatically different microbial population than that experienced by the general population in the absence of epidemic disease.

### Example 7: Triangulation Genotyping Analysis for Determination of emm-Type of Streptococcus pyogenes in Epidemic Surveillance

As a continuation of the epidemic surveillance investigation of Example 6, determination of sub-species characteristics (genotyping) of *Streptococcus pyogenes,* was carried out based on a strategy that generates strain-specific signatures according to the rationale of Multi-Locus Sequence Typing (MLST). In classic MLST analysis, internal fragments of several housekeeping genes are amplified and sequenced (Enright et al. Infection and Immunity, 2001, 69, 2416-2427). In classic MLST analysis, internal fragments of several housekeeping genes are amplified and sequenced. In the present investigation, bioagent identifying amplicons from housekeeping genes were produced using drill-down primers and analyzed by mass spectrometry. Since mass spectral analysis results in molecular mass, from which base composition can be determined, the challenge was to determine whether resolution of *emm* classification of strains of *Streptococcus pyogenes* could be determined.

For the purpose of development of a triangulation genotyping assay, an alignment was constructed of concatenated alleles of seven MLST housekeeping genes (glucose kinase (gki), glutamine transporter protein (gtr), glutamate racemase (murI), DNA mismatch repair protein (mutS), xanthine phosphoribosyl transferase (xpt), and acetyl-CoA acetyl transferase (yqiL)) from each of the 212 previously *emm*-typed strains of *Streptococcus pyogenes.* From this alignment, the number and location of primer pairs that would maximize strain identification via base composition was determined. As a result, 6 primer pairs were chosen as standard drill-down primers for determination of *emm*-type of *Streptococcus pyogenes.* These six primer pairs are displayed in Table 8. This drill-down set comprises primers with T modifications (note TMOD designation in primer names) which constitutes a functional improvement with regard to prevention of non-templated adenylation (*vide supra*) relative to originally selected primers which are displayed below in the same row.

**Table 8: Triangulation Genotyping Analysis Primer Pairs for Group A Streptococcus Drill-Down**

| **Primer Pair No.** | **Forward Primer Name** | **Forward Primer (SEQ ID NO:)** | **Reverse Primer Name** | **Reverse Primer (SEQ ID NO:)** | **Target Gene** |
|---|---|---|---|---|---|
| 442 | SP101_SPET11_358_387_ TMOD_F | 588 | SP101_SPET11_448_ 473_TMOD_R | 998 | gki |
| 80 | SP101_SPET11_358_387_ F | 126 | SP101_SPET11_448_ 473 TMOD R | 766 | gki |
| 443 | SP101_SPET11_600_629_ TMOD_F | 348 | SP101_SPET11_686_ 714_TMOD_R | 1018 | gtr |
| 81 | SP101_SPET11_600-629_ F | 62 | SP101_SPET11_686_ 714 R | 772 | gtr |
| 426 | SP101_SPET11_1314_133 6_TMOD_F | 363 | SP101_SPET11_1403 _1431_TMOD_R | 849 | murI |
| 86 | SP101_SPET11_1314_133 6_F | 68 | SP101_SPET11_1403 1431 R | 711 | murI |
| 430 | SP101_SPET11_1807_183 5_TMOD_F | 235 | SP101_SPET11_1901 _1927_TMOD_R | 1439 | mutS |
| 90 | SP101_SPET11_1807_183 5 F | 33 | SP101_SPET11_1901 _1927 R | 1412 | mutS |
| 438 | SP101_SPET11_3075_310 3_TMOD_F | 473 | SP101_SPET11_3168 _3196_TMOD_R | 875 | xpt |
| 96 | SP101_SPET11_3075_310 3 F | 108 | SP101_SPET11_3168 3196 R | 715 | xpt |
| 441 | SP101_SPET11_3511_353 5_TMOD_F | 531 | SP101_SPET11_3605 _3629_TMOD_R | 1294 | yqiL |
| 98 | SP101_SPET11_3511_353 5 F | 116 | SP101_SPET11_3605 3629 R | 832 | yqiL |

The primers of Table 8 were used to produce bioagent identifying amplicons from nucleic acid present in the clinical samples. The bioagent identifying amplicons which were subsequently analyzed by mass spectrometry and base compositions corresponding to the molecular masses were calculated.

Of the 51 samples taken during the peak of the November/December 2002 epidemic (Table 9A-C rows 1-3), all except three samples were found to represent *emm3,* a Group A *Streptococcus* genotype previously associated with high respiratory virulence. The three outliers were from samples obtained from healthy individuals and probably represent non-epidemic strains. Archived samples (Tables 9A-C rows 5-13) from historical collections showed a greater heterogeneity of base compositions and *emm* types as would be expected from different epidemics occurring at different places and dates. The results of the mass spectrometry analysis and *emm* gene sequencing were found to be concordant for the epidemic and historical samples.

**Table 9A: Base Composition Analysis of Bioagent Identifying Amplicons of Group A Streptococcus samples from Six Military Installations Obtained with Primer Pair Nos. 426 and 430**

| **# of Instances** | **emm-type by Mass Spectrometry** | **emm-Gene Sequencing** | **Location (sample)** | **Year** | **murI (Primer Pair No. 426)** | **mutS (Primer Pair No. 430)** |
|---|---|---|---|---|---|---|
| 48 | 3 | 3 | MCRD San Diego (Cultured) | 2002 | A39 G25 C20 T34 | A38 G27 C23 T33 |
| 2 | 6 | 6 | | | A40 G24 C20 T34 | A38 G27 C23 T33 |
| 1 | 28 | 28 | | | A39 G25 C20 T34 | A38 G27 C23 T33 |
| 15 | 3 | ND | | | A39 G25 C20 T34 | A38 G27 C23 T33 |
| 6 | 3 | 3 | NHRC San Diego-Archive (Cultured) | 2003 | A39 G25 C20 T34 | A38 G27 C23 T33 |
| 3 | 5,58 | 5 | | | A40 G24 C20 T34 | A38 G27 C23 T33 |
| 6 | 6 | 6 | | | A40 G24 C20 T34 | A38 G27 C23 T33 |
| 1 | 11 | 11 | | | A39 G25 C20 T34 | A38 G27 C23 T33 |
| 3 | 12 | 12 | | | A40 G24 C20 T34 | A38 G26 C24 T33 |
| 1 | 22 | 22 | | | A39 G25 C20 T34 | A38 G27 C23 T33 |
| 3 | 25,75 | 75 | | | A39 G25 C20 T34 | A38 G27 C23 T33 |
| 4 | 44/61,82,9 | 44/61 | | | A40 G24 C20 T34 | A38 G26 C24 T33 |
| 2 | 53,91 | 91 | | | A39 G25 C20 T34 | A38 G27 C23 T33 |
| 1 | 2 | 2 | Ft. Leonard Wood (Cultured) | 2003 | A39 G25 C20 T34 | A38 G27 C24 T32 |
| 2 | 3 | 3 | | | A39 G25 C20 T34 | A38 G27 C23 T33 |
| 1 | 4 | 4 | | | A39 G25 C20 T34 | A38 G27 C23 T33 |
| 1 | 6 | 6 | | | A40 G24 C20 T34 | A38 G27 C23 T33 |
| 11 | 25 or 75 | 75 | | | A39 G25 C20 T34 | A38 G27 C23 T33 |
| 1 | 25,75, 33, 34,4,52,84 | 75 | | | A39 G25 C20 T34 | A38 G27 C23 T33 |
| 1 | 44/61 or 82 or 9 | 44/61 | | | A40 G24 C20 T34 | A38 G26 C24 T33 |
| 2 | 5 or 58 | 5 | | | A40 G24 C20 T34 | A38 G27 C23 T33 |
| 3 | 1 | 1 | Ft. Sill (Cultured) | 2003 | A40 G24 C20 T34 | A38 G27 C23 T33 |
| 2 | 3 | 3 | | | A39 G25 C20 T34 | A38 G27 C23 T33 |
| 1 | 4 | 4 | | | A39 G25 C20 T34 | A38 G27 C23 T33 |
| 1 | 28 | 28 | | | A39 G25 C20 T34 | A38 G27 C23 T33 |
| 1 | 3 | 3 | Ft. | 2003 | A39 G25 C20 T34 | A38 G27 C23 T33 |
| 1 | 4 | 4 | Benning (Cultured) | | A39 G25 C20 T34 | A38 G27 C23 T33 |
| 3 | 6 | 6 | | | A40 G24 C20 T34 | A38 G27 C23 T33 |
| 1 | 11 | 11 | | | A39 G25 C20 T34 | A38 G27 C23 T33 |
| 1 | 13 | 94** | | | A40 G24 C20 T34 | A38 G27 C23 T33 |
| 1 | 44/61 or 82 or 9 | 82 | | | A40 G24 C20 T34 | A38 G26 C24 T33 |
| 1 | 5 or 58 | 58 | | | A40 G24 C20 T34 | A38 G27 C23 T33 |
| 1 | 78 or 89 | 89 | | | A39 G25 C20 T34 | A38 G27 C23 T33 |
| 2 | 5 or 58 | ND | Lackland AFB (Throat Swabs) | 2003 | A40 G24 C20 T34 | A38 G27 C23 T33 |
| 1 | 2 | | | | A39 G25 C20 T34 | A38 G27 C24 T32 |
| 1 | 81 or 90 | | | | A40 G24 C20 T34 | A38 G27 C23 T33 |
| 1 | 78 | | | | A38 G26 C20 T34 | A38 G27 C23 T33 |
| 3*** | No detection | | | | No detection | No detection |
| 7 | 3 | ND | MCRD San Diego (Throat Swabs) | 2002 | A39 G25 C20 T34 | A38 G27 C23 T33 |
| 1 | 3 | ND | | | No detection | A38 G27 C23 T33 |
| 1 | 3 | ND | | | No detection | No detection |
| 1 | 3 | ND | | | No detection | No detection |
| 2 | 3 | ND | | | No detection | A38 G27 C23 T33 |
| 3 | No detection | ND | | | No detection | No detection |

**Table 9B: Base Composition Analysis of Bioagent Identifying Amplicons of Group A Streptococcus samples from Six Military Installations Obtained with Primer Pair Nos. 438 and 441**

| **# of Instances** | **emm-type by Mass Spectrometry** | **emm-Gene Sequencing** | **Location (sample)** | **Year** | **xpt (Primer Pair No. 438)** | **yqiL (Primer Pair No. 441)** |
|---|---|---|---|---|---|---|
| 48 | 3 | 3 | MCRD San Diego (Cultured) | 2002 | A30 G36 C20 T36 | A40 G29 C19 T31 |
| 2 | 6 | 6 | | | A30 G36 C20 T36 | A40 G29 C19 T31 |
| 1 | 28 | 28 | | | A30 G36 C20 T36 | A41 G28 C18 T32 |
| 15 | 3 | ND | | | A30 G36 C20 T36 | A40 G29 C19 T31 |
| 6 | 3 | 3 | NHRC San Diego-Archive (Cultured) | 2003 | A30 G36 C20 T36 | A40 G29 C19 T31 |
| 3 | 5,58 | 5 | | | A30 G36 C20 T36 | A40 G29 C19 T31 |
| 6 | 6 | 6 | | | A30 G36 C20 T36 | A40 G29 C19 T31 |
| 1 | 11 | 11 | | | A30 G36 C20 T36 | A40 G29 C19 T31 |
| 3 | 12 | 12 | | | A30 G36 C19 T37 | A40 G29 C19 T31 |
| 1 | 22 | 22 | | | A30 G36 C20 T36 | A40 G29 C19 T31 |
| 3 | 25,75 | 75 | | | A30 G36 C20 T36 | A40 G29 C19 T31 |
| 4 | 44/61,82,9 | 44/61 | | | A30 G36 C20 T36 | A41 G28 C19 T31 |
| 2 | 53,91 | 91 | | | A30 G36 C19 T37 | A40 G29 C19 T31 |
| 1 | 2 | 2 | Ft. Leonard Wood (Cultured) | 2003 | A30 G36 C20 T36 | A40 G29 C19 T31 |
| 2 | 3 | 3 | | | A30 G36 C20 T36 | A40 G29 C19 T31 |
| 1 | 4 | 4 | | | A30 G36 C19 T37 | A41 G28 C19 T31 |
| 1 | 6 | 6 | | | A30 G36 C20 T36 | A40 G29 C19 T31 |
| 11 | 25 or 75 | 75 | | | A30 G36 C20 T36 | A40 G29 C19 T31 |
| 1 | 25,75, 33, 34,4,52,84 | 75 | | | A30 G36 C19 T37 | A40 G29 C19 T31 |
| 1 | 44/61 or 82 or 9 | 44/61 | | | A30 G36 C20 T36 | A41 G28 C19 T31 |
| 2 | 5 or 58 | 5 | | | A30 G36 C20 T36 | A40 G29 C19 T31 |
| 3 | 1 | 1 | Ft. Sill (Cultured) | 2003 | A30 G36 C19 T37 | A40 G29 C19 T31 |
| 2 | 3 | 3 | | | A30 G36 C20 T36 | A40 G29 C19 T31 |
| 1 | 4 | 4 | | | A30 G36 C19 T37 | A41 G28 C19 T31 |
| 1 | 28 | 28 | | | A30 G36 C20 T36 | A41 G28 C18 T32 |
| 1 | 3 | 3 | Ft. Benning (Cultured) | 2003 | A30 G36 C20 T36 | A40 G29 C19 T31 |
| 1 | 4 | 4 | | | A30 G36 C19 T37 | A41 G28 C19 T31 |
| 3 | 6 | 6 | | | A30 G36 C20 T36 | A40 G29 C19 T31 |
| 1 | 11 | 11 | | | A30 G36 C20 T36 | A40 G29 C19 T31 |
| 1 | 13 | 94** | | | A30 G36 C20 T36 | A41 G28 C19 T31 |
| 1 | 44/61 or 82 or 9 | 82 | | | A30 G36 C20 T36 | A41 G28 C19 T31 |
| 1 | 5 or 58 | 58 | | | A30 G36 C20 T36 | A40 G29 C19 T31 |
| 1 | 78 or 89 | 89 | | | A30 G36 C20 T36 | A41 G28 C19 T31 |
| 2 | 5 or 58 | ND | Lackland AFB (Throat Swabs) | 2003 | A30 G36 C20 T36 | A40 G29 C19 T31 |
| 1 | 2 | | | | A30 G36 C20 T36 | A40 G29 C19 T31 |
| 1 | 81 or 90 | | | | A30 G36 C20 T36 | A40 G29 C19 T31 |
| 1 | 78 | | | | A30 G36 C20 T36 | A41 G28 C19 T31 |
| 3*** | No detection | | | | No detection | No detection |
| 7 | 3 | ND | MCRD San Diego (Throat Swabs) | 2002 | A30 G36 C20 T36 | A40 G29 C19 T31 |
| 1 | 3 | ND | | | A30 G36 C20 T36 | A40 G29 C19 T31 |
| 1 | 3 | ND | | | A30 G36 C20 T36 | No detection |
| 1 | 3 | ND | | | No detection | A40 G29 C19 T31 |
| 2 | 3 | ND | | | A30 G36 C20 T36 | A40 G29 C19 T31 |
| 3 | No detection | ND | | | No detection | No detection |

**Table 9C: Base Composition Analysis of Bioagent Identifying Amplicons of Group A Streptococcus samples from Six Military Installations Obtained with Primer Pair Nos. 438 and 441**

| **# of Instances** | **emm-type by Mass Spectrometry** | **emm-Gene Sequencing** | **Location (sample)** | **Year** | **gki (Primer Pair No. 442)** | **gtr ((Primer Pair No. 443)** |
|---|---|---|---|---|---|---|
| 48 | 3 | 3 | MCRD San Diego (Cultured) | 2002 | A32 G35 C17 T32 | A39 G28 C16 T32 |
| 2 | 6 | 6 | | | A31 G35 C17 T33 | A39 G28 C15 T33 |
| 1 | 28 | 28 | | | A30 G36 C17 T33 | A39 G28 C16 T32 |
| 15 | 3 | ND | | | A32 G35 C17 T32 | A39 G28 C16 T32 |
| 6 | 3 | 3 | NHRC San Diego-Archive (Cultured) | 2003 | A32 G35 C17 T32 | A39 G28 C16 T32 |
| 3 | 5,58 | 5 | | | A30 G36 C20 T30 | A39 G28 C15 T33 |
| 6 | 6 | 6 | | | A31 G35 C17 T33 | A39 G28 C15 T33 |
| 1 | 11 | 11 | | | A30 G36 C20 T30 | A39 G28 C16 T32 |
| 3 | 12 | 12 | | | A31 G35 C17 T33 | A39 G28 C15 T33 |
| 1 | 22 | 22 | | | A31 G35 C17 T33 | A38 G29 C15 T33 |
| 3 | 25,75 | 75 | | | A30 G36 C17 T33 | A39 G28 C15 T33 |
| 4 | 44/61,82,9 | 44/61 | | | A30 G36 C18 T32 | A39 G28 C15 T33 |
| 2 | 53,91 | 91 | | | A32 G35 C17 T32 | A39 G28 C16 T32 |
| 1 | 2 | 2 | Ft. Leonard Wood (Cultured) | 2003 | A30 G36 C17 T33 | A39 G28 C15 T33 |
| 2 | 3 | 3 | | | A32 G35 C17 T32 | A39 G28 C16 T32 |
| 1 | 4 | 4 | | | A31 G35 C17 T33 | A39 G28 C15 T33 |
| 1 | 6 | 6 | | | A31 G35 C17 T33 | A39 G28 C15 T33 |
| 11 | 25 or 75 | 75 | | | A30 G36 C17 T33 | A39 G28 C15 T33 |
| 1 | 25,75, 33, 34,4,52,84 | 75 | | | A30 G36 C17 T33 | A39 G28 C15 T33 |
| 1 | 44/61 or 82 or 9 | 44/61 | | | A30 G36 C18 T32 | A39 G28 C15 T33 |
| 2 | 5 or 58 | 5 | | | A30 G36 C20 T30 | A39 G28 C15 T33 |
| 3 | 1 | 1 | Ft. Sill | 2003 | A30 G36 C18 T32 | A39 G28 C15 T33 |
| 2 | 3 | 3 | | | A32 G35 C17 T32 | A39 G28 C16 T32 |
| 1 | 4 | 4 | (Cultured) | | A31 G35 C17 T33 | A39 G28 C15 T33 |
| 1 | 28 | 28 | | | A30 G36 C17 T33 | A39 G28 C16 T32 |
| 1 | 3 | 3 | Ft. Benning (Cultured) | 2003 | A32 G35 C17 T32 | A39 G28 C16 T32 |
| 1 | 4 | 4 | | | A31 G35 C17 T33 | A39 G28 C15 T33 |
| 3 | 6 | 6 | | | A31 G35 C17 T33 | A39 G28 C15 T33 |
| 1 | 11 | 11 | | | A30 G36 C20 T30 | A39 G28 C16 T32 |
| 1 | 13 | 94** | | | A30 G36 C19 T31 | A39 G28 C15 T33 |
| 1 | 44/61 or 82 or 9 | 82 | | | A30 G36 C18 T32 | A39 G28 C15 T33 |
| 1 | 5 or 58 | 58 | | | A30 G36 C20 T30 | A39 G28 C15 T33 |
| 1 | 78 or 89 | 89 | | | A30 G36 C18 T32 | A39 G28 C15 T33 |
| 2 | 5 or 58 | ND | Lackland AFB (Throat Swabs) | 2003 | A30 G36 C20 T30 | A39 G28 C15 T33 |
| 1 | 2 | | | | A30 G36 C17 T33 | A39 G28 C15 T33 |
| 1 | 81 or 90 | | | | A30 G36 C17 T33 | A39 G28 C15 T33 |
| 1 | 78 | | | | A30 G36 C18 T32 | A39 G28 C15 T33 |
| 3*** | No detection | | | | No detection | No detection |
| 7 | 3 | ND | MCRD San Diego (Throat Swabs) | 2002 | A32 G35 C17 T32 | A39 G28 C16 T32 |
| 1 | 3 | ND | | | No detection | No detection |
| 1 | 3 | ND | | | A32 G35 C17 T32 | A39 G28 C16 T32 |
| 1 | 3 | ND | | | A32 G35 C17 T32 | No detection |
| 2 | 3 | ND | | | A32 G35 C17 T32 No | detection |
| 3 | No detection | ND | | | No detection | No detection |

### Example 8: Design of Calibrant Polynucleotides based on Bioagent Identifying Amplicons for Identification of Species of Bacteria (Bacterial Bioagent Identifying Amplicons)

This example describes the design of 19 calibrant polynucleotides based on bacterial bioagent identifying amplicons corresponding to the primers of the broad surveillance set (Table 5) and the *Bacillus anthracis* drill-down set (Table 6).

Calibration sequences were designed to simulate bacterial bioagent identifying amplicons produced by the T modified primer pairs shown in Tables 5 and 6 (primer names have the designation "TMOD"). The calibration sequences were chosen as a representative member of the section of bacterial genome from specific bacterial species which would be amplified by a given primer pair. The model bacterial species upon which the calibration sequences are based are also shown in Table 10. For example, the calibration sequence chosen to correspond to an amplicon produced by primer pair no. 361 is SEQ ID NO: 1445. In Table 10, the forward (_F) or reverse (_R) primer name indicates the coordinates of an extraction representing a gene of a standard reference bacterial genome to which the primer hybridizes e.g.: the forward primer name 16S_EC_713 732_TMOD_F indicates that the forward primer hybridizes to residues 713-732 of the gene encoding 16S ribosomal RNA in an *E*. *coli* reference sequence (in this case, the reference sequence is an extraction consisting of residues 4033120-4034661 of the genomic sequence of *E*. *coli* K12 (GenBank gi number 16127994). Additional gene coordinate reference information is shown in Table 11. The designation "TMOD" in the primer names indicates that the 5' end of the primer has been modified with a non-matched template T residue which prevents the PCR polymerase from adding non-templated adenosine residues to the 5' end of the amplification product, an occurrence which may result in miscalculation of base composition from molecular mass data (*vide supra*)*.*

The 19 calibration sequences described in Tables 10 and 11 were combined into a single calibration polynucleotide sequence (SEQ ID NO: 1464 - which is herein designated a "combination calibration polynucleotide") which was then cloned into a pCR^{®}-Blunt vector (Invitrogen, Carlsbad, CA). This combination calibration polynucleotide can be used in conjunction with the primers of Tables 5 or 6 as an internal standard to produce calibration amplicons for use in determination of the quantity of any bacterial bioagent. Thus, for example, when the combination calibration polynucleotide vector is present in an amplification reaction mixture, a calibration amplicon based on primer pair 346 (16S rRNA) will be produced in an amplification reaction with primer pair 346 and a calibration amplicon based on primer pair 363 (rpoC) will be produced with primer pair 363. Coordinates of each of the 19 calibration sequences within the calibration polynucleotide (SEQ ID NO: 1464) are indicated in Table 11.

**Table 10: Bacterial Primer Pairs for Production of Bacterial Bioagent Identifying Amplicons and Corresponding Representative Calibration Sequences**

| **Primer Pair No.** | **Forward Primer Name** | **Forward Primer (SEQ ID NO:)** | **Reverse Primer Name** | **Reverse Primer (SEQ ID NO:)** | **Calibration Sequence Model Species** | **Calibration Sequence (SEQ ID NO:)** |
|---|---|---|---|---|---|---|
| 361 | 16S_EC_1090_1111_2_T MOD_F | 697 | 16S_EC_1175_1196_TMOD_R | 1398 | *Bacillus anthracis* | 1445 |
| 346 | 16S_EC_713_732_TMOD_ F | 202 | 16S_EC_789_809_TMOD_R | 1110 | *Bacillus anthracis* | 1446 |
| 347 | 16S_EC_785_606_TMOD_ F | 560 | 16S_EC_880_897_TMOD_R | 1278 | *Bacillus anthracis* | 1447 |
| 348 | 16S_EC_960_981_TMOD_ F | 706 | 16S_EC_1054_1073_TMOD_R | 895 | *Bacillus anthracis* | 1448 |
| 349 | 23S_EC_1826_1843_TMO D_F | 401 | 23S_EC_1906_1924_TMOD_R | 1156 | *Bacillus anthracis* | 1449 |
| 360 | 23S_EC_2646_2667_TMO D_F | 409 | 23S_EC_2745_2765_TMOD_R | 1434 | *Bacillus anthracis* | 1450 |
| 350 | CAPC_BA_274_303_TMOD _F | 476 | CAPC_BA_349_376_TMOD_R | 1314 | *Bacillus anthracis* | 1451 |
| 351 | CYA_BA_1353_1379_TMO D_F | 355 | CYA_BA_1448_1467_TMOD_R | 1423 | *Bacillus anthracis* | 1452 |
| 352 | INFB_EC_1365_1393_TM OD_F | 687 | INFB_EC_1439_1467_TMOD_ R | 1411 | *Bacillus anthracis* | 1453 |
| 353 | LEF_BA_756_781_TMOD_ F | 220 | LEF_BA_843_872_TMOD_R | 1394 | *Bacillus anthracis* | 1454 |
| 356 | RPLB_EC_650_679_TMOD _F | 449 | RPLB_EC_739_762_TMOD_R | 1380 | *Clostridium botulinum* | 1455 |
| 449 | RPLB_EC_690_710_F | 309 | RPLB_EC_737_758_R | 1336 | *Clostridium botulinum* | 1456 |
| 359 | RPOB_EC_1845_1866_TM OD_F | 659 | RPOB_EC_1909_1929_TMOD_ R | 1250 | *Yersinia Pestis* | 1457 |
| 362 | RPOB_EC_3799_3821_TM OD_F | 581 | RPOB_EC_3862_3888_TMOD_ R | 1325 | *Burkholderia mallei* | 1458 |
| 363 | RPOC EC 2146 2174 TM OD_F | 284 | RPOC_EC_2227_224S_TMOD_ R | 898 | *Burkholderia mallei* | 1459 |
| 354 | RPOC_EC_2218_2241_TM OD_F | 405 | RPOC_EC_2 313_2337_TMOD_ R | 1072 | *Bacillus anthracis* | 1460 |
| 355 | SSPE_BA_215_137_TMOD _F | 255 | SSPE_BA_197_222-TMOD_R R | 1402 | *Bacillus anthracis* | 1461 |
| 367 | TUFB_EC_957_979_TMOD _F | 308 | TUFB_EC_1034_1058_TMOD_ R | 1276 | *Burkholderia mallei* | 1462 |
| 358 | VALS_EC_1105_1124_TM OD_F | 385 | VALS_EC_1195_1218_TMOD_ R | 1093 | *Yersinia Pestis* | 1463 |

**Table 11: Primer Pair Gene Coordinate References and Calibration Polynucleotide Sequence Coordinates within the Combination Calibration Polynucleotide**

| **Bacterial Gene and Species** | **Gene Extraction Coordinates of Genomic or Plasmid Sequence** | **Reference GenBank GI No. of Genomic (G) or Plasmid (P) Sequence** | **Primer Pair No.** | **Coordinates of Calibration Sequence in Combination Calibration Polynucleotide (SEQ ID NO: 1464)** |
|---|---|---|---|---|
| 16S *E*. *coli* | 4033120..4034661 | 16127994 (G) | 346 | 16..109 |
| 16S *E*. *coli* | 4033120..4034661 | 16127994 (G) | 347 | 83..190 |
| 16S *E*. *coli* | 4033120..4034661 | 16127994 (G) | 348 | 246..353 |
| 16S *E*. *coli* | 4033120..4034661 | 16127994 (G) | 361 | 368..469 |
| 23S *E*. *coli* | 4166220..4169123 | 16127994 (G) | 349 | 743..837 |
| 23S *E*. *coli* | 4166220..4169123 | 16127994 (G) | 360 | 865..981 |
| rpoB *E*. *coli*. | 4178823..4182851 (complement strand) | 16127994 (G) | 359 | 1591..1672 |
| rpoB *E*. *coli* | 4178823..4182851 (complement strand) | 16127994 (G) | 362 | 2081..2167 |
| rpoC *E*. *coli* | 4182928..4187151 | 16127994 (G) | 354 | 1810..1926 |
| rpoC *E*. *coli* | 4182928..4187151 | 16127994 (G) | 363 | 2183..2279 |
| infB *E*. *coli* | 3313655..3310983 (complement strand) | 16127994 (G) | 352 | 1692..1791 |
| tufB *E*. *coli* | 4173523..4174707 | 16127994 (G) | 367 | 2400..2498 |
| rplB *E*. *coli* | 3449001..3448180 | 16127994 (G) | 356 | 1945..2060 |
| rplB *E*. *coli* | 3449001..3448180 | 16127994 (G) | 449 | 1986..2055 |
| valS *E*. *coli* | 4481405..4478550 (complement strand) | 16127994 (G) | 358 | 1462..1572 |
| capC *B. anthracis* | 56074..55628 (complement strand) | 6470151 (P) | 350 | 2517..2616 |
| cya *B. anthracis* | 156626..154288 (complement strand) | 4894216 (P) | 351 | 1338..1449 |
| lef *B. anthracis* | 127442..129921 | 4894216 (P) | 353 | 1121..1234 |
| sspE *B. anthracis* | 226496..226783 | 30253828 (G) | 355 | 1007-1104 |

### Example 9: Use of a Calibration Polynucleotide for Determining the Quantity of Bacillus Anthracis in a Sample Containing a Mixture of Microbes

The process described in this example is shown in Figure 2. The capC gene is a gene involved in capsule synthesis which resides on the pX02 plasmid of *Bacillus anthracis.* Primer pair number 350 (see Tables 10 and 11) was designed to identify *Bacillus anthracis* via production of a bacterial bioagent identifying amplicon. Known quantities of the combination calibration polynucleotide vector described in Example 8 were added to amplification mixtures containing bacterial bioagent nucleic acid from a mixture of microbes which included the Ames strain of *Bacillus anthracis.* Upon amplification of the bacterial bioagent nucleic acid and the combination calibration polynucleotide vector with primer pair no. 350, bacterial bioagent identifying amplicons and calibration amplicons were obtained and characterized by mass spectrometry. A mass spectrum measured for the amplification reaction is shown in Figure 7. The molecular masses of the bioagent identifying amplicons provided the means for identification of the bioagent from which they were obtained (Ames strain of *Bacillus anthracis*) and the molecular masses of the calibration amplicons provided the means for their identification as well. The relationship between the abundance (peak height) of the calibration amplicon signals and the bacterial bioagent identifying amplicon signals provides the means of calculation of the copies of the pX02 plasmid of the Ames strain of *Bacillus anthracis.* Methods of calculating quantities of molecules based on internal calibration procedures are well known to those of ordinary skill in the art.

Averaging the results of 10 repetitions of the experiment described above, enabled a calculation that indicated that the quantity of Ames strain of *Bacillus anthracis* present in the sample corresponds to approximately 10 copies of pX02 plasmid.

### Example 10: Triangulation Genotyping Analysis of Campylobacter Species

A series of triangulation genotyping analysis primers were designed as described in Example 1 with the objective of identification of different strains of *Campylobacter jejuni.* The primers are listed in Table 12 with the designation "CJST_CJ." Housekeeping genes to which the primers hybridize and produce bioagent identifying amplicons include: tkt (transketolase), glyA (serine hydroxymethyltransferase), gltA (citrate synthase), aspA (aspartate ammonia lyase), glnA (glutamine synthase), pgm (phosphoglycerate mutase), and uncA (ATP synthetase alpha chain).

**Table 12: Campylobacter Genotyping Primer Pairs**

| **Primer Pair No.** | **Forward Primer Name** | **Forward Primer (SEQ ID NO:)** | **Reverse Primer Name** | **Reverse Primer (SEQ ID NO:)** | **Target Gene** |
|---|---|---|---|---|---|
| 1053 | CJST CJ 1080 1110 F | 681 | CJST CJ 1166 1198 R | 1022 | gltA |
| 1047 | CJST CJ 584 616 F | 315 | CJST CJ 663 692 R | 1379 | glnA |
| 1048 | CJST CJ 360 394 F | 346 | CJST CJ 442 476 R | 955 | aspA |
| 1049 | CJST CJ 2636 2668 F | 504 | CJST CJ 2753 2777 R | 1409 | tkt |
| 1054 | CJST CJ 2060 2090 F | 323 | CJST CJ 2148 2174 R | 1068 | pgm |
| 1064 | CJST CJ 1680 1713 F | 479 | CJST CJ 1795 1822 R | 938 | glyA |

The primers were used to amplify nucleic acid from 50 food product samples provided by the USDA, 25 of which contained *Campylobacter jejuni* and 25 of which contained *Campylobacter coli.* Primers used in this study were developed primarily for the discrimination of *Campylobacter jejuni* clonal complexes and for distinguishing *Campylobacter jejuni* from *Campylobacter coli.* Finer discrimination between *Campylobacter coli* types is also possible by using specific primers targeted to loci where closely-related *Campylobacter coli* isolates demonstrate polymorphisms between strains. The conclusions of the comparison of base composition analysis with sequence analysis are shown in Tables 13A-C.

**Table 13A - Results of Base Composition Analysis of 50 Campylobacter Samples with Drill-down MLST Primer Pair Nos: 1048 and 1047**

| **Group** | **species** | **Isolate origin** | **MLST type or Clonal Complex by Base Composition analysis** | **MLST Type or Clonal Complex by Sequence analysis** | **Strain** | **Base Composition of Bioagent Identifying Amplicon Obtained with Primer Pair No: 1048 (aspA)** | **Base Composition of Bioagent Identifying Amplicon Obtained with Primer Pair No: 1047 (glnA)** |
|---|---|---|---|---|---|---|---|
| J-1 | *C.jejuni* | Goose | ST 690 /692/707/991 | ST 991 | RM3673 | A30 G25 C16 T46 | A47 G21 C16 T25 |
| J-2 | *C. jejuni* | Human | Complex 206/48/353 | ST 356, complex 353 | RM4192 | A30 G25 C16 T46 | A48 G21 C17 T23 |
| J-3 | *C*. *jejuni* | Human | Complex 354/179 | ST 436 | RM4194 | A30 G25 C15 T47 | A48 G21 C18 T22 |
| J-4 | *C. jejuni* | Human | Complex 257 | ST 257, complex 257 | RM4197 | A30 G25 C16 T46 | A48 G21 C18 T22 |
| J-5 | *C. jejuni* | Human | Complex 52 | ST 52, complex 52 | RM4277 | A30 G25 C16 T46 | A48 G21 C17 T23 |
| J-6 | *C*. *jejuni* | Human | Complex 443 | ST 51, complex 443 | RM4275 | A30 G25 C15 T47 | A48 G21 C17 T23 |
| | | | | | RM4279 | A30 G25 C15 T47 | A48 G21 C17 T23 |
| J-7 | *C. jejuni* | Human | Complex 42 | ST 604, complex 42 | RM1864 | A30 G25 C15 T47 | A48 G21 C18 T22 |
| J-8 | *C. jejuni* | Human | Complex 42/49/362 | ST 362, complex 362 | RM3193 | A30 G25 C15 T47 | A48 G21 C18 T22 |
| J-9 | *C*. *jejuni* | Human | Complex 45/283 | ST 147, Complex 45 | RM3203 | A30 G25 C15 T47 | A47 G21 C18 T23 |
| | *C. jejuni* | Human | Consistent with 74 closely related sequence types (none belong to a clonal complex) | ST 828 | RM4183 | A31 G27 C20 T39 | A48 G21 C16 T24 |
| C-1 | *C. coli* | | | ST 832 | RM1169 | A31 G27 C20 T39 | A48 G21 C16 T24 |
| | | | | ST 1056 | RM1857 | A31 G27 C20 T39 | A48 G21 C16 T24 |
| | | Poultry | | ST 889 | RM1166 | A31 G27 C20 T39 | A48 G21 C16 T24 |
| | | | | ST 829 | RM1182 | A31 G27 C20 T39 | A48 G21 C16 T24 |
| | | | | ST 1050 | RM1518 | A31 G27 C20 T39 | A48 G21 C16 T24 |
| | | | | ST 1051 | RM1521 | A31 G27 C20 T39 | A48 G21 C16 T24 |
| | | | | ST 1053 | RM1523 | A31 G27 C20 T39 | A48 G21 C16 T24 |
| | | | | ST 1055 | RM1527 | A31 G27 C20 T39 | A48 G21 C16 T24 |
| | | | | ST 1017 | RM1529 | A31 G27 C20 T39 | A48 G21 C16 T24 |
| | | | | ST 860 | RM1840 | A31 G27 C20 T39 | A48 G21 C16 T24 |
| | | | | ST 1063 | RM2219 | A31 G27 C20 T39 | A48 G21 C16 T24 |
| | | | | ST 1066 | RM2241 | A31 G27 C20 T39 | A48 G21 C16 T24 |
| | | | | ST 1067 | R2243 | A31 G27 C20 T39 | A48 G21 C16 T24 |
| | | | | ST 1068 | RM2439 | A31 G27 C20 T39 | A48 G21 C16 T24 |
| | | Swine | | ST 1016 | RM3230 | A31 G27 C20 T39 | A48 G21 C16 T24 |
| | | | | ST 1069 | RM3231 | A31 G27 C20 T39 | A48 G21 C16 T24 |
| | | | | ST 1061 | RM1904 | A31 G27 C20 T39 | A48 G21 C16 T24 |
| | | Unknown | | ST 825 | RMIS34 | A31 G27 C20 T39 | A48 G21 C16 T24 |
| | | | | ST 901 | RM1505 | A31 G27 C20 T39 | A48 G21 C16 T24 |
| *C-2* | *C. coli* | Human | ST 895 | ST 895 | RM1532 | A31 G27 C19 T40 | A48 G21 C16 T24 |
| *C-3* | *C. coli* | Poultry | Consistent | ST 1064 | RM2223 | A31 G27 C20 T39 | A48 G21 C16 T24 |
| | | | with 63 closely related sequence types (none belong to a clonal complex) | ST 1082 | RM1178 | A31 G27 C20 T39 | A48 G21 C16 T24 |
| | | | | ST 1054 | RM1525 | A31 G27 C20 T39 | A48 G21 C16 T24 |
| | | | | ST 1049 | RM1517 | A31 G27 C20 T39 | A48 G21 C16 T24 |
| | | Marmoset | | ST 891 | RM1531 | A31 G27 C20 T39 | A48 G21 C16 T24 |

**Table 13B - Results of Base Composition Analysis of 50 Campylobacter Samples with Drill-down MLST Primer Pair Nos: 1053 and 1064**

| **Group** | **species** | **Isolate origin** | **MLST type or Clonal Complex by Base Composition analysis** | **MLST Type or Clonal Complex by Sequence analysis** | **Strain** | **Base Composition of Bioagent Identifying Amplicon Obtained with Primer Pair No: 1053 (gltA)** | **Base Composition of Bioagent Identifying Amplicon Obtained with Primer Pair No: 1064 (glyA)** |
|---|---|---|---|---|---|---|---|
| **J-1** | *C. jejuni* | Goose | ST 690 /692/707/991 | ST 991 | RM3673 | A24 G25 C23 T47 | A40 G29 C29 T45 A40 G29 |
| **J-2** | *C. jejuni* | Human | Complex 206/48/353 | ST 356, complex 353 | RM4192 | A24 G25 C23 T47 | A40 G29 C29 T45 |
| **J-3** | *C*. *jejuni* | Human | Complex 354/179 | ST 436 | RM4194 | A24 G25 C23 T47 | A40 G29 C29 T45 |
| **J-4** | *C. jejuni* | Human | Complex 257 | ST 257, complex 257 | RM4197 | A24 G25 C23 T47 | A40 G29 C29 T45 |
| **J-5** | *C*. *jejuni* | Human | Complex 52 | ST 52, complex 52 | RM4277 | A24 G25 C23 T47 | A39 G30 C26 T48 |
| **J-6** | *C*. *jejuni* | Human | Complex 443 | ST 51, complex 443 | RM4275 | A24 G25 C23 T47 | A39 G30 C28 T46 |
| | | | | | RM4279 | A24 G25 C23 T47 | A39 G30 C28 T46 |
| **J-7** | *C*. *jejuni* | Human | Complex 42 | ST 604, complex 42 | RM1864 | A24 G25 C23 T47 | A39 G30 C26 T48 |
| **J-8** | *C. jejuni* | Human | Complex 42/49/362 | ST 362, complex 362 | RM3193 | A24 G25 C23 T47 | A38 G31 C28 T46 |
| **J-9** | *C. jejuni* | Human | Complex 45/283 | ST 147, Complex 45 | RM3203 | A24 G25 C23 T47 | A38 G31 C28 T46 |
| | *C. jejuni* | Human | Consistent with 74 closely related sequence types (none belong to a clonal complex) | ST 828 | RM4183 | A23 G24 C26 T46 | A39 G30 C27 T47 |
| **C-1** | *C. coli* | | | ST 832 | RM1169 | A23 G24 C26 T46 | A39 G30 C27 T47 |
| | | | | ST 1056 | RM1857 | A23 G24 C26 T46 | A39 G30 C27 T47 |
| | | Poultry | | ST 889 | RM1166 | A23 G24 C26 T46 | A39 G30 C27 T47 |
| | | | | ST 829 | RM1182 | A23 G24 C26 T46 | A39 G30 C27 T47 |
| | | | | ST 1050 | RM1518 | A23 G24 C26 T46 | A39 G30 C27 T47 |
| | | | | ST 1051 | RM1521 | A23 G24 C26 T46 | A39 G30 C27 T47 |
| | | | | ST 1053 | RM1523 | A23 G24 C26 T46 | A39 G30 C27 T47 |
| | | | | ST 1055 | RM1527 | A23 G24 C26 T46 | A39 G30 C27 T47 |
| | | | | ST 1017 | RM1529 | A23 G24 C26 T46 | A39 G30 C27 T47 |
| | | | | ST 860 | RM1840 | A23 G24 C26 T46 | A39 G30 C27 T47 |
| | | | | ST 1063 | RM2219 | A23 G24 C26 T46 | A39 G30 C27 T47 |
| | | | | ST 1066 | RM2241 | A23 G24 C26 T46 | A39 G30 C27 T47 |
| | | | | ST 1067 | RM2243 | A23 G24 C26 T46 | A39 G30 C27 T47 |
| | | | | ST 1068 | RM2439 | A23 G24 C26 T46 | A39 G30 C27 T47 |
| | | Swine | | ST 1016 | RM3230 | A23 G24 C26 T46 | A39 G30 C27 T47 |
| | | | | ST 1069 | RM3231 | A23 G24 C26 T46 | NO DATA |
| | | | | ST 1061 | RM1904 | A23 G24 C26 T46 | A39 G30 C27 T47 |
| | | Unknown | | ST 825 | RM1534 | A23 G24 C26 T46 | A39 G30 C27 T47 |
| | | | | ST 901 | RM1505 | A23 G24 C26 T46 | A39 G30 C27 T47 |
| C-2 | C. coli | Human | ST 895 | ST 895 | RM1532 | A23 G24 C26 T46 | A39 G30 C27 T47 |
| C-3 | C. coli | Poultry | Consistent with 63 closely related sequence types (none belong to a clonal complex) | ST 1064 | RM2223 | A23 G24 C26 T46 | A39 G30 C27 T47 |
| | | | | ST 1082 | RM1178 | A23 G24 C26 T46 | A39 G30 C27 T47 |
| | | | | ST 1054 | RM1525 | A23 G24 C25 T47 | A39 G30 C27 T47 |
| | | | | ST 1049 | RM1517 | A23 G24 C26 T46 | A39 G30 C27 T47 |
| | | Marmoset | | ST 891 | RM1531 | A23 G24 C26 T46 | A39 G30 C27 T47 |

**Table 13C - Results of Base Composition Analysis of 50 Campylobacter Samples with Drill-down MLST Primer Pair Nos: 1054 and 1049**

| **Group** | **Species** | **Isolate origin** | **MLST type or Clonal Complex by Base Composition analysis** | **MLST Type or Clonal Complex by Sequence analysis** | **Strain** | **Base Composition of Bioagent Identifying Amplicon Obtained with Primer Pair No: 1054 (pgm)** | **Base Composition of Bioagent Identifying Amplicon Obtained with Primer Pair No: 1049 (tkt)** |
|---|---|---|---|---|---|---|---|
| **J-1** | *C. jejuni* | Goose | ST 690 /692/707/991 | ST 991 | RM3673 | A26 G33 C18 T38 | A41 G28 C35 T38 |
| **J-2** | *C. jejuni* | Human | Complex 206/48/353 | ST 356, complex 353 | RM4192 | A26 G33 C19 T37 | A41 G28 C36 T37 |
| **J-3** | *C. jejuni* | Human | Complex 354/179 | ST 436 | RM4194 | A27 G32 C19 T37 | A42 G28 C36 T36 |
| **J-4** | *C. jejuni* | Human | Complex 257 | ST 257, complex 257 | RM4197 | A27 G32 C19 T37 | A41 G29 C35 T37 |
| **J-5** | *C. jejuni* | Human | Complex 52 | ST 52, complex 52 | RM4277 | A26 G33 C18 T38 | A41 G28 C36 T37 |
| **J-6** | *C. jejuni* | Human | Complex 443 | ST 51, complex 443 | RM4275 | A27 G31 C19 T38 | A41 G28 C36 T37 |
| | | | | | RM4279 | A27 G31 C19 T38 | A41 G28 C36 T37 |
| **J-7** | *C. jejuni* | Human | Complex 42 | ST 604, complex 42 | RM1864 | A27 G32 C19 T37 | A42 G28 C35 T37 |
| **J-8** | *C. jejuni* | Human | Complex 42/49/362 | ST 362, complex 362 | RM3193 | A26 G33 C19 T37 | A42 G28 C35 T37 |
| **J-9** | *C. jejuni* | Human | Complex 45/283 | ST 147, Complex 45 | RM3203 | A28 G31 C19 T37 | A43 G28 C36 T35 |
| | *C. jejuni* | Human | Consistent with 74 closely related sequence types (none belong to a clonal complex) | ST 828 | RM4183 | A27 G30 C19 T39 | A46 G28 C32 T36 |
| **C-1** | *C. coli* | | | ST 832 | RM1169 | A27 G30 C19 T39 | A46 G28 C32 T36 |
| | | | | ST 1056 | RM1857 | A27 G30 C19 T39 | A46 G28 C32 T36 |
| | | Poultry | | ST 889 | RM1166 | A27 G30 C19 T39 | A46 G28 C32 T36 |
| | | | | ST 829 | RM1182 | A27 G30 C19 T39 | A46 G28 C32 T36 |
| | | | | ST 1050 | RM1518 | A27 G30 C19 T39 | A46 G28 C32 T36 |
| | | | | ST 1051 | RM1521 | A27 G30 C19 T39 | A46 G28 C32 T36 |
| | | | | ST 1053 | RM1523 | A27 G30 C19 T39 | A46 G28 C32 T36 |
| | | | | ST 1055 | RM1527 | A27 G30 C19 T39 | A46 G28 C32 T36 |
| | | | | ST 1017 | RM1529 | A27 G30 C19 T39 | A46 G28 C32 T36 |
| | | | | ST 860 | RM1840 | A27 G30 C19 T39 | A46 G28 C32 T36 |
| | | | | ST 1063 | RM2219 | A27 G30 C19 T39 | A46 G28 C32 T36 |
| | | | | ST 1066 | RM2241 | A27 G30 C19 T39 | A46 G28 C32 T36 |
| | | | | ST 1067 | RM2243 | A27 G30 C19 T39 | A46 G28 C32 T36 |
| | | | | ST 1068 | RM2439 | A27 G30 C19 T39 | A46 G28 C32 T36 |
| | | Swine | | ST 1016 | RM3230 | A27 G30 C19 T39 | A46 G28 C32 T36 |
| | | | | ST 1069 | RM3231 | A27 G30 C19 T39 | A46 G28 C32 T36 |
| | | | | ST 1061 | RM1904 | A27 G30 C19 T39 | A46 G28 C32 T36 |
| | | Unknown | | ST 825 | RM1534 | A27 G30 C19 T39 | A46 G28 C32 T36 |
| | | | | ST 901 | RM1505 | A27 G30 C19 T39 | A46 G28 C32 T36 |
| C-2 | *C. coli* | Human | ST 895 | ST 895 | RM1532 | A27 G30 C19 T39 | A45 G29 C32 T36 |
| C-3 | *C. coli* | Poultry | Consistent with 63 closely related sequence types (none belong to a clonal complex) | ST 1064 | RM2223 | A27 G30 C19 T39 | A45 G29 C32 T36 |
| | | | | ST 1082 | RM1178 | A27 G30 C19 T39 | A45 G29 C32 T36 |
| | | | | ST 1054 | RM1525 | A27 G30 C19 T39 | A45 G29 C32 T36 |
| | | | | ST 1049 | RM1517 | A27 G30 C19 T39 | A45 G29 C32 T36 |
| | | Marmoset | | ST 891 | RM1531 | A27 G30 C19 T39 | A45 G29 C32 T36 |

The base composition analysis method was successful in identification of 12 different strain groups. *Campylobacter jejuni* and *Campylobacter coli* are generally differentiated by all loci. Ten clearly differentiated *Campylobacter jejuni* isolates and 2 major *Campylobacter coli* groups were identified even though the primers were designed for strain typing of *Campylobacter jejuni.* One isolate (RM4183) which was designated as *Campylobacter jejuni* was found to group with *Campylobacter coli* and also appears to actually be *Campylobacter coli* by full MLST sequencing.

### Example 11: Identification of Acinetobacter baumannii Using Broad Range Survey and Division-Wide Primers in Epidemiological Surveillance

To test the capability of the broad range survey and division-wide primer sets of Table 5 in identification of *Acinetobacter* species, 183 clinical samples were obtained from individuals participating in, or in contact with individuals participating in Operation Iraqi Freedom (including US service personnel, US civilian patients at the Walter Reed Army Institute of Research (WRAIR), medical staff, Iraqi civilians and enemy prisoners. In addition, 34 environmental samples were obtained from hospitals in Iraq, Kuwait, Germany, the United States and the USNS Comfort, a hospital ship.

Upon amplification of nucleic acid obtained from the clinical samples, primer pairs 346-349, 360, 361, 354, 362 and 363 (Table 5) all produced bacterial bioagent amplicons which identified *Acinetobacter baumannii* in 215 of 217 samples. The organism *Klebsiella pneumoniae* was identified in the remaining two samples. In addition, 14 different strain types (containing single nucleotide polymorphisms relative to a reference strain of *Acinetobacter baumannii*) were identified and assigned arbitrary numbers from 1 to 14. Strain type 1 was found in 134 of the sample isolates and strains 3 and 7 were found in 46 and 9 of the isolates respectively.

The epidemiology of strain type 7 of *Acinetobacter baumannii* was investigated. Strain 7 was found in 4 patients and 5 environmental samples (from field hospitals in Iraq and Kuwait). The index patient infected with strain 7 was a pre-war patient who had a traumatic amputation in March of 2003 and was treated at a Kuwaiti hospital. The patient was subsequently transferred to a hospital in Germany and then to WRAIR. Two other patients from Kuwait infected with strain 7 were found to be non-infectious and were not further monitored. The fourth patient was diagnosed with a strain 7 infection in September of 2003 at WRAIR. Since the fourth patient was not related involved in Operation Iraqi Freedom, it was inferred that the fourth patient was the subject of a nosocomial infection acquired at WRAIR as a result of the spread of strain 7 from the index patient.

The epidemiology of strain type 3 of *Acinetobacter baumannii* was also investigated. Strain type 3 was found in 46 samples, all of which were from patients (US service members, Iraqi civilians and enemy prisoners) who were treated on the USNS Comfort hospital ship and subsequently returned to Iraq or Kuwait. The occurrence of strain type 3 in a single locale may provide evidence that at least some of the infections at that locale were a result of nosocomial infections.

This example thus illustrates an embodiment of the present invention wherein the methods of analysis of bacterial bioagent identifying amplicons provide the means for epidemiological surveillance.

### Example 12: Selection and Use of Triangulation Genotyping Analysis Primer Pairs for Acinetobacter baumanii

To combine the power of high-throughput mass spectrometric analysis of bioagent identifying amplicons with the sub-species characteristic resolving power provided by triangulation genotyping analysis, an additional 21 primer pairs were selected based on analysis of housekeeping genes of the genus *Acinetobacter.* Genes to which the drill-down triangulation genotyping analysis primers hybridize for production of bacterial bioagent identifying amplicons include anthranilate synthase component I (trpE), adenylate kinase (adk), adenine glycosylase (mutY), fumarate hydratase (fumC), and pyrophosphate phospho-hydratase (ppa). These 21 primer pairs are indicated with reference to sequence listings in Table 14. Primer pair numbers 1151-1154 hybridize to and amplify segments of trpE. Primer pair numbers 1155-1157 hybridize to and amplify segments of adk. Primer pair numbers 1158-1164 hybridize to and amplify segments of mutY. Primer pair numbers 1165-1170 hybridize to and amplify segments of fumC. Primer pair number 1171 hybridizes to and amplifies a segment of ppa. Primer pair numbers: 2846-2848 hybridize to and amplify segments of the parC gene of DNA topoisomerase which include a codon known to confer quinolone drug resistance upon sub-types of *Acinetobacter baumannii.* Primer pair numbers 2852-2854 hybridize to and amplify segments of the gyrA gene of DNA gyrase which include a codon known to confer quinolone drug resistance upon sub-types of *Acinetobacter baumannii.* Primer pair numbers 2922 and 2972 are speciating primers which are useful for identifying different species members of the genus *Acinetobacter.* The primer names given in Table 14A (with the exception of primer pair numbers 2846-2848, 2852-2854) indicate the coordinates to which the primers hybridize to a reference sequence which comprises a concatenation of the genes TrpE, efp (elongation factor p), adk, mutT, fumC, and ppa. For example, the forward primer of primer pair 1151 is named AB_MLST-11-OIF007_62_91_F because it hybridizes to the *Acinetobacter* primer reference sequence of strain type 11 in sample 007 of Operation Iraqi Freedom (OIF) at positions 62 to 91. DNA was sequenced from strain type 11 and from this sequence data and an artificial concatenated sequence of partial gene extractions was assembled for use in design of the triangulation genotyping analysis primers. The stretches of arbitrary residues "N"s in the concatenated sequence were added for the convenience of separation of the partial gene extractions (40N for AB_MLST (SEQ ID NO: 1444)).

The hybridization coordinates of primer pair numbers 2846-2848 are with respect to GenBank Accession number X95819. The hybridization coordinates of primer pair numbers 2852-2854 are with respect to GenBank Accession number AY642140. Sequence residue "I" appearing in the forward and reverse primers of primer pair number 2972 represents inosine.

**Table 14A: Triangulation Genotyping Analysis Primer Pairs for Identification of Sub-species characteristics (Strain Type) of Members of the Bacterial Genus Acinetobacter**

| **Primer Pair No.** | **Forward Primer Name** | **Forward Primer (SEQ ID NO:)** | **Reverse Primer Name** | **Reverse Primer (SEQ ID NO:)** |
|---|---|---|---|---|
| 1151 | AB_MLST-11-OIF007 62 91 F | 454 | AB_MLST-11-OIF007 169 203 R | 1418 |
| 1152 | AB_MLST-11-OIF007 185 214 F | 243 | AB_MLST-11-OIF007 291 324 R | 969 |
| 1153 | AB_MLST-11-OIF007 260 289 F | 541 | AB_MLST-11-OIF007 364 393 R | 1400 |
| 1154 | AB_MLST-11-OIF007 206 239 F | 436 | AB_MLST-11-OIF007 318 344 R | 1036 |
| 1155 | AB_MLST-11-OIF007 522 552 F | 378 | AB_MLST-11-OIF007 587 610 R | 1392 |
| 1156 | AB_MLST-11-OIF007 547 571 F | 250 | AB_MLST-11-OIF007 656 686 R | 902 |
| 1157 | AB_MLST-11-OIF007 601 627 F | 256 | AB_MLST-11-OIF007 710 736 R | 881 |
| 1158 | AB_MLST-11-OIF007 1202 1225 F | 384 | AB_MLST-11-OIF007 1266 1296 R | 878 |
| 1159 | AB_MLST-11-OIF007 1202 1225 F | 384 | AB_MLST-11-OIF007 1299 1316 R | 1199 |
| 1160 | AB_MLST-11-OIF007 1234 1264 F | 694 | AB_MLST-11-OIF007 1335 1362 R | 1215 |
| 1161 | AB MLST-11-OIF007 1327 1356 F | 225 | AB MLST-11-OIF007 1422 1448 R | 1212 |
| 1162 | AB MLST-11-OIF007 1345 1369 F | 383 | AB MLST-11-OIF007 1470 1494 R | 1083 |
| 1163 | AB MLST-11-OIF007 1351 1375 F | 662 | AB MLST-11-OIF007 1470 1494 R | 1083 |
| 1164 | AB MLST-11-OIF007 1387 1412 F | 422 | AB MLST-11-OIF007 1470 1494 R | 1083 |
| 1165 | AB MLST-11-OIF007 1542 1569 F | 194 | AB MLST-11-OIF007 1656 1680 R | 1173 |
| 1166 | AB MLST-11-OIF007 1566 1593 F | 684 | AB MLST-11-OIF007 1656 1680 R | 1173 |
| 1167 | AB MLST-11-OIF007 1611 1638 F | 375 | AB MLST-11-OIF007 1731 1757 R | 890 |
| 1168 | AB MLST-11-OIF007 1726 1752 F | 182 | AB MLST-11-OIF007 1790 1821 R | 1195 |
| 1169 | AB MLST-11-OIF007 1792 1826 F | 656 | AB MLST-11-OIF007 1876 1909 R | 1151 |
| 1170 | AB MLST-11-OIF007 1792 1826 F | 656 | AB MLST-11-OIF007 1895_1927_R | 1224 |
| 1171 | AB MLST-11-OIF007 1970 2002 F | 618 | AB MLST-11-OIF007 2097 2118 R | 1157 |
| 2846 | PARC X95819 33 58 F | 302 | PARC X95819 121 153 R | 852 |
| 2847 | PARC X95819 33 58 F | 199 | PARC X95819 157 178 R | 889 |
| 2848 | PARC X95819 33 58 F | 596 | PARC X95819 97 128 R | 1169 |
| 2852 | GYRA AY642140 -1 24 F | 150 | GYRA AY642140 71 100 R | 1242 |
| 2853 | GYRA AY642140 26 54 F | 166 | GYRA AY642140 121 146 R | 1069 |
| 2854 | GYRA AY642140 26 54 F | 166 | GYRA AY642140 58 89 R | 1168 |
| 2922 | AB MLST-11-OIF007 991 1018 F | 583 | AB_MLST-11-OIF007 1110 1137 R | 923 |
| 2972 | AB MLST-11-OIF007 1007 1034 F | 592 | AB MLST-11-OIF007 1126 1153 R | 924 |

**Table 14B: Triangulation Genotyping Analysis Primer Pairs for Identification of Sub-species characteristics (Strain Type) of Members of the Bacterial Genus Acinetobacter**

| **Primer Pair No.** | **Forward Primer (SEQ ID NO:)** | **SEQUENCE** | **Reverse Primer (SEQ ID NO:)** | **SEQUENCE** |
|---|---|---|---|---|
| 1151 | 454 | TGAGATTGCTGAACATTTAATGCTGATTGA | 1418 | TTGTACATTTGAAACAATATGCATGACATGTGAAT |
| 1152 | 243 | TATTGTTTCAAATGTACAAGGTGAAGTGCG | 969 | TCACAGGTTCTACTTCATCAATAATTTCCATTGC |
| 1153 | 541 | TGGAACGTTATCAGGTGCCCCAAAAATTCG | 1400 | TTGCAATCGACATTCCATTTCACCATGCC |
| 1154 | 436 | TGAAGTGCGTGATGATATCGATGCACTTGATGTA | 1036 | TCCGCCAAAAACTCCCCTTTTCACAGG |
| 1155 | 378 | TCGGTTTAGTAAAAGAACGTATTGCTCAACC | 1392 | TTCTGCTTGAGGAATAGTGCGTGG |
| 1156 | 250 | TCAACCTGACTGCGTGAATGGTTGT | 902 | TACGTTCTACGATTTCTTCATCAGGTACATC |
| 1157 | 256 | TCAAGCAGAAGCTTTGGAAGAAGAAGG | 881 | TACAACGTGATAAACACGACCAGAAGC |
| 1158 | 384 | TCGTGCCCGCAATTTGCATAAAGC | 878 | TAATGCCGGGTAGTGCAATCCATTCTTCTAG |
| 1159 | 384 | TCGTGCCCGCAATTTGCATAAAGC | 1199 | TGCACCTGCGGTCGAGCG |
| 1160 | 694 | TTGTAGCACAGCAAGGCAAATTTCCTGAAAC | 1215 | TGCCATCCATAATCACGCCATACTGACG |
| 1161 | 225 | TAGGTTTACGTCAGTATGGCGTGATTATGG | 1212 | TGCCAGTTTCCACATTTCACGTTCGTG |
| 1162 | 383 | TCGTGATTATGGATGGCAACGTGAA | 1083 | TCGCTTGAGTGTAGTCATGATTGCG |
| 1163 | 662 | TTATGGATGGCAACGTGAAACGCGT | 1083 | TCGCTTGAGTGTAGTCATGATTGCG |
| 1164 | 422 | TCTTTGCCATTGAAGATGACTTAAGC | 1083 | TCGCTTGAGTGTAGTCATGATTGCG |
| 1165 | 194 | TACTAGCGGTAAGCTTAAACAAGATTGC | 1173 | TGAGTCGGGTTCACTTTACCTGGCA |
| 1166 | 684 | TTGCCAATGATATTCGTTGGTTAGCAAG | 1173 | TGAGTCGGGTTCACTTTACCTGGCA |
| 1167 | 375 | TCGGCGAAATCCGTATTCCTGAAAATGA | 890 | TACCGGAAGCACCAGCGACATTAATAG |
| 1168 | 182 | TACCACTATTAATGTCGCTGGTGCTTC | 1195 | TGCAACTGAATAGATTGCAGTAAGTTATAAGC |
| 1169 | 656 | TTATAACTTACTGCAATCTATTCAGTTGCTTGGTG | 1151 | TGAATTATGCAAGAAGTGATCAATTTTCTCACGA |
| 1170 | 656 | TTATAACTTACTGCAATCTATTCAGTTGCTTGGTG | 1224 | TGCCGTAACTAAGAGAATTATGCAAGAA |
| 1171 | 618 | TGGTTATGTACCAAATACTTTGTCTGAAGATGG | 1157 | TGACGGCATCGATACCACCGTC |
| 2846 | 302 | TCCAAAAAAATCAGCGCGTACAGTGG | 852 | TAAAGGATAGCGGTAACTAAATGGCTGAGCCAT |
| 2847 | 199 | TACTTGGTAAATACCACCCACATGGTGA | 889 | TACCCCAGTTCCCCTGACCTTC |
| 2848 | 596 | TGGTAAATACCACCCACATGGTGAC | 1169 | TGAGCCATGAGTACCATGGCTTCATAACATGC |
| 2852 | 150 | TAAATCTCCCCGTGTCGTTGGTGAC | 1242 | TGCTAAAGTCTTGAGCCATACGAACAATGG |
| 2853 | 166 | TAATCGGTAAATATCACCCGCATGGTGAC | 1069 | TCGATCGAACCGAAGTTACCCTGACC |
| 2854 | 166 | TAATCGGTAAATATCACCCGCATGGTGAC | 1168 | TGAGCCATACGAACAATGGTTTCATAAACAGC |
| 2922 | 583 | TGGGCGATGCTGCGAAATGGTTAAAAGA | 923 | TAGTATCACCACGTACACCCGGATCAGT |
| 2972 | 592 | TGGGIGATGCTGCIAAATGGTTAAAAGA | 924 | TAGTATCACCACGTACICCIGGATCAGT |

Analysis of bioagent identifying amplicons obtained using the primers of Table 14B for over 200 samples from Operation Iraqi Freedom resulted in the identification of 50 distinct strain type clusters. The largest cluster, designated strain type 11 (ST11) includes 42 sample isolates, all of which were obtained from US service personnel and Iraqi civilians treated at the 28^{th} Combat Support Hospital in Baghdad. Several of these individuals were also treated on the hospital ship USNS Comfort. These observations are indicative of significant epidemiological correlation/linkage.

All of the sample isolates were tested against a broad panel of antibiotics to characterize their antibiotic resistance profiles. As an example of a representative result from antibiotic susceptibility testing, ST11 was found to consist of four different clusters of isolates, each with a varying degree of sensitivity/resistance to the various antibiotics tested which included penicillins, extended spectrum penicillins, cephalosporins, carbepenem, protein synthesis inhibitors, nucleic acid synthesis inhibitors, anti-metabolites, and anti-cell membrane antibiotics. Thus, the genotyping power of bacterial bioagent identifying amplicons, particularly drill-down bacterial bioagent identifying amplicons, has the potential to increase the understanding of the transmission of infections in combat casualties, to identify the source of infection in the environment, to track hospital transmission of nosocomial infections, and to rapidly characterize drug-resistance profiles which enable development of effective infection control measures on a time-scale previously not achievable.

### Example 13: Triangulation Genotyping Analysis and Codon Analysis of Acinetobacter baumannii Samples from Two Health Care Facilities

In this investigation, 88 clinical samples were obtained from Walter Reed Hospital and 95 clinical samples were obtained from Northwestern Medical Center. All samples from both healthcare facilities were suspected of containing sub-types of *Acinetobacter baumannii*, at least some of which were expected to be resistant to quinolone drugs. Each of the 183 samples was analyzed by the method of the present invention. DNA was extracted from each of the samples and amplified with eight triangulation genotyping analysis primer pairs represented by primer pair numbers: 1151, 1156, 1158, 1160, 1165, 1167, 1170, and 1171. The DNA was also amplified with speciating primer pair number 2922 and codon analysis primer pair numbers 2846-2848 which interrogate a codon present in the parC gene, and primer pair numbers 2852-2854 which bracket a codon present in the gyrA gene. The parC and gyrA codon mutations are both responsible for causing drug resistance in *Acinetobacter baumannii.* During evolution of drug resistant strains, the gyrA mutation usually occurs before the parC mutation. Amplification products were measured by ESI-TOF mass spectrometry as indicated in Example 4. The base compositions of the amplification products were calculated from the average molecular masses of the amplification products and are shown in Tables 15-18. The entries in each of the tables are grouped according to strain type number, which is an arbitrary number assigned to *Acinetobacter baumannii* strains in the order of observance beginning from the triangulation genotyping analysis OIF genotyping study described in Example 12. For example, strain type 11 which appears in samples from the Walter Reed Hospital is the same strain as the strain type 11 mentioned in Example 12. Ibis# refers to the order in which each sample was analyzed. Isolate refers to the original sample isolate numbering system used at the location from which the samples were obtained (either Walter Reed Hospital or Northwestern Medical Center). ST = strain type. ND = not detected. Base compositions highlighted with bold type indicate that the base composition is a unique base composition for the amplification product obtained with the pair of primers indicated.

**Table 15A: Base Compositions of Amplification Products of 88 A. baumannii Samples Obtained from Walter Reed Hospital and Amplified with Codon Analysis Primer Pairs Targeting the gyrA Gene**

| **Species** | **Ibis#** | **Isolate** | **ST** | **PP No: 2852 gyrA** | **PP No: 2853 gyrA** | **PP No: 2854 gyrA** |
|---|---|---|---|---|---|---|
| *A. baumannii* | 20 | 1082 | 1 | A25G23C22T31 | A29G28C22T42 | A17G13C14T20 |
| *A. baumannii* | 13 | 854 | 10 | A25G23C21T32 | A29G28C21T43 | A17G13C13T21 |
| *A. baumannii* | 22 | 1162 | 10 | A25G23C21T32 | A29G28C21T43 | A17G13C13T21 |
| *A. baumannii* | 27 | 1230 | 10 | A25G23C21T32 | A29G28C21T43 | A17G13C13T21 |
| *A. baumannii* | 31 | 1367 | 10 | A25G23C21T32 | A29G28C21T43 | A17G13C13T21 |
| *A. baumannii* | 37 | 1459 | 10 | A25G23C21T32 | A29G28C21T43 | A17G13C13T21 |
| *A. baumannii* | 55 | 1700 | 10 | A25G23C21T32 | A29G28C21T43 | A17G13C13T21 |
| *A. baumannii* | 64 | 1777 | 10 | A25G23C21T32 | A29G28C21T43 | A17G13C13T21 |
| *A. baumannii* | 73 | 1861 | 10 | A25G23C21T32 | A29G28C21T43 | A17G13C13T21 |
| *A. baumanni* | 74 | 1877 | 10 | ND | A29G28C21T43 | A17G13C13T21 |
| *A. baumannii* | 86 | 1972 | 10 | A25G23C21T32 | A29G28C21T43 | A17G13C13T21 |
| *A. baumannii* | 3 | 684 | 11 | A25G23C21T32 | A29G28C21T43 | A17G13C13T21 |
| *A. baumannii* | 6 | 720 | 11 | A25G23C21T32 | A29G28C21T43 | A17G13C13T21 |
| *A. baumannii* | 7 | 726 | 11 | A25G23C21T32 | A29G28C21T43 | A17G13C13T21 |
| *A. baumannii* | 19 | 1079 | 11 | A25G23C21T32 | A29G28C21T43 | A17G13C13T21 |
| *A. baumannii* | 21 | 1123 | 11 | A25G23C21T32 | A29G28C21T43 | A17G13C13T21 |
| *A. baumannii* | 23 | 1188 | 11 | A25G23C21T32 | A29G28C21T43 | A17G13C13T21 |
| *A. baumannii* | 33 | 1417 | 11 | A25G23C21T32 | A29G28C21T43 | A17G13C13T21 |
| *A. baumannii* | 34 | 1431 | 11 | A25G23C21T32 | A29G28C21T43 | A17G13C13T21 |
| *A. baumannii* | 38 | 1496 | 11 | A25G23C21T32 | A29G28C21T43 | A17G13C13T21 |
| *A. baumannii* | 40 | 1523 | 11 | A25G23C21T32 | A29G28C21T43 | A17G13C13T21 |
| *A. baumannii* | 42 | 1640 | 11 | A25G23C21T32 | A29G28C21T43 | A17G13C13T21 |
| *A. baumannii* | 50 | 1666 | 11 | A25G23C21T32 | A29G28C21T43 | A17G13C13T21 |
| *A. baumannii* | 51 | 1668 | 11 | A25G23C21T32 | A29G28C21T43 | A17G13C13T21 |
| *A. baumannii* | 52 | 1695 | 11 | A25G23C21T32 | A29G28C21T43 | A17G13C13T21 |
| *A. baumannii* | 65 | 1781 | 11 | A25G23C21T32 | A29G28C21T43 | A17G13C13T21 |
| *A. baumannii* | 44 | 1649 | 12 | A25G23C21T32 | A29G28C21T43 | A17G13C13T21 |
| *A. baumannii* | 49A | 1658.1 | 12 | A25G23C22T31 | A29G28C21T43 | A17G13C13T21 |
| *A. baumannii* | 49B | 1658.2 | 12 | A25G23C21T32 | A29G28C21T43 | A17G13C13T21 |
| *A. baumannii* | 56 | 1707 | 12 | A25G23C21T32 | A29G28C21T43 | A17G13C13T21 |
| *A. baumannii* | 80 | 1893 | 12 | A25G23C21T32 | A29G28C21T43 | A17G13C13T21 |
| *A. baumannii* | 5 | 693 | 14 | A25G23C21T32 | A29G28C21T43 | A17G13C13T21 |
| *A. baumannii* | 8 | 749 | 14 | A25G23C21T32 | A29G28C21T43 | A17G13C13T21 |
| *A. baumannii* | 10 | 839 | 14 | A25G23C21T32 | A29G28C21T43 | A17G13C13T21 |
| *A. baumannii* | 14 | 865 | 14 | A25G23C21T32 | A29G28C21T43 | A17G13C13T21 |
| *A. baumannii* | 16 | 888 | 14 | A25G23C21T32 | A29G28C21T43 | A17G13C13T21 |
| *A. baumannii* | 29 | 1326 | 14 | A25G23C21T32 | A29G28C21T43 | A17G13C13T21 |
| *A. baumannii* | 35 | 1440 | 14 | A25G23C21T32 | A29G28C21T43 | A17G13C13T21 |
| *A. baumannii* | 41 | 1524 | 14 | A25G23C21T32 | A29G28C21T43 | A17G13C13T21 |
| *A. baumannii* | 46 | 1652 | 14 | A25G23C21T32 | A29G28C21T43 | A17G13C13T21 |
| *A. baumannii* | 47 | 1653 | 14 | A25G23C21T32 | A29G28C21T43 | A17G13C13T21 |
| *A. baumannii* | 48 | 1657 | 14 | A25G23C21T32 | A29G28C21T43 | A17G13C13T21 |
| *A. baumannii* | 57 | 1709 | 14 | A25G23C21T32 | A29G28C21T43 | A17G13C13T21 |
| *A. baumannii* | 61 | 1727 | 14 | A25G23C21T32 | A29G28C21T43 | A17G13C13T21 |
| *A. baumannii* | 63 | 1762 | 14 | A25G23C21T32 | A29G28C21T43 | A17G13C13T21 |
| *A. baumannii* | 67 | 1806 | 14 | A25G23C21T32 | A29G28C21T43 | A17G13C13T21 |
| *A. baumannii* | 75 | 1881 | 14 | A25G23C21T32 | A29G28C21T43 | A17G13C13T21 |
| *A. baumannii* | 77 | 1886 | 14 | A25G23C21T32 | A29G28C21T43 | A17G13C13T21 |
| *A. baumannii* | 1 | 649 | 46 | A25G23C21T32 | A29G28C21T43 | A17G13C13T21 |
| *A. baumannii* | 2 | 653 | 46 | A25G23C21T32 | A29G28C21T43 | A17G13C13T21 |
| *A. baumannii* | 39 | 1497 | 16 | A25G23C21T32 | A29G28C21T43 | A17G13C13T21 |
| *A. baumannii* | 24 | 1198 | 15 | A25G23C21T32 | A29G28C21T43 | A17G13C13T21 |
| *A. baumannii* | 28 | 1243 | 15 | A25G23C21T32 | A29G28C21T43 | A17G13C13T21 |
| *A. baumannii* | 43 | 1648 | 15 | A25G23C21T32 | A29G28C21T43 | A17G13C13T21 |
| *A. baumannii* | 62 | 1746 | 15 | A25G23C21T32 | A29G28C21T43 | A17G13C13T21 |
| *A. baumannii* | 4 | 689 | 15 | A25G23C21T32 | A29G28C21T43 | A17G13C13T21 |
| *A. baumannii* | 68 | 1822 | 3 | A25G23C21T32 | A29G28C21T43 | A17G13C13T21 |
| *A. baumannii* | 69 | 1823A | 3 | A25G23C21T32 | A29G28C21T43 | A17G13C13T21 |
| *A. baumannii* | 70 | 1823B | 3 | A25G23C21T32 | A29G28C21T43 | A17G13C13T21 |
| *A. baumannii* | 71 | 1826 | 3 | A25G23C21T32 | A29G28C21T43 | A17G13C13T21 |
| *A. baumannii* | 72 | 1860 | 3 | A25G23C21T32 | A29G28C21T43 | A17G13C13T21 |
| *A. baumannii* | 81 | 1924 | 3 | A25G23C21T32 | A29G28C21T43 | A17G13C13T21 |
| *A. baumannii* | 82 | 1929 | 3 | A25G23C21T32 | A29G28C21T43 | A17G13C13T21 |
| *A. baumannii* | 85 | 1966 | 3 | A25G23C21T32 | A29G28C21T43 | A17G13C13T21 |
| *A. baumaninii* | 11 | 841 | 3 | A25G23C22T31 | A29G28C22T42 | A17G13C14T20 |
| *A. baumannii* | 32 | 1415 | 24 | A25G23C21T32 | A29G28C21T43 | A17G13C13T21 |
| *A. baumannii* | 45 | 1651 | 24 | A25G23C21T32 | A29G28C21T43 | A17G13C13T21 |
| *A. baumannii* | 54 | 1697 | 24 | A25G23C21T32 | A29G28C21T43 | A17G13C13T21 |
| *A. baumannii* | 58 | 1712 | 24 | A25G23C21T32 | A29G28C21T43 | A17G13C13T21 |
| *A. baumannii* | 60 | 1725 | 24 | A25G23C21T32 | A29G28C21T43 | A17G13C13T21 |
| *A. baumannii* | 66 | 1802 | 24 | A25G23C21T32 | A29G28C21T43 | A17G13C13T21 |
| *A. baumannii* | 76 | 1883 | 24 | A25G23C21T32 | A29G28C21T43 | A17G13C13T21 |
| *A. baumannii* | 78 | 1891 | 24 | A25G23C21T32 | A29G28C21T43 | A17G13C13T21 |
| *A. baumannii* | 79 | 1892 | 24 | A25G23C21T32 | A29G28C21T43 | A17G13C13T21 |
| *A. baumannii* | 83 | 1947 | 24 | A25G23C21T32 | A29G28C21T43 | A17G13C13T21 |
| *A. baumannii* | 84 | 1964 | 24 | A25G23C21T32 | A29G28C21T43 | A17G13C13T21 |
| *A. baumannii* | 53 | 1696 | 24 | A25G23C22T31 | A29G28C22T42 | A17G13C14T20 |
| *A. baumannii* | 36 | 1458 | 49 | A25G23C21T32 | A29G28C21T43 | A17G13C13T21 |
| *A. baumannii* | 59 | 1716 | 9 | A25G23C22T31 | A29G28C22T42 | A17G13C14T20 |
| *A. baumannii* | 9 | 805 | 30 | A25G23C22T31 | A29G28C22T42 | A17G13C14T20 |
| *A. baumannii* | 18 | 967 | 39 | A25G23C22T31 | A29G28C22T42 | A17G13C14T20 |
| *A. baumannii* | 30 | 1322 | 48 | A25G23C22T31 | A29G28C22T42 | A17G13C14T20 |
| *A. baumannii* | 26 | 1218 | 50 | A25G23C22T31 | A29G28C22T42 | A17G13C14T20 |
| *A*. *sp*. 13TU | 15 | 875 | A1 | A25G23C22T31 | A29G28C22T42 | A17G13C14T20 |
| *A*. *sp*. 13TU | 17 | 895 | A1 | A25G23C22T31 | A29G28C22T42 | A17G13C14T20 |
| *A*. *sp*. 3 | 12 | 853 | B7 | A25G22C22T32 | A30G29C22T40 | A17G13C14T20 |
| *A. johnsonii* | 25 | 1202 | NEW1 | A25G22C22T32 | A30G29C22T40 | A17G13C14T20 |
| *A*. *sp*. 2082 | 87 | 2082 | NEW2 | A25G22C22T32 | **A31G28C22T40** | A17G13C14T20 |

**Table 15B: Base Compositions Determined from A. baumannii DNA Samples Obtained from Walter Reed Hospital and Amplified with Codon Analysis Primer Pairs Targeting the parC Gene**

| **Species** | **Ibis#** | **Isolate** | **ST** | **PP No: 2846 parC** | **PP No: 2847 parC** | **PP No: 2848 parC** |
|---|---|---|---|---|---|---|
| *A. baumannii* | 20 | 1082 | 1 | A33G26C29T33 | A29G28C26T31 | A16G14C15T15 |
| *A. baumannii* | 13 | 854 | 10 | A33G26C28T34 | A29G28C25T32 | A16G14C14T16 |
| *A. baumannii* | 22 | 1162 | 10 | A33G26C28T34 | A29G28C25T32 | A16G14C14T16 |
| *A. baumannii* | 27 | 1230 | 10 | A33G26C28T34 | A29G28C25T32 | A16G14C14T16 |
| *A. baumannii* | 31 | 1367 | 10 | A33G26C28T34 | A29G28C25T32 | A16G14C14T16 |
| *A. baumannii* | 37 | 1459 | 10 | A33G26C28T34 | A29G28C25T32 | A16G14C14T16 |
| *A. baumannii* | 55 | 1700 | 10 | A33G26C28T34 | A29G28C25T32 | A16G14C14T16 |
| *A. baumannii* | 64 | 1777 | 10 | A33G26C28T34 | A29G28C25T32 | A16G14C14T16 |
| *A. baumannii* | 73 | 1861 | 10 | A33G26C28T34 | A29G28C25T32 | A16G14C14T16 |
| *A. baumannii* | 74 | 1877 | 10 | A33G26C28T34 | A29G28C25T32 | A16G14C14T16 |
| *A. baumannii* | 86 | 1972 | 10 | A33G26C28T34 | A29G28C25T32 | A16G14C14T16 |
| *A. baumannii* | 3 | 684 | 11 | A33G26C28T34 | A29G28C25T32 | A16G14C14T16 |
| *A. baumannii* | 6 | 720 | 11 | A33G26C28T34 | A29G28C25T32 | A16G14C14T16 |
| *A. baumannii* | 7 | 726 | 11 | A33G26C28T34 | A29G28C25T32 | A16G14C14T16 |
| *A. baumannii* | 19 | 1079 | 11 | A33G26C28T34 | A29G28C25T32 | A16G14C14T16 |
| *A. baumannii* | 21 | 1123 | 11 | A33G26C28T34 | A29G28C25T32 | A16G14C14T16 |
| *A. baumannii* | 23 | 1188 | 11 | A33G26C28T34 | A29G28C25T32 | A16G14C14T16 |
| *A. baumannii* | 33 | 1417 | 11 | A33G26C28T34 | A29G28C25T32 | A16G14C14T16 |
| *A. baumannii* | 34 | 1431 | 11 | A33G26C28T34 | A29G28C25T32 | A16G14C14T16 |
| *A. baumannii* | 38 | 1496 | 11 | A33G26C28T34 | A29G28C25T32 | A16G14C14T16 |
| *A. baumannii* | 40 | 1523 | 11 | A33G26C28T34 | A29G28C25T32 | A16G14C14T16 |
| *A. baumannii* | 42 | 1640 | 11 | A33G26C28T34 | A29G28C25T32 | A16G14C14T16 |
| *A. baumannii* | 50 | 1666 | 11 | A33G26C28T34 | A29G28C25T32 | A16G14C14T16 |
| *A. baumannii* | 51 | 1668 | 11 | A33G26C28T34 | A29G28C25T32 | A16G14C14T16 |
| *A. baumannii* | 52 | 1695 | 11 | A33G26C28T34 | A29G28C25T32 | A16G14C14T16 |
| *A. baumannii* | 65 | 1781 | 11 | A33G26C28T34 | A29G28C25T32 | A16G14C14T16 |
| *A. baumannii* | 44 | 1649 | 12 | A33G26C28T34 | A29G28C25T32 | A16G14C14T16 |
| *A. baumannii* | 49A | 1658.1 | 12 | A33G26C28T34 | A29G28C25T32 | A16G14C14T16 |
| *A. baumannii* | 49B | 1658.2 | 12 | A33G26C28T34 | A29G28C25T32 | A16G14C14T16 |
| *A. baumannii* | 56 | 1707 | 12 | A33G26C28T34 | A29G28C25T32 | A16G14C14T16 |
| *A. baumannii* | 80 | 1893 | 12 | A33G26C28T34 | A29G28C25T32 | A16G14C14T16 |
| *A. baumannii* | 5 | 693 | 14 | A33G26C28T34 | A29G28C25T32 | A16G14C14T16 |
| *A. baumannii* | 8 | 749 | 14 | A33G26C28T34 | A29G28C25T32 | A16G14C14T16 |
| *A. baumannii* | 10 | 839 | 14 | A33G26C28T34 | A29G28C25T32 | A16G14C14T16 |
| *A. baumannii* | 14 | 865 | 14 | A33G26C28T34 | A29G28C25T32 | A16G14C14T16 |
| *A. baumannii* | 16 | 888 | 14 | A33G26C28T34 | A29G28C25T32 | A16G14C14T16 |
| *A. baumannii* | 29 | 1326 | 14 | A33G26C28T34 | A29G28C25T32 | A16G14C14T16 |
| *A. baumannii* | 35 | 1440 | 14 | A33G26C28T34 | A29G28C25T32 | A16G14C14T16 |
| *A. baumannii* | 41 | 1524 | 14 | A33G26C28T34 | A29G28C25T32 | A16G14C14T16 |
| *A. baumannii* | 46 | 1652 | 14 | A33G26C28T34 | A29G28C25T32 | A16G14C14T16 |
| *A. baumannii* | 47 | 1653 | 14 | A33G26C28T34 | A29G28C25T32 | A16G14C14T16 |
| *A. baumannii* | 48 | 1657 | 14 | A33G26C28T34 | A29G28C25T32 | A16G14C14T16 |
| *A. baumannii* | 57 | 1709 | 14 | A33G26C28T34 | A29G28C25T32 | A16G14C14T16 |
| *A. baumannii* | 61 | 1727 | 14 | A33G26C28T34 | A29G28C25T32 | A16G14C14T16 |
| *A. baumannii* | 63 | 1762 | 14 | A33G26C28T34 | A29G28C25T32 | A16G14C14T16 |
| *A. baumannii* | 67 | 1806 | 14 | A33G26C28T34 | A29G28C25T32 | A16G14C14T16 |
| *A. baumannii* | 75 | 1881 | 14 | A33G26C28T34 | A29G28C25T32 | A16G14C14T16 |
| *A. baumannii* | 77 | 1886 | 14 | A33G26C28T34 | A29G28C25T32 | A16G14C14T16 |
| *A. baumannii* | 1 | 649 | 46 | A33G26C28T34 | A29G28C25T32 | A16G14C14T16 |
| *A. baumannii* | 2 | 653 | 46 | A33G26C28T34 | A29G28C25T32 | A16G14C14T16 |
| *A. baumannii* | 39 | 1497 | 16 | A33G26C28T34 | A29G28C25T32 | A16G14C14T16 |
| *A. baumannii* | 24 | 1198 | 15 | A33G26C28T34 | A29G29C23T33 | A16G14C14T16 |
| *A. baumannii* | 28 | 1243 | 15 | A33G26C28T34 | A29G29C23T33 | A16G14C14T16 |
| *A. baumannii* | 43 | 1648 | 15 | A33G26C28T34 | A29G29C23T33 | A16G14C14T16 |
| *A. baumannii* | 62 | 1746 | 15 | A33G26C28T34 | A29G29C23T33 | A16G14C14T16 |
| *A. baumannii* | 4 | 689 | 15 | A34G25C29T33 | A30G27C26T31 | A16G14C15T15 |
| *A. baumannii* | 68 | 1822 | 3 | A33G26C28T34 | A29G28C25T32 | A16G14C14T16 |
| *A. baumannii* | 69 | 1823A | 3 | A33G26C28T34 | A29G28C25T32 | A16G14C14T16 |
| *A. baumannii* | 70 | 1823B | 3 | A33G26C28T34 | A29G28C25T32 | A16G14C14T16 |
| *A. baumannii* | 71 | 1826 | 3 | A33G26C28T34 | A29G28C25T32 | A16G14C14T16 |
| *A. baumannii* | 72 | 1860 | 3 | A33G26C28T34 | A29G28C25T32 | A16G14C14T16 |
| *A. baumannii* | 81 | 1924 | 3 | A33G26C28T34 | A29G28C25T32 | A16G14C14T16 |
| *A. baumannii* | 82 | 1929 | 3 | A33G26C28T34 | A29G28C25T32 | A16G14C14T16 |
| *A. baumannii* | 85 | 1966 | 3 | A33G26C28T34 | A29G28C25T32 | A16G14C14T16 |
| *A. baumannii* | 11 | 841 | 3 | A33G26C29T33 | A29G28C26T31 | A16G14C15T15 |
| *A. baumannii* | 32 | 1415 | 24 | A33G26C29T33 | A29G28C26T31 | A16G14C15T15 |
| *A. baumannii* | 45 | 1651 | 24 | A33G26C29T33 | A29G28C26T31 | A16G14C15T15 |
| *A. baumannii* | 54 | 1697 | 24 | A33G26C29T33 | A29G28C26T31 | A16G14C15T15 |
| *A. baumannii* | 58 | 1712 | 24 | A33G26C29T33 | A29G28C26T31 | A16G14C15T15 |
| *A. baumannii* | 60 | 1725 | 24 | A33G26C29T33 | A29G28C26T31 | A16G14C15T15 |
| *A. baumannii* | 66 | 1802 | 24 | A33G26C29T33 | A29G28C26T31 | A16G14C15T15 |
| *A. baumannii* | 76 | 1883 | 24 | A33G26C29T33 | A29G28C26T31 | A16G14C15T15 |
| *A. baumannii* | 78 | 1891 | 24 | A34G25C29T33 | A30G27C26T31 | A16G14C15T15 |
| *A. baumannii* | 79 | 1892 | 24 | A33G26C29T33 | A29G28C26T31 | A16G14C15T15 |
| *A. baumannii* | 83 | 1947 | 24 | A34G25C29T33 | A30G27C26T31 | A16G14C15T15 |
| *A. baumannii* | 84 | 1964 | 24 | A33G26C29T33 | A29G28C26T31 | A16G14C15T15 |
| *A. baumannii* | 53 | 1696 | 24 | A33G26C29T33 | A29G28C26T31 | A16G14C15T15 |
| *A. baumannii* | 36 | 1458 | 49 | A34G26C29T32 | A30G28C24T32 | A16G14C15T15 |
| *A. baumannii* | 59 | 1716 | 9 | A33G26C29T33 | A29G28C26T31 | A16G14C15T15 |
| *A. baumannii* | 9 | 805 | 30 | A33G26C29T33 | A29G28C26T31 | A16G14C15T15 |
| *A. baumannii* | 18 | 967 | 39 | A33G26C29T33 | A29G28C26T31 | A16G14C15T15 |
| *A. baumannii* | 30 | 1322 | 48 | A33G26C29T33 | A29G28C26T31 | A16G14C15T15 |
| *A. baumannii* | 26 | 1218 | 50 | A33G26C29T33 | A29G28C26T31 | A16G14C15T15 |
| *A*. *sp*. 13TU | 15 | 875 | A1 | A32G26C28T35 | A28G28C24T34 | A16G14C15T15 |
| *A*. *sp*. 13TU | 17 | 895 | A1 | A32G26C28T35 | A28G28C24T34 | A16G14C15T15 |
| *A*. *sp*. 3 | 12 | 853 | B7 | A29G26C27T39 | A26G32C21T35 | A16G14C15T15 |
| *A. johnsonii* | 25 | 1202 | NEW1 | A32G28C26T35 | A29G29C22T34 | A16G14C15T15 |
| *A*. *sp*. 2082 | 87 | 2082 | NEW2 | **A33G27C26T35** | **A31G28C20T35** | A16G14C15T15 |

**Table 16A: Base Compositions Determined from A. baumannii DNA Samples Obtained from Northwestern Medical Center and Amplified with Codon Analysis Primer Pairs Targeting the gyrA Gene**

| **Species** | **Ibis#** | **Isolate** | **ST** | **PP No: 2852 gyrA** | **PP No: 2853 gyrA** | **PP No: 2854 gyrA** |
|---|---|---|---|---|---|---|
| *A. baumanni* | 54 | 536 | 3 | A25G23C21T32 | A29G28C21T43 | A17G13C13T21 |
| *A. baumanni* | 87 | 665 | 3 | A25G23C21T32 | A29G28C21T43 | A17G13C13T21 |
| *A. baumannii* | 8 | 80 | 10 | A25G23C21T32 | A29G28C21T43 | A17G13C13T21 |
| *A. baumannii* | 9 | 91 | 10 | A25G23C21T32 | A29G28C21T43 | A17G13C13T21 |
| *A. baumannii* | 10 | 92 | 10 | A25G23C21T32 | A29G28C21T43 | A17G13C13T21 |
| *A. baumannii* | 11 | 131 | 10 | A25G23C21T32 | A29G28C21T43 | A17G13C13T21 |
| *A. baumannii* | 12 | 137 | 10 | A25G23C21T32 | A29G28C21T43 | A17G13C13T21 |
| *A. baumannii* | 21 | 218 | 10 | A25G23C21T32 | A29G28C21T43 | A17G13C13T21. |
| *A. baumannii* | 26 | 242 | 10 | A25G23C21T32 | A29G28C21T43 | A17G13C13T21 |
| *A. baumannii* | 94 | 678 | 10 | A25G23C21T32 | A29G28C21T43 | A17G13C13T21 |
| *A. baumannii* | 1 | 9 | 10 | A25G23C21T32 | A29G28C21T43 | A17G13C13T21 |
| *A. baumannii* | 2 | 13 | 10 | A25G23C21T32 | A29G28C21T43 | A17G13C13T21 |
| *A. baumannii* | 3 | 19 | 10 | A25G23C21T32 | A29G28C21T43 | A17G13C13T21 |
| *A. baumannii* | 4 | 24 | 10 | A25G23C21T32 | A29G28C21T43 | A17G13C13T21 |
| *A. baumannii* | 5 | 36 | 10 | A25G23C21T32 | A29G28C21T43 | A17G13C13T21 |
| *A. baumannii* | 6 | 39 | 10 | A25G23C21T32 | A29G28C21T43 | A17G13C13T21 |
| *A. baumannii* | 13 | 139 | 10 | A25G23C21T32 | A29G28C21T43 | A17G13C13T21 |
| *A. baumannii* | 15 | 165 | 10 | A25G23C21T32 | A29G28C21T43 | A17G13C13T21 |
| *A. baumannii* | 16 | 170 | 10 | A25G23C21T32 | A29G28C21T43 | A17G13C13T21 |
| *A. baumannii* | 17 | 186 | 10 | A25G23C21T32 | A29G28C21T43 | A17G13C13T21 |
| *A. baumannii* | 20 | 202 | 10 | A25G23C21T32 | A29G28C21T43 | A17G13C13T21 |
| *A*. *baumannii* | 22 | 221 | 10 | A25G23C21T32 | A29G28C21T43 | A17G13C13T21 |
| *A. baumannii* | 24 | 234 | 10 | A25G23C21T32 | A29G28C21T43 | A17G13C13T21 |
| *A. baumannii* | 25 | 239 | 10 | A25G23C21T32 | A29G28C21T43 | A17G13C13T21 |
| *A. baumannii* | 33 | 370 | 10 | A25G23C21T32 | A29G28C21T43 | A17G13C13T21 |
| *A. baumannii* | 34 | 389 | 10 | A25G23C21T32 | A29G28C21T43 | A17G13C13T21 |
| *A. baumannii* | 19 | 201 | 14 | A25G23C21T32 | A29G28C21T43 | A17G13C13T21 |
| *A. baumannii* | 27 | 257 | 51 | A25G23C21T32 | A29G28C21T43 | A17G13C13T21 |
| *A. baumannii* | 29 | 301 | 51 | A25G23C21T32 | A29G28C21T43 | A17G13C13T21 |
| *A. baumannii* | 31 | 354 | 51 | A25G23C21T32 | A29G28C21T43 | A17G13C13T21 |
| *A. baumannii* | 36 | 422 | 51 | A25G23C21T32 | A29G28C21T43 | A17G13C13T21 |
| *A. baumannii* | 37 | 424 | 51 | A25G23C21T32 | A29G28C21T43 | A17G13C13T21 |
| *A. baumannii* | 38 | 434 | 51 | A25G23C21T32 | A29G28C21T43 | A17G13C13T21 |
| *A. baumannii* | 39 | 473 | 51 | A25G23C21T32 | A29G28C21T43 | A17G13C13T21 |
| *A. baumannii* | 40 | 482 | 51 | A25G23C21T32 | A29G28C21T43 | A17G13C13T21 |
| *A. baumannii* | 44 | 512 | 51 | A25G23C21T32 | A29G28C21T43 | A17G13C13T21 |
| *A. baumannii* | 45 | 516 | 51 | A25G23C21T32 | A29G28C21T43 | A17G13C13T21 |
| *A. baumannii* | 47 | 522 | 51 | A25G23C21T32 | A29G28C21T43 | A17G13C13T21 |
| *A. baumannii* | 48 | 526 | 51 | A25G23C21T32 | A29G28C21T43 | A17G13C13T21 |
| *A. baumannii* | 50 | 528 | 51 | A25G23C21T32 | A29G28C21T43 | A17G13C13T21 |
| *A. baumannii* | 52 | 531 | 51 | A25G23C21T32 | A29G28C21T43 | A17G13C13T21 |
| *A. baumannii* | 53 | 533 | 51 | A25G23C21T32 | A29G28C21T43 | A17G13C13T21 |
| *A. baumannii* | 56 | 542 | 51 | A25G23C21T32 | A29G28C21T43 | A17G13C13T21 |
| *A. baumannii* | 59 | 550 | 51 | A25G23C21T32 | A29G28C21T43 | A17G13C13T21 |
| *A. baumannii* | 62 | 556 | 51 | A25G23C21T32 | A29G28C21T43 | A17G13C13T21 |
| *A. baumannii* | 64 | 557 | 51 | A25G23C21T32 | A29G28C21T43 | A17G13C13T21 |
| *A. baumannii* | 70 | 588 | 51 | A25G23C21T32 | A29G28C21T43 | A17G13C13T21 |
| *A. baumannii* | 73 | 603 | 51 | A25G23C21T32 | A29G28C21T43 | A17G13C13T21 |
| *A. baumannii* | 74 | 605 | 51 | A25G23C21T32 | A29G28C21T43 | A17G13C13T21 |
| *A. baumannii* | 75 | 606 | 51 | A25G23C21T32 | A29G28C21T43 | A17G13C13T21 |
| *A. baumannii* | 77 | 611 | 51 | A25G23C21T32 | A29G28C21T43 | A17G13C13T21 |
| *A. baumannii* | 79 | 622 | 51 | A25G23C21T32 | A29G28C21T43 | A17G13C13T21 |
| *A. baumannii* | 83 | 643 | 51 | A25G23C21T32 | A29G28C21T43 | A17G13C13T21 |
| *A. baumannii* | 85 | 653 | 51 | A25G23C21T32 | A29G28C21T43 | A17G13C13T21 |
| *A. baumannii* | 89 | 669 | 51 | A25G23C21T32 | A29G28C21T43 | A17G13C13T21 |
| *A. baumannii* | 93 | 674 | 51 | A25G23C21T32 | A29G28C21T43 | A17G13C13T21 |
| *A. baumannii* | 23 | 228 | 51 | A25G23C21T32 | A29G28C21T43 | A17G13C13T21 |
| *A. baumannii* | 32 | 369 | 52 | A25G23C21T32 | A29G28C21T43 | A17G13C13T21 |
| *A. baumannii* | 35 | 393 | 52 | A25G23C21T32 | A29G28C21T43 | A17G13C13T21 |
| *A. baumannii* | 30 | 339 | 53 | A25G23C21T32 | A29G28C21T43 | A17G13C13T21 |
| *A. baumannii* | 41 | 485 | 53 | A25G23C21T32 | A29G28C21T43 | A17G13C13T21 |
| *A. baumannii* | 42 | 493 | 53 | A25G23C21T32 | A29G28C21T43 | A17G13C13T21 |
| *A. baumannii* | 43 | 502 | 53 | A25G23C21T32 | A29G28C21T43 | A17G13C13T21 |
| *A. baumannii* | 46 | 520 | 53 | A25G23C21T32 | A29G28C21T43 | A17G13C13T21 |
| *A. baumannii* | 49 | 527 | 53 | A25G23C21T32 | A29G28C21T43 | A17G13C13T21 |
| *A. baumannii* | 51 | 529 | 53 | A25G23C21T32 | A29G28C21T43 | A17G13C13T21 |
| *A. baumannii* | 65 | 562 | 53 | A25G23C21T32 | A29G28C21T43 | A17G13C13T21 |
| *A. baumannii* | 68 | 579 | 53 | A25G23C21T32 | A29G28C21T43 | A17G13C13T21 |
| *A. baumannii* | 57 | 546 | 54 | A25G23C21T32 | A29G28C21T43 | A17G13C13T21 |
| *A. baumannii* | 58 | 548 | 54 | A25G23C21T32 | A29G28C21T43 | A17G13C13T21 |
| *A. baumannii* | 60 | 552 | 54 | A25G23C21T32 | A29G28C21T43 | A17G13C13T21 |
| *A. baumannii* | 61 | 555 | 54 | A25G23C21T32 | A29G28C21T43 | A17G13C13T21 |
| *A. baumannii* | 63 | 557 | 54 | A25G23C21T32 | A29G28C21T43 | A17G13C13T21 |
| *A. baumannii* | 66 | 570 | 54 | A25G23C21T32 | A29G28C21T43 | A17G13C13T21 |
| *A. baumannii* | 67 | 578 | 54 | A25G23C21T32 | A29G28C21T43 | A17G13C13T21 |
| *A. baumannii* | 69 | 584 | 54 | A25G23C21T32 | A29G28C21T43 | A17G13C13T21 |
| *A. baumannii* | 71 | 593 | 54 | A25G23C21T32 | A29G28C21T43 | A17G13C13T21 |
| *A. baumanni* | 72 | 602 | 54 | A25G23C21T32 | A29G28C21T43 | A17G13C13T21 |
| *A. baumannii* | 76 | 609 | 54 | A25G23C21T32 | A29G28C21T43 | A17G13C13T21 |
| *A. baumannii* | 78 | 621 | 54 | A25G23C21T32 | A29G28C21T43 | A17G13C13T21 |
| *A. baumannii* | 80 | 625 | 54 | A25G23C21T32 | A29G28C21T43 | A17G13C13T21 |
| *A. baumannii* | 81 | 628 | 54 | A25G23C21T32 | A29G28C21T43 | A17G13C13T21 |
| *A. baumannii* | 82 | 632 | 54 | A25G23C21T32 | A29G28C21T43 | A17G13C13T21 |
| *A. baumanni* | 84 | 649 | 54 | A25G23C21T32 | A29G28C21T43 | A17G13C13T21 |
| *A. baumannii* | 86 | 655 | 54 | A25G23C21T32 | A29G28C21T43 | A17G13C13T21 |
| *A. baumannii* | 88 | 668 | 54 | A25G23C21T32 | A29G28C21T43 | A17G13C13T21 |
| *A. baumannii* | 90 | 671 | 54 | A25G23C21T32 | A29G28C21T43 | A17G13C13T21 |
| *A. baumannii* | 91 | 672 | 54 | A25G23C21T32 | A29G28C21T43 | A17G13C13T21 |
| *A. baumannii* | 92 | 673 | 54 | A25G23C21T32 | A29G28C21T43 | A17G13C13T21 |
| *A. baumannii* | 18 | 196 | 55 | A25G23C22T31 | A29G28C21T43 | A17G13C13T21 |
| *A. baumannii* | 55 | 537 | 27 | A25G23C21T32 | A29G28C21T43 | A17G13C13T21 |
| *A. baumannii* | 28 | 263 | 27 | A25G23C22T31 | A29G28C22T42 | A17G13C14T20 |
| *A*. *sp*. *3* | 14 | 164 | B7 | A25G22C22T32 | A30G29C22T40 | A17G13C14T20 |
| mixture | 7 | 71 | - | ND | ND | **A17G13C15T19** |

**Table 16B: Base Compositions Determined from A. baumannii DNA Samples Obtained from Northwestern Medical Center and Amplified with Codon Analysis Primer Pairs Targeting the parC Gene**

| **Species** | **Ibis#** | **Isolate** | **ST** | **PP No: 2846 parC** | **PP No: 2847 parC** | **PP No: 2848 parC** |
|---|---|---|---|---|---|---|
| *A. baumanii* | 54 | 536 | 3 | A33G26C28T34 | A29G28C25T32 | A16G14C14T16 |
| *A. baumannii* | 87 | 665 | 3 | A33G26C28T34 | A29G28C25T32 | A16G14C14T16 |
| *A. baumannii* | 8 | 80 | 10 | A33G26C28T34 | A29G28C25T32 | A16G14C14T16 |
| *A. baumannii* | 9 | 91 | 10 | A33G26C28T34 | A29G28C25T32 | A16G14C14T16 |
| *A. baumannii* | 10 | 92 | 10 | A33G26C28T34 | A29G28C25T32 | ND |
| *A. baumannii* | 11 | 131 | 10 | A33G26C28T34 | A29G28C25T32 | A16G14C14T16 |
| *A. baumannii* | 12 | 137 | 10 | A33G26C28T34 | A29G28C25T32 | A16G14C14T16 |
| *A. baumannii* | 21 | 218 | 10 | A33G26C28T34 | A29G28C25T32 | A16G14C14T16 |
| *A. baumannii* | 26 | 242 | 10 | A33G26C28T34 | A29G28C25T32 | A16G14C14T16 |
| *A. baumannii* | 94 | 678 | 10 | A33G26C28T34 | A29G28C25T32 | A16G14C14T16 |
| *A. baumannii* | 1 | 9 | 10 | A33G26C29T33 | A29G28C26T31 | A16G14C15T15 |
| *A. baumannii* | 2 | 13 | 10 | A33G26C29T33 | A29G28C26T31 | A16G14C15T15 |
| *A. baumannii* | 3 | 19 | 10 | A33G26C29T33 | A29G28C26T31 | A16G14C15T15 |
| *A. baumannii* | 4 | 24 | 10 | A33G26C29T33 | A29G28C26T31 | A16G14C15T15 |
| *A. baumannii* | 5 | 36 | 10 | A33G26C29T33 | A29G28C26T31 | A16G14C15T15 |
| *A. baumannii* | 6 | 39 | 10 | A33G26C29T33 | A29G28C26T31 | A16G14C15T15 |
| *A. baumannii* | 13 | 139 | 10 | A33G26C29T33 | A29G28C26T31 | A16G14C15T15 |
| *A. baumannii* | 15 | 165 | 10 | A33G26C29T33 | A29G28C26T31 | A16G14C15T15 |
| *A. baumannii* | 16 | 170 | 10 | A33G26C29T33 | A29G28C26T31 | A16G14C15T15 |
| *A. baumannii* | 17 | 186 | 10 | A33G26C29T33 | A29G28C26T31 | A16G14C15T15 |
| *A. baumannii* | 20 | 202 | 10 | A33G26C29T33 | A29G28C26T31 | A16G14C15T15 |
| *A. baumannii* | 22 | 221 | 10 | A33G26C29T33 | A29G28C26T31 | A16G14C15T15 |
| *A. baumannii* | 24 | 234 | 10 | A33G26C29T33 | A29G28C26T31 | A16G14C15T15 |
| *A. baumannii* | 25 | 239 | 10 | A33G26C29T33 | A29G28C26T31 | A16G14C15T15 |
| *A. baumannii* | 33 | 370 | 10 | A33G26C29T33 | A29G28C26T31 | A16G14C15T15 |
| *A. baumannii* | 34 | 389 | 10 | A33G26C29T33 | A29G28C26T31 | A16G14C15T15 |
| *A. baumannii* | 19 | 201 | 14 | A33G26C28T34 | A29G28C25T32 | A16G14C14T16 |
| *A. baumannii* | 27 | 257 | 51 | A33G26C28T34 | A29G28C25T32 | A16G14C14T16 |
| *A. baumannii* | 29 | 301 | 51 | A33G26C28T34 | A29G28C25T32 | A16G14C14T16 |
| *A. baumannii* | 31 | 354 | 51 | A33G26C28T34 | A29G28C25T32 | A16G14C14T16 |
| *A. baumannii* | 36 | 422 | 51 | A33G26C28T34 | A29G28C25T32 | A16G14C14T16 |
| *A. baumannii* | 37 | 424 | 51 | A33G26C28T34 | A29G28C25T32 | A16G14C14T16 |
| *A. baumannii* | 38 | 434 | 51 | A33G26C28T34 | A29G28C25T32 | A16G14C14T16 |
| *A. baumannii* | 39 | 473 | 51 | A33G26C28T34 | A29G28C25T32 | A16G14C14T16 |
| *A. baumannii* | 40 | 482 | 51 | A33G26C28T34 | A29G28C25T32 | A16G14C14T16 |
| *A. baumannii* | 44 | 512 | 51 | A33G26C28T34 | A29G28C25T32 | A16G14C14T16 |
| *A. baumannii* | 45 | 516 | 51 | A33G26C28T34 | A29G28C25T32 | A16G14C14T16 |
| *A. baumannii* | 47 | 522 | 51 | A33G26C28T34 | A29G28C25T32 | A16G14C14T16 |
| *A. baumannii* | 48 | 526 | 51 | A33G26C28T34 | A29G28C25T32 | A16G14C14T16 |
| *A. baumannii* | 50 | 528 | 51 | A33G26C28T34 | A29G28C25T32 | A16G14C14T16 |
| *A. baumannii* | 52 | 531 | 51 | A33G26C28T34 | A29G28C25T32 | A16G14C14T16 |
| *A. baumannii* | 53 | 533 | 51 | A33G26C28T34 | A29G28C25T32 | A16G14C14T16 |
| *A. baumannii* | 56 | 542 | 51 | A33G26C28T34 | A29G28C25T32 | A16G14C14T16 |
| *A. baumannii* | 59 | 550 | 51 | A33G26C28T34 | A29G28C25T32 | A16G14C14T16 |
| *A. baumannii* | 62 | 556 | 51 | A33G26C28T34 | A29G28C25T32 | A16G14C14T16 |
| *A. baumannii* | 64 | 557 | 51 | A33G26C28T34 | A29G28C25T32 | A16G14C14T16 |
| *A. baumannii* | 70 | 588 | 51 | A33G26C28T34 | A29G28C25T32 | A16G14C14T16 |
| *A. baumannii* | 73 | 603 | 51 | A33G26C28T34 | A29G28C25T32 | A16G14C14T16 |
| *A. baumannii* | 74 | 605 | 51 | A33G26C28T34 | A29G28C25T32 | A16G14C14T16 |
| *A. baumannii* | 75 | 606 | 51 | A33G26C28T34 | A29G28C25T32 | A16G14C14T16 |
| *A. baumannii* | 77 | 611 | 51 | A33G26C28T34 | A29G28C25T32 | A16G14C14T16 |
| *A. baumannii* | 79 | 622 | 51 | A33G26C28T34 | A29G28C25T32 | A16G14C14T16 |
| *A. baumannii* | 83 | 643 | 51 | A33G26C28T34 | A29G28C25T32 | A16G14C14T16 |
| *A. baumannii* | 85 | 653 | 51 | A33G26C28T34 | A29G28C25T32 | A16G14C14T16 |
| *A. baumannii* | 89 | 669 | 51 | A33G26C28T34 | A29G28C25T32 | A16G14C14T16 |
| *A. baumannii* | 93 | 674 | 51 | A33G26C28T34 | A29G28C25T32 | A16G14C14T16 |
| *A. baumannii* | 23 | 228 | 51 | A34G25C29T33 | A30G27C26T31 | A16G14C15T15 |
| *A. baumannii* | 32 | 369 | 52 | A34G25C28T34 | A30G27C25T32 | A16G14C14T16 |
| *A. baumannii* | 35 | 393 | 52 | A34G25C28T34 | A30G27C25T32 | A16G14C14T16 |
| *A. baumannii* | 30 | 339 | 53 | A34G25C29T33 | A30G27C26T31 | A16G14C15T15 |
| *A. baumannii* | 41 | 485 | 53 | A34G25C29T33 | A30G27C26T31 | A16G14C15T15 |
| *A. baumannii* | 42 | 493 | 53 | A34G25C29T33 | A30G27C26T31 | A16G14C15T15 |
| *A. baumannii* | 43 | 502 | 53 | A34G25C29T33 | A30G27C26T31 | A16G14C15T15 |
| *A. baumannii* | 46 | 520 | 53 | A34G25C29T33 | A30G27C26T31 | A16G14C15T15 |
| *A. baumannii* | 49 | 527 | 53 | A34G25C29T33 | A30G27C26T31 | A16G14C15T15 |
| *A. baumannii* | 51 | 529 | 53 | A34G25C29T33 | A30G27C26T31 | A16G14C15T15 |
| *A. baumannii* | 65 | 562 | 53 | A34G25C29T33 | A30G27C26T31 | A16G14C15T15 |
| *A. baumannii* | 68 | 579 | 53 | A34G25C29T33 | A30G27C26T31 | A16G14C15T15 |
| *A. baumannii* | 57 | 546 | 54 | A33G26C28T34 | A29G28C25T32 | A16G14C14T16 |
| *A. baumannii* | 58 | 548 | 54 | A33G26C28T34 | A29G28C25T32 | A16G14C14T16 |
| *A. baumannii* | 60 | 552 | 54 | A33G26C28T34 | A29G28C25T32 | A16G14C14T16 |
| *A. baumannii* | 61 | 555 | 54 | A33G26C28T34 | A29G28C25T32 | A16G14C14T16 |
| *A. baumannii* | 63 | 557 | 54 | A33G26C28T34 | A29G28C25T32 | A16G14C14T16 |
| *A. baumannii* | 66 | 570 | 54 | A33G26C28T34 | A29G28C25T32 | A16G14C14T16 |
| *A. baumannii* | 67 | 578 | 54 | A33G26C28T34 | A29G28C25T32 | A16G14C14T16 |
| *A. baumannii* | 69 | 584 | 54 | A33G26C28T34 | A29G28C25T32 | A16G14C14T16 |
| *A. baumannii* | 71 | 593 | 54 | A33G26C28T34 | A29G28C25T32 | A16G14C14T16 |
| *A. baumannii* | 72 | 602 | 54 | A33G26C28T34 | A29G28C25T32 | A16G14C14T16 |
| *A. baumannii* | 76 | 609 | 54 | A33G26C28T34 | A29G28C25T32 | A16G14C14T16 |
| *A. baumannii* | 78 | 621 | 54 | A33G26C28T34 | A29G28C25T32 | A16G14C14T16 |
| *A. baumannii* | 80 | 625 | 54 | A33G26C28T34 | A29G28C25T32 | A16G14C14T16 |
| *A. baumannii* | 81 | 628 | 54 | A33G26C28T34 | A29G28C25T32 | A16G14C14T16 |
| *A. baumannii* | 82 | 632 | 54 | A33G26C28T34 | A29G28C25T32 | A16G14C14T16 |
| *A. baumannii* | 84 | 649 | 54 | A33G26C28T34 | A29G28C25T32 | A16G14C14T16 |
| *A. baumannii* | 86 | 655 | 54 | A33G26C28T34 | A29G28C25T32 | A16G14C14T16 |
| *A. baumannii* | 88 | 668 | 54 | A33G26C28T34 | A29G28C25T32 | A16G14C14T16 |
| *A. baumannii* | 90 | 671 | 54 | A33G26C28T34 | A29G28C25T32 | A16G14C14T16 |
| *A. baumannii* | 91 | 672 | 54 | A33G26C28T34 | A29G28C25T32 | A16G14C14T16 |
| *A. baumannii* | 92 | 673 | 544 | A33G26C28T34 | A29G28C25T32 | A16G14C14T16 |
| *A. baumannii* | 18 | 196 | 55 | A33G27C28T33 | A29G28C25T31 | A15G14C15T16 |
| *A. baumannii* | 55 | 537 | 27 | A33G26C29T33 | A29G28C26T31 | A16G14C15T15 |
| *A. baumannii* | 28 | 263 | 27 | A33G26C29T33 | A29G28C26T31 | A16G14C15T15 |
| *A. sp. 3* | 14 | 164 | B7 | A35G25C29T32 | A30G28C17T39 | A16G14C15T15 |
| mixture | 7 | 71 | - | ND | ND | A17G14C15T14 |

**Table 17A: Base Compositions Determined from A. baumannii DNA Samples Obtained from Walter Reed Hospital and Amplified with Speciating Primer Pair No. 2922 and Triangulation Genotyping Analysis Primer Pair Nos.1151 and 1156**

| **Species** | **Ibis#** | **Isolate** | **ST** | **PP No: 2922 efp** | **PP No: 1151 trpE** | **PP No: 1156 Adk** |
|---|---|---|---|---|---|---|
| *A. baumannii* | 20 | 1082 | 1 | A45G34C25T43 | A44G35C21T42 | A44G32C26T38 |
| *A. baumannii* | 13 | 854 | 10 | A45G34C25T43 | A44G35C21T42 | A44G32C26T38 |
| *A. baumannii* | 22 | 1162 | 10 | A45G34C25T43 | A44G35C21T42 | A44G32C26T38 |
| *A. baumannii* | 27 | 1230 | 10 | A45G34C25T43 | A44G35C2lT42 | A44G32C26T38 |
| *A. baumannii* | 31 | 1367 | 10 | A45G34C25T43 | A44G35C21T42 | A44G32C26T38 |
| *A. baumannii* | 37 | 1459 | 10 | A45G34C25T43 | A44G35C21T42 | A44G32C26T38 |
| *A. baumannii* | 55 | 1700 | 10 | A45G34C25T43 | A44G35C21T42 | A44G32C26T38 |
| *A. baumannii* | 64 | 1777 | 10 | A45G34C25T43 | A44G35C21T42 | A44G32C26T38 |
| *A. baumannii* | 73 | 1861 | 10 | A45G34C25T43 | A44G35C21T42 | A44G32C26T38 |
| *A. baumannii* | 74 | 1877 | 10 | A45G34C25T43 | A44G35C21T42 | A44G32C26T38 |
| *A. baumannii* | 86 | 1972 | 10 | A45G34C25T43 | A44G35C21T42 | A44G32C26T38 |
| *A. baumannii* | 3 | 684 | 11 | A45G34C25T43 | A44G35C21T42 | A44G32C26T38 |
| *A. baumannii* | 6 | 720 | 11 | A45G34C25T43 | A44G35C21T42 | A44G32C26T38 |
| *A. baumannii* | 7 | 726 | 11 | A45G34C25T43 | A44G35C21T42 | A44G32C26T38 |
| *A. baumannii* | 19 | 1079 | 11 | A45G34C25T43 | A44G35C21T42 | A44G32C26T38 |
| *A. baumannii* | 21 | 1123 | 11 | A45G34C25T43 | A44G35C21T42 | A44G32C26T38 |
| *A. baumannii* | 23 | 1188 | 11 | A45G34C25T43 | A44G35C21T42 | A44G32C26T38 |
| *A. baumannii* | 33 | 1417 | 11 | A45G34C25T43 | A44G35C21T42 | A44G32C26T38 |
| *A. baumannii* | 34 | 1431 | 11 | A45G34C25T43 | A44G35C21T42 | A44G32C26T38 |
| *A. baumannii* | 38 | 1496 | 11 | A45G34C25T43 | A44G35C21T42 | A44G32C26T38 |
| *A. baumannii* | 40 | 1523 | 11 | A45G34C25T43 | A44G35C21T42 | A44G32C26T38 |
| *A. baumannii* | 42 | 1640 | 11 | A45G34C25T43 | A44G35C21T42 | A44G32C26T38 |
| *A. baumannii* | 50 | 1666 | 11 | A45G34C25T43 | A44G35C21T42 | A44G32C26T38 |
| *A. baumannii* | 51 | 1668 | 11 | A45G34C25T43 | A44G35C21T42 | A44G32C26T38 |
| *A. baumannii* | 52 | 1695 | 11 | A45G34C25T43 | A44G35C21T42 | A44G32C26T38 |
| *A. baumannii* | 65 | 1781 | 11 | A45G34C25T43 | A44G35C21T42 | A44G32C26T38 |
| *A. baumannii* | 44 | 1649 | 12 | A45G34C25T43 | A44G35C21T42 | A44G32C26T38 |
| *A. baumannii* | 49A | 1658.1 | 12 | A45G34C25T43 | A44G35C21T42 | A44G32C26T38 |
| *A. baumannii* | 49B | 1658.2 | 12 | A45G34C25T43 | A44G35C21T42 | A44G32C26T38 |
| *A. baumannii* | 56 | 1707 | 12 | A45G34C25T43 | A44G35C21T42 | A44G32C26T38 |
| *A. baumannii* | 80 | 1893 | 12 | A45G34C25T43 | A44G35C21T42 | A44G32C26T38 |
| *A. baumannii* | 5 | 693 | 14 | A44G35C25T43 | A44G35C22T41 | A44G32C27T37 |
| *A. baumannii* | 8 | 749 | 14 | A44G3SC25T43 | A44G35C22T41 | A44G32C27T37 |
| *A. baumannii* | 10 | 839 | 14 | A44G35C25T43 | A44G35C22T41 | A44G32C27T37 |
| *A. baumannii* | 14 | 865 | 14 | A44G35C25T43 | A44G35C22T41 | A44G32C27T37 |
| *A. baumannii* | 16 | 888 | 14 | A44G35C25T43 | A44G35C22T41 | A44G32C27T37 |
| *A. baumannii* | 29 | 1326 | 14 | A44G35C25T43 | A44G35C22T41 | A44G32C27T37 |
| *A. baumannii* | 35 | 1440 | 14 | A44G35C25T43 | ND | A44G32C27T37 |
| *A. baumannii* | 41 | 1524 | 14 | A44G35C25T43 | A44G35C22T41 | A44G32C27T37 |
| *A. baumannii* | 46 | 1652 | 14 | A44G35C25T43 | A44G35C22T41 | A44G32C27T37 |
| *A. baumannii* | 47 | 1653 | 14 | A44G35C25T43 | A44G35C22T41 | A44G32C27T37 |
| *A. baumannii* | 48 | 1657 | 14 | A44G35C25T43 | A44G35C22T41 | A44G32C27T37 |
| *A. baumannii* | 57 | 1709 | 14 | A44G35C25T43 | A44G35C22T41 | A44G32C27T37 |
| *A. baumannii* | 61 | 1727 | 14 | A44G35C25T43 | A44G35C22T41 | A44G32C27T37 |
| *A. baumannii* | 63 | 1762 | 14 | A44G35C25T43 | A44G35C22T41 | A44G32C27T37 |
| *A. baumannii* | 67 | 1806 | 14 | A44G35C25T43 | A44G35C22T41 | A44G32C27T37 |
| *A. baumannii* | 75 | 1881 | 14 | A44G35C25T43 | A44G35C22T41 | A44G32C27T37 |
| *A. baumannii* | 77 | 1886 | 14 | A44G35C25T43 | A44G35C22T41 | A44G32C27T37 |
| *A. baumannii* | 1 | 649 | 46 | A44G35C25T43 | A44G35C22T41 | A44G32C26T38' |
| *A. baumannii* | 2 | 653 | 46 | A44G35C25T43 | A44G35C22T41 | A44G32C26T38 |
| *A. baumannii* | 39 | 1497 | 16 | A44G35C25T43 | A44G35C22T41 | A44G32C27T37 |
| *A. baumannii* | 24 | 1198 | 15 | A44G35C25T43 | A44G35C22T41 | A44G32C26T38 |
| *A. baumannii* | 28 | 1243 | 15 | A44G35C25T43 | A44G35C22T41 | A44G32C26T38 |
| *A. baumannii* | 43 | 1648 | 15 | A44G35C25T43 | A44G35C22T41 | A44G32C26T38 |
| *A. baumannii* | 62 | 1746 | 15 | A44G35C25T43 | A44G35C22T41 | A44G32C26T38 |
| *A. baumannii* | 4 | 689 | 15 | A44G35C25T43 | A44G35C22T41 | A44G32C26T38 |
| *A. baumannii* | 68 | 1822 | 3 | A44G35C24T44 | A44G35C22T41 | A44G32C26T38 |
| *A. baumannii* | 69 | 1823A | 3 | A44G35C24T44 | A44G35C22T41 | A44G32C26T38 |
| *A. baumannii* | 70 | 1823B | 3 | A44G35C24T44 | A44G35C22T41 | A44G32C26T38 |
| *A. baumannii* | 71 | 1826 | 3 | A44G35C24T44 | A44G35C22T41 | A44G32C26T38 |
| *A. baumannii* | 72 | 1860 | 3 | A44G35C24T44 | A44G35C22T41 | A44G32C26T38 |
| *A. baumannii* | 81 | 1924 | 3 | A44G35C24T44 | A44G35C22T41 | A44G32C26T38 |
| *A. baumannii* | 82 | 1929 | 3 | A44G35C24T44 | A44G35C22T41 | A44G32C26T38 |
| *A. baumannii* | 85 | 1966 | 3 | A44G35C24T44 | A44G35C22T41 | A44G32C26T38 |
| *A. baumannii* | 11 | 841 | 3 | A44G35C24T44 | A44G35C22T41 | A44G32C26T38 |
| *A. baumannii* | 32 | 1415 | 24 | A44G35C25T43 | A43G36C20T43 | A44G32C27T37 |
| *A. baumannii* | 45 | 1651 | 24 | A44G35C25T43 | A43G36C20T43 | A44G32C27T37 |
| *A. baumannii* | 54 | 1697 | 24 | A44G35C25T43 | A43G36C20T43 | A44G32C27T37 |
| *A. baumannii* | 58 | 1712 | 24 | A44G35C25T43 | A43G36C20T43 | A44G32C27T37 |
| *A. baumannii* | 60 | 1725 | 24 | A44G35C25T43 | A43G36C20T43 | A44G32C27T37 |
| *A. baumannii* | 66 | 1802 | 24 | A44G35C25T43 | A43G36C20T43 | A44G32C27T37 |
| *A. baumannii* | 76 | 1883 | 24 | ND | A43G36C20T43 | A44G32C27T37 |
| *A. baumannii* | 78 | 1891 | 24 | A44G35C25T43 | A43G36C20T43 | A44G32C27T37 |
| *A. baumannii* | 79 | 1892 | 24 | A44G35C25T43 | A43G36C20T43 | A44G32C27T37 |
| *A. baumannii* | 83 | 1947 | 24 | A44G35C25T43 | A43G36C20T43 | A44G32C27T37 |
| *A. baumannii* | 84 | 1964 | 24 | A44G35C25T43 | A43G36C20T43 | A44G32C27T37 |
| *A. baumannii* | 53 | 1696 | 24 | A44G35C25T43 | A43G36C20T43 | A44G32C27T37 |
| *A. baumannii* | 36 | 1458 | 49 | A44G35C25T43 | A44G35C22T41 | A44G32C27T37 |
| *A. baumannii* | 59 | 1716 | 9 | A44G35C25 | A44G35C21T42 | A44G32C26T38 |
| *A. baumannii* | 9 | 805 | 30 | A44G35C25T43 | A44G35C19T44 | A44G32C27T37 |
| *A. baumannii* | 18 | 967 | 39 | A45G34C25T43 | A44G35C22T41 | A44G32C26T38 |
| *A. baumannii* | 30 | 1322 | 48 | A44G35C25T43 | A43G36C20T43 | A44G32C27T37 |
| *A. baumannii* | 26 | 1218 | 50 | A44G35C25T43 | A44G35C21T42 | A44G32C26T38 |
| *A. sp.* 13TU | 15 | 875 | A1 | A47G33C24T43 | A46G32C20T44 | A44G33C27T36 |
| *A. sp.* 13TU | 17 | 895 | A1 | A47G33C24T43 | A46G32C20T44 | A44G33C27T36 |
| *A. sp.* 3 | 12 | 853 | B7 | A46G35C24T42 | A42G34C20T46 | A43G33C24T40 |
| *A. johnsonii* | 25 | 1202 | NEW1 | A46G35C23T43 | A42G35C21T44 | A43G33C23T41 |
| *A. sp.* 2082 | 87 | 2082 | NEW2 | A46G36C22T43 | AA42G32C20T48 | A42G34C23T41 |

**Table 17B: Base Compositions Determined from A. baumannii DNA Samples Obtained from Walter Reed Hospital and Amplified with Triangulation Genotyping Analysis Primer Pair Nos. 1158 and 1160 and 1165**

| **Species** | **Ibis#** | **Isolate** | **ST** | **PP No: 1158 mutY** | **PP No: 1160 mutY** | **PP No: 1165 fumC** |
|---|---|---|---|---|---|---|
| *A. baumannii* | 20 | 1082 | 1 | A27G21C25T22 | A32G35C29T33 | A40G33C30T36 |
| *A. baumannii* | 13 | 854 | 10 | A27G21C26T21 | A32G35C28T34 | A40G33C30T36 |
| *A. baumannii* | 22 | 1162 | 10 | A27G21C26T21 | A32G35C28T34 | A40G33C30T36 |
| *A. baumannii* | 27 | 1230 | 10 | A27G21C26T21 | A32G35C28T34 | A40G33C30T36 |
| *A. baumannii* | 31 | 1367 | 10 | A27G21C26T21 | A32G35C28T34 | A40G33C30T36 |
| *A. baumannii* | 37 | 1459 | 10 | A27G21C26T21 | A32G35C28T34 | A40G33C30T36 |
| *A. baumannii* | 55 | 1700 | 10 | A27G21C26T21 | A32G35C28T34 | A40G33C30T36 |
| *A. baumannii* | 64 | 1777 | 10 | A27G21C26T21 | A32G35C28T34 | A40G33C30T36 |
| *A. baumannii* | 73 | 1861 | 10 | A27G21C26T21 | A32G35C28T34 | A40G33C30T36 |
| *A. baumannii* | 74 | 1877 | 10 | A27G21C26T21 | A32G35C28T34 | A40G33C30T36 |
| *A. baumannii* | 86 | 1972 | 10 | A27G21C26T21 | A32G35C28T34 | A40G33C30T36 |
| *A. baumannii* | 3 | 684 | 11 | A27G21C25T22 | A32G34C28T35 | A40G33C30T36 |
| *A. baumannii* | 6 | 720 | 11 | A27G21C25T22 | A32G34C28T35 | A40G33C30T36 |
| *A. baumannii* | 7 | 726 | 11 | A27G21C25T22 | A32G34C28T35 | A40G33C30T36 |
| *A. baumannii* | 19 | 1079 | 11 | A27G21C25T22 | A32G34C28T35 | A40G33C30T36 |
| *A. baumannii* | 21 | 1123 | 11 | A27G21C25T22 | A32G34C28T35 | A40G33C30T36 |
| *A. baumannii* | 23 | 1188 | 11 | A27G21C25T22 | A32G34C28T35 | A40G33C30T36 |
| *A. baumannii* | 33 | 1417 | 11 | A27G21C25T22 | A32G34C28T35 | A40G33C30T36 |
| *A. baumannii* | 34 | 1431 | 11 | A27G21C25T22 | A32G34C28T35 | A40G33C30T36 |
| *A. baumannii* | 38 | 1496 | 11 | A27G21C25T22 | A32G34C28T35 | A40G33C30T36 |
| *A. baumannii* | 40 | 1523 | 11 | A27G21C25T22 | A32G34C28T35 | A40G33C30T36 |
| *A. baumannii* | 42 | 1640 | 11 | A27G21C25T22 | A32G34C28T35 | A40G33C30T36 |
| *A. baumannii* | 50 | 1666 | 11 | A27G21C25T22 | A32G34C28T35 | A40G33C30T36 |
| *A. baumannii* | 51 | 1668 | 11 | A27G21C25T22 | A32G34C28T35 | A40G33C30T36 |
| *A. baumannii* | 52 | 1695 | 11 | A27G21C25T22 | A32G34C28T35 | A40G33C30T36 |
| *A. baumannii* | 65 | 1781 | 11 | A27G21C25T22 | A32G34C28T35 | A40G33C30T36 |
| *A. baumannii* | 44 | 1649 | 12 | A27G21C26T21 | A32G34C29T34 | A40G33C30T36 |
| *A. baumannii* | 49A | 1658.1 | 12 | A27G21C26T21 | A32G34C29T34 | A40G33C30T36 |
| *A. baumannii* | 49B | 1658.2 | 12 | A27G21C26T21 | A32G34C29T34 | A40G33C30T36 |
| *A. baumannii* | 56 | 1707 | 12 | A27G21C26T21 | A32G34C29T34 | A40G33C30T36 |
| *A. baumannii* | 80 | 1893 | 12 | A27G21C26T21 | A32G34C29T34 | A40G33C30T36 |
| *A. baumannii* | 5 | 693 | 14 | A27G21C25T22 | A31G36C28T34 | A40G33C29T37 |
| *A. baumannii* | 8 | 749 | 14 | A27G21C25T22 | A31G36C28T34 | A40G33C29T37 |
| *A. baumannii* | 10 | 839 | 14 | A27G21C25T22 | A31G36C28T34 | A40G33C29T37 |
| *A. baumannii* | 14 | 865 | 14 | A27G21C25T22 | A31G36C28T34 | A40G33C29T37 |
| *A. baumannii* | 16 | 888 | 14 | A27G21C25T22 | A31G36C28T34 | A40G33C29T37 |
| *A. baumannii* | 29 | 1326 | 14 | A27G21C25T22 | A31G36C28T34 | A40G33C29T37 |
| *A. baumannii* | 35 | 1440 | 14 | A27G21C25T22 | A31G36C28T34 | A40G33C29T37 |
| *A. baumannii* | 41 | 1524 | 14 | A27G21C25T22 | A31G36C28T34 | A40G33C29T37 |
| *A. baumannii* | 46 | 1652 | 14 | A27G21C25T22 | A31G36C28T34 | A40G33C29T37 |
| *A. baumannii* | 47 | 1653 | 14 | A27G21C25T22 | A31G36C28T34 | A40G33C29T37 |
| *A. baumannii* | 48 | 1657 | 14 | A27G21C25T22 | A31G36C28T34 | A40G33C29T37 |
| *A. baumannii* | 57 | 1709 | 14 | A27G21C25T22 | A31G36C28T34 | A40G33C29T37 |
| *A. baumannii* | 61 | 1727 | 14 | A27G21C25T22 | A31G36C28T34 | A40G33C29T37 |
| *A. baumannii* | 63 | 1762 | 14 | A27G21C25T22 | A31G36C28T34 | A40G33C29T37 |
| *A. baumannii* | 67 | 1806 | 14 | A27G21C25T22 | A31G36C28T34 | A40G33C29T37 |
| *A. baumannii* | 75 | 1881 | 14 | A27G21C25T22 | A31G36C28T34 | A40G33C29T37 |
| *A. baumannii* | 77 | 1886 | 14 | A27G21C25T22 | A31G36C28T34 | A40G33C29T37 |
| *A. baumannii* | 1 | 649 | 46 | A29G19C26T21 | A31G35C29T34 | A40G33C29T37 |
| *A. baumannii* | 2 | 653 | 46 | A29G19C26T21 | A31G35C29T34 | A40G33C29T37 |
| *A. baumannii* | 39 | 1497 | 16 | A29G19C26T21 | A31G35C29T34 | A40G34C29T36 |
| *A. baumannii* | 24 | 1198 | 15 | A29G19C26T21 | A31G35C29T34 | A40G33C29T37 |
| *A. baumannii* | 28 | 1243 | 15 | A29G19C26T21 | A31G35C29T34 | A40G33C29T37 |
| *A. baumannii* | 43 | 1648 | 15 | A29G19C26T21 | A31G35C29T34 | A40G33C29T37 |
| *A. baumannii* | 62 | 1746 | 15 | A29G19C26T21 | A31G35C29T34 | A40G33C29T37 |
| *A. baumannii* | 4 | 689 | 15 | A29G19C26T21 | A31G35C29T34 | A40G33C29T37 |
| *A. baumannii* | 68 | 1822 | 3 | A27G20C27T21 | A32G35C28T34 | A40G33C30T36 |
| *A. baumannii* | 69 | 1823A | 3 | A27G20C27T21 | A32G35C28T34 | A40G33C30T36 |
| *A. baumannii* | 70 | 1823B | 3 | A27G20C27T21 | A32G35C28T34 | A40G33C30T36 |
| *A. baumannii* | 71 | 1826 | 3 | A27G20C27T21 | A32G35C28T34 | A40G33C30T36 |
| *A. baumannii* | 72 | 1860 | 3 | A27G20C27T21 | A32G35C28T34 | A40G33C30T36 |
| *A. baumannii* | 81 | 1924 | 3 | A27G20C27T21 | A32G35C28T34 | A40G33C30T36 |
| *A. baumannii* | 82 | 1929 | 3 | A27G20C27T21 | A32G35C28T34 | A40G33C30T36 |
| *A. baumannii* | 85 | 1966 | 3 | A27G20C27T21 | A32G35C28T34 | A40G33C30T36 |
| *A. baumannii* | 11 | 841 | 3 | A27G20C27T21 | A32G35C28T34 | A40G33C30T36 |
| *A. baumannii* | 32 | 1415 | 24 | A27G21C26T21 | A32G35C28T34 | A40G33C30T36 |
| *A. baumannii* | 45 | 1651 | 24 | A27G21C26T21 | A32G35C28T34 | A40G33C30T36 |
| *A. baumannii* | 54 | 1697 | 24 | A27G21C26T21 | A32G35C28T34 | A40G33C30T36 |
| *A. baumannii* | 58 | 1712 | 24 | A27G21C26T21 | A32G35C28T34 | A40G33C30T36 |
| *A. baumannii* | 60 | 1725 | 24 | A27G21C26T21 | A32G35C28T34 | A40G33C30T36 |
| *A. baumannii* | 66 | 1802 | 24 | A27G21C26T21 | A32G35C28T34 | A40G33C30T36 |
| *A. baumannii* | 76 | 1883 | 24 | A27G21C26T21 | A32G35C28T34 | A40G33C30T36 |
| *A. baumannii* | 78 | 1891 | 24 | A27G21C26T21 | A32G35C28T34 | A40G33C30T36 |
| *A. baumannii* | 79 | 1892 | 24 | A27G21C26T21 | A32G35C28T34 | A40G33C30T36 |
| *A. baumannii* | 83 | 1947 | 24 | A27G21C26T21 | A32G35C28T34 | A40G33C30T36 |
| *A. baumannii* | 84 | 1964 | 24 | A27G21C26T21 | A32G35C28T34 | A40G33C30T36 |
| *A. baumannii* | 53 | 1696 | 24 | A27G21C26T21 | A32G35C28T34 | A40G33C30T36 |
| *A. baumannii* | 36 | 1458 | 49 | A27G20C27T21 | A32G35C28T34 | A40G33C30T36 |
| *A. baumannii* | 59 | 1716 | 9 | A27G21C25T22 | A32G35C28T34 | A39G33C30T37 |
| *A. baumannii* | 9 | 805 | 30 | A27G21C25T22 | A32G35C28T34 | A39G33C30T37 |
| *A. baumannii* | 18 | 967 | 39 | A27G21C26T21 | A32G35C28T34 | A39G33C30T37 |
| *A. baumannii* | 30 | 1322 | 48 | A28G21C24T22 | A32G35C29T33 | A40G33C30T36 |
| *A. baumannii* | 26 | 1218 | 50 | A27G21C25T22 | A31G36C28T34 | A40G33C29T37 |
| *A. sp.* 13TU | 15 | 875 | A1 | A27G21C25T22 | A30G36C26T37 | A41G34C28T36 |
| *A. sp.* 13TU | 17 | 895 | A1 | A27G21C25T22 | A30G36C26T37 | A41G34C28T36 |
| *A. sp.* 3 | 12 | 853 | B7 | A26G23C23T23 | A30G36C27T36 | A39G37C26T37 |
| *A. johnsonii* | 25 | 1202 | NEW1 | A25G23C24T23 | A30G35C30T34 | A38G37C26T38 |
| *A. sp.* 2082 | 87 | 2082 | NEW2 | A26G22C24T23 | A31G35C28T35 | A42G34C27T36 |

**Table 17C: Base Compositions Determined from A. baumannii DNA Samples Obtained from Walter Reed Hospital and Amplified with Triangulation Genotyping Analysis Primer Pair Nos. 1167 and 1170 and 1171**

| **Species** | **Ibis#** | **Isolate** | **ST** | **PP No: 1167 fumC** | **PP No: 1170 fumC** | **PP No: 1171 ppa** |
|---|---|---|---|---|---|---|
| *A. baumannii* | 20 | 1082 | 1 | A41G34C34T38 | A38G27C21T50 | A35G37C33T44 |
| *A. baumannii* | 13 | 854 | 10 | A41G34C34T38 | A38G27C21T50 | A35G37C33T44 |
| *A. baumannii* | 22 | 1162 | 10 | A41G34C34T38 | A38G27C21T50 | A35G37C33T44 |
| *A. baumannii* | 27 | 1230 | 10 | A41G34C34T38 | A38G27C21T50 | A35G37C33T44 |
| *A. baumannii* | 31 | 1367 | 10 | A41G34C34T38 | A38G27C21T50 | A35G37C33T44 |
| *A. baumannii* | 37 | 1459 | 10 | A41G34C34T38 | A38G27C2lT50 | A35G37C33T44 |
| *A. baumannii* | 55 | 1700 | 10 | A41G34C34T38 | A38G27C21T50 | A35G37C33T44 |
| *A. baumannii* | 64 | 1777 | 10 | A41G34C34T38 | A38G27C21T50 | A35G37C33T44 |
| *A. baumannii* | 73 | 1861 | 10 | A41G34C34T38 | A38G27C21T50 | A35G37C33T44 |
| *A. baumannii* | 74 | 1877 | 10 | A41G34C34T38 | A38G27C21T50 | A35G37C33T44 |
| *A. baumannii* | 86 | 1972 | 10 | A41G34C34T38 | A38G27C21T50 | A35G37C33T44 |
| *A. baumannii* | 3 | 684 | 11 | A41G34C34T38 | A38G27C21T50 | A35G37C33T44 |
| *A. baumannii* | 6 | 720 | 11 | A41G34C34T38 | A38G27C21T50 | A35G37C33T44 |
| *A. baumannii* | 7 | 726 | 11 | A41G34C34T38 | A38G27C21T50 | A35G37C33T44 |
| *A. baumannii* | 19 | 1079 | 11 | A41G34C34T38 | A38G27C21T50 | A35G37C33T44 |
| *A. baumannii* | 21 | 1123 | 11 | A41G34C34T38 | A38G27C21T50 | A35G37C33T44 |
| *A. baumannii* | 23 | 1188 | 11 | A41G34C34T3E | A38G27C2lT50 | A35G37C33T44 |
| *A. baumannii* | 33 | 1417 | 11 | A41G34C34T38 | A38G27C21T50 | A35G37C33T44 |
| *A. baumannii* | 34 | 1431 | 11 | A41G34C34T38 | A38G27C21T50 | A35G37C33T44 |
| *A. baumannii* | 38 | 1496 | 11 | A41G34C34T38 | A38G27C21T50 | A35G37C33T44 |
| *A. baumannii* | 40 | 1523 | 11 | A41G34C34T38 | A38G27C21T50 | A35G37C33T44 |
| *A. baumannii* | 42 | 1640 | 11 | A41G34C34T38 | A38G27C21T50 | A35G37C33T44 |
| *A. baumannii* | 50 | 1666 | 11 | A41G34C34T38 | A38G27C21T50 | A35G37C33T44 |
| *A. baumannii* | 51 | 1668 | 11 | A41G34C34T38 | A38G27C21T50 | A35G37C33T44 |
| *A. baumannii* | 52 | 1695 | 11 | A41G34C34T38 | A38G27C21T50 | A35G37C33T44 |
| *A. baumannii* | 65 | 1781 | 11 | A41G34C34T38 | A38G27C21T50 | A35G37C33T44 |
| A. *baumannii* | 44 | 1649 | 12 | A41G34C34T38 | A38G27C21T50 | A35G37C33T44 |
| *A. baumannii* | 49A | 1658.1 | 12 | A41G34C34T38 | A38G27C21T50 | A35G37C33T44 |
| *A. baumannii* | 49B | 1658.2 | 12 | A41G34C34T38 | A38G27C21T50 | A35G37C33T44 |
| *A. baumannii* | 56 | 1707 | 12 | A41G34C34T38 | A38G27C21T50 | A35G37C33T44 |
| *A. baumannii* | 80 | 1893 | 12 | A41G34C34T38 | A38G27C21T50 | A35G37C33T44 |
| *A. baumannii* | 5 | 693 | 14 | A40G35C34T38 | A38G27C21T50 | A35G37C30T47 |
| *A. baumannii* | 8 | 749 | 14 | A40G35C34T38 | A38G27C21T50 | A35G37C30T47 |
| *A. baumannii* | 10 | 839 | 14 | A40G35C34T38 | A38G27C21T50 | A35G37C30T47 |
| *A. baumannii* | 14 | 865 | 14 | A40G35C34T38 | A38G27C21T50 | A35G37C30T47 |
| *A. baumannii* | 16 | 888 | 14 | A40G35C34T38 | A38G27C21T50 | A35G37C30T47 |
| *A. baumannii* | 29 | 1326 | 14 | A40G35C34T38 | A38G27C21T50 | A35G37C30T47 |
| *A. baumannii* | 35 | 1440 | 14 | A40G35C34T38 | A38G27C21T50 | A35G37C30T47 |
| *A. baumannii* | 41 | 1524 | 14 | A40G35C34T38 | A38G27C21T50 | A35G37C30T47 |
| *A. baumannii* | 46 | 1652 | 14 | A40G35C34T38 | A38G27C21T50 | A35G37C30T47 |
| *A. baumannii* | 47 | 1653 | 14 | A40G35C34T38 | A38G27C21T50 | A35G37C30T47 |
| *A. baumannii* | 48 | 1657 | 14 | A40G35C34T38 | A38G27C21T50 | A35G37C30T47 |
| *A. baumannii* | 57 | 1709 | 14 | A40G35C34T38 | A38G27C21T50 | A35G37C30T47 |
| *A. baumannii* | 61 | 1727 | 14 | A40G35C34T38 | A38G27C21T50 | A35G37C30T47 |
| *A. baumannii* | 63 | 1762 | 14 | A40G35C34T38 | A38G27C21T50 | A35G37C30T47 |
| *A. baumannii* | 67 | 1806 | 14 | A40G35C34T38 | A38G27C21T50 | A35G37C30T47 |
| *A. baumannii* | 75 | 1881 | 14 | A40G35C34T38 | A38G27C21T50 | A35G37C30T47 |
| *A. baumannii* | 77 | 1886 | 14 | A40G35C34T38 | A38G27C21T50 | A35G37C30T47 |
| *A. baumannii* | 1 | 649 | 46 | A41G35C32T39 | A37G28C20T51 | A35G37C32T45 |
| *A. baumannii* | 2 | 653 | 46 | A41G35C32T39 | A37G28C20T51 | A35G37C32T45 |
| *A. baumannii* | 39 | 1497 | 16 | A41G35C32T39 | A37G28C20T51 | A35G37C30T47 |
| *A. baumannii* | 24 | 1198 | 15 | A41G35C32T39 | A37G28C20T51 | A35G37C30T47 |
| *A. baumannii* | 28 | 1243 | 15 | A41G35C32T39 | A37G28C20T51 | A35G37C30T47 |
| *A. baumannii* | 43 | 1648 | 15 | A41G35C32T39 | A37G28C20T51 | A35G37C30T47 |
| *A. baumannii* | 62 | 1746 | 15 | A41G35C32T39 | A37G28C20T51 | A35G37C30T47 |
| *A. baumannii* | 4 | 689 | 15 | A41G35C32T39 | A37G28C20T51 | A35G37C30T47 |
| *A. baumannii* | 68 | 1822 | 3 | A41G34C35T37 | A38G27C20T51 | A35G37C31T46 |
| *A. baumannii* | 69 | 1823A | 3 | A41G34C35T37 | A38G27C20T51 | A35G37C31T46 |
| *A. baumannii* | 70 | 1823B | 3 | A41G34C35T37 | A38G27C20T51 | A35G37C31T46 |
| *A. baumannii* | 71 | 1826 | 3 | A41G34C35T37 | A38G27C20T51 | A35G37C31T46 |
| *A. baumannii* | 72 | 1860 | 3 | A41G34C35T37 | A38G27C20T51 | A35G37C31T46 |
| *A. baumannii* | 81 | 1924 | 3 | A41G34C35T37 | A38G27C20T51 | A35G37C31T46 |
| *A. baumannii* | 82 | 1929 | 3 | A41G34C35T37 | A38G27C20T51 | A35G37C31T46 |
| *A. baumannii* | 85 | 1966 | 3 | A41G34C35T37 | A38G27C20T51 | A35G37C31T46 |
| *A. baumannii* | 11 | 841 | 3 | A41G34C35T37 | A38G27C20T51 | A35G37C31T46 |
| *A. baumannii* | 32 | 1415 | 24 | A40G35C34T38 | A39G26C22T49 | A35G37C33T44 |
| *A. baumannii* | 45 | 1651 | 24 | A40G35C34T38 | A39G26C22T49 | A35G37C33T44 |
| *A. baumannii* | 54 | 1697 | 24 | A40G35C34T38 | A39G26C22T49 | A35G37C33T44 |
| *A. baumannii* | 58 | 1712 | 24 | A40G35C34T38 | A39G26C22T49 | A35G37C33T44 |
| *A. baumannii* | 60 | 1725 | 24 | A40G35C34T38 | A39G26C22T49 | A35G37C33T44 |
| *A. baumannii* | 66 | 1802 | 24 | A40G35C34T38 | A39G26C22T49 | A35G37C33T44 |
| *A. baumannii* | 76 | 1883 | 24 | A40G35C34T38 | A39G26C22T49 | A35G37C33T44 |
| *A. baumannii* | 78 | 1891 | 24 | A40G35C34T38 | A39G26C22T49 | A35G37C33T44 |
| *A. baumannii* | 79 | 1892 | 24 | A40G35C34T38 | A39G26C22T49 | A35G37C33T44 |
| *A. baumannii* | 83 | 1947 | 24 | A40G35C34T38 | A39G26C22T49 | A35G37C33T44 |
| *A. baumannii* | 84 | 1964 | 24 | A40G35C34T38 | A39G26C22T49 | A35G37C33T44 |
| *A. baumannii* | 53 | 1696 | 24 | A40G35C34T38 | A39G26C22T49 | A35G37C33T44 |
| *A. baumannii* | 36 | 1458 | 49 | A40G35C34T38 | A39G26C22T49 | A35G37C30T47 |
| *A. baumannii* | 59 | 1716 | 9 | A40G35C32T40 | A38G27C20T51 | A36G35C31T47 |
| *A. baumannii* | 9 | 805 | 30 | A40G35C32T40 | A38G27C21T50 | A35G36C29T49 |
| *A. baumannii* | 18 | 967 | 39 | A40G35C33T39 | A38G27C20T51 | A35G37C30T47 |
| *A. baumannii* | 30 | 1322 | 48 | A40G35C35T37 | A38G27C21T50 | A35G37C30T47 |
| *A. baumannii* | 26 | 1218 | 50 | AA40G35C34T38 | A38G27C21T50 | A35G37C33T44 |
| *A*. *sp*. 13TU | 15 | 875 | A1 | A41G39C31T36 | A37G26C24T49 | A34G38C31T46 |
| *A*. *sp*. 13TU | 17 | 895 | A1 | A41G39C31T36 | A37G26C24T49 | A34G38C31T46 |
| *A*. *sp*. 3 | 12 | 853 | B7 | A43G37C30T37 | A36G27C24T49 | A34G37C31T47 |
| *A. johnsonii* | 25 | 1202 | NEW1 | **A42G38C31T36** | **A40G27C19T50** | A35G37C32T45 |
| *A*. *sp*. 2082 | 87 | 2082 | NEW2 | **A43G37C32T35** | **A37G26C21T52** | **A35G38C31T45** |

**Table 18A: Base Compositions Determined from A. baumannii DNA Samples Obtained from Northwestern Medical Center and Amplified with Speciating Primer Pair No. 2922 and Triangulation Genotyping Analysis Primer Pair Nos. 1151 and 1156**

| **Species** | **Ibis#** | **Isolate** | **ST** | **PP No: 2922 efp** | **PP No: 1151 trpE** | **PP No: 1156 adk** |
|---|---|---|---|---|---|---|
| *A. bumannii* | 54 | 536 | 3 | A44G35C24T44 | A44G35C22T41 | A44G32C26T38 |
| *A. baumannii* | 87 | 665 | 3 | A44G35C24T44 | A44G35C22T41 | A44G32C26T38 |
| *A. baumannii* | 8 | 80 | 10 | A45G34C25T43 | A44G35C21T42 | A44G32C26T38 |
| *A. baumannii* | 9 | 91 | 10 | A45G34C25T43 | A44G35C21T42 | A44G32C26T38 |
| *A. baumannii* | 10 | 92 | 10 | A45G34C25T43 | A44G35C21T42 | A44G32C26T38 |
| *A. baumannii* | 11 | 131 | 10 | A45G34C25T43 | A44G35C21T42 | A44G32C26T38 |
| *A. baumannii* | 12 | 137 | 10 | A45G34C25T43 | A44G35C21T42 | A44G32C26T38 |
| *A. baumannii* | 21 | 218 | 10 | A45G34C25T43 | A44G35C21T42 | A44G32C26T38 |
| *A. baumannii* | 26 | 242 | 10 | A45G34C25T43 | A44G35C21T42 | A44G32C26T38 |
| *A. baumannii* | 94 | 678 | 10 | A45G34C25T43 | A44G35C21T42 | A44G32C26T38 |
| *A. baumannii* | 1 | 9 | 10 | A45G34C25T43 | A44G35C21T42 | A44G32C26T38 |
| *A. baumannii* | 2 | 13 | 10 | A45G34C25T43 | A44G35C21T42 | A44G32C26T38 |
| *A. baumannii* | 3 | 19 | 10 | A45G34C25T43 | A44G35C21T42 | A44G32C26T38 |
| *A. baumannii* | 4 | 24 | 10 | A45G34C25T43 | A44G35C21T42 | A44G32C26T38 |
| *A. baumannii* | 5 | 36 | 10 | A45G34C25T43 | A44G35C21T42 | A44G32C26T38 |
| *A. baumannii* | 6 | 39 | 10 | A45G34C25T43 | A44G35C21T42 | A44G32C26T38 |
| *A. baumannii* | 13 | 139 | 10 | A45G34C25T43 | A44G35C2lT42 | A44G32C26T38 |
| *A. baumannii* | 15 | 165 | 10 | A45G34C25T43 | A44G35C21T42 | A44G32C26T38 |
| *A. baumannii* | 16 | 170 | 10 | A45G34C25T43 | A44G35C21T42 | A44G32C26T38 |
| *A. baumannii* | 17 | 186 | 10 | A45G34C25T43 | A44G35C21T42 | A44G32C26T38 |
| *A. baumannii* | 20 | 202 | 10 | A45G34C25T43 | A44G35C21T42 | A44G32C26T38 |
| *A. baumannii* | 22 | 221 | 10 | A45G34C25T43 | A44G35C21T42 | A44G32C26T38 |
| *A. baumannii* | 24 | 234 | 10 | A45G34C25T43 | A44G35C21T42 | A44G32C26T38 |
| *A. baumannii* | 25 | 239 | 10 | A45G34C25T43 | A44G35C21T42 | A44G32C26T38 |
| *A. baumannii* | 33 | 370 | 10 | A45G34C25T43 | A44G35C21T42 | A44G32C26T38 |
| *A. baumannii* | 34 | 389 | 10 | A45G34C25T43 | A44G35C21T42 | A44G32C26T38 |
| *A. baumannii* | 19 | 201 | 14 | A44G35C25T43 | A44G35C22T41 | A44G32C27T37 |
| *A. baumannii* | 27 | 257 | 51 | A44G35C25T43 | A43G36C20T43 | A44G32C26T38 |
| *A. baumannii* | 29 | 301 | 51 | A44G35C25T43 | A43G36C20T43 | A44G32C26T38 |
| *A. baumannii* | 31 | 354 | 51 | A44G35C25T43 | A43G36C20T43 | A44G32C26T38 |
| *A. baumannii* | 36 | 422 | 51 | A44G35C25T43 | A43G36C20T43 | A44G32C26T38 |
| *A. baumannii* | 37 | 424 | 51 | A44G35C25T43 | A43G36C20T43 | A44G32C26T38 |
| *A. baumannii* | 38 | 434 | 51 | A44G35C25T43 | A43G36C20T43 | A44G32C26T38 |
| *A. baumannii* | 39 | 473 | 51 | A44G35C25T43 | A43G36C20T43 | A44G32C26T38 |
| *A. baumannii* | 40 | 482 | 51 | A44G35C25T43 | A43G36C20T43 | A44G32C26T38 |
| *A. baumannii* | 44 | 512 | 51 | A44G35C25T43 | A43G36C20T43 | A44G32C26T38 |
| *A. baumannii* | 45 | 516 | 51 | A44G35C25T43 | A43G36C20T43 | A44G32C26T38 |
| *A. baumannii* | 47 | 522 | 51 | A44G35C25T43 | A43G36C20T43 | A44G32C26T38 |
| *A. baumannii* | 48 | 526 | 51 | A44G35C25T43 | A43G36C20T43 | A44G32C26T38 |
| *A. baumannii* | 50 | 528 | 51 | A44G35C25T43 | A43G36C20T43 | A44G32C26T38 |
| *A. baumannii* | 52 | 531 | 51 | A44G35C25T43 | A43G36C20T43 | A44G32C26T38 |
| *A. baumannii* | 53 | 533 | 51 | A44G35C25T43 | A43G36C20T43 | A44G32C26T38 |
| *A. baumannii* | 56 | 542 | 51 | A44G35C25T43 | A43G36C20T43 | A44G32C26T38 |
| *A. baumannii* | 59 | 550 | 51 | A44G35C25T43 | A43G36C20T43 | A44G32C26T38 |
| *A. baumannii* | 62 | 556 | 51 | A44G35C25T43 | A43G36C20T43 | A44G32C26T38 |
| *A. baumannii* | 64 | 557 | 51 | A44G35C25T43 | A43G36C20T43 | A44G32C26T38 |
| *A. baumannii* | 70 | 588 | 51 | A44G35C25T43 | A43G36C20T43 | A44G32C26T38 |
| *A. baumannii* | 73 | 603 | 51 | A44G35C25T43 | A43G36C20T43 | A44G32C26T38 |
| *A. baumannii* | 74 | 605 | 51 | A44G35C25T43 | A43G36C20T43 | A44G32C26T38 |
| *A. baumannii* | 75 | 606 | 51 | A44G35C25T43 | A43G36C20T43 | A44G32C26T38 |
| *A. baumannii* | 77 | 611 | 51 | A44G35C25T43 | A43G36C20T43 | A44G32C26T38 |
| *A. baumannii* | 79 | 622 | 51 | A44G35C25T43 | A43G36C20T43 | A44G32C26T38 |
| *A. baumannii* | 83 | 643 | 51 | A44G35C25T43 | A43G36C20T43 | A44G32C26T38 |
| *A. baumannii* | 85 | 653 | 51 | A44G35C25T43 | A43G36C20T43 | A44G32C26T38 |
| *A. baumannii* | 89 | 669 | 51 | A44G35C25T43 | A43G36C20T43 | A44G32C26T38 |
| *A. baumannii* | 93 | 674 | 51 | A44G35C25T43 | A43G36C20T43 | A44G32C26T38 |
| *A. baumannii* | 23 | 228 | 51 | A44G35C25T43 | A43G36C20T43 | A44G32C26T38 |
| *A. baumannii* | 32 | 369 | 52 | A44G35C25T43 | A43G36C20T43 | A44G32C26T38 |
| *A. baumannii* | 35 | 393 | 52 | A44G35C25T43 | A43G36C20T43 | A44G32C26T38 |
| *A. baumannii* | 30 | 339 | 53 | A44G35C25T43 | A44G35C19T44 | A44G32C27T37 |
| *A. baumannii* | 41 | 485 | 53 | A44G35C25T43 | A44G35C19T44 | A44G32C27T37 |
| *A. baumannii* | 42 | 493 | 53 | A44G35C25T43 | A44G35C19T44 | A44G32C27T37 |
| *A. baumannii* | 43 | 502 | 53 | A44G35C25T43 | A44G35C19T44 | A44G32C27T37 |
| *A. baumannii* | 46 | 520 | 53 | A44G35C25T43 | A44G35C19T44 | A44G32C27T37 |
| *A. baumannii* | 49 | 527 | 53 | A44G35C25T43 | A44G35C19T44 | A44G32C27T37 |
| *A. baumannii* | 51 | 529 | 53 | A44G35C25T43 | A44G35C19T44 | A44G32C27T37 |
| *A. baumannii* | 65 | 562 | 53 | A44G35C25T43 | A44G35C19T44 | A44G32C27T37 |
| *A. baumannii* | 68 | 579 | 53 | A44G35C25T43 | A44G35C19T44 | A44G32C27T37 |
| *A. baumannii* | 57 | 546 | 54 | A44G35C25T43 | A44G35C20T43 | A44G32C26T38 |
| *A. baumannii* | 58 | 548 | 54 | A44G35C25T43 | A44G35C20T43 | A44G32C26T38 |
| *A. baumannii* | 60 | 552 | 54 | A44G35C25T43 | A44G35C20T43 | A44G32C26T38 |
| *A. baumannii* | 61 | 555 | 54 | A44G35C25T43 | A44G35C20T43 | A44G32C26T38 |
| *A. baumannii* | 63 | 557 | 54 | A44G35C25T43 | A44G35C20T43 | A44G32C26T38 |
| *A. baumannii* | 66 | 570 | 54 | A44G35C25T43 | A44G35C20T43 | A44G32C26T38 |
| *A. baumannii* | 67 | 578 | 54 | A44G35C25T43 | A44G35C20T43 | A44G32C26T38 |
| *A. baumannii* | 69 | 584 | 54 | A44G35C25T43 | A44G35C20T43 | A44G32C26T38 |
| *A. baumannii* | 71 | 593 | 54 | A44G35C25T43 | A44G35C20T43 | A44G32C26T38 |
| *A. baumannii* | 72 | 602 | 54 | A44G35C25T43 | A44G35C20T43 | A44G32C26T38 |
| *A*. *baumannii* | 76 | 609 | 54 | A44G35C25T43 | A44G35C20T43 | A44G32C26T38 |
| *A*. *baumannii* | 78 | 621 | 54 | A44G35C25T43 | A44G35C20T43 | A44G32C26T38 |
| *A. baumannii* | 80 | 625 | 54 | A44G35C25T43 | A44G35C20T43 | A44G32C26T38 |
| *A. baumannii* | 81 | 628 | 54 | A44G35C25T43 | A44G35C20T43 | A44G32C26T38 |
| *A. baumannii* | 82 | 632 | 54 | A44G35C25T43 | A44G35C20T43 | A44G32C26T38 |
| *A. baumannii* | 84 | 649 | 54 | A44G35C25T43 | A44G35C20T43 | A444G32C26T38 |
| *A. baumannii* | 86 | 655 | 54 | A44G35C25T43 | A44G35C20T43 | A44G32C26T38 |
| *A. baumannii* | 88 | 668 | 54 | A44G35C25T43 | A44G35C20T43 | A44G32C26T38 |
| *A. baumannii* | 90 | 671 | 54 | A44G35C25T43 | A44G35C20T43 | A44G32C26T38 |
| *A. baumannii* | 91 | 672 | 54 | A44G35C25T43 | A44G35C20T43 | A44G32C26T38 |
| *A. baumannii* | 92 | 673 | 54 | A44G35C25T43 | A44G35C20T43 | A44G32C26T38 |
| *A. baumannii* | 18 | 196 | 55 | A44G35C25T43 | A44G35C20T43 | A44G32C27T37 |
| *A. baumannii* | 55 | 537 | 27 | A44G35C25T43 | A44G35C19T44 | A44G32C27T37 |
| *A. baumannii* | 28 | 263 | 27 | A44G35C25T43 | A44G35C19T44 | A44G32C27T37 |
| *A. sp. 3* | 14 | 164 | B7 | A46G35C24T42 | A42G34C20T46 | A43G33C24T40 |
| mixture | 7 | 71 | ? | mixture | ND | ND |

**Table 18B: Base Compositions Determined from A. baumannii DNA Samples Obtained from Northwestern Medical Center and Amplified with Triangulation Genotyping Analysis Primer Pair Nos. 1158,1160 and 1165**

| **Species** | **Ibis#** | **Isolate** | **ST** | **PP No: 1158 mutY** | **PP No: 1160 mutY** | **PP No: 1165 fumC** |
|---|---|---|---|---|---|---|
| *A. baumannii* | 54 | 536 | 3 | A27G20C27T21 | A32G35C28T34 | A40G33C30T36 |
| *A. baumannii* | 87 | 665 | 3 | A27G20C27T21 | A32G35C28T34 | A40G33C30T36 |
| *A. baumannii* | 8 | 80 | 10 | A27G21C26T21 | A32G35C28T34 | A40G33C30T36 |
| *A. baumannii* | 9 | 91 | 10 | A27G21C26T21 | A32G35C28T34 | A40G33C30T36 |
| *A. baumannii* | 10 | 92 | 10 | A27G21C26T21 | A32G35C28T34 | A40G33C30T36 |
| *A. baumannii* | 11 | 131 | 10 | A27G21C26T21 | A32G35C28T34 | A40G33C30T36 |
| *A. baumannii* | 12 | 137 | 10 | A27G21C26T21 | A32G35C28T34 | A40G33C30T36 |
| *A. baumannii* | 21 | 218 | 10 | A27G21C26T21 | A32G35C28T34 | A40G33C30T36 |
| *A. baumannii* | 26 | 242 | 10 | A27G21C26T21 | A32G35C28T34 | A40G33C30T36 |
| *A. baumannii* | 94 | 678 | 10 | A27G21C26T21 | A32G35C28T34 | A40G33C30T36 |
| *A. baumannii* | 1 | 9 | 10 | A27G21C26T21 | A32G35C28T34 | A40G33C30T36 |
| *A. baumannii* | 2 | 13 | 10 | A27G21C26T21 | A32G35C28T34 | A40G33C30T36 |
| *A. baumannii* | 3 | 19 | 10 | A27G21C26T21 | A32G35C28T34 | A40G33C30T36 |
| *A. baumannii* | 4 | 24 | 10 | A27G21C26T21 | A32G35C28T34 | A40G33C30T36 |
| *A. baumannii* | 5 | 36 | 10 | A27G21C26T21 | A32G35C28T34 | A40G33C30T36 |
| *A. baumannii* | 6 | 39 | 10 | A27G21C26T21 | A32G35C28T34 | A40G33C30T36 |
| *A. baumannii* | 13 | 139 | 10 | A27G21C26T21 | A32G35C28T34 | A40G33C30T36 |
| *A. baumannii* | 15 | 165 | 10 | A27G21C26T21 | A32G35C28T34 | A40G33C30T36 |
| *A. baumannii* | 16 | 170 | 10 | A27G21C26T21 | A32G35C28T34 | A40G33C30T36 |
| *A. baumannii* | 17 | 186 | 10 | A27G21C26T21 | A32G35C28T34 | A40G33C30T36 |
| *A. baumannii* | 20 | 202 | 10 | A27G21C26T21 | A32G35C28T34 | A40G33C30T36 |
| *A. baumannii* | 22 | 221 | 10 | A27G21C26T21 | A32G35C28T34 | A40G33C30T36 |
| *A. baumannii* | 24 | 234 | 10 | A27G21C26T21 | A32G35C28T34 | A40G33C30T36 |
| *A. baumannii* | 25 | 239 | 10 | A27G21C26T21 | A32G35C28T34 | A40G33C30T36 |
| *A. baumannii* | 33 | 370 | 10 | A27G21C26T21 | A32G35C28T34 | A40G33C30T36 |
| *A. baumannii* | 34 | 389 | 10 | A27G21C26T21 | A32G35C28T34 | A40G33C30T36 |
| *A. baumannii* | 19 | 201 | 14 | A27G21C25T22 | A31G36C28T34 | A40G33C29T37 |
| *A. baumannii* | 27 | 257 | 51 | A27G21C25T22 | A32G35C28T34 | A40G33C29T37 |
| *A. baumannii* | 29 | 301 | 51 | A27G21C25T22 | A32G35C28T34 | A40G33C29T37 |
| *A. baumannii* | 31 | 354 | 51 | A27G21C25T22 | A32G35C28T34 | A40G33C29T37 |
| *A. baumannii* | 36 | 422 | 51 | A27G21C25T22 | A32G35C28T34 | A40G33C29T37 |
| *A. baumannii* | 37 | 424 | 51 | A27G21C25T22 | A32G35C28T34 | A40G33C29T37 |
| *A. baumannii* | 38 | 434 | 51 | A27G21C25T22 | A32G35C28T34 | A40G33C29T37 |
| *A. baumannii* | 39 | 473 | 51 | A27G21C25T22 | A32G35C28T34 | A40G33C29T37 |
| *A. baumannii* | 40 | 482 | 51 | A27G21C25T22 | A32G35C28T34 | A40G33C29T37 |
| *A. baumannii* | 44 | 512 | 51 | A27G21C25T22 | A32G35C28T34 | A40G33C29T37 |
| *A. baumannii* | 45 | 516 | 51 | A27G21C25T22 | A32G35C28T34 | A40G33C29T37 |
| *A. baumannii* | 47 | 522 | 51 | A27G21C25T22 | A32G35C28T34 | A40G33C29T37 |
| *A. baumannii* | 48 | 526 | 51 | A27G21C25T22 | A32G35C28T34 | A40G33C29T37 |
| *A. baumannii* | 50 | 528 | 51 | A27G21C25T22 | A32G35C28T34 | A40G33C29T37 |
| *A. baumannii* | 52 | 531 | 51 | A27G21C25T22 | A32G35C28T34 | A40G33C29T37 |
| *A. baumannii* | 53 | 533 | 51 | A27G21C25T22 | A32G35C28T34 | A40G33C29T37 |
| A. *baumannii* | 56 | 542 | 51 | A27G21C25T22 | A32G35C28T34 | A40G33C29T37 |
| *A. baumannii* | 59 | 550 | 51 | A27G21C25T22 | A32G35C28T34 | A40G33C29T37 |
| *A. baumannii* | 62 | 556 | 51 | A27G21C25T22 | A32G35C28T34 | A40G33C29T37 |
| *A. baumannii* | 64 | 557 | 51 | A27G21C25T22 | A32G35C28T34 | A40G33C29T37 |
| *A. baumannii* | 70 | 588 | 51 | A27G21C25T22 | A32G35C28T34 | A40G33C29T37 |
| *A. baumannii* | 73 | 603 | 51 | A27G21C25T22 | A32G35C28T34 | A40G33C29T37 |
| *A. baumannii* | 74 | 605 | 51 | A27G21C25T22 | A32G35C28T34 | A40G33C29T37 |
| *A. baumannii* | 75 | 606 | 51 | A27G21C25T22 | A32G35C28T34 | A40G33C29T37 |
| *A. baumannii* | 77 | 611 | 51 | A27G21C25T22 | A32G35C28T34 | A40G33C29T37 |
| *A. baumannii* | 79 | 622 | 51 | A27G21C25T22 | A32G35C28T34 | A40G33C29T37 |
| A. *baumannii* | 83 | 643 | 51 | A27G21C25T22 | A32G35C28T34 | A40G33C29T37 |
| *A. baumannii* | 85 | 653 | 51 | A27G21C25T22 | A32G35C28T34 | A40G33C29T37 |
| *A. baumannii* | 89 | 669 | 51 | A27G21C25T22 | A32G35C28T34 | A40G33C29T37 |
| *A. baumannii* | 93 | 674 | 51 | A27G21C25T22 | A32G35C28T34 | A40G33C29T37 |
| *A. baumannii* | 23 | 228 | 51 | A27G21C25T22 | A32G35C28T34 | A40G33C29T37 |
| *A. baumannii* | 32 | 369 | 52 | A27G21C25T22 | A32G35C28T34 | A40G33C29T37 |
| *A. baumannii* | 35 | 393 | 52 | A27G21C25T22 | A32G35C28T34 | A40G33C29T37 |
| *A. baumannii* | 30 | 339 | 53 | A28G20C26T21 | A32G34C29T34 | A40G33C30T36 |
| *A. baumannii* | 41 | 485 | 53 | A28G20C26T21 | A32G34C29T34 | A40G33C30T36 |
| *A. baumannii* | 42 | 493 | 53 | A28G20C26T21 | A32G34C29T34 | A40G33C30T36 |
| *A. baumannii* | 43 | 502 | 53 | A28G20C26T21 | A32G34C29T34 | A40G33C30T36 |
| *A. baumannii* | 46 | 520 | 53 | A28G20C26T21 | A32G34C29T34 | A40G33C30T36 |
| *A. baumannii* | 49 | 527 | 53 | A28G20C26T21 | A32G34C29T34 | A40G33C30T36 |
| *A. baumannii* | 51 | 529 | 53 | A28G20C26T21 | A32G34C29T34 | A40G33C30T36 |
| *A. baumannii* | 65 | 562 | 53 | A28G20C26T21 | A32G34C29T34 | A40G33C30T36 |
| *A. baumannii* | 68 | 579 | 53 | A28G20C26T21 | A32G34C29T34 | A40G33C30T36 |
| *A. baumannii* | 57 | 546 | 54 | A27G21C26T21 | A32G34C29T34 | A40G33C30T36 |
| *A. baumannii* | 58 | 548 | 54 | A27G21C26T21 | A32G34C29T34 | A40G33C30T36 |
| *A. baumannii* | 60 | 552 | 54 | A27G21C26T21 | A32G34C29T34 | A40G33C30T36 |
| *A. baumannii* | 61 | 555 | 54 | A27G21C26T21 | A32G34C29T34 | A40G33C30T36 |
| *A. baumannii* | 63 | 557 | 54 | A27G21C26T21 | A32G34C29T34 | A40G33C30T36 |
| *A. baumannii* | 66 | 570 | 54 | A27G21C26T21 | A32G34C29T34 | A40G33C30T36 |
| *A. baumannii* | 67 | 578 | 54 | A27G21C26T21 | A32G34C29T34 | A40G33C30T36 |
| *A. baumannii* | 69 | 584 | 54 | A27G21C26T21 | A32G34C29T34 | A40G33C30T36 |
| *A. baumannii* | 71 | 593 | 54 | A27G21C26T21 | A32G34C29T34 | A40G33C30T36 |
| *A. baumannii* | 72 | 602 | 54 | A27G21C26T21 | A32G34C29T34 | A40G33C30T36 |
| *A. baumannii* | .76 | 609 | 54 | A27G21C26T21 | A32G34C29T34 | A40G33C30T36 |
| *A. baumannii* | 78 | 621 | 54 | A27G21C26T21 | A32G34C29T34 | A40G33C30T36 |
| *A. baumannii* | 80 | 625 | 54 | A27G21C26T21 | A32G34C29T34 | A40G33C30T36 |
| *A. baumannii* | 81 | 628 | 54 | A27G21C26T21 | A32G34C29T34 | A40G33C30T36 |
| *A. baumannii* | 82 | 632 | 54 | A27G21C26T21 | A32G34C29T34 | A40G33C30T36 |
| *A. baumannii* | 84 | 649 | 54 | A27G21C26T21 | A32G34C29T34 | A40G33C30T36 |
| *A. baumannii* | 86 | 655 | 54 | A27G21C26T21 | A32G34C29T34 | A40G33C30T36 |
| *A. baumannii* | 88 | 668 | 54 | A27G21C26T21 | A32G34C29T34 | A40G33C30T36 |
| *A. baumannii* | 90 | 671 | 54 | A27G21C26T21 | A32G34C29T34 | A40G33C30T36 |
| *A. baumannii* | 91 | 672 | 54 | A27G21C26T21 | A32G34C29T34 | A40G33C30T36 |
| *A. baumannii* | 92 | 673 | 54 | A27G21C26T21 | A32G34C29T34 | A40G33C30T36 |
| *A. baumannii* | 18 | 196 | 55 | A27G21C25T22 | A31G36C27T35 | A40G33C29T37 |
| *A. baumannii* | 55 | 537 | 27 | A27G21C25T22 | A32G35C28T34 | A40G33C30T36 |
| *A. baumannii* | 28 | 263 | 27 | A27G21C25T22 | A32G35C28T34 | A40G33C30T36 |
| *A. sp. 3* | 14 | 164 | B7 | A26G23C23T23 | A30G36C27T36 | A39G37C26T37 |
| mixture | 7 | 71 | ? | ND | ND | ND |

**Table 18C: Base Compositions Determined from A. baumannii DNA Samples Obtained from Northwestern Medical Center and Amplified with Triangulation Genotyping Analysis Primer Pair Nos.1167,1170 and 1171**

| **Species** | **Ibis#** | **Isolate** | **ST** | **PP No: 1167 fumC** | **PP No: 1170 fumC** | **PP No: 1171 ppa** |
|---|---|---|---|---|---|---|
| *A. baumannii* | 54 | 536 | 3 | A41G34C35T37 | A38G27C20T51 | A35G37C31T46 |
| *A. baumannii* | 87 | 665 | 3 | A41G34C35T37 | A38G27C20T51 | A35G37C31T46 |
| *A. baumannii* | 8 | 80 | 10 | A41G34C34T38 | A38G27C21T50 | A35G37C33T441 |
| *A. baumannii* | 9 | 91 | 10 | A41G34C34T38 | A38G27C21T50 | A35G37C33T44 |
| *A. baumannii* | 10 | 92 | 10 | A41G34C34T38 | A38G27C21T50 | A35G37C33T44 |
| *A. baumannii* | 11 | 131 | 10 | A41G34C34T38 | A38G27C21T50 | A35G37C33T44 |
| *A. baumannii* | 12 | 137 | 10 | A41G34C34T38 | A38G27C21T50 | A35G37C33T44 |
| *A. baumannii* | 21 | 218 | 10 | A41G34C34T38 | A38G27C21T50 | A35G37C33T44 |
| *A. baumannii* | 26 | 242 | 10 | A41G34C34T38 | A38G27C21T50 | A35G37C33T44 |
| *A. baumannii* | 94 | 678 | 10 | A41G34C34T38 | A38G27C21T50 | A35G37C33T44 |
| *A. baumannii* | 1 | 9 | 10 | A41G34C34T38 | A38G27C21T50 | A35G37C33T44 |
| *A. baumannii* | 2 | 13 | 10 | A41G34C34T38 | A38G27C21T50 | A35G37C33T44 |
| *A. baumannii* | 3 | 19 | 10 | A41G34C34T38 | A38G27C21T50 | A35G37C33T44 |
| *A. baumannii* | 4 | 24 | 10 | A41G34C34T38 | A38G27C21T50 | A35G37C33T44 |
| *A. baumannii* | 5 | 36 | 10 | A41G34C34T38 | A38G27C21T50 | A35G37C33T44 |
| *A. baumannii* | 6 | 39 | 10 | A41G34C34T38 | A38G27C21T50 | A35G37C33T44 |
| *A. baumannii* | 13 | 139 | 10 | A41G34C34T38 | A38G27C21T50 | A35G37C33T44 |
| *A. baumannii* | 15 | 165 | 10 | A41G34C34T38 | A38G27C21T50 | A35G37C33T44 |
| *A. baumannii* | 16 | 170 | 10 | A41G34C34T38 | A38G27C21T50 | A35G37C33T44 |
| *A. baumannii* | 17 | 186 | 10 | A41G34C34T38 | A38G27C21T50 | A35G37C33T44 |
| *A. baumannii* | 20 | 202 | 10 | A41G34C34T38 | A38G27C21T50 | A35G37C33T44 |
| *A. baumannii* | 22 | 221 | 10 | A41G34C34T38 | A38G27C21T50 | A35G37C33T44 |
| *A. baumannii* | 24 | 234 | 10 | A41G34C34T38 | A38G27C21T50 | A35G37C33T44 |
| *A. baumannii* | 25 | 239 | 10 | A41G34C34T38 | A38G27C21T50 | A35G37C33T44 |
| *A. baumannii* | 33 | 370 | 10 | A41G34C34T38 | A38G27C21T50 | A35G37C33T44 |
| *A. baumannii* | 34 | 389 | 10 | A41G34C34T38 | A38G27C21T50 | A35G37C33T44 |
| *A. baumannii* | 19 | 201 | 14 | A40G35C34T38 | A38G27C21T50 | A35G37C30T47 |
| *A. baumannii* | 27 | 257 | 51 | A40G35C34T38 | A38G27C21T50 | A35G37C30T47 |
| *A. baumannii* | 29 | 301 | 51 | A40G35C34T38 | A38G27C21T50 | A35G37C30T47 |
| *A. baumannii* | 31 | 354 | 51 | A40G35C34T38 | A38G27C21T50 | A35G37C30T47 |
| *A. baumannii* | 36 | 422 | 51 | A40G35C34T38 | A38G27C21T50 | A35G37C30T47 |
| *A. baumannii* | 37 | 424 | 51 | A40G35C34T38 | A38G27C21T50 | A35G37C30T47 |
| *A. baumannii* | 38 | 434 | 51 | A40G35C34T38 | A38G27C21T50 | A35G37C30T47 |
| *A. baumannii* | 39 | 473 | 51 | A40G35C34T38 | A38G27C21T50 | A35G37C30T47 |
| *A. baumannii* | 40 | 482 | 51 | A40G35C34T38 | A38G27C21T50 | A35G37C30T47 |
| *A. baumannii* | 44 | 512 | 51 | A40G35C34T38 | A38G27C21T50 | A35G37C30T47 |
| *A. baumannii* | 45 | 516 | 51 | A40G35C34T38 | A38G27C21T50 | A35G37C30T47 |
| *A. baumannii* | 47 | 522 | 51 | A40G35C34T38 | A38G27C21T50 | A35G37C30T47 |
| *A. baumannii* | 48 | 526 | 51 | A40G35C34T38 | A38G27C21T50 | A35G37C30T47 |
| *A. baumannii* | 50 | 528 | 51 | A40G35C34T38 | A38G27C21T50 | A35G37C30T47 |
| *A. baumannii* | 52 | 531 | 51 | A40G35C34T38 | A38G27C21T50 | A35G37C30T47 |
| *A. baumannii* | 53 | 533 | 51 | A40G35C34T38 | A38G27C21T50 | A35G37C30T47 |
| *A. baumannii* | 56 | 542 | 51 | A40G35C34T38 | A38G27C21T50 | A35G37C30T47 |
| *A. baumannii* | 59 | 550 | 51 | A40G35C34T38 | A38G27C21T50 | A35G37C30T47 |
| *A. baumannii* | 62 | 556 | 51 | A40G35C34T38 | A38G27C21T50 | A35G37C30T47 |
| *A. baumannii* | 64 | 557 | 51 | A40G35C34T38 | A38G27C21T50 | A35G37C30T47 |
| *A. baumannii* | 70 | 588 | 51 | A40G35C34T38 | A38G27C21T50 | A35G37C30T47 |
| *A. baumannii* | 73 | 603 | 51 | A40G35C34T38 | A38G27C21T50 | A35G37C30T47 |
| *A. baumannii* | 74 | 605 | 51 | A40G35C34T38 | A38G27C21T50 | A35G37C30T47 |
| *A. baumannii* | 75 | 606 | 51 | A40G35C34T38 | A38G27C21T50 | A35G37C30T47 |
| *A. baumannii* | 77 | 611 | 51 | A40G35C34T38 | A38G27C21T50 | A35G37C30T47 |
| *A. baumannii* | 79 | 622 | 51 | A40G35C34T38 | A38G27C21T50 | A35G37C30T47 |
| *A. baumannii* | 83 | 643 | 51 | A40G35C34T38 | A38G27C21T50 | A35G37C30T47 |
| *A. baumannii* | 85 | 653 | 51 | A40G35C34T38 | A38G27C21T50 | A35G37C30T47 |
| *A. baumannii* | 89 | 669 | 51 | A40G35C34T38 | A38G27C21T50 | A35G37C30T47 |
| *A. baumannii* | 93 | 674 | 51 | A40G35C34T38 | A38G27C21T50 | A35G37C30T47 |
| *A. baumannii* | 23 | 228 | 51 | A40G35C34T38 | A38G27C21T50 | A35G37C30T47 |
| *A. baumannii* | 32 | 369 | 52 | A40G35C34T38 | A38G27C21T50 | A35G37C31T46 |
| *A. baumannii* | 35 | 393 | 52 | A40G35C34T38 | A38G27C21T50 | A35G37C31T46 |
| *A. baumannii* | 30 | 339 | 53 | A40G35C35T37 | A38G27C21T50 | A35G37C31T46 |
| *A. baumannii* | 41 | 485 | 53 | A40G35C35T37 | A38G27C21T50 | A35G37C31T46 |
| *A. baumannii* | 42 | 493 | 53 | A40G35C35T37 | A38G27C21T50 | A35G37C31T46 |
| *A. baumannii* | 43 | 502 | 53 | A40G35C35T37 | A38G27C21T50 | A35G37C31T46 |
| *A. baumannii* | 46 | 520 | 53 | A40G35C35T37 | A38G27C21T50 | A35G37C31T46 |
| *A. baumannii* | 49 | 527 | 53 | A40G35C35T37 | A38G27C21T50 | A35G37C31T46 |
| *A. baumannii* | 51 | 529 | 53 | A40G35C35T37 | A38G27C21T50 | A35G37C31T46 |
| *A. baumannii* | 65 | 562 | 53 | A40G35C35T37 | A38G27C21T50 | A35G37C31T46 |
| *A. baumannii* | 68 | 579 | 53 | A40G35C35T37 | A38G27C21T50 | A35G37C31T46 |
| *A. baumannii* | 57 | 546 | 54 | A40G35C34T38 | A39G26C22T49 | A35G37C31T46 |
| *A. baumannii* | 58 | 548 | 54 | A40G35C34T38 | A39G26C22T49 | A35G37C31T46 |
| *A. baumannii* | 60 | 552 | 54 | A40G35C34T38 | A39G26C22T49 | A35G37C31T46 |
| *A. baumannii* | 61 | 555 | 54 | A40G35C34T38 | A39G26C22T49 | A35G37C31T46 |
| *A. baumannii* | 63 | 557 | 54 | A40G35C34T38 | A39G26C22T49 | A35G37C31T46 |
| *A. baumannii* | 66 | 570 | 54 | A40G35C34T38 | A39G26C22T49 | A35G37C31T46 |
| *A. baumannii* | 67 | 578 | 54 | A40G35C34T38 | A39G26C22T49 | A35G37C31T46 |
| *A. baumannii* | 69 | 584 | 54 | A40G35C34T38 | A39G26C22T49 | A35G37C31T46 |
| *A. baumannii* | 71 | 593 | 54 | A40G35C34T38 | A39G26C22T49 | A35G37C31T46 |
| *A. baumannii* | 72 | 602 | 54 | A40G35C34T38 | A39G26C22T49 | A35G37C31T46 |
| *A. baumannii* | 76 | 609 | 54 | A40G35C34T38 | A39G26C22T49 | A35G37C31T46 |
| *A. baumannii* | 78 | 621 | 54 | A40G35C34T38 | A39G26C22T49 | A35G37C31T46 |
| *A. baumannii* | 80 | 625 | 54 | A40G35C34T38 | A39G26C22T49 | A35G37C31T46 |
| A. baumannii | 81 | 628 | 54 | A40G35C34T38 | A39G26C22T49 | A35G37C31T46 |
| *A. baumannii* | 82 | 632 | 54 | A40G35C34T38 | A39G26C22T49 | A35G37C31T46 |
| *A. baumannii* | 84 | 649 | 54 | A40G35C34T38 | A39G26C22T49 | A35G37C31T46 |
| *A. baumannii* | 86 | 655 | 54 | A40G35C34T38 | A39G26C22T49 | A35G37C31T46 |
| *A. baumannii* | 88 | 668 | 54 | A40G35C34T38 | A39G26C22T49 | A35G37C31T46 |
| *A. baumannii* | 90 | 671 | 54 | A40G35C34T38 | A39G26C22T49 | A35G37C31T46 |
| *A. baumannii* | 91 | 672 | 54 | A40G35C34T38 | A39G26C22T49 | A35G37C31T46 |
| *A. baumannii* | 92 | 673 | 54 | A40G35C34T38 | A39G26C22T49 | A35G37C31T46 |
| *A. baumannii* | 18 | 196 | 55 | A42G34C33T38 | A38G27C20T51 | A35G37C31T46 |
| *A. baumannii* | 55 | 537 | 27 | A40G35C33T39 | A38G27C20T51 | A35G37C33T44 |
| *A. baumannii* | 28 | 263 | 27 | A40G35C33T39 | A38G27C20T51 | A35G37C33T44 |
| A. sp. 3 | 14 | 164 | B7 | A43G37C30T37 | A36G27C24T49 | A34G37C31T47 |
| mixture | 7 | 71 | - | ND | ND | ND |

Base composition analysis of the samples obtained from Walter Reed hospital indicated that a majority of the strain types identified were the same strain types already characterized by the OIF study of Example 12. This is not surprising since at least some patients from which clinical samples were obtained in OIF were transferred to the Walter Reed Hospital (WRAIR). Examples of these common strain types include: ST10, ST11, ST12, ST14, ST15, ST16 and ST46. A strong correlation was noted between these strain types and the presence of mutations in the gyrA and parC which confer quinolone drug resistance.

In contrast, the results of base composition analysis of samples obtained from Northwestern Medical Center indicate the presence of 4 major strain types: ST10, ST51, ST53 and ST54. All of these strain types have the gyrA quinolone resistance mutation and most also have the parC quinolone resistance mutation, with the exception of ST35. This observation is consistent with the current understanding that the gyrA mutation generally appears before the parC mutation and suggests that the acquisition of these drug resistance mutations is rather recent and that resistant isolates are taking over the wild-type isolates. Another interesting observation was that a single isolate of ST3 (isolate 841) displays a triangulation genotyping analysis pattern similar to other isolates of ST3, but the codon analysis amplification product base compositions indicate that this isolate has not yet undergone the quinolone resistance mutations in gyrA and parC.

The six isolates that represent species other than *Acinetobacter baumannii* in the samples obtained from the Walter Reed Hospital were each found to not carry the drug resistance mutations.

The results described above involved analysis of 183 samples using the methods and compositions of the present invention. Results were provided to collaborators at the Walter Reed hospital and Northwestern Medical center within a week of obtaining samples. This example highlights the rapid throughput characteristics of the analysis platform and the resolving power of triangulation genotyping analysis and codon analysis for identification of and determination of drug resistance in bacteria.

### Example 14: Identification of Drug Resistance Genes and Virulence Factors in Staphylococcus aureus

An eight primer pair panel was designed for identification of drug resistance genes and virulence factors of *Staphylococcus aureus* and is shown in Table 19. The primer sequences are found in Table 2 and are cross-referenced by the primer pair numbers, primer pair names or SEQ ID NOs listed in Table 19.

**Table 19: Primer Pairs for Identification of Drug Resistance Genes and Virulence Factors in Staphylococcus aureus**

| **Primer Pair No.** | **Forward Primer Name** | **Forward Primer (SEQ ID NO:)** | **Reverse Primer Name** | **Reverse Primer (SEQ ID NO:)** | **Target Gene** |
|---|---|---|---|---|---|
| 879 | MECA Y14051 4507 4530 F | 288 | MECA Y14051 4555 4581 R | 1269 | mecA |
| 2056 | MECI-R_NC003923-41798-41609 33 60 F | 698 | MECI-R_NC003923-41798-41609 86 113 R | 1420 | MecI-R |
| 2081 | ERMA NC002952-55890-56621 366 395 F | 217 | ERMA NC002952-55890-56621 438 465 R | 1167 | ermA |
| 2086 | ERMC_NC005908-2004-2738 85 116 F | 399 | ERMC_NC005908-2004-2738 173 206 R | 1041 | ermC |
| 2095 | PVLUK_NC003923-1529595-1531285 688 713 F | 456 | PVLUK_NC003923-1529595-1531285 775 804 R | 1261 | Pv-luk |
| 2249 | TUFB_NC002758-615038-616222 696 725 F | 430 | TUFB_NC002758-615038-616222 793 820 R | 1321 | tufB |
| 2256 | NUC_NC002758-894288-894974 316 345 F | 174 | NUC_NC002758-894288-894974 396 421 R | 853 | Nuc |
| 2313 | MUPR X75439 2486 2516 F | 172 | MUPR_X75439 2548 2574 R | 1360 | mupR |

Primer pair numbers 2256 and 2249 are confirmation primers designed with the aim of high level identification of *Staphylococcus aureus.* The nuc gene is a *Staphylococcus* aureus-specific marker gene. The tufB gene is a universal housekeeping gene but the bioagent identifying amplicon defined by primer pair number 2249 provides a unique base composition (A43 G28 C19 T35) which distinguishes *Staphylococcus aureus* from other members of the genus *Staphylococcus.*

High level methicillin resistance in a given strain of *Staphylococcus aureus* is indicated by bioagent identifying amplicons defined by primer pair numbers 879 and 2056. Analyses have indicated that primer pair number 879 is not expected to prime *S. sciuri* homolog or *Enterococcus faecalis*/*faciem* ampicillin-resistant PBP5 homologs.

Macrolide and erythromycin resistance in a given strain of *Staphylococcus aureus* is indicated by bioagent identifying amplicons defined by primer pair numbers 2081 and 2086.

Resistance to mupriocin in a given strain of *Staphylococcus aureus* is indicated by bioagent identifying amplicons defined by primer pair number 2313.

Virulence in a given strain of *Staphylococcus aureus* is indicated by bioagent identifying amplicons defined by primer pair number 2095. This primer pair can simultaneously and identify the pvl (lukS-PV) gene and the lukD gene which encodes a homologous enterotoxin. A bioagent identifying amplicon of the lukD gene has a six nucleobase length difference relative to the lukS-PV gene.

A total of 32 blinded samples of different strains of *Staphylococcus aureus* were provided by the Center for Disease Control (CDC). Each sample was analyzed by PCR amplification with the eight primer pair panel, followed by purification and measurement of molecular masses of the amplification products by mass spectrometry. Base compositions for the amplification products were calculated. The base compositions provide the information summarized above for each primer pair. The results are shown in Tables 20A and B. One result noted upon un-blinding of the samples is that each of the PVL+ identifications agreed with PVL+ identified in the same samples by standard PCR assays. These results indicate that the panel of eight primer pairs is useful for identification of drug resistance and virulence sub-species characteristics for *Staphylococcus aureus.* It is expected that a kit comprising one or more of the members of this panel will be a useful embodiment of the present invention.

**Table 20A: Drug Resistance and Virulence Identified in Blinded Samples of Various Strains of Staphylococcus aureus with Primer Pair Nos. 2081, 2086, 2095 and 2256**

| **Sample Index No.** | **Primer Pair No. 2081 (ermA)** | **Primer Pair No. 2086 (ermC)** | **Primer Pair No. 2095 (pv-luk)** | **Primer Pair No. 2256 (nuc)** |
|---|---|---|---|---|
| CDC0010 | - | - | PVL-/lukD+ | + |
| CDC0015 | - | - | PVL+/lukD+ | + |
| CDC0019 | - | + | PVL-/lukD+ | + |
| CDC0026 | + | - | PVL-/lukD+ | + |
| CDC0030 | + | - | PVL-/lukD+ | + |
| CDC004 | - | - | PVL+/lukD+ | + |
| CDC0014 | - | + | PVL+/lukD+ | + |
| CDC008 | - | - | PVL-/lukD+ | + |
| CDC001 | + | - | PVL-/lukD+ | + |
| CDC0022 | + | - | PVL-/lukD+ | + |
| CDC006 | + | - | PVL-/lukD+ | + |
| CDC007 | - | - | PVL-/lukD+ | + |
| CDCVRSA1 | + | - | PVL-/lukD+ | + |
| CDCVRSA2 | + | + | PVL-/lukD+ | + |
| CDC0011 | + | - | PVL-/lukD+ | + |
| CDC0012 | - | - | PVL+/lukD- | + |
| CDC0021 | + | - | PVL-/lukD+ | + |
| CDC0023 | + | - | PVL-/lukD+ | + |
| CDC0025 | + | - | PVL-/lukD+ | + |
| CDC005 | - | - | PVL-/lukD+ | + |
| CDC0018 | + | - | PVL+/lukD- | + |
| CDC002 | - | - | PVL-/lukD+ | + |
| CDC0028 | + | - | PVL-/lukD+ | + |
| CDC003 | - | - | PVL-/lukD+ | + |
| CDC0013 | - | - | PVL+/lukD+ | + |
| CDC0016 | - | - | PVL-/lukD+ | + |
| CDC0027 | + | - | PVL-/lukD+ | + |
| CDC0029 | - | - | PVL+/lukD+ | + |
| CDC0020 | - | + | PVL-/lukD+ | + |
| CDC0024 | - | - | PVL-/lukD+ | + |
| CDC0031 | - | - | PVL-/lukD+ | + |

**Table 20B: Drug Resistance and Virulence Identified in Blinded Samples of Various Strains of Staphylococcus aureus with Primer Pair Nos. 2249, 879, 2056, and 2313**

| **Sample Index No.** | **Primer Pair No. 2249 (tufB)** | **Primer Pair No. 879 (mecA)** | **Primer Pair No. 2056 (mecI-R)** | **Primer Pair No. 2313 (mupR)** |
|---|---|---|---|---|
| CDC0010 | *Staphylococcus aureus* | + | + | - |
| CDC0015 | *Staphylococcus aureus* | - | - | - |
| CDC0019 | *Staphylococcus aureus* | + | + | - |
| CDC0026 | *Staphylococcus aureus* | + | + | - |
| CDC0030 | *Staphylococcus aureus* | + | + | - |
| CDC004 | *Staphylococcus aureus* | + | + | - |
| CDC0014 | *Staphylococcus aureus* | + | + | - |
| CDC008 | *Staphylococcus aureus* | + | + | - |
| CDC001 | *Staphylococcus aureus* | + | + | - |
| CDC0022 | *Staphylococcus aureus* | + | + | - |
| CDC006 | *Staphylococcus aureus* | + | + | + |
| CDC007 | *Staphylococcus aureus* | + | + | - |
| CDCVRSA1 | *Staphylococcus aureus* | + | + | - |
| CDCVRSA2 | *Staphylococcus aureus* | + | + | - |
| CDC0011 | *Staphylococcus aureus* | - | - | - |
| CDC0012 | *Staphylococcus aureus* | + | + | - |
| CDC0021 | *Staphylococcus aureus* | + | + | - |
| CDC0023 | *Staphylococcus aureus* | + | + | - |
| CDC0025 | *Staphylococcus aureus* | + | + | - |
| CDC005 | *Staphylococcus aureus* | + | + | - |
| CDC0018 | *Staphylococcus aureus* | + | + | - |
| CDC002 | *Staphylococcus aureus* | + | + | - |
| CDC0028 | *Staphylococcus aureus* | + | + | - |
| CDC003 | *Staphylococcus aureus* | + | + | - |
| CDC0013 | *Staphylococcus aureus* | + | + | - |
| CDC0016 | *Staphylococcus aureus* | + | + | - |
| CDC0027 | *Staphylococcus aureus* | + | + | - |
| CDC0029 | *Staphylococcus aureus* | + | + | - |
| CDC0020 | *Staphylococcus aureus* | - | - | - |
| CDC0024 | *Staphylococcus aureus* | + | + | - |
| CDC0031 | *Staphylococcus scleiferi* | - | - | - |

### Example 15: Selection and Use of Triangulation Genotyping Analysis Primer Pairs for Staphylococcus aureus

To combine the power of high-throughput mass spectrometric analysis of bioagent identifying amplicons with the sub-species characteristic resolving power provided by triangulation genotyping analysis, a panel of eight triangulation genotyping analysis primer pairs was selected. The primer pairs are designed to produce bioagent identifying amplicons within six different housekeeping genes which are listed in Table 21. The primer sequences are found in Table 2 and are cross-referenced by the primer pair numbers, primer pair names or SEQ ID NOs listed in Table 21.

**Table 21: Primer Pairs for Triangulation Genotyping Analysis of Staphylococcus aureus**

| **Primer Pair No.** | **Forward Primer Name** | **Forward Primer (SEQ ID NO:)** | **Reverse Primer Name** | **Reverse Primer (SEQ ID NO:)** | **Target Gene** |
|---|---|---|---|---|---|
| 2146 | ARCC_NC003923-2725050-2724595_131_161_F | 437 | ARCC_NC003923-2725050-2724595 214 245 R | 1137 | arcC |
| 2149 | AROE_NC003923-1674726-1674277_30_62_F | 530 | AROE_NC003923-1674726-1674277 155 181 R | 891 | aroE |
| 2150 | AROE_NC003923-1674726-1674277_204_232_F | 474 | AROE_NC003923-1674726-1674277 308 335 R | 869 | aroE |
| 2156 | GMK_NC003923-1190906-1191334_301_329_F | 268 | GMK_NC003923-1190906-1191334 403 432 R | 1284 | gmk |
| 2157 | PTA₋NC003923-628885-629355_237_263_F | 418 | PTA_NC003923-628885-629355 314 345 R | 1301 | pta |
| 2161 | TPI_NC003923-830671-831072_1_34_F | 318 | TPI_NC003923-830671-831072 97 129 R | 1300 | tpi |
| 2163 | YQI NC003923-378916-379431_142_167_F | 440 | YQI_NC003923-378916-379431 259 284 R | 1076 | yqi |
| 2166 | YQI_NC003923-378916-379431_275_300_F | 219 | YQI_NC003923-378916-379431 364 396 R | 1013 | yqi |

The same samples analyzed for drug resistance and virulence in Example 14 were subjected to triangulation genotyping analysis. The primer pairs of Table 21 were used to produce amplification products by PCR, which were subsequently purified and measured by mass spectrometry. Base compositions were calculated from the molecular masses and are shown in Tables 22A and 22B.

**Table 22A: Triangulation Genotyping Analysis of Blinded Samples of Various Strains of Staphylococcus aureus with Primer Pair Nos. 2146, 2149, 2150 and 2156**

| **Sample Index No.** | **Strain** | **Primer Pair No. 2146 (arcC)** | **Primer Pair No. 2149(aroE)** | **Primer Pair No. 2150 (aroE)** | **Primer Pair No. 2156 (gmk)** |
|---|---|---|---|---|---|
| CDC0010 | COL | A44 G24 C18 T29 | A59 G24 C18 T51 | A40 G36 C13 T43 | A50 G30 C20 T32 |
| CDC0015 | COL | A44 G24 C18 T29 | A59 G24 C18 T51 | A40 G36 C13 T43 | A50 G30 C20 T32 |
| CDC0019 | COL | A44 G24 C18 T29 | A59 G24 C18 T51 | A40 G36 C13 T43 | A50 G30 C20 T32 |
| CDC0026 | COL | A44 G24 C18 T29 | A59 G24 C18 T51 | A40 G36 C13 T43 | A50 G30 C20 T32 |
| CDC0030 | COL | A44 G24 C18 T29 | A59 G24 C18 T51 | A40 G36 C13 T43 | A50 G30 C20 T32 |
| CDC004 | COL | A44 G24 C18 T29 | A59 G24 C18 T51 | A40 G36 C13 T43 | A50 G30 C20 T32 |
| CDC0014 | COL | A44 G24 C18 T29 | A59 G24 C18 T51 | A40 G36 C13 T43 | A50 G30 C20 T32 |
| CDC008 | ???? | A44 G24 C18 T29 | A59 G24 C18 T51 | A40 G36 C13 T43 | A50 G30 C20 T32 |
| CDC001 | Mu50 | A45 G23 C20 T27 | A58 G24 C18 T52 | A40 G36 C13 T43 | A51 G29 C21 T31 |
| CDC0022 | Mu50 | A45 G23 C20 T27 | A58 G24 C18 T52 | A40 G36 C13 T43 | A51 G29 C21 T31 |
| CDC006 | Mu50 | A45 G23 C20 T27 | A58 G24 C18 T52 | A40 G36 C13 T43 | A51 G29 C21 T31 |
| CDC0011 | MRSA252 | A45 G24 C18 T28 | A58 G24 C19 T51 | A41 G36 C12 T43 | A51 G29 C21 T31 |
| CDC0012 | MRSA252 | A45 G24 C18 T28 | A58 G24 C19 T51 | A41 G36 C12 T43 | A51 G29 C21 T31 |
| CDC0021 | MRSA252 | A45 G24 C18 T28 | A58 G24 C19 T51 | A41 G36 C12 T43 | A51 G29 C21 T31 |
| CDC0023 | ST:110 | A45 G24 C18 T28 | A59 G24 C18 T51 | A40 G36 C13 T43 | A50 G30 C20 T32 |
| CDC0025 | ST:110 | A45 G24 C18 T28 | A59 G24 C18 T51 | A40 G36 C13 T43 | A50 G30 C20 T32 |
| CDC005 | ST:338 | A44 G24 C18 T29 | A59 G23 C19 T51 | A40 G36 C14 T42 | A51 G29 C21 T31 |
| CDC0018 | ST:338 | A44 G24 C18 T29 | A59 G23 C19 T51 | A40 G36 C14 T42 | A51 G29 C21 T31 |
| CDC002 | ST:108 | A46 G23 C20 T26 | A58 G24 C19 T51 | A42 G36 C12 T42 | A51 G29 C20 T32 |
| CDC0028 | ST:108 | A46 G23 C20 T26 | A58 G24 C19 T51 | A42 G36 C12 T42 | A51 G29 C20 T32 |
| CDC003 | ST:107 | A45 G23 C20 T27 | A58 G24 C18 T52 | A40 G36 C13 T43 | A51 G29 C21 T31 |
| CDC0013 | ST:12 | ND | A59 G24 C18 T51 | A40 G36 C13 T43 | A51 G29 C21 T31 |
| CDC0016 | ST:120 | A45 G23 C18 T29 | A58 G24 C19 T51 | A40 G37 C13 T42 | A51 G29 C21 T31 |
| CDC0027 | ST:105 | A45 G23 C20 T27 | A58 G24 C18 T52 | A40 G36 C13 T43 | A51 G29 C21 T31 |
| CDC0029 | MSSA476 | A45 G23 C20 T27 | A58 G24 C19 T51 | A40 G36 C13 T43 | A50 G30 C20 T32 |
| CDC0020 | ST:15 | A44 G23 C21 T27 | A59 G23 C18 T52 | A40 G36 C13 T43 | A50 G30 C20 T32 |
| CDC0024 | ST:137 | A45 G23 C20 T27 | A57 G25 C19 T51 | A40 G36 C13 T43 | A51 G29 C22 T30 |
| CDC0031 | *** | No product | No product | No product | No product |

**Table 22B: Triangulation Genotyping Analysis of Blinded Samples of Various Strains of Staphylococcus aureus with Primer Pair Nos. 2146, 2149, 2150 and 2156**

| **Sample Index No.** | **Strain** | **Primer Pair No. 2157 (pta)** | **Primer Pair No. 2161 (tpi)** | **Primer Pair No. 2163 (yqi)** | **Primer Pair No. 2166 (yqi)** |
|---|---|---|---|---|---|
| CDC0010 | COL | A32 G25 C23 T29 | A51 G28 C22 T28 | A41 G37 C22 T43 | A37 G30 C18 T37 |
| CDC0015 | COL | A32 G25 C23 T29 | A51 G28 C22 T28 | A41 G37 C22 T43 | A37 G30 C18 T37 |
| CDC0019 | COL | A32 G25 C23 T29 | A51 G28 C22 T28 | A41 G37 C22 T43 | A37 G30 C18 T37 |
| CDC0026 | COL | A32 G25 C23 T29 | A51 G28 C22 T28 | A41 G37 C22 T43 | A37 G30 C18 T37 |
| CDC0030 | COL | A32 G25 C23 T29 | A51 G28 C22 T28 | A41 G37 C22 T43 | A37 G30 C18 T37 |
| CDC004 | COL | A32 G25 C23 T29 | A51 G28 C22 T28 | A41 G37 C22 T43 | A37 G30 C18 T37 |
| CDC0014 | COL | A32 G25 C23 T29 | A51 G28 C22 T28 | A41 G37 C22 T43 | A37 G30 C18 T37 |
| CDCDC008 | unknown | A32 G25 C23 T29 | A51 G28 C22 T28 | A41 G37 C22 T43 | A37 G30 C18 T37 |
| CDC001 | Mu50 | A33 G25 C22 T29 | A50 G28 C22 T29 | A42 G36 C22 T43 | A36 G31 C19 T36 |
| CDC0022 | Mu50 | A33 G25 C22 T29 | A50 G28 C22 T29 | A42 G36 C22 T43 | A36 G31 C19 T36 |
| CDC006 | Mu50 | A33 G25 C22 T29 | A50 G28 C22 T29 | A42 G36 C22 T43 | A36 G31 C19 T36 |
| CDC0011 | MRSA252 | A32 G25 C23 T29 | A50 G28 C22 T29 | A42 G36 C22 T43 | A37 G30 C18 T37 |
| CDC0012 | MRSA252 | A32 G25 C23 T29 | A50 G28 C22 T29 | A42 G36 C22 T43 | A37 G30 C18 T37 |
| CDC0021 | MESA252 | A32 G25 C23 T29 | A50 G28 C22 T29 | A42 G36 C22 T43 | A37 G30 C18 T37 |
| CDC0023 | ST:110 | A32 G25 C23 T29 | A51 G28 C22 T28 | A41 G37 C22 T43 | A37 G30 C18 T37 |
| CDC0025 | ST:110 | A32 G25 C23 T29 | A51 G28 C22 T28 | A41 G37 C22 T43 | A37 G30 C18 T37 |
| CDC005 | ST:338 | A32 G25 C24 T28 | A51 G27 C21 T30 | A42 G36 C22 T43 | A37 G30 C18 T37 |
| CDC0018 | ST:338 | A32 G25 C24 T28 | A51 G27 C21 T30 | A42 G36 C22 T43 | A37 G30 C18 T37 |
| CDC002 | ST:108 | A33 G25 C23 T28 | A50 G28 C22 T29 | A42 G36 C22 T43 | A37 G30 C18 T37 |
| CDC0028 | ST:108 | A33 G25 C23 T28 | A50 G28 C22 T29 | A42 G36 C22 T43 | A37 G30 C18 T37 |
| CDC003 | ST:107 | A32 G25 C23 T29 | A51 G28 C22 T28 | A41 G37 C22 T43 | A37 G30 C18 T37 |
| CDC0013 | ST:12 | A32 G25 C23 T29 | A51 G28 C22 T28 | A42 G36 C22 T43 | A37 G30 C18 T37 |
| CDC0016 | ST:120 | A32 G25 C24 T28 | A50 G28 C21 T30 | A42 G36 C22 T43 | A37 G30 C18 T37 |
| CDC0027 | ST:105 | A33 G25 C22 T29 | A50 G28 C22 T29 | A43 G36 C21 T43 | A36 G31 C19 T36 |
| CDC0029 | MSSA476 | A33 G25 C22 T29 | A50 G28 C22 T29 | A42 G36 C22 T43 | A36 G31 C19 T36 |
| CDC0020 | ST:15 | A33 G25 C22 T29 | A50 G28 C21 T30 | A42 G36 C22 T43 | A36 G31 C18 T37 |
| CDC0024 | ST:137 | A33 G25 C22 T29 | A51 G28 C22 T28 | A42 G36 C22 T43 | A37 G30 C18 T37 |
| CDC0031 | *** | A34 G25 C25 T25 | A51 G27 C24 T27 | No product | No product |

Note: *** The sample CDC0031 was identified as *Staphylococcus scleiferi* as indicated in Example 14. Thus, the triangulation genotyping primers designed for *Staphylococcus aureus* would generally not be expected to prime and produce amplification products of this organism. Tables 22A and 22B indicate that amplification products are obtained for this organism only with primer pair numbers 2157 and 2161.

A total of thirteen different genotypes of *Staphylococcus aureus* were identified according to the unique combinations of base compositions across the eight different bioagent identifying amplicons obtained with the eight primer pairs. These results indicate that this eight primer pair panel is useful for analysis of unknown or newly emerging strains of *Staphylococcus aureus.* It is expected that a kit comprising one or more of the members of this panel will be a useful embodiment of the present invention.

### Example 16: Selection and Use of Triangulation Genotyping Analysis Primer Pairs for Members of the Bacterial Genus Vibrio

To combine the power of high-throughput mass spectrometric analysis ofbioagent identifying amplicons with the sub-species characteristic resolving power provided by triangulation genotyping analysis, a panel of eight triangulation genotyping analysis primer pairs was selected. The primer pairs are designed to produce bioagent identifying amplicons within seven different housekeeping genes which are listed in Table 23. The primer sequences are found in Table 2 and are cross-referenced by the primer pair numbers, primer pair names or SEQ ID NOs listed in Table 23.

**Table 23: Primer Pairs for Triangulation Genotyping Analysis of Members of the Bacterial Genus Vibrio**

| **Primer Pair No.** | **Forward Primer Name** | **Forward Primer (SEQ ID NO:)** | **Reverse Primer Name** | **Reverse Primer (SEQ ID NO:)** | **Target Gene** |
|---|---|---|---|---|---|
| 1098 | RNASEP VBC 331 349 F | 325 | RNASEP VBC 388 414 R | 1163 | RNAse P |
| 2000 | CTXB NC002505 46 70 F | 278 | CTXB NC002505 132 162 R | 1039 | ctxB |
| 2001 | FUR NC002505 87 113 F | 465 | FUR NC002505 205 228 R | 1037 | fur |
| 2011 | GYRB_NC002505_1161_1190 F | 148 | GYRB NC002505 1255 1284 R | 1172 | gyrB |
| 2012 | OMPU NC002505 85 110 F | 190 | OMPU NC002505 154 180 R | 1254 | ompU |
| 2014 | OMPU NC002505 431 455 F | 266 | OMPU NC002505 544 567 R | 1094 | ompU |
| 2323 | CTXA_NC002505-1568114-1567341 122 149 F | 508 | CTXA_NC002505-1568114-1567341 186 214 R | 1297 | ctxA |
| 2927 | GAPA NC002505 694 721 F | 259 | GAPA NC 002505 29 58 R | 1060 | gapA |

A group of 50 bacterial isolates containing multiple strains of both environmental and clinical isolates of *Vibrio cholerae,* 9 other *Vibrio* species, and 3 species of Photobacteria were tested using this panel of primer pairs. Base compositions of amplification products obtained with these 8 primer pairs were used to distinguish amongst various species tested, including sub-species differentiation within *Vibrio cholerae* isolates. For instance, the non-O1/non-O139 isolates were clearly resolved from the O1 and the 0139 isolates, as were several of the environmental isolates of *Vibrio cho*/*erae* from the clinical isolates.

It is expected that a kit comprising one or more of the members of this panel will be a useful embodiment of the present invention.

### Example 17: Selection and Use of Triangulation Genotyping Analysis Primer Pairs for Members of the Bacterial Genus Pseudomonas

To combine the power of high-throughput mass spectrometric analysis of bioagent identifying amplicons with the sub-species characteristic resolving power provided by triangulation genotyping analysis, a panel of twelve triangulation genotyping analysis primer pairs was selected. The primer pairs are designed to produce bioagent identifying amplicons within seven different housekeeping genes which are listed in Table 24. The primer sequences are found in Table 2 and are cross-referenced by the primer pair numbers, primer pair names or SEQ ID NOs listed in Table 24.

**Table 24: Primer Pairs for Triangulation Genotyping Analysis of Members of the Bacterial Genus Pseudomonas**

| **Primer Pair No.** | **Forward Primer Name** | **Forward Primer (SEQ ID NO:)** | **Reverse Primer Name** | **Reverse Primer (SEQ ID NO:)** | **Target Gene** |
|---|---|---|---|---|---|
| 2949 | ACS_NC002516-970624-971013_299_316_F | 376 | ACS_NC002516-970624-971013_364_383_R | 1265 | acsA |
| 2950 | ARO_NC002516-26883-27380_4_26_F | 267 | ARO_NC002516-26883-27380_111_128_R | 1341 | aroE |
| 2951 | ARO_NC003516-26883-27380_356_377_F | 705 | ARO_NC002516-26883-27380_459_484_R | 1056 | aroE |
| 2954 | GUA_NC002516-4226546-4226174_155_178_F | 710 | GUA_NC002516-4226546-4226174_265_287_R | 1259 | guaA |
| 2956 | GUA_NC002516-4226546-4226174_242_263_F | 374 | GUA_NC002516-4226546-4226174_355_371_R | 1111 | guaA |
| 2957 | MUT_NC002516-5551158-5550717_5_26_F | 545 | MUT_NC002516-5551158-5550717_99_116_R | 978 | mutL |
| 2959 | NUO_NC002516-2984589-2984954_8_26_F | 249 | NUO_NC002516-2984589-2984954_97_117_R | 1095 | nuoD |
| 2960 | NUO_NC002516-2984589-2984954_218_239_F | 195 | NUO_NC002516-2984589-2984954_301_326_R | 1376 | nuoD |
| 2961 | PPS_NC002516-1915014-1915383_44_63_F | 311 | PPS_NC002516-1915014-1915383_140_165_R | 1014 | pps |
| 2962 | PPS_NC002516-1915014-1915383_240_258_F | 365 | PPS_NC002516-1915014-1915383_341_360_R | 1052 | pps |
| 2963 | TRP_NC002516-671831-672273_24_42_F | 527 | TRP_NC002516-671831-672273_131_150_R | 1071 | trpE |
| 2964 | TRP_NC002516-671831-672273_261_282_F | 490 | TRP_NC002516-671831-672273_362_383_R | 1182 | trpE |

It is expected that a kit comprising one or more of the members of this panel will be a useful embodiment of the present invention.

The present invention includes any combination of the various species and subgeneric groupings falling within the generic disclosure. This invention therefore includes the generic description of the invention with a proviso or negative limitation removing any subj ect matter from the genus, regardless of whether or not the excised material is specifically recited herein.

While in accordance with the patent statutes, description of the various embodiments and examples have been provided, the scope of the invention is not to be limited thereto or thereby. Modifications and alterations of the present invention will be apparent to those skilled in the art without departing from the scope of the present invention.

Therefore, it will be appreciated that the scope of this invention is to be defined by the appended claims, rather than by the specific examples which have been presented by way of example.

## Claims

1. A composition comprising:
(a) an oligonucleotide primer having SEQ NO:456; and
(b) an oligonucleotide primer having SEQ ID NO:1261.

2. The composition of claim 1 wherein either or both of said primers comprises at least one modified nucleobase.

3. The composition of claim 2 wherein said modified nucleobase is 5-propynyluracil or 5-propynylcytosine.

4. The composition of claim 1 wherein either or both of said primers comprises at least one universal nucleobase.

5. The composition of claim 4 wherein said universal nucleobase is inosine.

6. The composition of claim 1 wherein either or both of said primers further comprises a non-templated T residue on the 5'-end.

7. The composition of claim 1 wherein either or both of said primers comprises at least one non-template tag.

8. The composition of claim 1 wherein either or both of said primers comprises at least one molecular mass modifying tag.

9. A kit comprising the composition of claim 1, and further comprising one or more primer pairs from the group of primer pairs represented by SEQ ID NOs: 288:1269, 698:1420, 217:1167, 399:1041, 430:1321, 174:853, and 172:1360.

10. The kit of claim 9 further comprising one or more calibration polynucleotides.

11. The kit of claim 9 further comprising at least one anion exchange functional group linked to a magnetic bead.

12. A method for identification of a strain of *Staphylococcus aureus* in a sample comprising:
amplifying nucleic acid from said strain of *Staphylococcus aureus* using the composition of claim 1 to obtain an amplification product;
determining the molecular mass of said amplification product;
optionally, determining the base composition of said amplification product from said molecular mass; and
comparing said molecular mass or said base composition with a plurality of molecular masses or base compositions of known amplification products of strains of *Staphylococcus aureus* defined by the composition of claim 1, wherein a match between said molecular mass or base composition and a member of said plurality of molecular masses or base compositions identifies said strain of *Staphylococcus aureus.*

13. The method of claim 12 further comprising repeating said amplifying, determining, optionally determining, and comparing steps using at least one additional primer pair from the group of primer pairs represented by SEQ ID NOs: 288:1269, 698:1420, 217:1167, 399:1041, 430:1321, 174:853, and 172:1360.

14. A method for determination of the quantity of a strain of *Staphylococcus aureus* in a sample comprising:
contacting said sample with the composition of claim 1 and a known quantity of a calibration polynucleotide comprising a calibration sequence;
concurrently amplifying nucleic acid from said a strain of *Staphylococcus aureus* and nucleic acid from said calibration polynucleotide in said sample with the composition of claim 1 to obtain a first amplification product comprising a bacterial bioagent identifying amplicon and a second amplification product comprising a calibration amplicon;
determining the molecular mass and abundance for said bacterial bioagent identifying amplicon and said calibration amplicon; and
distinguishing said bacterial bioagent identifying amplicon from said calibration amplicon based on molecular mass, wherein comparison of bacterial bioagent identifying amplicon abundance and calibration amplicon abundance indicates the quantity of said strain of *Staphylococcus aureus* in said sample.

15. The method of claim 12 or 14 wherein said strain of *Staphylococcus aureus* is a virulent strain.

## Patentansprüche

1. Zusammensetzung, umfassend:
(a) einen Oligonukleotid-Primer mit der SEQ ID NO: 456; und
(b) einen Oligonukleotid-Primer mit der SEQ ID NO: 1261.

2. Zusammensetzung nach Anspruch 1, wobei einer der oder beide Primer wenigstens eine modifizierte Nukleobase umfasst bzw. umfassen.

3. Zusammensetzung nach Anspruch 2, wobei es sich bei der modifizierten Nukleobase um 5-Propinyluracil oder 5-Propinylcytosin handelt.

4. Zusammensetzung nach Anspruch 1, wobei einer der oder beide Primer wenigstens eine universelle Nukleobase umfasst bzw. umfassen.

5. Zusammensetzung nach Anspruch 4, wobei es sich bei der universellen Nukleobase um Inosin handelt.

6. Zusammensetzung nach Anspruch 1, wobei einer der oder beide Primer ferner einen Nicht-Matrizen-T-Rest am 5'-Ende umfasst bzw. umfassen.

7. Zusammensetzung nach Anspruch 1, wobei einer der oder beide Primer wenigstens ein Nicht-Matrizen-Tag umfasst bzw. umfassen.

8. Zusammensetzung nach Anspruch 1, wobei einer der oder beide Primer wenigstens ein die Molekülmasse modifizierendes Tag umfasst bzw. umfassen.

9. Kit, umfassend die Zusammensetzung nach Anspruch 1 und ferner umfassend ein oder mehrere Primer-Paare aus der durch die SEQ ID NO: 288:1269, 698:1420, 217:1167, 399:1041, 430:1321, 174:853 und 172:1360 repräsentierten Gruppe von Primer-Paaren.

10. Kit nach Anspruch 9, ferner umfassend ein oder mehrere Eichpolynukleotide.

11. Kit nach Anspruch 9, ferner umfassend wenigstens eine mit einem Magnetkügelchen verknüpfte funktionelle Anionenaustauschgruppe.

12. Verfahren zur Identifizierung eines *Staphylococcus* aureus-Stamms in einer Probe, wobei man Nukleinsäure aus dem *Staphylococcus* aureus-Stamm unter Verwendung der Zusammensetzung nach Anspruch 1 amplifiziert, so dass ein Amplifikationsprodukt erhalten wird;
die Molekülmasse des Amplifikationsprodukts bestimmt;
gegebenenfalls die Basenzusammensetzung des Amplifikationsprodukts anhand der Molekülmasse bestimmt; und
die Molekülmasse bzw. die Basenzusammensetzung mit mehreren Molekülmassen bzw. Basenzusammensetzungen bekannter Amplifikationsprodukte aus *Staphylococcus aureus*-Stämmen, wie durch die Zusammensetzung nach Anspruch 1 definiert, vergleicht, wobei eine Übereinstimmung zwischen der Molekülmasse bzw. der Basenzusammensetzung und einem Mitglied der mehreren Molekülmassen bzw. Basenzusammensetzungen den *Staphylococcus aureus*-Stamm identifiziert.

13. Verfahren nach Anspruch 12, bei dem man ferner den Amplifizierungs-, Bestimmungs-, gegebenenfalls durchgeführten Bestimmungs- und Vergleichsschritt unter Verwendung wenigstens eines zusätzlichen Primer-Paars aus der durch die SEQ ID NO: 288:1269, 698:1420, 217:1167, 399:1041, 430:1321, 174:853 und 172:1360 repräsentierten Gruppe von Primer-Paaren wiederholt.

14. Verfahren zur Bestimmung der Menge eines *Staphylococcus aureus*-Stamms in einer Probe, wobei man
die Probe mit der Zusammensetzung nach Anspruch 1 und einer bekannten Menge eines eine Eichsequenz umfassenden Eichpolynukleotids in Kontakt bringt; gleichzeitig Nukleinsäure aus dem *Staphylococcus aureus*-Stamm und Nukleinsäure aus dem Eichpolynukleotid aus der Probe mit der Zusammensetzung nach Anspruch 1 amplifiziert, so dass ein ein bakterielles Bioagens identifizierendes Amplifikat umfassendes erstes Amplifikationsprodukt sowie ein ein Eichamplifikat umfassendes zweites Amplifikationsprodukt erhalten werden;
die Molekülmasse und Häufigkeit für das bakterielles Bioagens identifizierende Amplifikat und das Eichamplifikat bestimmt;
und
das bakterielles Bioagens identifizierende Amplifikat von dem Eichamplifikat bezogen auf Molekülmasse unterscheidet, wobei der Vergleich der Häufigkeit von bakterielles Bioagens identifizierendem Amplifikat und der Häufigkeit von Eichamplifikat die Menge des *Staphylococcus* aureus-Stamms in der Probe anzeigt.

15. Verfahren nach Anspruch 12 oder 14, wobei es sich bei dem *Staphylococcus aureus*-Stamm um einen virulenten Stamm handelt.

## Revendications

1. Composition comprenant :
(a) une amorce oligonucléotidique ayant SEQ ID NO: 456 ; et
(b) une amorce oligonucléotidique ayant SEQ ID NO: 1261.

2. Composition de la revendication 1 dans laquelle l'une ou les deux desdites amorces comprend/comprennent au moins une nucléobase modifiée.

3. Composition de la revendication 2 dans laquelle ladite nucléobase modifiée est le 5-propynyluracile ou la 5-propynylcytosine.

4. Composition de la revendication 1 dans laquelle l'une ou les deux desdites amorces comprend/comprennent au moins une nucléobase universelle.

5. Composition de la revendication 4 dans laquelle ladite nucléobase universelle est l'inosine.

6. Composition de la revendication 1 dans laquelle l'une ou les deux desdites amorces comprend/comprennent en outre un résidu T non-matriciel sur l'extrémité 5'.

7. Composition de la revendication 1 dans laquelle l'une ou les deux desdites amorces comprend/comprennent au moins une étiquette non-matrice.

8. Composition de la revendication 1 dans laquelle l'une ou les deux desdites amorces comprend/comprennent au moins une étiquette de modification de masse moléculaire.

9. Kit comprenant la composition de la revendication 1, et comprenant en outre une ou plusieurs paires d'amorces du groupe de paires d'amorces représentées par SEQ ID NO: 288:1269, 698:1420, 217:1167, 399:1041, 430:1321, 174:853, et 172:1360.

10. Kit de la revendication 9 comprenant en outre un ou plusieurs polynucléotides étalons.

11. Kit de la revendication 9 comprenant en outre au moins un groupe fonctionnel d'échange d'anion lié à une bille magnétique.

12. Procédé pour l'identification d'une souche de *Staphylococcus aureus* dans un échantillon comprenant :
l'amplification d'acide nucléique de ladite souche de *Staphylococcus aureus* en utilisant la composition de la revendication 1 pour obtenir un produit d'amplification ;
la détermination de la masse moléculaire dudit produit d'amplification ;
facultativement, la détermination de la composition de bases dudit produit d'amplification à partir de ladite masse moléculaire ; et
la comparaison de ladite masse moléculaire ou de ladite composition de bases avec une pluralité de masses moléculaires ou de compositions de bases de produits d'amplification connus de souches de *Staphylococcus aureus* définis par la composition de la revendication 1, une correspondance entre ladite masse moléculaire ou la composition de bases et un membre de ladite pluralité de masses moléculaires ou/de compositions de bases identifie ladite souche de *Staphylococcus aureus.*

13. Procédé de la revendication 12 comprenant en outre des étapes de répétition de ladite amplification, détermination, facultativement détermination, et comparaison des étapes en utilisant au moins une paire d'amorces additionnelles dans le groupe de paires d'amorces représentées par SEQ ID NO: 288:1269, 698:1420, 217:1167, 399:1041, 430:1321, 174:853, et 172:1360.

14. Procédé pour la détermination de la quantité d'une souche de *Staphylococcus aureus* dans un échantillon comprenant :
la mise en contact dudit échantillon avec la composition de la revendication 1 et une quantité connue d'un polynucléotide étalon comprenant une séquence d'étalonnage ;
simultanément, l'amplification d'acide nucléique de ladite souche de *Staphylococcus aureus* et d'acide nucléique dudit polynucléotide étalon dans ledit échantillon avec la composition de la revendication 1 pour obtenir un premier produit d'amplification comprenant un amplicon d'identification de bioagent bactérien et un deuxième produit d'amplification comprenant un amplicon d'étalonnage ;
la détermination de la masse moléculaire et de l'abondance pour ledit amplicon d'identification de bioagent bactérien et ledit amplicon d'étalonnage ; et
la distinction dudit amplicon d'identification de bioagent bactérien sur la base de la masse moléculaire,
la comparaison de l'abondance d'amplicon d'identification de bioagent bactérien et de l'abondance d'amplicon d'étalonnage indiquant la quantité de ladite souche de *Staphylococcus aureus* dans ledit échantillon.

15. Procédé de la revendication 12 ou 14 dans lequel ladite souche de *Staphylococcus aureus* est une souche virulente.


### SEQUENCE LISTING

<110> Sampath, Rangarajan Hall, Thomas A. Eshoo, Mark W.
<120> COMPOSITIONS FOR IDENTIFICATION OF BACTERIA
<130> DIBIS-0083WO
<150> 60/674,118
   <151> 2005-04-21
<150> 60/705,631
   <151> 2005-08-03
<150> 60/732,539
   <151> 2005-11-01
<150> 60/773,124
   <151> 2006-02-13
<160> 1464
<170> FastSEQ for Windows Version 4.0
<210> 1
   <211> 24
   <212> DNA
   <213> Artificial Sequence
<220>
   <223> Primer
<400> 1
   aaactagata acagtagaca tcac 24
<210> 2
   <211> 29
   <212> DNA
   <213> Artificial Sequence
<220>
   <223> Primer
<400> 2
   aaccttaatt ggaaagaaac ccaagaagt 29
<210> 3
   <211> 17
   <212> DNA
   <213> Artificial Sequence
<220>
   <223> Primer
<400> 3
   aacgcacaat cagaagc 17

<210> 4
   <211> 25
   <212> DNA
   <213> Artificial Sequence
<220>
   <223> Primer
<400> 4
   aactaccgtc cgcagttcta cttcc 25
<210> 5
   <211> 25
   <212> DNA
   <213> Artificial Sequence
<220>
   <223> Primer
<400> 5
   aactaccgtc ctcagttcta cttcc 25
<210> 6
   <211> 18
   <212> DNA
   <213> Artificial Sequence
<220>
   <223> Primer
<400> 6
   aagacgacct gcacgggc 18
<210> 7
   <211> 18
   <212> DNA
   <213> Artificial Sequence
<220>
   <223> Primer
<400> 7
   aagcggtgga gcatgtgg 18
<210> 8
   <211> 26
   <212> DNA
   <213> Artificial Sequence
<220>
   <223> Primer
<400> 8
   aaggaaggcg tgatcaccgt tgaaga 26
<210> 9
   <211> 23
   <212> DNA
   <213> Artificial Sequence
<220>
   <223> Primer

<400> 9
   aaggtactcc ggggataaca ggc 23
<210> 10
   <211> 22
   <212> DNA
   <213> Artificial Sequence
<220>
   <223> Primer
<400> 10
   aagtcggaat cgctagtaat cg 22
<210> 11
   <211> 19
   <212> DNA
   <213> Artificial Sequence
<220>
   <223> Primer
<400> 11
   aatctgctat ttggtcagg 19
<210> 12
   <211> 22
   <212> DNA
   <213> Artificial Sequence
<220>
   <223> Primer
<400> 12
   acaacgaagt acaatacaag ac 22
<210> 13
   <211> 21
   <212> DNA
   <213> Artificial Sequence
<220>
   <223> Primer
<400> 13
   acaatacaag acaaaagaag g 21
<210> 14
   <211> 19
   <212> DNA
   <213> Artificial Sequence
<220>
   <223> Primer
<400> 14
   accacgccgt aaacgatga 19
<210> 15
   <211> 24
   <212> DNA
   <213> Artificial Sequence
<220>
   <223> Primer

<400> 15
   accatgacag aaggcatttt gaca 24
<210> 16
   <211> 22
   <212> DNA
   <213> Artificial Sequence
<220>
   <223> Primer
<400> 16
   acccagtgct gctgaaccgt gc 22
<210> 17
   <211> 19
   <212> DNA
   <213> Artificial Sequence
<220>
   <223> Primer
<400> 17
   accgagcaag gagaccagc 19
<210> 18
   <211> 19
   <212> DNA
   <213> Artificial Sequence
<220>
   <223> Primer
<400> 18
   acctgcccag tgctggaag 19
<210> 19
   <211> 17
   <212> DNA
   <213> Artificial Sequence
<220>
   <223> Primer
<400> 19
   acgcgaagaa ccttacc 17
<210> 20
   <211> 20
   <212> DNA
   <213> Artificial Sequence
<220>
   <223> Primer
<400> 20
   actcgttttt aatcagcccg 20
<210> 21
   <211> 20
   <212> DNA
   <213> Artificial Sequence
<220>
   <223> Primer

<400> 21
   agaacaccga tggcgaaggc 20
<210> 22
   <211> 23
   <212> DNA
   <213> Artificial Sequence
<220>
   <223> Primer
<400> 22
   agaatcaagt tcccaggggt tac 23
<210> 23
   <211> 20
   <212> DNA
   <213> Artificial Sequence
<220>
   <223> Primer
<400> 23
   agagtttgat catggctcag 20
<210> 24
   <211> 28
   <212> DNA
   <213> Artificial Sequence
<220>
   <223> Primer
<400> 24
   agcaggtggt gaaatcggcc acatgatt 28
<210> 25
   <211> 18
   <212> DNA
   <213> Artificial Sequence
<220>
   <223> Primer
<400> 25
   agcgtaaagg tgaacctt 18
<210> 26
   <211> 26
   <212> DNA
   <213> Artificial Sequence
<220>
   <223> Primer

<400> 26
   agcttttgca tattatatcg agccac 26
<210> 27
   <211> 28
   <212> DNA
   <213> Artificial Sequence
<220>
   <223> Primer
<400> 27
   aggacagagt gagtactttg accgaggt 28
<210> 28
   <211> 22
   <212> DNA
   <213> Artificial Sequence
<220>
   <223> Primer
<400> 28
   agtctcaaga gtgaacacgt aa 22
<210> 29
   <211> 32
   <212> DNA
   <213> Artificial Sequence
<220>
   <223> Primer
<400> 29
   agttataaac acggctttcc tatggcttat cc 32
<210> 30
   <211> 21
   <212> DNA
   <213> Artificial Sequence
<220>
   <223> Primer
<400> 30
   atactcctga ctgaccgata g 21
<210> 31
   <211> 20
   <212> DNA
   <213> Artificial Sequence
<220>
   <223> Primer
<400> 31
   atatcgacgg cggtgtttgg 20
<210> 32
   <211> 25
   <212> DNA
   <213> Artificial Sequence
<220>
   <223> Primer

<400> 32
   atcaatttgg tggccaagaa cctgg 25
<210> 33
   <211> 29
   <212> DNA
   <213> Artificial Sequence
<220>
   <223> Primer
<400> 33
   atgattacaa ttcaagaagg tcgtcacgc 29
<210> 34
   <211> 23
   <212> DNA
   <213> Artificial Sequence
<220>
   <223> Primer
<400> 34
   atggacaagg ttggcaagga agg 23
<210> 35
   <211> 20
   <212> DNA
   <213> Artificial Sequence
<220>
   <223> Primer
<400> 35
   atggccatgg cagaagctca 20
<210> 36
   <211> 25
   <212> DNA
   <213> Artificial Sequence
<220>
   <223> Primer
<400> 36
   atgtcgattg caatccgtac ttgtg 25
<210> 37
   <211> 19
   <212> DNA
   <213> Artificial Sequence
<220>
   <223> Primer
<400> 37
   atgttgggtt aagtcccgc 19
<210> 38
   <211> 25
   <212> DNA
   <213> Artificial Sequence
<220>
   <223> Primer

<400> 38
   atgttgggtt aagtcccgca acgag 25
<210> 39
   <211> 28
   <212> DNA
   <213> Artificial Sequence
<220>
   <223> Primer
<400> 39
   caaaacttat taggtaagcg tgttgact 28
<210> 40
   <211> 27
   <212> DNA
   <213> Artificial Sequence
<220>
   <223> Primer
<400> 40
   caaaggtaag caaggacgtt tccgtca 27
<210> 41
   <211> 27
   <212> DNA
   <213> Artificial Sequence
<220>
   <223> Primer
<400> 41
   caaaggtaag caaggtcgtt tccgtca 27
<210> 42
   <211> 17
   <212> DNA
   <213> Artificial Sequence
<220>
   <223> Primer
<400> 42
   caacgagcgc aaccctt 17
<210> 43
   <211> 17
   <212> DNA
   <213> Artificial Sequence
<220>
   <223> Primer

<400> 43
   caacggatgc tggcaag 17
<210> 44
   <211> 31
   <212> DNA
   <213> Artificial Sequence
<220>
   <223> Primer
<400> 44
   caagaagaaa aagagcttct aaaaagaata c 31
<210> 45
   <211> 23
   <212> DNA
   <213> Artificial Sequence
<220>
   <223> Primer
<400> 45
   caagcaaacg cacaatcaga agc 23
<210> 46
   <211> 19
   <212> DNA
   <213> Artificial Sequence
<220>
   <223> Primer
<400> 46
   caagtcatca tggccctta 19
<210> 47
   <211> 26
   <212> DNA
   <213> Artificial Sequence
<220>
   <223> Primer
<400> 47
   caataccgca acagcggtgg cttggg 26
<210> 48
   <211> 19
   <212> DNA
   <213> Artificial Sequence
<220>
   <223> Primer
<400> 48
   cactggaact gagacacgg 19
<210> 49
   <211> 20
   <212> DNA
   <213> Artificial Sequence
<220>
   <223> Primer

<400> 49
   cagaatcaag ttcccagggg 20
<210> 50
   <211> 24
   <212> DNA
   <213> Artificial Sequence
<220>
   <223> Primer
<400> 50
   cagagaccgt tttatcctat cagc 24
<210> 51
   <211> 20
   <212> DNA
   <213> Artificial Sequence
<220>
   <223> Primer
<400> 51
   cagcgtttcg gcgaaatgga 20
<210> 52
   <211> 29
   <212> DNA
   <213> Artificial Sequence
<220>
   <223> Primer
<400> 52
   caggagtcgt tcaactcgat ctacatgat 29
<210> 53
   <211> 24
   <212> DNA
   <213> Artificial Sequence
<220>
   <223> Primer
<400> 53 24
   caggtttagt accagaacat gcag 24
<210> 54
   <211> 23
   <212> DNA
   <213> Artificial Sequence
<220>
   <223> Primer

<400> 54
   catccacacg gtggtggtga agg 23
<210> 55
   <211> 23
   <212> DNA
   <213> Artificial Sequence
<220>
   <223> Primer
<400> 55
   ccacacgccg ttcttcaaca act 23
<210> 56
   <211> 28
   <212> DNA
   <213> Artificial Sequence
<220>
   <223> Primer
<400> 56
   ccacagttct acttccgtac tactgacg 28
<210> 57
   <211> 19
   <212> DNA
   <213> Artificial Sequence
<220>
   <223> Primer
<400> 57
   ccagcagccg cggtaatac 19
<210> 58
   <211> 19
   <212> DNA
   <213> Artificial Sequence
<220>
   <223> Primer
<400> 58
   ccgtaacttc gggagaagg 19
<210> 59
   <211> 20
   <212> DNA
   <213> Artificial Sequence
<220>
   <223> Primer

<400> 59
   ccgtggtatt ggagttattg 20
<210> 60
   <211> 29
   <212> DNA
   <213> Artificial Sequence
<220>
   <223> Primer
<400> 60
   cctatattaa tcgtttacag aaactggct 29
<210> 61
   <211> 19
   <212> DNA
   <213> Artificial Sequence
<220>
   <223> Primer
<400> 61
   cctgataagg gtgaggtcg 19
<210> 62
   <211> 30
   <212> DNA
   <213> Artificial Sequence
<220>
   <223> Primer
<400> 62
   ccttacttcg aactatgaat cttttggaag 30
<210> 63
   <211> 14
   <212> DNA
   <213> Artificial Sequence
<220>
   <223> Primer
<400> 63
   cgaagaacct tacc 14
<210> 64
   <211> 27
   <212> DNA
   <213> Artificial Sequence
<220>
   <223> Primer
<400> 64
   cgaagtacaa tacaagacaa aagaagg 27
<210> 65
   <211> 18
   <212> DNA
   <213> Artificial Sequence
<220>
   <223> Primer

<400> 65
   cgacgcgctg cgcttcac 18
<210> 66
   <211> 22
   <212> DNA
   <213> Artificial Sequence
<220>
   <223> Primer
<400> 66
   cgagagggaa acaacccaga cc 22
<210> 67
   <211> 30
   <212> DNA
   <213> Artificial Sequence
<220>
   <223> Primer
<400> 67
   cgagtatagc taaaaaaata gtttatgaca 30
<210> 68
   <211> 23
   <212> DNA
   <213> Artificial Sequence
<220>
   <223> Primer
<400> 68
   cgcaaaaaaa tccagctatt agc 23
<210> 69
   <211> 20
   <212> DNA
   <213> Artificial Sequence
<220>
   <223> Primer
<400> 69
   cgccgacttc gacggtgacc 20
<210> 70
   <211> 20
   <212> DNA
   <213> Artificial Sequence
<220>
   <223> Primer
<400> 70
   cggaattact gggcgtaaag 20
<210> 71
   <211> 21
   <212> DNA
   <213> Artificial Sequence
<220>
   <223> Primer

<400> 71
   cggattggag tctgcaactc g 21
<210> 72
   <211> 22
   <212> DNA
   <213> Artificial Sequence
<220>
   <223> Primer
<400> 72
   cggcgtactt caacgacagc ca 22
<210> 73
   <211> 24
   <212> DNA
   <213> Artificial Sequence
<220>
   <223> Primer
<400> 73
   cgtaactata acggtcctaa ggta 24
<210> 74
   <211> 26
   <212> DNA
   <213> Artificial Sequence
<220>
   <223> Primer
<400> 74
   cgtcagggta aattccgtga agttaa 26
<210> 75
   <211> 26
   <212> DNA
   <213> Artificial Sequence
<220>
   <223> Primer
<400> 75
   cgtcgggtga ttaaccgtaa caaccg 26
<210> 76
   <211> 26
   <212> DNA
   <213> Artificial Sequence
<220>
   <223> Primer
<400> 76
   cgtcgtgtaa ttaaccgtaa caaccg 26
<210> 77
   <211> 20
   <212> DNA
   <213> Artificial Sequence
<220>
   <223> Primer

<400> 77
   cgtggcggcg tggttatcga 20
<210> 78
   <211> 25
   <212> DNA
   <213> Artificial Sequence
<220>
   <223> Primer
<400> 78
   cgtgttgact attcggggcg ttcag 25
<210> 79
   <211> 18
   <212> DNA
   <213> Artificial Sequence
<220>
   <223> Primer
<400> 79
   ctagtacgag aggaccgg 18
<210> 80
   <211> 18
   <212> DNA
   <213> Artificial Sequence
<220>
   <223> Primer
<400> 80
   ctgacacctg cccggtgc 18
<210> 81
   <211> 24
   <212> DNA
   <213> Artificial Sequence
<220>
   <223> Primer
<400> 81
   ctggcaggta tgcgtggtct gatg 24
<210> 82
   <211> 23
   <212> DNA
   <213> Artificial Sequence
<220>
   <223> Primer
<400> 82
   ctggctaaaa ctttggcaac ggt 23
<210> 83
   <211> 24
   <212> DNA
   <213> Artificial Sequence
<220>
   <223> Primer
<400> 83
   ctgtccctag tacgagagga ccgg 24
<210> 84
   <211> 22
   <212> DNA
   <213> Artificial Sequence
<220>
   <223> Primer
<400> 84
   ctgttcttag tacgagagga cc 22
<210> 85
   <211> 24
   <212> DNA
   <213> Artificial Sequence
<220>
   <223> Primer

<400> 85
   cttctgcaac aagctgtgga acgc 24
<210> 86
   <211> 24
   <212> DNA
   <213> Artificial Sequence
<220>
   <223> Primer
<400> 86
   cttgctggta tgcgtggtct gatg 24
<210> 87
   <211> 29
   <212> DNA
   <213> Artificial Sequence
<220>
   <223> Primer
<400> 87
   cttggaggta agtctcattt tggtgggca 29
<210> 88
   <211> 19
   <212> DNA
   <213> Artificial Sequence
<220>
   <223> Primer
<400> 88
   cttgtacaca ccgcccgtc 19
<210> 89
   <211> 30
   <212> DNA
   <213> Artificial Sequence
<220>
   <223> Primer
<400> 89
   cttgtacttg tggctcacac ggctgtttgg 30
<210> 90
   <211> 21
   <212> DNA
   <213> Artificial Sequence
<220>
   <223> Primer
<400> 90
   cttttgcata ttatatcgag c 21
<210> 91
   <211> 20
   <212> DNA
   <213> Artificial Sequence
<220>
   <223> Primer
<400> 91
   gaatagcaat taatccaaat 20
<210> 92
   <211> 18
   <212> DNA
   <213> Artificial Sequence
<220>
   <223> Primer
<400> 92
   gaaagagttc ggattggg 18
<210> 93
   <211> 21
   <212> DNA
   <213> Artificial Sequence
<220>
   <223> Primer

<400> 93
   gaaggatata cggttgatgt c 21
<210> 94
   <211> 20
   <212> DNA
   <213> Artificial Sequence
<220>
   <223> Primer
<400> 94
   gaatagcaat taatccaaat 20
<210> 95
   <211> 20
   <212> DNA
   <213> Artificial Sequence
<220>
   <223> Primer
<400> 95
   gacacggtcc agactcctac 20
<210> 96
   <211> 18
   <212> DNA
   <213> Artificial Sequence
<220>
   <223> Primer
<400> 96
   gacagttcgg tccctatc 18
<210> 97
   <211> 18
   <212> DNA
   <213> Artificial Sequence
<220>
   <223> Primer
<400> 97
   gaccacctcg gcaaccgt 18
<210> 98
   <211> 30
   <212> DNA
   <213> Artificial Sequence
<220>
   <223> Primer

<400> 98
   gacctacagt aagaggttct gtaatgaacc 30
<210> 99
   <211> 16
   <212> DNA
   <213> Artificial Sequence
<220>
   <223> Primer
<400> 99
   gacgcctgcc cggtgc 16
<210> 100
   <211> 20
   <212> DNA
   <213> Artificial Sequence
<220>
   <223> Primer
<400> 100
   gacttaccaa cccgatgcaa 20
<210> 101
   <211> 20
   <212> DNA
   <213> Artificial Sequence
<220>
   <223> Primer
<400> 101
   gagagcaagc ggacctcata 20
<210> 102
   <211> 27
   <212> DNA
   <213> Artificial Sequence
<220>
   <223> Primer
<400> 102
   gagagtttga tcctggctca gaacgaa 27
<210> 103
   <211> 19
   <212> DNA
   <213> Artificial Sequence
<220>
   <223> Primer
<400> 103
   gaggaaagtc catgctcac 19
<210> 104
   <211> 19
   <212> DNA
   <213> Artificial Sequence
<220>
   <223> Primer

<400> 104
   gaggaaagtc catgctcgc 19
<210> 105
   <211> 17
   <212> DNA
   <213> Artificial Sequence
<220>
   <223> Primer
<400> 105
   gaggaaagtc cgggctc 17
<210> 106
   <211> 22
   <212> DNA
   <213> Artificial Sequence
<220>
   <223> Primer
<400> 106
   gataccctgg tagtccacac cg 22
<210> 107
   <211> 20
   <212> DNA
   <213> Artificial Sequence
<220>
   <223> Primer
<400> 107
   gatctggagg aataccggtg 20
<210> 108
   <211> 29
   <212> DNA
   <213> Artificial Sequence
<220>
   <223> Primer
<400> 108
   gatgactttt tagctaatgg tcaggcagc 29
<210> 109
   <211> 30
   <212> DNA
   <213> Artificial Sequence
<220>
   <223> Primer
<400> 109
   gattattgtt atcctgttat gccatttgag 30
<210> 110
   <211> 18
   <212> DNA
   <213> Artificial Sequence
<220>
   <223> Primer

<400> 110
   gcacaacctg cggctgcg 18
<210> 111
   <211> 27
   <212> DNA
   <213> Artificial Sequence
<220>
   <223> Primer
<400> 111
   gcactatgca cacgtagatt gtcctgg 27
<210> 112
   <211> 21
   <212> DNA
   <213> Artificial Sequence
<220>
   <223> Primer
<400> 112
   gccttgtaca cacctcccgt c 21
<210> 113
   <211> 20
   <212> DNA
   <213> Artificial Sequence
<220>
   <223> Primer
<400> 113
   gcgaagaacc ttaccaggtc 20
<210> 114
   <211> 20
   <212> DNA
   <213> Artificial Sequence
<220>
   <223> Primer
<400> 114
   gctacacacg tgctacaatg 20
<210> 115
   <211> 30
   <212> DNA
   <213> Artificial Sequence
<220>
   <223> Primer
<400> 115
   gctggtgaaa ataacccaga tgtcgtcttc 30
<210> 116
   <211> 25
   <212> DNA
   <213> Artificial Sequence
<220>
   <223> Primer
<400> 116
   gcttcaggaa tcaatgatgg agcag 25
<210> 117
   <211> 19
   <212> DNA
   <213> Artificial Sequence
<220>
   <223> Primer

<400> 117
   ggacggagaa ggctatgtt 19
<210> 118
   <211> 22
   <212> DNA
   <213> Artificial Sequence
<220>
   <223> Primer
<400> 118
   ggattagaga ccctggtagt cc 22
<210> 119
   <211> 26
   <212> DNA
   <213> Artificial Sequence
<220>
   <223> Primer
<400> 119
   ggattagata ccctggtagt ccacgc 26
<210> 120
   <211> 25
   <212> DNA
   <213> Artificial Sequence
<220>
   <223> Primer
<400> 120
   ggctcagcca tttagttacc gctat 25
<210> 121
   <211> 21
   <212> DNA
   <213> Artificial Sequence
<220>
   <223> Primer
<400> 121
   gggaactgaa acatctaagt a 21
<210> 122
   <211> 22
   <212> DNA
   <213> Artificial Sequence
<220>
   <223> Primer
<400> 122
   gggagcaaac aggattagat ac 22
<210> 123
   <211> 29
   <212> DNA
   <213> Artificial Sequence
<220>
   <223> Primer
<400> 123
   gggcaacagc agcggattgc gattgcgcg 29
<210> 124
   <211> 23
   <212> DNA
   <213> Artificial Sequence
<220>
   <223> Primer

<400> 124
   gggcagcgtt tcggcgaaat gga 23
<210> 125
   <211> 26
   <212> DNA
   <213> Artificial Sequence
<220>
   <223> Primer
<400> 125
   ggggagtgaa agagatcctg aaaccg 26
<210> 126
   <211> 30
   <212> DNA
   <213> Artificial Sequence
<220>
   <223> Primer
<400> 126
   ggggattcag ccatcaaagc agctattgac 30
<210> 127
   <211> 27
   <212> DNA
   <213> Artificial Sequence
<220>
   <223> Primer
<400> 127
   ggggattgat atcaccgata agaagaa 27
<210> 128
   <211> 24
   <212> DNA
   <213> Artificial Sequence
<220>
   <223> Primer
<400> 128
   ggtgaaagaa gttgcctcta aagc 24
<210> 129
   <211> 15
   <212> DNA
   <213> Artificial Sequence
<220>
   <223> Primer
<400> 129
   ggtggatgcc ttggc 15
<210> 130
   <211> 20
   <212> DNA
   <213> Artificial Sequence
<220>
   <223> Primer
<400> 130
   ggtgttaaat agcctggcag 20
<210> 131
   <211> 20
   <212> DNA
   <213> Artificial Sequence
<220>
   <223> Primer
<400> 131
   ggtttagtac cagaacatgc 20
<210> 132
   <211> 23
   <212> DNA
   <213> Artificial Sequence
<220>
   <223> Primer

<400> 132
   gtcaaagtgg cacgtttact ggc 23
<210> 133
   <211> 22
   <212> DNA
   <213> Artificial Sequence
<220>
   <223> Primer
<400> 133
   gtcgtgaaaa cgagctggaa ga 22
<210> 134
   <211> 30
   <212> DNA
   <213> Artificial Sequence
<220>
   <223> Primer
<400> 134
   gtgagatgtt gggttaagtc ccgtaacgag 30
<210> 135
   <211> 23
   <212> DNA
   <213> Artificial Sequence
<220>
   <223> Primer
<400> 135
   gtgcatgcgg atacagagca gag 23
<210> 136
   <211> 26
   <212> DNA
   <213> Artificial Sequence
<220>
   <223> Primer
<400> 136
   gtggcatgcc taatacatgc aagtcg 26
<210> 137
   <211> 18
   <212> DNA
   <213> Artificial Sequence
<220>
   <223> Primer
<400> 137
   gtgtagcggt gaaatgcg 18
<210> 138
   <211> 21
   <212> DNA
   <213> Artificial Sequence
<220>
   <223> Primer
<400> 138
   gttatcctgt tatgccattt g 21
<210> 139
   <211> 28
   <212> DNA
   <213> Artificial Sequence
<220>
   <223> Primer
<400> 139
   gttatttagc actcgttttt aatcagcc 28
<210> 140
   <211> 22
   <212> DNA
   <213> Artificial Sequence
<220>
   <223> Primer
<400> 140
   gttgtgaggt taagcgacta ag 22
<210> 141
   <211> 22
   <212> DNA
   <213> Artificial Sequence
<220>
   <223> Primer

<400> 141
   gttgtgaggt taagcgacta ag 22
<210> 142
   <211> 24
   <212> DNA
   <213> Artificial Sequence
<220>
   <223> Primer
<400> 142
   tctagtaata ataggaccct cagc 24
<210> 143
   <211> 15
   <212> DNA
   <213> Artificial Sequence
<220>
   <223> Primer
<400> 143
   tatggctcta ctcaa 15
<210> 144
   <211> 29
   <212> DNA
   <213> Artificial Sequence
<220>
   <223> Primer
<400> 144
   taaaacaaac tacggtaaca ttgatcgca 29
<210> 145
   <211> 33
   <212> DNA
   <213> Artificial Sequence
<220>
   <223> Primer
<400> 145
   taaaactttt gccgtaatga tgggtgaaga tat 33
<210> 146
   <211> 31
   <212> DNA
   <213> Artificial Sequence
<220>
   <223> Primer
<400> 146
   taaacacggc tttcctatgg cttatccaaa t 31
<210> 147
   <211> 28
   <212> DNA
   <213> Artificial Sequence
<220>
   <223> Primer

<400> 147
   taaaccccat cgggagcaag accgaata 28
<210> 148
   <211> 30
   <212> DNA
   <213> Artificial Sequence
<220>
   <223> Primer
<400> 148
   taaagcccgt gaaatgactc gtcgtaaagg 30
<210> 149
   <211> 28
   <212> DNA
   <213> Artificial Sequence
<220>
   <223> Primer
<400> 149
   taaagttggt tttattggtt ggcgcgga 28
<210> 150
   <211> 25
   <212> DNA
   <213> Artificial Sequence
<220>
   <223> Primer
<400> 150
   taaatctgcc cgtgtcgttg gtgac 25
<210> 151
   <211> 29
   <212> DNA
   <213> Artificial Sequence
<220>
   <223> Primer
<400> 151
   taacaactcg ccttatgaaa cgggatata 29
<210> 152
   <211> 20
   <212> DNA
   <213> Artificial Sequence
<220>
   <223> Primer
<400> 152
   taacacatgc aagtcgaacg 20
<210> 153
   <211> 29
   <212> DNA
   <213> Artificial Sequence
<220>
   <223> Primer
<400> 153
   taaccattca agaactagat cttcaggca 29
<210> 154
   <211> 30
   <212> DNA
   <213> Artificial Sequence
<220>
   <223> Primer

<400> 154
   taaccttaat tggaaagaaa cccaagaagt 30
<210> 155
   <211> 25
   <212> DNA
   <213> Artificial Sequence
<220>
   <223> Primer
<400> 155
   taacggttat catggcccag atggg 25
<210> 156
   <211> 27
   <212> DNA
   <213> Artificial Sequence
<220>
   <223> Primer
<400> 156
   taactctgat gtttttgatg ggaaggt 27
<210> 157
   <211> 27
   <212> DNA
   <213> Artificial Sequence
<220>
   <223> Primer
<400> 157
   taactgcatg gaacccttct ttactag 27
<210> 158
   <211> 27
   <212> DNA
   <213> Artificial Sequence
<220>
   <223> Primer
<400> 158
   taagaagccg gaaaccatca actaccg 27
<210> 159
   <211> 22
   <212> DNA
   <213> Artificial Sequence
<220>
   <223> Primer
<400> 159
   taagagcgca ccggtaagtt gg 22
<210> 160
   <211> 28
   <212> DNA
   <213> Artificial Sequence
<220>
   <223> Primer
<400> 160
   taagcatgct gtggcttatc gtgaaatg 28
<210> 161
   <211> 23
   <212> DNA
   <213> Artificial Sequence
<220>
   <223> Primer

<400> 161
   taagctgcca gcggaatgct ttc 23
<210> 162
   <211> 29
   <212> DNA
   <213> Artificial Sequence
<220>
   <223> Primer
<400> 162
   taaggatagt gcaacagaga tataccgcc 29
<210> 163
   <211> 30
   <212> DNA
   <213> Artificial Sequence
<220>
   <223> Primer
<400> 163
   taaggtatga caccggataa atcatataaa 30
<210> 164
   <211> 33
   <212> DNA
   <213> Artificial Sequence
<220>
   <223> Primer
<400> 164
   taaggtttat tgtctttgtg gagatgggga ttt 33
<210> 165
   <211> 31
   <212> DNA
   <213> Artificial Sequence
<220>
   <223> Primer
<400> 165
   taatcaagca ttggaagatg aaatgcatac c 31
<210> 166
   <211> 29
   <212> DNA
   <213> Artificial Sequence
<220>
   <223> Primer
<400> 166
   taatcggtaa atatcacccg catggtgac 29
<210> 167
   <211> 29
   <212> DNA
   <213> Artificial Sequence
<220>
   <223> Primer
<400> 167
   taatcggtaa gtatcaccct catggtgat 29
<210> 168
   <211> 24
   <212> DNA
   <213> Artificial Sequence
<220>
   <223> Primer

<400> 168
   taatcgtgga atacgggttt gcta 24
<210> 169
   <211> 30
   <212> DNA
   <213> Artificial Sequence
<220>
   <223> Primer
<400> 169
   taatgaaccc taatgaccat ccacacggtg 30
<210> 170
   <211> 27
   <212> DNA
   <213> Artificial Sequence
<220>
   <223> Primer
<400> 170
   taatgatgaa ttaggtgcgg gttcttt 27
<210> 171
   <211> 29
   <212> DNA
   <213> Artificial Sequence
<220>
   <223> Primer
<400> 171
   taatgggtaa atatcaccct catggtgac 29
<210> 172
   <211> 31
   <212> DNA
   <213> Artificial Sequence
<220>
   <223> Primer
<400> 172
   taattgggct ctttctcgct taaacacctt a 31
<210> 173
   <211> 25
   <212> DNA
   <213> Artificial Sequence
<220>
   <223> Primer
<400> 173
   tacaaagcaa gacactggct cacta 25
<210> 174
   <211> 30
   <212> DNA
   <213> Artificial Sequence
<220>
   <223> Primer
<400> 174
   tacaaaggtc aaccaatgac attcagacta 30
<210> 175
   <211> 34
   <212> DNA
   <213> Artificial Sequence
<220>
   <223> Primer
<400> 175
   tacaacatat tattaaagag acgggtttga atcc 34
<210> 176
   <211> 20
   <212> DNA
   <213> Artificial Sequence
<220>
   <223> Primer

<400> 176
   tacaagcact cccagctgca 20
<210> 177
   <211> 29
   <212> DNA
   <213> Artificial Sequence
<220>
   <223> Primer
<400> 177
   tacaatgctt gtttatgctg gtaaagcag 29
<210> 178
   <211> 25
   <212> DNA
   <213> Artificial Sequence
<220>
   <223> Primer
<400> 178
   tacacaacaa tggcggtaaa gatgg 25
<210> 179
   <211> 15
   <212> DNA
   <213> Artificial Sequence
<220>
   <223> Primer
<400> 179
   tacagagttt gcgac 15
<210> 180
   <211> 21
   <212> DNA
   <213> Artificial Sequence
<220>
   <223> Primer
<400> 180
   tacaggccgt gttgaacgtg g 21
<210> 181
   <211> 21
   <212> DNA
   <213> Artificial Sequence
<220>
   <223> Primer
<400> 181
   tacatgctag ccgcgtctta c 21
<210> 182
   <211> 27
   <212> DNA
   <213> Artificial Sequence
<220>
   <223> Primer
<400> 182
   taccactatt aatgtcgctg gtgcttc 27
<210> 183
   <211> 25
   <212> DNA
   <213> Artificial Sequence
<220>
   <223> Primer
<400> 183
   taccatgaca gaaggcattt tgaca 25
<210> 184
   <211> 19
   <212> DNA
   <213> Artificial Sequence
<220>
   <223> Primer

<400> 184
   taccccaaac cgacacagg 19
<210> 185
   <211> 23
   <212> DNA
   <213> Artificial Sequence
<220>
   <223> Primer
<400> 185
   taccccaggg aaagtgccac aga 23
<210> 186
   <211> 25
   <212> DNA
   <213> Artificial Sequence
<220>
   <223> Primer
<400> 186
   taccggcgca aaaagtcgag attgg 25
<210> 187
   <211> 21
   <212> DNA
   <213> Artificial Sequence
<220>
   <223> Primer
<400> 187
   tacctatatg cgccagaccg c 21
<210> 188
   <211> 26
   <212> DNA
   <213> Artificial Sequence
<220>
   <223> Primer
<400> 188
   tacgatttca cttccgcagc cagatt 26
<210> 189
   <211> 27
   <212> DNA
   <213> Artificial Sequence
<220>
   <223> Primer
<400> 189
   tacgcgtctt gaagcgtttc gttatga 27
<210> 190
   <211> 26
   <212> DNA
   <213> Artificial Sequence
<220>
   <223> Primer
<400> 190
   tacgctgacg gaatcaacca aagcgg 26
<210> 191
   <211> 21
   <212> DNA
   <213> Artificial Sequence
<220>
   <223> Primer

<400> 191
   tacggtgaat acgttcccgg g 21
<210> 192
   <211> 15
   <212> DNA
   <213> Artificial Sequence
<220>
   <223> Primer
<400> 192
   tacagagttt gcgac 15
<210> 193
   <211> 23
   <212> DNA
   <213> Artificial Sequence
<220>
   <223> Primer
<400> 193
   tactacttca agccgaactt ccg 23
<210> 194
   <211> 28
   <212> DNA
   <213> Artificial Sequence
<220>
   <223> Primer
<400> 194
   tactagcggt aagcttaaac aagattgc 28
<210> 195
   <211> 22
   <212> DNA
   <213> Artificial Sequence
<220>
   <223> Primer
<400> 195
   tactctcggt ggagaagctc gc 22
<210> 196
   <211> 22
   <212> DNA
   <213> Artificial Sequence
<220>
   <223> Primer
<400> 196
   tactggaaca aagtctgcga cc 22
<210> 197
   <211> 27
   <212> DNA
   <213> Artificial Sequence
<220>
   <223> Primer
<400> 197
   tacttactac ttcaagccga acttccg 27
<210> 198
   <211> 28
   <212> DNA
   <213> Artificial Sequence
<220>
   <223> Primer
<400> 198
   tacttacttg agaatccaca agctgcaa 28
<210> 199
   <211> 28
   <212> DNA
   <213> Artificial Sequence
<220>
   <223> Primer

<400> 199
   tacttggtaa ataccaccca catggtga 28
<210> 200
   <211> 32
   <212> DNA
   <213> Artificial Sequence
<220>
   <223> Primer
<400> 200
   tactttttta aaactaggga tgcgtttgaa gc 32
<210> 201
   <211> 27
   <212> DNA
   <213> Artificial Sequence
<220>
   <223> Primer
<400> 201
   tagaaatcaa ggtgatagtg gcaatga 27
<210> 202
   <211> 21
   <212> DNA
   <213> Artificial Sequence
<220>
   <223> Primer
<400> 202
   tagaacaccg atggcgaagg c 21
<210> 203
   <211> 22
   <212> DNA
   <213> Artificial Sequence
<220>
   <223> Primer
<400> 203
   tagaacgtcg cgagacagtt cg 22
<210> 204
   <211> 21
   <212> DNA
   <213> Artificial Sequence
<220>
   <223> Primer
<400> 204
   tagactgccc aggacacgct g 21
<210> 205
   <211> 29
   <212> DNA
   <213> Artificial Sequence
<220>
   <223> Primer
<400> 205
   tagataattg ggctctttct cgcttaaac 29
<210> 206
   <211> 22
   <212> DNA
   <213> Artificial Sequence
<220>
   <223> Primer
<400> 206
   tagataccct ggtagtccac gc 22
<210> 207
   <211> 28
   <212> DNA
   <213> Artificial Sequence
<220>
   <223> Primer

<400> 207
   tagatgaaaa aggcgaagtg gctaatgg 28
<210> 208
   <211> 28
   <212> DNA
   <213> Artificial Sequence
<220>
   <223> Primer
<400> 208
   tagatgaaaa gggcgaagtg gctaatgg 28
<210> 209
   <211> 30
   <212> DNA
   <213> Artificial Sequence
<220>
   <223> Primer
<400> 209
   tagcaacaaa tatatctgaa gcagcgtact 30
<210> 210
   <211> 29
   <212> DNA
   <213> Artificial Sequence
<220>
   <223> Primer
<400> 210
   tagcaggtgg tgaaatcggc cacatgatt 29
<210> 211
   <211> 26
   <212> DNA
   <213> Artificial Sequence
<220>
   <223> Primer
<400> 211
   tagcatcaga actgttgttc cgctag 26
<210> 212
   <211> 24
   <212> DNA
   <213> Artificial Sequence
<220>
   <223> Primer
<400> 212
   tagcccagca caatttgtga ttca 24
<210> 213
   <211> 34
   <212> DNA
   <213> Artificial Sequence
<220>
   <223> Primer
<400> 213
   tagcctttaa cgaaaatgta aaaatgcgtt ttga 34
<210> 214
   <211> 27
   <212> DNA
   <213> Artificial Sequence
<220>
   <223> Primer

<400> 214
   tagcgaatgt ggctttactt cacaatt 27
<210> 215
   <211> 19
   <212> DNA
   <213> Artificial Sequence
<220>
   <223> Primer
<400> 215
   tagcgtaaag gtgaacctt 19
<210> 216
   <211> 20
   <212> DNA
   <213> Artificial Sequence
<220>
   <223> Primer
<400> 216
   tagctaatgg tcaggcagcc 20
<210> 217
   <211> 30
   <212> DNA
   <213> Artificial Sequence
<220>
   <223> Primer
<400> 217
   tagctatctt atcgttgaga agggatttgc 30
<210> 218
   <211> 22
   <212> DNA
   <213> Artificial Sequence
<220>
   <223> Primer
<400> 218
   tagctggcgc gaaattaggt gt 22
<210> 219
   <211> 26
   <212> DNA
   <213> Artificial Sequence
<220>
   <223> Primer

<400> 219
   tagctggcgg tatggagaat atgtct 26
<210> 220
   <211> 27
   <212> DNA
   <213> Artificial Sequence
<220>
   <223> Primer
<400> 220
   tagcttttgc atattatatc gagccac 27
<210> 221
   <211> 29
   <212> DNA
   <213> Artificial Sequence
<220>
   <223> Primer
<400> 221
   taggaattac ggctgataaa gcgtataaa 29
<210> 222
   <211> 35
   <212> DNA
   <213> Artificial Sequence
<220>
   <223> Primer
<400> 222
   taggcgaaga tatacaaaga gtattagaag ctaga 35
<210> 223
   <211> 25
   <212> DNA
   <213> Artificial Sequence
<220>
   <223> Primer
<400> 223
   taggcgtgaa agcaagctac cgttt 25
<210> 224
   <211> 25
   <212> DNA
   <213> Artificial Sequence
<220>
   <223> Primer
<400> 224
   taggtgctgg ttacgcagat caaga 25
<210> 225
   <211> 30
   <212> DNA
   <213> Artificial Sequence
<220>
   <223> Primer

<400> 225
   taggtttacg tcagtatggc gtgattatgg 30
<210> 226
   <211> 23
   <212> DNA
   <213> Artificial Sequence
<220>
   <223> Primer
<400> 226
   tagtaccgaa gctggtcata cga 23
<210> 227
   <211> 17
   <212> DNA
   <213> Artificial Sequence
<220>
   <223> Primer
<400> 227
   tagtacgaga ggaccgg 17
<210> 228
   <211> 18
   <212> DNA
   <213> Artificial Sequence
<220>
   <223> Primer
<400> 228
   tagtcccgca acgagcgc 18
<210> 229
   <211> 27
   <212> DNA
   <213> Artificial Sequence
<220>
   <223> Primer
<400> 229
   tagtgataga actgtaggca caatcgt 27
<210> 230
   <211> 22
   <212> DNA
   <213> Artificial Sequence
<220>
   <223> Primer
<400> 230
   tagttgctca aacagctggg ct 22
<210> 231
   <211> 29
   <212> DNA
   <213> Artificial Sequence
<220>
   <223> Primer
<400> 231
   tataagtggg taaaccgtga atatcgtgt 29
<210> 232
   <211> 24
   <212> DNA
   <213> Artificial Sequence
<220>
   <223> Primer

<400> 232
   tatacttcaa cgcctgctgc tttc 24
<210> 233
   <211> 22
   <212> DNA
   <213> Artificial Sequence
<220>
   <223> Primer
<400> 233
   tatcgctcag gcgaactcca ac 22
<210> 234
   <211> 23
   <212> DNA
   <213> Artificial Sequence
<220>
   <223> Primer
<400> 234
   tatgaccaaa ctcatcagac gag 23
<210> 235
   <211> 30
   <212> DNA
   <213> Artificial Sequence
<220>
   <223> Primer
<400> 235
   tatgattaca attcaagaag gtcgtcacgc 30
<210> 236
   <211> 27
   <212> DNA
   <213> Artificial Sequence
<220>
   <223> Primer
<400> 236
   tatgcagtgg aacgatggtt tccaaga 27
<210> 237
   <211> 24
   <212> DNA
   <213> Artificial Sequence
<220>
   <223> Primer
<400> 237
   tatgctgacc gaccagtggt acgt 24
<210> 238
   <211> 21
   <212> DNA
   <213> Artificial Sequence
<220>
   <223> Primer
<400> 238
   tatggccatg gcagaagctc a 21
<210> 239
   <211> 15
   <212> DNA
   <213> Artificial Sequence
<220>
   <223> Primer
<400> 239
   tatggctcta ctcaa 15
<210> 240
   <211> 31
   <212> DNA
   <213> Artificial Sequence
<220>
   <223> Primer
<400> 240
   tatgtccaag aagcatagca aaaaaagcaa t 31
<210> 241
   <211> 27
   <212> DNA
   <213> Artificial Sequence
<220>
   <223> Primer

<400> 241
   tattcaaggt ggtcctttga tgcatgt 27
<210> 242
   <211> 22
   <212> DNA
   <213> Artificial Sequence
<220>
   <223> Primer
<400> 242
   tattggacaa cggtcgtcgc gg 22
<210> 243
   <211> 30
   <212> DNA
   <213> Artificial Sequence
<220>
   <223> Primer
<400> 243
   tattgtttca aatgtacaag gtgaagtgcg 30
<210> 244
   <211> 30
   <212> DNA
   <213> Artificial Sequence
<220>
   <223> Primer
<400> 244
   tatttcacat gtaattttga tattcgcact 30
<210> 245
   <211> 32
   <212> DNA
   <213> Artificial Sequence
<220>
   <223> Primer
<400> 245
   tcaaaaagcc ctaggtaaag agattccata tc 32
<210> 246
   <211> 29
   <212> DNA
   <213> Artificial Sequence
<220>
   <223> Primer
<400> 246
   tcaaactggg caatcggaac tggtaaatc 29
<210> 247
   <211> 26
   <212> DNA
   <213> Artificial Sequence
<220>
   <223> Primer
<400> 247
   tcaaatgtac aaggtgaagt gcgtga 26
<210> 248
   <211> 29
   <212> DNA
   <213> Artificial Sequence
<220>
   <223> Primer
<400> 248
   tcaacaacct cttggaggta aagctcagt 29
<210> 249
   <211> 19
   <212> DNA
   <213> Artificial Sequence
<220>
   <223> Primer
<400> 249
   tcaacctcgg cccgaacca 19
<210> 250
   <211> 25
   <212> DNA
   <213> Artificial Sequence
<220>
   <223> Primer
<400> 250
   tcaacctgac tgcgtgaatg gttgt 25
<210> 251
   <211> 27
   <212> DNA
   <213> Artificial Sequence
<220>
   <223> Primer

<400> 251
   tcaacgaagg taaaaaccat ctcaacg 27
<210> 252
   <211> 27
   <212> DNA
   <213> Artificial Sequence
<220>
   <223> Primer
<400> 252
   tcaacggtaa cttctatgtt acttctg 27
<210> 253
   <211> 26
   <212> DNA
   <213> Artificial Sequence
<220>
   <223> Primer
<400> 253
   tcaactcgaa ttttcaacag gtacca 26
<210> 254
   <211> 17
   <212> DNA
   <213> Artificial Sequence
<220>
   <223> Primer
<400> 254
   tcaagaagaa aaagagc 17
<210> 255
   <211> 24
   <212> DNA
   <213> Artificial Sequence
<220>
   <223> Primer
<400> 255
   tcaagcaaac gcacaatcag aagc 24
<210> 256
   <211> 27
   <212> DNA
   <213> Artificial Sequence
<220>
   <223> Primer
<400> 256
   tcaagcagaa gctttggaag aagaagg 27
<210> 257
   <211> 27
   <212> DNA
   <213> Artificial Sequence
<220>
   <223> Primer
<400> 257
   tcaagccgta cgtattatta ggtgctg 27
<210> 258
   <211> 27
   <212> DNA
   <213> Artificial Sequence
<220>
   <223> Primer

<400> 258
   tcaataccgc aacagcggtg gcttggg 27
<210> 259
   <211> 28
   <212> DNA
   <213> Artificial Sequence
<220>
   <223> Primer
<400> 259
   tcaatgaacg accaacaagt gattgatg 28
<210> 260
   <211> 28
   <212> DNA
   <213> Artificial Sequence
<220>
   <223> Primer
<400> 260
   tcaatgaacg atcaacaagt gattgatg 28
<210> 261
   <211> 24
   <212> DNA
   <213> Artificial Sequence
<220>
   <223> Primer
<400> 261
   tcacatatcg tgagcaatga actg 24
<210> 262
   <211> 30
   <212> DNA
   <213> Artificial Sequence
<220>
   <223> Primer
<400> 262
   tcaccaggtt caactcaaaa aatattaaca 30
<210> 263
   <211> 25
   <212> DNA
   <213> Artificial Sequence
<220>
   <223> Primer
<400> 263
   tcaccagttt gccacgtatc ttcaa 25
<210> 264
   <211> 28
   <212> DNA
   <213> Artificial Sequence
<220>
   <223> Primer
<400> 264
   tcaccctcat ggtgactcat ctatttat 28
<210> 265
   <211> 28
   <212> DNA
   <213> Artificial Sequence
<220>
   <223> Primer
<400> 265
   tcaccctcat ggtgattcag ctgtttat 28
<210> 266
   <211> 25
   <212> DNA
   <213> Artificial Sequence
<220>
   <223> Primer
<400> 266
   tcaccgatat catggcttac cacgg 25
<210> 267
   <211> 23
   <212> DNA
   <213> Artificial Sequence
<220>
   <223> Primer

<400> 267
   tcaccgtgcc gttcaaggaa gag 23
<210> 268
   <211> 29
   <212> DNA
   <213> Artificial Sequence
<220>
   <223> Primer
<400> 268
   tcacctccaa gtttagatca cttgagaga 29
<210> 269
   <211> 26
   <212> DNA
   <213> Artificial Sequence
<220>
   <223> Primer
<400> 269
   tcacgataag aaaaccggtc aagagg 26
<210> 270
   <211> 27
   <212> DNA
   <213> Artificial Sequence
<220>
   <223> Primer
<400> 270
   tcactcttac atataaggaa ggcgctc 27
<210> 271
   <211> 25
   <212> DNA
   <213> Artificial Sequence
<220>
   <223> Primer
<400> 271
   tcagaccatg ctcgcagaga aactt 25
<210> 272
   <211> 25
   <212> DNA
   <213> Artificial Sequence
<220>
   <223> Primer
<400> 272
   tcagagaccg ttttatccta tcagc 25
<210> 273
   <211> 26
   <212> DNA
   <213> Artificial Sequence
<220>
   <223> Primer

<400> 273
   tcagcaaatg catcacaaac agataa 26
<210> 274
   <211> 25
   <212> DNA
   <213> Artificial Sequence
<220>
   <223> Primer
<400> 274
   tcagcatatg cacatggaac acctc 25
<210> 275
   <211> 26
   <212> DNA
   <213> Artificial Sequence
<220>
   <223> Primer
<400> 275
   tcagcatatg cacatggaac acctca 26
<210> 276
   <211> 22
   <212> DNA
   <213> Artificial Sequence
<220>
   <223> Primer
<400> 276
   tcagccatca aagcagctat tg 22
<210> 277
   <211> 21
   <212> DNA
   <213> Artificial Sequence
<220>
   <223> Primer
<400> 277
   tcagcgcgta cagtgggtga t 21
<210> 278
   <211> 25
   <212> DNA
   <213> Artificial Sequence
<220>
   <223> Primer
<400> 278
   tcagcgtatg cacatggaac tcctc 25
<210> 279
   <211> 23
   <212> DNA
   <213> Artificial Sequence
<220>
   <223> Primer
<400> 279
   tcagctacat cgactatgcg atg 23
<210> 280
   <211> 30
   <212> DNA
   <213> Artificial Sequence
<220>
   <223> Primer
<400> 280
   tcagctagac cttttaggta aagctaagct 30
<210> 281
   <211> 29
   <212> DNA
   <213> Artificial Sequence
<220>
   <223> Primer

<400> 281
   tcagctattt ttccaggtat ccaaggtgg 29
<210> 282
   <211> 23
   <212> DNA
   <213> Artificial Sequence
<220>
   <223> Primer
<400> 282
   tcagctgtcg cagttcatgg acc 23
<210> 283
   <211> 25
   <212> DNA
   <213> Artificial Sequence
<220>
   <223> Primer
<400> 283
   tcaggaaaag ggcattttac ccttg 25
<210> 284
   <211> 30
   <212> DNA
   <213> Artificial Sequence
<220>
   <223> Primer
<400> 284
   tcaggagtcg ttcaactcga tctacatgat 30
<210> 285
   <211> 31
   <212> DNA
   <213> Artificial Sequence
<220>
   <223> Primer
<400> 285
   tcaggagtcg ttcaactcga tctacatgat g 31
<210> 286
   <211> 30
   <212> DNA
   <213> Artificial Sequence
<220>
   <223> Primer
<400> 286
   tcaggatgga aataaccacc aattcactac 30
<210> 287
   <211> 23
   <212> DNA
   <213> Artificial Sequence
<220>
   <223> Primer
<400> 287
   tcaggcattg cggttgggat ggc 23
<210> 288
   <211> 24
   <212> DNA
   <213> Artificial Sequence
<220>
   <223> Primer

<400> 288
   tcaggtactg ctatccaccc tcaa 24
<210> 289
   <211> 22
   <212> DNA
   <213> Artificial Sequence
<220>
   <223> Primer
<400> 289
   tcaggtggct tacacggcgt ag 22
<210> 290
   <211> 26
   <212> DNA
   <213> Artificial Sequence
<220>
   <223> Primer
<400> 290
   tcagtatgta tccaccgtag ccagtc 26
<210> 291
   <211> 23
   <212> DNA
   <213> Artificial Sequence
<220>
   <223> Primer
<400> 291
   tcagttccgt tatcgccatt gca 23
<210> 292
   <211> 24
   <212> DNA
   <213> Artificial Sequence
<220>
   <223> Primer
<400> 292
   tcagttccgt tatcgccatt gcat 24
<210> 293
   <211> 25
   <212> DNA
   <213> Artificial Sequence
<220>
   <223> Primer
<400> 293
   tcagttccgt tatcgccatt gcatt 25
<210> 294
   <211> 24
   <212> DNA
   <213> Artificial Sequence
<220>
   <223> Primer
<400> 294
   tcagttcggc ggtcagcgct tcgg 24
<210> 295
   <211> 34
   <212> DNA
   <213> Artificial Sequence
<220>
   <223> Primer

<400> 295
   tcagttttaa tgtctcgtat gatcgaatca aaag 34
<210> 296
   <211> 24
   <212 DNA
   <213> Artificial Sequence
<220>
   <223> Primer
<400> 296
   tcatccacac ggtggtggtg aagg 24
<210> 297
   <211> 28
   <212> DNA
   <213> Artificial Sequence
<220>
   <223> Primer
<400> 297
   tcatcctaag ccaagtgtag actctgta 28
<210> 298
   <211> 31
   <212> DNA
   <213> Artificial Sequence
<220>
   <223> Primer
<400> 298
   tcatgataat atctttgaaa tcggctcagg a 31
<210> 299
   <211> 33
   <212> DNA
   <213> Artificial Sequence
<220>
   <223> Primer
<400> 299
   tcatgttgag cttaaaccta tagaagtaaa agc 33
<210> 300
   <211> 27
   <212> DNA
   <213> Artificial Sequence
<220>
   <223> Primer
<400> 300
   tcattatcat gcgccaatga gtgcaga 27
<210> 301
   <211> 28
   <212> DNA
   <213> Artificial Sequence
<220>
   <223> Primer
<400> 301
   tcattcaaga actagatctt caggcaag 28
<210> 302
   <211> 26
   <212> DNA
   <213> Artificial Sequence
<220>
   <223> Primer
<400> 302
   tccaaaaaaa tcagcgcgta cagtgg 26
<210> 303
   <211> 29
   <212> DNA
   <213> Artificial Sequence
<220>
   <223> Primer

<400> 303
   tccaaaccag gtgtatcaag aacatcagg 29
<210> 304
   <211> 29
   <212> DNA
   <213> Artificial Sequence
<220>
   <223> Primer
<400> 304
   tccaaataag tggcgttaca aatactgaa 29
<210> 305
   <211> 32
   <212> DNA
   <213> Artificial Sequence
<220>
   <223> Primer
<400> 305
   tccaacgaag tacaatacaa gacaaaagaa gg 32
<210> 306
   <211> 32
   <212> DNA
   <213> Artificial Sequence
<220>
   <223> Primer
<400> 306
   tccaaggtac actaaactta cttgagctaa tg 32
<210> 307
   <211> 24
   <212> DNA
   <213> Artificial Sequence
<220>
   <223> Primer
<400> 307
   tccaatgcca caaactcgtg aaca 24
<210> 308
   <211> 24
   <212> DNA
   <213> Artificial Sequence
<220>
   <223> Primer
<400> 308
   tccacacgcc gttcttcaac aact 24
<210> 309
   <211> 21
   <212> DNA
   <213> Artificial Sequence
<220>
   <223> Primer
<400> 309
   tccacacggt ggtggtgaag g 21
<210> 310
   <211> 26
   <212> DNA
   <213> Artificial Sequence
<220>
   <223> Primer
<400> 310
   tccaccaaga gcaagatcaa ataggc 26
<210> 311
   <211> 20
   <212> DNA
   <213> Artificial Sequence
<220>
   <223> Primer

<400> 311
   tccacggtca tggagcgcta 20
<210> 312
   <211> 30
   <212> DNA
   <213> Artificial Sequence
<220>
   <223> Primer
<400> 312
   tccacttatc gcaaatggaa aattaagcaa 30
<210> 313
   <211> 33
   <212> DNA
   <213> Artificial Sequence
<220>
   <223> Primer
<400> 313
   tccagatgga caaattttct tagaaactga ttt 33
<210> 314
   <211> 26
   <212> DNA
   <213> Artificial Sequence
<220>
   <223> Primer
<400> 314
   tccagcacga attgctgcta tgaaag 26
<210> 315
   <211> 33
   <212> DNA
   <213> Artificial Sequence
<220>
   <223> Primer
<400> 315
   tccaggacaa atgtatgaaa aatgtccaag aag 33
<210> 316
   <211> 25
   <212> DNA
   <213> Artificial Sequence
<220>
   <223> Primer
<400> 316
   tccattgttc gtatggctca agact 25
<210> 317
   <211> 31
   <212> DNA
   <213> Artificial Sequence
<220>
   <223> Primer
<400> 317
   tcccaattaa ttctgccatt tttccaggta t 31
<210> 318
   <211> 34
   <212> DNA
   <213> Artificial Sequence
<220>
   <223> Primer
<400> 318
   tcccacgaaa cagatgaaga aattaacaaa aaag 34
<210> 319
   <211> 30
   <212> DNA
   <213> Artificial Sequence
<220>
   <223> Primer

<400> 319
   tcccagctag accttttagg taaagctaag 30
<210> 320
   <211> 26
   <212> DNA
   <213> Artificial Sequence
<220>
   <223> Primer
<400> 320
   tcccaggtga cgatgtacct gtaatc 26
<210> 321
   <211> 26
   <212> DNA
   <213> Artificial Sequence
<220>
   <223> Primer
<400> 321
   tccccaggac accctgaaat ttcaac 26
<210> 322
   <211> 34
   <212> DNA
   <213> Artificial Sequence
<220>
   <223> Primer
<400> 322
   tcccccacgc tttaattgtt tatgatgatt tgag 34
<210> 323
   <211> 31
   <212> DNA
   <213> Artificial Sequence
<220>
   <223> Primer
<400> 323
   tcccggactt aatatcaatg aaaattgtgg a 31
<210> 324
   <211> 28
   <212> DNA
   <213> Artificial Sequence
<220>
   <223> Primer
<400> 324
   tcccggagct tttatgacta aagcagat 28
<210> 325
   <211> 19
   <212> DNA
   <213> Artificial Sequence
<220>
   <223> Primer
<400> 325
   tccgcggagt tgactgggt 19
<210> 326
   <211> 20
   <212> DNA
   <213> Artificial Sequence
<220>
   <223> Primer
<400> 326
   tccgctgaat ctgtcgccgc 20
<210> 327
   <211> 23
   <212> DNA
   <213> Artificial Sequence
<220>
   <223> Primer

<400> 327
   tccggctcac gttattatgg tac 23
<210> 328
   <211> 27
   <212> DNA
   <213> Artificial Sequence
<220>
   <223> Primer
<400> 328
   tccgtacgta ttattaggtg ctggtca 27
<210> 329
   <211> 30
   <212> DNA
   <213> Artificial Sequence
<220>
   <223> Primer
<400> 329
   tccgttatcg ccattgcatt atttggaact 30
<210> 330
   <211> 33
   <212> DNA
   <213> Artificial Sequence
<220>
   <223> Primer
<400> 330
   tccgttctta caaatagcaa tagaacttga agc 33
<210> 331
   <211> 36
   <212> DNA
   <213> Artificial Sequence
<220>
   <223> Primer
<400> 331
   tccgttgatt attgttatcc tgttatgcca tttgag 36
<210> 332
   <211> 34
   <212> DNA
   <213> Artificial Sequence
<220>
   <223> Primer
<400> 332
   tcctaatgga cttaatatca atgaaaattg tgga 34
<210> 333
   <211> 22
   <212> DNA
   <213> Artificial Sequence
<220>
   <223> Primer

<400> 333
   tcctagagga atggctgcca cg 22
<210> 334
   <211> 30
   <212> DNA
   <213> Artificial Sequence
<220>
   <223> Primer
<400> 334
   tcctatatta atcgtttaca gaaactggct 30
<210> 335
   <211> 31
   <212> DNA
   <213> Artificial Sequence
<220>
   <223> Primer
<220>
   <221> modified_base
   <222> (15) ... (15)
   <223> I
<400> 335
   tcctcaatga acgatcaaca agtgattgat g 31
<210> 336
   <211> 31
   <212> DNA
   <213> Artificial Sequence
<220>
   <223> Primer
<400> 336
   tcctcaatga atgatcaaca agtgattgat g 31
<210> 337
   <211> 31
   <212> DNA
   <213> Artificial Sequence
<220>
   <223> Primer
<220>
   <221> modified base
   <222> (15)...(15)
   <223> I
<220>
   <221> modified_base
   <222> (24)...(24)
   <223> I
<400> 337
   tcctcgatga acgatcaaca agtgattgat g 31
<210> 338
   <211> 31
   <212> DNA
   <213> Artificial Sequence
<220>
   <223> Primer
<220>
   <221> modified_base
   <222> (15)...(15)
   <223> I
<220>
   <221> modified_base
   <222> (24)...(24)
   <223> I
<400> 338
   tcctcgatga atgatcaaca agtgattgat g 31
<210> 339
   <211> 31
   <212> DNA
   <213> Artificial Sequence
<220>
   <223> Primer
   220>
<221> modified_base
   <222> (6)...(6)
   <223> I
<220>
   <221> modified_base
   <222> (15)...(15)
   <223> I

<400> 339
   tcctcgatga acgatcaaca agtgattgat g 31
<210> 340
   <211> 31
   <212> DNA
   <213> Artificial Sequence
<220>
   <223> Primer
<220>
   <221> modified_base
   <222> (6)...(6)
   <223> I
<220>
   <221> modified_base
   <222> (12)...(12)
   <223> I
   220>
<221> modified_base
   <222> (15)...(15)
   <223> I
<220>
   <221> modified_base
   <222> (24)...(24)
   <223> I
<400> 340
   tcctcgatga acgatcaaca agtgattgat g 31
<210> 341
   <211> 20
   <212> DNA
   <213> Artificial Sequence
<220>
   <223> Primer
<400> 341
   tcctgaaaaa tggagcacgg 20
<210> 342
   <211> 23
   <212> DNA
   <213> Artificial Sequence
<220>
   <223> Primer
<400> 342
   tcctgaagca agtgcattta cga 23
<210> 343
   <211> 28
   <212> DNA
   <213> Artificial Sequence
<220>
   <223> Primer
<400> 343
   tcctgaccga cccattattc cctttatc 28
<210> 344
   <211> 33
   <212> DNA
   <213> Artificial Sequence
<220>
   <223> Primer
<400> 344
   tcctgatgct caaagtgctt ttttagatcc ttt 33
<210> 345
   <211> 34
   <212> DNA
   <213> Artificial Sequence
<220>
   <223> Primer

<400> 345
   tcctgttatc cctgaagtag ttaatcaagt ttgt 34
<210> 346
   <211> 35
   <212> DNA
   <213> Artificial Sequence
<220>
   <223> Primer
<400> 346
   tcctgttatc cctgaagtag ttaatcaagt ttgtt 35
<210> 347
   <211> 36
   <212> DNA
   <213> Artificial Sequence
<220>
   <223> Primer
<400> 347
   tcctgttatt cctgaagtag ttaatcaagt ttgtta 36
<210> 348
   <211> 31
   <212> DNA
   <213> Artificial Sequence
<220>
   <223> Primer
<400> 348
   tccttacttc gaactatgaa tcttttggaa g 31
<210> 349
   <211> 29
   <212> DNA
   <213> Artificial Sequence
<220>
   <223> Primer
<400> 349
   tccttatagg gatggctatc agtaatgtt 29
<210> 350
   <211> 22
   <212> DNA
   <213> Artificial Sequence
<220>
   <223> Primer
<400> 350
   tccttgaccg cctttccgat ac 22
<210> 351
   <211> 33
   <212> DNA
   <213> Artificial Sequence
<220>
   <223> Primer

<400> 351
   tccttgcttt agttttaagt gcatgtaatt caa 33
<210> 352
   <211> 32
   <212> DNA
   <213> Artificial Sequence
<220>
   <223> Primer
<400> 352
   tcctttgata tattatgcga tggaaggttg gt 32
<210> 353
   <211> 30
   <212> DNA
   <213> Artificial Sequence
<220>
   <223> Primer
<400> 353
   tcctttgatg catgtaattg ctgcaaaagc 30
<210> 354
   <211> 31
   <212> DNA
   <213> Artificial Sequence
<220>
   <223> Primer
<400> 354
   tcgaaagctt ttgcatatta tatcgagcca c 31
<210> 355
   <211> 28
   <212> DNA
   <213> Artificial Sequence
<220>
   <223> Primer
<400> 355
   tcgaagtaca atacaagaca aaagaagg 28
<210> 356
   <211> 28
   <212> DNA
   <213> Artificial Sequence
<220>
   <223> Primer
<400> 356
   tcgacaacac cattatctat ggtgtgaa 28
<210> 357
   <211> 23
   <212> DNA
   <213> Artificial Sequence
<220>
   <223> Primer
<400> 357
   tcgacctttg gcaggaacta gac 23
<210> 358
   <211> 17
   <212> DNA
   <213> Artificial Sequence
<220>
   <223> Primer

<400> 358
   tcgagcaggc gctgccg 17
<210> 359
   <211> 31
   <212> DNA
   <213> Artificial Sequence
<220>
   <223> Primer
<400> 359
   tcgagtatag ctaaaaaaat agtttatgac a 31
<210> 360
   <211> 26
   <212> DNA
   <213> Artificial Sequence
<220>
   <223> Primer
<400> 360
   tcgatctggt ttcatgctgt ttcagt 26
<210> 361
   <211> 28
   <212> DNA
   <213> Artificial Sequence
<220>
   <223> Primer
<400> 361
   tcgatgaacg accaacaagt gattgatg 28
<210> 362
   <211> 24
   <212> DNA
   <213> Artificial Sequence
<220>
   <223> Primer
<400> 362
   tcgattaggc agcaacgaaa gccg 24
<210> 363
   <211> 24
   <212> DNA
   <213> Artificial Sequence
<220>
   <223> Primer
<400> 363
   tcgcaaaaaa atccagctat tagc 24
<210> 364
   <211> 26
   <212> DNA
   <213> Artificial Sequence
<220>
   <223> Primer
<400> 364
   tcgccaatca aaactaaggg aatggc 26
<210> 365
   <211> 19
   <212> DNA
   <213> Artificial Sequence
<220>
   <223> Primer
<400> 365
   tcgccatcgt caccaaccg 19
<210> 366
   <211> 16
   <212> DNA
   <213> Artificial Sequence
<220>
   <223> Primer

<400> 366
   tcgcccgcga ggacgt 16
<210> 367
   <211> 21
   <212> DNA
   <213> Artificial Sequence
<220>
   <223> Primer
<400> 367
   tcgccgactt cgacggtgac c 21
<210> 368
   <211> 22
   <212 DNA
   <213> Artificial Sequence
<220>
   <223> Primer
<400> 368
   tcgccggcaa tgccattgga ta 22
<210> 369
   <211> 23
   <212> DNA
   <213> Artificial Sequence
<220>
   <223> Primer
<400> 369
   tcgccgtgga aaaatcctac gct 23
<210> 370
   <211> 31
   <212> DNA
   <213> Artificial Sequence
<220>
   <223> Primer
<400> 370
   tcgcgttgca acaaaacttt ctaaagtatg t 31
<210> 371
   <211> 24
   <212> DNA
   <213> Artificial Sequence
<220>
   <223> Primer
<400> 371
   tcgctacagg ccctttagga caag 24
<210> 372
   <211> 27
   <212> DNA
   <213> Artificial Sequence
<220>
   <223> Primer
<400> 372
   tcgctatctt atcgttgaga agggatt 27
<210> 373
   <211> 24
   <212> DNA
   <213> Artificial Sequence
<220>
   <223> Primer
<400> 373
   tcggaatctg atgttgcagt tgtt 24
<210> 374
   <211> 22
   <212> DNA
   <213> Artificial Sequence
<220>
   <223> Primer
<400> 374
   tcggccgcac cttcatcgaa gt 22
<210> 375
   <211> 28
   <212> DNA
   <213> Artificial Sequence
<220>
   <223> Primer

<400> 375
   tcggcgaaat ccgtattcct gaaaatga 28
<210> 376
   <211> 18
   <212> DNA
   <213> Artificial Sequence
<220>
   <223> Primer
<400> 376
   tcggcgcctg cctgatga 18
<210> 377
   <211> 23
   <212> DNA
   <213> Artificial Sequence
<220>
   <223> Primer
<400> 377
   tcgggtgatg atgcgcgtga agg 23
<210> 378
   <211> 31
   <212> DNA
   <213> Artificial Sequence
<220>
   <223> Primer
<400> 378
   tcggtttagt aaaagaacgt attgctcaac c 31
<210> 379
   <211> 28
   <212> DNA
   <213> Artificial Sequence
<220>
   <223> Primer
<400> 379
   tcgtacgtat tattaggtgc tggtcact 28
<210> 380
   <211> 21
   <212> DNA
   <213> Artificial Sequence
<220>
   <223> Primer
<400> 380
   tcgtatggct caatggtgga g 21
<210> 381
   <211> 30
   <212> DNA
   <213> Artificial Sequence
<220>
   <223> Primer
<400> 381
   tcgtcttttt gattctttcc ctgataatgc 30
<210> 382
   <211> 32
   <212> DNA
   <213> Artificial Sequence
<220>
   <223> Primer
<400> 382
   tcgtcttttt gattctttcc ctgataatgc tc 32
<210> 383
   <211> 25
   <212> DNA
   <213> Artificial Sequence
<220>
   <223> Primer

<400> 383
   tcgtgattat ggatggcaac gtgaa 25
<210> 384
   <211> 24
   <212> DNA
   <213> Artificial Sequence
<220>
   <223> Primer
<400> 384
   tcgtgcccgc aatttgcata aagc 24
<210> 385
   <211> 21
   <212> DNA
   <213> Artificial Sequence
<220>
   <223> Primer
<400> 385
   tcgtggcggc gtggttatcg a 21
<210> 386
   <211> 27
   <212> DNA
   <213> Artificial Sequence
<220>
   <223> Primer
<400> 386
   tcgtgttgaa cgtggtcaaa tcaaagt 27
<210> 387
   <211> 23
   <212> DNA
   <213> Artificial Sequence
<220>
   <223> Primer
<400> 387
   tcgttcctgg aacacgatga cgc 23
<210> 388
   <211> 27
   <212> DNA
   <213> Artificial Sequence
<220>
   <223> Primer
<400> 388
   tcgtttggtg gtggtagatg aaaaagg 27
<210> 389
   <211> 11
   <212> DNA
   <213> Artificial Sequence
<220>
   <223> Primer
<400> 389
   tccaccctca a 11
<210> 390
   <211> 25
   <212> DNA
   <213> Artificial Sequence
<220>
   <223> Primer

<400> 390
   tctaaaacac caggtcaccc agaag 25
<210> 391
   <211> 24
   <212> DNA
   <213> Artificial Sequence
<220>
   <223> Primer
<400> 391
   tctaaatggt cgtgcagttg cgtg 24
<210> 392
   <211> 30
   <212> DNA
   <213> Artificial Sequence
<220>
   <223> Primer
<400> 392
   tctactgatt ttggtaatct tgcagcacag 30
<210> 393
   <211> 24
   <212> DNA
   <213> Artificial Sequence
<220>
   <223> Primer
<400> 393
   tctagtaata ataggaccct cagc 24
<210> 394
   <211> 31
   <212> DNA
   <213> Artificial Sequence
<220>
   <223> Primer
<400> 394
   tctcaaggtg atattggtgt aggtaactta a 31
<210> 395
   <211> 24
   <212> DNA
   <213> Artificial Sequence
<220>
   <223> Primer
<400> 395
   tctcattacg ttgcatcgga aaca 24
<210> 396
   <211> 30
   <212> DNA
   <213> Artificial Sequence
<220>
   <223> Primer
<400> 396
   tctcgatgaa cgaccaacaa gtgattgatg 30
<210> 397
   <211> 28
   <212> DNA
   <213> Artificial Sequence
<220>
   <223> Primer
<400> 397
   tctcgtggtg cacaagtaac ggatatta 28
<210> 398
   <211> 32
   <212> DNA
   <213> Artificial Sequence
<220>
   <223> Primer

<400> 398
   tctgaaatga atagtgatag aactgtaggc ac 32
<210> 399
   <211> 32
   <212> DNA
   <213> Artificial Sequence
<220>
   <223> Primer
<400> 399
   tctgaacatg ataatatctt tgaaatcggc tc 32
<210> 400
   <211> 30
   <212> DNA
   <213> Artificial Sequence
<220>
   <223> Primer
<400> 400
   tctgaatgtc tatatggagg tacaacacta 30
<210> 401
   <211> 19
   <212> DNA
   <213> Artificial Sequence
<220>
   <223> Primer
<400> 401
   tctgacacct gcccggtgc 19
<210> 402
   <211> 20
   <212> DNA
   <213> Artificial Sequence
<220>
   <223> Primer
<400> 402
   tctgcccgtg tcgttggtga 20
<210> 403
   <211> 24
   <212> DNA
   <213> Artificial Sequence
<220>
   <223> Primer
<400> 403
   tctggaggca caccaaataa aaca 24
<210> 404
   <211> 21
   <212> DNA
   <213> Artificial Sequence
<220>
   <223> Primer
<400> 404
   tctggataac ggtcgtcgcg g 21
<210> 405
   <211> 25
   <212> DNA
   <213> Artificial Sequence
<220>
   <223> Primer

<400> 405
   tctggcaggt atgcgtggtc tgatg 25
<210> 406
   <211> 24
   <212> DNA
   <213> Artificial Sequence
<220>
   <223> Primer
<400> 406
   tctggctaaa actttggcaa cggt 24
<210> 407
   <211> 29
   <212> DNA
   <213> Artificial Sequence
<220>
   <223> Primer
<400> 407
   tctggtccaa caaaaggaac gattacagg 29
<210> 408
   <211> 25
   <212> DNA
   <213> Artificial Sequence
<220>
   <223> Primer
<400> 408
   tctgtcccta gtacgagagg accgg 25
<210> 409
   <211> 23
   <212> DNA
   <213> Artificial Sequence
<220>
   <223> Primer
<400> 409
   tctgttctta gtacgagagg acc 23
<210> 410
   <211> 26
   <212> DNA
   <213> Artificial Sequence
<220>
   <223> Primer
<400> 410
   tcttatgcca agaggacaga gtgagt 26
<210> 411
   <211> 29
   <212> DNA
   <213> Artificial Sequence
<220>
   <223> Primer
<400> 411
   tcttatgcca agaggacaga gtgagtact 29
<210> 412
   <211> 31
   <212> DNA
   <213> Artificial Sequence
<220>
   <223> Primer

<400> 412
   tcttattcca acttcaaacc gaactatgac g 31
<210> 413
   <211> 29
   <212> DNA
   <213> Artificial Sequence
<220>
   <223> Primer
<400> 413
   tcttctcatc ctatggctat tatgcttgc 29
<210> 414
   <211> 33
   <212> DNA
   <213> Artificial Sequence
<220>
   <223> Primer
<400> 414
   tcttgatact tgtaatgtgg gcgataaata tgt 33
<210> 415
   <211> 32
   <212> DNA
   <213> Artificial Sequence
<220>
   <223> Primer
<400> 415
   tcttgcagca gtttatttga tgaacctaaa gt 32
<210> 416
   <211> 28
   <212> DNA
   <213> Artificial Sequence
<220>
   <223> Primer
<400> 416
   tcttgctctt tcgtgagttc agtaaatg 28
<210> 417
   <211> 31
   <212> DNA
   <213> Artificial Sequence
<220>
   <223> Primer
<400> 417
   tcttgtactt gtggctcaca cggctgtttg g 31
<210> 418
   <211> 27
   <212> DNA
   <213> Artificial Sequence
<220>
   <223> Primer

<400> 418
   tcttgtttat gctggtaaag cagatgg 27
<210> 419
   <211> 28
   <212> DNA
   <213> Artificial Sequence
<220>
   <223> Primer
<400> 419
   tctttatggt ggagatgact gaaaccga 28
<210> 420
   <211> 28
   <212> DNA
   <213> Artificial Sequence
<220>
   <223> Primer
<400> 420
   tctttcttga atgctggtgt acgtatcg 28
<210> 421
   <211> 25
   <212> DNA
   <213> Artificial Sequence
<220>
   <223> Primer
<400> 421
   tctttgaaat cggctcagga aaagg 25
<210> 422
   <211> 26
   <212> DNA
   <213> Artificial Sequence
<220>
   <223> Primer
<400> 422
   tctttgccat tgaagatgac ttaagc 26
<210> 423
   <211> 29
   <212> DNA
   <213> Artificial Sequence
<220>
   <223> Primer
<400> 423
   tcttttacaa aaggggaaaa agttgactt 29
<210> 424
   <211> 34
   <212> DNA
   <213> Artificial Sequence
<220>
   <223> Primer
<400> 424
   tgaaaaatgt ccaagaagca tagcaaaaaa agca 34
<210> 425
   <211> 30
   <212> DNA
   <213> Artificial Sequence
<220>
   <223> Primer

<400> 425
   tgaaaagggt gaagtagcaa atggagatag 30
<210> 426
   <211> 32
   <212> DNA
   <213> Artificial Sequence
<220>
   <223> Primer
<400> 426
   tgaaaagtat ggatttgaac aactcgtgaa ta 32
<210> 427
   <211> 27
   <212> DNA
   <213> Artificial Sequence
<220>
   <223> Primer
<400> 427
   tgaaatctca ttacgttgca tcggaaa 27
<210> 428
   <211> 30
   <212> DNA
   <213> Artificial Sequence
<220>
   <223> Primer
<400> 428
   tgaaattgct acaggccctt taggacaagg 30
<210> 429
   <211> 25
   <212> DNA
   <213> Artificial Sequence
<220>
   <223> Primer
<400> 429
   tgaacgctgg tggcatgctt aacac 25
<210> 430
   <211> 30
   <212> DNA
   <213> Artificial Sequence
<220>
   <223> Primer
<400> 430
   tgaacgtggt caaatcaaag ttggtgaaga 30
<210> 431
   <211> 31
   <212> DNA
   <213> Artificial Sequence
<220>
   <223> Primer
<400> 431
   tgaacgtggt caaatcaaag ttggtgaaga a 31
<210> 432
   <211> 27
   <212> DNA
   <213> Artificial Sequence
<220>
   <223> Primer
<400> 432
   tgaagcttgt tctttagcag gacttca 27
<210> 433
   <211> 28
   <212> DNA
   <213> Artificial Sequence
<220>
   <223> Primer

<400> 433
   tgaaggtgga cgtcacactc cattcttc 28
<210> 434
   <211> 26
   <212> DNA
   <213> Artificial Sequence
<220>
   <223> Primer
<400> 434
   tgaagtagaa atgactgaac gtccga 26
<210> 435
   <211> 28
   <212> DNA
   <213> Artificial Sequence
<220>
   <223> Primer
<400> 435
   tgaagtagaa ggtgcaaagc aagttaga 28
<210> 436
   <211> 34
   <212> DNA
   <213> Artificial Sequence
<220>
   <223> Primer
<400> 436
   tgaagtgcgt gatgatatcg atgcacttga tgta 34
<210> 437
   <211> 31
   <212> DNA
   <213> Artificial Sequence
<220>
   <223> Primer
<400> 437
   tgaatagtga tagaactgta ggcacaatcg t 31
<210> 438
   <211> 31
   <212> DNA
   <213> Artificial Sequence
<220>
   <223> Primer
<400> 438
   tgaatgctta tttacctgca ctcccacaac t 31
<210> 439
   <211> 31
   <212> DNA
   <213> Artificial Sequence
<220>
   <223> Primer
<400> 439
   tgaattagtt caatcatttg ttgaacgacg t 31
<210> 440
   <211> 26
   <212> DNA
   <213> Artificial Sequence
<220>
   <223> Primer
<400> 440
   tgaattgctg ctatgaaagg tggctt 26
<210> 441
   <211> 27
   <212> DNA
   <213> Artificial Sequence
<220>
   <223> Primer
<400> 441
   tgacagcgaa gaaggttaga cttgtcc 27

<210> 442
   <211> 26
   <212> DNA
   <213> Artificial Sequence
<220>
   <223> Primer
<400> 442
   tgacatccgg ctcacgttat tatggt 26
<210> 443
   <211> 27
   <212> DNA
   <213> Artificial Sequence
<220>
   <223> Primer
<400> 443
   tgacatccgg ctcacgttat tatggta 27
<210> 444
   <211> 28
   <212> DNA
   <213> Artificial Sequence
<220>
   <223> Primer
<400> 444
   tgacatccgg ctcacgttat tatggtac 28
<210> 445
   <211> 29
   <212> DNA
   <213> Artificial Sequence
<220>
   <223> Primer
<400> 445
   tgacatgata ataaccgatt gaccgaaga 29
<210> 446
   <211> 22
   <212> DNA
   <213> Artificial Sequence
<220>
   <223> Primer
<400> 446
   tgacatgctt gtccgttcag gc 22
<210> 447
   <211> 31
   <212> DNA
   <213> Artificial Sequence
<220>
   <223> Primer
<400> 447
   tgacatggac tccccctata taactcttga g 31
<210> 448
   <211> 23
   <212> DNA
   <213> Artificial Sequence
<220>
   <223> Primer
<400> 448
   tgaccaggtg atggccatgt tcg 23
<210> 449
   <211> 31
   <212> DNA
   <213> Artificial Sequence
<220>
   <223> Primer
<400> 449
   tgacctacag taagaggttc tgtaatgaac c 31
<210> 450
   <211> 23
   <212> DNA
   <213> Artificial Sequence
<220>
   <223> Primer

<400> 450
   tgacgatctt cgcggtgact agt 23
<210> 451
   <211> 26
   <212> DNA
   <213> Artificial Sequence
<220>
   <223> Primer
<400> 451
   tgacggccta tacggtgttg gtttct 26
<210> 452
   <211> 24
   <212> DNA
   <213> Artificial Sequence
<220>
   <223> Primer
<400> 452
   tgacgtcatc ggtaagtacc accc 24
<210> 453
   <211> 27
   <212> DNA
   <213> Artificial Sequence
<220>
   <223> Primer
<400> 453
   tgagatggat ttaaacctgt tcaccgc 27
<210> 454
   <211> 30
   <212> DNA
   <213> Artificial Sequence
<220>
   <223> Primer
<400> 454
   tgagattgct gaacatttaa tgctgattga 30
<210> 455
   <211> 32
   <212> DNA
   <213> Artificial Sequence
<220>
   <223> Primer
<400> 455
   tgagcaatgg ggctttgaaa gaatttttaa at 32
<210> 456
   <211> 26
   <212> DNA
   <213> Artificial Sequence
<220>
   <223> Primer
<400> 456
   tgagctgcat caactgtatt ggatag 26
<210> 457
   <211> 28
   <212> DNA
   <213> Artificial Sequence
<220>
   <223> Primer

<400> 457
   tgagctttta gttgactttt tcaacagc 28
<210> 458
   <211> 20
   <212> DNA
   <213> Artificial Sequence
<220>
   <223> Primer
<400> 458
   tgaggaccgt gtcgcgctca 20
<210> 459
   <211> 35
   <212> DNA
   <213> Artificial Sequence
<220>
   <223> Primer
<400> 459
   tgagggtttt atgcttaaag ttggttttat tggtt 35
<210> 460
   <211> 30
   <212> DNA
   <213> Artificial Sequence
<220>
   <223> Primer
<400> 460
   tgaggtggtg gataactcaa ttgatgaagc 30
<210> 461
   <211> 29
   <212> DNA
   <213> Artificial Sequence
<220>
   <223> Primer
<400> 461
   tgagtaacat ccatatttct gccatacgt 29
<210> 462
   <211> 22
   <212> DNA
   <213> Artificial Sequence
<220>
   <223> Primer
<400> 462
   tgagtaagtt ccacccgcac gg 22
<210> 463
   <211> 30
   <212> DNA
   <213> Artificial Sequence
<220>
   <223> Primer
<400> 463
   tgagtcactt gaagttgata caaatcctct 30
<210> 464
   <211> 28
   <212> DNA
   <213> Artificial Sequence
<220>
   <223> Primer
<400> 464
   tgagtgatga aggccttagg gttgtaaa 28
<210> 465
   <211> 27
   <212> DNA
   <213> Artificial Sequence
<220>
   <223> Primer
<400> 465
   tgagtgccaa catatcagtg ctgaaga 27
<210> 466
   <211> 30
   <212> DNA
   <213> Artificial Sequence
<220>
   <223> Primer

<400> 466
   tgagtttaac agttcaccat atgaaacagg 30
<210> 467
   <211> 25
   <212> DNA
   <213> Artificial Sequence
<220>
   <223> Primer
<400> 467
   tgatacttca acgcctgctg ctttc 25
<210> 468
   <211> 25
   <212> DNA
   <213> Artificial Sequence
<220>
   <223> Primer
<400> 468
   tgatcactgg tgctgctcag atgga 25
<210> 469
   <211> 30
   <212> DNA
   <213> Artificial Sequence
<220>
   <223> Primer
<400> 469
   tgatcatccg tggtataacg atttattagt 30
<210> 470
   <211> 29
   <212> DNA
   <213> Artificial Sequence
<220>
   <223> Primer
<400> 470
   tgatcgttga gaagggattt gcgaaaaga 29
<210> 471
   <211> 29
   <212> DNA
   <213> Artificial Sequence
<220>
   <223> Primer
<400> 471
   tgatctcaga atctaataat tgggacgaa 29
<210> 472
   <211> 30
   <212> DNA
   <213> Artificial Sequence
<220>
   <223> Primer
<400> 472
   tgatcttaaa aatttccgcc aacttcattc 30
<210> 473
   <211> 30
   <212> DNA
   <213> Artificial Sequence
<220>
   <223> Primer
<400> 473
   tgatgacttt ttagctaatg gtcaggcagc 30
<210> 474
   <211> 29
   <212> DNA
   <213> Artificial Sequence
<220>
   <223> Primer
<400> 474
   tgatggcaag tggatagggt ataatacag 29
<210> 475
   <211> 24
   <212> DNA
   <213> Artificial Sequence
<220>
   <223> Primer
<400> 475
   tgattaccat gagtggcaag caag 24
<210> 476
   <211> 31
   <212> DNA
   <213> Artificial Sequence
<220>
   <223> Primer

<400> 476
   tgattattgt tatcctgtta tgccatttga g 31
<210> 477
   <211> 19
   <212> DNA
   <213> Artificial Sequence
<220>
   <223> Primer
<400> 477
   tgattccggt gcccgtggt 19
<210> 478
   <211> 19
   <212> DNA
   <213> Artificial Sequence
<220>
   <223> Primer
<400> 478
   tgattctggt gcccgtggt 19
<210> 479
   <211> 34
   <212> DNA
   <213> Artificial Sequence
<220>
   <223> Primer
<400> 479
   tgattttgct aaatttagag aaattgcgga tgaa 34
<210> 480
   <211> 24
   <212> DNA
   <213> Artificial Sequence
<220>
   <223> Primer
<400> 480
   tgcaaaatct gcaacgagct ttgg 24
<210> 481
   <211> 23
   <212> DNA
   <213> Artificial Sequence
<220>
   <223> Primer
<400> 481
   tgcaaaggag gtactcagac cat 23
<210> 482
   <211> 25
   <212> DNA
   <213> Artificial Sequence
<220>
   <223> Primer
<400> 482
   tgcaagcaaa cgcacaatca gaagc 25
<210> 483
   <211> 20
   <212> DNA
   <213> Artificial Sequence
<220>
   <223> Primer
<400> 483
   tgcaagcgcg accacatacg 20
<210> 484
   <211> 23
   <212> DNA
   <213> Artificial Sequence
<220>
   <223> Primer

<400> 484
   tgcaagcttc tggtgctagc att 23
<210> 485
   <211> 24
   <212> DNA
   <213> Artificial Sequence
<220>
   <223> Primer
<400> 485
   tgcaagtggt acttcaacat gggg 24
<210> 486
   <211> 30
   <212> DNA
   <213> Artificial Sequence
<220>
   <223> Primer
<400> 486
   tgcaagttaa gaaagctgtt gcaggtttat 30
<210> 487
   <211> 33
   <212> DNA
   <213> Artificial Sequence
<220>
   <223> Primer
<400> 487
   tgcaattgct ttagttttaa gtgcatgtaa ttc 33
<210> 488
   <211> 31
   <212> DNA
   <213> Artificial Sequence
<220>
   <223> Primer
<400> 488
   tgcacaatca gaagctaaga aagcgcaagc t 31
<210> 489
   <211> 24
   <212> DNA
   <213> Artificial Sequence
<220>
   <223> Primer
<400> 489
   tgcacacgcc gttcttcaac aact 24
<210> 490
   <211> 22
   <212> DNA
   <213> Artificial Sequence
<220>
   <223> Primer
<400> 490
   tgcacatcgt gtccaacgtc ac 22
<210> 491
   <211> 32
   <212> DNA
   <213> Artificial Sequence
<220>
   <223> Primer
<400> 491
   tgcaccggct attaagaatt actttgccaa ct 32
<210> 492
   <211> 23
   <212> DNA
   <213> Artificial Sequence
<220>
   <223> Primer

<400> 492
   tgcacgatgc ggaatggttc aca 23
<210> 493
   <211> 28
   <212> DNA
   <213> Artificial Sequence
<220>
   <223> Primer
<400> 493
   tgcacgccga ctatgttaag aacatgat 28
<210> 494
   <211> 30
   <212> DNA
   <213> Artificial Sequence
<220>
   <223> Primer
<400> 494
   tgcacttatc gcaaatggaa aattaagcaa 30
<210> 495
   <211> 21
   <212> DNA
   <213> Artificial Sequence
<220>
   <223> Primer
<400> 495
   tgcagggaac agctttaggc a 21
<210> 496
   <211> 22
   <212> DNA
   <213> Artificial Sequence
<220>
   <223> Primer
<400> 496
   tgcatacaaa cagtcggagc ct 22
<210> 497
   <211> 23
   <212> DNA
   <213> Artificial Sequence
<220>
   <223> Primer
<400> 497
   tgcataccgg taagttggca aca 23
<210> 498
   <211> 27
   <212> DNA
   <213> Artificial Sequence
<220>
   <223> Primer
<400> 498
   tgcatattat atcgagccac agcatcg 27
<210> 499
   <211> 33
   <212> DNA
   <213> Artificial Sequence
<220>
   <223> Primer
<400> 499
   tgcattattt ggaactattg caactgctaa tgc 33
<210> 500
   <211> 28
   <212> DNA
   <213> Artificial Sequence
<220>
   <223> Primer

<400> 500
   tgccaagagg acagagtgag tactttga 28
<210> 501
   <211> 31
   <212> DNA
   <213> Artificial Sequence
<220>
   <223> Primer
<400> 501
   tgccggacaa ttacgattca tcgagtatta a 31
<210> 502
   <211> 33
   <212> DNA
   <213> Artificial Sequence
<220>
   <223> Primer
<400> 502
   tgccgtaatg ataggtgaag atatacaaag agt 33
<210> 503
   <211> 23
   <212> DNA
   <213> Artificial Sequence
<220>
   <223> Primer
<400> 503
   tgccgtgttg aacgtggtca aat 23
<210> 504
   <211> 33
   <212> DNA
   <213> Artificial Sequence
<220>
   <223> Primer
<400> 504
   tgcctagaag atcttaaaaa tttccgccaa ctt 33
<210> 505
   <211> 33
   <212> DNA
   <213> Artificial Sequence
<220>
   <223> Primer
<400> 505
   tgcctatctt tttgctgata tagcacatat tgc 33
<210> 506
   <211> 27
   <212> DNA
   <213> Artificial Sequence
<220>
   <223> Primer
<400> 506
   tgcctcgaag ctgaatataa ccaagtt 27
<210> 507
   <211> 22
   <212> DNA
   <213> Artificial Sequence
<220>
   <223> Primer
<400> 507
   tgcctgtagg gaatcctgct ga 22
<210> 508
   <211> 25
   <212> DNA
   <213> Artificial Sequence
<220>
   <223> Primer
<400> 508
   tgcctgttct tagtacgaga ggacc 25
<210> 509
   <211> 23
   <212> DNA
   <213> Artificial Sequence
<220>
   <223> Primer

<400> 509
   tgcgcagctc ttggtatcga gtt 23
<210> 510
   <211> 20
   <212> DNA
   <213> Artificial Sequence
<220>
   <223> Primer
<400> 510
   tgcgcggaag atgtaacggg 20
<210> 511
   <211> 30
   <212> DNA
   <213> Artificial Sequence
<220>
   <223> Primer
<400> 511
   tgcggatcgt ttggtggttg tagatgaaaa 30
<210> 512
   <211> 20
   <212> DNA
   <213> Artificial Sequence
<220>
   <223> Primer
<400> 512
   tgcgggtagg gagcttgagc 20
<210> 513
   <211> 31
   <212> DNA
   <213> Artificial Sequence
<220>
   <223> Primer
<400> 513
   tgcgtacaat acgctttatg aaattttaac a 31
<210> 514
   <211> 27
   <212> DNA
   <213> Artificial Sequence
<220>
   <223> Primer
<400> 514
   tgcgtataaa aaacacagat ggcagca 27
<210> 515
   <211> 21
   <212> DNA
   <213> Artificial Sequence
<220>
   <223> Primer
<400> 515
   tgcgtttacc gcaatgcgtg c 21
<210> 516
   <211> 29
   <212> DNA
   <213> Artificial Sequence
<220>
   <223> Primer
<400> 516
   tgctacggta ggatctcctt atcctattg 29
<210> 517
   <211> 30
   <212> DNA
   <213> Artificial Sequence
<220>
   <223> Primer

<400> 517
   tgctagtcaa tctatcattc cggttgatac 30
<210> 518
   <211> 27
   <212> DNA
   <213> Artificial Sequence
<220>
   <223> Primer
<400> 518
   tgctagttat ggtacagagt ttgcgac 27
<210> 519
   <211> 26
   <212> DNA
   <213> Artificial Sequence
<220>
   <223> Primer
<400> 519
   tgctatggtg ttaccttccc tatgca 26
<210> 520
   <211> 27
   <212> DNA
   <213> Artificial Sequence
<220>
   <223> Primer
<400> 520
   tgctcaaccc gatcctaaat tagacga 27
<210> 521
   <211> 27
   <212> DNA
   <213> Artificial Sequence
<220>
   <223> Primer
<400> 521
   tgctcaatct aaacctaaag tcgaaga 27
<210> 522
   <211> 33
   <212> DNA
   <213> Artificial Sequence
<220>
   <223> Primer
<400> 522
   tgctcgagtg attgactttg ctaaatttag aga 33
<210> 523
   <211> 24
   <212> DNA
   <213> Artificial Sequence
<220>
   <223> Primer
<400> 523
   tgctcgtaag ggtctggcgg atac 24
<210> 524
   <211> 29
   <212> DNA
   <213> Artificial Sequence
<220>
   <223> Primer
<400> 524
   tgctcgtggt gcacaagtaa cggatatta 29
<210> 525
   <211> 26
   <212> DNA
   <213> Artificial Sequence
<220>
   <223> Primer

<400> 525
   tgctgaggcc tggaccgatt atttac 26
<210> 526
   <211> 27
   <212> DNA
   <213> Artificial Sequence
<220>
   <223> Primer
<400> 526
   tgctggtaac agagccttat aggcgca 27
<210> 527
   <211> 19
   <212> DNA
   <213> Artificial Sequence
<220>
   <223> Primer
<400> 527
   tgctggtacg ggtcgagga 19
<210> 528
   <211> 31
   <212> DNA
   <213> Artificial Sequence
<220>
   <223> Primer
<400> 528
   tgctggtgaa aataacccag atgtcgtctt c 31
<210> 529
   <211> 32
   <212> DNA
   <213> Artificial Sequence
<220>
   <223> Primer
<400> 529
   tgctgtagct tatcgcgaaa tgtctttgat tt 32
<210> 530
   <211> 28
   <212> DNA
   <213> Artificial Sequence
<220>
   <223> Primer
<400> 530
   tgcttattta cctgcactcc cacaactg 28
<210> 531
   <211> 26
   <212> DNA
   <213> Artificial Sequence
<220>
   <223> Primer
<400> 531
   tgcttcagga atcaatgatg gagcag 26
<210> 532
   <211> 27
   <212> DNA
   <213> Artificial Sequence
<220>
   <223> Primer

<400> 532
   tgcttcggat ccagcagcac ttcaata 27
<210> 533
   <211> 19
   <212> DNA
   <213> Artificial Sequence
<220>
   <223> Primer
<400> 533
   tgcttctggt gctagcatt 19
<210> 534
   <211> 32
   <212> DNA
   <213> Artificial Sequence
<220>
   <223> Primer
<400> 534
   tgctttccta tggcttatcc aaatttagat cg 32
<210> 535
   <211> 29
   <212> DNA
   <213> Artificial Sequence
<220>
   <223> Primer
<400> 535
   tgcttttgat ggtgatgcag atcgtttgg 29
<210> 536
   <211> 24
   <212> DNA
   <213> Artificial Sequence
<220>
   <223> Primer
<400> 536
   tggaaagcca tgcgtctgac atct 24
<210> 537
   <211> 23
   <212> DNA
   <213> Artificial Sequence
<220>
   <223> Primer
<400> 537
   tggaaaggtg ttgcagctac tca 23
<210> 538
   <211> 31
   <212> DNA
   <213> Artificial Sequence
<220>
   <223> Primer
<400> 538
   tggaaatggc agctagaata gtagctaaaa t 31
<210> 539
   <211> 30
   <212> DNA
   <213> Artificial Sequence
<220>
   <223> Primer
<400> 539
   tggaacaaaa tagtctctcg gattttgact 30
<210> 540
   <211> 33
   <212> DNA
   <213> Artificial Sequence
<220>
   <223> Primer

<400> 540
   tggaacagga attaattctc atcctgatta tcc 33
<210> 541
   <211> 30
   <212> DNA
   <213> Artificial Sequence
<220>
   <223> Primer
<400> 541
   tggaacgtta tcaggtgccc caaaaattcg 30
<210> 542
   <211> 23
   <212> DNA
   <213> Artificial Sequence
<220>
   <223> Primer
<400> 542
   tggaactatt gcaactgcta atg 23
<210> 543
   <211> 30
   <212> DNA
   <213> Artificial Sequence
<220>
   <223> Primer
<400> 543
   tggaacttga agctctcgct cttaaagatg 30
<210> 544
   <211> 19
   <212> DNA
   <213> Artificial Sequence
<220>
   <223> Primer
<400> 544
   tggaagatct gggtcaggc 19
<210> 545
   <211> 22
   <212> DNA
   <213> Artificial Sequence
<220>
   <223> Primer
<400> 545
   tggaagtcat caagcgcctg gc 22
<210> 546
   <211> 30
   <212> DNA
   <213> Artificial Sequence
<220>
   <223> Primer
<400> 546
   tggaataaca aaacatgaag gaaaccactt 30
<210> 547
   <211> 32
   <212> DNA
   <213> Artificial Sequence
<220>
   <223> Primer
<400> 547
   tggaatgatg ataaagattt cgcagatagc ta 32
<210> 548
   <211> 29
   <212> DNA
   <213> Artificial Sequence
<220>
   <223> Primer

<400> 548
   tggacaatag acaatcactt ggatttaca 29
<210> 549
   <211> 28
   <212> DNA
   <213> Artificial Sequence
<220>
   <223> Primer
<400> 549
   tggacacata tcgtgagcaa tgaactga 28
<210> 550
   <211> 23
   <212> DNA
   <213> Artificial Sequence
<220>
   <223> Primer
<400> 550
   tggacggcat cacgattctc tac 23
<210> 551
   <211> 19
   <212> DNA
   <213> Artificial Sequence
<220>
   <223> Primer
<400> 551
   tggactcctc ggtggtcgc 19
<210> 552
   <211> 19
   <212> DNA
   <213> Artificial Sequence
<220>
   <223> Primer
<400> 552
   tggagcacgg cttctgatc 19
<210> 553
   <211> 30
   <212> DNA
   <213> Artificial Sequence
<220>
   <223> Primer
<400> 553
   tggagcttga agctatcgct cttaaagatg 30
<210> 554
   <211> 22
   <212> DNA
   <213> Artificial Sequence
<220>
   <223> Primer
<400> 554
   tggaggtgtc actccacacg aa 22
<210> 555
   <211> 25
   <212> DNA
   <213> Artificial Sequence
<220>
   <223> Primer

<400> 555
   tggaggttgt tgtatgtatg gtggt 25
<210> 556
   <211> 26
   <212> DNA
   <213> Artificial Sequence
<220>
   <223> Primer
<400> 556
   tggatattca ccgaacacta gggttg 26
<210> 557
   <211> 27
   <212> DNA
   <213> Artificial Sequence
**<220>**
   <223> Primer
<400> 557
   tggatggcat ggtgaaatgg atatgtc 27
<210> 558
   <211> 25
   <212> DNA
   <213> Artificial Sequence
<220>
   <223> Primer
<400> 558
   tggatgggga ttagcggtta caatg 25
<210> 559
   <211> 26
   <212> DNA
   <213> Artificial Sequence
<220>
   <223> Primer
<400> 559
   tggatgttaa gggtgatttt cccgaa 26
<210> 560
   <211> 23
   <212> DNA
   <213> Artificial Sequence
<220>
   <223> Primer
<400> 560
   tggattagag accctggtag tcc 23
<210> 561
   <211> 19
   <212> DNA
   <213> Artificial Sequence
<220>
   <223> Primer
<400> 561
   tggcacggcc atctccgtg 19
<210> 562
   <211> 34
   <212> DNA
   <213> Artificial Sequence
<220>
   <223> Primer
<400> 562
   tggcactctt gcctttaata ttagtaaact atca 34
<210> 563
   <211> 31
   <212> DNA
   <213> Artificial Sequence
<220>
   <223> Primer

<400> 563
   tggcagctag aatagtagct aaaatcccta c 31
<210> 564
   <211> 28
   <212> DNA
   <213> Artificial Sequence
<220>
   <223> Primer
<400> 564
   tggcagtttt acaaggtgct gtttcatc 28
<210> 565
   <211> 31
   <212> DNA
   <213> Artificial Sequence
<220>
   <223> Primer
<400> 565
   tggcatttct tatgaagctt gttctttagc a 31
<210> 566
   <211> 24
   <212> DNA
   <213> Artificial Sequence
<220>
   <223> Primer
<400> 566
   tggccagcgc ttcggtgaaa tgga 24
<210> 567
   <211> 21
   <212> DNA
   <213> Artificial Sequence
<220>
   <223> Primer
<400> 567
   tggcccgaaa gaagctgagc g 21
<210> 568
   <211> 32
   <212> DNA
   <213> Artificial Sequence
<220>
   <223> Primer
<400> 568
   tggcctaatg ggcttaatat caatgaaaat tg 32
<210> 569
   <211> 22
   <212> DNA
   <213> Artificial Sequence
<220>
   <223> Primer
<400> 569
   tggcgaacct ggtgaacgaa gc 22
<210> 570
   <211> 26
   <212> DNA
   <213> Artificial Sequence
<220>
   <223> Primer
<400> 570
   tggcgagtgg atagggtata atacag 26
<210> 571
   <211> 27
   <212> DNA
   <213> Artificial Sequence
<220>
   <223> Primer
<400> 571
   tggcgtagta gagctattta cagacac 27
<210> 572
   <211> 26
   <212> DNA
   <213> Artificial Sequence
<220>
   <223> Primer

<400> 572
   tggcaagtgg atagggtata atacag 26
<210> 573
   <211> 25
   <212> DNA
   <213> Artificial Sequence
<220>
   <223> Primer
<400> 573
   tggctccttg gtatgactct gcttc 25
<210> 574
   <211> 25
   <212> DNA
   <213> Artificial Sequence
<220>
   <223> Primer
<400> 574
   tggctgacat cctacatgac tgtga 25
<210> 575
   <211> 31
   <212> DNA
   <213> Artificial Sequence
<220>
   <223> Primer
<400> 575
   tggcttatcc aaatttagat cgtggtttta c 31
<210> 576
   <211> 30
   <212> DNA
   <213> Artificial Sequence
<220>
   <223> Primer
<400> 576
   tgggacttga agctatcgct cttaaagatg 30
<210> 577
   <211> 30
   <212> DNA
   <213> Artificial Sequence
<220>
   <223> Primer
<400> 577
   tgggatgaaa aagcgttctt ttatccatga 30
<210> 578
   <211> 34
   <212> DNA
   <213> Artificial Sequence
<220>
   <223> Primer
<400> 578
   tgggattatt gttatcctgt tatgccattt gaga 34
<210> 579
   <211> 32
   <212> DNA
   <213> Artificial Sequence
<220>
   <223> Primer
<400> 579
   tgggatttta aaaaacattg gtaacatcgc ag 32
<210> 580
   <211> 30
   <212> DNA
   <213> Artificial Sequence
<220>
   <223> Primer

<400> 580
   tgggcaacag cagcggattg cgattgcgcg 30
<210> 581
   <211> 24
   <212> DNA
   <213> Artificial Sequence
<220>
   <223> Primer
<400> 581
   tgggcagcgt ttcggcgaaa tgga 24
<210> 582
   <211> 33
   <212> DNA
   <213> Artificial Sequence
<220>
   <223> Primer
<400> 582
   tgggcctaat gggcttaata tcaatgaaaa ttg 33
<210> 583
   <211> 28
   <212> DNA
   <213> Artificial Sequence
<220>
   <223> Primer
<400> 583
   tgggcgatgc tgcgaaatgg ttaaaaga 28
<210> 584
   <211> 27
   <212> DNA
   <213> Artificial Sequence
<220>
   <223> Primer
<400> 584
   tgggcgtgag caatgaactg attatac 27
<210> 585
   <211> 18
   <212> DNA
   <213> Artificial Sequence
<220>
   <223> Primer
<400> 585
   tgggcgtgga acgtccac 18
<210> 586
   <211> 24
   <212> DNA
   <213> Artificial Sequence
<220>
   <223> Primer
<400> 586
   tgggctcttt ctcgcttaaa cacc 24
<210> 587
   <211> 25
   <212> DNA
   <213> Artificial Sequence
<220>
   <223> Primer
<400> 587
   tgggctcttt ctcgcttaaa cacct 25
<210> 588
   <211> 31
   <212> DNA
   <213> Artificial Sequence
<220>
   <223> Primer

<400> 588
   tggggattca gccatcaaag cagctattga c 31
<210> 589
   <211> 28
   <212> DNA
   <213> Artificial Sequence
<220>
   <223> Primer
<400> 589
   tggggattga tatcaccgat aagaagaa 28
<210> 590
   <211> 33
   <212> DNA
   <213> Artificial Sequence
<220>
   <223> Primer
<400> 590
   tggggcttta aatattccaa ttgaagattt tca 33
<210> 591
   <211> 33
   <212> DNA
   <213> Artificial Sequence
<220>
   <223> Primer
<400> 591
   tggggctttg ctttatagtt ttttacattt aag 33
<210> 592
   <211> 28
   <212> DNA
   <213> Artificial Sequence
<220>
   <223> Primer
   220>
<221> modified base
   <222> (5) ... (5)
   <223> I
<220>
   <221> modified base
   <222> (14)...(14)
   <223> I
<400> 592
   tgggtgatgc tgctaaatgg ttaaaaga 28
<210> 593
   <211> 35
   <212> DNA
   <213> Artificial Sequence
<220>
   <223> Primer
<400> 593
   tgggtcgtgg ttttacagaa aatttcttat atatg 35
<210> 594
   <211> 28
   <212> DNA
   <213> Artificial Sequence
<220>
   <223> Primer
<400> 594
   tgggtgacat tcatcaattt catcgttc 28
<210> 595
   <211> 32
   <212> DNA
   <213> Artificial Sequence
<220>
   <223> Primer
<400> 595
   tgggtttaca catatcgtga gcaatgaact ga 32
<210> 596
   <211> 25
   <212> DNA
   <213> Artificial Sequence
<220>
   <223> Primer

<400> 596
   tggtaaatac cacccacatg gtgac 25
<210> 597
   <211> 24
   <212> DNA
   <213> Artificial Sequence
<220>
   <223> Primer
<400> 597
   tggtaacaga gccttatagg cgca 24
<210> 598
   <211> 28
   <212> DNA
   <213> Artificial Sequence
<220>
   <223> Primer
<400> 598
   tggtaacaga gccttatagg cgcatatg 28
<210> 599
   <211> 30
   <212> DNA
   <213> Artificial Sequence
<220>
   <223> Primer
<400> 599
   tggtaagagc gcaccggtaa gttggtaaca 30
<210> 600
   <211> 18
   <212> DNA
   <213> Artificial Sequence
<220>
   <223> Primer
<400> 600
   tggtacagag tttgcgac 18
<210> 601
   <211> 26
   <212> DNA
   <213> Artificial Sequence
<220>
   <223> Primer
<400> 601
   tggtacatgt gccttcattg atgctg 26
<210> 602
   <211> 18
   <212> DNA
   <213> Artificial Sequence
<220>
   <223> Primer
<400> 602
   tggtacagag tttgcgac 18
<210> 603
   <211> 24
   <212> DNA
   <213> Artificial Sequence
<220>
   <223> Primer

<400> 603
   tggtactcac ttagcgggtt tccg 24
<210> 604
   <211> 24
   <212> DNA
   <213> Artificial Sequence
<220>
   <223> Primer
<400> 604
   tggtatgata tgatgcctgc acca 24
<210> 605
   <211> 21
   <212> DNA
   <213> Artificial Sequence
<220>
   <223> Primer
<400> 605
   tggtatgcgt ggtctgatgg c 21
<210> 606
   <211> 31
   <212> DNA
   <213> Artificial Sequence
<220>
   <223> Primer
<400> 606
   tggtattcta ttttgctgat aatgacctcg c 31
<210> 607
   <211> 25
   <212> DNA
   <213> Artificial Sequence
<220>
   <223> Primer
<400> 607
   tggtcaaatc aaagttggtg aagaa 25
<210> 608
   <211> 29
   <212> DNA
   <213> Artificial Sequence
<220>
   <223> Primer
<400> 608
   tggtcttatg ccaagaggac agagtgagt 29
<210> 609
   <211> 24
   <212> DNA
   <213> Artificial Sequence
<220>
   <223> Primer
<400> 609
   tggtgactcg gcatgttatg aagc 24
<210> 610
   <211> 30
   <212> DNA
   <213> Artificial Sequence
<220>
   <223> Primer
<400> 610
   tggtgacttc ataatggatg aagttgaagt 30
<210> 611
   <211> 27
   <212> DNA
   <213> Artificial Sequence
<220>
   <223> Primer
<400> 611
   tggtgcgagt gcttatgctc gtattat 27
<210> 612
   <211> 13
   <212> DNA
   <213> Artificial Sequence
<220>
   <223> Primer

<400> 612
   tggtgctagc att 13
<220> 613
   <211> 24
   <212> DNA
   <213> Artificial Sequence
<220>
   <223> Primer
<400> 613
   tggtgctttc tggcgcttaa acga 24
<210> 614
   <211> 27
   <212> DNA
   <213> Artificial Sequence
<220>
   <223> Primer
<400> 614
   tggtggacat ttaacacatg gtgcaaa 27
<210> 615
   <211> 26
   <222> DNA
   <213> Artificial Sequence
<220>
   <223> Primer
<400> 615
   tggtggtgaa atagatagga ctgctt 26
<210> 616
   <211> 13
   <212> DNA
   <213> Artificial Sequence
<220>
   <223> Primer
<400> 616
   tggtgctagc att 13
<210> 617
   <211> 26
   <212> DNA
   <213> Artificial Sequence
<220>
   <223> Primer
<400> 617
   tggttatcgc tcaggcgaac tccaac 26
<210> 618
   <211> 33
   <212> DNA
   <213> Artificial Sequence
<220>
   <223> Primer
<400> 618
   tggttatgta ccaaatactt tgtctgaaga tgg 33
<210> 619
   <211> 31
   <212> DNA
   <213> Artificial Sequence
<220>
   <223> Primer
<400> 619
   tggtttagat aattccttag gatctatgcg t 31
<210> 620
   <211> 31
   <212> DNA
   <213> Artificial Sequence
<220>
   <223> Primer
<220>
   <221> modified base
   <222> (3)...(3)
   <223> I
<220>
   <221> modified_base
   <222> (6) ... (6)
   <223> I
<220>
   <221> modified_base
   <222> (9)...(9)
   <223> I
<220>
   <221> modified_base
   <222> (12)...(13)
   <223> I
<220>
   <221> modified_base
   <222> (15)...(15)
   <223> I
<400> 620
   tgtcctactg tttgtggttc tgtaatgaac c 31
<210> 621
   <211> 24
   <212> DNA
   <213> Artificial Sequence
<220>
   <223> Primer

<400> 621
   tgtaactatc acccgcacgg tgat 24
<210> 622
   <211> 30
   <212> DNA
   <213> Artificial Sequence
<220>
   <223> Primer
<400> 622
   tgtaagctct acaacccaca aaaccttacg 30
<210> 623
   <211> 30
   <212> DNA
   <213> Artificial Sequence
<220>
   <223> Primer
<400> 623
   tgtaatgaac cctaatgacc atccacacgg 30
<210> 624
   <211> 31
   <212> DNA
   <213> Artificial Sequence
<220>
   <223> Primer
<400> 624
   tgtacccgct gaattaacga atttatacga c 31
<210> 625
   <211> 24
   <212> DNA
   <213> Artificial Sequence
<220>
   <223> Primer
<400> 625
   tgtactcggt aagtatcacc cgca 24
<210> 626
   <211> 21
   <212> DNA
   <213> Artificial Sequence
<220>
   <223> Primer
<400> 626
   tgtactgcta tccaccctca a 21
<210> 627
   <211> 24
   <212> DNA
   <213> Artificial Sequence
<220>
   <223> Primer
<400> 627
   tgtagccgct aagcactacc atcc 24
<210> 628
   <211> 30
   <212> DNA
   <213> Artificial Sequence
<220>
   <223> Primer
<400> 628
   tgtagcttat cgcgaaatgt ctttgatttt 30
<210> 629
   <211> 33
   <212> DNA
   <213> Artificial Sequence
<220>
   <223> Primer
<400> 629
   tgtatggtgg tgtaacgtta catgataata atc 33
<210> 630
   <211> 27
   <212> DNA
   <213> Artificial Sequence
<220>
   <223> Primer
<400> 630
   tgtattaggg gcatacagtc ctcatcc 27
<210> 631
   <211> 24
   <212> DNA
   <213> Artificial Sequence
<220>
   <223> Primer

<400> 631
   tgtcaaagtg gcacgtttac tggc 24
<210> 632
   <211> 24
   <212> DNA
   <213> Artificial Sequence
<220>
   <223> Primer
<400> 632
   tgtcatgggt aaatatcacc ctca 24
<210> 633
   <211> 28
   <212> DNA
   <213> Artificial Sequence
<220>
   <223> Primer
<400> 633
   tgtccaagaa gcatagcaaa aaaagcaa 28
<210> 634
   <211> 23
   <212> DNA
   <213> Artificial Sequence
<220>
   <223> Primer
<400> 634
   tgtcgatgca acgcgaagaa cct 23
<210> 635
   <211> 25
   <212> DNA
   <213> Artificial Sequence
<220>
   <223> Primer
<400> 635
   tgtcggtaca cgatattctt cacga 25
<210> 636
   <211> 27
   <212> DNA
   <213> Artificial Sequence
<220>
   <223> Primer
<400> 636
   tgtgaataaa tcacgattga ttgagca 27
<210> 637
   <211> 26
   <212> DNA
   <213> Artificial Sequence
<220>
   <223> Primer
<400> 637
   tgtggagtaa cactgcatga aaacaa 26
<210> 638
   <211> 27
   <212> DNA
   <213> Artificial Sequence
<220>
   <223> Primer
<400> 638
   tgtggtcaaa tcaaagttgg tgaagaa 27
<210> 639
   <211> 25
   <212> DNA
   <213> Artificial Sequence
<220>
   <223> Primer
<400> 639
   tgttcaagag ctagatcttc aggca 25
<210> 640
   <211> 26
   <212> DNA
   <213> Artificial Sequence
<220>
   <223> Primer

<400> 640
   tgttcaagag ctagatcttc aggcaa 26
<210> 641
   <211> 28
   <212> DNA
   <213> Artificial Sequence
<220>
   <223> Primer
<400> 641
   tgttcgctgt ttcacaaaca acattcca 28
<210> 642
   <211> 32
   <212> DNA
   <213> Artificial Sequence
<220>
   <223> Primer
<400> 642
   tgttctttag caggacttca caaacttgat aa 32
<210> 643
   <211> 29
   <212> DNA
   <213> Artificial Sequence
<220>
   <223> Primer
<400> 643
   tgttgaacgt ggtcaaatca aagttggtg 29
<210> 644
   <211> 31
   <212> DNA
   <213> Artificial Sequence
<220>
   <223> Primer
<400> 644
   tgttgggagt attccttacc atttaagcac a 31
<210> 645
   <211> 23
   <212> DNA
   <213> Artificial Sequence
<220>
   <223> Primer
<400> 645
   tgttggtgct ttctggcgct taa 23
<210> 646
   <211> 38
   <212> DNA
   <213> Artificial Sequence
<220>
   <223> Primer
<400> 646
   tccttgttgt cctactgttt gtggttctgt aatgaacc 38
<210> 647
   <211> 29
   <212> DNA
   <213> Artificial Sequence
<220>
   <223> Primer
<220>
   <221> modified_base
   <222> (7) ... (7)
   <223> I
<220>
   <221> modified_base
   <222> (10)...(10)
   <223> I
<220>
   <221> modified_base
   <222> (13)...(13)
   <223> I
<220>
   <221> modified_base
   <222> (16) ... (16)
   <223> I
<220>
   <221> modified_base
   <222> (19)...(20)
   <223> I
<220>
   <221> modified_base
   <222> (22)...(22)
   <223> I

<400> 647
   ttaaagttgg ttttattggt tggcgcgga 29
<210> 648
   <211> 30
   <212> DNA
   <213> Artificial Sequence
<220>
   <223> Primer
<400> 648
   ttaacatgaa ggaaaccact ttgataatgg 30
<210> 649
   <211> 26
   <212> DNA
   <213> Artificial Sequence
<220>
   <223> Primer
<400> 649
   ttaacggtta tcatggccca gatggg 26
<210> 650
   <211> 22
   <212> DNA
   <213> Artificial Sequence
<220>
   <223> Primer
<400> 650
   ttaagtcccg caacgagcgc aa 22
<210> 651
   <211> 22
   <212> DNA
   <213> Artificial Sequence
<220>
   <223> Primer
<400> 651
   ttaagtcccg caacgatcgc aa 22
<210> 652
   <211> 28
   <212> DNA
   <213> Artificial Sequence
<220>
   <223> Primer
<400> 652
   ttaatttgcc aaaaatgcaa ccaggtag 28
<210> 653
   <211> 27
   <212> DNA
   <213> Artificial Sequence
<220>
   <223> Primer
<400> 653
   ttacacatat cgtgagcaat gaactga 27
<210> 654
   <211> 31
   <212> DNA
   <213> Artificial Sequence
<220>
   <223> Primer
<400> 654
   ttacaggaag tttaggtggt aatctaaaag g 31
<210> 655
   <211> 30
   <212> DNA
   <213> Artificial Sequence
<220>
   <223> Primer

<400> 655
   ttactccatt attgcttggt tacactttcc 30
<210> 656
   <211> 35
   <212> DNA
   <213> Artificial Sequence
<220>
   <223> Primer
<400> 656
   ttataactta ctgcaatcta ttcagttgct tggtg 35
<210> 657
   <211> 26
   <212> DNA
   <213> Artificial Sequence
<220>
   <223> Primer
<400> 657
   ttataccgga aacttcccga aaggag 26
<210> 658
   <211> 32
   <212> DNA
   <213> Artificial Sequence
<220>
   <223> Primer
<400> 658
   ttatcagcta gaccttttag gtaaagctaa gc 32
<210> 659
   <211> 23
   <212> DNA
   <213> Artificial Sequence
<220>
   <223> Primer
<400> 659
   ttatcgctca ggcgaactcc aac 23
<210> 660
   <211> 28
   <212> DNA
   <213> Artificial Sequence
<220>
   <223> Primer
<400> 660
   ttatcgtttg tggagctagt gcttatgc 28
<210> 661
   <211> 30
   <212> DNA
   <213> Artificial Sequence
<220>
   <223> Primer
<400> 661
   ttatgaagcg tgttctttag caggacttca 30
<210> 662
   <211> 25
   <212> DNA
   <213> Artificial Sequence
<220>
   <223> Primer
<400> 662
   ttatggatgg caacgtgaaa cgcgt 25
<210> 663
   <211> 21
   <212> DNA
   <213> Artificial Sequence
<220>
   <223> Primer
<400> 663
   ttattgttat cctgttatgc c 21
<210> 664
   <211> 25
   <212> DNA
   <213> Artificial Sequence
<220>
   <223> Primer

<400> 664
   ttatttacct gcactcccac aactg 25
<210> 665
   <211> 33
   <212> DNA
   <213> Artificial Sequence
<220>
   <223> Primer
<400> 665
   ttcaaaaact ccaggccatc ctgaaatttc aac 33
<210> 666
   <211> 28
   <212> DNA
   <213> Artificial Sequence
<220>
   <223> Primer
<400> 666
   ttcaacaggt accaatgatt tgatctca 28
<210> 667
   <211> 28
   <212> DNA
   <213> Artificial Sequence
<220>
   <223> Primer
<400> 667
   ttcccaccga tatcatggct taccacgg 28
<210> 668
   <211> 25
   <212> DNA
   <213> Artificial Sequence
<220>
   <223> Primer
<400> 668
   ttccgtaagt cggctaaaac agtcg 25
<210> 669
   <211> 24
   <212> DNA
   <213> Artificial Sequence
<220>
   <223> Primer
<400> 669
   ttcctccttt tgaaagcgac ggtt 24
<210> 670
   <211> 17
   <212> DNA
   <213> Artificial Sequence
<220>
   <223> Primer
<400> 670
   ttcctcggcc gcctggc 17
<210> 671
   <211> 29
   <212> DNA
   <213> Artificial Sequence
<220>
   <223> Primer
<400> 671
   ttcctgaccg acccattatt ccctttatc 29
<210> 672
   <211> 22
   <212> DNA
   <213> Artificial Sequence
<220>
   <223> Primer

<400> 672
   ttcgatgcaa cgcgaagaac ct 22
<210> 673
   <211> 27
   <212> DNA
   <213> Artificial Sequence
<220>
   <223> Primer
<400> 673
   ttcgccaatc aaaactaagg gaatggc 27
<210> 674
   <211> 20
   <212> DNA
   <213> Artificial Sequence
<220>
   <223> Primer
<400> 674
   ttcggcggtc agcgcttcgg 20
<210> 675
   <211> 26
   <212> DNA
   <213> Artificial Sequence
<220>
   <223> Primer
<400> 675
   ttctaaaaca ccaggtcacc cagaag 26
<210> 676
   <211> 27
   <212> DNA
   <213> Artificial Sequence
<220>
   <223> Primer
<400> 676
   ttctatctcg ttggtttatt cggagtt 27
<210> 677
   <211> 30
   <212> DNA
   <213> Artificial Sequence
<220>
   <223> Primer
<400> 677
   ttctgaatgt ctatatggag gtacaacact 30
<210> 678
   <211> 21
   <212> DNA
   <213> Artificial Sequence
<220>
   <223> Primer
<400> 678
   ttgactgccc aggtcacgct g 21
<210> 679
   <211> 22
   <212> DNA
   <213> Artificial Sequence
<220>
   <223> Primer
<400> 679
   ttgactgcgg cacaacacgg at 22
<210> 680
   <211> 33
   <212> DNA
   <213> Artificial Sequence
<220>
   <223> Primer
<400> 680
   ttgagaagac atccggctca cgttattatg gta 33
<210> 681
   <211> 31
   <212> DNA
   <213> Artificial Sequence
<220>
   <223> Primer

<400> 681
   ttgagggtat gcaccgtctt tttgattctt t 31
<210> 682
   <211> 24
   <212> DNA
   <213> Artificial Sequence
<220>
   <223> Primer
<400> 682
   ttgcaactgc tgatttagct caga 24
<210> 683
   <211> 22
   <212> DNA
   <213> Artificial Sequence
<220>
   <223> Primer
<400> 683
   ttgcacaagc aaggcgctat tt 22
<210> 684
   <211> 28
   <212> DNA
   <213> Artificial Sequence
<220>
   <223> Primer
<400> 684
   ttgccaatga tattcgttgg ttagcaag 28
<210> 685
   <211> 31
   <212> DNA
   <213> Artificial Sequence
<220>
   <223> Primer
<400> 685
   ttgcccgcgg tgcggaagta accgatatta c 31
<210> 686
   <211> 23
   <212> DNA
   <213> Artificial Sequence
<220>
   <223> Primer
<400> 686
   ttgcgaatag aacgatggct cgt 23
<210> 687
   <211> 30
   <212> DNA
   <213> Artificial Sequence
<220>
   <223> Primer
<400> 687
   ttgctcgtgg tgcacaagta acggatatta 30
<210> 688
   <211> 31
   <212> DNA
   <213> Artificial Sequence
<220>
   <223> Primer
<400> 688
   ttgctcgtgg tgcacaagta acggatatta c 31
<210> 689
   <211> 31
   <212> DNA
   <213> Artificial Sequence
<220>
   <223> Primer
<220>
   <221> modified_base
   <222> (15)...(15)
   <223> I
<220>
   <221> modified_base
   <222> (29) ... (29)
   <223> I

<400> 689
   ttgctcgtgg tgcacaagta acggatatta c 31
<210> 690
   <211> 29
   <212> DNA
   <213> Artificial Sequence
<220>
   <223> Primer
<400> 690
   ttgcttaaag ttggttttat tggttggcg 29
<210> 691
   <211> 32
   <212> DNA
   <213> Artificial Sequence
<220>
   <223> Primer
<400> 691
   ttggtccttt ttatacgaaa gaagaagttg aa 32
<210> 692
   <211> 25
   <212> DNA
   <213> Artificial Sequence
<220>
   <223> Primer
<400> 692
   ttgtaaatgc cggtgcttca gatcc 25
<210> 693
   <211> 22
   <212> DNA
   <213> Artificial Sequence
<220>
   <223> Primer
<400> 693
   ttgtacacac cgcccgtcat ac 22
<210> 694
   <211> 31
   <212> DNA
   <213> Artificial Sequence
<220>
   <223> Primer
<400> 694
   ttgtagcaca gcaaggcaaa tttcctgaaa c 31
<210> 695
   <211> 30
   <212> DNA
   <213> Artificial Sequence
<220>
   <223> Primer
<400> 695
   ttgtatgtat ggtggtgtaa cgttacatga 30
<210> 696
   <211> 27
   <212> DNA
   <213> Artificial Sequence
<220>
   <223> Primer
<400> 696
   ttgtatgtat ggtggtgtaa ctgagca 27
<210> 697
   <211> 23
   <212> DNA
   <213> Artificial Sequence
<220>
   <223> Primer
<400> 697
   tttaagtccc gcaacgagcg caa 23
<210> 698
   <211> 28
   <212> DNA
   <213> Artificial Sequence
<220>
   <223> Primer
<400> 698
   tttacacata tcgtgagcaa tgaactga 28
<210> 699
   <211> 30
   <212> DNA
   <213> Artificial Sequence
<220>
   <223> Primer

<400> 699
   tttacactac ttttattcat tgccctaacg 30
<210> 700
   <211> 19
   <212> DNA
   <213> Artificial Sequence
<220>
   <223> Primer
<400> 700
   tttacagctt tatgcaccg 19
<210> 701
   <211> 29
   <212> DNA
   <213> Artificial Sequence
<220>
   <223> Primer
<400> 701
   tttcacacag cgtgtttata gttctacca 29
<210> 702
   <211> 30
   <212> DNA
   <213> Artificial Sequence
<220>
   <223> Primer
<400> 702
   tttcacatgt aattttgata ttcgcactga 30
<210> 703
   <211> 28
   <212> DNA
   <213> Artificial Sequence
<220>
   <223> Primer
<400> 703
   tttcatctta tcgaggaccc gaaatcga 28
<210> 704
   <211> 25
   <212> DNA
   <213> Artificial Sequence
<220>
   <223> Primer
<400> 704
   tttcctcctt ttgaaagcga cggtt 25
<210> 705
   <211> 22
   <212> DNA
   <213> Artificial Sequence
<220>
   <223> Primer
<400> 705
   tttcgaaggg cctttcgacc tg 22
<210> 706
   <211> 23
   <212> DNA
   <213> Artificial Sequence
<220>
   <223> Primer
<400> 706
   tttcgatgca acgcgaagaa cct 23
<210> 707
   <211> 27
   <212> DNA
   <213> Artificial Sequence
<220>
   <223> Primer
<400> 707
   tttgatttta cgccgtcctc caggtcg 27
<210> 708
   <211> 32
   <212> DNA
   <213> Artificial Sequence
<220>
   <223> Primer

<400> 708
   tttgcggatg aagtaggtgc ctatcttttt gc 32
<210> 709
   <211> 32
   <212> DNA
   <213> Artificial Sequence
<220>
   <223> Primer
<400> 709
   ttttatgctt aaagttggtt ttattggttg gc 32
<210> 710
   <211> 24
   <212> DNA
   <213> Artificial Sequence
<220>
   <223> Primer
<400> 710
   ttttgaaggt gatccgtgcc aacg 24
<210> 711
   <211> 29
   <212> DNA
   <213> Artificial Sequence
<220>
   <223> Primer
<400> 711
   aaactatttt tttagctata ctcgaacac 29
<210> 712
   <211> 19
   <212> DNA
   <213> Artificial Sequence
<220>
   <223> Primer
<400> 712
   aacatagcct tctccgtcc 19
<210> 713
   <211> 21
   <212> DNA
   <213> Artificial Sequence
<220>
   <223> Primer
<400> 713
   aacttcgcct tcggtcatgt t 21
<210> 714
   <211> 17
   <212> DNA
   <213> Artificial Sequence
<220>
   <223> Primer
<400> 714
   aaggaggtga tccagcc 17
<210> 715
   <211> 29
   <212> DNA
   <213> Artificial Sequence
<220>
   <223> Primer
<400> 715
   aatcgacgac catcttggaa agatttctc 29
<210> 716
   <211> 21
   <212> DNA
   <213> Artificial Sequence
<220>
   <223> Primer
<400> 716
   acaaaaggca cgccatcacc c 21
<210> 717
   <211> 21
   <212> DNA
   <213> Artificial Sequence
<220>
   <223> Primer
<400> 717
   acaaaaggta cgccgtcacc c 21
<210> 718
   <211> 18
   <212> DNA
   <213> Artificial Sequence
<220>
   <223> Primer
<220>
   <221> modified_base
   <222> (12)...(12)
   <223> I
<400> 718
   acaacacgag ctgacgac 18
<210> 719
   <211> 18
   <212> DNA
   <213> Artificial Sequence
<220>
   <223> Primer

<400> 719
   acaacacgag ctgacgac 18
<210> 720
   <211> 18
   <212> DNA
   <213> Artificial Sequence
<220>
   <223> Primer
<220>
   <221> modified_base
   <222> (16)...(16)
   <223> I
<400> 720
   acaacacgag ctgacgac 18
<210> 721
   <211> 18
   <212> DNA
   <213> Artificial Sequence
<220>
   <223> Primer
<220>
   <221> modified_base
   <222> (14) ... (14)
   <223> I
<400> 721
   acaacacgag ctgacgac 18
<210> 722
   <211> 18
   <212> DNA
   <213> Artificial Sequence
<220>
   <223> Primer
<220>
   <221> modified_base
   <222> (11) ... (11)
   <223> I
<400> 722
   acaacacgag ctgacgac 18
<210> 723
   <211> 18
   <212> DNA
   <213> Artificial Sequence
<220>
   <223> Primer
<220>
   <221> modified base
   <222> (10)...(10)
   <223> I
<220>
   <221> modified_base
   <222> (13) ... (13)
   <223> I
<400> 723
   acaacacgag ctgacgac 18
<210> 724
   <211> 18
   <212> DNA
   <213> Artificial Sequence
<220>
   <223> Primer
<220>
   <221> modified_base
   <222> (8)...(8)
   <223> I
<220>
   <221> modified_base
   <222> (10)...(10)
   <223> I
<220>
   <221> modified_base
   <222> (13)...(13)
   <223> I

<400> 724
   acaacacgag ctgacgac 18
<210> 725
   <211> 18
   <212> DNA
   <213> Artificial Sequence
<220>
   <223> Primer
<220>
   <221> modified_base
   <222> (8) ... (8)
   <223> I
<220>
   <221> modified_base
   <222> (10)...(10)
   <223> I
<220>
   <221> modified_base
   <222> (13) ... (13)
   <223> I
<220>
   <221> modified_base
   <222> (16)...(16)
   <223> I
<400> 725
   acaacacgag ctgacgac 18
<210> 726
   <211> 20
   <212> DNA
   <213> Artificial Sequence
<220>
   <223> Primer
<400> 726
   acaaccatgc accacctgtc 20
<210> 727
   <211> 12
   <212> DNA
   <213> Artificial Sequence
<220>
   <223> Primer
<400> 727
   acacgagctg ac 12
<210> 728
   <211> 28
   <212> DNA
   <213> Artificial Sequence
<220>
   <223> Primer
<400> 728
   accactttta ataaggtttg tagctaac 28
<210> 729
   <211> 27
   <212> DNA
   <213> Artificial Sequence
<220>
   <223> Primer

<400> 729
   acctgcaata tctaatgcac tcttacg 27
<210> 730
   <211> 24
   <212> DNA
   <213> Artificial Sequence
<220>
   <223> Primer
<400> 730
   acctgcatcc ctaaacgtac ttgc 24
<210> 731
   <211> 22
   <212> DNA
   <213> Artificial Sequence
<220>
   <223> Primer
<400> 731
   accttgttac gacttcaccc ca 22
<210> 732
   <211> 20
   <212> DNA
   <213> Artificial Sequence
<220>
   <223> Primer
<400> 732
   acgaactgga tgtcgccgtt 20
<210> 733
   <211> 18
   <212> DNA
   <213> Artificial Sequence
<220>
   <223> Primer
<400> 733
   acgacacgag ctgacgac 18
<210> 734
   <211> 18
   <212> DNA
   <213> Artificial Sequence
<220>
   <223> Primer
<400> 734
   acgacacgag ctgacgac 18
<210> 735
   <211> 20
   <212> DNA
   <213> Artificial Sequence
<220>
   <223> Primer
<400> 735
   acgagctgac gacagccatg 20
<210> 736
   <211> 19
   <212> DNA
   <213> Artificial Sequence
<220>
   <223> Primer
<400> 736
   acgccatcag gccacgcat 19
<210> 737
   <211> 21
   <212> DNA
   <213> Artificial Sequence
<220>
   <223> Primer
<400> 737
   acgcgggcat gcagagatgc c 21
<210> 738
   <211> 18
   <212> DNA
   <213> Artificial Sequence
<220>
   <223> Primer

<400> 738
   acggcacgag gtagtcgc 18
<210> 739
   <211> 20
   <212> DNA
   <213> Artificial Sequence
<220>
   <223> Primer
<400> 739
   acggttacct tgttacgact 20
<210> 740
   <211> 20
   <212> DNA
   <213> Artificial Sequence
<220>
   <223> Primer
<400> 740
   acgtccttca tcgcctctga 20
<210> 741
   <211> 27
   <212> DNA
   <213> Artificial Sequence
<220>
   <223> Primer
<400> 741
   acgtttttcg ttttgaacga taatgct 27
<210> 742
   <211> 17
   <212> DNA
   <213> Artificial Sequence
<220>
   <223> Primer
<400> 742
   actgctgcct cccgtag 17
<210> 743
   <211> 20
   <212> DNA
   <213> Artificial Sequence
<220>
   <223> Primer
<400> 743
   acttagatgc tttcagcggt 20
<210> 744
   <211> 20
   <212> DNA
   <213> Artificial Sequence
<220>
   <223> Primer
<400> 744
   agacctcctg cgtgcaaagc 20
<210> 745
   <211> 31
   <212> DNA
   <213> Artificial Sequence
<220>
   <223> Primer
<400> 745
   agataaagaa tcacgaatat caatttgtag c 31
<210> 746
   <211> 22
   <212> DNA
   <213> Artificial Sequence
<220>
   <223> Primer
<400> 746
   agccgacatc gaggtgccaa ac 22
<210> 747
   <211> 28
   <212> DNA
   <213> Artificial Sequence
<220>
   <223> Primer
<400> 747
   agctgctaga tgagcttctg ccatggcc 28
<210> 748
   <211> 23
   <212> DNA
   <213> Artificial Sequence
<220>
   <223> Primer

<400> 748
   aggatagatt tatttcttgt tcg 23
<210> 749
   <211> 20
   <212> DNA
   <213> Artificial Sequence
<220>
   <223> Primer
<400> 749
   agtccatccc ggtcctctcg 20
<210> 750
   <211> 22
   <212> DNA
   <213> Artificial Sequence
<220>
   <223> Primer
<400> 750
   ataagccatg ttctgttcca tc 22
<210> 751
   <211> 18
   <212> DNA
   <213> Artificial Sequence
<220>
   <223> Primer
<400> 751
   ataagccggg ttctgtcg 18
<210> 752
   <211> 26
   <212> DNA
   <213> Artificial Sequence
<220>
   <223> Primer
<400> 752
   atatgattat cattgaactg cggccg 26
<210> 753
   <211> 19
   <212> DNA
   <213> Artificial Sequence
<220>
   <223> Primer
<400> 753
   atcccctgct tctgctgcc 19
<210> 754
   <211> 30
   <212> DNA
   <213> Artificial Sequence
<220>
   <223> Primer
<400> 754
   attcaagagc catttctttt ggtaaaccac 30
<210> 755
   <211> 22
   <212> DNA
   <213> Artificial Sequence
<220>
   <223> Primer
<400> 755
   attgcccaga aatcaaatca tc 22
<210> 756
   <211> 33
   <212> DNA
   <213> Artificial Sequence
<220>
   <223> Primer

<400> 756
   attgcttctt acttgcttag cataaatttt cca 33
<210> 757
   <211> 21
   <212> DNA
   <213> Artificial Sequence
<220>
   <223> Primer
<400> 757
   attgtagcac gtgtgtagcc c 21
<210> 758
   <211> 24
   <212> DNA
   <213> Artificial Sequence
<220>
   <223> Primer
<400> 758
   caagcggttt gcctcaaata gtca 24
<210> 759
   <211> 22
   <212> DNA
   <213> Artificial Sequence
<220>
   <223> Primer
<400> 759
   caatctgctg acggatctga gc 22
<210> 760
   <211> 15
   <212> DNA
   <213> Artificial Sequence
<220>
   <223> Primer
<400> 760 15
   caccgggcag gcgtc 15
<210> 761
   <211> 20
   <212> DNA
   <213> Artificial Sequence
<220>
   <223> Primer
<400> 761 20
   cacggctacc ttgttacgac 20
<210> 762
   <211> 20
   <212> DNA
   <213> Artificial Sequence
<220>
   <223> Primer
<400> 762 20
   cagataaaga atcgctccag 20
<210> 763
   <211> 25
   <212> DNA
   <213> Artificial Sequence
<220>
   <223> Primer
<400> 763 25
   catgacagcc aagacctcac ccacc 25
<210> 764
   <211> 18
   <212> DNA
   <213> Artificial Sequence
<220>
   <223> Primer
<400> 764 18
   catgatggtc acaaccgg 18
<210> 765
   <211> 20
   <212> DNA
   <213> Artificial Sequence
<220>
   <223> Primer
<400> 765 20
   ccaaacaccg ccgtcgatat 20
<210> 766
   <211> 26
   <212> DNA
   <213> Artificial Sequence
<220>
   <223> Primer
<400> 766
   ccaacctttt ccacaacaga atcagc 26
<210> 767
   <211> 27
   <212> DNA
   <213> Artificial Sequence
<220>
   <223> Primer

<400> 767
   ccaagtgctg gtttacccca tggagta 27
<210> 768
   <211> 23
   <212> DNA
   <213> Artificial Sequence
<220>
   <223> Primer
<400> 768
   ccacttttaa taaggtttgt agc 23
<210> 769
   <211> 27
   <212> DNA
   <213> Artificial Sequence
<220>
   <223> Primer
<400> 769
   ccagcagtta ctgtcccctc atctttg 27
<210> 770
   <211> 28
   <212> DNA
   <213> Artificial Sequence
<220>
   <223> Primer
<400> 770
   ccataaggtc accgtcacca ttcaaagc 28
<210> 771
   <211> 18
   <212> DNA
   <213> Artificial Sequence
<220>
   <223> Primer
<400> 771
   ccatgcagca cctgtctc 18
<210> 772
   <211> 29
   <212> DNA
   <213> Artificial Sequence
<220>
   <223> Primer
<400> 772
   cccatttttt cacgcatgct gaaaatatc 29
<210> 773
   <211> 21
   <212> DNA
   <213> Artificial Sequence
<220>
   <223> Primer
<400> 773
   cccccgtcaa ttcctttgag t 21
<210> 774
   <211> 24
   <212> DNA
   <213> Artificial Sequence
<220>
   <223> Primer
<400> 774
   ccctgtagta gaagaggtaa ccac 24
<210> 775
   <211> 21
   <212> DNA
   <213> Artificial Sequence
<220>
   <223> Primer

<400> 775
   ccgacaagga atttcgctac c 21
<210> 776
   <211> 23
   <212> DNA
   <213> Artificial Sequence
<220>
   <223> Primer
<400> 776
   ccgcggtcga attgcatgcc ttc 23
<210> 777
   <211> 17
   <212> DNA
   <213> Artificial Sequence
<220>
   <223> Primer
<400> 777
   ccggtcctct cgtacta 17
<210> 778
   <211> 18
   <212> DNA
   <213> Artificial Sequence
<220>
   <223> Primer
<400> 778
   ccgtgctcca tttttcag 18
<210> 779
   <211> 25
   <212> DNA
   <213> Artificial Sequence
<220>
   <223> Primer
<400> 779
   cctacccaac gttcaccaag ggcag 25
<210> 780
   <211> 17
   <212> DNA
   <213> Artificial Sequence
<220>
   <223> Primer
<400> 780
   cctcctgcgt gcaaagc 17
<210> 781
   <211> 20
   <212> DNA
   <213> Artificial Sequence
<220>
   <223> Primer
<400> 781
   cctgtagtag aagaggtaac 20
<210> 782
   <211> 18
   <212> DNA
   <213> Artificial Sequence
<220>
   <223> Primer
<400> 782
   ccttctcccg aagttacg 18
<210> 783
   <211> 20
   <212> DNA
   <213> Artificial Sequence
<220>
   <223> Primer
<400> 783
   ccttgttacg acttcacccc 20
<210> 784
   <211> 24
   <212> DNA
   <213> Artificial Sequence
<220>
   <223> Primer

<400> 784
   cgaacggcca gagtagtcaa cacg 24
<210> 785
   <211> 24
   <212> DNA
   <213> Artificial Sequence
<220>
   <223> Primer
<400> 785
   cgaacggcct gagtagtcaa cacg 24
<210> 786
   <211> 24
   <212> DNA
   <213> Artificial Sequence
<220>
   <223> Primer
<400> 786
   cgacttgacg gttaacattt cctg 24
<210> 787
   <211> 21
   <212> DNA
   <213> Artificial Sequence
<220>
   <223> Primer
<400> 787
   cgagttgcag actgcgatcc g 21
<210> 788
   <211> 21
   <212> DNA
   <213> Artificial Sequence
<220>
   <223> Primer
<400> 788
   cgagttgcag actgcgatcc g 21
<210> 789
   <211> 25
   <212> DNA
   <213> Artificial Sequence
<220>
   <223> Primer
<400> 789
   cgcaccatgc gtagagatga agtac 25
<210> 790
   <211> 25
   <212> DNA
   <213> Artificial Sequence
<220>
   <223> Primer
<400> 790
   cgcaccgtgg gttgagatga agtac 25
<210> 791
   <211> 18
   <212> DNA
   <213> Artificial Sequence
<220>
   <223> Primer

<400> 791
   cgcatttcac cgctacac 18
<210> 792
   <211> 20
   <212> DNA
   <213> Artificial Sequence
<220>
   <223> Primer
<400> 792
   cgcggtcggc tcgttgatga 20
<210> 793
   <211> 21
   <212> DNA
   <213> Artificial Sequence
<220>
   <223> Primer
<400> 793
   cggctgctgg cacgaagtta g 21
<210> 794
   <211> 15
   <212> DNA
   <213> Artificial Sequence
<220>
   <223> Primer
<400> 794
   cggcttcaag acccc 15
<210> 795
   <211> 24
   <212> DNA
   <213> Artificial Sequence
<220>
   <223> Primer
<400> 795
   cggtacgaac tggatgtcgc cgtt 24
<210> 796
   <211> 15
   <212> DNA
   <213> Artificial Sequence
<220>
   <223> Primer
<400> 796
   cgtactcccc aggcg 15
<210> 797
   <211> 30
   <212> DNA
   <213> Artificial Sequence
<220>
   <223> Primer
<400> 797
   cgtataagct gcaccataag cttgtaatgc 30
<210> 798
   <211> 21
   <212> DNA
   <213> Artificial Sequence
<220>
   <223> Primer
<400> 798
   cgtggactac cagggtatct a 21
<210> 799
   <211> 21
   <212> DNA
   <213> Artificial Sequence
<220>
   <223> Primer
<400> 799
   ctatcggtca gtcaggagta t 21
<210> 800
   <211> 22
   <212> DNA
   <213> Artificial Sequence
<220>
   <223> Primer

<400> 800
   cttctacatt tttagccatc ac 22
<210> 801
   <211> 20
   <212> DNA
   <213> Artificial Sequence
<220>
   <223> Primer
<400> 801
   ctttacgccc agtaattccg 20
<210> 802
   <211> 25
   <212> DNA
   <213> Artificial Sequence
<220>
   <223> Primer
<400> 802
   ctttcgcttt ctcgaactca accat 25
<210> 803
   <211> 17
   <212> DNA
   <213> Artificial Sequence
<220>
   <223> Primer
<400> 803
   gaatatcaat ttgtagc 17
<210> 804
   <211> 27
   <212> DNA
   <213> Artificial Sequence
<220>
   <223> Primer
<400> 804
   gaccccaacc tggccttttg tcgttga 27
<210> 805
   <211> 19
   <212> DNA
   <213> Artificial Sequence
<220>
   <223> Primer
<400> 805
   gaccgttata gttacggcc 19
<210> 806
   <211> 19
   <212> DNA
   <213> Artificial Sequence
<220>
   <223> Primer
<400> 806
   gacgggcggt gtgtacaag 19
<210> 807
   <211> 19
   <212> DNA
   <213> Artificial Sequence
<220>
   <223> Primer
<400> 807
   gacgggcggt gtgtacaag 19
<210> 808
   <211> 19
   <212> DNA
   <213> Artificial Sequence
<220>
   <223> Primer
<400> 808
   gacgggcggt gtgtacaag 19
<210> 809
   <211> 21
   <212> DNA
   <213> Artificial Sequence
<220>
   <223> Primer

<400> 809
   gacgtcatcc ccaccttcct c 21
<210> 810
   <211> 22
   <212> DNA
   <213> Artificial Sequence
<220>
   <223> Primer
<400> 810
   gacgtcatcc ccaccttcct cc 22
<210> 811
   <211> 19
   <212> DNA
   <213> Artificial Sequence
<220>
   <223> Primer
<400> 811
   gagcatcagc gtgcgtgct 19
<210> 812
   <211> 25
   <212> DNA
   <213> Artificial Sequence
<220>
   <223> Primer
<400> 812
   gagctgcgcc aacgaataaa tcgtc 25
<210> 813
   <211> 29
   <212> DNA
   <213> Artificial Sequence
<220>
   <223> Primer
<400> 813
   gattggcgat aaagtgatat tttctaaaa 29
<210> 814
   <211> 26
   <212> DNA
   <213> Artificial Sequence
<220>
   <223> Primer
<400> 814
   gcccaccaga aagactagca ggataa 26
<210> 815
   <211> 21
   <212> DNA
   <213> Artificial Sequence
<220>
   <223> Primer
<400> 815
   gccgtccatc tgagcagcac c 21
<210> 816
   <211> 21
   <212> DNA
   <213> Artificial Sequence
<220>
   <223> Primer
<400> 816
   gccgtccatt tgagcagcac c 21
<210> 817
   <211> 19
   <212> DNA
   <213> Artificial Sequence
<220>
   <223> Primer

<400> 817
   gccttgcgac cgtactccc 19
<210> 818
   <211> 18
   <212> DNA
   <213> Artificial Sequence
<220>
   <223> Primer
<400> 818
   gcgaccgtac tccccagg 18
<210> 819
   <211> 19
   <212> DNA
   <213> Artificial Sequence
<220>
   <223> Primer
<400> 819
   gcgctccacg tcttcacgc 19
<210> 820
   <211> 19
   <212> DNA
   <213> Artificial Sequence
<220>
   <223> Primer
<400> 820
   gcgtgacagg caggtattc 19
<210> 821
   <211> 28
   <212> DNA
   <213> Artificial Sequence
<220>
   <223>.Primer
<400> 821
   gcgtgacgac cttcttgaat tgtaatca 28
<210> 822
   <211> 24
   <212> DNA
   <213> Artificial Sequence
<220>
   <223> Primer
<400> 822
   gcgttccaca gcttgttgca gaag 24
<210> 823
   <211> 18
   <212> DNA
   <213> Artificial Sequence
<220>
   <223> Primer
<400> 823
   gctgctggca cggagtta 18
<210> 824
   <211> 26
   <212> DNA
   <213> Artificial Sequence
<220>
   <223> Primer

<400> 824
   gctgctttga tggctgaatc cccttc 26
<210> 825
   <211> 21
   <212> DNA
   <213> Artificial Sequence
<220>
   <223> Primer
<400> 825
   gctggattcg cctttgctac g 21
<210> 826
   <211> 20
   <212> DNA
   <213> Artificial Sequence
<220>
   <223> Primer
<400> 826
   gcttacacac ccggcctatc 20
<210> 827
   <211> 23
   <212> DNA
   <213> Artificial Sequence
<220>
   <223> Primer
<400> 827
   ggaatttacc agcgatagac acc 23
<210> 828
   <211> 30
   <212> DNA
   <213> Artificial Sequence
<220>
   <223> Primer
<400> 828
   ggataattgg tcgtaacaag ggatagtgag 30
<210> 829
   <211> 25
   <212> DNA
   <213> Artificial Sequence
<220>
   <223> Primer
<400> 829
   ggcatcacca tttccttgtc cttcg 25
<210> 830
   <211> 18
   <212> DNA
   <213> Artificial Sequence
<220>
   <223> Primer
<400> 830
   ggccgtactc cccaggcg 18
<210> 831
   <211> 20
   <212> DNA
   <213> Artificial Sequence
<220>
   <223> Primer
<400> 831
   ggcgcttgta cttaccgcac 20
<210> 832
   <211> 25
   <212> DNA
   <213> Artificial Sequence
<220>
   <223> Primer

<400> 832
   gggtctacac ctgcacttgc ataac 25
<210> 833
   <211> 16
   <212> DNA
   <213> Artificial Sequence
<220>
   <223> Primer
<400> 833
   gggtttcccc attcgg 16
<210> 834
   <211> 20
   <212> DNA
   <213> Artificial Sequence
<220>
   <223> Primer
<400> 834
   ggtaaccctt gtctttgaat 20
<210> 835
   <211> 19
   <212> DNA
   <213> Artificial Sequence
<220>
   <223> Primer
<400> 835
   ggtaaggttc ttcgcgttg 19
<210> 836
   <211> 28
   <212> DNA
   <213> Artificial Sequence
<220>
   <223> Primer
<400> 836
   ggtataacgc atcgcagcaa aagattta 28
<210> 837
   <211> 28
   <212> DNA
   <213> Artificial Sequence
<220>
   <223> Primer
<400> 837
   gtaacccttg tctttgaatt gtatttgc 28
<210> 838
   <211> 22
   <212> DNA
   <213> Artificial Sequence
<220>
   <223> Primer
<400> 838
   gtaagccatg ttttgttcca tc 22
<210> 839
   <211> 22
   <212> DNA
   <213> Artificial Sequence
<220>
   <223> Primer
<400> 839
   gtatctaatc ctgtttgctc cc 22
<210> 840
   <211> 27
   <212> DNA
   <213> Artificial Sequence
<220>
   <223> Primer
<400> 840
   gtccgacttg acggtcaaca tttcctg 27
<210> 841
   <211> 18
   <212> DNA
   <213> Artificial Sequence
<220>
   <223> Primer

<400> 841
   gtgcgccctt tctaactt 18
<210> 842
   <211> 22
   <212> DNA
   <213> Artificial Sequence
<220>
   <223> Primer
<400> 842
   gtgctggttt accccatgga gt 22
<210> 843
   <211> 21
   <212> DNA
   <213> Artificial Sequence
<220>
   <223> Primer
<400> 843
   gttcaaatgc ctggataccc a 21
<210> 844
   <211> 24
   <212> DNA
   <213> Artificial Sequence
<220>
   <223> Primer
<400> 844
   gttgtcacca ggcattacca tttc 24
<210> 845
   <211> 24
   <212> DNA
   <213> Artificial Sequence
<220>
   <223> Primer
<400> 845
   gttgtcgcca ggcataacca tttc 24
<210> 846
   <211> 24
   <212> DNA
   <213> Artificial Sequence
<220>
   <223> Primer
<400> 846
   gtttcatgct tagatgcttt cagc 24
<210> 847
   <211> 25
   <212> DNA
   <213> Artificial Sequence
<220>
   <223> Primer
<400> 847
   gtttttcgtt gcgtacgatg atgtc 25
<210> 848
   <211> 27
   <212> DNA
   <213> Artificial Sequence
<220>
   <223> Primer
<400> 848
   taaacgtccg ataccaatgg ttcgctc 27
<210> 849
   <211> 30
   <212> DNA
   <213> Artificial Sequence
<220>
   <223> Primer
<400> 849
   taaactattt ttttagctat actcgaacac 30
<210> 850
   <211> 28
   <212> DNA
   <213> Artificial Sequence
<220>
   <223> Primer

<400> 850
   taaagacacc gctgggttta aatgtgca 28
<210> 851
   <211> 27
   <212> DNA
   <213> Artificial Sequence
<220>
   <223> Primer
<400> 851
   taaagagacg tttggtagtt catttgc 27
<210> 852
   <211> 33
   <212> DNA
   <213> Artificial Sequence
<220>
   <223> Primer
<400> 852
   taaaggatag cggtaactaa atggctgagc cat 33
<210> 853
   <211> 26
   <212> DNA
   <213> Artificial Sequence
<220>
   <223> Primer
<400> 853
   taaatgcact tgcttcaggg ccatat 26
<210> 854
   <211> 31
   <212> DNA
   <213> Artificial Sequence
<220>
   <223> Primer
<400> 854
   taaattccgc aaagactttg gcattaggtg t 31
<210> 855
   <211> 29
   <212> DNA
   <213> Artificial Sequence
<220>
   <223> Primer
<400> 855
   taacaaatcc cgtctgagtt cctcttgca 29
<210> 856
   <211> 28
   <212> DNA
   <213> Artificial Sequence
<220>
   <223> Primer
<400> 856
   taacaacgtt accttcgcga tccactaa 28
<210> 857
   <211> 23
   <212> DNA
   <213> Artificial Sequence
<220>
   <223> Primer

<400> 857
   taaccatttc gcgtaagatt caa 23
<210> 858
   <211> 29
   <212> DNA
   <213> Artificial Sequence
<220>
   <223> Primer
<400> 858
   taaccacccc aagatttatc tttttgcca 29
<210> 859
   <211> 23
   <212> DNA
   <213> Artificial Sequence
<220>
   <223> Primer
<400> 859
   taaccatttc gcgtaagatt caa 23
<210> 860
   <211> 39
   <212> DNA
   <213> Artificial Sequence
<220>
   <223> Primer
<400> 860
   taacccttgt ctttgaattg tatttgcaat taatcctgg 39
<210> 861
   <211> 29
   <212> DNA
   <213> Artificial Sequence
<220>
   <223> Primer
<400> 861
   taaccgtttc caaaggtact gtattttgt 29
<210> 862
   <211> 34
   <212> DNA
   <213> Artificial Sequence
<220>
   <223> Primer
<400> 862
   taaccgtttc caaaggtact gtattttgtt tacc 34
<210> 863
   <211> 26
   <212> DNA
   <213> Artificial Sequence
<220>
   <223> Primer
<400> 863
   taactcctct tccttcaaca ggtgga 26
<210> 864
   <211> 29
   <212> DNA
   <213> Artificial Sequence
<220>
   <223> Primer
<400> 864
   taactgaccc aaagctgaaa gctttactg 29
<210> 865
   <211> 33
   <212> DNA
   <213> Artificial Sequence
<220>
   <223> Primer

<400> 865
   taagacaagg ttttgtggat tttttagctt gtt 33
<210> 866
   <211> 26
   <212> DNA
   <213> Artificial Sequence
<220>
   <223> Primer
<400> 866
   taagagtgat gcgggctggt tcaaca 26
<210> 867
   <211> 25
   <212> DNA
   <213> Artificial Sequence
<220>
   <223> Primer
<400> 867
   taagcaatac ctttacttgc accac 25
<210> 868
   <211> 27
   <212> DNA
   <213> Artificial Sequence
<220>
   <223> Primer
<400> 868
   taagcaatac ctttacttgc accacct 27
<210> 869
   <211> 28
   <212> DNA
   <213> Artificial Sequence
<220>
   <223> Primer
<400> 869
   taagcaatac ctttacttgc accacctg 28
<210> 870
   <211> 31
   <212> DNA
   <213> Artificial Sequence
<220>
   <223> Primer
<400> 870
   taagcaccat ataagtctac ttttttccct t 31
<210> 871
   <211> 27
   <212> DNA
   <213> Artificial Sequence
<220>
   <223> Primer
<400> 871
   taagccagca agagctgtat agttcca 27
<210> 872
   <211> 26
   <212> DNA
   <213> Artificial Sequence
<220>
   <223> Primer

<400> 872
   taagctcccg tatcttgagt cgcctc 26
<210> 873
   <211> 23
   <212> DNA
   <213> Artificial Sequence
<220>
   <223> Primer
<400> 873
   taagttacct tgcccgtcaa cca 23
<210> 874
   <211> 27
   <212> DNA
   <213> Artificial Sequence
<220>
   <223> Primer
<400> 874
   taagttcctt cgctagtatg ttggctt 27
<210> 875
   <211> 30
   <212> DNA
   <213> Artificial Sequence
<220>
   <223> Primer
<400> 875
   taatcgacga ccatcttgga aagatttctc 30
<210> 876
   <211> 23
   <212> DNA
   <213> Artificial Sequence
<220>
   <223> Primer
<400> 876
   taatctggct gcggaagtga aat 23
<210> 877
   <211> 25
   <212> DNA
   <213> Artificial Sequence
<220>
   <223> Primer
<400> 877
   taatctggct gcggaagtga aatcg 25
<210> 878
   <211> 31
   <212> DNA
   <213> Artificial Sequence
<220>
   <223> Primer

<400> 878
   taatgccggg tagtgcaatc cattcttcta g 31
<210> 879
   <211> 24
   <212> DNA
   <213> Artificial Sequence
<220>
   <223> Primer
<400> 879
   taatgcgata ctggcctgca agtc 24
<210> 880
   <211> 33
   <212> DNA
   <213> Artificial Sequence
<220>
   <223> Primer
<400> 880
   tacaaccttc ggataatcag gatgagaatt aat 33
<210> 881
   <211> 27
   <212> DNA
   <213> Artificial Sequence
<220>
   <223> Primer
<400> 881
   tacaacgtga taaacacgac cagaagc 27
<210> 882
   <211> 35
   <212> DNA
   <213> Artificial Sequence
<220>
   <223> Primer
<400> 882
   tacaactggt tcaaaaacat taagctgtaa ttgtc 35
<210> 883
   <211> 32
   <212> DNA
   <213> Artificial Sequence
<220>
   <223> Primer
<400> 883
   tacagcttta aagccagcaa aatgaattac ag 32
<210> 884
   <211> 22
   <212> DNA
   <213> Artificial Sequence
<220>
   <223> Primer
<400> 884
   tacaggagca gcaggcttca ag 22
<210> 885
   <211> 26
   <212> DNA
   <213> Artificial Sequence
<220>
   <223> Primer
<400> 885
   tacatcgttt cgcccaagat caatca 26
<210> 886
   <211> 33
   <212> DNA
   <213> Artificial Sequence
<220>
   <223> Primer
<400> 886
   tacatctcct tcgatagaaa tttcattgct atc 33
<210> 887
   <211> 25
   <212> DNA
   <213> Artificial Sequence
<220>
   <223> Primer

<400> 887
   taccaaagcg tgcacgatag ttgag 25
<210> 888
   <211> 27
   <212> DNA
   <213> Artificial Sequence
<220>
   <223> Primer
<400> 888
   taccatctac ccaaacatta gcaccaa 27
<210> 889
   <211> 22
   <212> DNA
   <213> Artificial Sequence
<220>
   <223> Primer
<400> 889
   taccccagtt cccctgacct tc 22
<210> 890
   <211> 27
   <212> DNA
   <213> Artificial Sequence
<220>
   <223> Primer
<400> 890
   taccggaagc accagcgaca ttaatag 27
<210> 891
   <211> 27
   <212> DNA
   <213> Artificial Sequence
<220>
   <223> Primer
<400> 891
   tacctgcatt aatcgcttgt tcatcaa 27
<210> 892
   <211> 22
   <212> DNA
   <213> Artificial Sequence
<220>
   <223> Primer
<400> 892
   taccttaccg ccaaagctgt ct 22
<210> 893
   <211> 24
   <212> DNA
   <213> Artificial Sequence
<220>
   <223> Primer
<400> 893
   taccttagga ccgttatagt tacg 24
<210> 894
   <211> 24
   <212> DNA
   <213> Artificial Sequence
<220>
   <223> Primer
<400> 894
   taccttttcc acaacagaat cagc 24
<210> 895
   <211> 21
   <212> DNA
   <213> Artificial Sequence
<220>
   <223> Primer

<400> 895
   tacgagctga cgacagccat g 21
<210> 896
   <211> 23
   <212> DNA
   <213> Artificial Sequence
<220>
   <223> Primer
<400> 896
   tacgagctga cgacagccat gca 23
<210> 897
   <211> 22
   <212> DNA
   <213> Artificial Sequence
<220>
   <223> Primer
<400> 897
   tacgcattac tcacccgtcc gc 22
<210> 898
   <211> 20
   <212> DNA
   <213> Artificial Sequence
<220>
   <223> Primer
<400> 898
   tacgccatca ggccacgcat 20
<210> 899
   <211> 27
   <212> DNA
   <213> Artificial Sequence
<220>
   <223> Primer
<400> 899
   tacgctaagc cacgtccata tttatca 27
<210> 900
   <211> 35
   <212> DNA
   <213> Artificial Sequence
<220>
   <223> Primer
<400> 900
   tacgtatgta aattccgcaa agactttggc attag 35
<210> 901
   <211> 30
   <212> DNA
   <213> Artificial Sequence
<220>
   <223> Primer
<400> 901
   tacgtcgcct ttaacttggt tatattcagc 30
<210> 902
   <211> 31
   <212> DNA
   <213> Artificial Sequence
<220>
   <223> Primer
<400> 902
   tacgttctac gatttcttca tcaggtacat c 31
<210> 903
   <211> 24
   <212> DNA
   <213> Artificial Sequence
<220>
   <223> Primer

<400> 903
   tacgtttgta tcttctgcag aacc 24
<210> 904
   <211> 29
   <212> DNA
   <213> Artificial Sequence
<220>
   <223> Primer
<400> 904
   tacacctggt ttcgttttga tgatttgta 29
<210> 905
   <211> 24
   <212> DNA
   <213> Artificial Sequence
<220>
   <223> Primer
<400> 905
   tactagacga cgggtcaggt aacc 24
<210> 906
   <211> 32
   <212> DNA
   <213> Artificial Sequence
<220>
   <223> Primer
<400> 906
   tacttcagct tcgtccaata aaaaatcaca at 32
<210> 907
   <211> 31
   <212> DNA
   <213> Artificial Sequence
<220>
   <223> Primer
<400> 907
   tactttaagg ggctatcttt accatgaacc t 31
<210> 908
   <211> 27
   <212> DNA
   <213> Artificial Sequence
<220>
   <223> Primer
<400> 908
   tagagagtag ccatcttcac cgttgtc 27
<210> 909
   <211> 23
   <212> DNA
   <213> Artificial Sequence
<220>
   <223> Primer
<400> 909
   tagcaccaat caccctttcc tgt 23
<210> 910
   <211> 28
   <212> DNA
   <213> Artificial Sequence
<220>
   <223> Primer
<400> 910
   tagcagcaaa agttatcaca cctgcagt 28
<210> 911
   <211> 25
   <212> DNA
   <213> Artificial Sequence
<220>
   <223> Primer

<400> 911
   tagcagctag ctcgtaacca gtgta 25
<210> 912
   <211> 31
   <212> DNA
   <213> Artificial Sequence
<220>
   <223> Primer
<400> 912
   tagccatacg taccattgct tcataaatag a 31
<210> 913
   <211> 22
   <212> DNA
   <213> Artificial Sequence
<220>
   <223> Primer
<400> 913
   tagcccagct gtttgagcaa ct 22
<210> 914
   <211> 20
   <212> DNA
   <213> Artificial Sequence
<220>
   <223> Primer
<400> 914
   tagccgcggt cgaattgcat 20
<210> 915
   <211> 25
   <212> DNA
   <213> Artificial Sequence
<220>
   <223> Primer
<400> 915
   tagccttggc aacatcagca aaact 25
<210> 916
   <211> 24
   <212> DNA
   <213> Artificial Sequence
<220>
   <223> Primer
<400> 916
   tagccttttc tccggcgtag atct 24
<210> 917
   <211> 34
   <212> DNA
   <213> Artificial Sequence
<220>
   <223> Primer
<400> 917
   tagcgatttc tactcctaga gttgaaattt cagg 34
<210> 918
   <211> 29
   <212> DNA
   <213> Artificial Sequence
<220>
   <223> Primer
<400> 918
   tagctgctag atgagcttct gccatggcc 29
<210> 919
   <211> 23
   <212> DNA
   <213> Artificial Sequence
<220>
   <223> Primer
<400> 919
   taggatgaaa gcattccgct ggc 23
<210> 920
   <211> 28
   <212> DNA
   <213> Artificial Sequence
<220>
   <223> Primer

<400> 920
   taggatgagc attatcaggg aaagaatc 28
<210> 921
   <211> 23
   <212> DNA
   <213> Artificial Sequence
<220>
   <223> Primer
<400> 921
   taggattttt ccacggcggc atc 23
<210> 922
   <211> 31
   <212> DNA
   <213> Artificial Sequence
<220>
   <223> Primer
<400> 922
   taggcataac catttcagta ccttctggta a 31
<210> 923
   <211> 28
   <212> DNA
   <213> Artificial Sequence
<220>
   <223> Primer
<400> 923
   tagtatcacc acgtacaccc ggatcagt 28
<210> 924
   <211> 28
   <212> DNA
   <213> Artificial Sequence
<220>
   <223> Primer
<220>
   <221> modified_base
   <222> (17) ... (17)
   <223> I
<220>
   <221> modified_base
   <222> (20) ... (20)
   <223> I
<400> 924
   tagtatcacc acgtacaccc ggatcagt 28
<210> 925
   <211> 32
   <212> DNA
   <213> Artificial Sequence
<220>
   <223> Primer
<400> 925
   tagtcctttc tgaattttac catcaaaggt ac 32
<210> 926
   <211> 32
   <212> DNA
   <213> Artificial Sequence
<220>
   <223> Primer
<400> 926
   tagtcttttg gaacaccgtc tttaattaaa gt 32
<210> 927
   <211> 30
   <212> DNA
   <213> Artificial Sequence
<220>
   <223> Primer

<400> 927
   tagtgttgta cctccatata gacattcaga 30
<210> 928
   <211> 29
   <212> DNA
   <213> Artificial Sequence
<220>
   <223> Primer
<400> 928
   tagttgaagt tgcactatat actgttgga 29
<210> 929
   <211> 23
   <212> DNA
   <213> Artificial Sequence
<220>
   <223> Primer
<400> 929
   tataacgcac atcgtcaggg tga 23
<210> 930
   <211> 26
   <212> DNA
   <213> Artificial Sequence
<220>
   <223> Primer
<400> 930
   tatagcacca tccatctgag cggcac 26
<210> 931
   <211> 29
   <212> DNA
   <213> Artificial Sequence
<220>
   <223> Primer
<400> 931
   tatatgaaca ataccagttc cttctgagt 29
<210> 932
   <211> 27
   <212> DNA
   <213> Artificial Sequence
<220>
   <223> Primer
<400> 932
   tatatgatta tcattgaact gcggccg 27
<210> 933
   <211> 28
   <212> DNA
   <213> Artificial Sequence
<220>
   <223> Primer
<400> 933
   tatccattga accaaagtta ccttggcc 28
<210> 934
   <211> 20
   <212> DNA
   <213> Artificial Sequence
<220>
   <223> Primer
<400> 934
   tatcccctgc ttctgctgcc 20
<210> 935
   <211> 28
   <212> DNA
   <213> Artificial Sequence
<220>
   <223> Primer
<400> 935
   tatcgacaga tccaaagtta ccatgccc 28
<210> 936
   <211> 27
   <212> DNA
   <213> Artificial Sequence
<220>
   <223> Primer

<400> 936
   tatggtctat ttcaatggca gttacga 27
<210> 937
   <211> 24
   <212> DNA
   <213> Artificial Sequence
<220>
   <223> Primer
<400> 937
   tatgtgctca cgagtttgcg gcat 24
<210> 938
   <211> 28
   <212> DNA
   <213> Artificial Sequence
<220>
   <223> Primer
<400> 938
   tatgtgtagt tgagcttact acatgagc 28
<210> 939
   <211> 25
   <212> DNA
   <213> Artificial Sequence
<220>
   <223> Primer
<400> 939
   tattcttcgt tactcatgcc ataca 25
<210> 940
   <211> 23
   <212> DNA
   <213> Artificial Sequence
<220>
   <223> Primer
<400> 940
   tattgcccag aaatcaaatc atc 23
<210> 941
   <211> 30
   <212> DNA
   <213> Artificial Sequence
<220>
   <223> Primer
<400> 941
   tattgcggat caccatgatg atattcttgc 30
<210> 942
   <211> 31
   <212> DNA
   <213> Artificial Sequence
<220>
   <223> Primer
<400> 942
   tattgctttt tttgctatgc ttcttggaca t 31
<210> 943
   <211> 24 .
   <212> DNA
   <213> Artificial Sequence
<220>
   <223> Primer
<400> 943
   tattggaaat accggcagca tctc 24
<210> 944
   <211> 30
   <212> DNA
   <213> Artificial Sequence
<220>
   <223> Primer
<400> 944
   tatttgggtt tcattccact cagattctgg 30
<210> 945
   <211> 32
   <212> DNA
   <213> Artificial Sequence
<220>
   <223> Primer
<400> 945
   tcaaaaacaa agaattcatt ttctggtcca aa 32
<210> 946
   <211> 34
   <212> DNA
   <213> Artificial Sequence
<220>
   <223> Primer
<400> 946
   tcaaaacgca tttttacatc ttcgttaaag gcta 34
<210> 947
   <211> 29
   <212> DNA
   <213> Artificial Sequence
<220>
   <223> Primer

<400> 947
   tcaaaacttg ctctagacca tttaactcc 29
<210> 948
   <211> 30
   <212> DNA
   <213> Artificial Sequence
<220>
   <223> Primer
<400> 948
   tcaaaatctt ttgattcgat catacgagac 30
<210> 949
   <211> 27
   <212> DNA
   <213> Artificial Sequence
<220>
   <223> Primer
<400> 949
   tcaaacgatc cgcatcacca tcaaaag 27
<210> 950
   <211> 30
   <212> DNA
   <213> Artificial Sequence
<220>
   <223> Primer
<400> 950
   tcaaagaacc agcacctaat tcatcattta 30
<210> 951
   <211> 30
   <212> DNA
   <213> Artificial Sequence
<220>
   <223> Primer
<400> 951
   tcaaagaacc cgcacctaat tcatcattta 30
<210> 952
   <211> 26
   <212> DNA
   <213> Artificial Sequence
<220>
   <223> Primer
<400> 952
   tcaacaacac ctccttattc ccactc 26
<210> 953
   <211> 29
   <212> DNA
   <213> Artificial Sequence
<220>
   <223> Primer
<400> 953
   tcaacaatca gatagatgtc agacgcatg 29
<210> 954
   <211> 28
   <212> DNA
   <213> Artificial Sequence
<220>
   <223> Primer
<400> 954
   tcaacaccag cgttacctaa agtacctt 28
<210> 955
   <211> 35
   <212> DNA
   <213> Artificial Sequence
<220>
   <223> Primer

<400> 955
   tcaactggtt caaaaacatt aagttgtaat tgtcc 35
<210> 956
   <211> 28
   <212> DNA
   <213> Artificial Sequence
<220>
   <223> Primer
<400> 956
   tcaacttctg ccattaaaag taatgcca 28
<210> 957
   <211> 24
   <212> DNA
   <213> Artificial Sequence
<220>
   <223> Primer
<400> 957
   tcaagcgatc tacccgcatt acaa 24
<210> 958
   <211> 30
   <212> DNA
   <213> Artificial Sequence
<220>
   <223> Primer
<400> 958
   tcaagcgcca tctctttcgg taatccacat 30
<210> 959
   <211> 30
   <212> DNA
   <213> Artificial Sequence
<220>
   <223> Primer
<400> 959
   tcaagcgcca tttcttttgg taaaccacat 30
<210> 960
   <211> 31
   <212> DNA
   <213> Artificial Sequence
<220>
   <223> Primer
<400> 960
   tcaagctata tgctacaact ggttcaaaaa c 31
<210> 961
   <211> 30
   <212> DNA
   <213> Artificial Sequence
<220>
   <223> Primer
<400> 961
   tcaagctcta caccataaaa aaagctctca 30
<210> 962
   <211> 28
   <212> DNA
   <213> Artificial Sequence
<220>
   <223> Primer
<400> 962
   tcaaggttct caccgtttac cttaggag 28
<210> 963
   <211> 32
   <212> DNA
   <213> Artificial Sequence
<220>
   <223> Primer
<400> 963
   tcaagtgctt ttacttctat aggtttaagc tc 32
<210> 964
   <211> 30
   <212> DNA
   <213> Artificial Sequence
<220>
   <223> Primer
<400> 964
   tcaatacaga gtctacactt ggcttaggat 30
<210> 965
   <211> 25
   <212> DNA
   <213> Artificial Sequence
<220>
   <223> Primer
<400> 965
   tcaatctcga ctttttgtgc cggta 25
<210> 966
   <211> 28
   <212> DNA
   <213> Artificial Sequence
<220>
   <223> Primer

<400> 966
   tcacaaggac cattataatc aatgccaa 28
<210> 967
   <211> 23
   <212> DNA
   <213> Artificial Sequence
<220>
   <223> Primer
<400> 967
   tcacaccaag tagtgcaagg atc 23
<210> 968
   <211> 29
   <212> DNA
   <213> Artificial Sequence
<220>
   <223> Primer
<400> 968
   tcacacctgt aagtgagaaa aaggttgat 29
<210> 969
   <211> 34
   <212> DNA
   <213> Artificial Sequence
<220>
   <223> Primer
<400> 969
   tcacaggttc tacttcatca ataatttcca ttgc 34
<210> 970
   <211> 34
   <212> DNA
   <213> Artificial Sequence
<220>
   <223> Primer
<400> 970
   tcaccagctt cagcgtagtc taataattta cgga 34
<210> 971
   <211> 22
   <212> DNA
   <213> Artificial Sequence
<220>
   <223> Primer
<400> 971
   tcaccatgcg cccgttcaca ta 22
<210> 972
   <211> 37
   <212> DNA
   <213> Artificial Sequence
<220>
   <223> Primer
<400> 972
   tcaccgataa ataaaatacc taaagttaat gccattg 37
<210> 973
   <211> 29
   <212> DNA
   <213> Artificial Sequence
<220>
   <223> Primer
<400> 973
   tcacctacag ctttaaagcc agcaaaatg 29
<210> 974
   <211> 28
   <212> DNA
   <213> Artificial Sequence
<220>
   <223> Primer
<400> 974
   tcacgatacc tgcatcatca aattggtt 28
<210> 975
   <211> 32
   <212> DNA
   <213> Artificial Sequence
<220>
   <223> Primer

<400> 975
   tcacgatcta aatttggata agccatagga aa 32
<210> 976
   <211> 24
   <212> DNA
   <213> Artificial Sequence
<220>
   <223> Primer
<400> 976
   tcacgcgacg agtgccatcc attg 24
<210> 977
   <211> 22
   <212> DNA
   <213> Artificial Sequence
<220>
   <223> Primer
<400> 977
   tcacgcgcat catcaccagt ca 22
<210> 978
   <211> 18
   <212> DNA
   <213> Artificial Sequence
<220>
   <223> Primer
<400> 978
   tcacgggcca gctcgtct 18
<210> 979
   <211> 28
   <212> DNA
   <213> Artificial Sequence
<220>
   <223> Primer
<400> 979
   tcacgtcgtc cgacttcacg gtcagcat 28
<210> 980
   <211> 26
   <212> DNA
   <213> Artificial Sequence
<220>
   <223> Primer
<400> 980
   tcagaatcga tgccaaatgc gtcatc 26
<210> 981
   <211> 30
   <212> DNA
   <213> Artificial Sequence
<220>
   <223> Primer
<400> 981
   tcagatataa atggaacaaa tggagccact 30
<210> 982
   <211> 32
   <212> DNA
   <213> Artificial Sequence
<220>
   <223> Primer
<400> 982
   tcagcgtagt ctaataattt acggaacatt tc 32
<210> 983
   <211> 25
   <212> DNA
   <213> Artificial Sequence
<220>
   <223> Primer
<400> 983
   tcagctgtta acggcttcaa gaccc 25
<210> 984
   <211> 29
   <212> DNA
   <213> Artificial Sequence
<220>
   <223> Primer

<400> 984
   tcaggtatga aacacgatta gtcctttct 29
<210> 985
   <211> 29
   <212> DNA
   <213> Artificial Sequence
<220>
   <223> Primer
<400> 985
   tcagtttgca cttcaaaaga aattgtgtt 29
<210> 986
   <211> 29
   <212> DNA
   <213> Artificial Sequence
<220>
   <223> Primer
<400> 986
   tcataactag catttgtgct ttgaatgct 29
<210> 987
   <211> 31
   <212> DNA
   <213> Artificial Sequence
<220>
   <223> Primer
<400> 987
   tcataagggt tgcgttgcag attatcttta c 31
<210> 988
   <211> 26
   <212> DNA
   <213> Artificial Sequence
<220>
   <223> Primer
<400> 988
   tcatctggtt taggatctgg ttgact 26
<210> 989
   <211> 25
   <212> DNA
   <213> Artificial Sequence
<220>
   <223> Primer
<400> 989
   tcatctgtgg tatggcgggt aagtt 25
<210> 990
   <211> 26
   <212> DNA
   <213> Artificial Sequence
<220>
   <223> Primer
<400> 990
   tcatgacagc caagacctca cccacc 26
<210> 991
   <211> 31
   <212> DNA
   <213> Artificial Sequence
<220>
   <223> Primer
<400> 991
   tcatgataga actacctggt tgcatttttg g 31
<210> 992
   <211> 28
   <212> DNA
   <213> Artificial Sequence
<220>
   <223> Primer
<400> 992
   tcatgtgcta atgttactgc tggatctg 28
<210> 993
   <211> 31
   <212> DNA
   <213> Artificial Sequence
<220>
   <223> Primer
<400> 993
   tcattaggta aaatgtctgg acatgatcca a 31
<210> 994
   <211> 28
   <212> DNA
   <213> Artificial Sequence
<220>
   <223> Primer

<400> 994
   tcatttattt cttcgctttt ctcgctac 28
<210> 995
   <211> 20
   <212> DNA
   <213> Artificial Sequence
<220>
   <223> Primer
<400> 995
   tcatttgtgc tttgaatgct 20
<210> 996
   <211> 28
   <212> DNA
   <213> Artificial Sequence
<220>
   <223> Primer
<400> 996
   tccaaacgat ctgcatcacc atcaaaag 28
<210> 997
   <211> 29
   <212> DNA
   <213> Artificial Sequence
<220>
   <223> Primer
<400> 997
   tccaacccag aaccacatac tttattcac 29
<210> 998
   <211> 27
   <212> DNA
   <213> Artificial Sequence
<220>
   <223> Primer
<400> 998
   tccaaccttt tccacaacag aatcagc 27
<210> 999
   <211> 24
   <212> DNA
   <213> Artificial Sequence
<220>
   <223> Primer
<400> 999
   tccaagtgct ggtttacccc atgg 24
<210> 1000
   <211> 26
   <212> DNA
   <213> Artificial Sequence
<220>
   <223> Primer
<400> 1000
   tccaagtgct ggtttacccc atggag 26
<210> 1001
   <211> 28
   <212> DNA
   <213> Artificial Sequence
<220>
   <223> Primer
<400> 1001
   tccaagtttg acttaaacgt accatcgc 28
<210> 1002
   <211> 32
   <212> DNA
   <213> Artificial Sequence
<220>
   <223> Primer
<400> 1002
   tccacactgg attgtaattt accttgttct tt 32
<210> 1003
   <211> 26
   <212> DNA
   <213> Artificial Sequence
<220>
   <223> Primer
<400> 1003
   tccaccacct caaagaccat gtggtg 26
<210> 1004
   <211> 23
   <212> DNA
   <213> Artificial Sequence
<220>
   <223> Primer
<400> 1004
   tccagcaggt tctgacggaa acg 23
<210> 1005
   <211> 28
   <212> DNA
   <213> Artificial Sequence
<220>
   <223> Primer

<400> 1005
   tccagcagtt actgtcccct catctttg 28
<210> 1006
   <211> 30
   <212> DNA
   <213> Artificial Sequence
<220>
   <223> Primer
<400> 1006
   tccaggcatt accatttcta ctccttctgg 30
<210> 1007
   <211> 29
   <212> DNA
   <213> Artificial Sequence
<220>
   <223> Primer
<400> 1007
   tccataaggt caccgtcacc attcaaagc 29
<210> 1008
   <211> 35
   <212> DNA
   <213> Artificial Sequence
<220>
   <223> Primer
<220>
   <221> modified_base
   <222> (21) ... (21)
   <223> I
<220>
   <221> modified_base
   <222> (30)...(30)
   <223> I
<400> 1008
   tccatacctt tatgcaactt tgtatcaact ggaat 35
<210> 1009
   <211> 28
   <212> DNA
   <213> Artificial Sequence
<220>
   <223> Primer
<400> 1009
   tccatattgt tgcataaaac ctgttggc 28
<210> 1010
   <211> 27
   <212> DNA
   <213> Artificial Sequence
<220>
   <223> Primer
<400> 1010
   tccatccata gaaccaaagt taccttg 27
<210> 1011
   <211> 26
   <212> DNA
   <213> Artificial Sequence
<220>
   <223> Primer
<400> 1011
   tccatcgcag tcacgtttac tgttgg 26
<210> 1012
   <211> 32
   <212> DNA
   <213> Artificial Sequence
<220>
   <223> Primer
<400> 1012
   tccatcgcca gtttttgcat aatcgctaaa aa 32
<210> 1013
   <211> 33
   <212> DNA
   <213> Artificial Sequence
<220>
   <223> Primer

<400> 1013
   tccatctgtt aaaccatcat ataccatgct atc 33
<210> 1014
   <211> 26
   <212> DNA
   <213> Artificial Sequence
<220>
   <223> Primer
<400> 1014
   tccatttccg acacgtcgtt gatcac 26
<210> 1015
   <211> 27
   <212> DNA
   <213> Artificial Sequence
<220>
   <223> Primer
<400> 1015
   tcccaatcta acttccacat accatct 27
<210> 1016
   <211> 29
   <212> DNA
   <213> Artificial Sequence
<220>
   <223> Primer
<400> 1016
   tcccaatctt ttgattcgat catacgaga 29
<210> 1017
   <211> 27
   <212> DNA
   <213> Artificial Sequence
<220>
   <223> Primer
<400> 1017
   tcccatacct atggcgataa ctgtcat 27
<210> 1018
   <211> 30
   <212> DNA
   <213> Artificial Sequence
<220>
   <223> Primer
<400> 1018
   tcccattttt tcacgcatgc tgaaaatatc 30
<210> 1019
   <211> 15
   <212> DNA
   <213> Artificial Sequence
<220>
   <223> Primer
<400> 1019
   tccccacctt cctcc 15
<210> 1020
   <211> 25
   <212> DNA
   <213> Artificial Sequence
<220>
   <223> Primer
<400> 1020
   tccccatctc cgcaaagaca ataaa 25
<210> 1021
   <211> 33
   <212> DNA
   <213> Artificial Sequence
<220>
   <223> Primer
<400> 1021
   tccccattta ataattccac ctactatcac act 33
<210> 1022
   <211> 33
   <212> DNA
   <213> Artificial Sequence
<220>
   <223> Primer

<400> 1022
   tcccctcatg tttaaatgat caggataaaa agc 33
<210> 1023
   <211> 31
   <212> DNA
   <213> Artificial Sequence
<220>
   <223> Primer
<400> 1023
   tcccctttaa agcaccatta ctcattatag t 31
<210> 1024
   <211> 33
   <212> DNA
   <213> Artificial Sequence
<220>
   <223> Primer
<400> 1024
   tcccgaacaa tgagttgtat caactatttt tac 33
<210> 1025
   <211> 21
   <212> DNA
   <213> Artificial Sequence
<220>
   <223> Primer
<400> 1025
   tcccgctggc aaataaactc g 21
<210> 1026
   <211> 25
   <212> DNA
   <213> Artificial Sequence
<220>
   <223> Primer
<400> 1026
   tcccggctag agattctgta tacga 25
<210> 1027
   <211> 28
   <212> DNA
   <213> Artificial Sequence
<220>
   <223> Primer
<400> 1027
   tcccgtctga gttcctcttg catgatca 28
<210> 1028
   <211> 32
   <212> DNA
   <213> Artificial Sequence
<220>
   <223> Primer
<400> 1028
   tccctaatag tagaaataac tgcatcagta gc 32
<210> 1029
   <211> 34
   <212> DNA
   <213> Artificial Sequence
<220>
   <223> Primer
<400> 1029
   tcccttattt ttctttctac taccttcgga taat 34
<210> 1030
   <211> 30
   <212> DNA
   <213> Artificial Sequence
<220>
   <223> Primer
<400> 1030
   tcccttcctt aatatgagaa ggaaaccact 30
<210> 1031
   <211> 32
   <212> DNA
   <213> Artificial Sequence
<220>
   <223> Primer
<400> 1031
   tccgaaactt gttttgtagc tttaatttga gc 32
<210> 1032
   <211> 21
   <212> DNA
   <213> Artificial Sequence
<220>
   <223> Primer

<400> 1032
   tccgaagttg ccctggccgt c 21
<210> 1033
   <211> 24
   <212> DNA
   <213> Artificial Sequence
<220>
   <223> Primer
<400> 1033
   tccgagacca gcgtaggtgt aacg 24
<210> 1034
   <211> 22
   <212> DNA
   <213> Artificial Sequence
<220>
   <223> Primer
<400> 1034
   tccgataagc cggattctgt gc 22
<210> 1035
   <211> 28
   <212> DNA
   <213> Artificial Sequence
<220>
   <223> Primer
<400> 1035
   tccgcaaaga ctttggcatt aggtgtga 28
<210> 1036
   <211> 27
   <212> DNA
   <213> Artificial Sequence
<220>
   <223> Primer
<400> 1036
   tccgccaaaa actccccttt tcacagg 27
<210> 1037
   <211> 24
   <212> DNA
   <213> Artificial Sequence
<220>
   <223> Primer
<400> 1037
   tccgccttca aaatggtggc gagt 24
<210> 1038
   <211> 31
   <212> DNA
   <213> Artificial Sequence
<220>
   <223> Primer
<400> 1038
   tccggctaga gattctgtat acgaaaatat c 31
<210> 1039
   <211> 31
   <212> DNA
   <213> Artificial Sequence
<220>
   <223> Primer
<400> 1039
   tccggctaga gattctgtat acgacaatat c 31
<210> 1040
   <211> 23
   <212> DNA
   <213> Artificial Sequence
<220>
   <223> Primer
<400> 1040
   tccggtaact gggtcagctc gaa 23
<210> 1041
   <211> 34
   <212> DNA
   <213> Artificial Sequence
<220>
   <223> Primer

<400> 1041
   tccgtagttt tgcataattt atggtctatt tcaa 34
<210> 1042
   <211> 27
   <212> DNA
   <213> Artificial Sequence
<220>
   <223> Primer
<400> 1042
   tccgtcatcg ctgacagaaa ctgagtt 27
<210> 1043
   <211> 28
   <212> DNA
   <213> Artificial Sequence
<220>
   <223> Primer
<400> 1043
   tccgtctatc cacaagttaa ttggtact 28
<210> 1044
   <211> 26
   <212> DNA
   <213> Artificial Sequence
<220>
   <223> Primer
<400> 1044
   tcctacccaa cgttcaccaa gggcag 26
<210> 1045
   <211> 29
   <212> DNA
   <213> Artificial Sequence
<220>
   <223> Primer
<400> 1045
   tcctccttgt gcctcaaaac gcattttta 29
<210> 1046
   <211> 30
   <212> DNA
   <213> Artificial Sequence
<220>
   <223> Primer
<400> 1046
   tcctctatgc aacttagtat caacaggaat 30
<210> 1047
   <211> 24
   <212> DNA
   <213> Artificial Sequence
<220>
   <223> Primer
<400> 1047
   tcctcttggg ccacgcaaag tttt 24
<210> 1048
   <211> 27
   <212> DNA
   <213> Artificial Sequence
<220>
   <223> Primer
<400> 1048
   tcctcttttc acaggctcta cttcatc 27
<210> 1049
   <211> 30
   <212> DNA
   <213> Artificial Sequence
<220>
   <223> Primer
<400> 1049
   tcctgaagat ctagttcttg aatggttact 30
<210> 1050
   <211> 27
   <212> DNA
   <213> Artificial Sequence
<220>
   <223> Primer
<400> 1050
   tcctgcaata tctaatgcac tcttacg 27
<210> 1051
   <211> 25
   <212> DNA
   <213> Artificial Sequence
<220>
   <223> Primer

<400> 1051
   tcctgcagct ctacctgctc catta 25
<210> 1052
   <211> 20
   <212> DNA
   <213> Artificial Sequence
<220>
   <223> Primer
<400> 1052
   tcctggccat cctgcaggat 20
<210> 1053
   <211> 27
   <212> DNA
   <213> Artificial Sequence
<220>
   <223> Primer
<400> 1053
   tcctgtttta tagccgccaa gagtaag 27
<210> 1054
   <211> 21
   <212> DNA
   <213> Artificial Sequence
<220>
   <223> Primer
<400> 1054
   tccttcacgc gcatcatcac c 21
<210> 1055
   <211> 27
   <212> DNA
   <213> Artificial Sequence
<220>
   <223> Primer
<400> 1055
   tccttctgat gcctgatgga ccaggag 27
<210> 1056
   <211> 26
   <212> DNA
   <213> Artificial Sequence
<220>
   <223> Primer
<400> 1056
   tccttggcat acatcatgtc gtagca 26
<210> 1057
   <211> 33
   <212> DNA
   <213> Artificial Sequence
<220>
   <223> Primer
<400> 1057
   tccttgtgct tcaaaacgca tttttacatt ttc 33
<210> 1058
   <211> 28
   <212> DNA
   <213> Artificial Sequence
<220>
   <223> Primer
<400> 1058
   tcctttaaaa taaccgctag tagctcct 28
<210> 1059
   <211> 30
   <212> DNA
   <213> Artificial Sequence
<220>
   <223> Primer

<400> 1059
   tcctttatgc aacttagtat caaccggaat 30
<210> 1060
   <211> 30
   <212> DNA
   <213> Artificial Sequence
<220>
   <223> Primer
<400> 1060
   tcctttatgc aacttggtat caacaggaat 30
<210> 1061
   <211> 30
   <212> DNA
   <213> Artificial Sequence
<220>
   <223> Primer
<400> 1061
   tcctttatgc aacttggtat caaccggaat 30
<210> 1062
   <211> 29
   <212> DNA
   <213> Artificial Sequence
<220>
   <223> Primer
<400> 1062
   tcctttcaat gttacagaaa actctacag 29
<210> 1063
   <211> 24
   <212> DNA
   <213> Artificial Sequence
<220>
   <223> Primer
<400> 1063
   tcgaaccgaa gttaccctga ccat 24
<210> 1064
   <211> 30
   <212> DNA
   <213> Artificial Sequence
<220>
   <223> Primer
<400> 1064
   tcgaattcag ctaaatactt ttcagcatct 30
<210> 1065
   <211> 26
   <212> DNA
   <213> Artificial Sequence
<220>
   <223> Primer
<400> 1065
   tcgacctgga ggacgacgta aaatca 26
<210> 1066
   <211> 25
   <212 DNA
   <213> Artificial Sequence
<220>
   <223> Primer
<400> 1066
   tcgacgacca tcttggaaag atttc 25
<210> 1067
   <211> 25
   <212> DNA
   <213> Artificial Sequence
<220>
   <223> Primer
<400> 1067
   tcgagccgaa gttaccctgt ccgtc 25
<210> 1068
   <211> 27
   <212> DNA
   <213> Artificial Sequence
<220>
   <223> Primer
<400> 1068
   tcgatccgca tcaccatcaa aagcaaa 27
<210> 1069
   <211> 26
   <212> DNA
   <213> Artificial Sequence
<220>
   <223> Primer

<400> 1069
   tcgatcgaac cgaagttacc ctgacc 26
<210> 1070
   <211> 28
   <212> DNA
   <213> Artificial Sequence
<220>
   <223> Primer
<400> 1070
   tcgatcgtga ctctctttat tttcagtt 28
<210> 1071
   <211> 20
   <212> DNA
   <213> Artificial Sequence
<220>
   <223> Primer
<400> 1071
   tcgatctcct tggcgtccga 20
<210> 1072
   <211> 26
   <212> DNA
   <213> Artificial Sequence
<220>
   <223> Primer
<400> 1072
   tcgcaccgtg ggttgagatg aagtac 26
<210> 1073
   <211> 18
   <212> DNA
   <213> Artificial Sequence
<220>
   <223> Primer
<400> 1073
   tcgcagcgtg cgtggcac 18
<210> 1074
   <211> 26
   <212> DNA
   <213> Artificial Sequence
<220>
   <223> Primer
<400> 1074
   tcgcaggctt acagaacgct ctccta 26
<210> 1075
   <211> 23
   <212> DNA
   <213> Artificial Sequence
<220>
   <223> Primer
<400> 1075
   tcgcagttca tcagcacgaa gcg 23
<210> 1076
   <211> 26
   <212> DNA
   <213> Artificial Sequence
<220>
   <223> Primer
<400> 1076
   tcgccagcta gcacgatgtc attttc 26
<210> 1077
   <211> 31
   <212> DNA
   <213> Artificial Sequence
<220>
   <223> Primer
<400> 1077
   tcgccatagc taagttgttt attgtttcca t 31
<210> 1078
   <211> 22
   <212> DNA
   <213> Artificial Sequence
<220>
   <223> Primer
<400> 1078
   tcgcctggtg caggcatcat at 22
<210> 1079
   <211> 24
   <212> DNA
   <213> Artificial Sequence
<220>
   <223> Primer
<400> 1079
   tcgcgctgta tttttcctcc gaga 24
<210> 1080
   <211> 18
   <212> DNA
   <213> Artificial Sequence
<220>
   <223> Primer

<400> 1080
   tcgctacctt aggaccgt 18
<210> 1081
   <211> 29
   <212> DNA
   <213> Artificial Sequence
<220>
   <223> Primer
<400> 1081
   tcgctcagca ataattcact ataagccga 29
<210> 1082
   <211> 29
   <212> DNA
   <213> Artificial Sequence
<220>
   <223> Primer
<400> 1082
   tcgctctctc aagtgatcta aacttggag 29
<210> 1083
   <211> 25
   <212> DNA
   <213> Artificial Sequence
<220>
   <223> Primer
<400> 1083
   tcgcttgagt gtagtcatga ttgcg 25
<210> 1084
   <211> 32
   <212> DNA
   <213> Artificial Sequence
<220>
   <223> Primer
<400> 1084
   tcggaaacaa agaattcatt ttctggtcca aa 32
<210> 1085
   <211> 33
   <212> DNA
   <213> Artificial Sequence
<220>
   <223> Primer
<400> 1085
   tcggaaatat tctttcaata cctttatgca act 33
<210> 1086
   <211> 34
   <212> DNA
   <213> Artificial Sequence
<220>
   <223> Primer
<400> 1086
   tcggaaatat tctttcaata cctttatgca actt 34
<210> 1087
   <211> 34
   <212> DNA
   <213> Artificial Sequence
<220>
   <223> Primer
<220>
   <221> modified_base
   <222> (20)...(20)
   <223> I
<220>
   <221> modified base
   <222> (26) ... (26)
   <223> I
<400> 1087
   tcggaaatat tctttcaata cctttatgca actt 34
<210> 1088
   <211> 20
   <212> DNA
   <213> Artificial Sequence
<220>
   <223> Primer
<400> 1088
   tcggactcgc tttcgctacg 20
<210> 1089
   <211> 20
   <212> DNA
   <213> Artificial Sequence
<220>
   <223> Primer

<400> 1089
   tcggataagc tgccacaagg 20
<210> 1090
   <211> 19
   <212> DNA
   <213> Artificial Sequence
<220>
   <223> Primer
<400> 1090
   tcggcatcac gccgtcgtc 19
<210> 1091
   <211> 20
   <212> DNA
   <213> Artificial Sequence
<220>
   <223> Primer
<400> 1091
   tcggcgaaca tggccatcac 20
<210> 1092
   <211> 33
   <212> DNA
   <213> Artificial Sequence
<220>
   <223> Primer
<400> 1092
   tcgggcgtag tttttagtaa ttaaatcaga agt 33
<210> 1093
   <211> 25
   <212> DNA
   <213> Artificial Sequence
<220>
   <223> Primer
<400> 1093
   tcggtacgaa ctggatgtcg ccgtt 25
<210> 1094
   <211> 24
   <212> DNA
   <213> Artificial Sequence
<220>
   <223> Primer
<400> 1094
   tcggtcagca aaacggtagc ttgc 24
<210> 1095
   <211> 21
   <212> DNA
   <213> Artificial Sequence
<220>
   <223> Primer

<400> 1095
   tcggtggtgg tagccgatct c 21
<210> 1096
   <211> 31
   <212> DNA
   <213> Artificial Sequence
<220>
   <223> Primer
<400> 1096
   tcggtttaag ctctacatga tcgtaaggat a 31
<210> 1097
   <211> 29
   <212> DNA
   <213> Artificial Sequence
<220>
   <223> Primer
<400> 1097
   tcggtttcag tcatctccac cataaaggt 29
<210> 1098
   <211> 26
   <212> DNA
   <213> Artificial Sequence
<220>
   <223> Primer
<400> 1098
   tcgtatgacc agcttcggta ctacta 26
<210> 1099
   <211> 32
   <212> DNA
   <213> Artificial Sequence
<220>
   <223> Primer
<400> 1099
   tcgtcaacac taccattatt accatgcatc tc 32
<210> 1100
   <211> 26
   <212> DNA
   <213> Artificial Sequence
<220>
   <223> Primer
<400> 1100
   tcgtccgact taacggtcag catttc 26
<210> 1101
   <211> 29
   <212> DNA
   <213> Artificial Sequence
<220>
   <223> Primer

<400> 1101
   tcgtccgact taacggtcag catttcctg 29
<210> 1102
   <211> 31
   <212> DNA
   <213> Artificial Sequence
<220>
   <223> Primer
<400> 1102
   tcgtccgact taacggtcag catttcctgc a 31
<210> 1103
   <211> 26
   <212> DNA
   <213> Artificial Sequence
<220>
   <223> Primer
<400> 1103
   tcgtcctctc gaatctccga tatacc 26
<210> 1104
   <211> 20
   <212> DNA
   <213> Artificial Sequence
<220>
   <223> Primer
<400> 1104
   tcgtcgcgga cttcgaagcc 20
<210> 1105
   <211> 26
   <212> DNA
   <213> Artificial Sequence
<220>
   <223> Primer
<400> 1105
   tcgtcggact taacggtcag catttc 26
<210> 1106
   <211> 29
   <212> DNA
   <213> Artificial Sequence
<220>
   <223> Primer
<400> 1106
   tcgtcggact taacggtcag catttcctg 29
<210> 1107
   <211> 31
   <212> DNA
   <213> Artificial Sequence
<220>
   <223> Primer
<400> 1107
   tcgtcggact taacggtcag catttcctgc a 31
<210> 1108
   <211> 28
   <212> DNA
   <213> Artificial Sequence
<220>
   <223> Primer
<400> 1108
   tcgtcgtatt tatagtgacc agcaccta 28
<210> 1109
   <211> 28
   <212> DNA
   <213> Artificial Sequence
<220>
   <223> Primer
<400> 1109
   tcgtgcctaa caaatcccgt ctgagttc 28
<210> 1110
   <211> 22
   <212> DNA
   <213> Artificial Sequence
<220>
   <223> Primer
<400> 1110
   tcgtggacta ccagggtatc ta 22
<210> 1111
   <211> 17
   <212> DNA
   <213> Artificial Sequence
<220>
   <223> Primer
<400> 1111
   tcgtgggcct tgccggt 17
<210> 1112
   <211> 28
   <212> DNA
   <213> Artificial Sequence
<220>
   <223> Primer

<400> 1112
   tcgttaatta atctggctgc ggaagtga 28
<210> 1113
   <211> 27
   <212> DNA
   <213> Artificial Sequence
<220>
   <223> Primer
<400> 1113
   tcgttgagat ggtttttacc ttcgttg 27
<210> 1114
   <211> 25
   <212> DNA
   <213> Artificial Sequence
<220>
   <223> Primer
<400> 1114
   tcgtttaagc gccagaaagc accaa 25
<210> 1115
   <211> 21
   <212> DNA
   <213> Artificial Sequence
<220>
   <223> Primer
<400> 1115
   tcgtttcacc ctgtcatgcc g 21
<210> 1116
   <211> 26
   <212> DNA
   <213> Artificial Sequence
<220>
   <223> Primer
<400> 1116
   tctttcgtat aaaaaggacc aattgg 26
<210> 1117
   <211> 35
   <212> DNA
   <213> Artificial Sequence
<220>
   <223> Primer
<400> 1117
   tctacaacac ttgattgtaa tttgccttgt tcttt 35
<210> 1118
   <211> 24
   <212> DNA
   <213> Artificial Sequence
<220>
   <223> Primer
<400> 1118
   tctagcggaa caacagttct gatg 24
<210> 1119
   <211> 27
   <212> DNA
   <213> Artificial Sequence
<220>
   <223> Primer
<400> 1119
   tctatagagt ccggactttc ctcgtga 27
<210> 1120
   <211> 30
   <212> DNA
   <213> Artificial Sequence
<220>
   <223> Primer
<400> 1120
   tctataggta ctgtagtttg ttttccgtct 30
<210> 1121
   <211> 27
   <212> DNA
   <213> Artificial Sequence
<220>
   <223> Primer

<400> 1121
   tctcacctac agctttaaag ccagcaa 27
<210> 1122
   <211> 31
   <212> DNA
   <213> Artificial Sequence
<220>
   <223> Primer
<400> 1122
   tctcacctac agctttaaag ccagcaaaat g 31
<210> 1123
   <211> 25
   <212> DNA
   <213> Artificial Sequence
<220>
   <223> Primer
<400> 1123
   tctcatcccg atattaccgc catga 25
<210> 1124
   <211> 29
   <212> DNA
   <213> Artificial Sequence
<220>
   <223> Primer
<400> 1124
   tctcatgaaa aaggctcagg agatacaag 29
<210> 1125
   <211> 27
   <212> DNA
   <213> Artificial Sequence
<220>
   <223> Primer
<400> 1125
   tctcttaccc caccctttca cccttac 27
<210> 1126
   <211> 31
   <212> DNA
   <213> Artificial Sequence
<220>
   <223> Primer
<400> 1126
   tctctttcaa agcaccattg ctcattatag t 31
<210> 1127
   <211> 24
   <212> DNA
   <213> Artificial Sequence
<220>
   <223> Primer
<400> 1127
   tctgcatttt tgcgagcctg tcta 24
<210> 1128
   <211> 28
   <212> DNA
   <213> Artificial Sequence
<220>
   <223> Primer
<400> 1128
   tctgcctgag atgtcgaaaa aaacgttg 28
<210> 1129
   <211> 26
   <212> DNA
   <213> Artificial Sequence
<220>
   <223> Primer
<400> 1129
   tctggcccct ccatacatgt atttag 26
<210> 1130
   <211> 23
   <212> DNA
   <213> Artificial Sequence
<220>
   <223> Primer

<400> 1130
   tctggctgcg gaagtgaaat cgt 23
<210> 1131
   <211> 23
   <212> DNA
   <213> Artificial Sequence
<220>
   <223> Primer
<400> 1131
   tctgggtgac ctggtgtttt aga 23
<210> 1132
   <211> 20
   <212> DNA
   <213> Artificial Sequence
<220>
   <223> Primer
<400> 1132
   tctgtttcag ttgcaaattc 20
<210> 1133
   <211> 34
   <212> DNA
   <213> Artificial Sequence
<220>
   <223> Primer
<400> 1133
   tcttcacact tttagaatca accgttttat tgtc 34
<210> 1134
   <211> 35
   <212> DNA
   <213> Artificial Sequence
<220>
   <223> Primer
<400> 1134
   tcttcagcgt agtctaataa tttacggaac atttc 35
<210> 1135
   <211> 30
   <212> DNA
   <213> Artificial Sequence
<220>
   <223> Primer
<400> 1135
   tcttccaagg atagatttat ttcttgttcg 30
<210> 1136
   <211> 33
   <212> DNA
   <213> Artificial Sequence
<220>
   <223> Primer
<400> 1136
   tcttctgtaa agggtggttt attattcatc cca 33
<210> 1137
   <211> 32
   <212> DNA
   <213> Artificial Sequence
<220>
   <223> Primer
<400> 1137
   tcttcttctt tcgtataaaa aggaccaatt gg 32
<210> 1138
   <211> 28
   <212> DNA
   <213> Artificial Sequence
<220>
   <223> Primer
<400> 1138
   tcttcttgaa aaattgttgt cccgaaac 28
<210> 1139
   <211> 31
   <212> DNA
   <213> Artificial Sequence
<220>
   <223> Primer
<400> 1139
   tcttctttcg tataaaaagg accaattggt t 31
<210> 1140
   <211> 18
   <212> DNA
   <213> Artificial Sequence
<220>
   <223> Primer

<400> 1140
   tcttgacagc atccgttg 18
<210> 1141
   <211> 32
   <212> DNA
   <213> Artificial Sequence
<220>
   <223> Primer
<400> 1141
   tcttgagcat tggttcttac ttgttttgca ta 32
<210> 1142
   <211> 23
   <212> DNA
   <213> Artificial Sequence
<220>
   <223> Primer
<400> 1142
   tcttgagcca tacgtaccat tgc 23
<210> 1143
   <211> 32
   <212> DNA
   <213> Artificial Sequence
<220>
   <223> Primer
<400> 1143
   tcttggctta ggatgaaaat atagtggtgg ta 32
<210> 1144
   <211> 39
   <212> DNA
   <213> Artificial Sequence
<220>
   <223> Primer
<400> 1144
   tctttaagtt cttccaagga tagatttatt tcttgttcg 39
<210> 1145
   <211> 31
   <212> DNA
   <213> Artificial Sequence
<220>
   <223> Primer
<400> 1145
   tcttttcttt gcttaatttt ccatttgcga t 31
<210> 1146
   <211> 14
   <212> DNA
   <213> Artificial Sequence
<220>
   <223> Primer
<400> 1146
   tgttactgct ggat 14
<210> 1147
   <211> 25
   <212> DNA
   <213> Artificial Sequence
<220>
   <223> Primer
<400> 1147
   tgaacatttg cgacggtata cccat 25
<210> 1148
   <211> 31
   <212> DNA
   <213> Artificial Sequence
<220>
   <223> Primer
<400> 1148
   tgaatatgta atgcaaacca gtctttgtca t 31
<210> 1149
   <211> 21
   <212> DNA
   <213> Artificial Sequence
<220>
   <223> Primer
<400> 1149
   tgaatcttga aacaccatac g 21
<210> 1150
   <211> 26
   <212> DNA
   <213> Artificial Sequence
<220>
   <223> Primer
<400> 1150
   tgaatcttga aacaccatac gtaacg 26
<210> 1151
   <211> 34
   <212> DNA
   <213> Artificial Sequence
<220>
   <223> Primer

<400> 1151
   tgaattatgc aagaagtgat caattttctc acga 34
<210> 1152
   <211> 34
   <212> DNA
   <213> Artificial Sequence
<220>
   <223> Primer
<400> 1152
   tgaattcttt caaagcacca ttgctcatta tagt 34
<210> 1153
   <211> 30
   <212> DNA
   <213> Artificial Sequence
<220>
   <223> Primer
<400> 1153
   tgacaggaca caatctgcat gaagtctgag 30
<210> 1154
   <211> 25
   <212> DNA
   <213> Artificial Sequence
<220>
   <223> Primer
<400> 1154
   tgacccaaag ctgaaagctt tactg 25
<210> 1155
   <211> 28
   <212> DNA
   <213> Artificial Sequence
<220>
   <223> Primer
<400> 1155
   tgaccccaac ctggcctttt gtcgttga 28
<210> 1156
   <211> 20
   <212> DNA
   <213> Artificial Sequence
<220>
   <223> Primer
<400> 1156
   tgaccgttat agttacggcc 20
<210> 1157
   <211> 22
   <212> DNA
   <213> Artificial Sequence
<220>
   <223> Primer
<400> 1157
   tgacggcatc gataccaccg tc 22
<210> 1158
   <211> 20
   <212> DNA
   <213> Artificial Sequence
<220>
   <223> Primer
<400> 1158
   tgacgtcatc cccaccttcc 20
<210> 1159
   <211> 22
   <212> DNA
   <213> Artificial Sequence
<220>
   <223> Primer
<400> 1159
   tgacgtcatc cccaccttcc tc 22
<210> 1160
   <211> 20
   <212> DNA
   <213> Artificial Sequence
<220>
   <223> Primer
<400> 1160
   tgacgtcatg cccaccttcc 20
<210> 1161
   <211> 20
   <212> DNA
   <213> Artificial Sequence
<220>
   <223> Primer

<400> 1161
   tgacgtcatg gccaccttcc 20
<210> 1162
   <211> 32
   <212> DNA
   <213> Artificial Sequence
<220>
   <223> Primer
<400> 1162
   tgacttaaac gtaccatcgc ttcatataca ga 32
<210> 1163
   <211> 27
   <212> DNA
   <213> Artificial Sequence
<220>
   <223> Primer
<400> 1163
   tgactttcct cccccttatc agtctcc 27
<210> 1164
   <211> 31
   <212> DNA
   <213> Artificial Sequence
<220>
   <223> Primer
<400> 1164
   tgagatgtcg aaaaaaacgt tggcaaaata c 31
<210> 1165
   <211> 31
   <212> DNA
   <213> Artificial Sequence
<220>
   <223> Primer
<400> 1165
   tgagatgttg atgatttacc agttccgatt g 31
<210> 1166
   <211> 20
   <212> DNA
   <213> Artificial Sequence
<220>
   <223> Primer
<400> 1166
   tgagcatcag cgtgcgtgct 20
<210> 1167
   <211> 28
   <212> DNA
   <213> Artificial Sequence
<220>
   <223> Primer
<400> 1167
   tgagcatttt tatatccatc tccaccat 28
<210> 1168
   <211> 32
   <212> DNA
   <213> Artificial Sequence
<220>
   <223> Primer
<400> 1168
   tgagccatac gaacaatggt ttcataaaca gc 32
<210> 1169
   <211> 32
   <212> DNA
   <213> Artificial Sequence
<220>
   <223> Primer

<400> 1169
   tgagccatga gtaccatggc ttcataacat gc 32
<210> 1170
   <211> 26
   <212> DNA
   <213> Artificial Sequence
<220>
   <223> Primer
<400> 1170
   tgagcgtgtg gaaaaggact tggatg 26
<210> 1171
   <211> 29
   <212> DNA
   <213> Artificial Sequence
<220>
   <223> Primer
<400> 1171
   tgagctggtg ctatatgaac aataccagt 29
<210> 1172
   <211> 30
   <212> DNA
   <213> Artificial Sequence
<220>
   <223> Primer
<400> 1172
   tgagtcaccc tccacaatgt atagttcaga 30
<210> 1173
   <211> 25
   <212> DNA
   <213> Artificial Sequence
<220>
   <223> Primer
<400> 1173
   tgagtcgggt tcactttacc tggca 25
<210> 1174
   <211> 27
   <212> DNA
   <213> Artificial Sequence
<220>
   <223> Primer
<400> 1174
   tgagtctaca cttggcttag gatgaaa 27
<210> 1175
   <211> 32
   <212> DNA
   <213> Artificial Sequence
<220>
   <223> Primer
<400> 1175
   tgagttaaaa tgcgattgat ttcagtttcc aa 32
<210> 1176
   <211> 32
   <212> DNA
   <213> Artificial Sequence
<220>
   <223> Primer
<400> 1176
   tgagtttgaa ccatttcaga gcgaatatct ac 32
<210> 1177
   <211> 28
   <212> DNA
   <213> Artificial Sequence
<220>
   <223> Primer
<400> 1177
   tgagtttgca cttcaaaaga aattgtgt 28
<210> 1178
   <211> 29
   <212> DNA
   <213> Artificial Sequence
<220>
   <223> Primer
<400> 1178
   tgataaaaag cactaagcga tgaaacagc 29
<210> 1179
   <211> 27
   <212> DNA
   <213> Artificial Sequence
<220>
   <223> Primer

<400> 1179
   tgataatgaa gggaaacctt tttcacg 27
<210> 1180
   <211> 32
   <212> DNA
   <213> Artificial Sequence
<220>
   <223> Primer
<400> 1180
   tgatattgaa ctggtgtacc ataatagttg cc 32
<210> 1181
   <211> 30
   <212> DNA
   <213> Artificial Sequence
<220>
   <223> Primer
<400> 1181
   tgatcctgaa tgtttatatc tttaacgcct 30
<210> 1182
   <211> 22
   <212> DNA
   <213> Artificial Sequence
<220>
   <223> Primer
<400> 1182
   tgatctccat ggcgcggatc tt 22
<210> 1183
   <211> 19
   <212> DNA
   <213> Artificial Sequence
<220>
   <223> Primer
<400> 1183
   tgatgcgggc tggttcaac 19
<210> 1184
   <211> 24
   <212> DNA
   <213> Artificial Sequence
<220>
   <223> Primer
<400> 1184
   tgatgcgggc tggttcaaca agag 24
<210> 1185
   <211> 30
   <212> DNA
   <213> Artificial Sequence
<220>
   <223> Primer
<400> 1185
   tgatggtcta tttcaatggc agttacgaaa 30
<210> 1186
   <211> 19
   <212> DNA
   <213> Artificial Sequence .
<220>
   <223> Primer
<400> 1186
   tgattatcag cggaagtag 19
<210> 1187
   <211> 28
   <212> DNA
   <213> Artificial Sequence
<220>
   <223> Primer
<400> 1187
   tgattcaaat gcagaaccat caaactcg 28
<210> 1188
   <211> 31
   <212> DNA
   <213> Artificial Sequence
<220>
   <223> Primer
<400> 1188
   tgattcgatc atacgagaca ttaaaactga g 31
<210> 1189
   <211> 30
   <212> DNA
   <213> Artificial Sequence
<220>
   <223> Primer
<400> 1189
   tgattggcga taaagtgata ttttctaaaa 30
<210> 1190
   <211> 22
   <212> DNA
   <213> Artificial Sequence
<220>
   <223> Primer
<400> 1190
   tgattgtttt gcagctgatt gt 22
<210> 1191
   <211> 22
   <212> DNA
   <213> Artificial Sequence
<220>
   <223> Primer

<400> 1191
   tgattgtttt gcagctgatt gt 22
<210> 1192
   <211> 30
   <212> DNA
   <213> Artificial Sequence
<220>
   <223> Primer
<400> 1192
   tgcaaaagta acggttacat ctgctccaat 30
<210> 1193
   <211> 33
   <212> DNA
   <213> Artificial Sequence
<220>
   <223> Primer
<400> 1193
   tgcaacaatt aatgctccga caattaaagg att 33
<210> 1194
   <211> 24
   <212> DNA
   <213> Artificial Sequence
<220>
   <223> Primer
<400> 1194
   tgcaactcat ctggtttagg atct 24
<210> 1195
   <211> 32
   <212> DNA
   <213> Artificial Sequence
<220>
   <223> Primer
<400> 1195
   tgcaactgaa tagattgcag taagttataa gc 32
<210> 1196
   <211> 23
   <212> DNA
   <213> Artificial Sequence
<220>
   <223> Primer
<400> 1196
   tgcaagagca accctagtgt tcg 23
<210> 1197
   <211> 29
   <212> DNA
   <213> Artificial Sequence
<220>
   <223> Primer
<400> 1197
   tgcaagggaa acctagaatt acaaaccct 29
<210> 1198
   <211> 29
   <212> DNA
   <213> Artificial Sequence
<220>
   <223> Primer

<400> 1198
   tgcaatgtgt gctatgtcag caaaaagat 29
<210> 1199
   <211> 18
   <212> DNA
   <213> Artificial Sequence
<220>
   <223> Primer
<400> 1199
   tgcacctgcg gtcgagcg 18
<210> 1200
   <211> 24
   <212> DNA
   <213> Artificial Sequence
<220>
   <223> Primer
<400> 1200
   tgcacgcaaa cgctttactt cagc 24
<210> 1201
   <211> 26
   <212> DNA
   <213> Artificial Sequence
<220>
   <223> Primer
<400> 1201
   tgcacgtctg tttcagttgc aaattc 26
<210> 1202
   <211> 13
   <212> DNA
   <213> Artificial Sequence
<220>
   <223> Primer
<400> 1202
   tgcagctgat tgt 13
<210> 1203
   <211> 26
   <212> DNA
   <213> Artificial Sequence
<220>
   <223> Primer
<400> 1203
   tgcataggga aggtaacacc atagtt 26
<210> 1204
   <211> 25
   <212> DNA
   <213> Artificial Sequence
<220>
   <223> Primer
<400> 1204
   tgcatcacca tttccttgtc cttcg 25
<210> 1205
   <211> 35
   <212> DNA
   <213> Artificial Sequence
<220>
   <223> Primer
<400> 1205
   tgcatgaagc ataaaaactg tatcaagtgc tttta 35
<210> 1206
   <211> 36
   <212> DNA
   <213> Artificial Sequence
<220>
   <223> Primer

<400> 1206
   tgcatgctta ctcaaatcat cataaacaat taaagc 36
<210> 1207
   <211> 29
   <212> DNA
   <213> Artificial Sequence
<220>
   <223> Primer
<400> 1207
   tgcattgtac cgaagtagtt cacattgtt 29
<210> 1208
   <211> 25
   <212> DNA
   <213> Artificial Sequence
<220>
   <223> Primer
<400> 1208
   tgccaagtgc tggtttaccc catgg 25
<210> 1209
   <211> 26
   <212> DNA
   <213> Artificial Sequence
<220>
   <223> Primer
<400> 1209
   tgccactttg acaactcctg ttgctg 26
<210> 1210
   <211> 21
   <212> DNA
   <213> Artificial Sequence
<220>
   <223> Primer
<400> 1210
   tgccagcgac agaccatcgt a 21
<210> 1211
   <211> 24
   <212> DNA
   <213> Artificial Sequence
<220>
   <223> Primer
<400> 1211
   tgccagctta gtcatacgga cttc 24
<210> 1212
   <211> 27
   <212> DNA
   <213> Artificial Sequence
<220>
   <223> Primer
<400> 1212
   tgccagtttc cacatttcac gttcgtg 27
<210> 1213
   <211> 30
   <212> DNA
   <213> Artificial Sequence
<220>
   <223> Primer

<400> 1213
   tgccatacgt accatcgttt cataaacagc 30
<210> 1214
   <211> 24
   <212> DNA
   <213> Artificial Sequence
<220>
   <223> Primer
<400> 1214
   tgccatagca aagcctacag catt 24
<210> 1215
   <211> 28
   <212> DNA
   <213> Artificial Sequence
<220>
   <223> Primer
<400> 1215
   tgccatccat aatcacgcca tactgacg 28
<210> 1216
   <211> 30
   <212> DNA
   <213> Artificial Sequence
<220>
   <223> Primer
<400> 1216
   tgccatttcc atgtactctt ctctaacatt 30
<210> 1217
   <211> 27
   <212> DNA
   <213> Artificial Sequence
<220>
   <223> Primer
<400> 1217
   tgcccaccag aaagactagc aggataa 27
<210> 1218
   <211> 20
   <212> DNA
   <213> Artificial Sequence
<220>
   <223> Primer
<400> 1218
   tgcccaggta caacctgcat 20
<210> 1219
   <211> 27
   <212> DNA
   <213> Artificial Sequence
<220>
   <223> Primer
<400> 1219
   tgccccattg ctcatgatag tagctac 27
<210> 1220
   <211> 30
   <212> DNA
   <213> Artificial Sequence
<220>
   <223> Primer
<400> 1220
   tgccctttct aaaagtcttg agtgaagata 30
<210> 1221
   <211> 25
   <212> DNA
   <213> Artificial Sequence
<220>
   <223> Primer

<400> 1221
   tgcccttttg taaaagcagg gctat 25
<210> 1222
   <211> 23
   <212> DNA
   <213> Artificial Sequence
<220>
   <223> Primer
<400> 1222
   tgccgataag ccggattctg tgc 23
<210> 1223
   <211> 28
   <212> DNA
   <213> Artificial Sequence
<220>
   <223> Primer
<400> 1223
   tgccgtaaca tagaagttac cgttgatt 28
<210> 1224
   <211> 33
   <212> DNA
   <213> Artificial Sequence
<220>
   <223> Primer
<400> 1224
   tgccgtaact aacataagag aattatgcaa gaa 33
<210> 1225
   <211> 33
   <212> DNA
   <213> Artificial Sequence
<220>
   <223> Primer
<400> 1225
   tgccgtatac gaaaatatct tatcatttag cgt 33
<210> 1226
   <211> 25
   <212> DNA
   <213> Artificial Sequence
<220>
   <223 Primer
<400> 1226
   tgcctaacaa atcccgtctg agttc 25
<210> 1227
   <211> 20
   <212> DNA
   <213> Artificial Sequence
<220>
   <223> Primer
<400> 1227
   tgcctcgcgc aacctacccg 20
<210> 1228
   <211> 20
   <212> DNA
   <213> Artificial Sequence
<220>
   <223> Primer
<400> 1228
   tgcctcgtgc aacccacccg 20
<210> 1229
   <211> 22
   <212> DNA
   <213> Artificial Sequence
<220>
   <223> Primer

<400> 1229
   tgcgaggaac ttcacgtcct gc 22
<210> 1230
   <211> 30
   <212> DNA
   <213> Artificial Sequence
<220>
   <223> Primer
<400> 1230
   tgcgatggta ggtatcttag caatcattct 30
<210> 1231
   <211> 24
   <212> DNA
   <213> Artificial Sequence
<220>
   <223> Primer
<400> 1231
   tgcgcgagct tttatttggg tttc 24
<210> 1232
   <211> 29
   <212> DNA
   <213> Artificial Sequence
<220>
   <223> Primer
<400> 1232
   tgcgctaatt cttcaacttc ttctttcgt 29
<210> 1233
   <211> 32
   <212> DNA
   <213> Artificial Sequence
<220>
   <223> Primer
<400> 1233
   tgcgctatca acgattttga caatatatgt ga 32
<210> 1234
   <211> 23
   <212> DNA
   <213> Artificial Sequence
<220>
   <223> Primer
<400> 1234
   tgcggcagca ctatcaccat cca 23
<210> 1235
   <211> 21
   <212> DNA
   <213> Artificial Sequence
<220>
   <223> Primer
<400> 1235
   tgcgggctgg ttcaacaaga g 21
<210> 1236
   <211> 23
   <212> DNA
   <213> Artificial Sequence
<220>
   <223> Primer
<400> 1236
   tgcgggtgat acttaccgag tac 23
<210> 1237
   <211> 22
   <212> DNA
   <213> Artificial Sequence
<220>
   <223> Primer
<400> 1237
   tgcggtctgg cgcatatagg ta 22
<210> 1238
   <211> 30
   <212> DNA
   <213> Artificial Sequence
<220>
   <223> Primer
<400> 1238
   tgcgtagtct aataatttac ggaacatttc 30
<210> 1239
   <211> 29
   <212> DNA
   <213> Artificial Sequence
<220>
   <223> Primer

<400> 1239
   tgcgtgacga ccttcttgaa ttgtaatca 29
<210> 1240
   <211> 23
   <212> DNA
   <213> Artificial Sequence
<220>
   <223> Primer
<400> 1240
   tgcgtggact accagggtat cta 23
<210> 1241
   <211> 13
   <212> DNA
   <213> Artificial Sequence
<220>
   <223> Primer
<400> 1241
   tgcagctgat tgt 13
<210> 1242
   <211> 30
   <212> DNA
   <213> Artificial Sequence
<220>
   <223> Primer
<400> 1242
   tgctaaagtc ttgagccata cgaacaatgg 30
<210> 1243
   <211> 24
   <212> DNA
   <213> Artificial Sequence
<220>
   <223> Primer
<400> 1243
   tgctagacct ttacgtgcac cgtg 24
<210> 1244
   <211> 23
   <212> DNA
   <213> Artificial Sequence
<220>
   <223> Primer
<400> 1244
   tgctaggcca tcaggccacg cat 23
<210> 1245
   <211> 34
   <212> DNA
   <213> Artificial Sequence
<220>
   <223> Primer
<400> 1245
   tgctatatgc tacaactggt tcaaaaacat taag 34
<210> 1246
   <211> 29
   <212> DNA
   <213> Artificial Sequence
<220>
   <223> Primer
<400> 1246
   tgctcacctg ctacaacaag tccagcaat 29
<210> 1247
   <211> 29
   <212> DNA
   <213> Artificial Sequence
<220>
   <223> Primer

<400> 1247
   tgctcttacc tcaccgttcc acccttacc 29
<210> 1248
   <211> 29
   <212> DNA
   <213> Artificial Sequence
<220>
   <223> Primer
<400> 1248
   tgctgctttc gcatggttaa ttgcttcaa 29
<210> 1249
   <211> 27
   <212> DNA
   <213> Artificial Sequence
<220>
   <223> Primer
<400> 1249
   tgctgctttg atggctgaat ccccttc 27
<210> 1250
   <211> 22
   <212> DNA
   <213> Artificial Sequence
<220>
   <223> Primer
<400> 1250
   tgctggattc gcctttgcta cg 22
<210> 1251
   <211> 21
   <212> DNA
   <213> Artificial Sequence
<220>
   <223> Primer
<400> 1251
   tgctgtaggg aaatcagggc c 21
<210> 1252
   <211> 18
   <212> DNA
   <213> Artificial Sequence
<220>
   <223> Primer
<400> 1252
   tgcttagatg ctttcagc 18
<210> 1253
   <211> 34
   <212> DNA
   <213> Artificial Sequence
<220>
   <223> Primer
<400> 1253
   tgcttcaaaa cgcattttta cattttcgtt aaag 34
<210> 1254
   <211> 27
   <212> DNA
   <213> Artificial Sequence
<220>
   <223> Primer

<400> 1254
   tgcttcagca cggccaccaa cttctag 27
<210> 1255
   <211> 31
   <212> DNA
   <213> Artificial Sequence
<220>
   <223> Primer
<400> 1255
   tgcttcagcg tagtctaata atttacggaa c 31
<210> 1256
   <211> 20
   <212> DNA
   <213> Artificial Sequence
<220>
   <223> Primer
<400> 1256
   tgcttctctt ccgggtcggc 20
<210> 1257
   <211> 32
   <212> DNA
   <213> Artificial Sequence
<220>
   <223> Primer
<400> 1257
   tgcttgctca aatcatcata aacaattaaa gc 32
<210> 1258
   <211> 31
   <222> DNA
   <213> Artificial Sequence
<220>
   <223> Primer
<400> 1258
   tgcttgctct ttcaagcagt cttgaatgaa g 31
<210> 1259
   <211> 23
   <212> DNA
   <213> Artificial Sequence
<220>
   <223> Primer
<400> 1259
   tgcttggtgg cttcttcgtc gaa 23
<210> 1260
   <211> 32
   <212> DNA
   <213> Artificial Sequence
<220>
   <223> Primer
<400> 1260
   tgctttgtaa tctagttcct gaatagtaac ca 32
<210> 1261
   <211> 30
   <212> DNA
   <213> Artificial Sequence
<220>
   <223> Primer

<400> 1261
   tggaaaactc atgaaattaa agtgaaagga 30
<210> 1262
   <211> 29
   <212> DNA
   <213> Artificial Sequence
<220>
   <223> Primer
<400> 1262
   tggaaaccgg ctaagtgagt accaccatc 29
<210> 1263
   <211> 30
   <212> DNA
   <213> Artificial Sequence
<220>
   <223> Primer
<400> 1263
   tggaacaccg tctttaatta aagtatctcc 30
<210> 1264
   <211> 24
   <212> DNA
   <213> Artificial Sequence
<220>
   <223> Primer
<400> 1264
   tggaatttac cagcgataga cacc 24
<210> 1265
   <211> 20
   <212> DNA
   <213> Artificial Sequence
<220>
   <223> Primer
<400> 1265
   tggaccacgc cgaagaacgg 20
<210> 1266
   <211> 30
   <212> DNA
   <213> Artificial Sequence
<220>
   <223> Primer
<400> 1266
   tggacgatat tcacggttta cccacttata 30
<210> 1267
   <211> 31
   <212> DNA
   <213> Artificial Sequence
<220>
   <223> Primer
<400> 1267
   tggactaata acaatgagct cattgtactg a 31
<210> 1268
   <211> 31
   <212> DNA
   <213> Artificial Sequence
<220>
   <223> Primer

<400> 1268
   tggataattg gtcgtaacaa gggatagtga g 31
<210> 1269
   <211> 27
   <212> DNA
   <213> Artificial Sequence
<220>
   <223> Primer
<400> 1269
   tggatagacg tcatatgaag gtgtgct 27
<210> 1270
   <211> 27
   <212> DNA
   <213> Artificial Sequence
<220>
   <223> Primer
<400> 1270
   tggatcactg cttacgaact cagcttc 27
<210> 1271
   <211> 26
   <212> DNA
   <213> Artificial Sequence
<220>
   <223> Primer
<400> 1271
   tggatgtgct cacgagtctg tggcat 26
<210> 1272
   <211> 23
   <212> DNA
   <213> Artificial Sequence
<220>
   <223> Primer
<400> 1272
   tggcaacagc tcaacacctt tgg 23
<210> 1273
   <211> 26
   <212> DNA
   <213> Artificial Sequence
<220>
   <223> Primer
<400> 1273
   tggcaccgtg ggttgagatg aagtac 26
<210> 1274
   <211> 19
   <212> DNA
   <213> Artificial Sequence
<220>
   <223> Primer
<400> 1274
   tggcacgagc ctgacctgt 19
<210> 1275
   <211> 34
   <212> DNA
   <213> Artificial Sequence
<220>
   <223> Primer
<400> 1275
   tggcagcaat agtttgacgt acaaatgcac acat 34
<210> 1276
   <211> 26
   <212> DNA
   <213> Artificial Sequence
<220>
   <223> Primer
<400> 1276
   tggcatcacc atttccttgt ccttcg 26
<210> 1277
   <211> 29
   <212> DNA
   <213> Artificial Sequence
<220>
   <223> Primer

<400> 1277
   tggccacttt tatcagcaac cttacagtc 29
<210> 1278
   <211> 19
   <212> DNA
   <213> Artificial Sequence
<220>
   <223> Primer
<400> 1278
   tggccgtact ccccaggcg 19
<210> 1279
   <211> 20
   <212> DNA
   <213> Artificial Sequence
<220>
   <223> Primer
<400> 1279
   tggcgatgca ctggcttgag 20
<210> 1280
   <211> 24
   <212> DNA
   <213> Artificial Sequence
<220>
   <223> Primer
<400> 1280
   tggctcataa gacgcgcttg taga 24
<210> 1281
   <211> 22
   <212> DNA
   <213> Artificial Sequence
<220>
   <223> Primer
<400> 1281
   tggctgcgga agtgaaatcg ta 22
<210> 1282
   <211> 19
   <212> DNA
   <213> Artificial Sequence
<220>
   <223> Primer
<400> 1282
   tggctgcttc taagccaac 19
<210> 1283
   <211> 26
   <212> DNA
   <213> Artificial Sequence
<220>
   <223> Primer
<400> 1283
   tggcttgaga atttaggatc cggcac 26
<210> 1284
   <211> 30
   <212> DNA
   <213> Artificial Sequence
<220>
   <223> Primer
<400> 1284
   tgggacgtaa tcgtataaat tcatcatttc 30
<210> 1285
   <211> 36
   <212> DNA
   <213> Artificial Sequence
<220>
   <223> Primer
<400> 1285
   tgggataaca ttggttggaa tataagcaga aacatc 36
<210> 1286
   <211> 30
   <212> DNA
   <213> Artificial Sequence
<220>
   <223> Primer
<400> 1286
   tgggatggag gtgtagaagg tgttatcatc 30
<210> 1287
   <211> 30
   <212> DNA
   <213> Artificial Sequence
<220>
   <223> Primer
<400> 1287
   tgggatggag gtgtagaagg tgttatcatc 30
<210> 1288
   <211> 32
   <212> DNA
   <213> Artificial Sequence
<220>
   <223> Primer
<400> 1288
   tgggcaccat ttatccacaa attgattggt at 32
<210> 1289
   <211> 31
   <212> DNA
   <213> Artificial Sequence
<220>
   <223> Primer

<400> 1289
   tggggacttc cttaccactt ttagtatcta a 31
<210> 1290
   <211> 33
   <212> DNA
   <213> Artificial Sequence
<220>
   <223> Primer
<400> 1290
   tggggatatg gaggtgtaga aggtgttatc atc 33
<210> 1291
   <211> 27
   <212> DNA
   <213> Artificial Sequence
<220>
   <223> Primer
<400> 1291
   tggggtaaga cgcggctagc atgtatt 27
<210> 1292
   <211> 25
   <212> DNA
   <213> Artificial Sequence
<220>
   <223> Primer
<400> 1292
   tgggtacgaa ctggatgtcg ccgtt 25
<210> 1293
   <211> 27
   <212> DNA
   <213> Artificial Sequence
<220>
   <223> Primer
<400> 1293
   tgggtaggtt tttatctgtg acgcctt 27
<210> 1294
   <211> 26
   <212> DNA
   <213> Artificial Sequence
<220>
   <223> Primer
<400> 1294
   tgggtctaca cctgcacttg cataac 26
<210> 1295
   <211> 24
   <212> DNA
   <213> Artificial Sequence
<220>
   <223> Primer
<400> 1295
   tgggtgctgg tttaccccat ggag 24
<210> 1296
   <211> 29
   <212> DNA
   <213> Artificial Sequence
<220>
   <223> Primer

<400> 1296
   tgggttgcgt tgcagattat ctttaccaa 29
<210> 1297
   <211> 25
   <212> DNA
   <213> Artificial Sequence
<220>
   <223> Primer
<400> 1297
   tgggtttcgc gcttagatgc tttca 25
<210> 1298
   <211> 18
   <212> DNA
   <213> Artificial Sequence
<220>
   <223> Primer
<400> 1298
   tggtaaccct tgtctttg 18
<210> 1299
   <211> 31
   <212> DNA
   <213> Artificial Sequence
<220>
   <223> Primer
<400> 1299
   tggtaaccct tgtctttgaa ttgtatttgc a 31
<210> 1300
   <211> 33
   <212> DNA
   <213> Artificial Sequence
<220>
   <223> Primer
<400> 1300
   tggtacaaca tcgttagctt taccactttc acg 33
<210> 1301
   <211> 32
   <212> DNA
   <213> Artificial Sequence
<220>
   <223> Primer
<400> 1301
   tggtacacct ggtttcgttt tgatgatttg ta 32
<210> 1302
   <211> 30
   <212> DNA
   <223> Artificial Sequence
<220>
   <223> Primer
<400> 1302
   tggtacttca acttcatcca ttatgaagtc 30
<210> 1303
   <211> 31
   <212> DNA
   <213> Artificial Sequence
<220>
   <223> Primer
<400> 1303
   tggtatattc gttaattaat ctggctgcgg a 31
<210> 1304
   <211> 21
   <212> DNA
   <213> Artificial Sequence
<220>
   <223> Primer
<400> 1304
   tggtctgagt acctcctttg c 21
<210> 1305
   <211> 31
   <212> DNA
   <213> Artificial Sequence
<220>
   <223> Primer
<400> 1305
   tggtgggtat cttagcaatc attctaatag c 31
<210> 1306
   <211> 32
   <212> DNA
   <213> Artificial Sequence
<220>
   <223> Primer

<400> 1306
   tggtgttcta gtatagattg aggtagtggt ga 32
<210> 1307
   <211> 24
   <212> DNA
   <213> Artificial Sequence
<220>
   <223> Primer
<400> 1307
   tggttagaag tcgtaacgtg gacc 24
<210> 1308
   <211> 25
   <212> DNA
   <213> Artificial Sequence
<220>
   <223> Primer
<400> 1308
   tggttcaaca agagttgccg ttgca 25
<210> 1309
   <211> 31
   <212> DNA
   <213> Artificial Sequence
<220>
   <223> Primer
<400> 1309
   tggttcttac ttgctttgca taaactttcc a 31
<210> 1310
   <211> 31
   <212> DNA
   <213> Artificial Sequence
<220>
   <223> Primer
<400> 1310
   tggttgtagt tcctgtagtt gttgcattaa c 31
<210> 1311
   <211> 30
   <212> DNA
   <213> Artificial Sequence
<220>
   <223> Primer
<400> 1311
   tggtttgtca gaatcacgtt ctggagttgg 30
<210> 1312
   <211> 28
   <212> DNA
   <213> Artificial Sequence
<220>
   <223> Primer
<400> 1312
   tgtaaaagca gggctataat aaggactc 28
<210> 1313
   <211> 29
   <212> DNA
   <213> Artificial Sequence
<220>
   <223> Primer
<400> 1313
   tgtaaattcc gcaaagactt tggcattag 29
<210> 1314
   <211> 29
   <212> DNA
   <213> Artificial Sequence
<220>
   <223> Primer
<400> 1314
   tgtaaccctt gtctttgaat tgtatttgc 29
<210> 1315
   <211> 31
   <212> DNA
   <213> Artificial Sequence
<220>
   <223> Primer
<400> 1315
   tgtaattaac cgaaggttct gtagaagtat g 31
<210> 1316
   <211> 28
   <212> DNA
   <213> Artificial Sequence
<220>
   <223> Primer

<400> 1316
   tgtacaagga ccattataat caatgcca 28
<210> 1317
   <211> 31
   <212> DNA
   <213> Artificial Sequence
<220>
   <223> Primer
<400> 1317
   tgtacaataa ggagtcacct tatgtccctt a 31
<210> 1318
   <211> 30
   <212> DNA
   <213> Artificial Sequence
<220>
   <223> Primer
<400> 1318
   tgtacaccat ttatccacaa attgattggt 30
<210> 1319
   <211> 29
   <212> DNA
   <213> Artificial Sequence
<220>
   <223> Primer
<400> 1319
   tgtaggcaag tgcataagaa attgataca 29
<210> 1320
   <211> 27
   <212> DNA
   <213> Artificial Sequence
<220>
   <223> Primer
<400> 1320
   tgtcaatatg aaggtgctct gtggata 27
<210> 1321
   <211> 28
   <212> DNA
   <213> Artificial Sequence
<220>
   <223> Primer
<400> 1321
   tgtcaccagc ttcagcgtag tctaataa 28
<210> 1322
   <211> 19
   <212> DNA
   <213> Artificial Sequence
<220>
   <223> Primer
<400> 1322
   tgtcactccc gacacgcca 19
<210> 1323
   <211> 29
   <212> DNA
   <213> Artificial Sequence
<220>
   <223> Primer
<400> 1323
   tgtcagctaa gctaataacg tttgtagag 29
<210> 1324
   <211> 26
   <212> DNA
   <213> Artificial Sequence
<220>
   <223> Primer
<400> 1324
   tgtcatcaag caccccaaaa tgaact 26
<210> 1325
   <211> 28
   <212> DNA
   <213> Artificial Sequence
<220>
   <223> Primer

<400> 1325
   tgtccgactt gacggtcaac atttcctg 28
<210> 1326
   <211> 28
   <212> DNA
   <213> Artificial Sequence
<220>
   <223> Primer
<400> 1326
   tgtccgactt gacggtcagc atttcctg 28
<210> 1327
   <211> 28
   <212> DNA
   <213> Artificial Sequence
<220>
   <223> Primer
<400> 1327
   tgtccgactt gacggttagc atttcctg 28
<210> 1328
   <211> 21
   <212> DNA
   <213> Artificial Sequence
<220>
   <223> Primer
<400> 1328
   tgtcgcagca tctgttcctg c 21
<210> 1329
   <211> 29
   <212> DNA
   <213> Artificial Sequence
<220>
   <223> Primer
<400> 1329
   tgtctattgt cgattgttac ctgtacagt 29
<210> 1330
   <211> 27
   <212> DNA
   <213> Artificial Sequence
<220>
   <223> Primer
<400> 1330
   tgtgaacatt tgcgacggta tacccat 27
<210> 1331
   <211> 29
   <212> DNA
   <213> Artificial Sequence
<220>
   <223> Primer
<400> 1331
   tgtgaagaac tttcaaatct gtgaatcca 29
<210> 1332
   <211> 24
   <212> DNA
   <213> Artificial Sequence
<220>
   <223> Primer
<400> 1332
   tgtgatatgg aggtgtagaa ggtg 24
<210> 1333
   <211> 27
   <212> DNA
   <213> Artificial Sequence
<220>
   <223> Primer
<400> 1333
   tgtgatatgg aggtgtagaa ggtgtta 27
<210> 1334
   <211> 24
   <212> DNA
   <213> Artificial Sequence
<220>
   <223> Primer

<400> 1334
   tgtgcaggca tcatgtcata ccaa 24
<210> 1335
   <211> 31
   <212> DNA
   <213> Artificial Sequence
<220>
   <223> Primer
<400> 1335
   tgtgctgctt tcgcatggtt aattgcttca a 31
<210> 1336
   <211> 22
   <212> DNA
   <213> Artificial Sequence
<220>
   <223> Primer
<400> 1336
   tgtgctggtt taccccatgg ag 22
<210> 1337
   <211> 23
   <212> DNA
   <213> Artificial Sequence
<220>
   <223> Primer
<400> 1337
   tgtgctggtt taccccatgg agt 23
<210> 1338
   <211> 15
   <212> DNA
   <213> Artificial Sequence
<220>
   <223> Primer
<400> 1338
   tgtgctttga atgct 15
<210> 1339
   <211> 28
   <212> DNA
   <213> Artificial Sequence
<220>
   <223> Primer
<400> 1339
   tgtgcttttt ttgctgccat agcaaagc 28
<210> 1340
   <211> 26
   <212> DNA
   <213> Artificial Sequence
<220>
   <223> Primer
<400> 1340
   tgtggccgat ttcaccacct gctcct 26
<210> 1341
   <211> 18
   <212> DNA
   <213> Artificial Sequence
<220>
   <223> Primer
<400> 1341
   tgtgttgtcg ccgcgcag 18
<210> 1342
   <211> 20
   <212> DNA
   <213> Artificial Sequence
<220>
   <223> Primer
<400> 1342
   tgttaacggc ttcaagaccc 20
<210> 1343
   <211> 28
   <212> DNA
   <213> Artificial Sequence
<220>
   <223> Primer
<400> 1343
   tgttaagtgt gttgcggctg tctttatt 28
<210> 1344
   <211> 36
   <212> DNA
   <213> Artificial Sequence
<220>
   <223> Primer
<400> 1344
   tgttaatggt aacccttgtc tttgaattgt atttgc 36
<210> 1345
   <211> 22
   <212> DNA
   <213> Artificial Sequence
<220>
   <223> Primer

<400> 1345
   tgttactcac ccgtctgcca ct 22
<210> 1346
   <211> 14
   <212> DNA
   <213> Artificial Sequence
<220>
   <223> Primer
<400> 1346
   tgttactgct ggat 14
<210> 1347
   <211> 34
   <212> DNA
   <213> Artificial Sequence
<220>
   <223> Primer
<400> 1347
   tgttcatgtt taaatgatca ggataaaaag cact 34
<210> 1348
   <211> 30
   <212> DNA
   <213> Artificial Sequence
<220>
   <223> Primer
<400> 1348
   tgttccaata gcagttccgc ccaaattgat 30
<210> 1349
   <211> 30
   <212> DNA
   <213> Artificial Sequence
<220>
   <223> Primer
<400> 1349
   tgttctggat tgattgcaca atcaccaaag 30
<210> 1350
   <211> 30
   <212> DNA
   <213> Artificial Sequence
<220>
   <223> Primer
<400> 1350
   tgttcttgat acacctggtt tcgttttgat 30
<210> 1351
   <211> 30
   <212> DNA
   <213> Artificial Sequence
<220>
   <223> Primer
<400> 1351
   tgttgaagct gtacttgacc tgattttacg 30
<210> 1352
   <211> 19
   <212> DNA
   <213> Artificial Sequence
<220>
   <223> Primer
<400> 1352
   tgttgaccat gcttcttag 19
<210> 1353
   <211> 28
   <212> DNA
   <213> Artificial Sequence
<220>
   <223> Primer
<400> 1353
   tgttgtgccg cagtcaaata tctaaata 28
<210> 1354
   <211> 25
   <212> DNA
   <213> Artificial Sequence
<220>
   <223> Primer

<400> 1354
   tgtttgtgat gcatttgctg agcta 25
<210> 1355
   <211> 33
   <212> DNA
   <213> Artificial Sequence
<220>
   <223> Primer
<400> 1355
   tgttttatgt gtagttgagc ttactacatg agc 33
<210> 1356
   <211> 29
   <212> DNA
   <213> Artificial Sequence
<220>
   <223> Primer
<400> 1356
   tgttttgtat ccaagtgctg gtttacccc 29
<210> 1357
   <211> 11
   <212> DNA
   <213> Artificial Sequence
<220>
   <223> Primer
<400> 1357
   tactcatgcc a 11
<210> 1358
   <211> 11
   <212> DNA
   <213> Artificial Sequence
<220>
   <223> Primer
<400> 1358
   tattcttcgt t 11
<210> 1359
   <211> 17
   <212> DNA
   <213> Artificial Sequence
<220>
   <223> Primer
<400> 1359
   ttacttctaa cccactc 17
<210> 1360
   <211> 27
   <212> DNA
   <213> Artificial Sequence
<220>
   <223> Primer
<400> 1360
   ttaatctggc tgcggaagtg aaatcgt 27
<210> 1361
   <211> 28
   <212> DNA
   <213> Artificial Sequence
<220>
   <223> Primer
<400> 1361
   ttaccatctt caaatacccg aacagtaa 28
<210> 1362
   <211> 26
   <212> DNA
   <213> Artificial Sequence
<220>
   <223> Primer
<400> 1362
   ttaccgagca ggttctgacg gaaacg 26
<210> 1363
   <211> 20
   <212> DNA
   <213> Artificial Sequence
<220>
   <223> Primer

<400> 1363
   ttacgccatc aggccacgca 20
<210> 1364
   <211> 17
   <212> DNA
   <213> Artificial Sequence
<220>
   <223> Primer
<400> 1364
   ttactcadcc gtccgcc 17
<210> 1365
   <211> 20
   <212> DNA
   <213> Artificial Sequence
<220>
   <223> Primer
<400> 1365
   ttactcaccc gtccgccgct 20
<210> 1366
   <211> 34
   <212> DNA
   <213> Artificial Sequence
<220>
   <223> Primer
<400> 1366
   ttacttcctt accactttta gtatctaaag cata 34
<210> 1367
   <211> 17
   <212> DNA
   <213> Artificial Sequence
<220>
   <223> Primer
<400> 1367
   ttacttctaa cccactc 17
<210> 1368
   <211> 21
   <212> DNA
   <213> Artificial Sequence
<220>
   <223> Primer
<400> 1368
   ttagaagtcg taacgtggac c 21
<210> 1369
   <211> 22
   <212> DNA
   <213> Artificial Sequence
<220>
   <223> Primer
<400> 1369
   ttagatgctt tcagcactta tc 22
<210> 1370
   <211> 26
   <212> DNA
   <213> Artificial Sequence
<220>
   <223> Primer
<400> 1370
   ttcaaaacct tgctctcgcc aaacaa 26
<210> 1371
   <211> 24
   <212> DNA
   <213> Artificial Sequence
<220>
   <223> Primer
<400> 1371
   ttcaaaagtt gctcgagacc attg 24
<210> 1372
   <211> 23
   <212> DNA
   <213> Artificial Sequence
<220>
   <223> Primer

<400> 1372
   ttcaaaatgc ggaggcgtat gtg 23
<210> 1373
   <211> 22
   <212> DNA
   <213> Artificial Sequence
<220>
   <223> Primer
<400> 1373
   ttcaacaaga gttgccgttg ca 22
<210> 1374
   <211> 28
   <212> DNA
   <213> Artificial Sequence
<220>
   <223> Primer
<400> 1374
   ttcaacactc tcacctacag ctttaaag 28
<210> 1375
   <211> 24
   <212> DNA
   <213> Artificial Sequence
<220>
   <223> Primer
<400> 1375
   ttcaagtgct tgctcaccat tgtc 24
<210> 1376
   <211> 26
   <212> DNA
   <213> Artificial Sequence
<220>
   <223> Primer
<400> 1376
   ttcaggtaca gcaggtggtt caggat 26
<210> 1377
   <211> 23
   <212> DNA
   <213> Artificial Sequence
<220>
   <223> Primer
<400> 1377
   ttcaggtcca tcgggttcat gcc 23
<210> 1378
   <211> 29
   <212> DNA
   <213> Artificial Sequence
<220>
   <223> Primer
<400> 1378
   ttcataagca atacctttac ttgcaccac 29
<210> 1379
   <211> 30
   <212> DNA
   <213> Artificial Sequence
<220>
   <223> Primer
<400> 1379
   ttcattttct ggtccaaagt aagcagtatc 30
<210> 1380
   <211> 25
   <212> DNA
   <213> Artificial Sequence
<220>
   <223> Primer
<400> 1380
   ttccaagtgc tggtttaccc catgg 25
<210> 1381
   <211> 33
   <212> DNA
   <213> Artificial Sequence
<220>
   <223> Primer
<400> 1381
   ttccaccttg gatacctgga aaaatagctg aat 33
<210> 1382
   <211> 36
   <212> DNA
   <213> Artificial Sequence
<220>
   <223> Primer

<400> 1382
   ttccatttca actaattcta ataattcttc atcgtc 36
<210> 1383
   <211> 28
   <212> DNA
   <213> Artificial Sequence
<220>
   <223> Primer
<400> 1383
   ttcccctgac cttcgattaa aggatagc 28
<210> 1384
   <211> 27
   <212> DNA
   <213> Artificial Sequence
<220>
   <223> Primer
<400> 1384
   ttcgcgcatc caggagaagt acatgtt 27
<210> 1385
   <211> 15
   <212> DNA
   <213> Artificial Sequence
<220>
   <223> Primer
<400> 1385
   ttcgctcgcc gctac 15
<210> 1386
   <211> 18
   <212> DNA
   <213> Artificial Sequence
<220>
   <223> Primer
<400> 1386
   ttcgctctcg gcctggcc 18
<210> 1387
   <211> 22
   <212> DNA
   <213> Artificial Sequence
<220>
   <223> Primer
<400> 1387
   ttcggtataa cgcatcgcag ca 22
<210> 1388
   <211> 26
   <212> DNA
   <213> Artificial Sequence
<220>
   <223> Primer
<400> 1388
   ttcgtgctgg attttgtcct tgtcct 26
<210> 1389
   <211> 21
   <212> DNA
   <213> Artificial Sequence
<220>
   <223> Primer
<400> 1389
   ttcgtgctta gatgctttca g 21
<210> 1390
   <211> 24
   <212> DNA
   <213> Artificial Sequence
<220>
   <223> Primer
<400> 1390
   ttctgagcta aatcagcagt tgca 24
<210> 1391
   <211> 23
   <212> DNA
   <213> Artificial Sequence
<220>
   <223> Primer
<400> 1391
   ttctgcgaat caatcgcacg ctg 23
<210> 1392
   <211> 24
   <212> DNA
   <213> Artificial Sequence
<220>
   <223> Primer

<400> 1392
   ttctgcttga ggaatagtgc gtgg 24
<210> 1393
   <211> 24
   <212> DNA
   <213> Artificial Sequence
<220>
   <223> Primer
<400> 1393
   ttctgggtga cctggtgttt taga 24
<210> 1394
   <211> 31
   <212> DNA
   <213> Artificial Sequence
<220>
   <223> Primer
<400> 1394
   ttcttccaag gatagattta tttcttgttc g 31
<210> 1395
   <211> 24
   <212> DNA
   <213> Artificial Sequence
<220>
   <223> Primer
<400> 1395
   ttcttgaacg cgaggtttcg attg 24
<210> 1396
   <211> 22
   <212> DNA
   <213> Artificial Sequence
<220>
   <223> Primer
<400> 1396
   ttgacatcgt ccctcttcac ag 22
<210> 1397
   <211> 26
   <212> DNA
   <213> Artificial Sequence
<220>
   <223> Primer
<400> 1397
   ttgacatttg catgcttcaa agcctg 26
<210> 1398
   <211> 23
   <212> DNA
   <213> Artificial Sequence
<220>
   <223> Primer
<400> 1398
   ttgacgtcat ccccaccttc ctc 23
<210> 1399
   <211> 24
   <212> DNA
   <213> Artificial Sequence
<220>
   <223> Primer

<400> 1399
   ttgacgttgc atgttcgagc ccat 24
<210> 1400
   <211> 30
   <212> DNA
   <213> Artificial Sequence
<220>
   <223> Primer
<400> 1400
   ttgcaatcga catatccatt tcaccatgcc 30
<210> 1401
   <211> 27
   <212> DNA
   <213> Artificial Sequence
<220>
   <223> Primer
<400> 1401
   ttgcacgtct gtttcagttg caaattc 27
<210> 1402
   <211> 27
   <212> DNA
   <213> Artificial Sequence
<220>
   <223> Primer
<400> 1402
   ttgcacgtct gtttcagttg caaattc 27
<210> 1403
   <211> 20
   <212> DNA
   <213> Artificial Sequence
<220>
   <223> Primer
<400> 1403
   ttgcatcggg ttggtaagtc 20
<210> 1404
   <211> 27
   <212> DNA
   <213> Artificial Sequence
<220>
   <223> Primer
<400> 1404
   ttgccacttt gacaactcct gttgctg 27
<210> 1405
   <211> 25
   <212> DNA
   <213> Artificial Sequence
<220>
   <223> Primer
<400> 1405
   ttgccatagc aaagcctaca gcatt 25
<210> 1406
   <211> 30
   <212> DNA
   <213> Artificial Sequence
<220>
<220> Primer
<400> 1406
   ttgccattca tggtatttaa gtgtagcaga 30
<210> 1407
   <211> 21
   <212> DNA
   <213> Artificial Sequence
<220>
   <223> Primer

<400> 1407
   ttgcgccata cgtaccatcg t 21
<210> 1408
   <211> 25
   <212> DNA
   <213> Artificial Sequence
<220>
   <223> Primer
<400> 1408
   ttgcgttgca gattatcttt accaa 25
<210> 1409
   <211> 25
   <212> DNA
   <213> Artificial Sequence
<220>
   <223> Primer
<400> 1409
   ttgctgccat agcaaagcct acagc 25
<210> 1410
   <211> 30
   <212> DNA
   <213> Artificial Sequence
<220>
   <223> Primer
<400> 1410
   ttgctgcttt cgcatggtta atcgcttcaa 30
<210> 1411
   <211> 30
   <212> DNA
   <213> Artificial Sequence
<220>
   <223> Primer
<400> 1411
   ttgctgcttt cgcatggtta attgcttcaa 30
<210> 1412
   <211> 27
   <212> DNA
   <213> Artificial Sequence
<220>
   <223> Primer
<400> 1412
   ttggacctgt aatcagctga atactgg 27
<210> 1413
   <211> 22
   <212> DNA
   <213> Artificial Sequence
<220>
   <223> Primer
<400> 1413
   ttggccatca gaccacgcat ac 22
<210> 1414
   <211> 22
   <212> DNA
   <213> Artificial Sequence

<220>
   <223> Primer
<400> 1414
   ttggccatca ggccacgcat ac 22
<210> 1415
   <211> 28
   <212> DNA
   <213> Artificial Sequence
<220>
   <223> Primer
<400> 1415
   ttggcgacgg tatacccata gctttata 28
<210> 1416
   <211> 17
   <212> DNA
   <213> Artificial Sequence
<220>
   <223> Primer
<400> 1416
   ttggtgcgct tggcgta 17
<210> 1417
   <211> 32
   <212> DNA
   <213> Artificial Sequence
<220>
   <223> Primer
<400> 1417
   ttggttctta cttgttttgc ataaactttc ca 32
<210> 1418
   <211> 35
   <212> DNA
   <213> Artificial Sequence
<220>
   <223> Primer
<400> 1418
   ttgtacattt gaaacaatat gcatgacatg tgaat 35
<210> 1419
   <211> 23
   <212> DNA
   <213> Artificial Sequence
<220>
   <223> Primer
<400> 1419
   ttgtcagact catcgcgaac atc 23
<210> 1420
   <211> 28
   <212> DNA
   <213> Artificial Sequence
<220>
   <223> Primer
<400> 1420
   ttgtgatatg gaggtgtaga aggtgtta 28
<210> 1421
   <211> 26
   <212> DNA
   <213> Artificial Sequence
<220>
   <223> Primer
<400> 1421
   ttgtgattgt tttgcagctg attgtg 26
<210> 1422
   <211> 27
   <212> DNA
   <213> Artificial Sequence
<220>
   <223> Primer
<400> 1422
   ttgtggccga tttcaccacc tgctcct 27
<210> 1423
   <211> 21
   <212> DNA
   <213> Artificial Sequence
<220>
   <223> Primer
<400> 1423
   ttgttaacgg cttcaagacc c 21
<210> 1424
   <211> 32
   <212> DNA
   <213> Artificial Sequence
<220>
   <223> Primer
<400> 1424
   ttgtttattg tttccatatg ctacacactt tc 32
<210> 1425
   <211> 23
   <212> DNA
   <213> Artificial Sequence
<220>
   <223> Primer
<400> 1425
   tttaagcgcc agaaagcacc aac 23
<210> 1426
   <211> 25
   <212> DNA
   <213> Artificial Sequence
<220>
   <223> Primer

<400> 1426
   tttacctcgc ctttccaccc ttacc 25
<210> 1427
   <211> 29
   <212> DNA
   <213> Artificial Sequence
<220>
   <223> Primer
<400> 1427
   tttagctact attctagctg ccatttcca 29
<210> 1428
   <211> 27
   <212> DNA
   <213> Artificial Sequence
<220>
   <223> primer
<400> 1428
   tttatgacca gcttcggtac tactaaa 27
<210> 1429
   <211> 30
   <212> DNA
   <213> Artificial Sequence
<220>
   <223> Primer
<400> 1429
   tttatggtct atttcaatgg cagttacgaa 30
<210> 1430
   <211> 37
   <212> DNA
   <213> Artificial Sequence
<220>
   <223> Primer
<220>
   <221> modified_base
   <222> (23)...(23)
   <223> I
<220>
   <221> modified_base
   <222> (32)...(32)
   <223> I
<400> 1430
   tttcaatacc tttatgcaac tttgtatcaa ctggaat 37
<210> 1431
   <211> 30
   <212> DNA
   <213> Artificial Sequence
<220>
   <223> Primer

<400> 1431
   tttcacagca tgcacgtctg tttcagttgc 30
<210> 1432
   <211> 35
   <212> DNA
   <213> Artificial Sequence
<220>
   <223> Primer
<400> 1432
   tttccccgat ctaaatttgg ataagccata ggaaa 35
<210> 1433
   <211> 27
   <212> DNA
   <213> Artificial Sequence
<220>
   <223> Primer
<400> 1433
   tttccgatgc aacgtaatga gatttca 27
<210> 1434
   <211> 22
   <212> DNA
   <213> Artificial Sequence
<220>
   <223> Primer
<400> 1434
   tttcgtgctt agatgctttc ag 22
<210> 1435
   <211> 30
   <212> DNA
   <213> Artificial Sequence
<220>
   <223> Primer
<400> 1435
   tttcttgaag agtatgagct gctccgtaag 30
<210> 1436
   <211> 22
   <212> DNA
   <213> Artificial Sequence
<220>
   <223> Primer
<400> 1436
   tttgcacctt accgccaaag ct 22
<210> 1437
   <211> 28
   <212> DNA
   <213> Artificial Sequence
<220>
   <223> Primer
<400> 1437
   tttgctcatg atctgcatga agcataaa 28
<210> 1438
   <211> 24
   <212> DNA
   <213> Artificial Sequence
<220>
   <223> Primer

<400> 1438
   tttgctctcc gccaaagttt ccac 24
<210> 1439
   <211> 28
   <212> DNA
   <213> Artificial Sequence
<220>
   <223> Primer
<400> 1439
   tttggacctg taatcagctg aatactgg 28
<210> 1440
   <211> 29
   <212> DNA
   <213> Artificial Sequence
<220>
   <223> Primer
<400> 1440
   tttgtgaaac agcgaacatt ttcttggta 29
<210> 1441
   <211> 20
   <212> DNA
   <213> Artificial Sequence
<220>
   <223> Primer
<400> 1441
   ttttccagcc atgcagcgac 20
<210> 1442
   <211> 34
   <212> DNA
   <213> Artificial Sequence
<220>
   <223> Primer
<220>
   <221> modified_base
   <222> (29) ... (29)
   <223> I
<400> 1442
   ttttcccttt atgcaactta gtatcaactg gaat 34
<210> 1443
   <211> 29
   <212> DNA
   <213> Artificial Sequence
<220>
   <223> Primer
<400> 1443
   ttttgctcat gatctgcatg aagcataaa 29
<210> 1444
   <211> 2118
   <212> DNA
   <213> Artificial Sequence
<220>
   <223> Concatenation of A. baumannii genes
<220>
   <221> misc_feature
   <222> 447-486, 778-817, 1162-1201, 1495-1534, 1928-1967, 2115-2118
   <223> n = A,T,C or G

<400> 1444
   cgcgcggtaa aactaaagaa gaagatatag cattagaaaa agatttgctg tctgatgaaa 60
   aagagattgc tgaacattta atgctgattg atcttgggcg aaacgatgta gggcgtgtat 120
   cgaaaatagg taaagtccaa gtcacggatc aaatggtgat cgagcgttat tcacatgtca 180
   tgcatattgt ttcaaatgta caaggtgaag tgcgtgatga tatcgatgca cttgatgtat 240
   ttaaagccac ctttccagca ggaacgttat caggtgcccc aaaaattcgt gcaatggaaa 300
   ttattgatga agtagaacct gtgaaaaggg gagtttttgg cggggctgtt ggttatttgg 360
   gatggcatgg tgaaatggat atgtcgattg caatccgtac ttgtgttatc cgtgataaaa 420
   aggtgtatgt acaggctggt gcagggnnnn nnnnnnnnnn nnnnnnnnnn nnnnnnnnnn 480
   nnnnnnggaa tctggcggtt tagtttcaga tgaactcatt atcggtttag taaaagaacg 540
   tattgctcaa cctgactgcg tgaatggttg tattttcgac ggcttcccac gcactattcc 600
   tcaagcagaa gctttggaaa aagaagggat cagcattgat catgtaattg aaattgatgt 660
   acctgatgaa gaaatcgtaa aacgtctttc tggtcgtcgt cagcatcctg cttctggtcg 720
   tgtttatcac gttgtataca atccacctaa agtggaaggt aaagatgatg tcacaggnnn 780
   nnnnnnnnnn nnnnnnnnnn nnnnnnnnnn nnnnnnncgt tcaaccgtgt aaaattacgt 840
   aaccttaaaa ctggtaaagt tttagaaaaa acttttaaat ctggtgatac tttagaagct 900
   gctgacatcg tagaagtaga aatgaactac ctatacaacg atggcgaaat gtggcacttc 960
   atggacccag aaagcttcga acaaattgca gctgacaaaa ctgcaatggg tgatgctgct 1020
   aaatggttaa aagacgactc aaatgaaaca tgtacaatca tgttattcaa cggcgttcct 1080
   ttaaacgtaa atgcacctaa cttcgttgta ttgaaagttg ttgaaactga tccgggcgta 1140
   cgtggtgata cttctggtgg tnnnnnnnnn nnnnnnnnnn nnnnnnnnnn nnnnnnnnnn 1200
   ntcgtgcccg yaatttgcat aaagctgccg gccttgtagc acagcaaggc aaatttcctg 1260
   aaactctaga agaatggatt gcactacccg gcattggtcg ctcgaccgca ggtgcactca 1320
   tgtctttagg tttacgtcag tatggcgtga ttatggatgg caacgtgaaa cgcgttttag 1380
   cccgtttctt tgccattgaa gatgacttaa gcaaaccaca gcacgaacgt gaaatgtgga 1440
   aactggctga agagctttgt cccacccaac gcaatcatga ctacactcaa gcgannnnnn 1500
   nnnnnnnnnn nnnnnnnnnn nnnnnnnnnn nnnnttaaaa acactagcgg taagcttaaa 1560
   caagattgcc aatgatattc gttggttagc aagtggtcca cgttgcggct tcggcgaaat 1620
   ccgtattcct gaaaatgaac ctggttcaag tatcatgcca ggtaaagtga acccgactca 1680
   aagtgaagcc atgaccatgg ttgttgctca agtacttggc aacgatacca ctattaatgt 1740
   cgctggtgct tctggtaact tcgagctcaa tgtatttatg ccagtgattg cttataactt 1800
   actgcaatct attcagttgc ttggtgatgc atgtaatagt tttaatgatc actgtgcagt 1860
   agggatcgag ccaaatcgtg agaaaattga tcatttcttg cataattctc ttatgttagt 1920
   tacggcannn nnnnnnnnnn nnnnnnnnnn nnnnnnnnnn nnnnnnnccc ggttatgtac 1980
   caaatacttt gtctgaagat ggtgacccat tagacgtact tgttgtaact ccacatcctg 2040
   ttgctgccgg ttctgtaatt cgttgccgcc cagtgggcaa attaaacatg gaagacgacg 2100
   gtggtatcga tgccnnnn                                               2118
<210> 1445
   <211> 102
   <212> DNA
   <213> Artificial Sequence
<220>
   <223> Calibration Polynucleotide
<400> 1445
   tttaagtccc gcaacgagcg caacccttga tcttagttgt ttagttgggc actctaaggt 60
   gactgccggt gacaaaccgg aggaaggtgg ggatgacgtc aa                    102
<210> 1446
   <211> 94
   <212> DNA
   <213> Artificial Sequence
<220>
   <223> Calibration Polynucleotide
<400> 1446
   tagaacaccg atggcgaagg cgactttctg gtctgtaact gacactgaga aagcgtgggg 60
   agcaaacagg attagatacc ctggtagtcc acga                               94
<210> 1447
   <211> 108
   <212> DNA
   <213> Artificial Sequence
<220>
   <223> Calibration Polynucleotide
<400> 1447
   tggattagag accctggtag tccacgccgt aaacgatgag tgctaagtgt tagaggcctt 60
   tagtgctgaa gttaacgcat taagcactcc gcctggggag tacggcca              108
<210> 1448
   <211> 108
   <212> DNA
   <213> Artificial Sequence
<220>
   <223> Calibration Polynucleotide
<400> 1448
   tttcgatgca acgcgaagaa ccttaccagg tcttgacatc ctctgacaac cctagcttct 60
   ccttcgggag cagagtgaca ggtggtgcat ggctgtcgtc agctcgta              108
<210> 1449
   <211> 95
   <212> DNA
   <213> Artificial Sequence
<220>
   <223> Calibration Polynucleotide
<400> 1449
   tctgacacct gcccggtgct ggaaggttaa ggagaggggt tagcgtaact ctgaactgaa 60
   gccccagtaa acggcggccg taactataac ggtca                              95
<210> 1450
   <211> 117
   <212> DNA
   <213> Artificial Sequence
<220>
   <223> Calibration Polynucleotide
<400> 1450
   tctgttctta gtacgagagg accgggatgg acgcaccggt accagttgtt ctgccaaggg 60
   catagctggg tagctatgtg cggaagggat aagtgctgaa agcatctaag cacgaaa 117
<210> 1451
   <211> 100
   <212> DNA
   <213> Artificial Sequence
<220>
   <223> Calibration Polynucleotide
<400> 1451
   tgattattgt tatcctgtta tgccatttga gatttttgag tggtattgga gttattgttc 60
   caggattaat tgcaaataca attcaaagac aagggttaca 100
<210> 1452
   <211> 112
   <212> DNA
   <213> Artificial Sequence
<220>
   <223> Calibration Polynucleotide
<400> 1452
   tcgaagtaca atacaagaca aaagaaggta aaattactgt tttaggggaa aaattcaaga 60
   aatatagaag tgatggctaa aaatgtagaa ggggtcttga agccgttaac aa 112
<210> 1453
   <211> 100
   <212> DNA
   <213> Artificial Sequence
<220>
   <223> Calibration Polynucleotide

<400> 1453
   ttgctcgtgg tgcacaagta acggatatta caatcattgt tgttgcagct gatgacggcg 60
   taataaacag ttgaagcaat taaccatgcg aaagcagcaa 100
<210> 1454
   <211> 114
   <212> DNA
   <213> Artificial Sequence
<220>
   <223> Calibration Polynucleotide
<400> 1454
   tagcttttgc atattatatc gagccacagc atcgtgatgt tttacagctt tatgcaccgg 60
   aagcttttaa tggataaatt taacgaacaa gaaataaatc tatccttgga agaa 114
<210> 1455
   <211> 116
   <212> DNA
   <213> Artificial Sequence
<220>
   <223> Calibration Polynucleotide
<400> 1455
   tgacctacag taagaggttc tgtaatgaac cctaatgacc atccacacgg tggtggtgaa 60
   ggtagatctc ctatcggaaa gtccacgtac tccatggggt aaaccagcac ttggaa 116
<210> 1456
   <211> 70
   <212> DNA
   <213> Artificial Sequence
<220>
   <223> Calibration Polynucleotide
<400> 1456
   tccacacggt ggtggtgaag gtagatctcc tatcggaaag tccacgtact ccatggggta 60
   aaccagcaca 70
<210> 1457
   <211> 82
   <212> DNA
   <213> Artificial Sequence
<220>
   <223> Calibration Polynucleotide
<400> 1457
   ttatcgctca ggcgaactcc aacctggatg atgaaggccg ctttttagaa ggtgacttgt 60
   cgtagcaaag gcgaatccag ca 82
<210> 1458
   <211> 87
   <212> DNA
   <213> Artificial Sequence
<220>
   <223> Calibration Polynucleotide
<400> 1458
   tgggcagcgt ttcggcgaaa tggaagtggc tcgaagcgta tggcgcttcg tacgtgctgc 60
   aggaaatgtt gaccgtcaag tcggaca 87
<210> 1459
   <211> 97
   <212> DNA
   <213> Artificial Sequence
<220>
   <223> Calibration Polynucleotide
<400> 1459
   tcaggagtcg ttcaactcga tctacatgat ggccgaccgc ccggggttcg gcggtgcaga 60
   ttcgtcagct ggccggcatg cgtggcctga tggcgta 97
<210> 1460
   <211> 117
   <212> DNA
   <213> Artificial Sequence
<220>
   <223> Calibration Polynucleotide
<400> 1460
   tctggcaggt atgcgtggtc tgatggccaa tccatctggt cgtatcatcg aacttccaat 60
   caagtttccg tgaaggttta acagtacttg agtacttcat ctcaacccac ggtgcga 117
<210> 1461
   <211> 98
   <212> DNA
   <213> Artificial Sequence
<220>
   <223> Calibration Polynucleotide
<400> 1461
   tcaagcaaac gcacaatcag aagctaagaa agcgcaagct tctggaaagc acaaatgcta 60
   gttatggtac agaatttgca actgaaacag acgtgcaa 98
<210> 1462
   <211> 99
   <212> DNA
   <213> Artificial Sequence
<220>
   <223> Calibration Polynucleotide
<400> 1462
   tccacacgcc gttcttcaac aactaccgtg ttctacttcc gtacgacgga cgtgacgggc 60
   tcgatcgagc tgccgaagga caaggaaatg gtgatgcca                         99
<210> 1463
   <211> 111
   <212> DNA
   <213> Artificial Sequence
<220>
   <223> Calibration Sequence

<400> 1463
   tcgtggcggc gtggttatcg aacccatgct gaccgatcaa tggtacgtgc acaccgcccc 60
   ccaaagtcgc gattgaagcc gtagagaacg gcgacatcca gttcgtaccg a          111
<210> 1464
   <211> 2100
   <212> DNA
   <213> Artificial Sequence
<220>
   <223> Combination Calibration Polynucleotide
<400> 1464
   gaagtagaga tatggaggaa caccagtggc gaaggcgact ttctggtctg taactgacac 60
   tgagaaagcg tggggagcaa acaggattag ataccctggt agtccacgcc gtaaacgatg 120
   agtgctaagt gttagaggcc tttagtgctg aagttaacgc attaagcact ccgcctgggg 180
   agtacggccg caaggctgaa actcaaagga attgacgggg cacaagcggt ggagcatgtg 240
   gtttaattcg aagcaacgcg aagaacctta ccaggtcttg acatcctctg acaaccctag 300
   cttctccttc gggagcagag tgacaggtgg tgcatggttg tcgtcagctc gtgtcgtgag 360
   atgttgggtt aagtcccgca acgagcgcaa cccttgatct tagttgttta gttgggcact 420
   ctaaggtgac tgccggtgac aaaccggagg aaggtgggga tgacgtcaaa tcatcatgcc 480
   ccagtaccgt gagggaaagg tgaaaagcac cccggaaggg gagtgaaaga gatcctgaaa 540
   ccgtgtgcca tagtcagagc ccgttaacgg gtgatggcgt gccttttgta gaatgaaccg 600
   gcgagttata agatccgtag tcaaaaggga aacagcccag accgccagct aaggtcccaa 660
   agtgtgtatt gaaaaggatg tggagttgct tagacaacta ggatgttggc ttagaagcag 720
   ccaccattta aagagtatag ggggtgacac ctgcccggtg ctggaaggtt aaggagaggg 780
   gttagcgtaa ctctgaactg aagccccagt aaacggcggc cgtaactata acggtcctaa 840
   ggtagcgaaa gaaatttgag aggagctgtc cttagtacga gaggaccggg atggacgcac 900
   cggtaccagt tgttctgcca agggcatagc tgggtagcta tgtgcggaag ggataagtgc 960
   tgaaagcatc taagcatgaa gcccccctca agatgagagc agtaaaacaa gcaaacgcac 1020
   aatcagaagc taagaaagcg caagcttctg gaaagcacaa atgctagtta tggtacagaa 1080
   tttgcaactg aaacagacgt gcatgctgtg aaatttgcga aagcttttgc atattatatc 1140
   gagccacagc atcgtgatgt tttacagctt tatgcaccgg aagcttttaa tggataaatt 1200
   taacgaacaa gaaataaatc tatccttgga agaacttaaa gatcaacgga tgctggcaag 1260
   atatgaaaaa taagataaaa cagcactatc aacactggag cgattcttta tctgaagaag 1320
   gaagagcgat gaaaacaacg aagtacaata caagacaaaa gaaggtaaaa ttactgtttt 1380
   aggggaaaaa ttcaagaaat atagaagtga tggctaaaaa tgtagaaggg gtcttgaagc 1440
   cgttaacagc tgttatggcg accgtggcgg cgtggttatc gaacccatgc tgaccgatca 1500
   atggtacgtg cacaccgccc cccaaagtcg cgattgaagc cgtagagaac ggcgagatcc 1560
   agttcgtccc taaacagtac ggcaacttcg ttatcgctca ggcgaactcc aacctggatg 1620
   atgaaggccg ctttttagaa ggtgacttgt cgtagcaaag gcgaatcaag cctgtttagc 1680
   cacaactatg cgtgctcgtg gtgcacaagt aacggatatt acaatcattg ttgttgcagc 1740
   tgatgacggc gtaataaaca gttgaagcga ttaaccatgc gaaagcagca ggagtaccaa 1800
   ctttactcag cttgctggta tgcgtggtct gatggccaat ccatctggtc gtatcatcga 1860
   acttccaatc aagtttccgt gaaggtttaa cagtacttga gtacttcatc tctacgcatg 1920
   gtgcgcgtaa aggtcatggg agtaagacct acagtaagag gttctgtaat gaaccctaat 1980
   gaccatccac acggtggtgg tgaaggtaga tctcctatcg gaaagtccac gtactccatg 2040
   gggtaaacca gcacttggat acaaaacaag cgcagttcgg cggccagcgc ttcggtgaaa 2100